# EUROPEAN PATENT APPLICATION

(11) **EP 4 775 570 A1**
(43) Date of publication of application: **15.07.2026**
(21) Application number: 24862054.4
(22) Date of filing: 06.09.2024
(51) Int. Cl.: C07D 401/14, C07D 209/44, C07D 401/12, C07D 403/04, C07D 405/14, C07D 413/14, A61P 35/00, A61K 31/4709

(54) **CYP11A1 INHIBITOR, PREPARATION METHOD THEREFOR AND USE THEREOF**

(30) Priority: 08.09.2023 CN 202311163184; 06.06.2024 CN 202410733904
(71) Applicant: Shenzhen Zhongge Biological Technology Co., Ltd., Shenzhen, Guangdong 518017 (CN)
(72) Inventor: HUANG, Minhao, Shenzhen, Guangdong 518017 (CN); HUA, Zihao, Shenzhen, Guangdong 518017 (CN); QIU, Shuwan, Shenzhen, Guangdong 518017 (CN); GOBBO, Gianpaolo, Shenzhen, Guangdong 518017 (CN); LI, Yangming, Shenzhen, Guangdong 518017 (CN); FANG, Dong, Shenzhen, Guangdong 518017 (CN); ZHU, Xinying, Shenzhen, Guangdong 518017 (CN); CHEN, Bin, Shenzhen, Guangdong 518017 (CN); WANG, Shaohui, Shenzhen, Guangdong 518017 (CN); ZHANG, Peiyu, Shenzhen, Guangdong 518017 (CN)
(74) Representative: Botti & Ferrari S.p.A.
(86) International application number: PCT/CN2024/117298
(87) International publication number: WO 2025/051216

(57) **Abstract**

The present application relates to a compound represented by formula (I). The compound can inhibit steroid hormone dependent conditions and diseases of CYP11Al. The present application further comprises a pharmaceutical composition comprising the compound represented by formula (I), and a method for treating condition related to CYP11A1 activity.

## Description

### Cross-Reference to Related Applications

This application claims priority of Chinese Patent Application No. 202311163184.9 filed on September 8, 2023 and Chinese Patent Application No. 202410733904.9 filed on June 6, 2024, the contents of which are incorporated herein by reference in their entirety and for all purposes.

### Technical Field

The present disclosure relates to the field of medicine, specifically relates to a CYP11A1 inhibitor, preparation methods therefor and use thereof.

### Background

The treatment of steroid receptor dependent diseases such as androgen receptor (AR) dependent cancer and estrogen receptor (ER) dependent cancer has been extensively investigated. For example, prostate cancer is one of the most common cancers among men worldwide. Even though patients with localized prostate cancer have a high 5-year survival rate, those who progress to castration-resistant prostate cancer (CRPC) during the 5-year follow-up period have a very poor prognosis. The androgen receptor (AR) signaling axis is crucial in all stages of prostate cancer. During the stage of castration-resistant prostate cancer (CRPC), the disease characterizes in high AR expression, AR amplification, and persistent activation of the AR signaling axis by residual tissue/tumor androgens and by other steroid hormones and intermediates of steroid biosynthesis. Therefore, the treatment of advanced prostate cancer involves androgen deprivation therapy (ADT), such as hormonal manipulation using gonadotropin releasing hormone (GnRH) agonists/antagonists or surgical castration, AR antagonists, or CYP17A1 inhibitors (such as, abiraterone acetate in combination with prednisone). Although such therapies can initially lead to disease regression, majority of patients eventually develop a disease that is refractory to currently available therapies. Increased progesterone levels in patients treated with abiraterone acetate has been hypothesized to be one of the resistance mechanisms. Several nonclinical and clinical studies have indicated upregulation of enzymes that catalyze steroid biosynthesis at the late stage of CRPC. Recently, there have been reports that 11β-OH androstenedione can be metabolized into 11-ketotestosterone (11-K-T) and 11-ketodihydrotestosterone (11-K-DHT) which can bind and activate AR as efficiently as testosterone and dihydrotestosterone. It has been shown that these steroids are found in high levels in plasma and tissue in prostate cancer patients, suggesting their role as AR agonists in CRPC. Furthermore, it has been addressed that prostate cancer resistance to CYP17A1 inhibition may still remain steroid dependent and responsive to therapies that can further suppress de novo intra-tumoral steroid synthesis upstream of CYP17A1, such as by CYP11A1 inhibition therapy (Cai, C. et al., 1(20), 6503-6513, 2011).

CYP11A1, also called cholesterol side chain cleavage enzyme, is a mitochondrial monooxygenase which catalyzes the conversion of cholesterol to pregnenolone, the precursor of all steroid hormones. By inhibiting CYP11A1, the key enzyme of steroid biosynthesis upstream of CYP17A1, the total block of the whole steroid biosynthesis can be achieved. CYP11A1 inhibitors may therefore have a great potential for treating steroid hormone dependent cancers (including prostate cancer), even in advanced stages of the disease, and especially in those patients who appear to be hormone refractory.

### Summary

It has been found that the compound represented by formula (0) are potent CYP11A1 inhibitors. The compounds of the disclosure are therefore useful as medicaments in the treatment of steroid hormone dependent disorders and diseases where CYP11A1 inhibition is desired.

In one aspect, the present disclosure provides a compound represented by formula (0), or a stereoisomer, a tautomer, a diastereoisomer, a racemate, a cis-trans isomer, an isotope labeled compound (preferably deuterated compound), an N-oxide, a metabolite, an ester, a prodrug, a crystalline form, a hydrate, a solvate, or a pharmaceutically acceptable salt thereof: 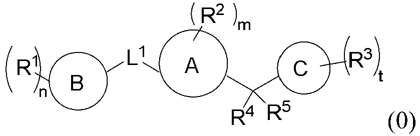 wherein:
ring A is C₆₋₁₀ aryl, 5-6 membered heteroaryl, or 5-12 membered heterocyclyl, wherein the heteroatom in the 5-6 membered heteroaryl is selected from N, O and S, and the number of the heteroatom is 1, 2, 3, or 4; the heteroatom in the 5-12 membered heterocyclyl is selected from N, O and S, the number of the heteroatom is 1, 2, 3, or 4;
L¹ is a bond, O, S, -NH-, C₁₋₆ alkylene that is unsubstituted or substituted by one or more L¹⁻¹, C₂₋₆ alkenylene that is unsubstituted or substituted by one or more L¹⁻², C₂₋₆ alkynylene that is unsubstituted or substituted by one or more L¹⁻³, *-C₁₋₆ alkylene-S- that is unsubstituted or substituted by one or more L¹⁻⁴, *-S-C₁₋₆ alkylene- that is unsubstituted or substituted by one or more L¹⁻⁵, *-C₁₋₆ alkylene-O- that is unsubstituted or substituted by one or more L¹⁻⁶, *-O-C₁₋₆ alkylene- that is unsubstituted or substituted by one or more L¹⁻⁷, *-O-C₆₋₁₀ arylene- that is unsubstituted or substituted by one or more L¹⁻⁸, *-C₆₋₁₀ arylene-O- that is unsubstituted or substituted by one or more L¹⁻⁸, *-SO₂-C₁₋₃ alkylene- that is unsubstituted or substituted by one or more L¹⁻⁹, *-C(=O)-NH-, *-NH-C(=O)-, *-C₁₋₆ alkylene-O-C₁₋₆ alkylene- that is unsubstituted or substituted by one or more L¹⁻¹⁰ or *-C₁₋₆ alkylene-C(O)- that is unsubstituted or substituted by one or more L¹⁻¹⁰; wherein the mark "*" indicates the bond connected to ring B;
preferably, L¹ is a bond, O, -NH-, C₁₋₆ alkylene that is unsubstituted or substituted by one or more L¹⁻¹, C₂₋₆ alkenylene that is unsubstituted or substituted by one or more L¹⁻², C₂₋₆ alkynylene, *-C₁₋₆ alkylene-S-, *-C₁₋₆ alkylene-O-, *-O-C₁₋₆ alkylene-, *-C₆₋₁₀ arylene-O-, *-C₁₋₆ alkylene-C(O)-; wherein the mark "*" indicates the bond connected to ring B;
each L¹⁻¹, L¹⁻², L¹⁻³, L¹⁻⁴, L¹⁻⁵, L¹⁻⁶, L¹⁻⁷, L¹⁻⁸, L¹⁻⁹, L¹⁻¹⁰ is independently halogen, OH, C₁₋₆ alkyl, C₃₋₆ cycloalkyl or 3-6 membered heterocyclyl, wherein the heteroatom in the 3-6 membered heterocyclyl is selected from N, O and S, and the number of the heteroatom is 1, 2, 3, or 4; wherein the C₁₋₆ alkyl, the C₃₋₆ cycloalkyl or the 3-6 membered heterocyclyl is optionally substituted by deuterium, halogen, OH, CN or -NH₂,
ring B is 5-12 membered heterocyclyl, wherein the heteroatom in the 5-12 membered heterocyclyl is selected from N, O and S, and the number of the heteroatom is 1, 2, 3, or 4;
each R¹ is independently deuterium, halogen, cyano, hydroxy, oxo, C₁₋₆ alkoxy, R¹⁻¹, -C(=O)-R¹⁻¹, -(CR¹⁻²R¹⁻³)_{q}-C(=O)-OR¹⁻⁴, NR¹⁻⁷R¹⁻⁸, -C(=O)-NR¹⁻⁷R¹⁻⁸, -C(=NH)-(R¹⁻⁹), -S(O)₂-R¹⁻⁹, -S(O)₂-NR¹⁻⁵R¹⁻⁶, -NR¹⁻⁵S(O)₂-R¹⁻¹⁰, -R¹⁻¹⁰-OH, or -P(=O)-R¹⁻²R¹⁻³;
preferably, each R¹ is independently cyano, hydroxy, -C(=O)-R¹⁻¹, -(CR¹⁻²R¹⁻³)_{q}-C(=O)-OR¹⁻⁴, NR¹⁻⁷R¹⁻⁸, -C(=O)-NR¹⁻⁷R¹⁻⁸, -C(=NH)-(R¹⁻⁹), -S(O)₂-R¹⁻⁹, -S(O)₂-NR¹⁻⁵R¹⁻⁶, -NR¹⁻⁵S(O)₂-R¹⁻¹⁰, -R¹⁻¹⁰-OH, or -P(=O)-R¹⁻²R¹⁻³, or alternatively, two R¹ connected to the same carbon atom form an oxo group (=O);
preferably, each R¹ is independently -C(=O)-R¹⁻¹, -(CR¹⁻²R¹⁻³)_{q}-C(=O)-OR¹⁻⁴, NR¹⁻⁷R¹⁻⁸, -C(=O)-NR¹⁻⁷R¹⁻⁸, -C(=NH)-(R¹⁻⁹), -S(O)₂- R¹⁻⁹, -NR¹⁻⁵S(O)₂-R¹⁻¹⁰, -R¹⁻¹⁰-OH or -P(=O)-R¹⁻²R¹⁻³, or alternatively, two R¹ connected to the same carbon atom form an oxo group (=O);
each R² is independently deuterium, OH, NH₂, halogen, CN, NO₂, C₁₋₆ alkyl, C₂₋₆ alkenyl, C₁₋₆ haloalkyl, C₁₋₆ deuteroalkyl, C₂₋₆ haloalkenyl, -O-C₁₋₆ alkyl, C₁₋₆ alkylene-O-C₁₋₆ alkyl, -O-C₁₋₆ haloalkyl, -O-C₂₋₆ alkenyl, -O-C₃₋₁₀ cyclohydrocarbyl, -S-C₁₋₆ alkyl, -S-C₃₋₁₀ cyclohydrocarbyl, -C₃₋₁₀ cyclohydrocarbyl, 3-10 membered heterocyclyl, C₆₋₁₀ aryl, 5-10 membered heteroaryl, -C₁₋₆ alkylene-C₃₋₁₀ cyclohydrocarbyl, -OC₁₋₆ alkylene-C₃₋₁₀ cyclohydrocarbyl, -C(=O)NR²⁻¹R²⁻², -NR²⁻³-C(=O)-R²⁻⁴, or alternatively, two R² connected to the same carbon atom form an oxo group (=O), wherein the heteroatom in the 3-10 membered heterocyclyl or the 5-10 membered heteroaryl is selected from N, O and S, and the number of the heteroatom is 1, 2, 3, or 4;
optionally, two adjacent R² form 5-6 membered heterocyclyl, wherein the heteroatom in the 5-6 membered heterocyclyl is selected from N, O and S, and the number of the heteroatom is 1, 2, 3, or 4;
R¹⁻¹, R¹⁻², R¹⁻³, R¹⁻⁴, R¹⁻⁵, R¹⁻⁶, R¹⁻⁷ and R¹⁻⁸ are each independently H, C₁₋₆ alkyl, C₁₋₆ haloalkyl, C₁₋₆ deuteroalkyl, -C₁₋₆ alkylene-OH, -C₁₋₆ alkylene-CN, -C₃₋₁₀ cyclohydrocarbyl, or 3-10 membered heterocyclyl, wherein the heteroatom in the 3-10 membered heterocyclyl is selected from N, O and S, and the number of the heteroatom is 1, 2, 3, or 4;
R¹⁻⁹ and R¹⁻¹⁰ are each independently C₁₋₆ alkyl, C₁₋₆ haloalkyl, -C₁₋₆ alkylene-CN, C₂₋₆ alkenyl, C₂₋₆ haloalkenyl, C₃₋₁₀ cyclohydrocarbyl, C₆₋₁₀ aryl, 5-10 membered heteroaryl, C₁₋₆ alkyl-C₆₋₁₀ arylene-;
R²⁻¹, R²⁻², R²⁻³ or R²⁻⁴ are each independently H, halogen or C₁₋₆ alkyl;
ring C is independently 5-membered nitrogen-containing heterocyclobenzene or 5-membered nitrogen-containing heterocyclopyridine;
each R³ is independently cyano, hydroxy, halogen, or C₁₋₆ alkyl, or alternatively, two R³ connected to the same carbon atom form an oxo group (=O);
R⁴ and R⁵ are each independently H, deuterium, halogen, C₁₋₆ alkyl, C₁₋₆ haloalkyl, C₁₋₆ deuteroalkyl or R⁴ and R⁵ together with the atoms to which they are connected form a C₃₋₁₀ cyclohydrocarbyl or a 3-10 membered heterocyclyl,
preferably, R⁴ and R⁵ are each independently H, deuterium, halogen, C₁₋₆ alkyl; more preferably, R⁴ and R⁵ are both H or deuterium, or one of R⁴ and R⁵ is H, and the other is deuterium, halogen, or C₁₋₄ alkyl;
q is 0 or 1;
m, t and n are each independently 0, 1, 2, 3 or 4; and
wherein:
   1) when ring A is benzene ring and L¹ is *-CH₂-O-, wherein the mark "*" indicates the bond connected to ring B, the compound represented by formula (0) is not 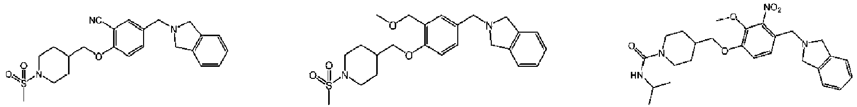 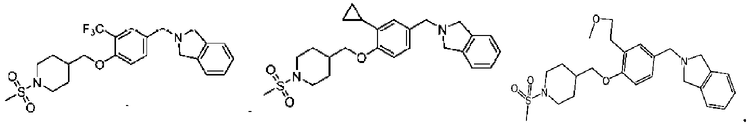 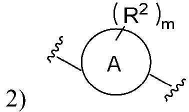 is not

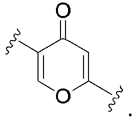

In some embodiments according to the first aspect, each R¹ is independently oxo, -C(=O)-R¹⁻¹, -(CR¹⁻²R¹⁻³)_{q}-C(=O)-OR¹⁻⁴, -C(=O)-NR¹⁻⁷R¹⁻⁸, -C(=NH)-(R¹⁻⁹), -S(O)₂-R¹⁻⁹, -NR¹⁻⁵S(O)₂-R¹⁻¹⁰, -R¹⁻¹⁰-OH or -P(=O)-R¹⁻²R¹⁻³.

In some embodiments according to the first aspect, the R¹ is each independently oxo, -C(=O)-R¹⁻¹, -(CR¹⁻²R¹⁻³)_{q}-C(=O)-OR¹⁻⁴, -C(=O)-NR¹⁻⁷R¹⁻⁸, -(C=NH)-(C₁₋₆ alkyl), -S(O)₂-C₁₋₆ alkyl, -S(O)₂-C₁₋₆ haloalkyl, -S(O)₂-C₆₋₁₀ arylene-C₁₋₆ alkyl, -S(O)₂-C₃₋₁₀ cycloalkyl, -S(O)₂-5-10 membered heteroaryl, -S(O)₂-C₂₋₆ alkenyl, -NR¹⁻⁵S(O)₂-C₁₋₆ alkyl, or -P(=O)-R¹⁻²R¹⁻³ ; preferably oxo, -C(=O)-R¹⁻¹, -C(=O)-OR¹⁻⁴, -C(=O)-NR¹⁻⁷R¹⁻⁸, -(C=NH)-(C₁₋₃ alkyl), -S(O)₂-C₁₋₃ alkyl, -S(O)₂-C₁₋₃ haloalkyl, -S(O)₂-phenylene-C₁₋₃ alkyl, -S(O)₂-C₃₋₆ cycloalkyl, -S(O)₂-5-6 membered heteroaryl, -S(O)₂-C₂₋₃ alkenyl, -NR¹⁻⁵S(O)₂-C₁₋₃ alkyl, -P(=O)-R¹⁻²R¹⁻³ ; more preferably -S(O)₂-C₁₋₃ alkyl, -C(=O)-OC₁₋₆ alkyl, -S(O)₂-C₁₋₃ haloalkyl, -S(O)₂-phenylene-C₁₋₃ alkyl, -S(O)₂-C₃₋₆ cycloalkyl, -S(O)₂-5-6 membered heteroaryl, -C(=O)-C₁₋₆ alkyl, -C(=O)-C₁₋₆ haloalkyl, -C( = O)-C₁₋₆ alkylene-CN, -S(O)₂-C₂₋₃ alkenyl, -C( = O)-NHC₁₋₆ alkyl; most preferably -S(O)₂-C₁₋₃ alkyl;.

In some embodiments according to the first aspect, the R¹⁻¹ and R¹⁻⁴ are each independently H, C₁₋₆ alkyl, preferably C₁₋₃ alkyl, C₁₋₆ haloalkyl, -C₁₋₆ alkylene-CN, or -C₃₋₁₀ cyclohydrocarbyl.

In some embodiments according to the first aspect, the R¹⁻², R¹⁻³, R¹⁻⁵, R¹⁻⁶, R¹⁻⁷ and R¹⁻⁸ are each independently H or C₁₋₆ alkyl, preferably H or C₁₋₃ alkyl.

In some embodiments according to the first aspect, the C₁₋₆ alkyl in R¹⁻¹, R¹⁻², R¹⁻³, R¹⁻⁴, R¹⁻⁵, R¹⁻⁶, R¹⁻⁷, R¹⁻⁸, R¹⁻⁹ and R¹⁻¹⁰ is independently methyl, ethyl, isopropyl, n-butyl, isobutyl or tert-butyl, preferably C₁₋₄ alkyl, more preferably methyl or tert-butyl.

In some embodiments according to the first aspect, the -C₁₋₆ alkylene-CN in R¹⁻¹, R¹⁻², R¹⁻³, R¹⁻⁴, R¹⁻⁵, R¹⁻⁶, R¹⁻⁷, R¹⁻⁸, R¹⁻⁹ and R¹⁻¹⁰ is independently -methylene-CN, -ethylene-CN, -isopropylene-CN, -n-butylene-CN, -isobutylene-CN or -tert-butylene-CN, preferably -C₁₋₄ alkylene-CN, more preferably -methylene-CN.

In some embodiments according to the first aspect, the -C₃₋₁₀ cyclohydrocarbyl in R¹⁻¹, R¹⁻², R¹⁻³, R¹⁻⁴, R¹⁻⁵, R¹⁻⁶, R¹⁻⁷, R¹⁻⁸, R¹⁻⁹ and R¹⁻¹⁰ is independently -C₃₋₁₀ cycloalkyl, preferably -C₃₋₆ cycloalkyl, for example cyclopropyl, cyclobutyl, cyclopentyl or cyclohexyl, and preferably cyclopropyl.

In some embodiments according to the first aspect, the R¹ is

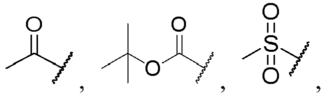

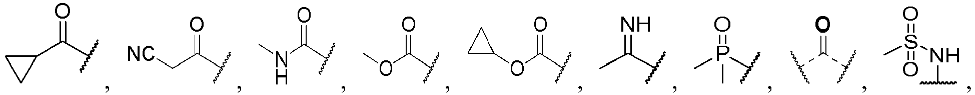

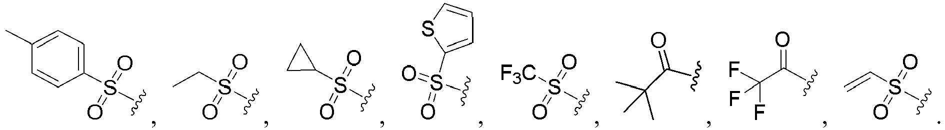

In some embodiments according to the first aspect, the ring B is 5-12 membered heterocyclyl, wherein the heteroatom in the 5-12 membered heterocyclyl is selected from N, O and S, and the number of the heteroatom is 1, 2, 3, or 4, wherein at least one heteroatom is N, preferably the number of the heteroatom is 1, 2 or 3.

In some embodiments according to the first aspect, the ring B is 5-6 membered monocyclic heterocyclyl, 6-12 membered (preferably 9-12 membered) fused bicyclic heterocyclyl or 9-12 membered spirocyclic heterocyclyl; most preferably, the ring B is 6 membered monocyclic heterocyclyl(such as piperidinyl, piperazinyl).

In some embodiments according to the first aspect, the ring B is 5-6 membered monocyclic heterocyclyl or 9-12 membered fused bicyclic heterocyclyl (such as benzo-6-membered heterocycloalkyl or pyrido-6-membered heterocycloalkyl).

In some embodiments according to the first aspect, the 5-6 membered monocyclic heterocyclyl in ring B is independently 5-6 membered monocyclic heterocyclyl, preferably the number of the heteroatom is 1, for example azacyclopentane or azacyclohexane, preferably azacyclohexane.

In some embodiments according to the first aspect, the 6-12 membered fused bicyclic heterocyclyl in ring B is independently 6-10 membered fused bicyclic heterocyclyl, for example benzoazaclohexane, pyridoazacyclohexane, thienoazaclohexane, pyrrolotetrahydropyridinyl, pyrazolotetrahydropyridinyl, imidazolotetrahydropyridinyl, indolinyl, indolinonyl, pyrrolidinocyclopropyl, cyclopentaaziridinyl, pyrrolidinocyclobutyl, pyrrolidinopyrrolidinyl, pyrrolidinopiperidinyl, pyrrolidinopiperazinyl, piperidinomorpholinyl, 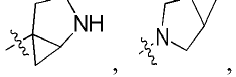 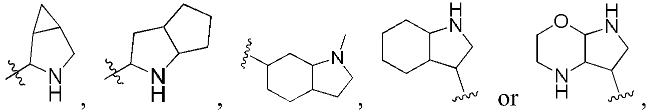 preferably benzoazaclohexane, pyridoazacyclohexane or thienoazaclohexane.

In some embodiments according to the first aspect, the 6-12 membered fused bicyclic heterocyclyl in the ring B is independently 9-12 membered fused bicyclic heterocyclyl, for example benzoazacyclopentane, benzoazaclohexane, benzoazepane, pyridoazacyclohexane, pyrimidoazacyclohexane, thienoazaclohexane, pyrrolidinocyclopentane, preferably benzoazaclohexane, or pyridoazacyclohexane.

In some embodiments according to the first aspect, the 9-12-membered spirocyclic heterocyclyl in ring B is independently 7-10-membered mono-spirocyclic heterocyclyl, optionally wherein the heteroatom may be N or O, preferably 3-membered/4-membered ring system, 3-membered/5-membered ring system, 3-membered/6-membered ring system, 4-membered/4-membered ring system, 4-membered/5-membered ring system, 4-membered/6-membered ring system, 5-membered/5-membered ring system, 5-membered/6-membered ring system and 6-membered/6-membered ring system, for example 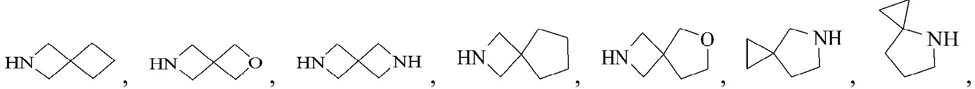 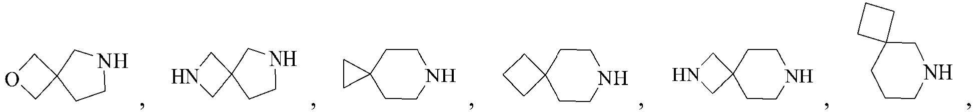 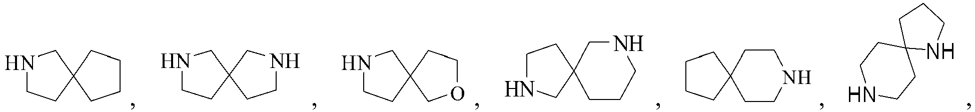 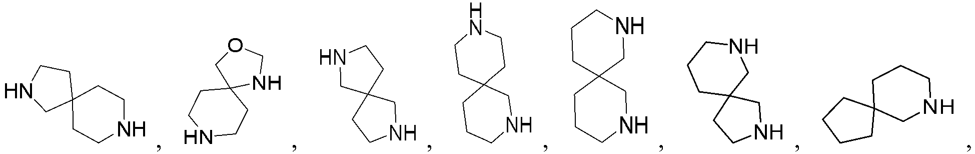 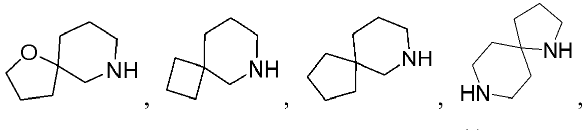 and further for example

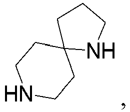

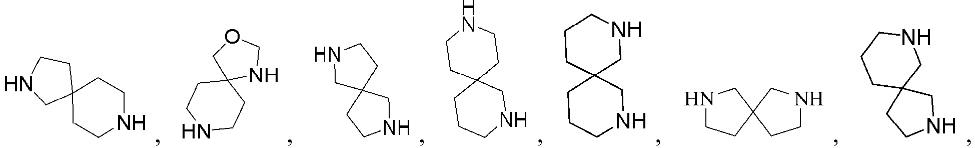

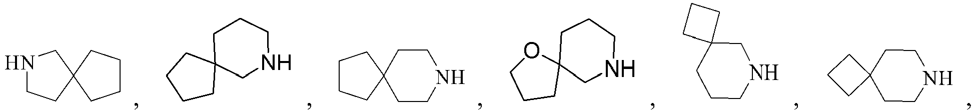

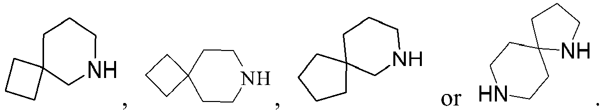

In some embodiments according to the first aspect, the 9-12-membered spirocyclic heterocyclyl in ring B is independently 9-12-membered mono-spirocyclic heterocyclyl, optionally wherein the heteroatom may be N or O, preferably 4-membered/6-membered ring system, 5-membered/5-membered ring system, 5-membered/6-membered ring system and 6-membered/6-membered ring system, for example 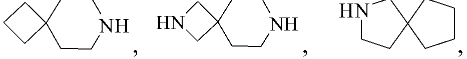 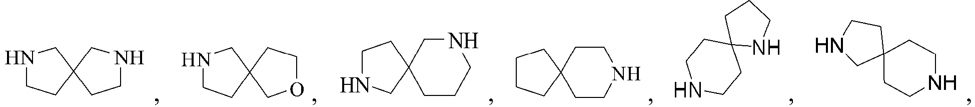 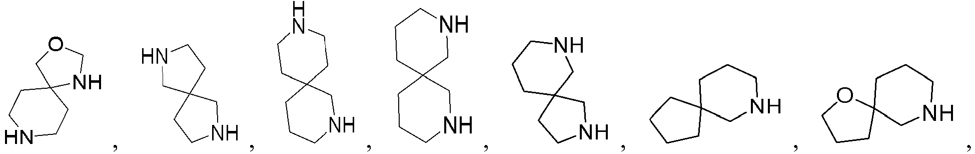 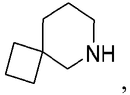 and further for example

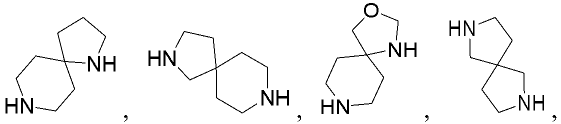

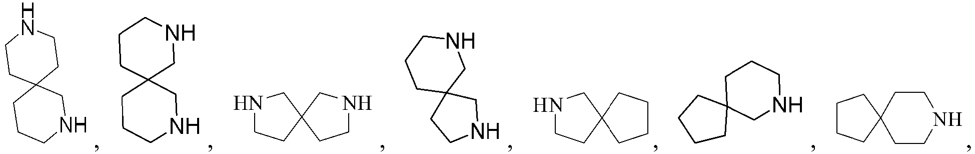

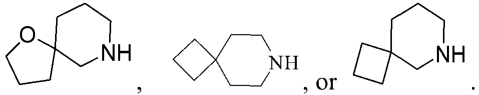

In some embodiments according to the first aspect, the ring B is 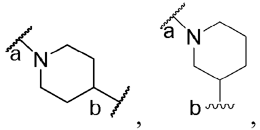 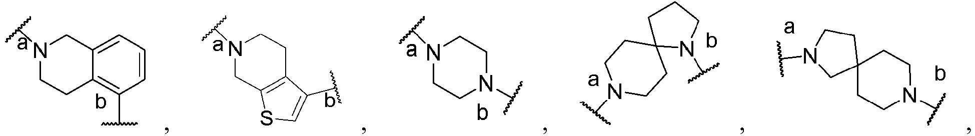 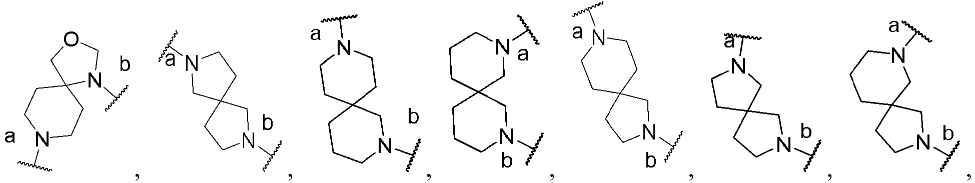 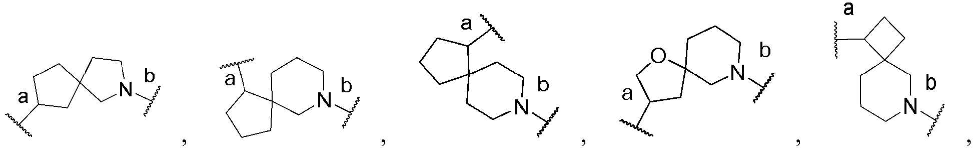 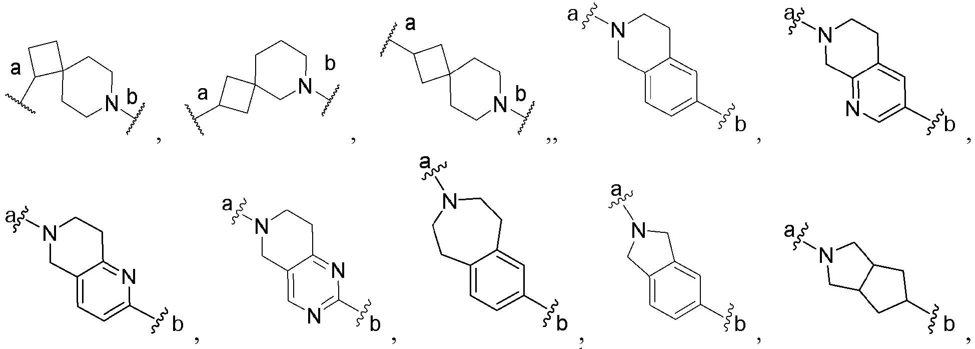 wherein the mark "a" indicates the bond connected to R¹, and the mark "b" indicates the bond connected to L¹.

In some embodiments according to the first aspect, the ring B is 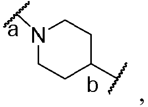 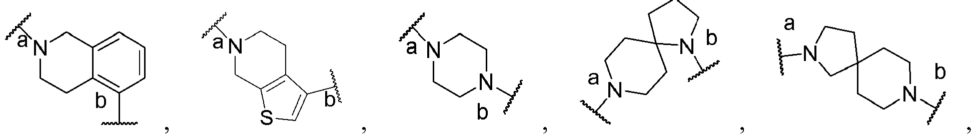 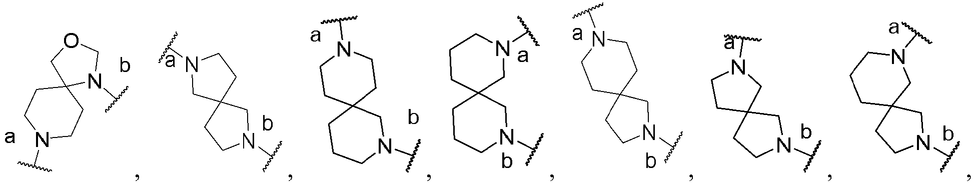 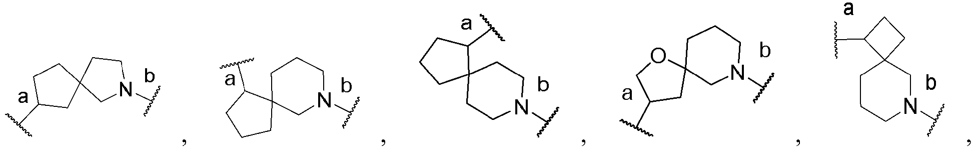 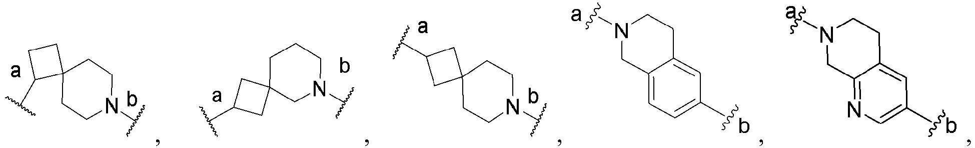 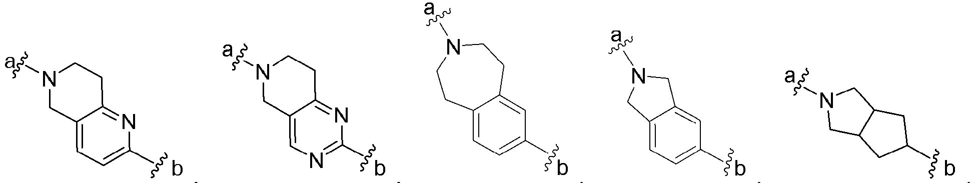 wherein the mark "a" , indicates the bond connected to R¹, and the mark "b" indicates the bond connected to L¹; preferably, the ring B is

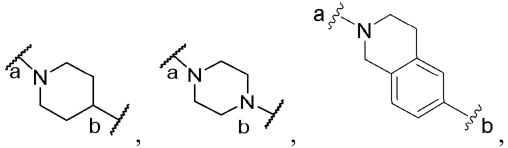

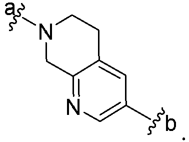

In some embodiments according to the first aspect, the ring B is

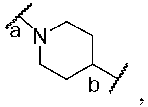

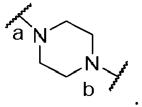

In some embodiments according to the first aspect, the L¹ is a bond, O, -NH-, C₁₋₄ alkylene that is unsubstituted or substituted by one or more L¹⁻¹, C₂₋₄ alkenylene that is unsubstituted or substituted by one or more L¹⁻², C₂₋₄ alkynylene, *-C₁₋₄ alkylene-S-, *-C₁₋₄ alkylene-O-, *-O-C₁₋₄ alkylene-, *-phenylene-O-, *-C₁₋₄ alkylene-C(O)-.

In some embodiments according to the first aspect, the L¹ is C₁₋₄ alkylene that is unsubstituted or substituted by one or more L¹⁻¹, C₂₋₄ alkenylene that is unsubstituted or substituted by one or more L¹⁻².

In some embodiments according to the first aspect, in the C₁₋₆ alkylene that is unsubstituted or substituted by one or more L¹⁻¹ and the C₂₋₆ alkenylene that is unsubstituted or substituted by one or more L¹⁻², the "more" means 1, 2, 3, or 4, preferably 1 or 2.

In some embodiments according to the first aspect, L¹ is a bond, C₁₋₂ alkylene that is unsubstituted or substituted by one or more L¹⁻¹, C₂₋₃ alkenylene that is unsubstituted or substituted by one or more L¹⁻², C₂₋₃ alkynylene, *-C₁₋₂ alkylene-S-, *-C₁₋₂ alkylene-O-, *-C₁₋₂ alkylene-C(O)-.

In some embodiments according to the first aspect, L¹ is C₁₋₂ alkylene that is unsubstituted or substituted by one or more L¹⁻¹, C₂₋₃ alkenylene that is unsubstituted or substituted by one or more L¹⁻².

In some embodiments according to the first aspect, the L¹⁻¹, L¹⁻², L¹⁻³, L¹⁻⁴, L¹⁻⁵, L¹⁻⁶ L¹⁻⁷, L¹⁻⁸, L¹⁻⁹ and L¹⁻¹⁰ are each independently halogen, OH, C₁₋₆ alkyl or C₃₋₆ cycloalkyl.

In some embodiments according to the first aspect, the halogen in L¹⁻¹, L¹⁻², L¹⁻³, L¹⁻⁴, L¹⁻⁵, L¹⁻⁶, L¹⁻⁷, L¹⁻⁸, L¹⁻⁹, and L¹⁻¹⁰ is each independently F, Cl, Br or I, preferably F, Cl, or Br, more preferably F.

In some embodiments according to the first aspect, the C₁₋₆ alkyl in L¹⁻¹, L¹⁻², L¹⁻³, L¹⁻⁴, L¹⁻⁵, L¹⁻⁶, L¹⁻⁷, L¹⁻⁸, L¹⁻⁹, and L¹⁻¹⁰ is independently methyl, ethyl, n-propyl, isopropyl, n-butyl, isobutyl or tert-butyl, preferably methyl.

In some embodiments according to the first aspect, the C₃₋₆ cycloalkyl in L¹⁻¹, L¹⁻², L¹⁻³, L¹⁻⁴, L¹⁻⁵, L¹⁻⁶, L¹⁻⁷, L¹⁻⁸, L¹⁻⁹ and L¹⁻¹⁰ is each independently cyclopropyl, cyclobutyl, cyclopentyl or cyclohexyl, preferably cyclopropyl.

In some embodiments according to the first aspect, the C₁₋₆ alkylene in C₁₋₆ alkylene that is unsubstituted or substituted by one or more L¹⁻¹, *-C₁₋₆ alkylene-S-, *-C₁₋₆ alkylene-O-, *-O-C₁₋₆ alkylene- or *-C₁₋₆ alkylene-C(O)- is each independently -CH₂-, -CH₂-CH₂-, -C(CH₃)-CH₂-, -CH(CH₃)-CH(CH₃)-, -CH₂CH₂-CH(CH₃)- or -CH₂CH(CH₃)-CH₂-.

In some embodiments according to the first aspect, the C₂₋₆ alkenylene in C₂₋₆ alkenylene that is unsubstituted or substituted by one or more L¹⁻² is each independently -CH=CH-, -CH=CHCH₂-, -CH₂CH=CHCH₂-, -CH=CHCH₂CH₂-, -C(CH₃)=CHCH₂-, -C(CH₃)=CH- or -C(CH₃)=C(CH₃)-.

In some embodiments according to the first aspect, the C₂₋₆ alkynylene in L¹ is each independently -C≡C-, -CH₂-C≡C-, -CH₂-C≡C-CH₂-, -CH₂-CH₂-C≡C-, -C≡C-C≡C-.

In some embodiments according to the first aspect, the C₆₋₁₀ arylene in *-C₆₋₁₀ arylene-O- of L¹ is phenyl, naphthyl, preferably phenyl.

In some embodiments according to the first aspect, the L¹ is *-CH₂-O-, -CH₂-CH₂-, *-CH₂-CH₂-O-, *-O-CH₂-, *-CH₂-S-, *-CH₂-CH₂-S-, -CH=CH-, -CH₂-, a bond, -NH-, -O-, *-CH=CF-, *-CF=CH-, *-CH=C(CH₃)-, *-C(CH₃)=CH-, -CF=CF-, -C(CH₃)=C(CH₃)-, *-CH₂-CHF-, *-CHF-CH₂-, *-CH₂-CH(CH₃)-, *-C(CH₃)-CH₂-, -CHF-CHF-, -CH(CH₃)-CH(CH₃)-, *-CH₂-CF₂-, *-CF₂-CH₂-, *-CH₂-CH(cyclopropane)-, *-CH(cyclopropane)-CH₂-, *-CH=C(cyclopropane)-, *-C(cyclopropane)=CH-, *-CH₂-CH(OH)-, -C≡C-, 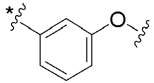 , or *-CH₂-C(O)-; wherein the mark "*" indicates the bond connected to ring B.

In some embodiments according to the first aspect, the L¹ is *-CH₂-O-, -CH₂-CH₂-, *-CH₂-CHF-, *-CH=CF-, *-CH₂-CH(OH)-, *-CH₂-S-, *-CH₂-CH₂-S-, -CH=CH-, a bond, -C≡ C-, *-CH₂-C(O)-.

In some embodiments according to the first aspect, the L¹ is *-CH₂-O-, -CH₂-CH₂-, -CH=CH-; wherein the mark "*" indicates the bond connected to ring B.

In some embodiments according to the first aspect, the L¹ is -CH₂-CH₂-, -CH=CH-; wherein the mark "*" indicates the bond connected to ring B.

In some embodiments according to the first aspect, the R² is independently OH, halogen, CN, C₁₋₆ alkyl, C₂₋₆ alkenyl, C₁₋₆ haloalkyl, C₁₋₆ deuteroalkyl, -O-C₁₋₆ alkyl, -O-C₁₋₆ haloalkyl, -O-C₂₋₆ alkenyl, -O-C₃₋₁₀ cyclohydrocarbyl, -S-C₁₋₆ alkyl, -C₃₋₁₀ cyclohydrocarbyl, 3-10 membered heterocyclyl, 5-10 membered heteroaryl, -C₁₋₆ alkylene-C₃₋₁₀ cyclohydrocarbyl, -OC₁₋₆ alkylene-C₃₋₁₀ cyclohydrocarbyl, or -C(=O)NR²⁻¹R²⁻², or alternatively, two R² connected to the same carbon atom form an oxo group (=O), wherein the heteroatom in the 3-10 membered heterocyclyl or the 5-10 membered heteroaryl is selected from N, O and S, and the number of the heteroatom is 1, 2, 3, or 4;
or alternatively, adjacent R² groups form 5-6 membered heterocyclyl, wherein the heteroatom in the 5-6 membered heterocyclyl is selected from N, O and S, and the number of the heteroatom is 1, 2, 3, or 4.

In some embodiments according to the first aspect, the R² is independently OH, halogen, CN, C₁₋₆ alkyl, C₂₋₆ alkenyl, C₁₋₆ haloalkyl, C₁₋₆ deuteroalkyl, -O-C₁₋₆ alkyl, -O-C₁₋₆ haloalkyl, -O-C₂₋₆ alkenyl, -O-C₃₋₁₀ cycloalkyl, -S-C₁₋₆ alkyl, -C₃₋₁₀ cycloalkyl, 3-10 membered heterocyclyl, 5-10 membered heteroaryl, -C₁₋₆ alkylene-C₃₋₁₀ cycloalkyl, -OC₁₋₆ alkylene-C₃₋₁₀ cycloalkyl, or -C(=O)NR²⁻¹R²⁻², or alternatively, two R² connected to the same carbon atom form an oxo group (=O).

In some embodiments according to the first aspect, each R² is independently OH, halogen, CN, C₁₋₄ alkyl, C₂₋₄ alkenyl, C₁₋₄ haloalkyl, C₁₋₄ deuteroalkyl, -O-C₁₋₄ alkyl, -O-C₃₋₆ cycloalkyl, -S-C₁₋₄ alkyl, -O-C₂₋₄ alkenyl, -O-C₁₋₄ haloalkyl, -C₃₋₆ cycloalkyl, 3-6 membered heterocyclyl, 5-6 membered heteroaryl, -C₁₋₄ alkylene-C₃₋₆ cycloalkyl, -OC₁₋₄ alkylene-C₃₋₆ cycloalkyl, -C(=O)NHC₁₋₄ alkyl or two R² connected to the same carbon atom form an oxo group (=O).

In some embodiments according to the first aspect, in R², the halogen is each independently F, Cl, Br or I, preferably F, Cl, or Br.

In some embodiments according to the first aspect, in R², the C₁₋₆ alkyl in C₁₋₆ alkyl, -O-C₁₋₆ alkyl, and -S-C₁₋₆ alkyl is each independently methyl, ethyl, n-propyl, isopropyl, n-butyl, isobutyl, sec-butyl or tert-butyl, preferably methyl, ethyl, isopropyl, sec-butyl or tert-butyl.

In some embodiments according to the first aspect, in R², the C₂₋₆ alkenyl in C₂₋₆ alkenyl and -O-C₂₋₆ alkenyl is each independently vinyl, propenyl, isopropenyl, butenyl or isobutenyl, preferably vinyl, propenyl, or isobutenyl.

In some embodiments according to the first aspect, in R², the C₁₋₆ haloalkyl in C₁₋₆ haloalkyl, -O-C₁₋₆ haloalkyl is each independently C₁₋₃ haloalkyl, for example -CH₂F, -CHF₂, -CF₃, -CH₂Cl, -CHCl₂, -CCl₃, -CH₂Cl, -CH₂CH₂F, -CHClCH₃ or -CH(CH₃)CH₂F, preferably -CF₃.

In some embodiments according to the first aspect, in R², the C₁₋₆ deuteroalkyl preferably C₁₋₃ deuteroalkyl, for example -CD₃, -CHD₂, -CH₂D; preferably -CD₃.

In some embodiments according to the first aspect, in R², the C₃₋₁₀ cyclohydrocarbyl in -O-C₃₋₁₀ cyclohydrocarbyl, -S-C₃₋₁₀ cyclohydrocarbyl, -C₃₋₁₀ cyclohydrocarbyl, -C₁₋₆ alkylene-C₃₋₁₀ cyclohydrocarbyl, -OC₁₋₆ alkylene-C₃₋₁₀ cyclohydrocarbyl is each independently -C₃₋₈ cycloalkyl, -C₃₋₈ cycloalkenyl, preferably -C₃₋₆ cycloalkyl, -C₃₋₆ cycloalkenyl, C₃₋₆ cycloalkyl for example cyclopropyl, cyclobutyl, cyclopentyl or cyclohexyl, and for further example cyclopropyl; C₃₋₆ cycloalkenyl for example cyclopropenyl, cyclobutenyl, cyclopentenyl or cyclohexenyl.

In some embodiments according to the first aspect, in R², the 3-10 membered heterocyclyl is independently 3-6 membered heterocyclyl, the 3-6 membered heterocyclyl is independently oxacyclopropyl, azacyclopropyl, azetidinyl, oxetanyl, tetrahydrofuranyl, tetrahydrothienyl, dioxolyl, pyrrolidinyl, pyrrolidinonyl, oxazolidinyl, thiazolidinyl, pyrazolidinyl, imidazolidinyl, pyrrolinyl, tetrahydropyranyl or piperidinyl, preferably oxacyclopropyl, azacyclopropyl, azetidinyl or oxetanyl.

In some embodiments according to the first aspect, in R², the 5-10 membered heteroaryl is independently pyridinyl, pyrimidinyl, pyrazinyl, pyridazinyl, thiazolyl, thienyl, oxazolyl, furanyl, pyrrolyl, pyrazolyl, triazolyl, tetrazolyl, isoxazolyl, isothiazolyl, imidazolyl, triazinyl, oxadiazolyl, thiadiazolyl, benzothiazolyl, benzoisothiazolyl, imidazolopyridinyl, quinolinyl, indolyl, pyrrolopyridazinyl or benzofuranyl, preferably oxazolyl, furanyl or thiazolyl.

In some embodiments according to the first aspect, in R², the R²⁻¹ and R²⁻² are each independently H or C₁₋₃ alkyl, preferably H or methyl.

In some embodiments according to the first aspect, in R², the adjacent R² groups form 5-6 membered heterocyclyl, wherein the heteroatom in the 5-6 membered heterocyclyl is selected from N or O, and the number of the heteroatom is 1 or 2, preferably azacyclopentane, azacyclohexane, azetidine, oxacyclopentane oxacyclohexane or oxetane, more preferably oxacyclopentane or oxacyclohexane.

In some embodiments according to the first aspect, the halogen is independently F, Cl, Br or I, preferably F, Cl or Br.

In some embodiments according to the first aspect, the R² is independently 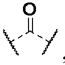 OH, F, Cl, Br, CN, -CH₂F, -CHF₂, -CF₃, -CD₃, -S-CH₃, -O-CH-(CH₃)₂, -O-CH(CH₃)-CH₂CH₃, methyl, ethyl, n-propyl, isopropyl, vinyl, propenyl, isopropenyl, methoxy, ethoxy, cyclopropyl, cyclopropylmethyl-, 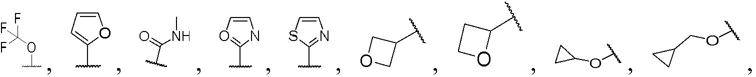 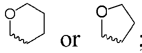 , wherein, the wavy lines " 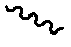 " in 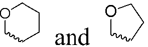 indicate the position of fusion with ring A.

In some embodiments according to the first aspect, m is independently 0, 1, 2 or 3, preferably 0, 1 or 2.

In some embodiments according to the first aspect, n is independently 0, 1, 2 or 3, preferably 0 or 1.

In some embodiments according to the first aspect, t is independently 0, 1, 2 or 3, preferably 0 or 1.

In some embodiments according to the first aspect, ring A is C₆₋₁₀ aryl, 5-6 membered heteroaryl, or 6 membered heterocyclyl.

In some embodiments according to the first aspect, the C₆₋₁₀ aryl in ring A is independently phenyl or naphthyl, preferably phenyl.

In some embodiments according to the first aspect, the 5-6 membered heteroaryl in ring A is independently 5-6 membered nitrogen-containing heteroaryl, for example pyridinyl, pyridazinyl, pyrimidinyl, pyrazinyl, thiazolyl, pyranyl, pyrrolyl, furanyl, thienyl or imidazolyl, preferably pyridinyl, pyridazinyl, pyrimidinyl or pyrazinyl.

In some embodiments according to the first aspect, the 5-12 membered heterocyclyl in ring A is independently oxacyclopropyl, azacyclopropyl, azetidinyl, oxetanyl, tetrahydrofuranyl, tetrahydrothienyl, dioxolyl, pyrrolidinyl, pyrrolidinonyl, oxazolidinyl, thiazolidinyl, pyrazolidinyl, imidazolidinyl, pyrrolinyl, pyranyl, tetrahydropyranyl,

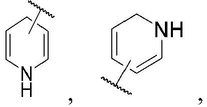

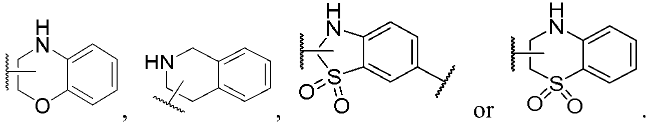

In some embodiments according to the first aspect, the 5-12 membered heterocyclyl in ring A is tetrahydrofuranyl, tetrahydrothienyl, dioxolyl, pyrrolidinyl, oxazolidinyl, thiazolidinyl, pyrazolidinyl, imidazolidinyl, pyrrolinyl, pyranyl, tetrahydropyranyl,

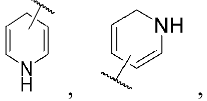

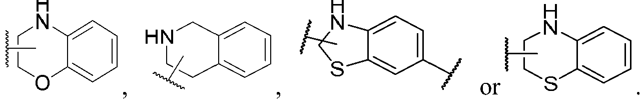

In some embodiments according to the first aspect, the ring A is: 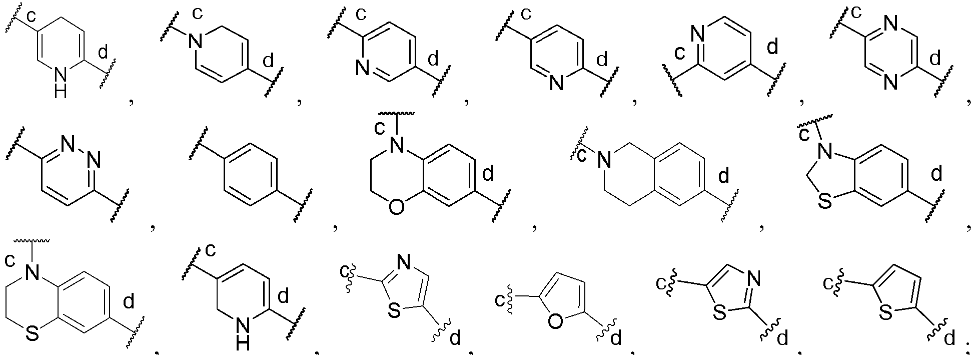 wherein the mark "c" indicates the bond connected to L¹, and the mark "d" indicates the bond connected to

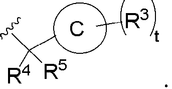

In some embodiments according to the first aspect, the ring A is: 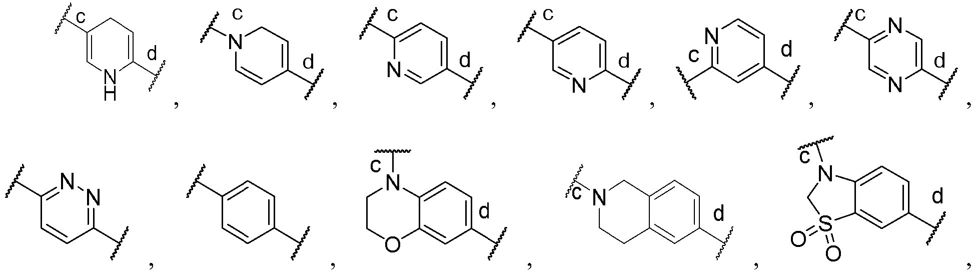 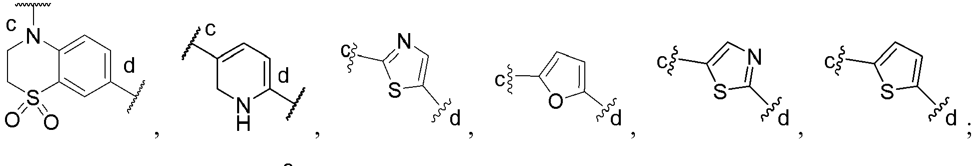 preferably the ring A is 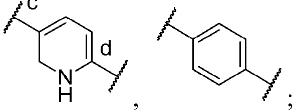 wherein the mark "c" indicates the bond connected to L¹, and the mark "d" indicates the bond connected to

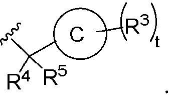

In some embodiments according to the first aspect, the 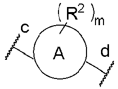 moiety is:

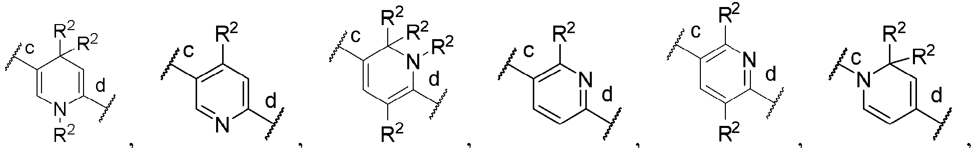

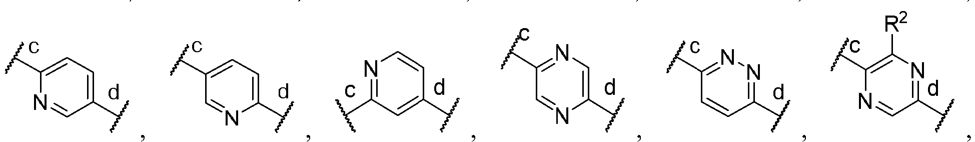

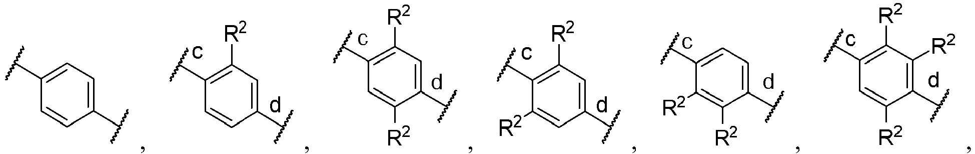

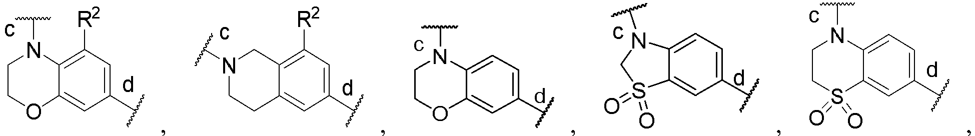

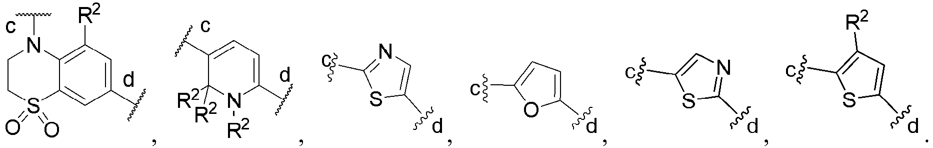

In some embodiments according to the first aspect, the 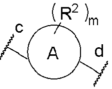 moiety is:

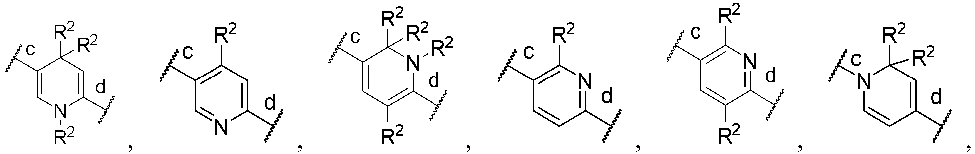

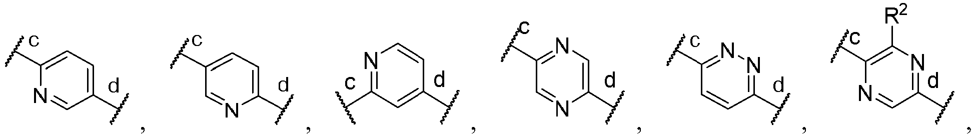

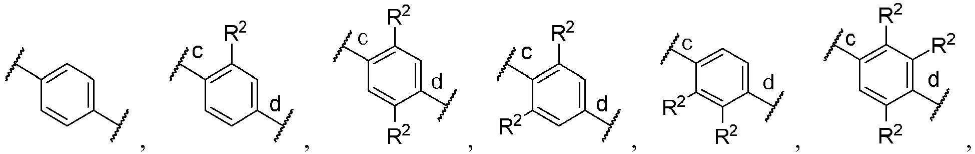

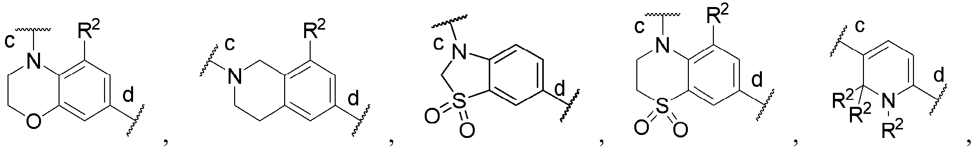

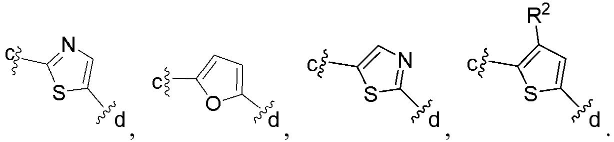

In some embodiments according to the first aspect, the 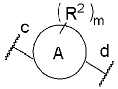 moiety is:

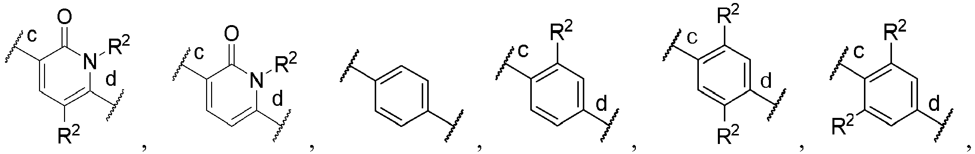

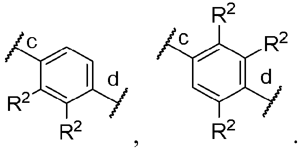

In some embodiments according to the first aspect, the 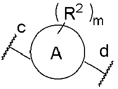 moiety is:

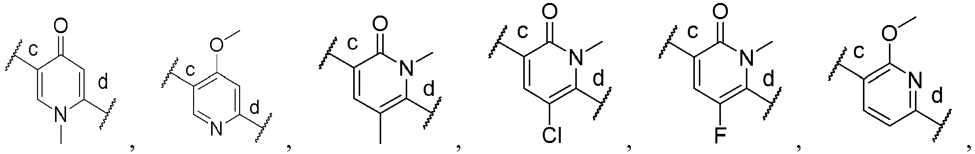

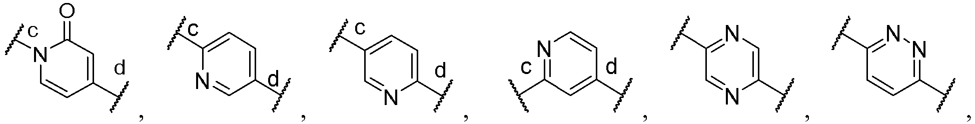

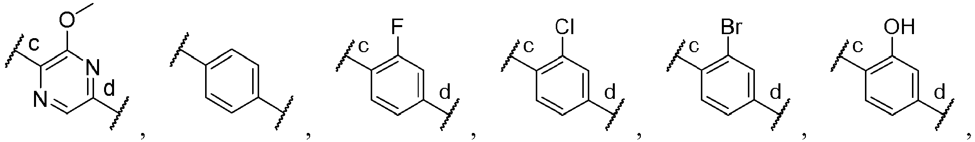

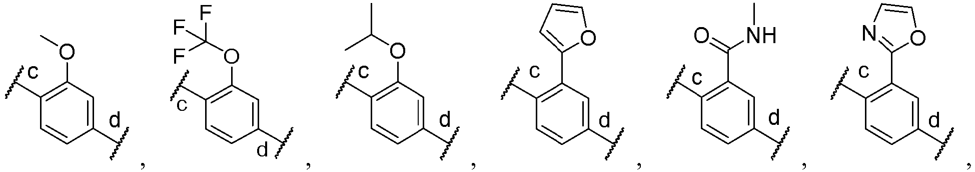

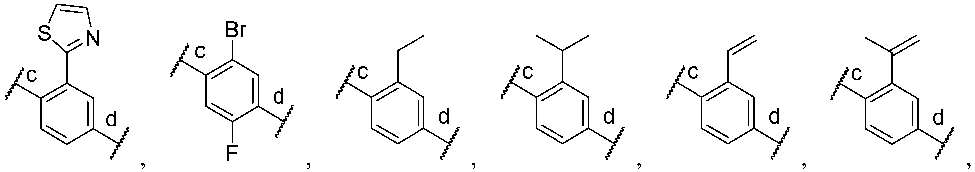

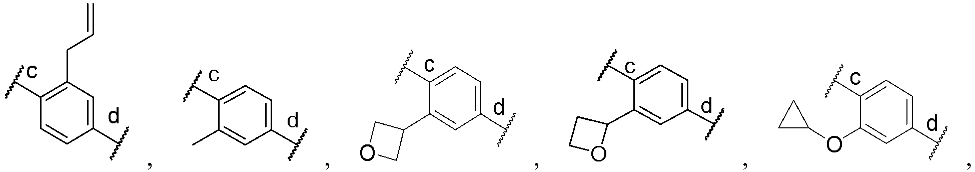

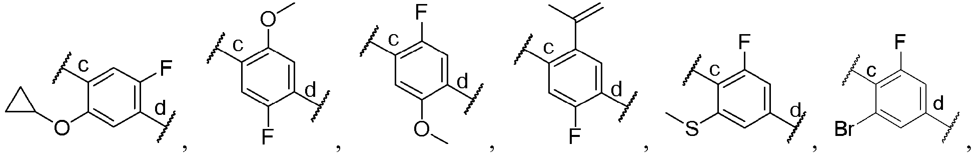

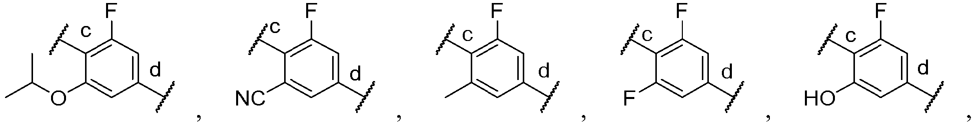

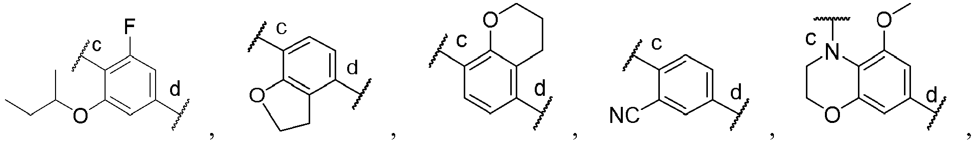

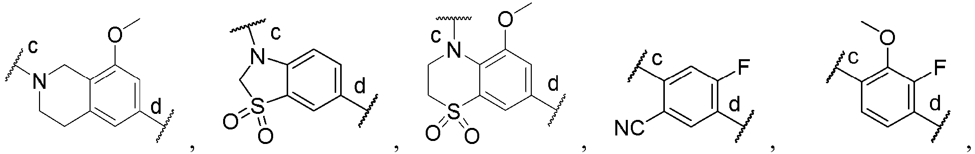

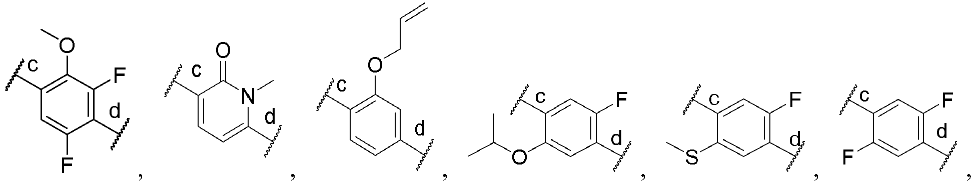

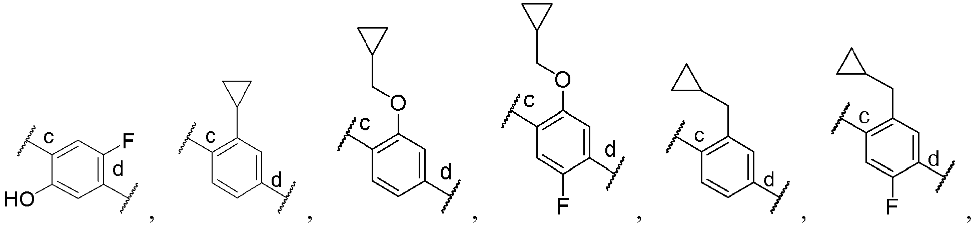

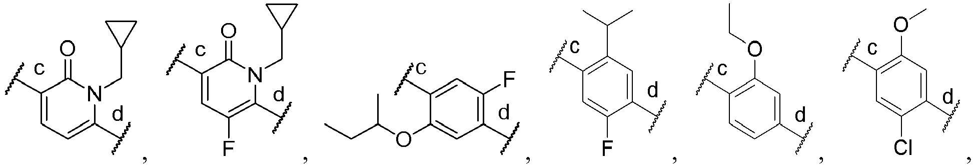

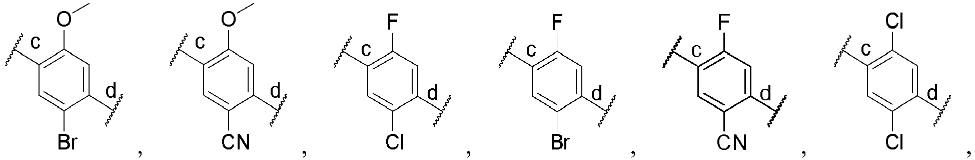

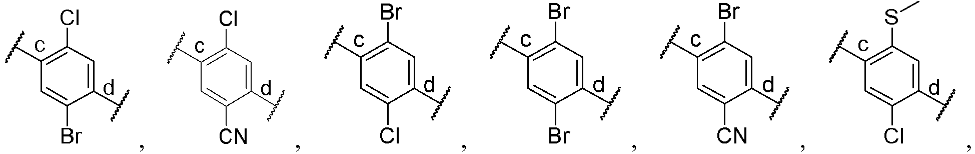

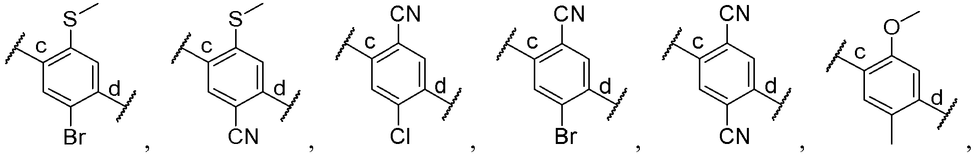

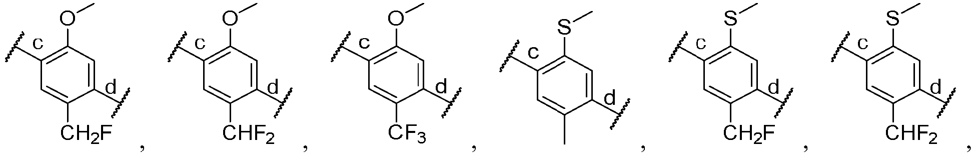

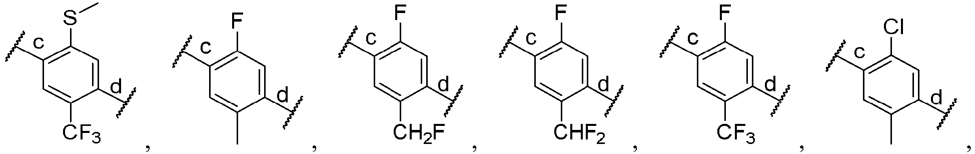

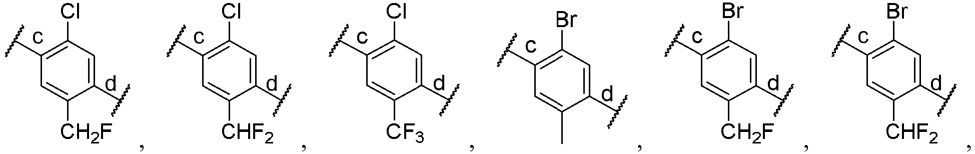

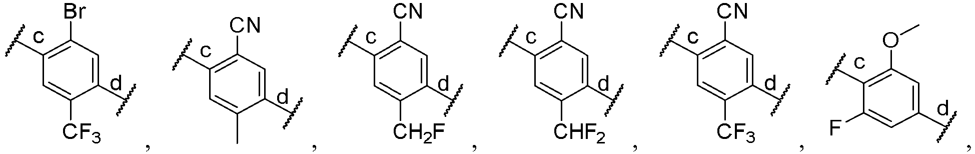

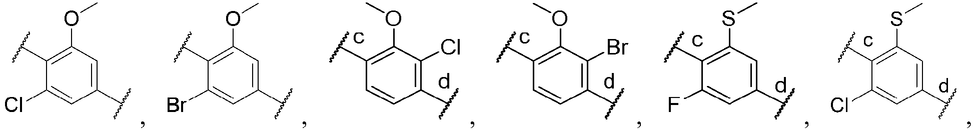

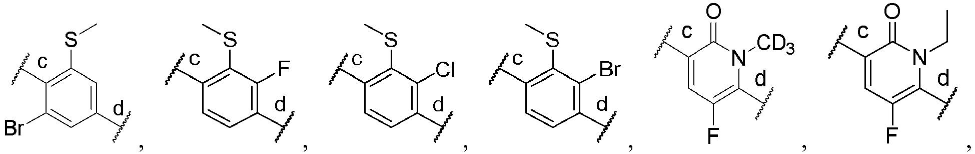

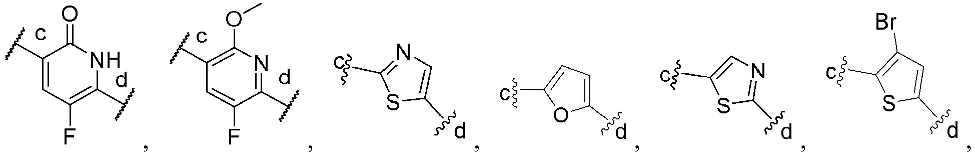

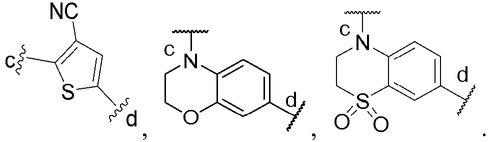

In some embodiments according to the first aspect, the 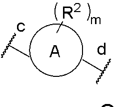 moiety is:

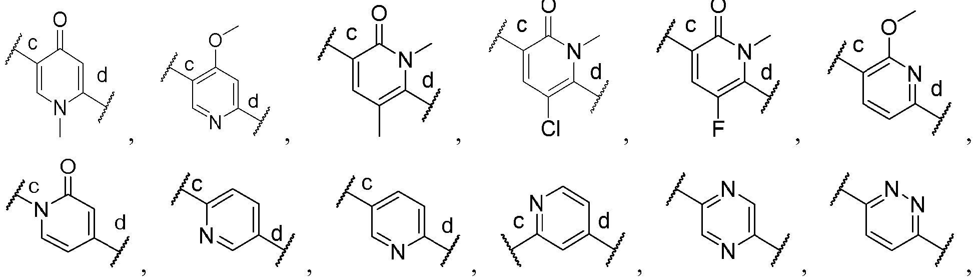

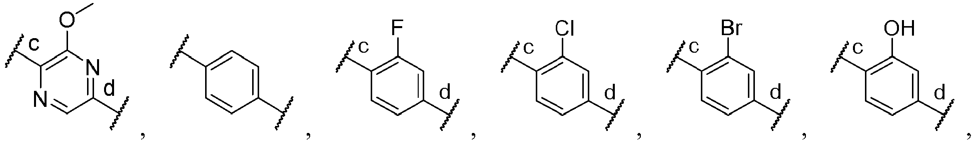

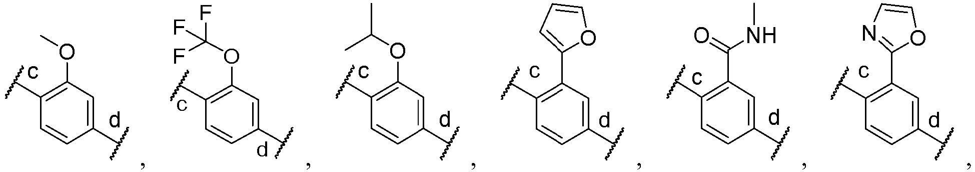

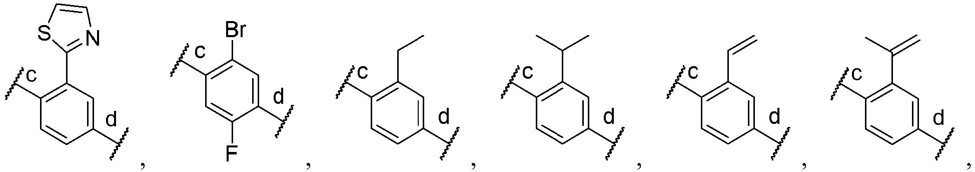

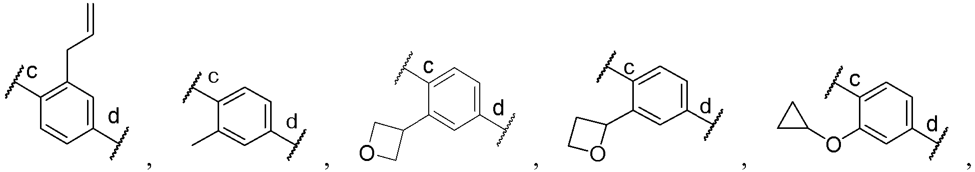

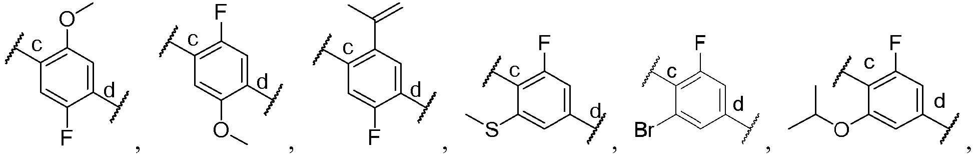

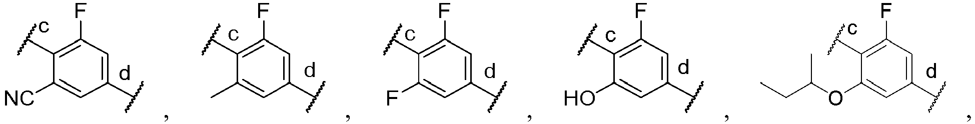

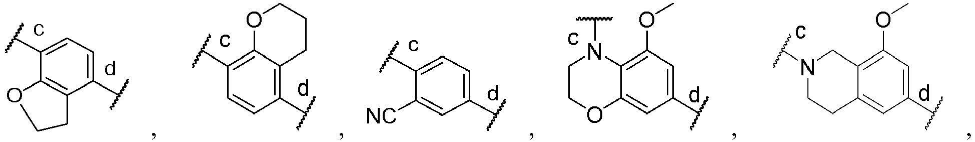

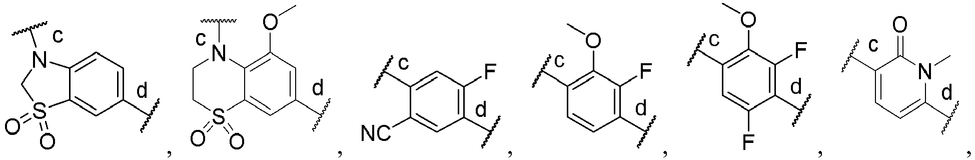

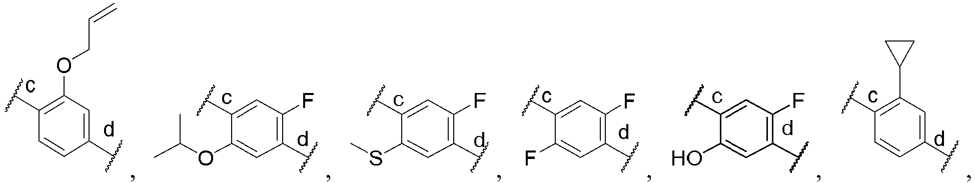

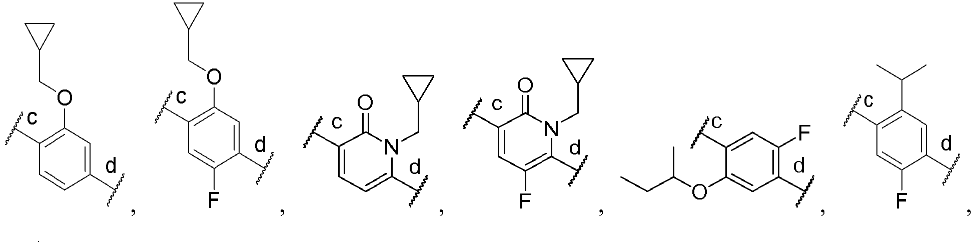

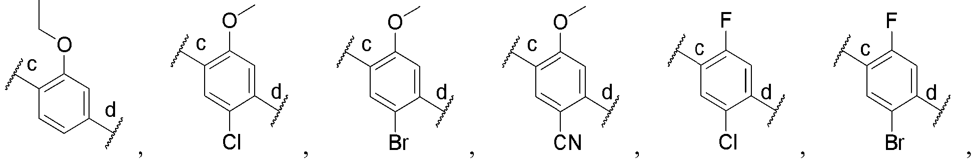

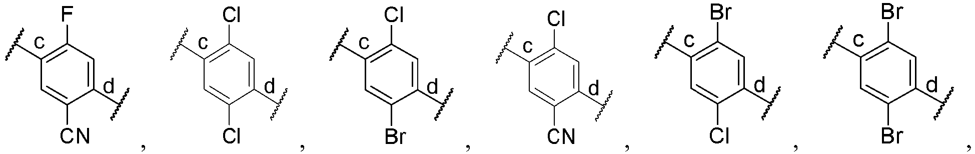

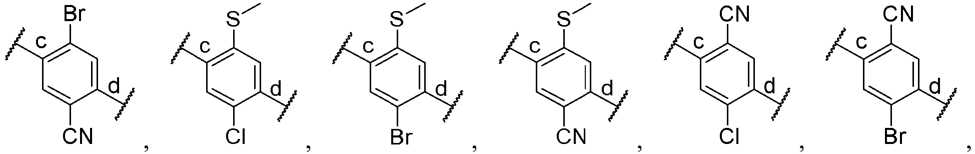

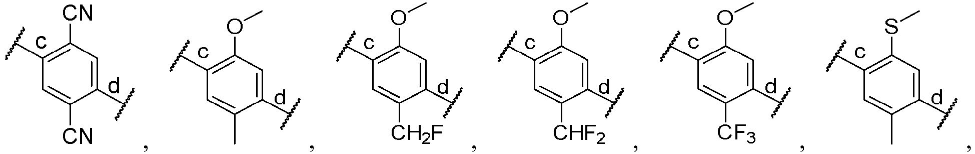

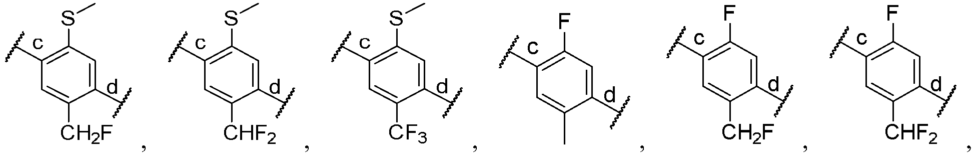

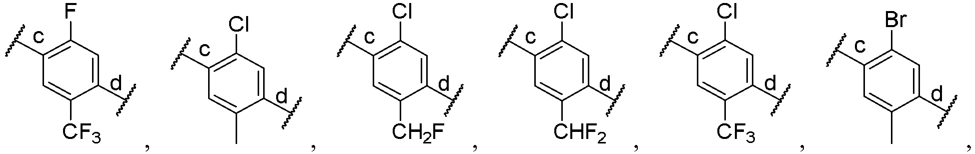

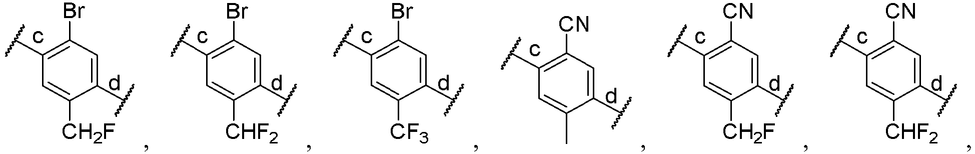

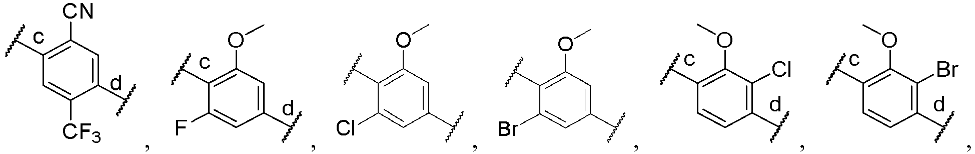

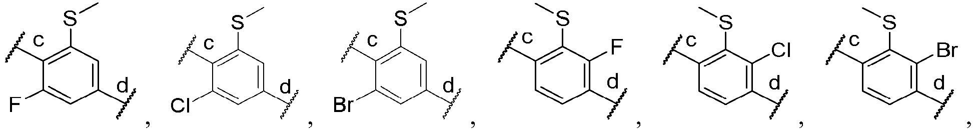

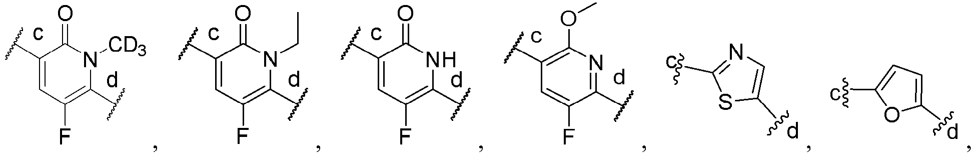

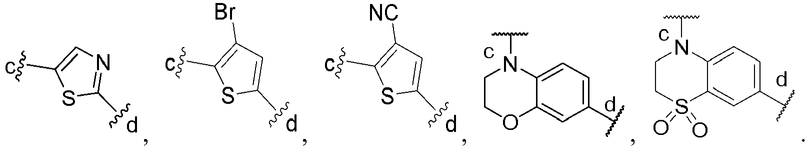

In some embodiments according to the first aspect, the 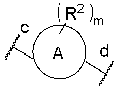 moiety is:

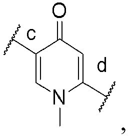

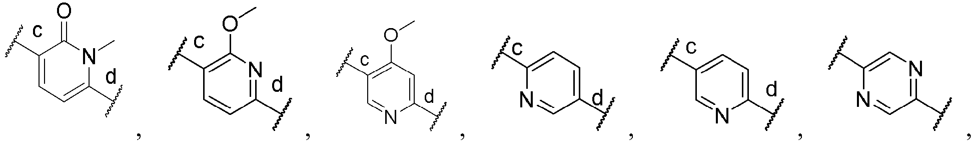

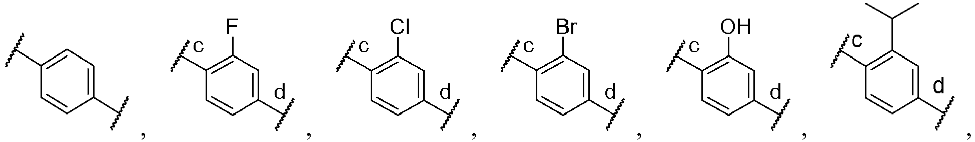

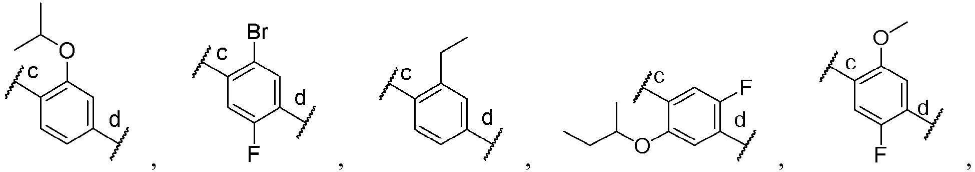

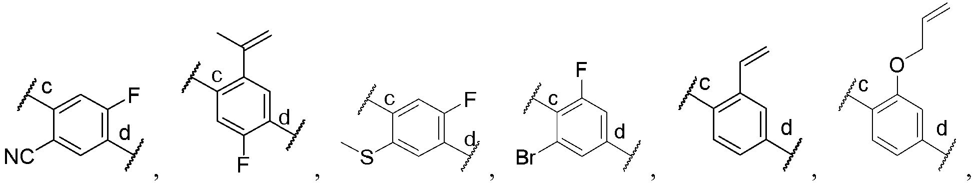

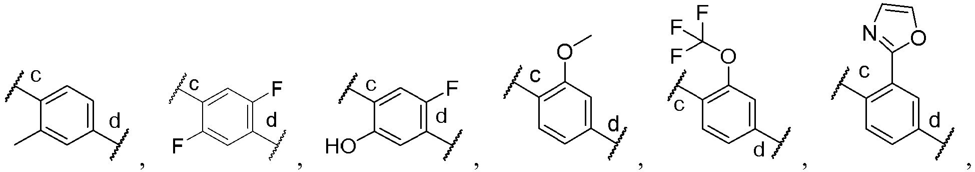

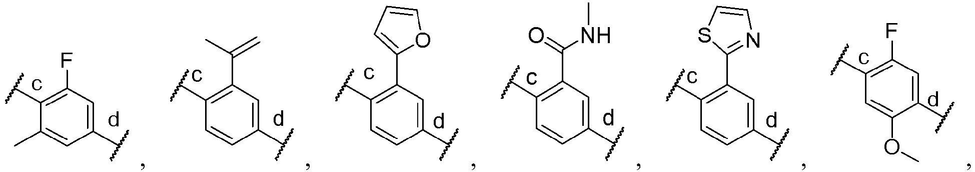

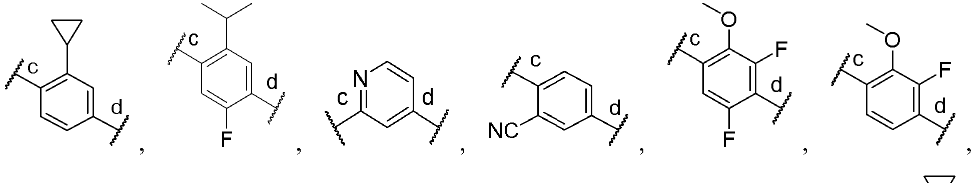

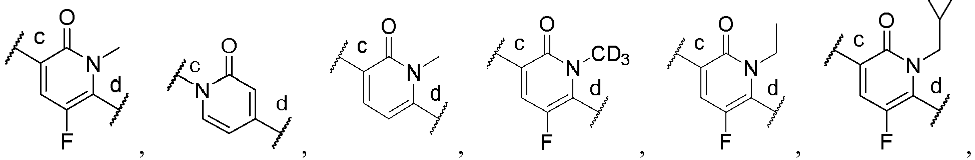

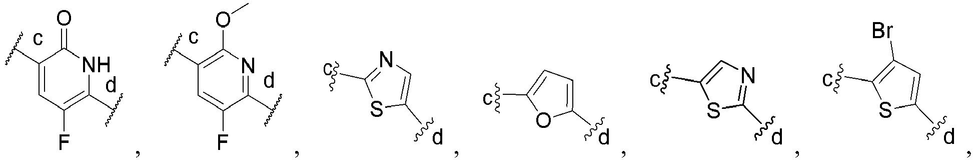

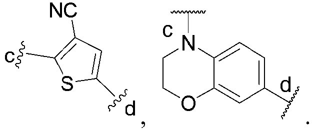

In some embodiments according to the first aspect, the 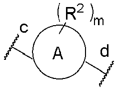 moiety is:

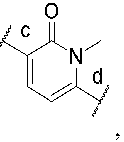

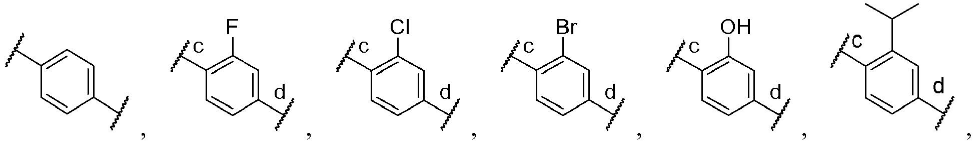

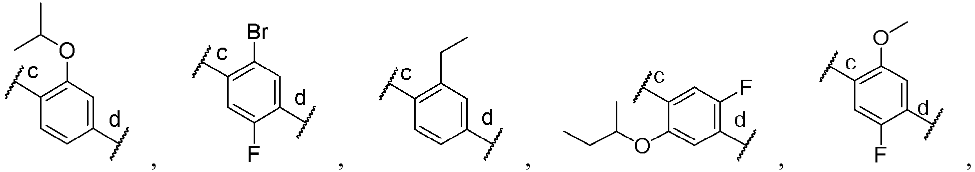

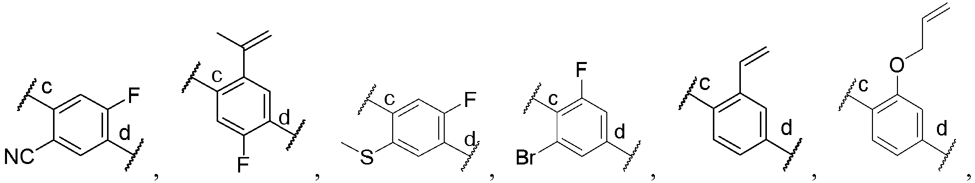

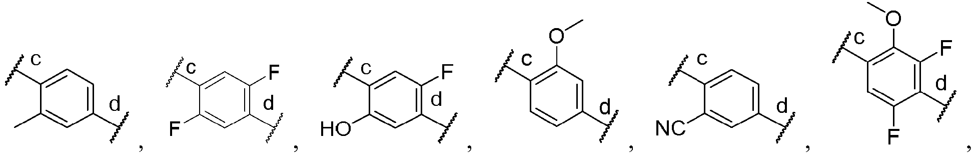

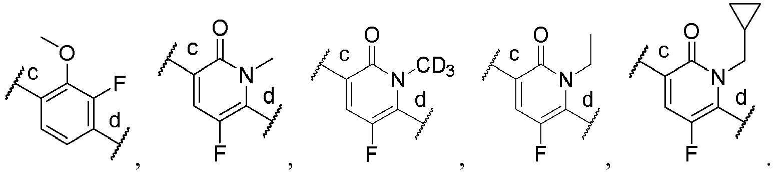

In some embodiments according to the first aspect, ring C is 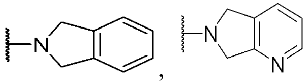 or

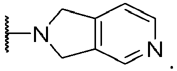

In some embodiments according to the first aspect, the present disclosure provides the compound represented by formula (0) as described above, wherein the compound has a structure shown by formula (I):

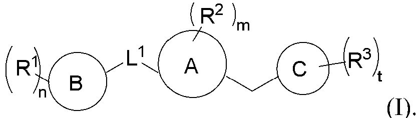

In some embodiments according to the first aspect, the present disclosure provides the compound represented by formula (0) as described above, wherein the compound has a structure shown by formula (I-20'), (I-21') or (I-22'): 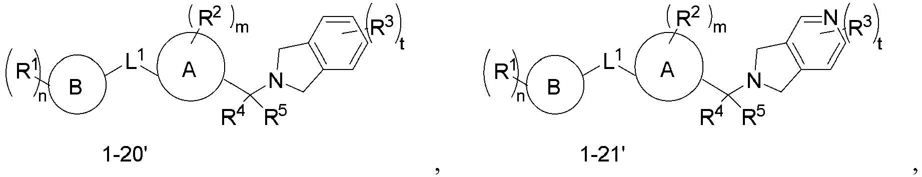 or

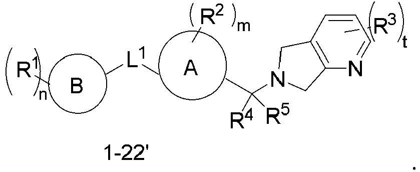

In some embodiments according to the first aspect, the present disclosure provides the compound represented by formula (I-20'), (I-21') or (I-22') as described above, wherein the compound has a structure shown by formula (I-20), (I-21) or (I-22): 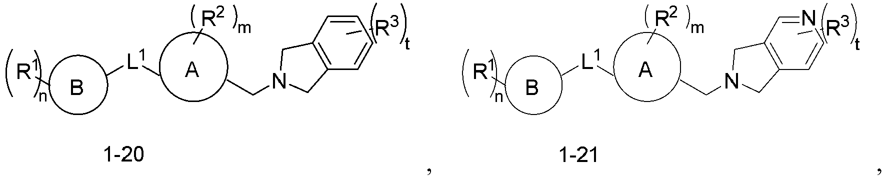 or

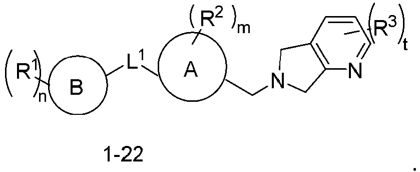

In some embodiments according to the first aspect, the present disclosure provides the compound represented by formula (I-20') as described above, wherein the compound has a structure shown by formula (I-A), (I-B), (I-C), (I-D), (I-E), (I-F), (I-G), (I-H), (I-I), (I-J), (I-K), (I-L), (I-M), (I-N), (I-O), (I-P), (I-Q) or (I-R): 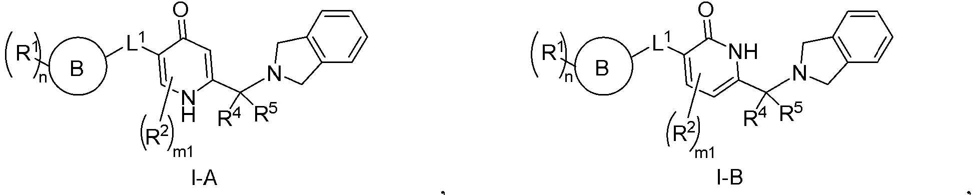 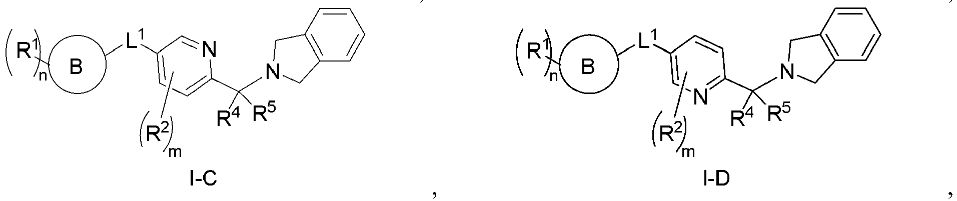 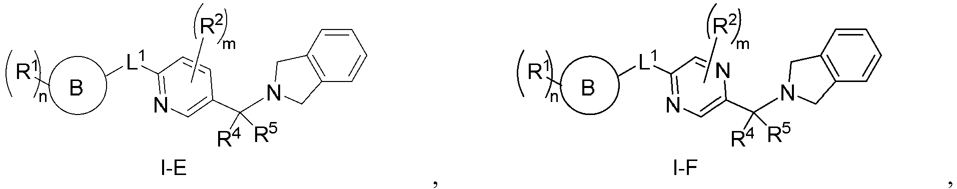 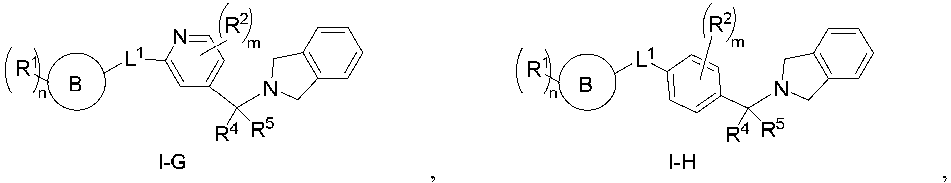 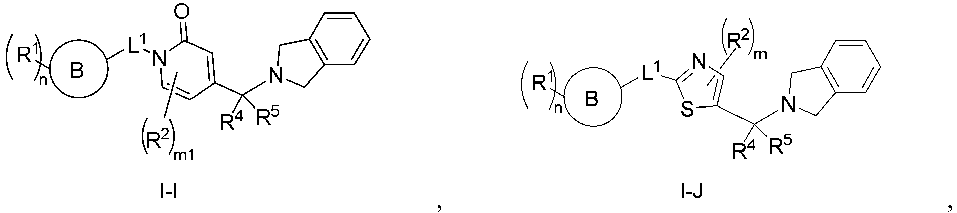 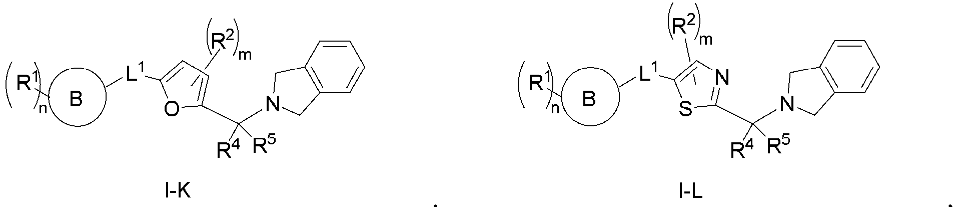 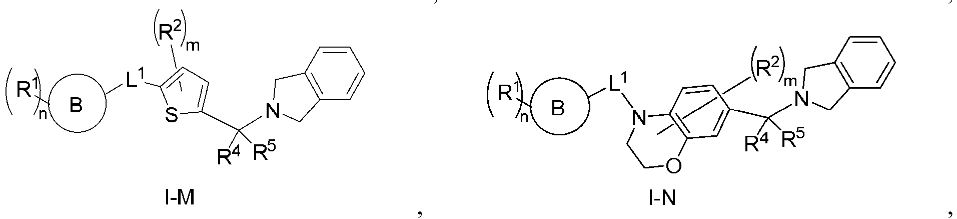 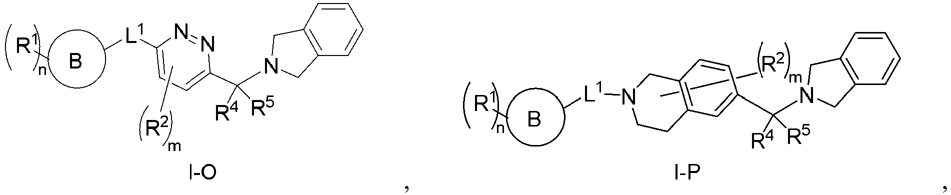 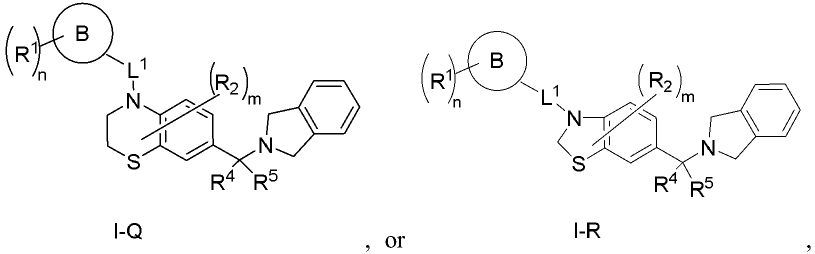 wherein m1 is 0, 1 or 2.

In some embodiments according to the first aspect, the present disclosure provides the compound represented by formula (0) as described above, wherein the compound has a structure shown by formula (II-A), (II-B), (II-C) or (II-D):

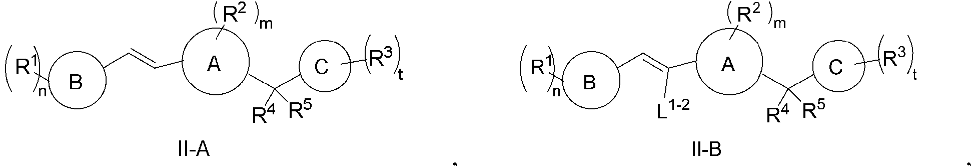

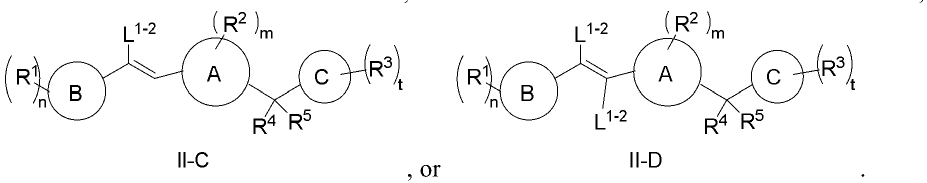

In some embodiments according to the first aspect, the present disclosure provides the compound represented by formula (0) as described above, wherein the compound has a structure shown by formula (II-A-1), (II-B-1), (II-C-1) or (II-D-1):

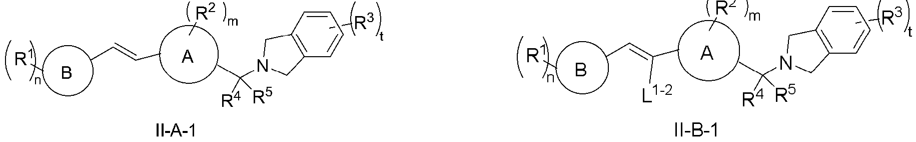

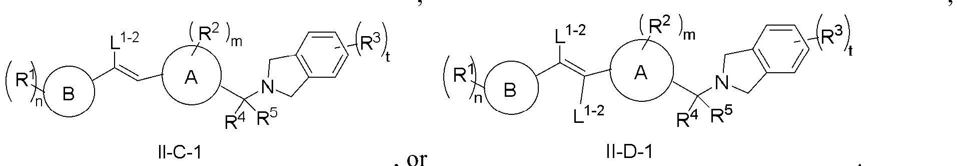

In some embodiments according to the first aspect, the present disclosure provides the compound represented by formula (0) as described above, wherein the compound has a structure shown by formula (II-A-2), (II-B-2), (II-C-2), (II-D-2), (II-A-3), (II-B-3), (II-C-3) or (II-D-3): 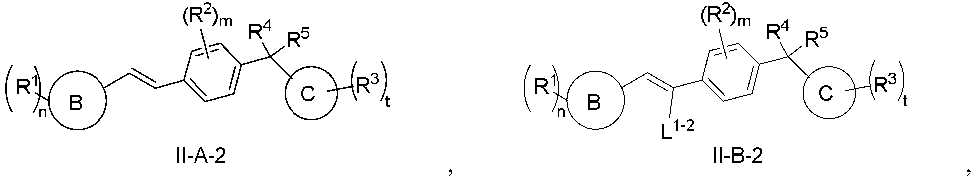 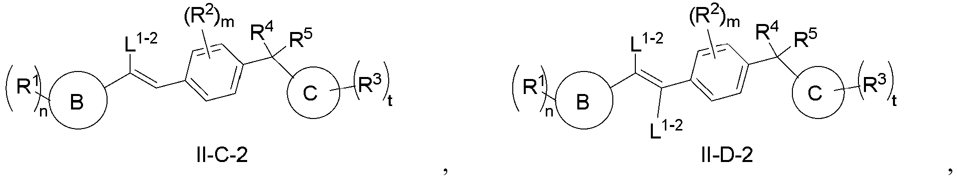 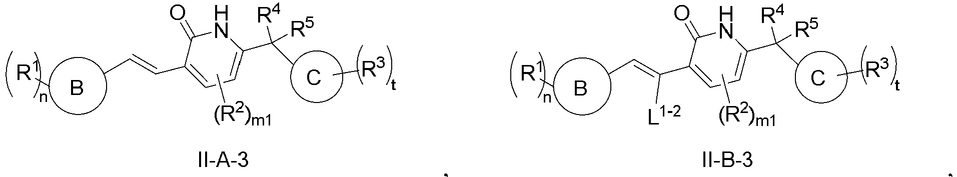 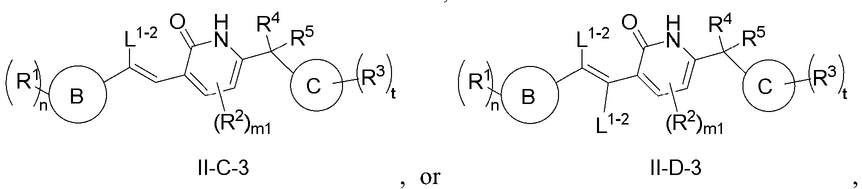 wherein m1 is 0, 1 or 2.

In some embodiments according to the first aspect, the present disclosure provides the compound represented by formula (0) as described above, wherein the compound has a structure shown by formula (II'-A-2), (II'-B-2), (II'-C-2), (II'-D-2), (II'-A-3), (II'-B-3), (II'-C-3) or (II'-D-3): 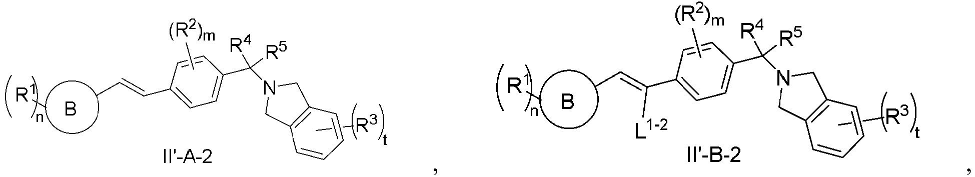 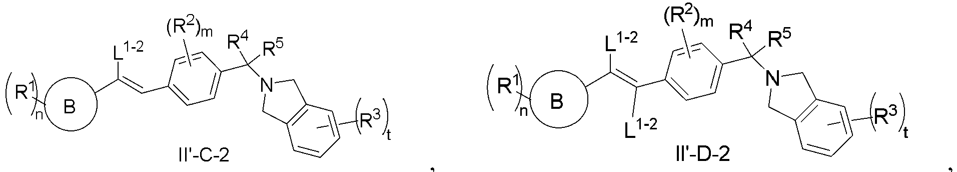 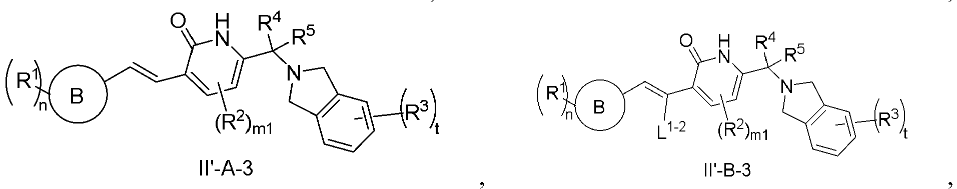 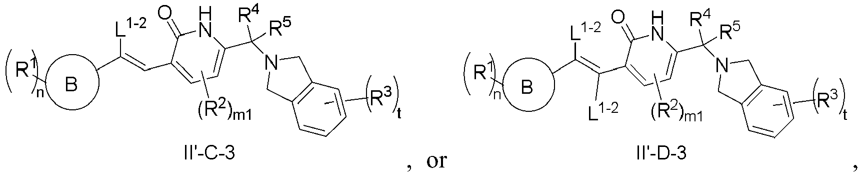 wherein m1 is 0, 1 or 2, preferably m1 is 2; m is 0, 1, 2 or 3, preferably m is 1 or 2; L¹⁻² is halogen, C₁₋₆ alkyl, C₃₋₆ cycloalkyl, preferably L¹⁻² is F, methyl, cyclopropyl; more preferably L¹⁻² is F.

In some embodiments according to the first aspect, the present disclosure provides the compound represented by formula (0) as described above, wherein the compound has a structure shown by formula (I-H-1): 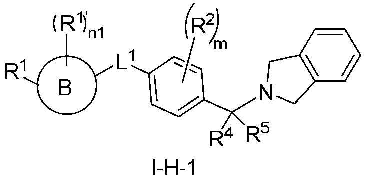 wherein:
R¹ and R^{1'} are each independently deuterium, halogen, cyano, hydroxy, C₁₋₆ alkoxy, R¹⁻¹, -C(=O)-R¹⁻¹, -C(=O)-OR¹⁻⁴, -NR¹⁻⁷R¹⁻⁸, -C(=O)-NR¹⁻⁷R¹⁻⁸, -C(=NH)-(R¹⁻⁹), -S(O)₂-R¹⁻⁹, -S(O)₂-NR¹⁻⁵R¹⁻⁶, -NR¹⁻⁵S(O)₂-R¹⁻¹⁰, -R¹⁻¹⁰-OH, or -P(=O)-R¹⁻²R¹⁻³;
R¹⁻¹, R¹⁻², R¹⁻³, R¹⁻⁴, R¹⁻⁵, R¹⁻⁶, R¹⁻⁷ and R¹⁻⁸ are each independently H, C₁₋₆ alkyl, C₁₋₆ haloalkyl, C₁₋₆ deuteroalkyl, -C₁₋₆ alkylene-OH, -C₁₋₆ alkylene-CN, -C₃₋₁₀ cycloalkyl, or 3-10 membered heterocyclyl, wherein the heteroatom in the 3-10 membered heterocyclyl is selected from N, O and S, and the number of the heteroatom is 1, 2, 3, or 4; preferably, R¹⁻¹, R¹⁻², R¹⁻³, R¹⁻⁴, R¹⁻⁵, R¹⁻⁶, R¹⁻⁷ and R¹⁻⁸ are each independently H, C₁₋₄ alkyl, C₁₋₄ haloalkyl, C₁₋₄ deuteroalkyl, -C₁₋₄ alkylene-OH, -C₁₋₄ alkylene-CN, -C₃₋₆ cycloalkyl, or 3-6 membered heterocyclyl;
R¹⁻⁹ and R¹⁻¹⁰ are each independently C₁₋₆ alkyl, C₁₋₆ haloalkyl, -C₁₋₆ alkylene-CN, C₂₋₆ alkenyl, C₂₋₆ haloalkenyl, C₃₋₁₀ cycloalkyl, C₆₋₁₀ aryl, 5-10 membered heteroaryl, C₁₋₆ alkyl-C₆₋₁₀ arylene-; preferably, R¹⁻⁹ and R¹⁻¹⁰ are each independently C₁₋₄ alkyl, C₁₋₄ haloalkyl, -C₁₋₄ alkylene-CN, C₂₋₄ alkenyl, C₂₋₄ haloalkenyl, C₃₋₆ cycloalkyl, C₆₋₁₀ aryl, 5-10 membered heteroaryl, C₁₋₄ alkyl-C₆₋₁₀ arylene-;
preferably R¹ is -C(=O)-R¹⁻¹, -C(=O)-OR¹⁻⁴, -C(=O)-NR¹⁻⁷R¹⁻⁸, -C(=NH)-(R¹⁻⁹), -S(O)₂-R¹⁻⁹, -S(O)₂-NR¹⁻⁵R¹⁻⁶, -NR¹⁻⁵S(O)₂-R¹⁻¹⁰ or -P(=O)-R¹⁻²R¹⁻³; R^{1'} is deuterium, halogen, cyano, hydroxy, C₁₋₆ alkoxy, R¹⁻¹, -NR¹⁻⁷R¹⁻⁸, -R¹⁻¹⁰-OH;
preferably R¹ is -C(=O)-R¹⁻¹, -C(=O)-OR¹⁻⁴, -C(=O)-NR¹⁻⁷R¹⁻⁸, -S(O)₂-R¹⁻⁹; R^{1'} is deuterium, halogen, cyano, hydroxy, C₁₋₆ alkoxy, R¹⁻¹, -NR¹⁻⁷R¹⁻⁸, -R¹⁻¹⁰-OH;
preferably R¹ is -C(=O)-R¹⁻¹, -C(=O)-OR¹⁻⁴, -S(O)₂-R¹⁻⁹; R^{1'} is hydrogen, deuterium, halogen, cyano, hydroxy, C₁₋₆ alkoxy, -NH₂, -NH(C₁₋₆ alkyl), -N(C₁₋₆ alkyl)₂, C₁₋₆ alkyl, C₁₋₆ haloalkyl, C₁₋₆ deuteroalkyl, -C₁₋₆ alkylene-OH, -C₁₋₆ alkylene-CN, -C₃₋₁₀ cycloalkyl, or 3-10 membered heterocyclyl;
preferably R¹ is -C(=O)-C₁₋₆ alkyl, -C(=O)-C₁₋₆ haloalkyl, -C(=O)-C₃₋₁₀ cycloalkyl, -C(= O)-C₁₋₆ alkylene-CN, -C( = O)-OC₁₋₆ alkyl, -C( = O)-OC₃₋₁₀ cycloalkyl, -S(O)₂-C₁₋₆ alkyl, -S(O)₂-C₆₋₁₀ arylene-C₁₋₆ alkyl, -S(O)₂-C₃₋₁₀ cycloalkyl, -S(O)₂-5-10 membered heteroaryl, -S(O)₂-C₁₋₆ haloalkyl, -S(O)₂-C₂₋₆ alkenyl, -C(=O)-NH(C₁₋₆ alkyl); R^{1'} is hydrogen, deuterium, halogen, cyano, hydroxy, C₁₋₆ alkoxy, -NH₂, -NH(C₁₋₆ alkyl), -N(C₁₋₆ alkyl)₂, C₁₋₆ alkyl, C₁₋₆ haloalkyl, C₁₋₆ deuteroalkyl, -C₁₋₆ alkylene-OH, -C₁₋₆ alkylene-CN, -C₃₋₁₀ cycloalkyl, or 3-10 membered heterocyclyl;
preferably R¹ is -C(=O)-C₁₋₄ alkyl, -C(=O)-C₁₋₄ haloalkyl, -C(=O)-C₃₋₆ cycloalkyl, -C(= O)-C₁₋₄ alkylene-CN, -C( = O)-OC₁₋₄ alkyl, -C( = O)-OC₃₋₆ cycloalkyl, -S(O)₂-C₁₋₄ alkyl, -S(O)₂-phenylene-C₁₋₄ alkyl, -S(O)₂-C₃₋₆ cycloalkyl, -S(O)₂-5-6 membered heteroaryl, -S(O)₂-C₁₋₄ haloalkyl, -S(O)₂-C₂₋₄ alkenyl, -C(=O)-NH(C₁₋₄ alkyl); R^{1'} is hydrogen, deuterium, halogen, cyano, hydroxy, C₁₋₄ alkoxy, -NH₂, -NH(C₁₋₄ alkyl), -N(C₁₋₄ alkyl)₂, C₁₋₄ alkyl, C₁₋₄ haloalkyl, C₁₋₄ deuteroalkyl, -C₁₋₄ alkylene-OH, -C₁₋₄ alkylene-CN, -C₃₋₆ cycloalkyl, or 3-6 membered heterocyclyl;
preferably R¹ is -S(O)₂-C₁₋₄ alkyl, -S(O)₂-C₂₋₄ alkenyl, -S(O)₂-phenylene-C₁₋₄ alkyl, -S(O)₂-C₃₋₆ cycloalkyl, -S(O)₂-5-6 membered heteroaryl, -C(=O)-OC₁₋₄ alkyl, -C(=O)-C₁₋₄ alkyl, -C(=O)-C₁₋₄ alkylene-CN; R^{1'} is hydrogen, deuterium, halogen, cyano, hydroxy, C₁₋₄ alkyl;
n1 is each independently 0, 1, 2, or 3; preferably, n1 is each independently 0, 1, or 2; more preferably, n1 is 0;
ring B is 5-12 membered heterocyclyl, wherein the heteroatom in the 5-12 membered heterocyclyl is selected from N, O and S, and the number of the heteroatom is 1, 2, 3, or 4; preferably ring B is 5-6 membered monocyclic heterocyclyl, 9-12 membered fused bicyclic heterocyclyl or 9-12 membered spirocyclic heterocyclyl; preferably ring B is 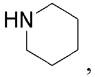 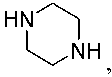 preferably 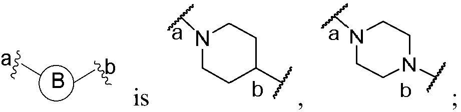
L¹ is a bond, O, -NH-, C₁₋₆ alkylene that is unsubstituted or substituted by one or more L¹⁻¹, C₂₋₆ alkenylene that is unsubstituted or substituted by one or more L¹⁻², C₂₋₆ alkynylene that is unsubstituted or substituted by one or more L¹⁻³, *-C₁₋₆ alkylene-S- that is unsubstituted or substituted by one or more L¹⁻⁴, *-C₁₋₆ alkylene-O- that is unsubstituted or substituted by one or more L¹⁻⁶, *-O-C₁₋₆ alkylene- that is unsubstituted or substituted by one or more L¹⁻⁷, *-C₆₋₁₀ arylene-O- that is unsubstituted or substituted by one or more L¹⁻⁸, *-C₁₋₆ alkylene-C(O)- that is unsubstituted or substituted by one or more L¹⁻¹⁰;
preferably L¹ is a bond, O, -NH-, C₁₋₄ alkylene that is unsubstituted or substituted by one or more L¹⁻¹, C₂₋₄ alkenylene that is unsubstituted or substituted by one or more L¹⁻², C₂₋₄ alkynylene that is unsubstituted or substituted by one or more L¹⁻³, *-C₁₋₄ alkylene-S- that is unsubstituted or substituted by one or more L¹⁻⁴, *-C₁₋₄ alkylene-O- that is unsubstituted or substituted by one or more L¹⁻⁶, *-O-C₁₋₄ alkylene- that is unsubstituted or substituted by one or more L¹⁻⁷, *-phenylene-O- that is unsubstituted or substituted by one or more L¹⁻⁸, *-C₁₋₂ alkylene-C(O)- that is unsubstituted or substituted by one or more L¹⁻¹⁰;
each L¹⁻¹, L¹⁻², L¹⁻³, L¹⁻⁴, L¹⁻⁶, L¹⁻⁷, L¹⁻⁸, L¹⁻¹⁰ is independently halogen, OH, C₁₋₆ alkyl, C₃₋₆ cycloalkyl or 3-6 membered heterocyclyl, wherein the heteroatom in the 3-6 membered heterocyclyl is selected from N, O and S, and the number of the heteroatom is 1, 2, 3, or 4; wherein the C₁₋₆ alkyl, the C₃₋₆ cycloalkyl or the 3-6 membered heterocyclyl is optionally substituted by deuterium, halogen, OH, CN or -NH₂;
preferably L¹ is -CH₂-CH₂-, *-CH₂-O-, -CH=CH-;
more preferably L¹ is -CH₂-CH₂-, -CH=CH-;
each R² is independently deuterium, OH, NH₂, halogen, CN, C₁₋₆ alkyl, C₂₋₆ alkenyl, C₁₋₆ haloalkyl, C₁₋₆ deuteroalkyl, C₂₋₆ haloalkenyl, -O-C₁₋₆ alkyl, -C₁₋₆ alkylene-O-C₁₋₆ alkyl, -O-C₁₋₆ haloalkyl, -O-C₂₋₆ alkenyl, -O-C₃₋₁₀ cycloalkyl, -S-C₁₋₆ alkyl, -S-C₃₋₁₀ cycloalkyl, -C₃₋₁₀ cycloalkyl, 3-10 membered heterocyclyl, C₆₋₁₀ aryl, 5-10 membered heteroaryl, -C₁₋₆ alkylene-C₃₋₁₀ cycloalkyl, -C(=O)NH(C₁₋₆ alkyl), or optionally, two adjacent R² form 5-6 membered heterocyclyl, wherein the heteroatom in the 5-6 membered heterocyclyl is selected from N, O and S, and the number of the heteroatom is 1, 2, 3, or 4;
preferably R² is independently deuterium, OH, NH₂, halogen, CN, C₁₋₄ alkyl, C₂₋₄ alkenyl, C₁₋₄ haloalkyl, C₁₋₄ deuteroalkyl, C₂₋₄ haloalkenyl, -O-C₁₋₄ alkyl, -C₁₋₄ alkylene-O-C₁₋₄ alkyl, -O-C₁₋₄ haloalkyl, -O-C₂₋₄ alkenyl, -O-C₃₋₆ cycloalkyl, -S-C₁₋₄ alkyl, -S-C₃₋₆ cycloalkyl, -C₃₋₆ cycloalkyl, 3-6 membered heterocyclyl, C₆₋₁₀ aryl, 5-6 membered heteroaryl, -C₁₋₄ alkylene-C₃₋₆ cycloalkyl, -C(=O)NH(C₁₋₄ alkyl);
m is 0, 1, 2, 3 or 4; preferably, m is 0, 1, 2 or 3;
R⁴ and R⁵ are each independently H, deuterium, halogen, C₁₋₆ alkyl; preferably, R⁴ and R⁵ are both H or deuterium, or one of R⁴ and R⁵ is H, and the other is deuterium, halogen, or C₁₋₄ alkyl.

In some embodiments according to the first aspect, the present disclosure provides the compound represented by formula (I-H-1) as described above, wherein the compound has a structure shown by formula (I-H-1A), (I-H-1B), (I-H-1C), (I-H-1D):

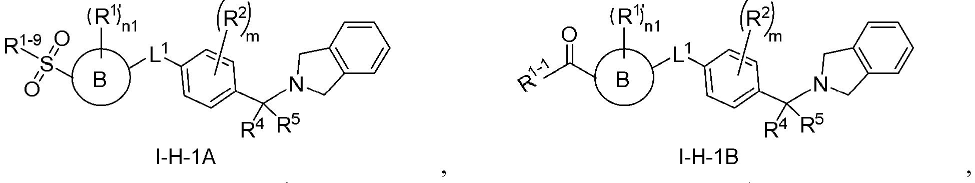

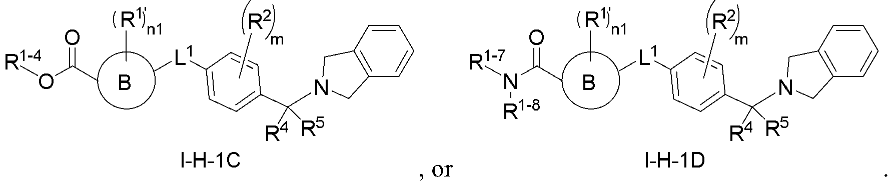

In some embodiments according to the first aspect, the present disclosure provides the compound represented by formula (I) as described above, wherein the compound has a structure shown by formula (I-1), formula (I-2), formula (I-3), formula (I-4) or formula (I-5):

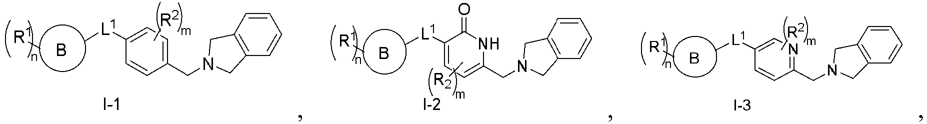

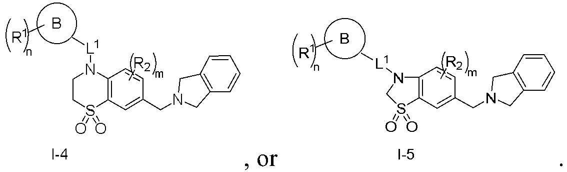

In some embodiments, the m is independently 0, 1, 2, 3 or 4, preferably 2 or 4.

In some embodiments, the R² is independently OH, CN, halogen, C₁₋₃ alkyl, -O-C₁₋₃ alkyl, C₂₋₄ alkenyl, or C₁₋₃ haloalkyl, preferably C₁₋₃ alkyl, CN, or halogen, more preferably methyl or F.

In some embodiments, the present disclosure provides the compound represented by formula (I) as described above, wherein the compound has a structure represented by formula (I-6), formula (I-7), formula (I-8), formula (I-23), formula (I-6') or formula (I-7'):

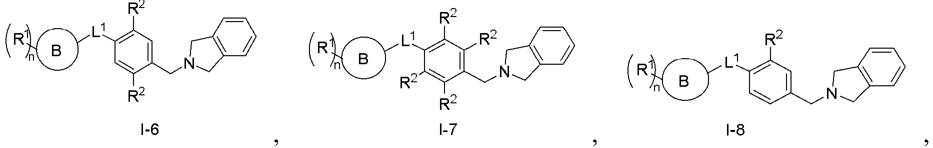

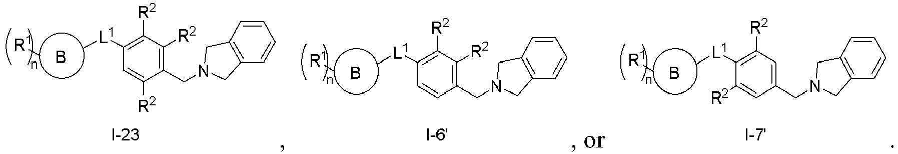

In some embodiments, the L¹ is each independently a bond, O, S, -NH-, C₁₋₆ alkylene that is unsubstituted or substituted by one or more L¹⁻¹, C₂₋₆ alkenylene that is unsubstituted or substituted by one or more L¹⁻², *-C₁₋₆ alkylene-S-, *-S-C₁₋₆ alkylene-, *-C₁₋₆ alkylene-O-, *-O-C₁₋₆ alkylene-, preferably *-CH₂-S-, *-CH₂CH₂-S-, *-CH₂-O-, ethylene or vinylene, more preferably *-CH₂-O-, ethylene or vinylene, more preferably ethylene or vinylene.

In some embodiments, the present disclosure provides the compound represented by formula (0) as described above, wherein the compound has a structure represented by formula (I-10), formula (I-11), formula (I-12), formula (I-13), formula (I-24), formula (I-25), formula (I-12'), formula (I-13'), formula (I-121), formula(I-131), formula(I-120), or formula(I-130):

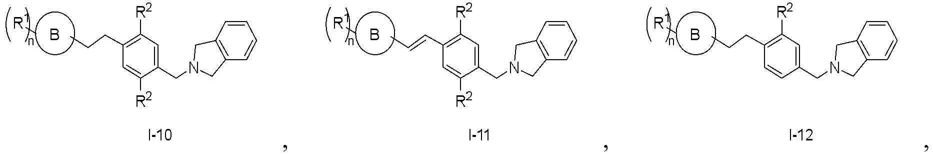

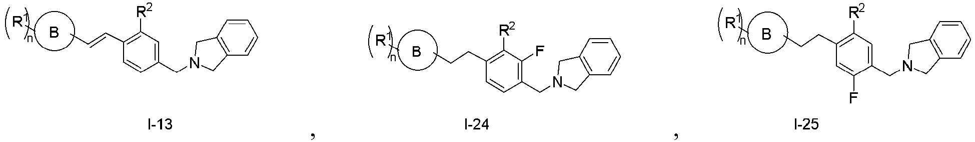

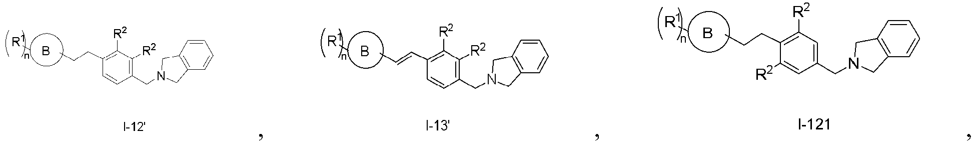

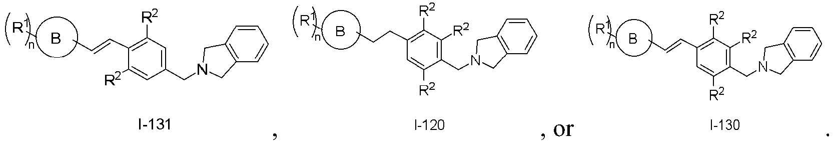

In some embodiments, ring B is independently 5-10 membered heterocyclyl, preferably 5-10 membered nitrogen-containing heterocyclyl, more preferably 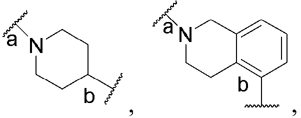 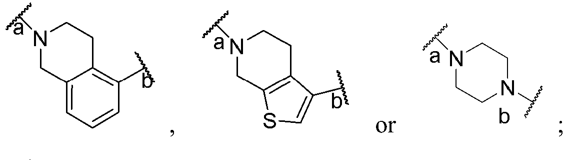 most preferably 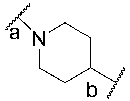 or

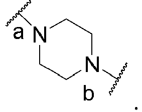

In some embodiments, the present disclosure provides the compound represented by formula (0) as described above, wherein the compound has a structure represented by formula (I-16), formula (I-17), formula (I-18), formula (I-170), formula (1-171) or formula (I-172):

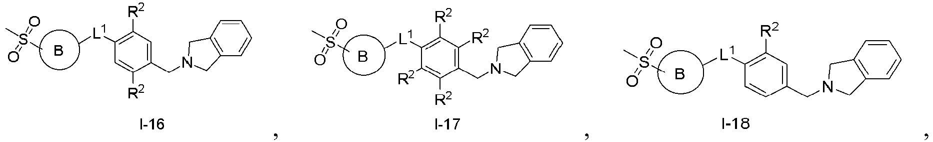

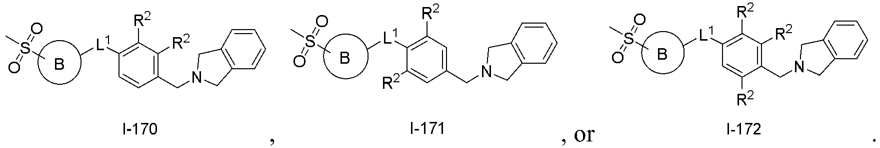

When the ring A of the compounds of the present disclosure is phenyl, the activity (e.g., NCI-H295R cell pregnenolone biosynthesis inhibition) of compounds in which the substituent R² is cyclopropyl is inferior to that of compounds in which the substituent R² is H, halogen (e.g., F, Cl, Br), hydroxyl, alkyl (e.g., C₁₋₃ alkyl), alkenyl (e.g., C₂₋₃ alkenyl), alkoxy (e.g., C₁₋₃ alkoxy), alkenoxy (e.g., C₂₋₃ alkenyloxy) and other compounds.

In some embodiments, the present disclosure provides the compound represented by formula (0) as described above, wherein the compound has a structure represented by formula (I-B-1): 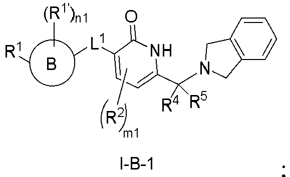 wherein:
R¹ and R^{1'} are each independently deuterium, halogen, cyano, hydroxy, C₁₋₆ alkoxy, R¹⁻¹, -C(=O)-R¹⁻¹, -C(=O)-OR¹⁻⁴, -NR¹⁻⁷R¹⁻⁸, -C(=O)-NR¹⁻⁷R¹⁻⁸, -C(=NH)-(R¹⁻⁹), -S(O)₂-R¹⁻⁹, -S(O)₂-NR¹⁻⁵R¹⁻⁶, -NR¹⁻⁵S(O)₂-R¹⁻¹⁰, -R¹⁻¹⁰-OH, or -P(=O)-R¹⁻²R¹⁻³;
R¹⁻¹, R¹⁻², R¹⁻³, R¹⁻⁴, R¹⁻⁵, R¹⁻⁶, R¹⁻⁷ and R¹⁻⁸ are each independently H, C₁₋₆ alkyl, C₁₋₆ haloalkyl, C₁₋₆ deuteroalkyl, -C₁₋₆ alkylene-OH, -C₁₋₆ alkylene-CN, -C₃₋₁₀ cycloalkyl, or 3-10 membered heterocyclyl, wherein the heteroatom in the 3-10 membered heterocyclyl is selected from N, O and S, and the number of the heteroatom is 1, 2, 3, or 4; preferably, R¹⁻¹, R¹⁻², R¹⁻³, R¹⁻⁴, R¹⁻⁵, R¹⁻⁶, R¹⁻⁷ and R¹⁻⁸ are each independently H, C₁₋₄ alkyl, C₁₋₄ haloalkyl, C₁₋₄ deuteroalkyl, -C₁₋₄ alkylene-OH, -C₁₋₄ alkylene-CN, -C₃₋₆ cycloalkyl, or 3-6 membered heterocyclyl;
R¹⁻⁹ and R¹⁻¹⁰ are each independently C₁₋₆ alkyl, C₁₋₆ haloalkyl, -C₁₋₆ alkylene-CN, C₂₋₆ alkenyl, C₂₋₆ haloalkenyl, C₃₋₁₀ cycloalkyl, C₆₋₁₀ aryl, 5-10 membered heteroaryl, C₁₋₆ alkyl-C₆₋₁₀ arylene-; preferably, R¹⁻⁹ and R¹⁻¹⁰ are each independently C₁₋₄ alkyl, C₁₋₄ haloalkyl, -C₁₋₄ alkylene-CN, C₂₋₄ alkenyl, C₂₋₄ haloalkenyl, C₃₋₆ cycloalkyl, C₆₋₁₀ aryl, 5-10 membered heteroaryl, C₁₋₄ alkyl-C₆₋₁₀ arylene-;
preferably R¹ is -C(=O)-R¹⁻¹, -C(=O)-OR¹⁻⁴, -C(=O)-NR¹⁻⁷R¹⁻⁸, -C(=NH)-(R¹⁻⁹), -S(O)₂-R¹⁻⁹, -S(O)₂-NR¹⁻⁵R¹⁻⁶, -NR¹⁻⁵S(O)₂-R¹⁻¹⁰ or -P(=O)-R¹⁻²R¹⁻³; R^{1'} is deuterium, halogen, cyano, hydroxy, C₁₋₆ alkoxy, R¹⁻¹, -NR¹⁻⁷R¹⁻⁸, -R¹⁻¹⁰-OH;
preferably R¹ is -C(=O)-R¹⁻¹, -C(=O)-OR¹⁻⁴, -S(O)₂-R¹⁻⁹; R^{1'} is hydrogen, deuterium, halogen, cyano, hydroxy, C₁₋₆ alkoxy, -NH₂, -NH(C₁₋₆ alkyl), -N(C₁₋₆ alkyl)₂, C₁₋₆ alkyl, C₁₋₆ haloalkyl, C₁₋₆ deuteroalkyl, -C₁₋₆ alkylene-OH, -C₁₋₆ alkylene-CN, -C₃₋₁₀ cycloalkyl, or 3-10 membered heterocyclyl;
preferably R¹ is -S(O)₂-C₁₋₆ alkyl, -S(O)₂-C₃₋₁₀ cycloalkyl, -C(=O)-OC₁₋₆ alkyl, -C(= O)-C₁₋₆ alkyl, -C(=O)-C₁₋₆ alkylene-CN; R^{1'} is hydrogen, deuterium, halogen, cyano, hydroxy, -NH₂, C₁₋₆ alkyl, C₁₋₆ haloalkyl, C₁₋₆ deuteroalkyl, C₁₋₆ alkoxy;
preferably R¹ is -S(O)₂-C₁₋₄ alkyl, -S(O)₂-C₃₋₆ cycloalkyl, -C(=O)-OC₁₋₄ alkyl, -C(=O)-C₁₋₄ alkyl, -C(=O)-C₁₋₄ alkylene-CN; R^{1'} is hydrogen, deuterium, halogen, cyano, hydroxy, -NH₂, C₁₋₄ alkyl, C₁₋₄ haloalkyl, C₁₋₄ deuteroalkyl, C₁₋₄ alkoxy;
n1 is each independently 0, 1, 2, or 3; preferably, n1 is each independently 0, 1, or 2; more preferably, n1 is 0;
ring B is 5-12 membered heterocyclyl, wherein the heteroatom in the 5-12 membered heterocyclyl is selected from N, O and S, and the number of the heteroatom is 1, 2, 3, or 4; preferably ring B is 5-6 membered monocyclic heterocyclyl, 9-12 membered fused bicyclic heterocyclyl or 9-12 membered spirocyclic heterocyclyl; preferably ring B is 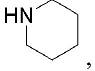 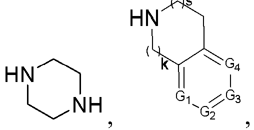 wherein, G₁, G₂, G₃, G₄ is CH or N, at least one of G₁, G₂, G₃, G₄ is CH, s and k are each independently 0, 1 or 2; preferably ring B is 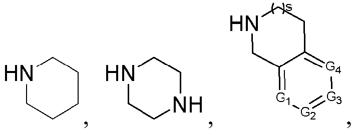 wherein, G₁, G₂, G₃, G₄ is CH or N, at least one of G₁, G₂, G₃, G₄ is CH, s is 0, 1 or 2; preferably 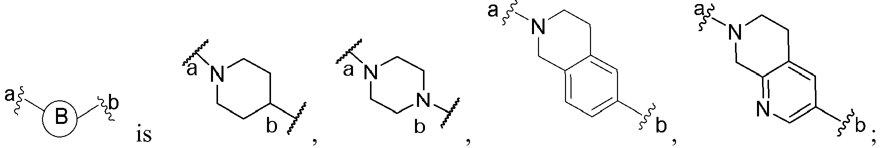
L¹ is a bond, C₁₋₆ alkylene that is unsubstituted or substituted by one or more L¹⁻¹, C₂₋₆ alkenylene that is unsubstituted or substituted by one or more L¹⁻², C₂₋₆ alkynylene that is unsubstituted or substituted by one or more L¹⁻³, *-C₁₋₆ alkylene-S- that is unsubstituted or substituted by one or more L¹⁻⁴, *-C₁₋₆ alkylene-O- that is unsubstituted or substituted by one or more L¹⁻⁶, *-O-C₁₋₆ alkylene- that is unsubstituted or substituted by one or more L¹⁻⁷, *-C₆₋₁₀ arylene-O- that is unsubstituted or substituted by one or more L¹⁻⁸, *-C₁₋₆ alkylene-C(O)- that is unsubstituted or substituted by one or more L¹⁻¹⁰;
preferably L¹ is a bond, C₁₋₄ alkylene that is unsubstituted or substituted by one or more L¹⁻¹, C₂₋₄ alkenylene that is unsubstituted or substituted by one or more L¹⁻², C₂₋₄ alkynylene that is unsubstituted or substituted by one or more L¹⁻³, *-C₁₋₄ alkylene-O- that is unsubstituted or substituted by one or more L¹⁻⁶, *-C₁₋₂ alkylene-C(O)- that is unsubstituted or substituted by one or more L¹⁻¹⁰;
each L¹⁻¹, L¹⁻², L¹⁻³, L¹⁻⁴, L¹⁻⁶, L¹⁻⁷, L¹⁻⁸, L¹⁻¹⁰ is independently halogen, OH, C₁₋₆ alkyl, C₃₋₆ cycloalkyl or 3-6 membered heterocyclyl, wherein the heteroatom in the 3-6 membered heterocyclyl is selected from N, O and S, and the number of the heteroatom is 1, 2, 3, or 4; wherein the C₁₋₆ alkyl, the C₃₋₆ cycloalkyl or the 3-6 membered heterocyclyl is optionally substituted by deuterium, halogen, OH, CN or -NH₂;
preferably L¹ is a bond, -CH₂-CH₂-, *-CH₂-CHF-, *-CH₂-CH(OH)-, *-CH₂-O-, -CH=CH-, *-CH=CF-, -C≡C-, *-CH₂-C(O)-;
preferably L¹ is -CH₂-CH₂- or -CH=CH-;
each R² is independently deuterium, halogen, CN, C₁₋₆ alkyl, C₁₋₆ haloalkyl, C₁₋₆ deuteroalkyl, -C₁₋₆ alkylene-C₃₋₁₀ cycloalkyl;
preferably R² is independently halogen, C₁₋₆ alkyl, C₁₋₆ haloalkyl, C₁₋₆ deuteroalkyl, -C₁₋₄ alkylene-C₃₋₆ cycloalkyl;
preferably R² is independently F, Cl, Br, C₁₋₄ alkyl, C₁₋₄ haloalkyl, C₁₋₄ deuteroalkyl;
m1 is 0, 1 or 2, preferably, m1 is 1 or 2; more preferably, m1 is 2;
R⁴ and R⁵ are each independently H, deuterium, halogen, C₁₋₆ alkyl; preferably, R⁴ and R⁵ are both H or deuterium, or one of R⁴ and R⁵ is H, and the other is deuterium, halogen, or C₁₋₄ alkyl.

In some embodiments, the present disclosure provides the compound represented by formula (0) as described above, wherein the compound has a structure represented by formula (I-B-1A), (I-B-1B) or (I-B-1C): 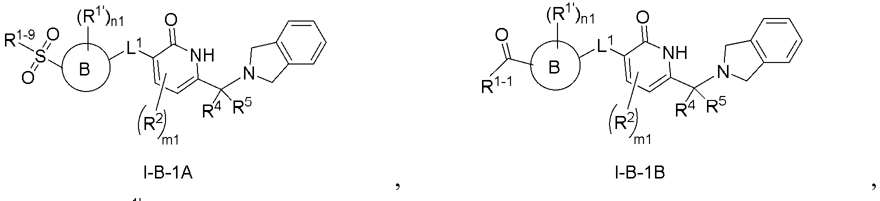 or

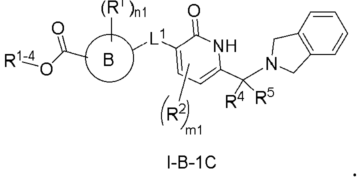

At least some of the compounds of the present disclosure in which R¹ is cyanoacetyl have PK (pharmacokinetic) properties superior to compounds in which R¹ is methylsulfonyl.

At least some of the compounds of the present disclosure in which L¹ is alkenylene (e.g. -CH=CH-) have PK (pharmacokinetic) properties superior to compounds in which L¹ is alkylene (e.g. -CH2-CH2-).

When 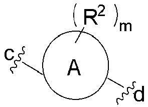 of the compounds of the present disclosure is 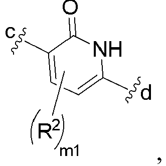 at least some of the compounds in which the ring N atom is substituted with a non-hydrogen substituent (e.g., methyl, ethyl, -CD₃) have PK (pharmacokinetic) properties superior to compounds in which the ring N atom is substituted with hydrogen (i.e., NH); and at least some of the compounds in which the oxo group para-position 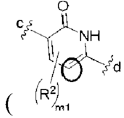 the circled portion) is substituted with an F have PK (pharmacokinetic) properties superior to those compounds in which the oxo group para-position is not substituted.

In some embodiments, the present disclosure provides the compound represented by formula (0) as described above, wherein the compound has a structure represented by formula (I-B-1A-1), (I-B-1A-2), (I-B-1B-1), (I-B-1B-2), (I-B-1C-1) or (I-B-1C-2): 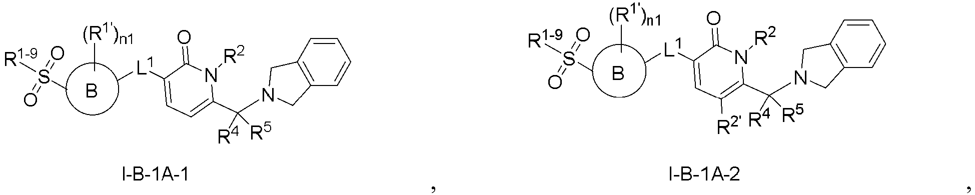 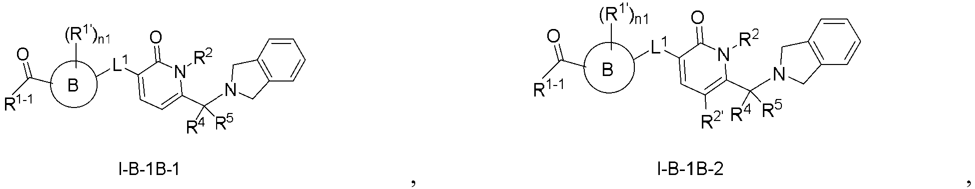 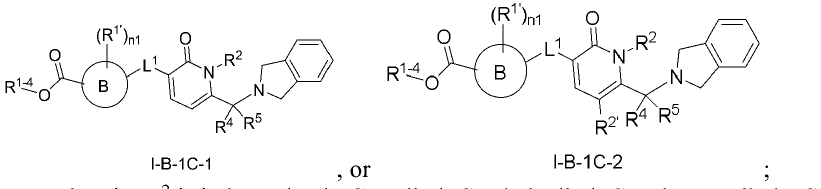 wherein, R² is independently C₁₋₆ alkyl, C₁₋₆ haloalkyl, C₁₋₆ deuteroalkyl, -C₁₋₄ alkylene-C₃₋₆ cycloalkyl; R^{2'} is halogen; preferably R² is independently C₁₋₄ alkyl, C₁₋₄ haloalkyl, C₁₋₄ deuteroalkyl; R^{2'} is F.

In some embodiments, the present disclosure provides the compound represented by formula (0) as described above, wherein the compound has a structure represented by formula (III-1), (III-2), (III-3), (III-4), (III-5), or (III-6): 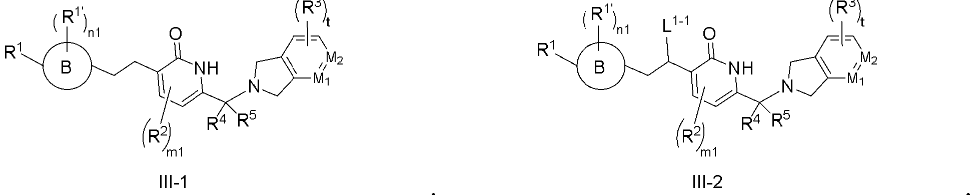 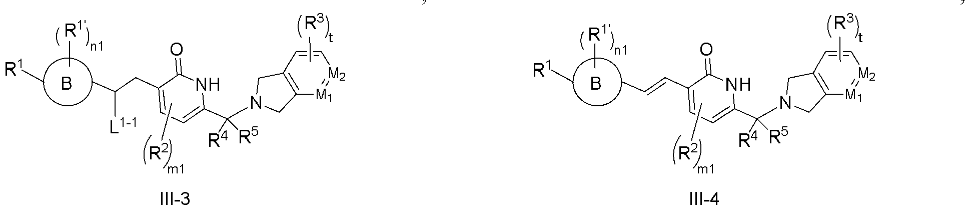 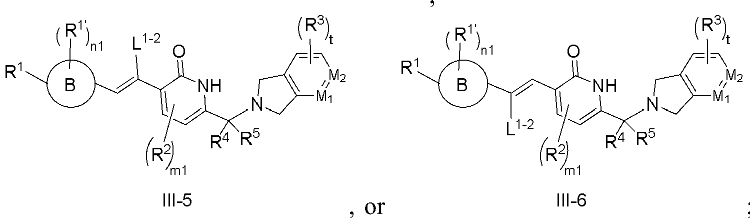 ; wherein, m1 is 0, 1 or 2, preferably m1 is 1 or 2, more preferably m1 is 2; M₁, M₂ each independently is CH or N, preferably at least one of M₁, M₂ is selected from CH; L¹⁻¹ is selected from halogen, OH, C₁₋₄ alkyl, preferably F, OH; L¹⁻² is selected from halogen, C₁₋₄ alkyl, preferably F.

In some embodiments, the present disclosure provides the compound represented by formula (0) as described above, provided that: when the 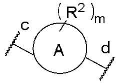 moiety is 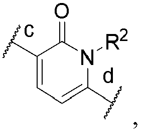 wherein the mark "c" indicates the bond connected to L¹, and the mark "d" indicates the bond connected to -C(R⁴R⁵)-; L¹ is not *-CH₂-O-, wherein the mark "*" indicates the bond connected to ring B.

In some embodiments, the present disclosure provides a compound represented by formula (0), or a stereoisomer, a tautomer, a diastereoisomer, a racemate, a cis-trans isomer, an isotope labeled compound (preferably deuterated compound), an N-oxide, a metabolite, an ester, a prodrug, a crystalline form, a hydrate, a solvate, or a pharmaceutically acceptable salt thereof, wherein the compound is selected from:

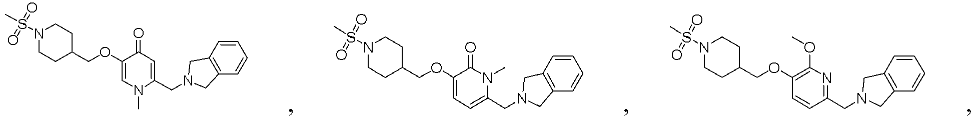

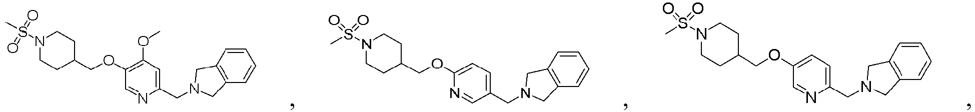

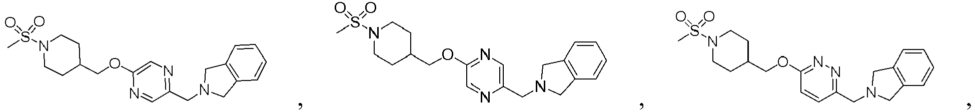

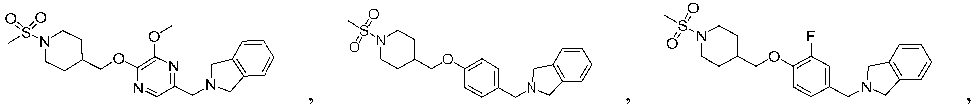

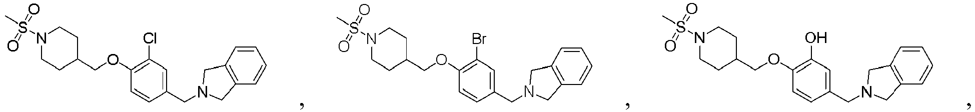

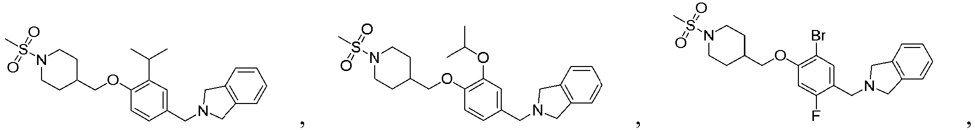

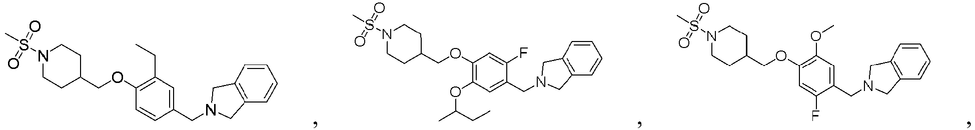

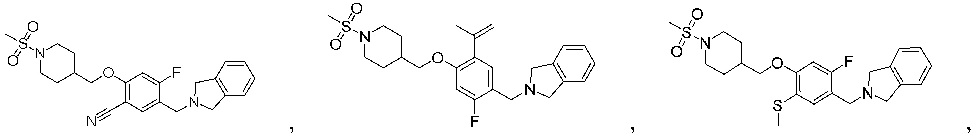

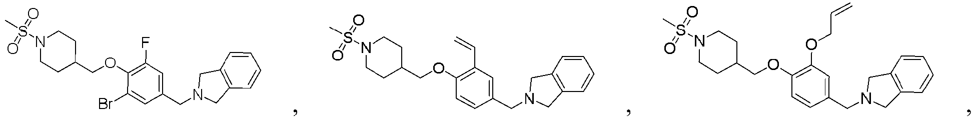

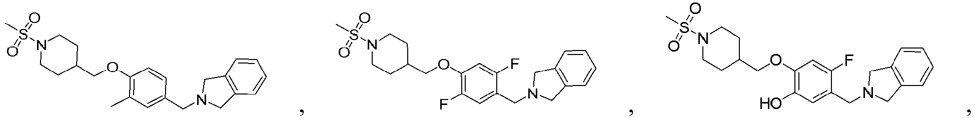

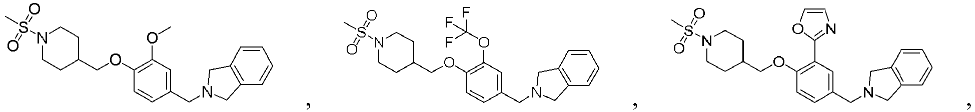

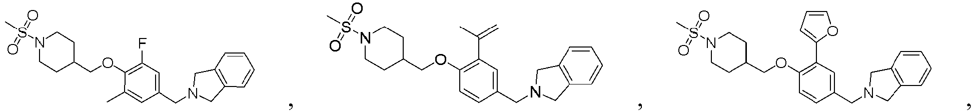

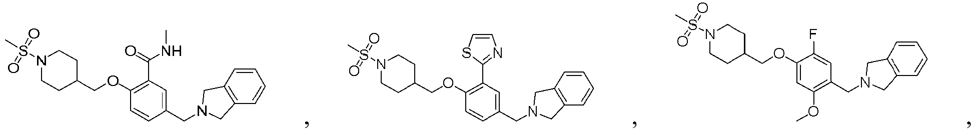

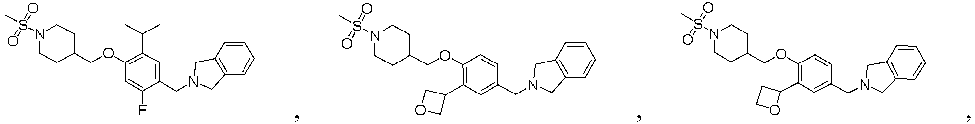

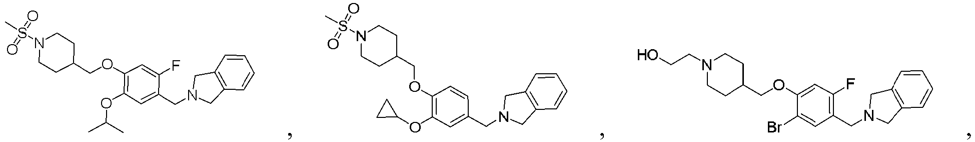

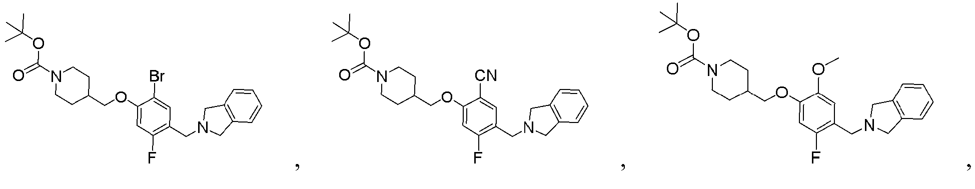

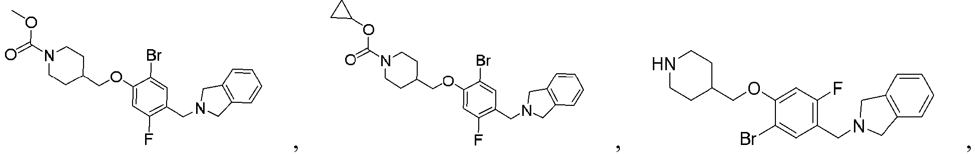

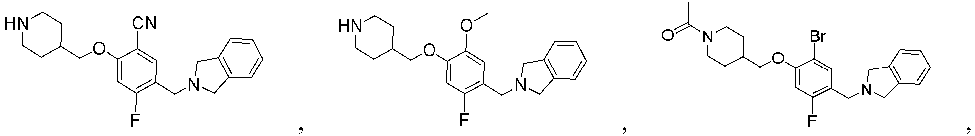

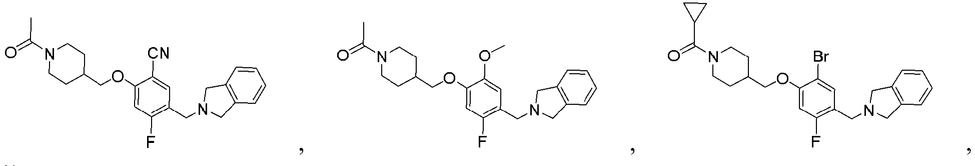

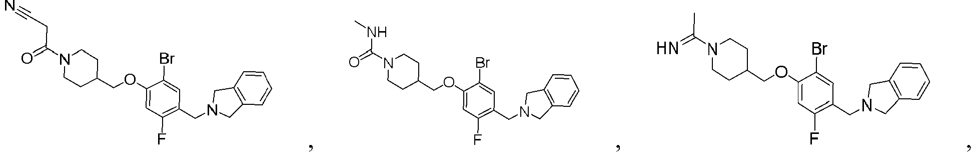

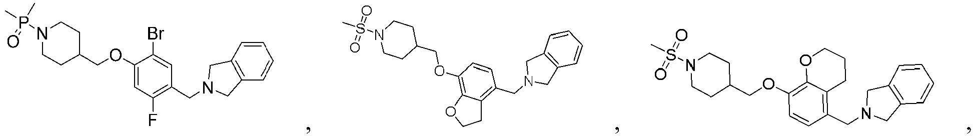

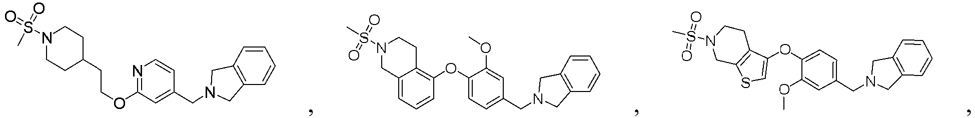

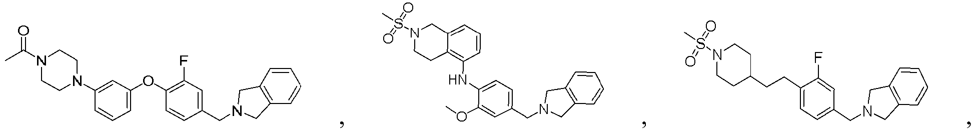

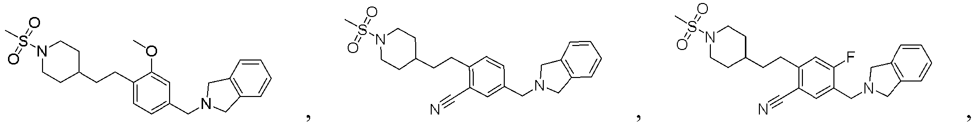

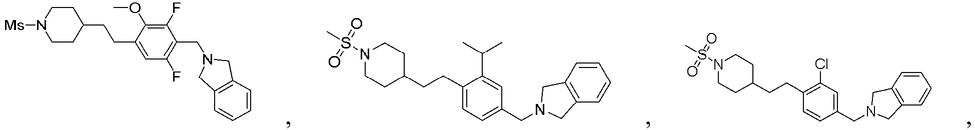

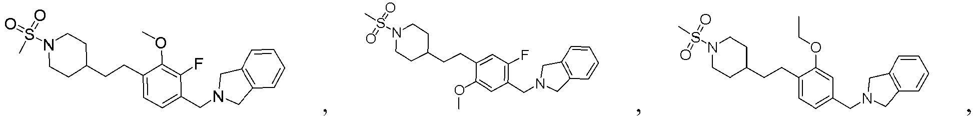

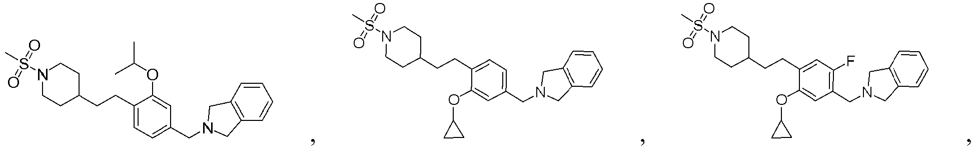

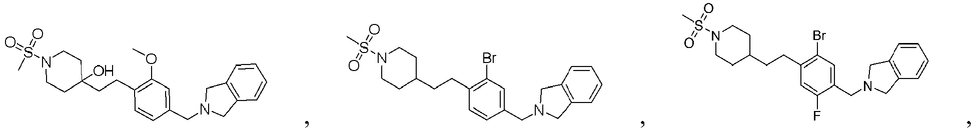

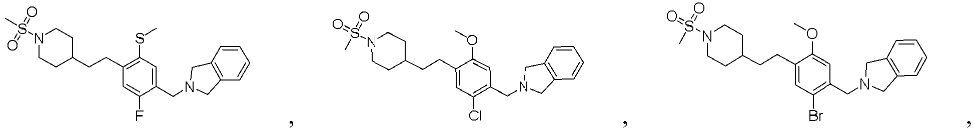

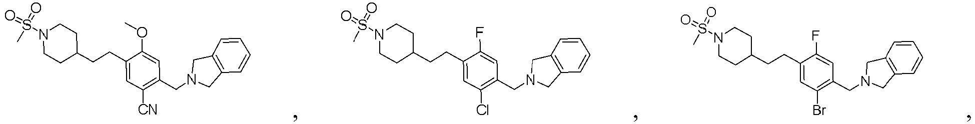

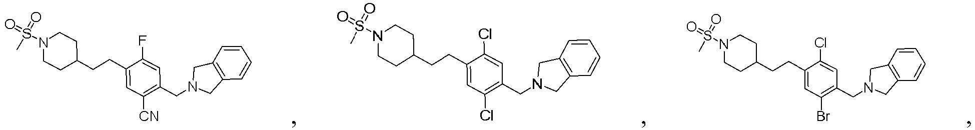

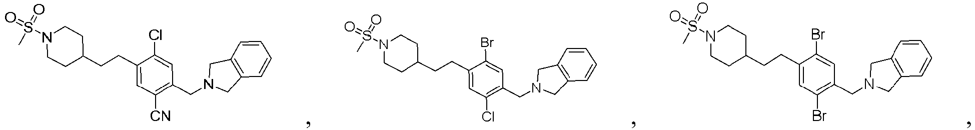

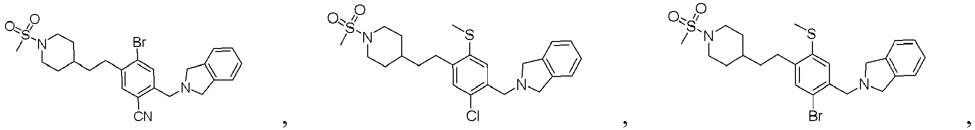

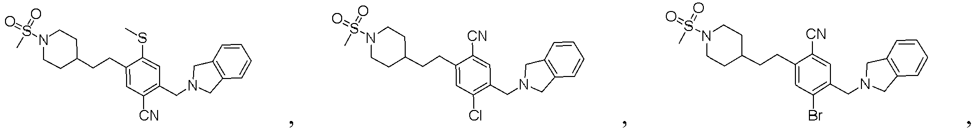

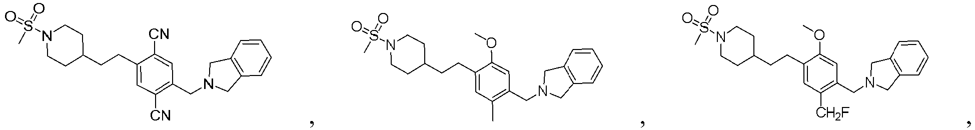

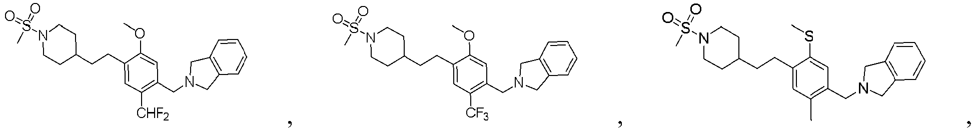

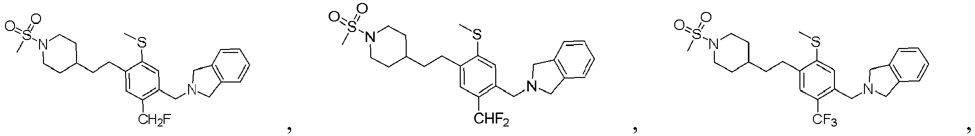

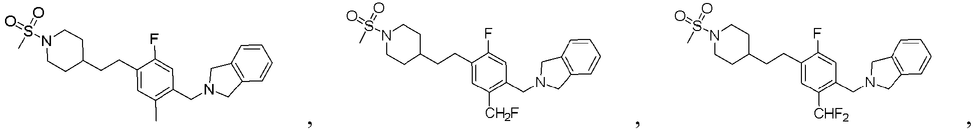

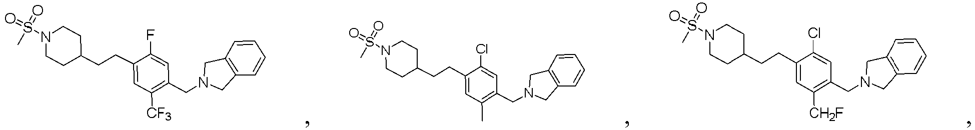

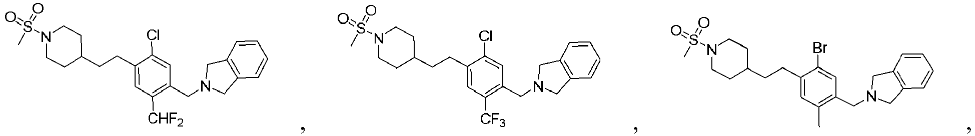

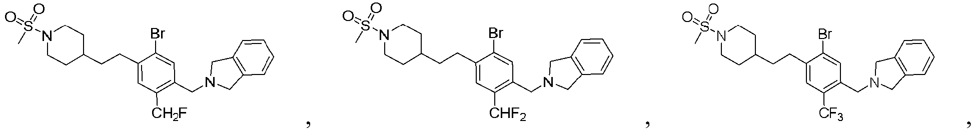

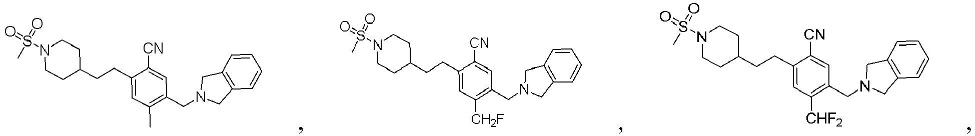

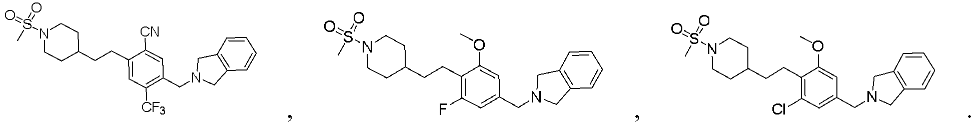

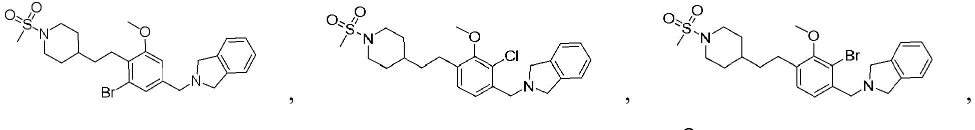

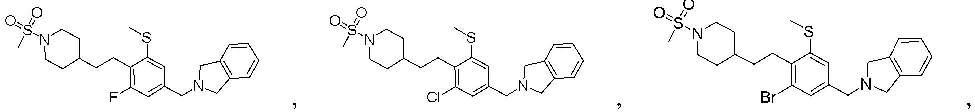

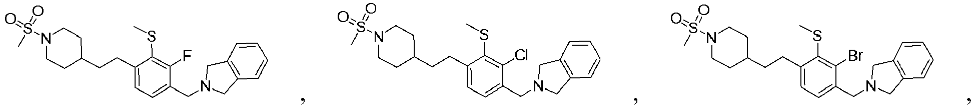

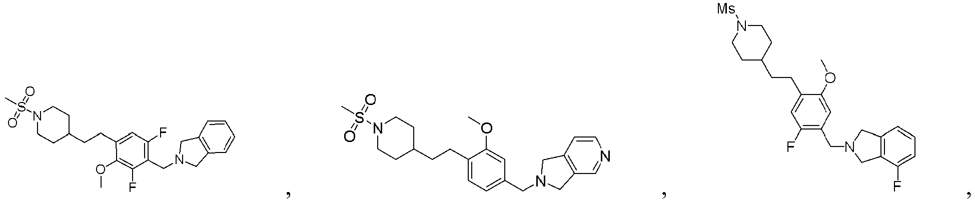

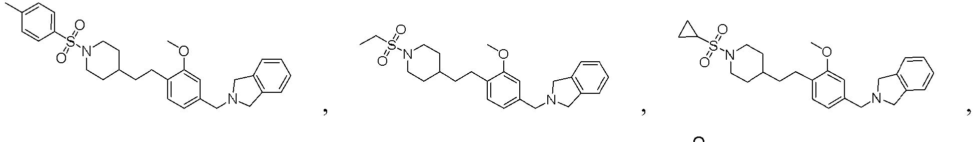

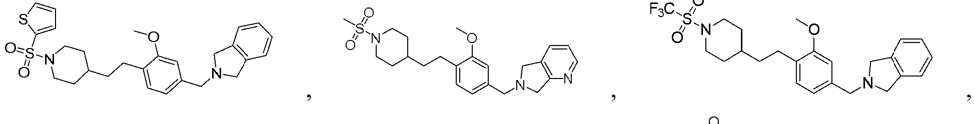

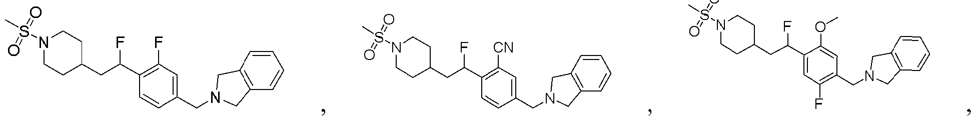

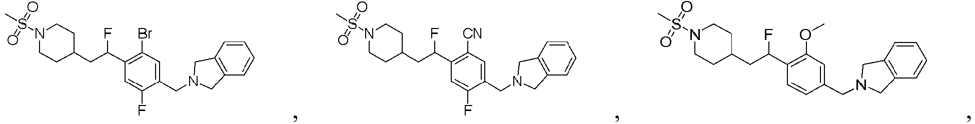

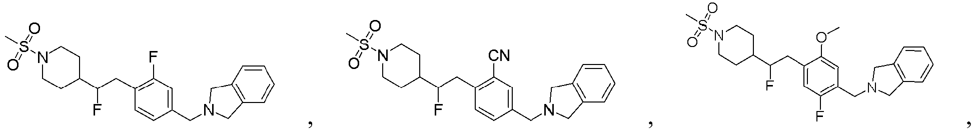

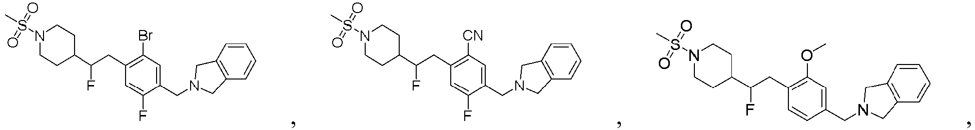

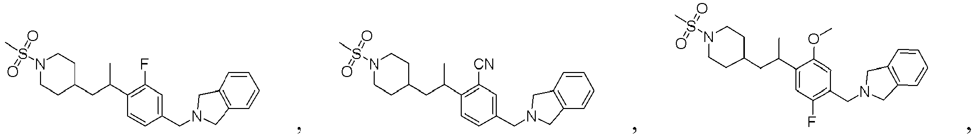

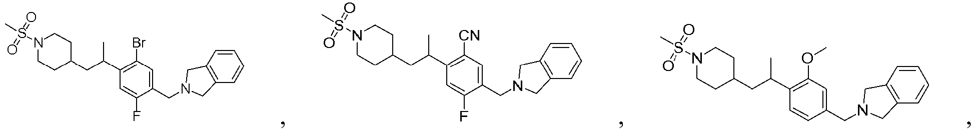

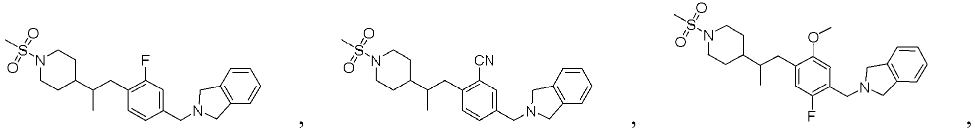

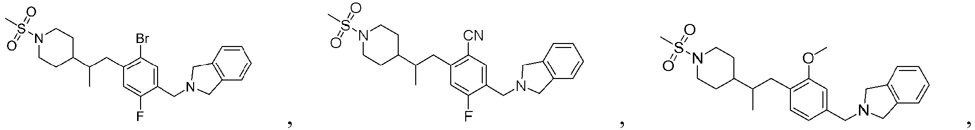

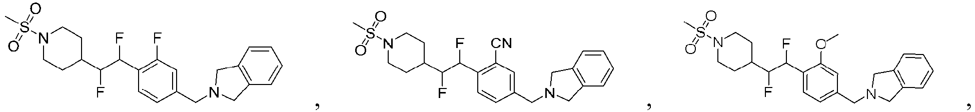

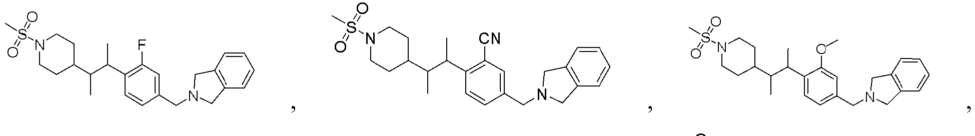

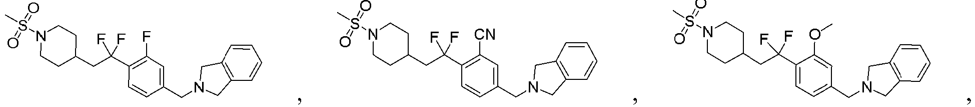

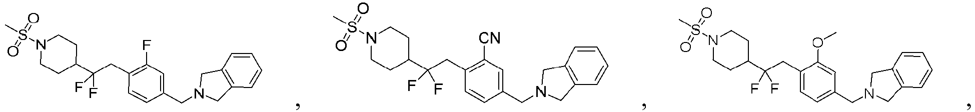

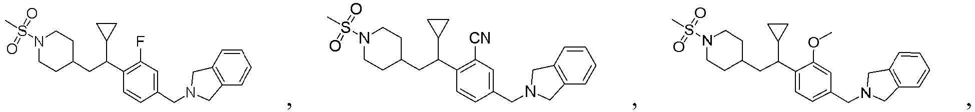

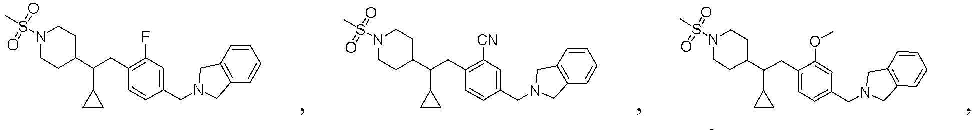

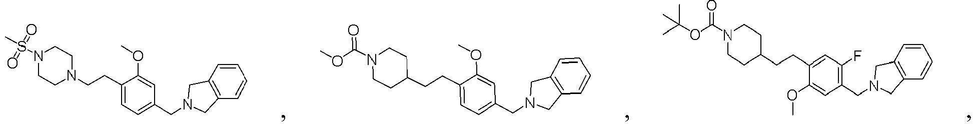

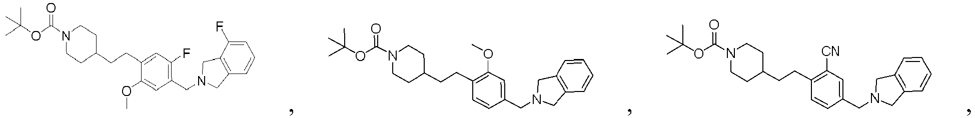

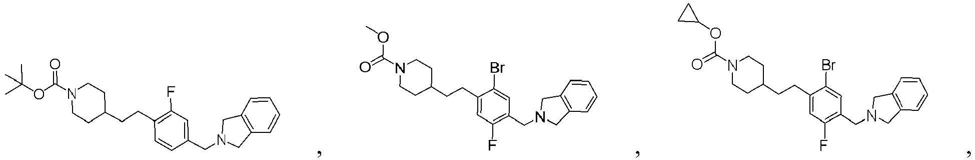

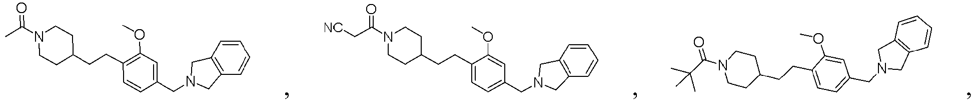

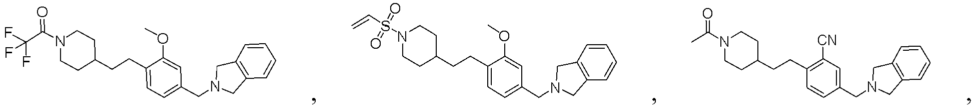

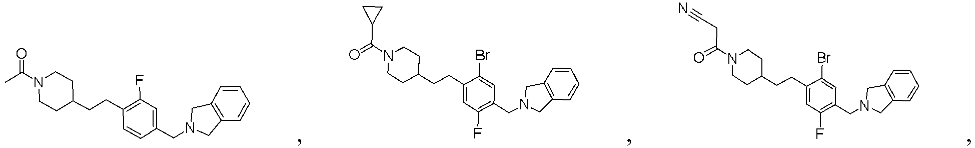

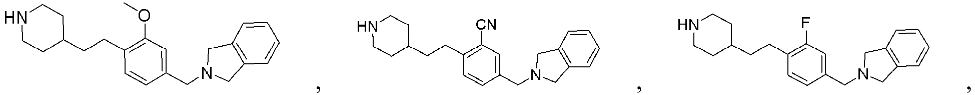

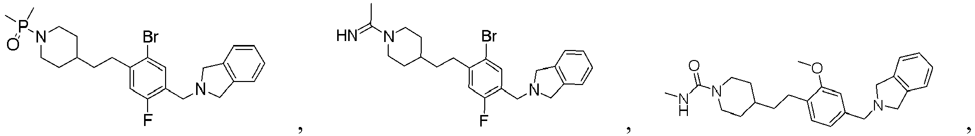

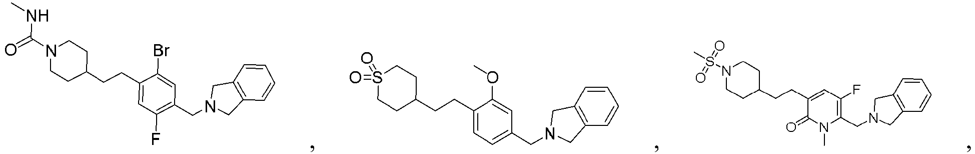

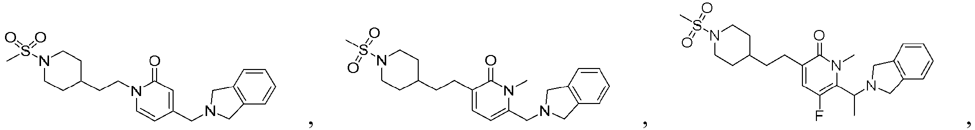

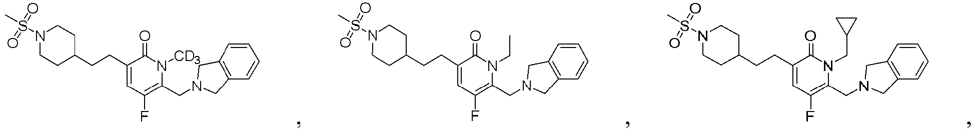

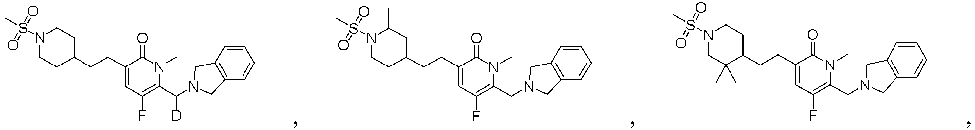

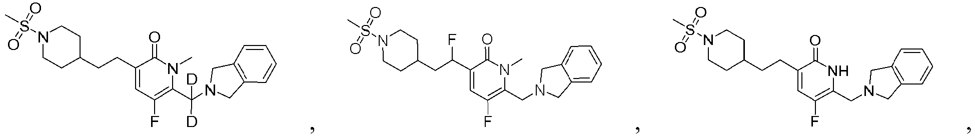

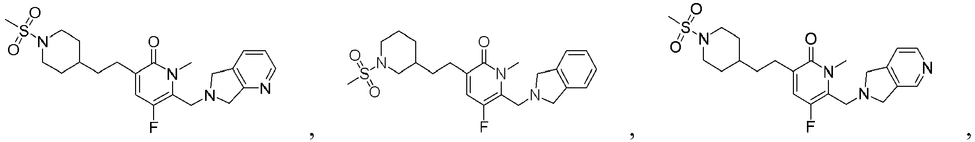

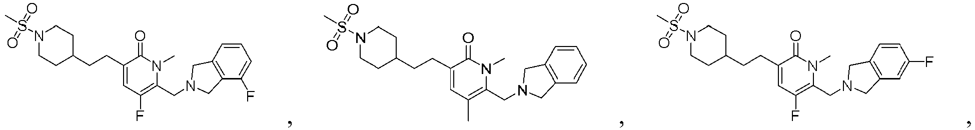

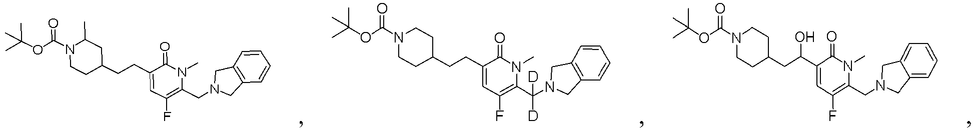

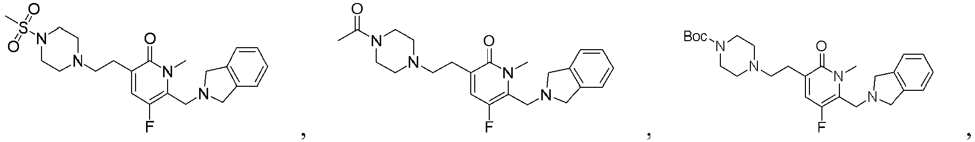

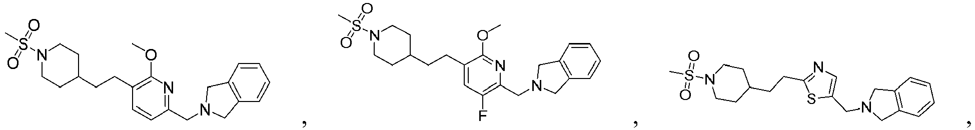

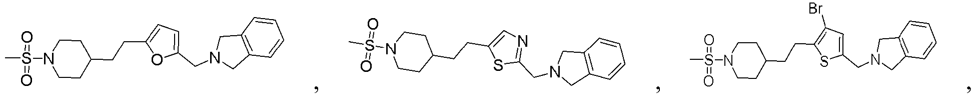

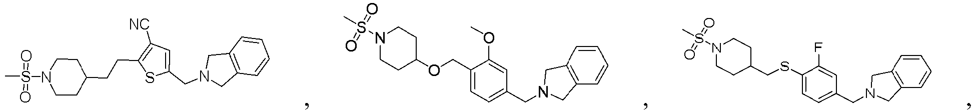

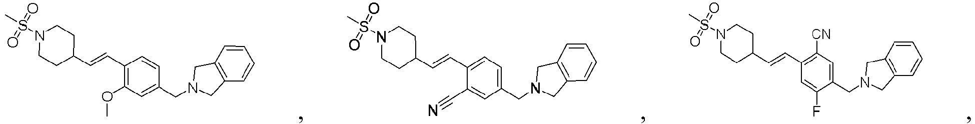

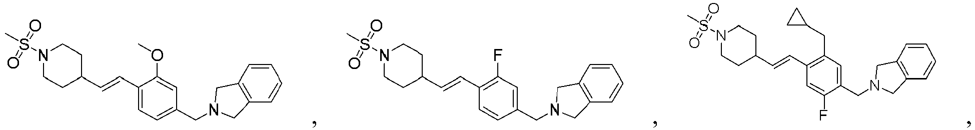

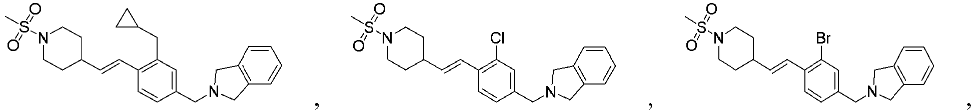

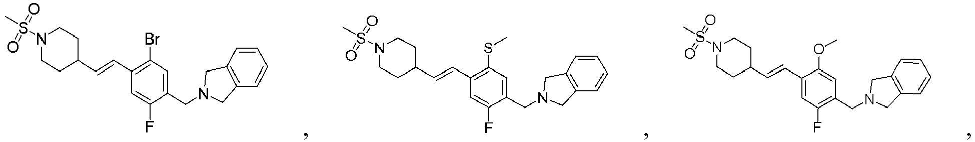

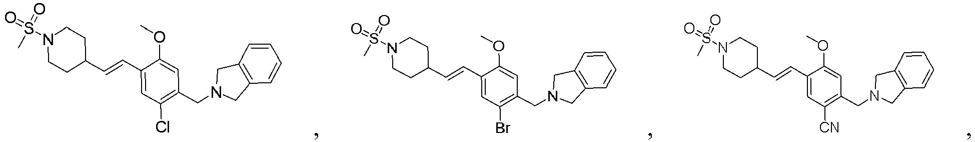

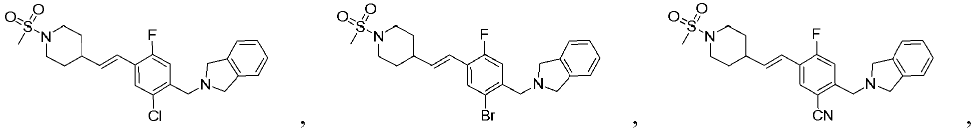

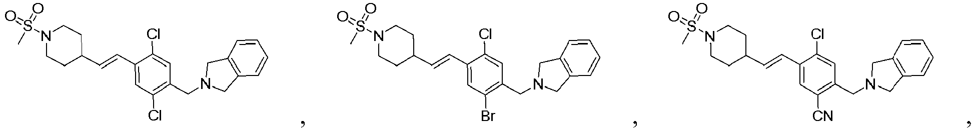

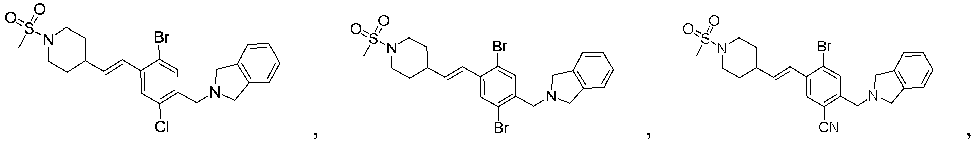

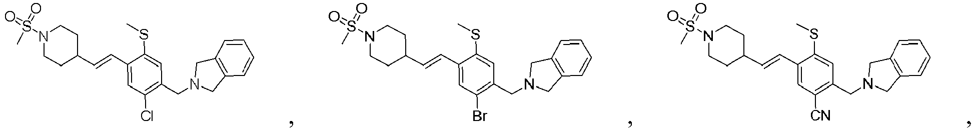

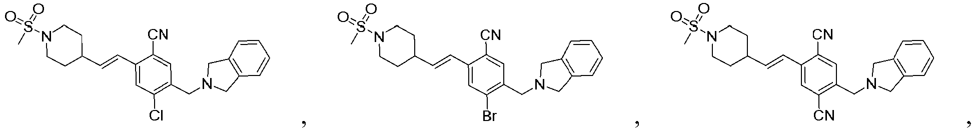

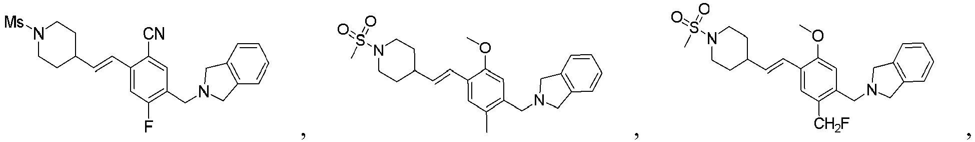

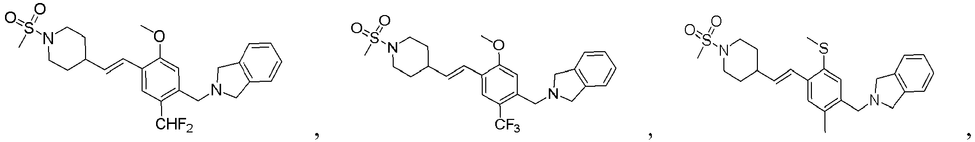

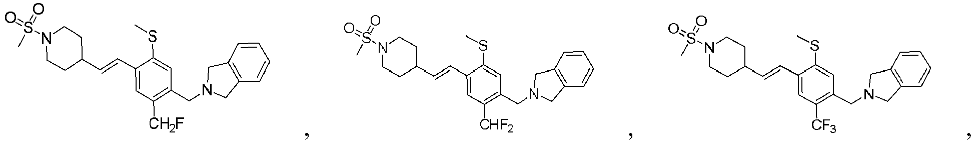

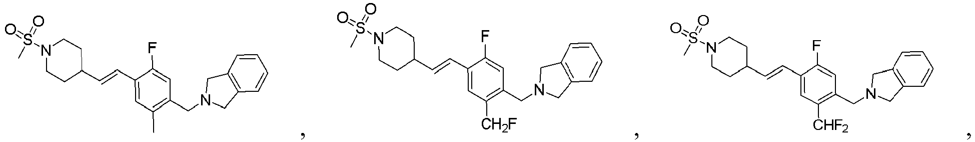

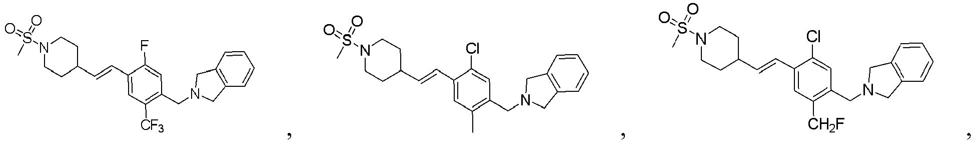

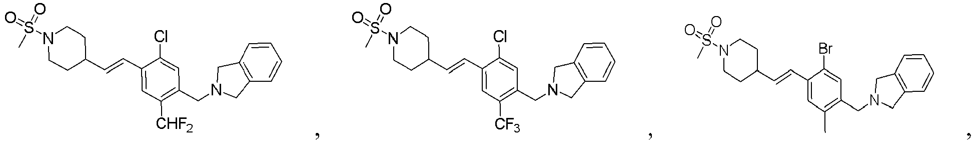

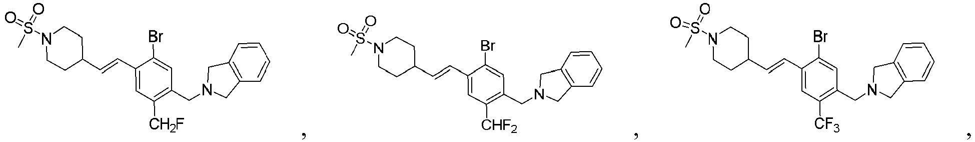

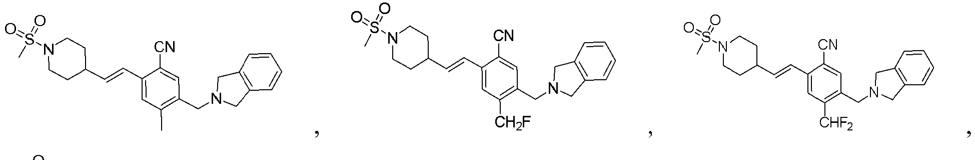

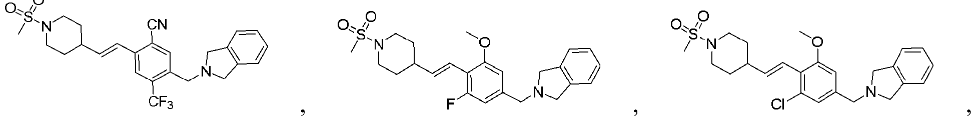

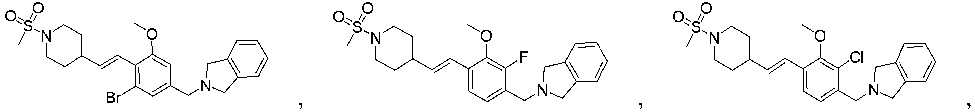

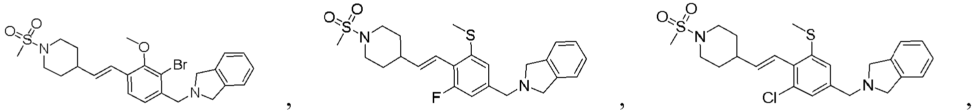

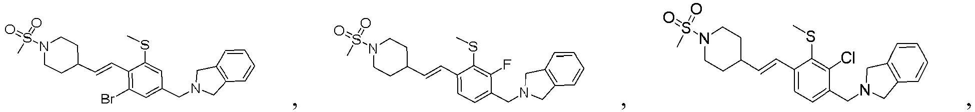

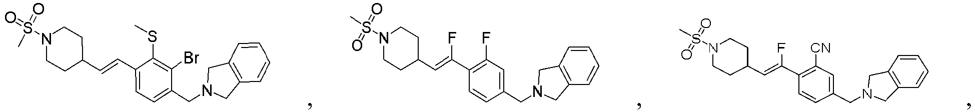

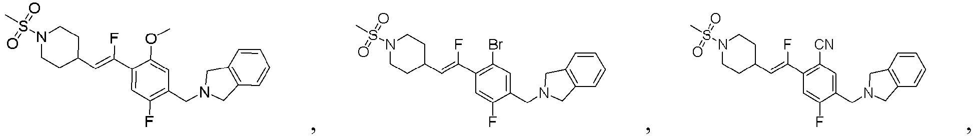

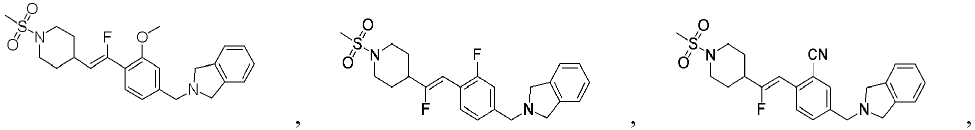

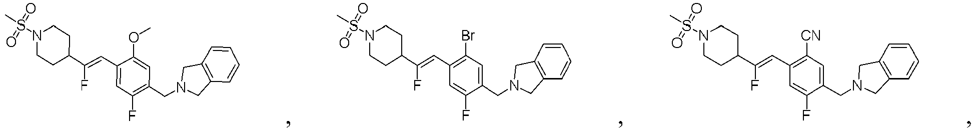

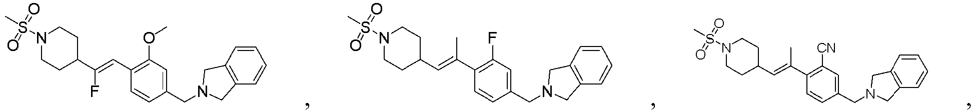

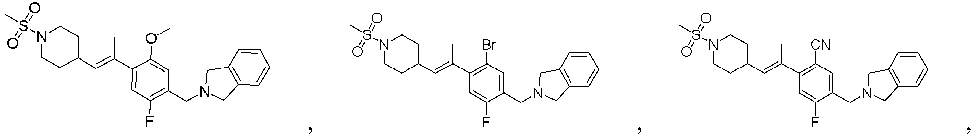

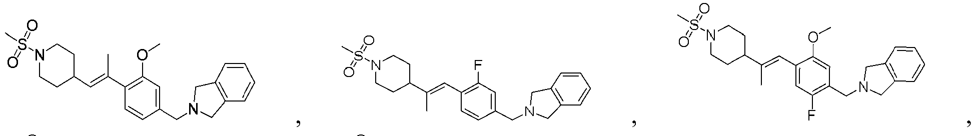

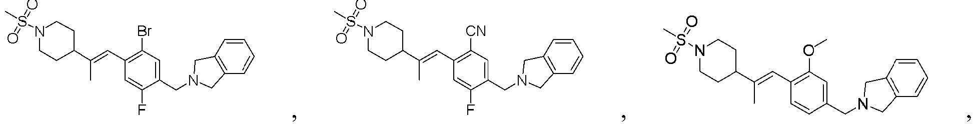

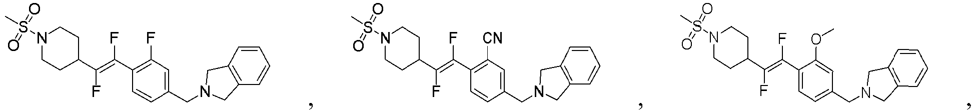

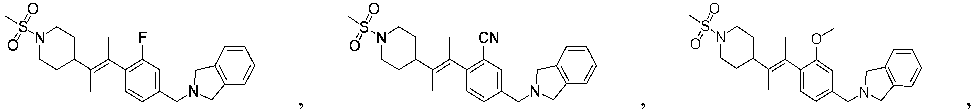

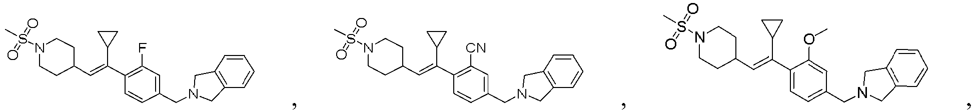

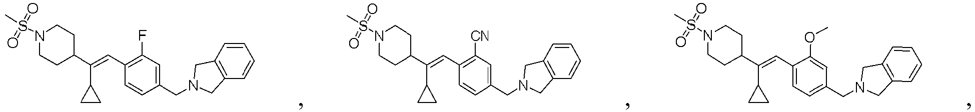

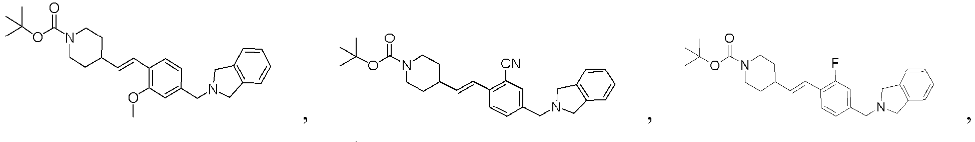

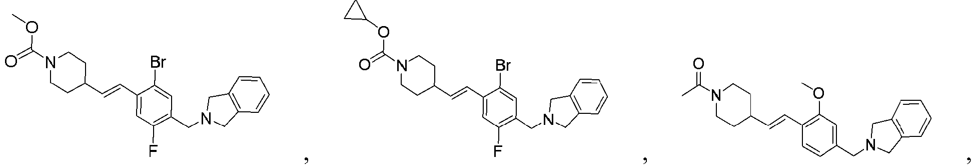

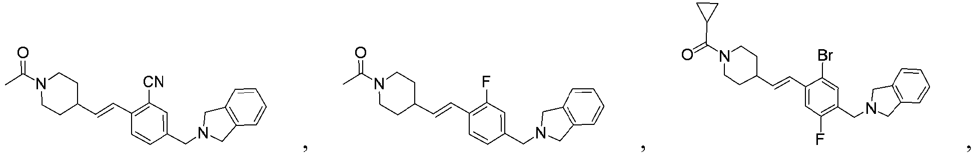

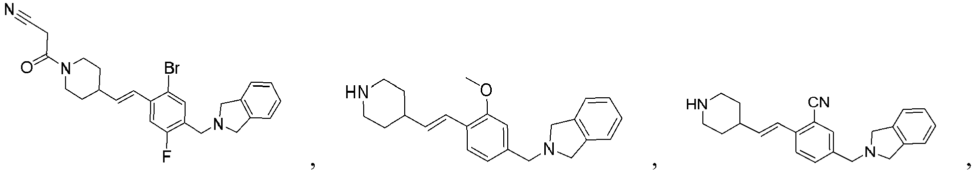

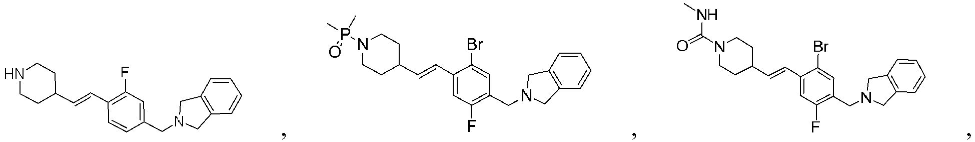

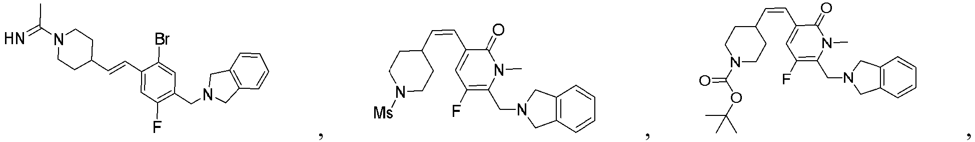

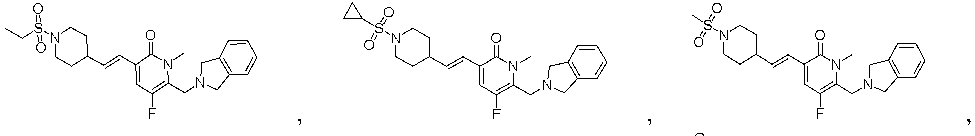

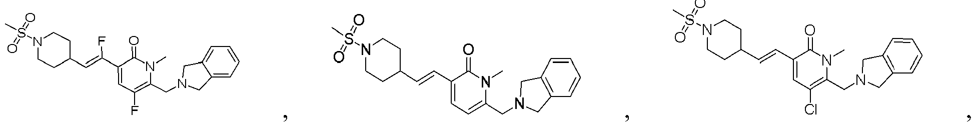

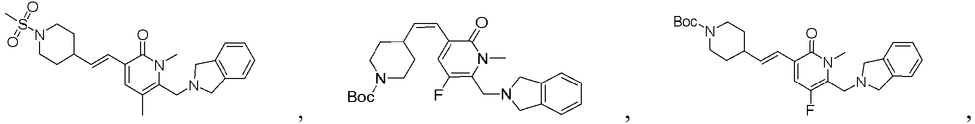

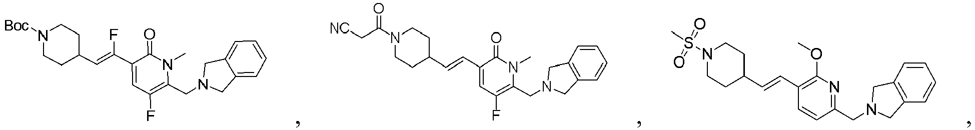

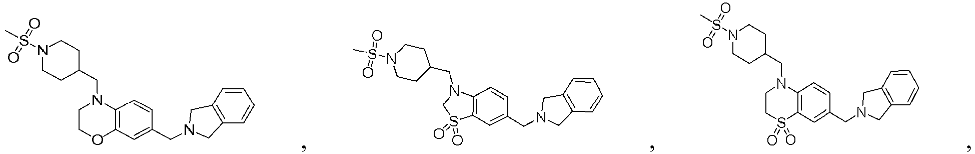

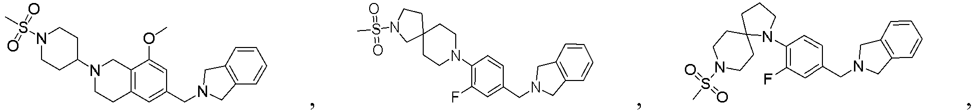

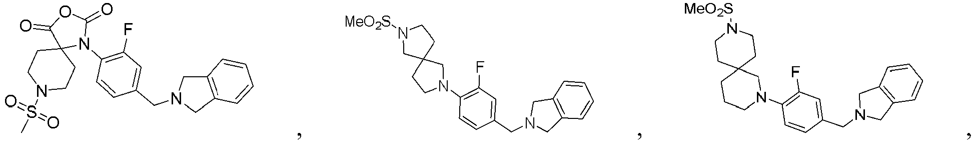

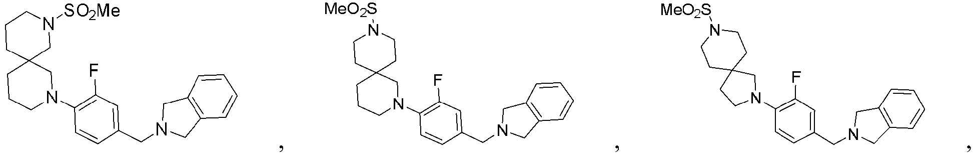

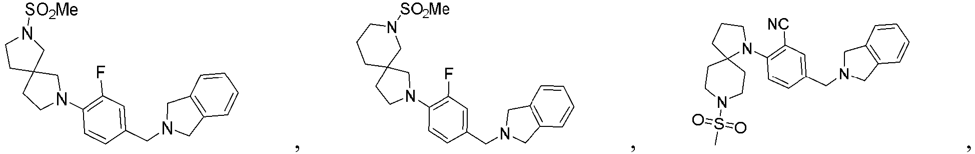

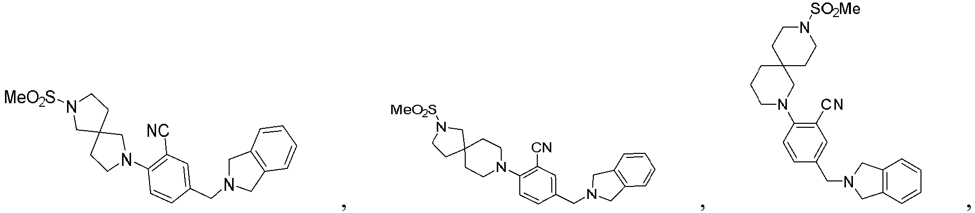

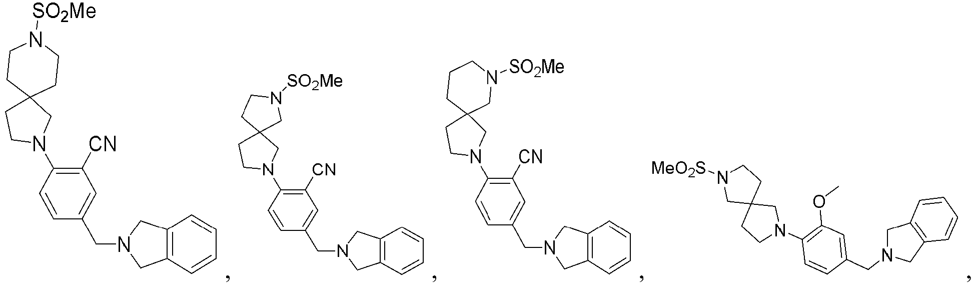

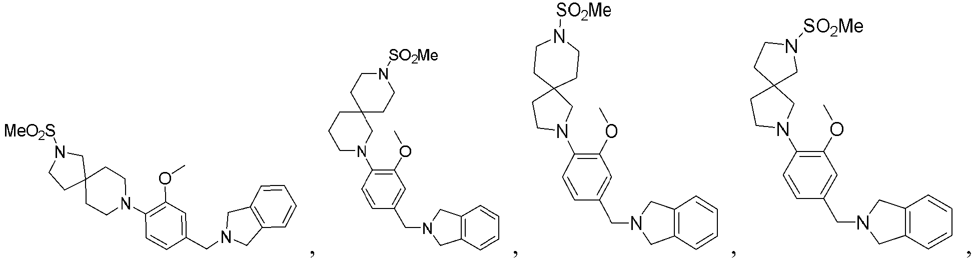

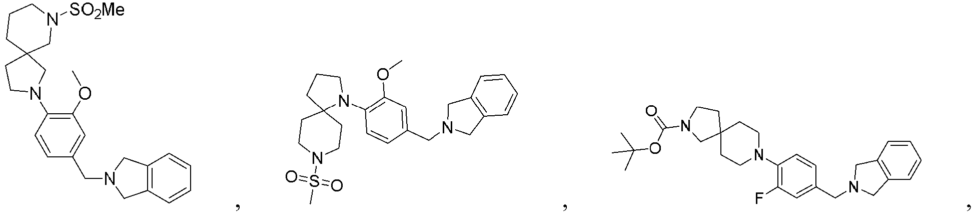

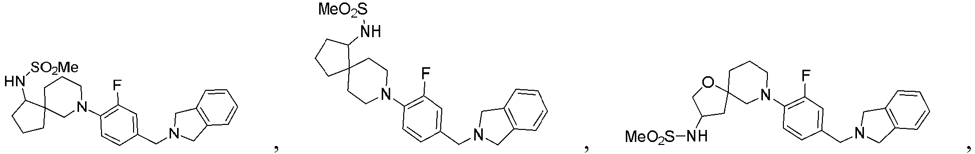

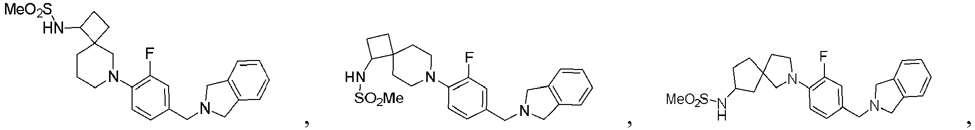

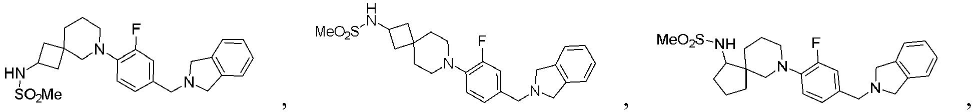

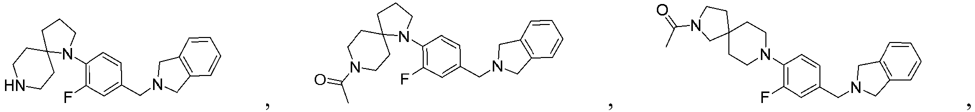

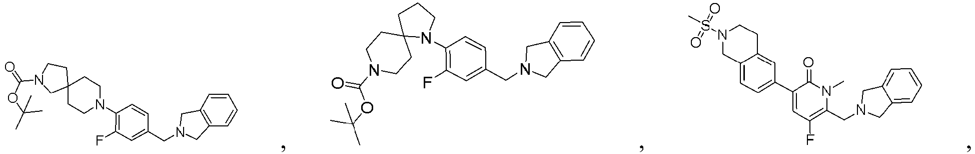

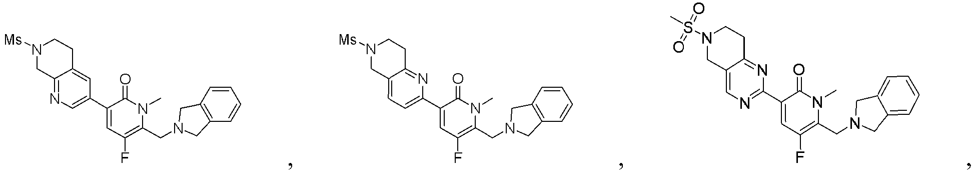

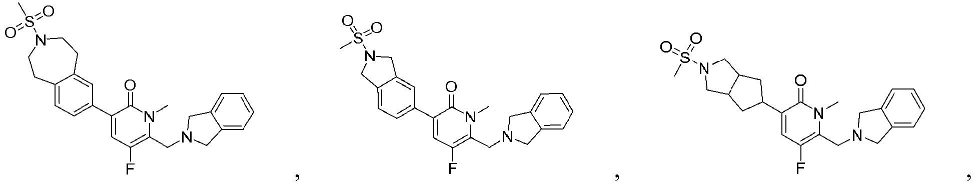

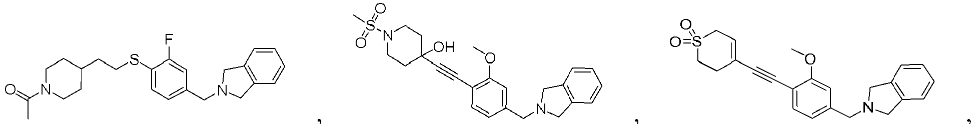

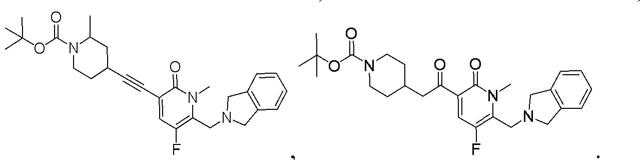

In some embodiments, the present disclosure provides a compound represented by formula (0), or a stereoisomer, a tautomer, a diastereoisomer, a racemate, a cis-trans isomer, an isotope labeled compound (preferably deuterated compound), an N-oxide, a metabolite, an ester, a prodrug, a crystalline form, a hydrate, a solvate, or a pharmaceutically acceptable salt thereof, wherein the compound is not

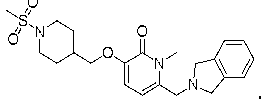

In another aspect, the present disclosure provides a method for preparing the compound represented by formula (I): 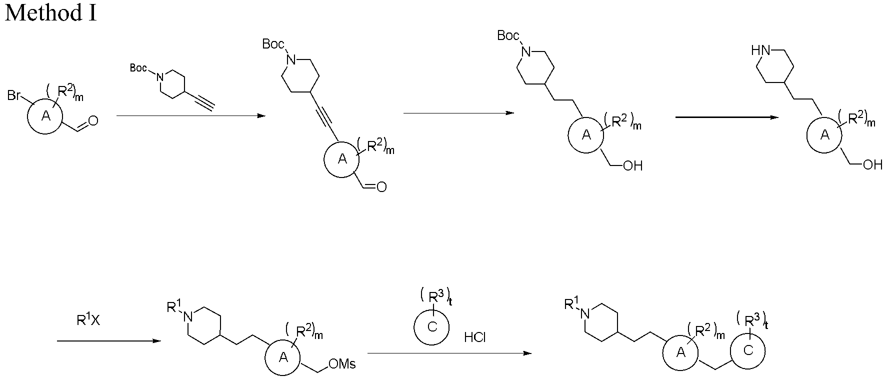
wherein ring A is preferably C₆₋₁₀ aryl; R¹ is preferably R¹⁻¹, -C(=O)-R¹⁻¹, -(CR¹⁻²R¹⁻³)_{q}-C(=O)-OR¹⁻⁴, -C(=O)-NR¹⁻⁷R¹⁻⁸, -S(O)₂-NR¹⁻⁵R¹⁻⁶, -C(=NH)-(R¹⁻⁹), -S(O)₂-R¹⁻⁹, - R¹⁻¹⁰-OH, -P(=O)-R¹⁻²R¹⁻³;
preferably, 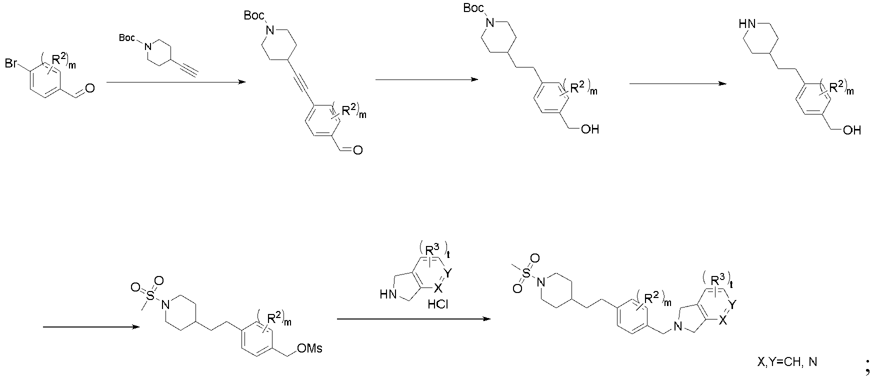 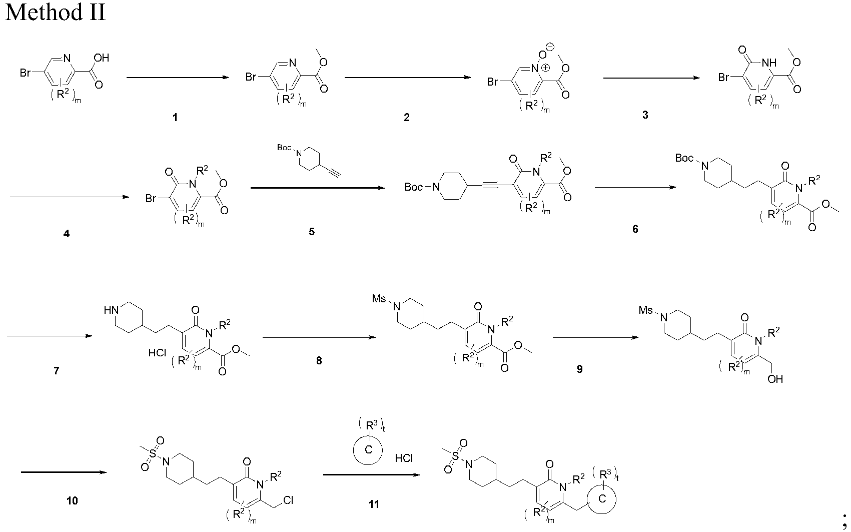
ring C is preferably 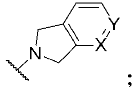 X or Y is independently CH or N;

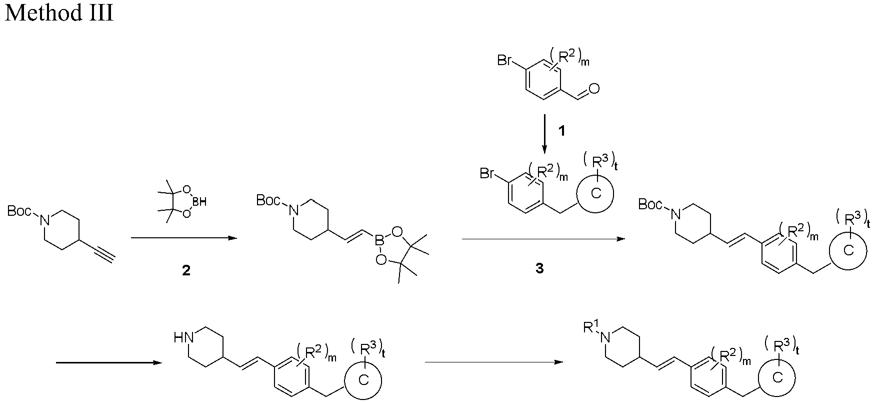

preferably,

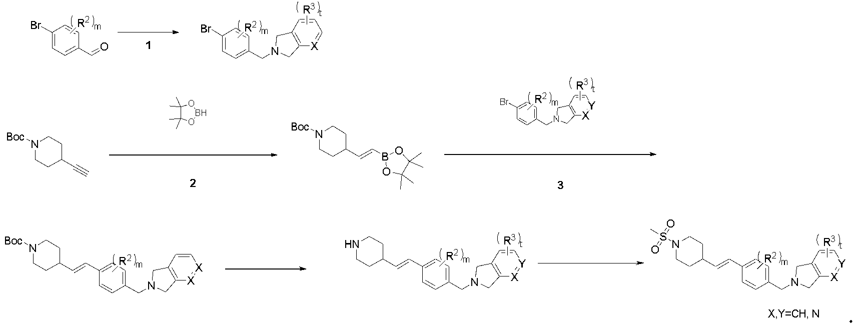

In another aspect, the present disclosure provides a pharmaceutical composition, comprising the above-defined compound represented by formula (0), or a stereoisomer, tautomer, a diastereoisomer, a racemate, a cis-trans isomer, an isotope labeled compound (preferably deuterated compound), an N-oxide, a metabolite, an ester, prodrug, a crystalline form, a hydrate, a solvate, or a pharmaceutically acceptable salt thereof, as well as pharmaceutically acceptable carriers;
preferably, the pharmaceutical composition comprises the compound represented by formula (0) and at least one additional active ingredient selected from the following:
- glucocorticoids;
- mineralocorticoids;
- nonsteroidal androgen receptor antagonists;
- steroidogenesis inhibitors;
- chemotherapeutic agents;
- antiestrogen drugs;
- epigenetic regulators;
- mTOR inhibitors (e.g., Everolimus);
- AKT inhibitors (e.g., AZ5363);
- radiopharmaceuticals (e.g., Alpharadin);
- GnRH/LHRH analogs (e.g., Leuprorelin);
- PI3K inhibitors; and
- CDK4/6 inhibitors.

In another aspect, the present disclosure provides a method for treating steroid receptor (particularly androgen receptor) dependent conditions and diseases by using the compound represented by formula (0) or the pharmaceutical composition thereof, wherein, the method comprises administering to an individual in need thereof a therapeutically effective amount of above-defined compound represented by formula (0) or a stereoisomer, a tautomer, diastereoisomers, a racemate, a cis-trans isomer, an isotope labeled compound (preferably deuterated compound), an N-oxide, a metabolite, an ester, a prodrug, a crystalline form, a hydrate, a solvate, or a pharmaceutically acceptable salt thereof or the pharmaceutical composition thereof, wherein the steroid receptor dependent diseases is preferably cancer, preferably prostate cancer, more preferably castration-resistant prostate cancer (CRPC);
wherein administering therapeutically effective amount of the compound represented by formula (0) or the pharmaceutical composition thereof, preferably also administering glucocorticoids and/or mineralocorticoids, and optionally administering with one or more anticancer drugs;
wherein administering therapeutically effective amount of the compound represented by formula (0) or the pharmaceutical composition thereof with one or more anticancer drugs, wherein the anticancer drug is preferably selected from:
   - nonsteroidal androgen receptor antagonists;
   - steroidogenesis inhibitor;
   - chemotherapeutic agents;
   - antiestrogen drugs;
   - epigenetic regulators;
   - mTOR inhibitors (e.g., Everolimus);
   - AKT inhibitors (e.g., AZ5363);
   - radiopharmaceuticals (e.g., Alpharadin);
   - GnRH/LHRH analogs (e.g., Leuprorelin);
   - PI3K inhibitors; and
   - CDK4/6 inhibitors.

### Definition of Terms

Unless otherwise defined in the following text, all technical and scientific terms used herein have the same meanings as those commonly understood by those skilled in the art. The mention of the technology used herein intends to refer to the technology commonly understood in the art, including those modifications and equivalent replacements obvious to those skilled in the art. Although it is believed that the following terms are well understood by those skilled in the art, the following definitions are still elaborated to better explain the present disclosure.

The terms "comprising", "including", "having", "containing" or "involving" and their other variations as used herein are inclusive or open-ended, and do not exclude the presence of any other element or method step that is not enumerated, that is, these terms also encompass the terms "substantially consisting of' and "consisting of").

As used herein, the term "alkane" refers to linear or branched saturated aliphatic hydrocarbons.

As used herein, the term "alkyl" refers to linear or branched monovalent saturated aliphatic hydrocarbon, i.e., the moiety obtained by the loss of one hydrogen atom from an alkane. In some embodiments, alkyl has from 1 to 12, such as from 1 to 6 (e.g., 1, 2, 3, 4, 5 or 6) carbon atoms. For example, as used herein, the term "C₁₋₆ alkyl" refers to a linear or branched moiety having from 1 to 6 carbon atoms, including "C₂₋₆ alkyl", "C₂₋₅ alkyl", "C₁₋₄ alkyl", "C₁₋₃ alkyl" and "C₁₋₂ alkyl". The examples of "C₁₋₆ alkyl" include methyl, ethyl, n-propyl, isopropyl, n-butyl, isobutyl, sec-butyl, tert-butyl, n-pentyl and n-hexyl. The alkyl is optionally substituted by one or more (such as, from 1 to 3) suitable substituents such as halogen (hereby the moiety is called "haloalkyl", such as CF₃, C₂F₅, CHF₂, CH₂F, CH₂CF₃, CH₂Cl, -CH₂CH₂CF₃, or the like). The term "C₁₋₄ alkyl" refers to alkyl having from 1 to 4 carbon atoms (i.e., methyl, ethyl, n-propyl, isopropyl, n-butyl, isobutyl, sec-butyl or tert-butyl).

As used herein, the term "alkylene" refers to linear or branched bivalent saturated aliphatic hydrocarbon obtained by the loss of a further hydrogen atom from the "alkyl" stated above. In some embodiments, alkylene has from 1 to 12 carbon atoms, preferably 1, 2, 3, 4, 5 or 6 carbon atoms, such as methylele, ethylene, propylene or butylene.

As used herein, the term "alkenyl" refers to linear or branched monovalent aliphatic hydrocarbon comprising one or more double bonds. In some embodiments, alkenyl has 2-6 carbon atoms ("C₂₋₆ alkenyl"), such as from 2 to 4 carbon atoms ("C₂₋₄ alkenyl"), or from 2 to 3 carbon atoms ("C₂₋₃ alkenyl"). Examples of alkenyl include such as -CH=CH₂, -CH₂CH=CH₂, -C(CH₃)=CH₂, -CH₂-CH=CH-CH₃, 2-pentenyl, 3-pentenyl, 4-pentenyl, 2-hexenenyl, 3-hexenenyl, 4-hexenenyl, 5-hexenenyl, 2-methyl-2-propenyl and 4-methyl-3-pentenyl. When the compound of the present disclosure contains alkenyl, the compound may be in the pure E (entgegen) configuration, the pure Z (zusammen) configuration, or any mixture thereof. The term "alkenylene" refers to corresponding divalent moiety, including such as "C₂₋₆ alkenylene", "C₂₋₄ alkenylene", and "C₂₋₃ alkenylene" etc., with particular examples including, but not limited to: -CH=CH-, -CH₂CH=CH-, -C(CH₃)=CH-, butenylene, pentenylene, hexenenylene, cyclopentenylene, cyclohexenenylene, etc.

As used herein, the term "alkynyl" refers to linear or branched monovalent aliphatic hydrocarbon comprising one or more triple bonds. In some embodiments, alkynyl has 2, 3, 4, 5 or 6 carbon atoms ("C₂₋₆ alkynyl"), such as from 2 to 4 carbon atoms ("C₂₋₄ alkynyl"), or from 2 to 3 carbon atoms ("C₂₋₃ alkynyl"), such as ethynyl, 2-propynyl, 2-butynyl, 1,3-butadiynyl, etc. Alkynyl is optionally substituted by one or more (such as, from 1 to 3) substituents that may be same or different from each other. The term "alkynylene" refers to corresponding divalent moiety, including such as "C₂₋₆ alkynylene", "C₂₋₄ alkynylene", "C₂₋₃ alkynylene" and the like, with particular examples including, but not limited to: 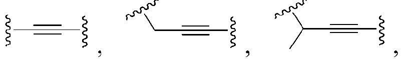 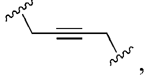 etc. Alkynylene is optionally substituted by one or more (such as, from 1 to 3) substituents that may be same or different from each other.

As used herein, the terms "cyclohydrocarbyl", "hydrocarbyl ring" and "cyclohydrocarbylene" refer to monocyclic or multicyclic fused hydrocarbon ring containing such as 3-10 (suitably 3-8, and more suitably 3-7, 3-6, 4-6 or 5-6) cyclic carbon atoms that may be saturated (i.e., "cycloalkyl" and "cycloalkylene") or partially unsaturated (i.e., having one or more double bonds (i.e., "cycloalkenyl" and "cycloalkenylene") and/or triple bonds within the ring), including, but not limited to, cyclopropyl(ene) ring, cyclobutyl(ene) ring, cyclopentyl(ene) ring, cyclohexyl(ene) ring, cycloheptyl(ene) ring, cyclooctyl(ene) ring, cyclononyl(ene) ring, cyclobutenyl(ene) ring, cyclopentenyl(ene) ring, cyclohexenenyl(ene) ring, cycloheptenyl(ene) ring, cyclooctenyl(ene) ring, cyclononenyl(ene) ring, etc. In some embodiments, cyclohydrocarbyl includes cyclohydrocarbyl fused with aryl group, as long as the whole ring system is nonaromatic.

As used herein, the term "fused" refers to two or more cyclic moieties sharing two adjacent atoms with each other.

As used herein, the terms "cycloalkyl" and "cycloalkylene" refer to saturated monocyclic or multicyclic (such as, bicyclic) fused hydrocarbon rings (e.g., monocyclic ring such as cyclopropyl, cyclobutyl, cyclopentyl, cyclohexyl, cycloheptyl, cyclooctyl, cyclononyl; or bicyclic ring such as 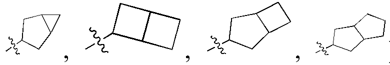 ). Cycloalkyl and cycloalkylene have from 3 to 10 carbon atoms, suitably 3-8, such as 3-7, 3-6, 4-6 or 5-6 carbon atoms. For example, the term "C₃₋₆ cycloalkyl" refers to saturated monocyclic or multicyclic (such as, bicyclic ) hydrocarbon ring having from 3 to 6 cyclic carbon atoms (e.g., cyclopropyl, cyclobutyl, cyclopentyl or cyclohexyl). Cycloalkyl and "cycloalkylene" is optionally substituted by one or more (such as, from 1 to 3) suitable substituents (e.g., methyl or halogen), such as cyclopropyl substituted with methyl.

As used herein, the terms "cycloalkenyl" and "cycloalkenylene" refer to monocyclic or multicyclic (such as, bicyclic) fused hydrocarbon ring (e.g., monocyclic moiety, e.g., cyclopropenyl, cyclobutenyl, cyclopentenyl, cyclopentadienyl, cyclohexenenyl, cyclohexadienyl, cycloheptenyl, cyclooctenyl, cyclononenyl; or bicyclic moiety) having one or more double bonds within the ring. Cycloalkenyl and "cycloalkenylene" have from 3 to 10 carbon atoms, suitably 3-8, such as 3-7, 3-6, 4-6 or 5-6. Cycloalkenyl and cycloalkenylene are optionally substituted by one or more (such as, from 1 to 3) suitable substituents, such as cyclopentenyl substituted with methyl.

As used herein, the term "heterocyclyl", "heterocycle" and "heterocyclylene" refer to monovalent or bivalent, monocyclic or polycyclic (e.g., bicyclic, tricyclic)overall non-aromatic ring structure that is saturated (i.e., "heterocyclyl" and "heterocyclylene") or partially unsaturated (e.g., having one or more double bonds within the ring (i.e., "heterocycloalkenyl", "heterocycloalkenylene" or "benzoheterocycloalkyl" and "benzoheterocycloalkylene")), having 2, 3, 4, 5, 6, 7, 8 or 9 carbon atoms and one or more (e.g., 1, 2, 3 or 4) heteroatom-containing moieties selected from O, S, S(O), S(O)₂ and NR' within the ring, wherein R' is hydrogen atom, C₁₋₆ alkyl or C₁₋₆ haloalkyl. One or more cyclic carbon atoms in the heterocyclyl may be replaced by C(O), S(O)₂, 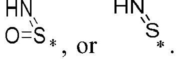 The mark "*" indicates the atom replacing the cyclic carbon atom. The heterocyclyl may be connected to the rest of the molecule through any one of the carbon atoms, or through nitrogen atom (if present). The heterocyclyl may be a fused ring, a spiro ring, a bridged ring or a combination thereof. The heterocyclyl comprising a monocyclic group is a non-aromatic ring and at least one of the fused rings comprised in a bicyclic or tricyclic group is a non-aromatic ring (e.g., benzoheterocycloalkyl, benzoheterocycloalkenyl, pyridoheterocycloalkyl, etc.). Particularly, 3-10 membered heterocyclyl refers to a moiety having 3-10 (e.g., 3-8, 3-7, 3-6, 4-6 or 5-6) atoms within the ring, including both carbon atom and heteroatom. Heterocyclyl includes nitrogen-containing heterocyclyl, oxygen-containing heterocyclyl and sulfur-containing heterocyclyl. Examples that may be enumerated include, but are not limited to oxiranyl, azacyclopropyl, azetidinyl, oxetanyl, tetrahydrofuranyl, tetrahydrothienyl, dioxolyl (dioxolinyl), pyrrolidinyl, pyrrolidinonyl, oxazolidinyl, thiazolidinyl, pyrazolidinyl, imidazolidinyl, pyrazolidinyl, pyrrolinyl, tetrahydropyranyl, piperidinyl, hexahydropyrimidinyl, triazinidinyl, morpholinyl, dithianyl, thiomorpholinyl, piperazinyl, trithianyl, azacyclooctyl, dihydropyrrolyl, dihydroimidazolyl, and azacyclooctenyl. Heterocyclyl is optionally substituted by one or more (such as, from 1 to 3) suitable substituents (e.g., halogen, OH, NH₂, C₁₋₆ alkyl, C₁₋₆ haloalkyl).

As used herein, the term "nitrogen-containing heterocyclyl" refers to saturated or unsaturated, monocyclic or bicyclic moiety, having 2, 3, 4, 5, 6, 7, 8 or 9 carbon atoms and at least one nitrogen atom within the ring and optionally including one or more (e.g., 1, 2, 3, or 4) ring members selected from N, O, C(O), S, S(O) and S(=O)₂. One or more carbon atoms in the nitrogen-containing heterocyclyl may be replaced by C(O). Nitrogen-containing heterocyclyl may be connected to the rest of the molecule through any one of the carbon atoms, or through nitrogen atom. Nitrogen-containing heterocyclyl may be a saturated nitrogen-containing monocyclic. Particularly, 3-10 membered nitrogen-containing heterocycle is a moiety having 3-10 carbon atoms and heteroatoms (at least one heteroatom is nitrogen atom) within the ring, including but not limited to 3-membered nitrogen-containing heterocycle (e.g., azacyclopropyl), 4-membered nitrogen-containing heterocycle (e.g., azetidinyl), 5-membered nitrogen-containing heterocycle (e.g., pyrrolyl, pyrrolidinyl (pyrrolidine ring), pyrrolinyl, pyrrolidinonyl, imidazolyl, imidazolidinyl, imidazolinyl, pyrazolyl, pyrazolinyl), 6-membered nitrogen-containing heterocycle (e.g., piperidinyl (piperidine ring), morpholinyl, thiomorpholinyl, piperazinyl), 7-membered nitrogen-containing heterocycle, etc.

As used herein, the term "heterocyclyl", including nitrogen-containing heterocyclyl, oxygen-containing heterocyclyl and sulfur-containing heterocyclyl, covers fused cyclic moiety, wherein the fused cyclic moiety may be connected to any other group through atom in any ring of the moiety. Therefore, the term heterocyclyl according to the present disclosure further includes but is not limited to heterocyclyl fused with heterocyclyl, heterocyclyl fused with cycloalkyl, monoheterocyclyl fused with monoheterocyclyl, monoheterocyclyl fused with monocycloalkyl, such as 3-7 membered (mono)heterocyclyl fused with 3-7 membered (mono)heterocyclyl, 3-7 membered (mono)heterocyclyl fused with (mono)cycloalkyl, 3-7 membered (mono)heterocyclyl fused with C₄₋₆ (mono)cycloalkyl, with example including but not limited to pyrrolidinocyclopropyl, cyclopentaazacyclopropyl, pyrrolidinocyclobutyl, pyrrolidinopyrrolidinyl, pyrrolidinopiperidinyl, pyrrolidinopiperazinyl, piperidomorpholinyl, 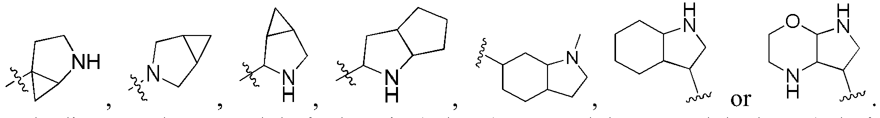 In some embodiments, heterocyclyl further includes heterocyclyl or cyclohydrocarbyl fused with heteroaryl, and heterocyclyl fused with aryl, as long as the whole ring system is nonaromatic. In some embodiments, heterocyclyl includes C₅₋₆ monocyclic cyclohydrocarbyl fused with 5-6 membered monocyclic heteroaryl, 5-6 membered monocyclic heterocyclyl fused with 5-6 membered monocyclic heteroaryl, and benzo-fused 5-6 membered monocyclic heterocyclyl, such as pyrrolotetrahydropyridinyl, pyrazolotetrahydropyridinyl, imidazolotetrahydropyridinyl, indolinyl or indolinonyl.

As used herein, the term "aryl" refers to monocyclic or fused multicyclic homocarbon moiety with conjugated π electron systems. For example, as used herein, the term "C₆₋₁₄ aryl" refers to aryl having from 6 to 14 (e.g., from 6 to 12) carbon atoms, such as phenyl or naphthyl. Aryl is optionally substituted by one or more (such as, from 1 to 3) suitable substituents (e.g., halogen, OH, CN, NO₂, C₁₋₆ alkyl, etc.).

As used herein, the term "heteroaryl" refers to monocyclic or multicyclic (e.g., bicyclic or tricyclic) aromatic ring system, having from 5 to 14 ring atoms, such as 5, 6, 7, 8, 9, 10, 11, 12, 13 or 14 ring atoms, and in particular, having 1, 2, 3, 4, 5, 6, 7, 8, 9, 10, 11, 12 or 13 carbon atoms, and 1, 2, 3, 4 or 5 heteroatoms that may be same or different from each other and independently selected from N, O, S and S(O)₂. One or more carbon atoms in the heteroaryl may be replaced by C(O). Heteroaryl may be fused with benzene ring. Examples of heteroaryl include but are not limited to: pyridinyl, pyridonyl, pyrimidinyl, pyrimidinonyl, pyrazinyl, pyridazinyl, thiazolyl, thienyl, oxazolyl, furanyl, pyrrolyl, pyrazolyl, triazolyl, tetrazolyl, isoxazolyl, isothiazolyl, imidazolyl, triazinyl, oxadiazolyl, thiadiazolyl, benzothiazolyl, benzoisothiazolyl, imidazolopyridinyl, quinolinyl, indolyl, pyrrolopyridazinyl, benzofuranyl, benzothienyl, indazolyl, benzoxazolyl, benzisoxazolyl, quinazolinyl, pyrrolopyridinyl, pyrrolopyrimidinyl, imidazolopyridazinyl, pyrrolopyridinyl, triazolopyridinyl, isoquinolinyl, tetrahydroisoquinolinyl, benzimidazolyl, cinnolinyl, indolizinyl, phthalazinyl, isoindolyl, pteridinyl, purinyl, furazanyl, benzofurazanyl, quinoxalinyl, naphthyridinyl or furanopyridinyl. Heteroaryl may be optionally substituted by one or more (e.g., 1, 2, 3 or 4) suitable substituents.

As used herein, the term "halo" or "halogen" includes F, Cl, Br or I.

As used herein, the term "haloalkyl" refers to alkyl as defined herein, which is substituted with one or more (such as, from 1 to 3) halogen atoms that may be same or different from each other. The terms "C₁₋₆ haloalkyl", "C₁₋₄ haloalkyl" and "C₁₋₃ haloalkyl" refer respectively to haloalkyl moieties having from 1 to 6 carbon atoms, from 1 to 4 carbon atoms and from 1 to 3 carbon atoms, such as -CF₃, -C₂F₅, -CHF₂, -CH₂F, -CH₂CF₃, -CH₂Cl, -CH₂CH₂CF₃, or the like.

The term "substituted" refers to the replacement of one or more (e.g., 1, 2, 3 or 4) hydrogen atoms on particular atom by indicated group(s), with the proviso that the normal atomic valence of the specified atom in the current situation is not exceeded and such substitution results in a stable compound. The combination of substituents and/or variables is only allowed when such combination results in a stable compound.

When a moiety is described as "being optionally substituted by" or "optionally substituted", the moiety may be: (1) unsubstituted, or (2) substituted. When a carbon atom in a moiety is described as being optionally substituted by one or more substituents from a substituent list, one or more hydrogen atoms on the carbon atom (to the degree of any present hydrogen) may separately and/or together be substituted by independently selected optional substituents. When a nitrogen atom in a moiety is described as being optionally substituted by one or more substituents from a substituent list, one or more hydrogen atoms on the nitrogen atom (to the degree of any present hydrogen) may separately and/or together be substituted by independently selected optional substituents. The optional substituent may be selected from: halogen, OH, SH, CN, NO₂, C₁₋₆ alkyl, C₁₋₆ haloalkyl, C₂₋₆ alkenyl, C₂₋₆ alkynyl, -O-C₁₋₆ alkyl, -O-halo C₁₋₆ alkyl, -O-C₂₋₆ alkenyl, -O-C₂₋₆ alkynyl, -S-C₁₋₆ alkyl, NH₂, -NH(C₁₋₆ alkyl), -N(C₁₋₆ alkyl)₂, -C₁₋₆ alkylene-OH, -C₁₋₆ alkylene-SH, -C₁₋₆ alkylene-CN, -C₁₋₆ alkylene-NH₂, -C₁₋₆ alkylene-NH(C₁₋₆ alkyl), -C₁₋₆ alkylene-N(C₁₋₆ alkyl)₂, -C₁₋₆ alkyl-O-C₁₋₆ alkyl, -C₀₋₆ alkylene-C(O)OH, -C₀₋₆ alkylene-C(O)OC₁₋₆ alkyl, -C₀₋₆ alkylene-C(O)NH₂, -C₀₋₆ alkylene-C(O)NH(C₁₋₆ alkyl), -C₀₋₆ alkylene-C(O)N(C₁₋₆ alkyl)₂, -C₀₋₆ alkylene-S(O)₂C₁₋₆ alkyl, -C₀₋₆ alkylene-S(O)₂NH₂, -C₀₋₆ alkylene-S(O)₂NH(C₁₋₆ alkyl), -C₀₋₆ alkylene-S(O)₂N(C₁₋₆ alkyl)₂, -NH-C(O)C₁₋₆ alkyl, -N(C₁₋₆ alkyl)-C(O)C₁₋₆ alkyl, -NH-C(O)OH, -NH-C(O)OC₁₋₆ alkyl, -N(C₁₋₆ alkyl)-C(O)OC₁₋₆ alkyl, -NH-C(O)NH₂, -NH-C(O)NH(C₁₋₆ alkyl), -NH-C(O)N(C₁₋₆ alkyl)₂, -NH-S(O)₂-C₁₋₆ alkyl, -N(C₁₋₆ alkyl)-S(O)₂-C₁₋₆ alkyl, -C₀₋₆ alkylene-C₃₋₁₀ cycloalkyl, -C₀₋₆ alkylene-(3-10 membered heterocyclyl), -C₀₋₆ alkylene-phenyl and -C₀₋₆ alkylene-(5-10 membered heteroaryl).

When substituents are described as "being independently selected from" a group of substituents, the substituents are selected independently from each other. Therefore, each substituent may be same as or different from (an)other substituent(s).

As used herein, the term "one or more" refers to one or more than one under reasonable conditions, such as 2, 3, 4, 5 or 10.

Unless otherwise specified, as used herein, the substituent may be connected through any suitable position thereon.

When a bond connected to a substituent is shown as a bond passing through a bond between two atoms of a cyclic moiety (i.e., a "suspending bond"), such substituent may be connected to any atom in the cyclic moiety, unless otherwise specified. When the suspending bond is shown as being connected to an available ring member carrying a replaceable hydrogen atom, such replaceable hydrogen atom has been actually replaced (i.e., absent).

The present disclosure further includes all pharmaceutically acceptable isotopically labeled compounds, which is otherwise same as the compound of the present disclosure except that one or more atoms have been replaced by atoms with the same atomic number but different atomic masses or mass numbers from those dominant in nature. Examples of isotopes suitable for inclusion in the compounds of the present disclosure include but are not limited to isotopes of hydrogen (e.g., deuterium (D, ²H), tritium (T, ³H)); isotopes of carbon (e.g., ¹¹C, ¹³C and ¹⁴C); isotopes of chlorine (e.g., ³⁶Cl); isotopes of fluorine (e.g., ¹⁸F); isotopes of iodine (e.g., ¹²³I and ¹²⁵I); isotopes of nitrogen (e.g., ¹³N and ¹⁵N); isotopes of oxygen (e.g., ¹⁵O, ¹⁷O and ¹⁸O); isotopes of phosphorus (e.g., ³²P); and isotopes of sulfur (e.g., ³⁵S). Some compounds of the present disclosure that are isotopically labeled (e.g., those incorporated with radioactive isotopes) can be used in drug and/or substrate tissue distribution studies (e.g., assays). Radioisotopes tritium (i.e., ³H) and carbon-14 (i.e., ¹⁴C) are particularly suitable for this purpose due to their ease of incorporation and detection. The compound substituted by positron emission isotopes (e.g., ¹¹C, ¹⁸F, ¹⁵O and ¹³N) can find use in positron emission tomography (PET) technologies for testing the occupancy of substrate receptors. The compounds of the present disclosure labeled with isotopes can be prepared by using appropriate isotope-labeled reagents instead of previously used unlabeled reagents, through methods similar to those described in the accompanying schemes and/or examples and preparations. The pharmaceutically acceptable solvates of the present disclosure include those in which the solvent forming the solvate may be isotopically substituted, such as D₂O, acetone-*d₆* or DMSO-*d₆*. In some embodiments, the isotopically labeled compound of the present disclosure is a deuterated compound.

As used herein, the term "oxo" refers to

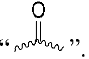

The term "stereoisomers" refers to isomers formed by the presence of at least one asymmetric center, and they are same in chemical composition but different in spatial arrangements of atoms or groups. When a compound has one or more (e.g., 1, 2, 3 or 4) asymmetric centers, it may lead to the production of racemic mixtures, single enantiomers, diastereomers mixtures, and individual diastereomers. Specific individual molecules can also exist as geometric isomers (cis/trans). Similarly, the compound of the present disclosure can exist as mixture of two or more different structural forms (commonly referred to as tautomers) in a rapid equilibrium state. Representative examples of tautomers include ketone/enol tautomers, phenol/ketone tautomers, nitroso/oxime tautomers, imine/enamine tautomers, etc. It should be understood that the scope of the present disclosure covers all such isomers or mixtures thereof in any proportion (e.g., 60%, 65%, 70%, 75%, 80%, 85%, 90%, 95%, 96%, 97%, 98%, 99%).

The term "diastereomer" refers to a stereoisomer with two or more centers of chirality and whose molecules are not mirror images of one another. Diastereomers have different physical properties, e.g., melting points, boiling points, spectral properties, and reactivities. Mixtures of diastereomers may separate under high resolution analytical procedures such as electrophoresis and chromatography.

"Enantiomers" refer to two stereoisomers of a compound which are non-superimposable mirror images of one another.

The term "chiral" refers to molecules which are non-superimposable on their mirror images, while the term "achiral" refers to molecules which are superimposable on their mirror images.

The compound of the present disclosure may be prepared in racemic form, or a single enantiomer can be produced by enantioselective synthesis or resolution.

As used herein, the term "cis-trans isomers" or "geometric isomers" arise from the inability to rotate freely of a double bond, or a single bond of cyclic carbon atoms. Compounds provided herein include all cis-, trans-, syn-, anti-, E(entgegen)- and Z(zusammen)- isomers, as well as corresponding mixtures.

Solid line ( 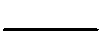 ), solid wedge ( 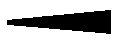 ) or dashed wedge ( 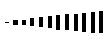 ) may be used herein for depicting the chemical bonds of the compound of the present disclosure, wherein solid line depicting the bond connected to an asymmetric carbon atom indicates the inclusion of all possible stereoisomers at the carbon atom (e.g., particular enantiomer, racemic mixture, and the like). Solid wedge or dashed wedge depicting the bond connected to an asymmetric carbon atom indicates the presence of the indicated stereoisomer. When present in racemic mixtures, solid or dashed wedge defines a relative stereochemistry rather than absolute stereochemistry. Unless otherwise specified, it is intended that the compounds of the present disclosure exist as stereoisomers (including cis and trans isomers, optical isomers (e.g., R and S enantiomers), diastereomers, geometric isomers, rotational isomers, conformational isomers, atropisomers, and their mixtures). Compounds of the present disclosure can exhibit more than one type of isomerism and may be composed of their mixtures (e.g., racemic mixtures, and diastereomers).

It should also be understood that certain compounds of the present disclosure can be used for treatment in free form, or, where appropriate, in their pharmaceutically acceptable derivative form. In the present disclosure, pharmaceutically acceptable derivatives include, but are not limited to, pharmaceutically acceptable salts, esters, solvates, metabolites, or prodrugs, which, when being administered to a subject in need thereof, can directly or indirectly provide compounds of the present disclosure or metabolites or residues thereof. Thus, when mention is made to "compounds of the present disclosure", it is intended that various derivatives of the compound stated above are included.

The term "pharmaceutically acceptable" refers to the fact that a substance or composition must be chemically and/or toxicologically compatible with other components of the formulation and/or mammals to be treated with it.

Pharmaceutically acceptable salts of compounds of the present disclosure include acid and base addition salts thereof.

Suitable acid addition salts are formed by acids that form pharmaceutically acceptable salts. Examples include aspartate, benzoate, bicarbonate/carbonate, bisulfate/sulfate, fumarate, glucoheptonate, gluconate, glucuronate, hexafluorophosphate, hydrobromide/bromide, hydroiodide/iodide, maleate, malonate, methylsulfate, naphthylate, nicotinate, nitrate, orotate, oxalate, palmitate, and other similar salts.

Suitable base addition salts are formed by bases that form pharmaceutically acceptable salts. Examples include aluminum salts, arginine salts, choline salts, diethylamine salts, lysine salts, magnesium salts, meglumine salts, potassium salts, and other similar salts.

For review of suitable salts, see Stahl and Wermuth, "Handbook of Pharmaceutical Salts: Properties, Selection, and Use" (Wiley-VCH, 2002). Methods for preparing pharmaceutically acceptable salts of compounds of the present disclosure are well known to those skilled in the art.

As used herein, the term "ester" refers to esters derived from the general formula compound in the present disclosure, including physiologically hydrolyzable esters (compounds of the present disclosure that can be hydrolyzed under physiological conditions to release free acid or alcohol form). Compounds of the present disclosure may per se are esters.

The present disclosure encompasses all possible crystalline forms or polymorphic forms of compounds of the present disclosure, which may be a single polycrystalline substance or a mixture of multiple polycrystalline substances in any ratio.

Compound of the present disclosure can exist as solvates (preferably hydrate), wherein compound of the present disclosure comprises polar solvent, such as water, methanol, or ethanol, as structural element of the lattice of the compound. Polar solvent, especially water, can exist in stoichiometric or non-stoichiometric quantities.

Those skilled in the art will understand that not all nitrogen-containing heterocycles can form N-oxides since their oxidation requires lone pair electrons on nitrogen atom available for forming oxides, and the identification of nitrogen-containing heterocycles capable of N-oxides is well known to those skilled in the art. Those skilled in the art will also recognize that tertiary amines can form N-oxides The synthesis methods for forming N-oxides of heterocycles and tertiary amines are well-known to those skilled in the art, including the oxidation of heterocycles and tertiary amines using peroxyacids such as peroxyacetic acid and m-chloroperoxybenzoic acid (MCPBA), hydrogen peroxide, alkyl hydrogen peroxide such as tert-butyl hydrogen peroxide, sodium perborate, and dioxirane such as dimethyldioxirane. These methods for preparing N-oxides have been widely described and reviewed in the literature, see such as : T. L. Gilchrist, Comprehensive Organic Synthesis, vol. 7, pp 748-750; A. R. Katritzky and A. J. Boulton, Eds., Academic Press; , and G. W. H. Cheeseman and E. S. G. Werstiuk, Advances in Heterocyclic Chemistry, vol. 22, pp 390-392, A. R. Katritzky and A. J. Boulton, Eds., Academic Press.

The present disclosure also includes metabolites of compounds of the present disclosure, i.e., substances formed in the body after the administration of compounds of the present disclosure, within its scope. Such substances may be produced from the oxidation, reduction, hydrolysis, amidation, deamidation, esterification, de-esterification, enzymatic hydrolysis, etc. of administered compound. Thus, the present disclosure includes metabolites of the compounds of the present disclosure, including the compounds resulted from contacting the compounds of the present disclosure t with mammals for sufficient time to produce its metabolites.

The present disclosure further includes prodrugs of compounds of the present disclosure, i.e., certain derivatives of compounds of the present disclosure, within its scope, which can have little or no pharmacological activity on their own but, after administered into or onto the subject, can be converted into compounds of the present disclosure with desired activity through hydrolysis and lysis. Such prodrugs are generally functional group derivatives of the compound, which can be easily converted into the desired therapeutic activity compound in vivo. Additional information on the use of prodrugs can be found in "Pro-drugs as Novel Delivery Systems", vol. 14, ACS Symposium Series (T. Higuchi and V. Stella) and in "Bioreversible Carriers in Drug Design," Pergamon Press, 1987 (E. B. Roche ed., American Pharmaceutical Association). The prodrugs of the present disclosure can be prepared, for example, by replacing appropriate functional groups present in the compounds of the present disclosure with certain parts known to those skilled in the art as a "pro-moiety" (for example, those described in "Design of Prodrugs", H. Bundgaard (Elsevier, 1985)).

The present disclosure further includes compounds of the present disclosure carrying protective groups. In any process for preparing the compound of the present disclosure, the protection of sensitive or reactive groups on any relevant molecule may be necessary and/or desirable, thereby forming chemically protected forms of the compounds of the present disclosure. This can be achieved through conventional protective groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, which are herein incorporated by reference. Such protective groups can be removed in appropriate subsequent stages using methods known in the art.

As used herein, the term "about" refers to within ± 10% of the stated value, preferably within ± 5%, more preferably within ±2%.

### Examples

Detailed description of the embodiments of the present disclosure will be described here below in conjunction with examples. However, those skilled in the art will understand that the following examples are only illustrative and should not be construed as limiting the scope of the present disclosure. In case where specific conditions are not specified in the examples, they are performed according to conventional conditions or manufacturer's recommended conditions. In cases where the manufacturer of the reagents or instruments used is not specified, they are all conventional products that are commercially available.

NMR was determined using Bruker Avance III 400 Nuclear Magnetic Resonance Spectrome, with chemical shift (δ) given in 10⁻⁶ (ppm). Solvent used was deuterated methanol (CD₃OD), deuterated chloroform (CDCl₃) or hexadeuterated dimethylsulfoxide (DMSO-d6) etc., and internal standard was tetramethyl silane (TMS).

MS was determined using Agilent (ESI) mass spectrometer (Agilent 1260, Agilent 6125B).

High performance liquid chromatography (HPLC) was determined using the following conditions: SUIMADZU HPLC (LC-2030 Plus), Waters Sunfire C18 column (3.5 µm, 4.6×100 mm), UV detection at 220 and 254 nm wavelength, eluted by 5-95% acetonitrile (containing 0.05%-0.1% v/v formic acid or trifluoroacetic acid or ammonium bicarbonate ) at a flow rate of 1.8 mL/min.

Reverse phase purification was performed using Biotage Isolera rapid purification system.

Thin layer chromatography separation and purification was performed using silica gel plate for thin layer chromatography (aluminum plate produced by Meck (20 cm x 20 cm x 1 mm), or GF 254 produced in Yantai).

Microwave reactions were carried out using Biotage Initiator + (400 W, RT ~ 300°C) microwave reactor.

Reactions were generally monitored by TLC or LCMS. Common developing solvent systems: dichloromethane/methanol, n-hexane/ethyl acetate, petroleum ether/ethyl acetate, wherein the volume ratio of the solvents was adjusted in accordance with the polarity of the compound, or by adding triethylamine.

The silica gel used for column chromatography was generally 100-200 mesh silica gel. Common eluent systems: dichloromethane/methanol, petroleum ether/ethyl acetate, wherein the volume ratio of the solvents was adjusted in accordance with the polarity of the compound, or by adding a small amount of triethylamine.

The reagents and solvents used in the present disclosure were commercially purchased from Aldrich Chemical Company, Energy Chemical, J&K Scientific, Shanghai Bidepharm Technology Co., Ltd., Pharmablock Technology Co., Ltd, and Shanghai Titan Technology Co., Ltd., among others.

In routine synthesis methods and synthesis examples of compounds and intermediates of the present disclosure, the following abbreviations are used, and their meanings are shown below.

| abbreviation | meaning |
|---|---|
| Et₃N | triethylamine |
| DMF | N,N-dimethyl formamide |
| MeOH | methanol |
| DIPEA or DIEA | N,N-diisopropylethanamine |
| Pd(dppf)Cl₂ | 1,1'-bis(diphenylphosphino)ferrocene palladium dichloride |
| Pd(dppf)Cl₂.CH₂ Cl₂ | [1,1'-bis(diphenylphosphino)ferrocene] palladium dichloride dichloromethane complex |
| NaOAc | sodium acetate |
| Pd/C | palladium/carbon |
| HATU | O-(7-azobenzotriazol-1-yl)-N,N,N',N'-tetramethylurea hexafluorophosphate |
| Boc | tertbutoxycarbonyl |
| THF | tetrahydrofuran |
| SFC | supercritical fluid chromatography |
| Ti(OiPr)₄ | tetraisopropyl titanate |
| DCM | dichloromethane |
| EA | ethyl acetate |
| OTf | trifluoromethanesulfonyloxy |
| Me | methyl |
| DMSO | dimethyl sulfoxide |
| AcONH₄ | ammonium acetate |
| EtOH | ethanol |
| NaBH(OAc)₃ | sodium triacetoxyborohydride |
| DCE | 1,2-dichloroethane |
| iPrMgCl•LiCl | isopropyl magnesium chloride lithium chloride |
| NaBH₃CN | sodium cyanoborohydride |
| HOAc | acetic acid |
| DMP | Dess-Martin periodinane |
| Boc₂O | di-tertbutyl dicarbonate |
| PPh₃ | triphenyl phosphine |
| Ti(OEt)₄ | tetraethoxy titanium |
| LC-MS | liquid chromatography-mass spectrometer |
| NMR | nuclear magnetic resonance spectroscopy |
| HPLC | high performance liquid chromatography |
| rt. | room temperature |
| sat. | saturated |
| DMAP | 4-dimethylaminopyridine |
| TMSCN | trimethylcyanosilane |
| TEA | triethylamine |
| Hr or hrs | hour(s) |
| LiBHEt₃ | lithium triethylborohydride |
| DIAD | diisopropyl azodicarboxylate |
| MsCl | methylsulfonyl chloride |
| B₂Pin₂ | bis(pinacolato)diboron |
| Pd(PPh₃)₄ | tetra(triphenylphosphine) palladium |
| XPhos Pd G3 | methanesulfonic acid (2-dicyclohexylphosphino-2',4',6'-tri-isopropyl-1,1'-biphenyl)(2'-a mino-1,1'-biphenyl-2-yl) palladium(II) |
| PtBu₃ | tritertbutylphosphine |
| Pd₂(dba)₃ | tri(di-benzylidene acetone) di-palladium |
| Pd(PPh₃)₂Cl₂ | bis(triphenylphosphine) palladium (II) dichloride |
| P(t-Bu)₃BF₄ | tri(2-methylprop-2-yl)phosphine tetrafluoro-λ5-borane |
| TCFH | N,N,N',N'-tetramethylchloroformamidinium hexafluorophosphate |
| JohnPhos | 2-(di-tertbutylphosphine)biphenyl |

### Synthesis examples

### Example 1: Preparation of compound 1

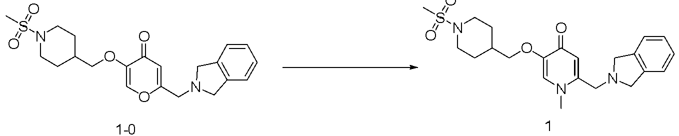

To a solution of compound 1-0 (60 mg, 0.14 mmol) and methanamine hydrochloride (97 mg, 1.43 mmol) in methanol (5 mL) was added potassium carbonate (193 mg, 0.14 mmol) at 25°C. The mixture was heated to 60°C and stirred for 16 hours. LCMS indicated the reaction was completed. Water (10 mL) was added to quench the reaction, and the mixture was extracted with ethyl acetate (10 mL×3). The combined organic phase was washed with saturated saline (20 ml), dried over anhydrous sodium sulfate, and filtered. The filtrate was concentrated under reduced pressure, and the residue was purified by preparative high-performance liquid chromatography (with ammonium bicarbonate), to obtain compound 1. LCMS: m/z 432.0 [M+H]⁺; ¹H NMR (400MHz, CDCl₃) δ: 7.12 - 7.25 (m, 4H), 7.03 (s, 1H), 6.49 (s, 1H), 3.94 (s, 4H), 3.86 - 3.83 (m, 4H), 3.81 (s, 3H), 3.75 (s, 2H), 2.79 (s, 3H), 2.64 - 2.76 (m, 2H), 1.99 - 2.12 (m, 3H), 1.36 - 1.51 (m, 2H).

### Example 2: Preparation of compound 2

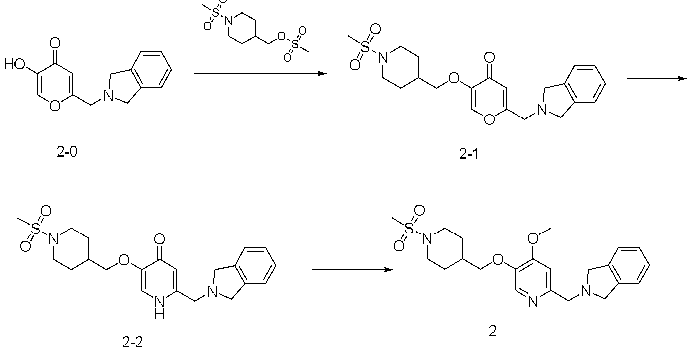

Step 1: To a solution of compound 2-0 (1.00 g, 3.29 mmol) and potassium carbonate (910 mg, 6.58 mmol) in N,N-dimethyl formamide (10 mL) was added (1-(methyl sulfonyl) piperidin-4-ylmethyl) mesylate (1.10 g, 3.95 mmol). The mixture was heated to 80°C and stirred for 16 hours. LCMS indicated the reaction was completed. The reaction was diluted with water (50 mL), and then washed with dichloromethane (50 × 3mL). The combined organic phase was dried over anhydrous sodium sulfate and filtered. The filtrate was concentrated under reduced pressure, and the residue was purified through silica gel column (petroleum ether:ethyl acetate = 2:1) to obtain Intermediate 2-1. LCMS: m/z 419.0 [M+H]⁺.

Step 2: To a solution of ammonium hydroxide (3 mL, 23.37 mmol, 28%-30%) in methanol (3 mL) was added Intermediate 2-1 (750 mg, 1.79 mmol). The mixture was heated to 80°C and stirred for 16 hours. LCMS indicated the reaction was completed. The reaction mixture was concentrated under reduced pressure, and the residue was purified through preparative thin layer chromatography silica gel plate (dichloromethane: methanol = 10:1) to obtain Intermediate 2-2. LCMS: m/z 418.0 [M+H]⁺.

Step 3: To a solution of Intermediate 2-2 (150 mg, 0.36 mmol) in methanol (1 mL) and dichloromethane (3 mL) was added (trimethylsilyl) diazomethane (82 mg, 23.37 mmol) dropwise at 25°C and the mixture was stirred for 16 hours. LCMS indicated the reaction was completed. The reaction mixture was concentrated under reduced pressure, and the residue was purified through preparative high-performance liquid chromatography (with ammonium bicarbonate), to obtain compound 2. LCMS: m/z 432.0 [M+H]⁺; ¹H NMR (400 MHz, CDCl₃) δ: 8.05 (s, 1H), 7.21 (s, 4H), 7.08 - 7.16 (m, 1H), 4.00 - 4.06 (m, 6H), 3.94 (d, *J =* 6.0 Hz, 2H), 3.91 (s, 3H), 3.85 - 3.90 (m, 2H), 2.80 (s, 3H), 2.72 (td, *J =* 12.1, 2.1 Hz, 2H), 1.92-2.09 (m, 3H), 1.53 - 1.51 (m, 2H).

### Example 3: Preparation of compound 3

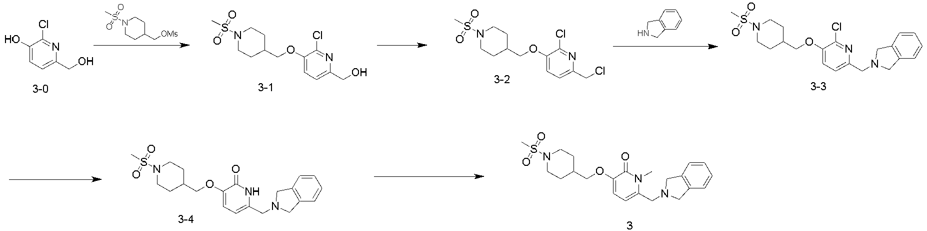

Step 1: To a solution of compound 3-0 (1.50 g, 9.40 mmol) and [1-(methyldioxo-λ6-thiolanyl) piperidin-4-yl] methyl mesylate (2.55 g, 9.40 mmol) in dimethyl sulfoxide (10.0 mL) was added potassium carbonate (2.60 g, 18.8 mmol) at 20°C. The mixture was stirred at 80°C for 5 hours. LCMS indicated the reaction was completed. The mixture was added with water (150 mL) and extracted with ethyl acetate (120 mL×2). The combined organic phase washed with saturated sodium chloride solution (120 mL), dried over anhydrous sodium sulfate, and filtered. The filtrate was concentrated under reduced pressure, and the residue was purified through silica gel column chromatography (ethyl acetate:petroleum ether = 1:5) to obtain Intermediate 3-1. LCMS: m/z 335.1 [M+H]⁺.

Step 2: To a solution of Intermediate 3-1 (1.20 g, 3.58 mmol) in toluene (20.0 mL) was added thionyl chloride (0.39 mL, 5.38 mmol) dropwise at 0°C. The mixture was stirred at 20°C for 2 hours. LCMS indicated the reaction was completed. The reaction was quenched by adding saturated sodium bicarbonate solution (50 mL) and the mixture was extracted with ethyl acetate (80 mL×2). The combined organic phase was washed with saturated sodium chloride solution (50 mL), dried over anhydrous sodium sulfate, and filtered. The filtrate was concentrated under reduced pressure, and the residue was purified through silica gel column chromatography (ethyl acetate:petroleum ether = 1:1) to obtain Intermediate 3-2. LCMS: m/z 353.0 [M+H]⁺.

Step 3: To a solution of Intermediate 3-2 (100 mg, 0.20 mmol) and 2,3-dihydro-1H-isoindole (0.02 mL, 0.20 mmol) in dimethyl formamide (2.00 mL) was added potassium carbonate (82.2 mg, 0.59 mmol) at 25°C. The mixture was stirred at 100°C for 2 hours. LCMS indicated the reaction was completed. The mixture was added with water (30 mL) and extracted with ethyl acetate (20 mL×2). The combined organic phase was washed with saturated sodium chloride solution (20 mL), dried over anhydrous sodium sulfate, and filtered. The filtrate was concentrated under reduced pressure and purified through silica gel column chromatography (ethyl acetate:petroleum ether = 1:1) to obtain Intermediate 3-3. LCMS: m/z 436.2 [M+H]⁺.

Step 4: To a solution of Intermediate 3-3 (400 mg, 0.92mmol) in dimethyl sulfoxide (0.50 mL) was added potassium hydroxide solution (2.00 mL, 10 mol/L) at 25°C. The mixture was stirred at 130°C for 36 hours, and then purified through preparative high-performance liquid chromatography to obtain Intermediate 3-4. LCMS: m/z 418.2 [M+H]⁺.

Step 5: To a solution of Intermediate 3-4 (14 mg, 0.04 mmol) in dimethyl formamide (0.50 mL) was added sodium hydride (2mg, 0.05 mmol) at -40°C. The mixture was stirred at -40°C for 2 minutes and then added with methyl iodide (3µL, 0.04 mmol). The mixture was stirred at -40°C for 30 minutes. LCMS indicated the reaction was complete. The mixture was added with water (10 mL) and extracted with ethyl acetate (10 mL×2). The combined organic phase was washed with saturated sodium chloride solution (10 mL), dried over anhydrous sodium sulfate, and filtered. The filtrate was concentrated under reduced pressure, and the residue was purified through preparative high-performance liquid chromatography to obtain compound 3. LCMS: m/z 432.2 [M+H]⁺; ¹H NMR (400 MHz, DMSO-*d₆*): δ 7.40 - 7.35 (m, 2H), 7.34-7.29 (m, 2H), 6.64 (d, *J* = 7.6 Hz, 1H), 6.42 (d, *J* = 7.6 Hz, 1H), 4.50 (s, 4H), 4.28 (s, 2H), 3.86 (br d, *J* = 11.8 Hz, 2H), 3.80 (br d, *J =* 6.3 Hz, 2H), 3.65 (s, 3H), 2.79 (s, 3H), 2.74 - 2.66 (m, 2H), 2.03 (br d, *J* = 12.1 Hz, 3H), 1.52 - 1.36 (m, 2H).

### Example 4: Preparation of compound 4

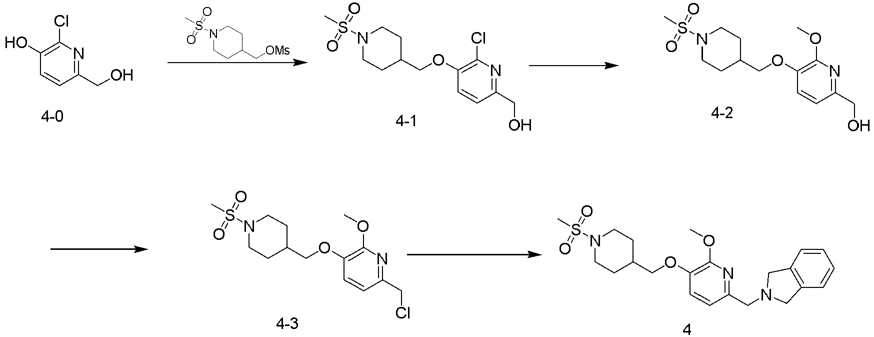

Step 1: To compound 4-0 (0.50 g, 3.13 mmol) dissolved in N,N-dimethyl formamide (20.0 mL) was added (1-(methyl sulfonyl)piperidin-4-yl) methyl mesylate (1.02 g, 3.76 mmol), followed by potassium carbonate(1.08 g, 7.83 mmol). The mixture was heated to 70°C and stirred for 12 hours. LCMS indicated the reaction was completed. The mixture was quenched by adding water (100 mL) and extracted with ethyl acetate (120 mL×2). The combined organic phase was washed with saturated sodium chloride aqueous solution (120 mL), dried over anhydrous sodium sulfate, and filtered. The filtrate was concentrated under reduced pressure to obtain Intermediate 4-1. LCMS: m/z 335.0[M+H]⁺.

Step 2: To a mixture of Intermediate 4-1 (0.50 g, 0.30 mmol) in methanol (5 ml) was added sodium methoxide (167 µL, 3.00 mmol) at 25°C. The mixture was heated to 90°C and stirred for 6 hours. LCMS indicated the reaction was completed. The mixture was quenched by adding water (10 mL) and extracted with ethyl acetate (20 mL×2). The combined organic phase was washed with saturated sodium chloride solution (20 mL), dried over anhydrous sodium sulfate, and filtered. The filtrate was concentrated under reduced pressure, and the residue was purified through silica gel column chromatography (ethyl acetate:petroleum ether = 1:4) to obtain Intermediate 4-2. LCMS: m/z 331.1[M+H]⁺.

Step 3: To a mixture of Intermediate 4-2 (200 mg, 0.61 mmol) in dichloromethane (10 ml) was slowly added thionyl chloride (0.44 mL, 6.05 mmol) dropwise at 25°C, and the mixture was stirred for 12 hours. LCMS indicated the reaction was completed. The mixture was quenched by adding water (100 mL) and extracted with ethyl acetate (120 mL×2). The combined organic phase was washed with saturated sodium chloride solution (120 mL), dried over anhydrous sodium sulfate, and filtered. The filtrate was concentrated under reduced pressure to obtain Intermediate 4-3. LCMS: m/z 349.1[M+H]⁺.

Step 4: To a solution of Intermediate 4-3 (130 mg, 0.37 mmol) and 2,3-dihydro-1H-isoindole hydrochloride (64 mg, 0.41 mmol) in acetonitrile (2 ml) was added triethylamine (258 µL, 1.86 mmol) dropwise at 25°C and the mixture was stirred for 3 hours. LCMS indicated the reaction was completed. The mixture was quenched by adding water (20 mL) and extracted with ethyl acetate (60 mL×2). The combined organic phase was washed with saturated sodium chloride solution (40 mL), dried over anhydrous sodium sulfate, and filtered. The filtrate was concentrated under reduced pressure, and the residue was purified through preparative high-performance liquid chromatography to obtain compound 4. LCMS: m/z 432.2[M+H]⁺; ¹H NMR (400 MHz, DMSO-*d₆*) δ: 7.27 (d, *J* = 7.9 Hz, 1H), 7.15 - 7.24 (m, 4H), 6.96 (d, *J* = 7.9 Hz, 1H), 3.92 (s, 4H), 3.84 - 3.89 (m, 5H), 3.83 (s, 2H), 3.59 (d, *J=* 11.8 Hz, 2H), 2.85 (s, 3H), 2.69 - 2.77 (m, 2H), 1.86 (d, *J =* 10.5 Hz, 3H), 1.28 - 1.41 (m, 2H).

### Example 5: Preparation of compound 5 and compound 6

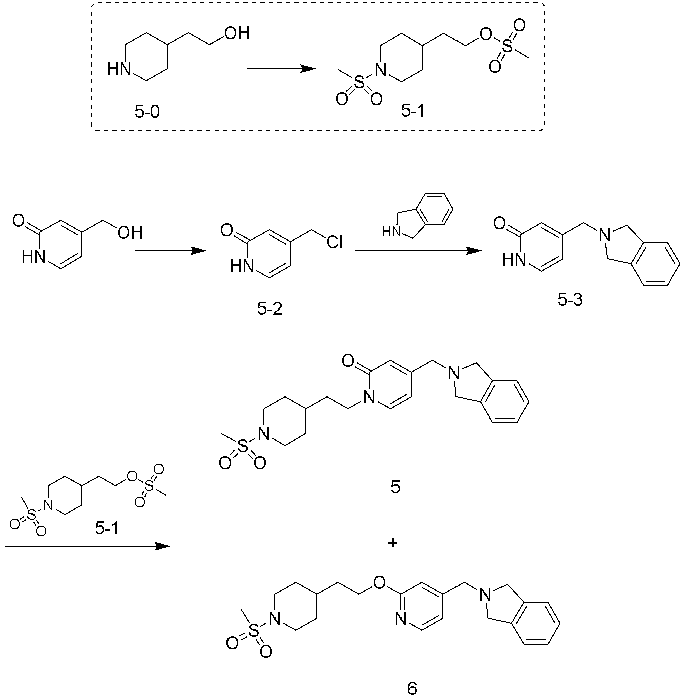

Step 1: To acetonitrile (30.0 mL) were added compound 5-0 (2.00 g, 15.48 mmol) and potassium carbonate (10.70 g, 77.40 mmol) at 25°C, followed by adding methanesulfonyl chloride (5.99 mL, 77.40mmol) dropwise, and the mixture was continuously stirred at 25°C for 12 hours. The reaction mixture was poured into water (50.0 mL) and extracted with ethyl acetate (200.0 mL). After dried over anhydrous sodium sulfate and filtered. the filtrate was concentrated under reduced pressure to obtain Intermediate 5-1. LCMS: m/z 286.1 [M+H]⁺; ¹H NMR (400 MHz, DMSO-*d₆*): δ 4.25 (t, 2H, *J* = 6.4 Hz), 3.5-3.6 (m, 2H), 3.1-3.2 (m, 3H), 2.83 (s, 3H), 2.6-2.7 (m, 2H), 1.7-1.8 (m, 2H), 1.6-1.7 (m, 2H), 1.4-1.6 (m, 1H), 1.1 - 1.3 (m, 2H).

Step 2: To a solution of 4-hydroxymethylpyridine-2(1H)-one (900 mg, 7.19 mmol) in dichloromethane (9.0 mL) was added thionyl chloride (1.30 mL, 17.98mmol) dropwise at 25°C. The mixture was heated to 50°C and stirred for 1 hour. LCMS indicated the reaction was completed. The reaction mixture was concentrated under reduced pressure to obtain Intermediate 5-2. LCMS: m/z 144.0/146.0 [M+H]⁺.

Step 3: To a solution of N,N-dimethyl formamide (3.0 mL) were added Intermediate 5-2 (200 mg, 1.39 mmol) and isoindoline (200 mg, 1.67mmol) and potassium carbonate (770 mg, 5.57 mmol) at 25°C. The mixture was heated to 80°C and stirred for 3 hours. LCMS indicated the reaction was completed. The reaction mixture was filtered to obtain a solution of Intermediate 5-3 in N,N-dimethyl formamide. LCMS: m/z 227.1 [M+H]⁺.

Step 4: To a solution of Intermediate 5-3 in N,N-dimethyl formamide were added 2-(1-(methyl sulfonyl)piperidin-4-yl)ethyl mesylate (300 mg, 1.06mmol) and potassium carbonate (364 mg, 2.64 mmol) at 25°C. The mixture was heated to 85°C and stirred for 2 hours. LCMS indicated the reaction was completed. The reaction mixture was filtered. The filtrate was concentrated under reduced pressure, and the residue was purified through reverse-phase preparative chromatography to obtain compound 5. LCMS: m/z 416.2 [M+H]⁺. ¹H NMR (400 MHz, DMSO-*d₆*): δ 7.64 (d, 1H, *J= 7.0* Hz), 7.1-7.3 (m, 4H), 6.34 (s, 1H), 6.25 (dd, 1H, *J=* 1.6, 7.0 Hz), 3.90 -3.86 (m, 6H), 3.69 (s, 2H), 3.52 (d, 2H, *J* = 11.9 Hz), 2.83 (s, 3H), 2.6-2.7 (m, 2H), 1.81 (d, 2H, *J=* 10.9 Hz), 1.59 (d, 2H, *J=* 7.6 Hz), 1.3-1.4 (m, 1H), 1.21 (s, 2H).

Compound 6 (65 mg). LCMS: m/z 416.2 [M+H]⁺. ¹H NMR (400 MHz, DMSO-*d₆*): δ 8.10 (d, *J* = 5.1 Hz, 1H), 7.1-7.3 (m, 4H), 6.98 (d, *J=* 5.1 Hz, 1H), 6.77 (s, 1H), 4.30 (t, *J* = 6.5 Hz, 2H), 3.8-3.9 (m, 6H), 3.53 (d, *J =* 11.8 Hz, 2H), 2.83 (s, 3H), 2.67 (dt, 2H, J=1.9, 11.9 Hz), 1.80 (d, *J = 11.5* Hz, 2H), 1.68 (q, *J =* 6.5 Hz, 2H), 1.57 (dd, *J =* 3.8, 6.8, 10.5 Hz, 1H), 1.23 (d, *J* = 3.9, 12.2 Hz, 2H).

### Example 6: Preparation of compound 7

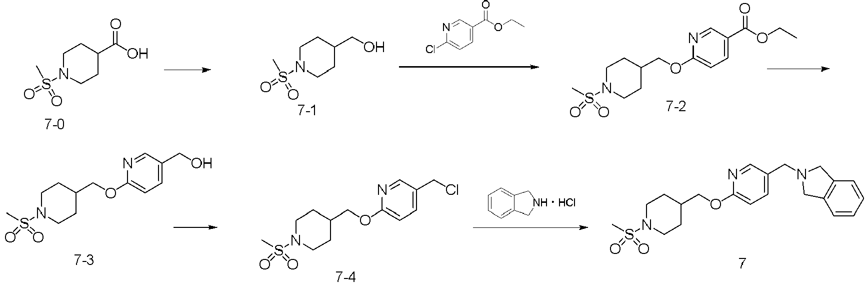

Step 1: To a solution of compound 7-0 (2.01 g, 9.65 mmol) in tetrahydrofuran (20 mL) was added a solution of lithium aluminum hydride (11.6 mL, 28.95 mmol, 2.5 M) in toluene at 0°C and the mixture was stirred for 3 hours. LCMS indicated the reaction was completed. The reaction mixture was added with ethyl acetate (10 mL), adjusted to pH 2 with aqueous hydrochloride (8 mL, 5 M) and extracted with ethyl acetate (25 mL× 3). The organic phases were combined and washed with saturated saline solution (30 mL), dried over anhydrous sodium sulfate, and filtered. The filtrate was concentrated under reduced pressure to obtain Intermediate 7-1. LCMS: m/z 194.0 [M+H]⁺.

Step 2: To a solution of Intermediate 7-1 (500 mg, 2.59 mmol) in tetrahydrofuran (10 mL) was added sodium hydride (310 mg, 7.76 mmol) at 0°C, and the mixture was stirred for 30 minutes, and then added with Ethyl 6-chloronicotinate (577 mg, 3.10 mmol) and stirred for 16 hours. LCMS indicated the reaction was completed. The reaction mixture was added with methanol (5 mL), concentrated under reduced pressure to obtain Intermediate 7-2. LCMS: m/z 343.0 [M+H]⁺.

Step 3: To Intermediate 7-2(400 mg, 1.17 mmol) dissolved into toluene (5mL) was slowly added a solution of diisobutyl aluminum hydride (2 mL, 2.92 mmol, 1.5 M) in toluene dropwise at 0°C and under nitrogen atmosphere, and the mixture was stirred for 16 hours. LCMS indicated the reaction was completed. Methanol (5 mL) was added to quench the reaction, after being further stirred for 30 minutes, potassium sodium tartrate solution (5 mL, 4 M) was added and the mixture was stirred for 2 hours. The mixture was extracted with dichloromethane (20 × 2 mL). The combined organic phase was washed with saline solution (20 mL), dried over anhydrous sodium sulfate, and filtered. The filtrate was concentrated under reduced pressure, and the residue was purified through preparative thin layer chromatography silica gel plate (dichloromethane:methanol = 10:1) to obtain Intermediate 7-3. LCMS: m/z 301.0 [M+H]⁺.

Step 4: To a solution of Intermediate 7-3 (100 mg, 0.33 mmol) in dichloromethane (3 mL) was added thionyl chloride (315 mg, 2.64 mmol) dropwise at 25°C, and the mixture was stirred for 2 hours. LCMS indicated the reaction was completed. The mxiture was concentrated under reduced pressure to obtain Intermediate 7-4. LCMS: m/z 319.0 [M+H]⁺.

Step 5: To a solution of Intermediate 7-4 (100 mg, 0.31 mmol) and diisopropylethanamine (203 mg, 1.57 mmol) in acetonitrile (5 mL) was added 1H-isoindole hydrochloride (73 mg, 0.47 mmol) at 25°C and the mixture was stirred for 16 hours. LCMS indicated the reaction was completed. The mixture was concentrated under reduced pressure, and the residue was purified through preparative high-performance liquid chromatography (with ammonium bicarbonate) to obtain compound 7. LCMS: m/z 402.0 [M+H]⁺; ¹H NMR (400 MHz, CDCl₃) δ: 8.09 - 8.14 (m, 1H), 7.71 (dd, *J* = 8.5, 2.0 Hz, 1H), 7.19 (s, 4H), 6.74 (d, *J* = 8.4 Hz, 1H), 4.20 (d, *J=* 6.0 Hz, 2H), 3.94 (s, 4H), 3.88 (br d, *J* = 2.5 Hz, 4H), 2.80 (s, 3H), 2.72 (td, *J=* 12.0, 2.1 Hz, 2H), 1.94 - 2.00 (m, 3H),1.51 (br dd, *J=* 12.1, 2.6 Hz, 2H).

### Example 7: Preparation of compound 8

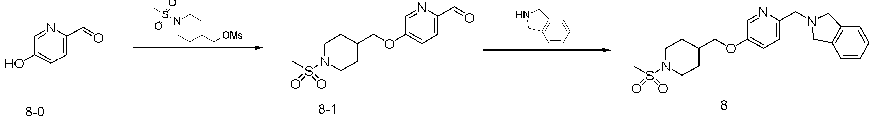

Step 1: To a solution of compound 8-0 (700 mg, 5.69 mmol) and potassium carbonate (1.57 g, 11.37 mmol) in N,N-dimethyl formamide (3.00 mL) was added methyl (1-(methyl sulfonyl)piperidin-4-yl) mesylate (1.08 g, 3.98 mmol) at 25°C. The mixture was heated to 100°C and stirred for 1 hour. The reaction mixture was added with water (15 mL) and extracted with ethyl acetate (10 mL×3). The combined organic phase was washed with saturated saline solution (10 mL), dried over anhydrous sodium sulfate, and filtered. The filtrate was concentrated under reduced pressure to obtain Intermediate 8-1. LCMS: m/z 299.0 [M+H]⁺.

Step 2: To a solution of Intermediate 8-1 (300 mg, crude) and isoindoline (462 mg, 3.02 mmol), and acetic acid (0.5 mL) in 1,2-dichloroethane (5 mL) was added sodium triacetoxyhydroborate (530 mg, 2.52 mmol) at 25°C and the mixture was stirred for 2 hours. The reaction mixture was added with saturated sodium bicarbonate aqueous solution (15 mL) and extracted with ethyl acetate (15 mL×3). The combined organic phase was (30 mL) washed with saturated saline solution, dried over anhydrous sodium sulfate, and filtered. The filtrate was concentrated, and the residue was purified through preparative high-performance liquid chromatography to obtain compound 8. LCMS: m/z 402.1 [M+H]⁺; ¹H NMR (400 MHz, CDCl₃) δ: 8.26 (d, *J= 2.5* Hz, 1H), 7.42 (d, *J* = 8.5 Hz, 1H), 7.24 - 7.08 (m, 5H), 4.10 - 3.96 (m, 6H), 3.89 (br d, *J =* 5.9 Hz, 4H), 2.84 - 2.74 (m, 3H), 2.75 - 2.64 (m, 2H), 2.00 - 1.91 (m, 3H), 1.59 - 1.41 (m, 2H).

### Example 8: Preparation of compound 9

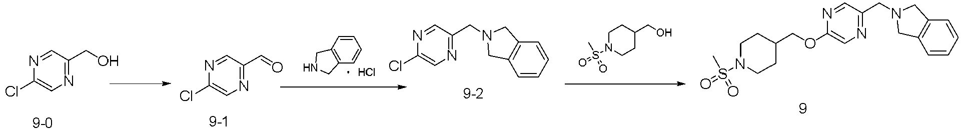

Step 1: Into dichloromethane (20 mL) were dissolved 5-chloropyrazin-2-methanol (1.00 g, 6.92 mmol) and manganese dioxide (6.02 g, 69.2 mmol) at 50°C and the mixture was stirred for 16 hours. The reaction was monitored by LCMS. The reaction mixture was filtered, and the filtrate was concentrated under reduced pressure to obtain Intermediate 9-1. LCMS: m/z 143.0[M+H]⁺.

Step 2: Into 1,2-dichloroethane (20 mL) were dissolved Intermediate 9-1 (700 mg, 4.91 mmol), 1H-isoindole hydrochloride(1.14 g, 7.37 mmol) and sodium borohydride acetate (3.11 g, 14.73 mmol), and acetic acid (0.28 ml, 4.91 mmol) at 25°C, and the mixture was stirred for 16 hours. LCMS indicated the reaction was completed. The reaction mixture was extracted with dichloromethane (20 ml x 3). The combined organic phase was washed with saturated saline solution (60 mL), dried over anhydrous sodium sulfate, and filtered. The filtrate was concentrated under reduced pressure, and the residue was purified through silica gel column chromatography (ethyl acetate:petroleum ether = 3:1) to obtain Intermediate 9-2. LCMS: m/z 246.1[M+H]⁺.

Step 3: 1-(methyl sulfonyl)piperidin-4-methanol (235 mg, 1.22 mmol) and sodium hydride (26 mg, 1.47 mmol) were dissolved in N,N-dimethylacetamide (5 mL) at 0°C and the mixture was stirred for 30 minutes, to which was added Intermediate 9-2 (200 mg, 0.81 mmol) and the mixture was stirred for 16 hours. LCMS indicated the reaction was completed. The reaction mixture was extracted with dichloromethane (20 mL x 3). The combined organic phase was washed with saturated saline solution (60 mL), dried over anhydrous sodium sulfate, and filtered. Solvent was removed by rotary evaporation under reduced pressure, and the residue was purified through preparative high-performance liquid chromatography (with ammonium bicarbonate) to obtain compound 9. LCMS: m/z 403.2[M+H]⁺; ¹H NMR (400MHz, CDCl₃) δ 8.25(s, 1H), 8.21(d, *J* = 1.3 Hz, 1H), 7.18 - 7.25(m, 4H), 4.23(d, J = 6.0 Hz, 2H), 4.14 - 4.10(br s, 6H), 3.88(br d, *J= 11.9* Hz, 2H), 2.80(s, 3H), 2.72(td, *J =* 12.0, 2.2 Hz, 2H), 1.93 - 2.02(m, 3H), 1.24 - 1.51(m, 2H).

### Example 9: Compound 10 and compound 11

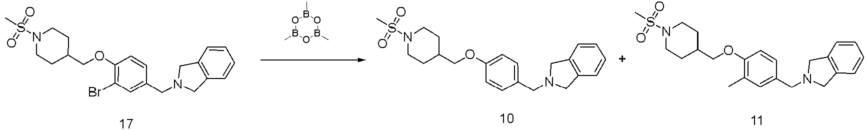

To a solution of compound 17 (350 mg, 0.7mol) and trimethylboroxine (370 mg, 2.9 mmol) in dioxane (8.0 mL) and water (1.0 mL) were added potassium carbonate (303 mg, 2.1 mmol) and [1,1'-bis(diphenylphosphino)ferrocene]palladium dichloride (55 mg, 0.07 mmol) at 25°C. After purging with nitrogen, the reaction mixture was continuously stirred at 100°C for 16 hours. LCMS indicated the reaction was completed. The reaction mixture was filtered, and the filtrate was dried by rotatory evaporation. The crude product was purified by preparative high-performance liquid chromatography to obtain compound 10. LCMS: m/z 401.1 [M+H]⁺; ¹H NMR (400 MHz, CDCl₃) δ:7.26 (d, *J=* 8.0 Hz, 2H), 7.11 (s, 4H), 6.79 (d, *J=* 8.1 Hz, 2H), 3.98 - 3.66 (m,10H), 2.73 (s, 3H), 2.64 (t, *J=* 11.6 Hz, 2H), 2.00 - 1.84 (m, 3H), 1.48 - 1.36 (m, 2H).

Compound 11. LCMS: m/z 415.1 [M+H]⁺; ¹H NMR (400 MHz, CDCl₃) δ: 7.26 - 7.13 (m, 6H), 6.77 (d, *J=* 8.0 Hz, 1H), 3.99 (s, 4H), 3.93 - 3.81 (m, 6H),2.81 (s, 3H), 2.78 - 2.69 (m, 2H), 2.23 (s, 3H), 1.99 (d, *J=* 10.5 Hz, 3H), 1.62 - 1.51 (m, 2H).

### Example 10: Compound 12

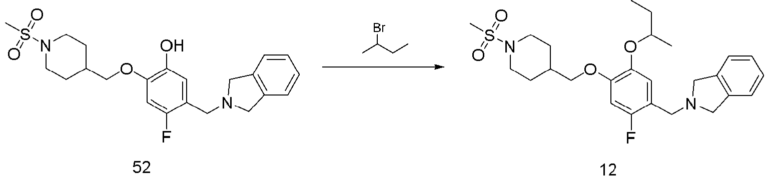

Into N,N-dimethyl formamide (2 mL) were dissolved compound 52 (120 mg, 0.28 mmol), 2-bromobutane (0.04 mL, 0.33 mmol), cesium carbonate (270 mg, 0.83 mmol) at 25°C under nitrogen atmosphere. The mixture was heated to 75°C and stirred for 2 hours. LCMS indicated the reaction was completed. The reaction mixture was diluted with water (50 mL) and extracted with ethyl acetate (30 mL×3). The combined organic phase was washed with water (30 mL×3), dried over anhydrous sodium sulfate, and filtered. The filtrate was concentrated under reduced pressure, and the residue was purified through preparative high-performance liquid chromatography (with ammonium bicarbonate) to obtain compound 12. LCMS: m/z 491.3 [M+H]⁺; ¹H NMR (400 MHz, CDCl₃) δ: 7.19 (s, 4H), 7.04 - 7.00 (m, 1H), 6.62 (d, *J* = 11.0 Hz, 1H), 4.18 (q, *J=* 6.0 Hz, 1H), 3.96 (s, 4H), 3.92 - 3.85 (m, 4H), 3.84 (d, *J=* 6.0 Hz, 2H), 2.80 (s, 3H), 2.72 (dt, *J* = 2.3, 12.0 Hz, 2H), 2.03 - 1.94 (m, 3H), 1.77 - 1.72 (m, 1H), 1.61 (ddd, *J* = 6.2, 7.3, 13.7 Hz, 1H), 1.56 - 1.44 (m, 2H), 1.25 (d, *J=* 6.1 Hz, 3H), 0.98 (t, *J=* 7.5 Hz, 3H).

### Example 11: Compound 13

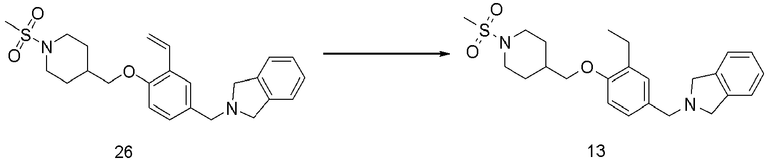

Into tetrahydrofuran (2 mL) and methanol (2 mL) were added compound 26 (100 mg, 0.23 mmol), palladium/carbon (25 mg, 0.23 mmol) at 25°C under nitrogen atmosphere. After atmosphere was replaced with hydrogen for three times, the reaction mixture was stirred under hydrogen system for 2 hours. LCMS indicated the reaction was completed. The reaction mixture was filtered. The filtrate was concentrated under reduced pressure, and the residue was purified through preparative high-performance liquid chromatography (with ammonium bicarbonate) to obtain compound 13. LCMS: m/z 429.3 [M+H]⁺; ¹H NMR (400 MHz, CDCl₃) δ: 7.22 (d, *J= 1.9* Hz, 1H), 7.20 - 7.17 (m, 5H), 6.78 (d, *J=* 8.3 Hz, 1H), 3.92 (s, 4H), 3.92 - 3.90 (m, 1H), 3.88 (br s, 1H), 3.87 (s, 1H), 3.86 (s, 1H), 3.85 (s, 2H), 2.81 (s, 3H), 2.74 (dt, *J =* 1.9, 12.0 Hz, 2H), 2.66 (q, *J=* 7.5 Hz, 2H), 2.02 - 1.92 (m, 3H), 1.63 - 1.49 (m, 2H), 1.22 (t, *J=* 7.6 Hz, 3H)

### Example 12: Compound 14

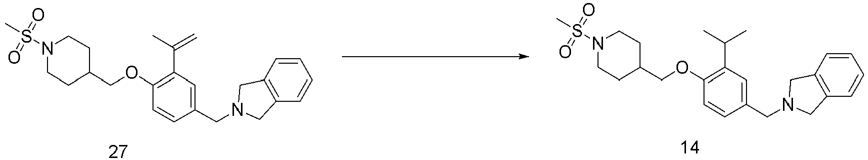

Into a solution of methanol (5 mL) was added compound 27 (100 mg, 0.23 mmol) and palladium-carbon (10 mg, 0.1 mmol) at 25°C and under nitrogen atmosphere. After atmosphere was replaced with hydrogen for three times, the reaction mixture was stirred for 8 hours. LCMS indicated the reaction was completed. The reaction mixture was filtered. The filtrate was concentrated, and the residue was purified through preparative high-performance liquid chromatography (with ammonium bicarbonate) to obtain compound 14. LCMS: m/z 443.3 [M+H]⁺; ¹H NMR (400MHz, CDCl₃) δ: 7.26 (d, *J* = 1.9 Hz, 1H), 7.19 (s, 5H), 6.79 (d, *J* = 8.3 Hz, 1H), 3.94 (s, 4H), 3.90 (br d, *J =* 12.1 Hz, 2H), 3.86 (s, 3H), 3.31 (quin, *J =* 6.9 Hz, 1H), 2.81 (s, 3H), 2.70 - 2.79 (m, 2H), 1.91 - 2.01 (m, 3H), 1.50 - 1.63 (m, 3H), 1.24 (d, *J=* 6.9 Hz, 6H).

### Example 13: Compound 15

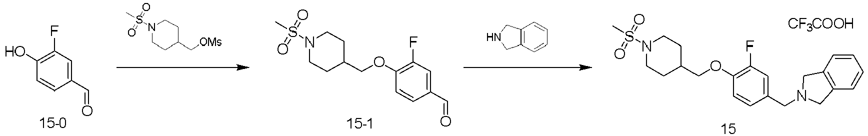

Step 1: To a mixture of Intermediate 15-0 (130 mg, 0.76 mmol) in N,N-dimethyl formamide (2.5mL) was added (1-(methyl sulfonyl)piperidin-4-yl)methyl mesylate (249 mg, 0.92 mmol) at room temperature, followed by potassium carbonate (264 mg, 1.91mmol). The mixture was heated to 70°C and stirred for 12 hours. LCMS indicated the reaction was completed. The mixture was quenched by adding water (20 mL) and extracted with ethyl acetate (60 mL×2). The combined organic phase was washed with saturated sodium chloride solution (40 mL), dried over anhydrous sodium sulfate, and filtered. The filtrate was concentrated under reduced pressure, and the residue was used for the next step without further purification to obtain Intermediate 15-1. LCMS: m/z 316.1[M+H]⁺.

Step 2: Into 1,2-dichloroethane (10 ml) were dissolved Intermediate 15-1 (200 mg, 0.63 mmol) and 2,3-dihydro-1H-isoindole hydrochloride (148 mg, 0.95 mmol) at room temperature. Into the mixture were added acetic acid (240 µL, 4.20 mmol) and sodium triacetoxyborohydride (334 mg, 1.59 mmol) and the mixture was stirred at 25°C for 3 hours. LCMS indicated the reaction was completed. The mixture was quenched by adding water (20 mL) and extracted with ethyl acetate (60 mL×2). The combined organic phase was washed with saturated sodium chloride solution (40 mL), dried over anhydrous sodium sulfate, and filtered. The filtrate was concentrated under reduced pressure, and the residue was purified through reverse-phase preparative chromatography (with trifluoroacetic acid) to obtain trifluoroacetate salt of compound 15. LCMS: m/z 419.1[M+H]⁺; ¹H NMR (400 MHz, DMSO-*d₆*) δ: 10.8 - 11.0 (m, 1H), 7.4-7.5 (m, 1H), 7.3-7.4 (m, 5H), 7.3 - 7.3 (m, 1H), 4.5 - 4.6 (m, 6H), 4.0 - 4.0 (m, 2H), 3.61 (br d, *J=* 11.8 Hz, 2H), 2.8 - 2.9 (m, 3H), 2.7 - 2.8 (m, 2H), 1.8 - 1.9 (m, 3H), 1.3 - 1.4 (m, 2H); ¹⁹ F NMR (376 MHz, DMSO-*d₆*) δ: -73.87 (s, 1F), -134.23 (s, 1F)

### Example 14: Compound 16

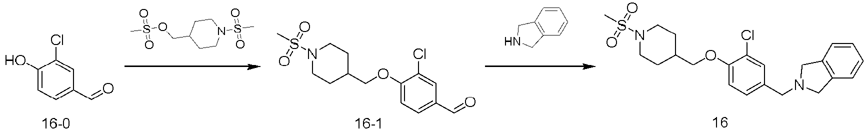

Step 1: Into a mixture of (1-(methyl sulfonyl)piperidin-4-yl)methyl mesylate (415 mg, 1.53 mmol) in N,N-dimethyl formamide (10 mL) was added starting material 16-0 at 25°C, followed by potassium carbonate (441.36 mg, 3.19 mmol). The mixture was heated to 70°C and stirred for 12 hours. LCMS indicated the reaction was completed. The mixture was quenched by adding water (20 mL) and filtered to obtain Intermediate 16-1. LCMS: m/z 332.1[M+H]⁺.

Step 2: Into 1,2-dichloroethane (3 ml) were dissolved Intermediate 16-1 (270mg, 0.81 mmol) and 1H-isoindole hydrochloride (187 mg, 1.22 mmol) at 25°C. The mixture was added with acetic acid (200 µL, 3.50 mmol) and sodium triacetoxyborohydride (429 mg, 2.03 mmol) at 25°C and the mixture was stirred for 12 hours. LCMS indicated the reaction was completed. The reaction was quenched with water (20 mL), and the mixture was extracted with ethyl acetate (60 mL×2). The combined organic phase was washed with saturated sodium chloride solution (40 mL), dried over anhydrous sodium sulfate, and filtered. The filtrate was concentrated under reduced pressure and purified through reverse-phase preparative chromatography to obtain compound 16, LCMS: m/z 435.2 [M+H]⁺; ¹H NMR (400 MHz, DMSO-*d₆*) δ: 7.41 (d, *J=* 1.9 Hz, 1H), 7.28 (m, *J* = 8.4, 1.8 Hz, 1H), 7.15 - 7.24(m, 4H), 7.12 (d, *J* = 8.4 Hz, 1H), 3.96(d, *J =* 5.9 Hz, 2H), 3.76 - 3.84 (m, 6H), 3.60 (d, *J* = 11.8 Hz, 2H), 2.86 (s, 3H), 2.75 (t, *J =* 11.1 Hz, 2H), 1.89 (d, *J =* 11.4 Hz, 3H),1.33-1.45 (m, 2H)

### Example 15: Compound 17

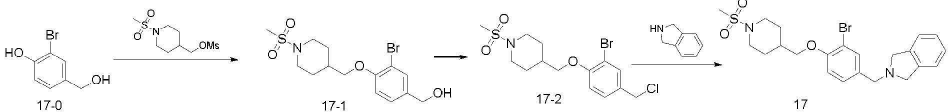

Step 1: To a solution of starting material 17-0 (2.20 g, 10.84 mmol) and [1-(methylsulfonyl)piperidin-4-yl]methyl mesylate (4.41 g, 16.25 mmol) in N,N-dimethyl formamide(50 mL) was added potassium carbonate (3.00 g, 21.67 mmol) at 25°C. The mixture was heated to 80°C and stirred for 16 hours. LCMS indicated the reaction was completed. The reaction was quenched by adding water (100 mL) and extracted with ethyl acetate (100 mL×3). The combined organic phase was washed with saturated saline solution (30 mL), dried over anhydrous sodium sulfate, and filtered. The filtrate was concentrated under reduced pressure, and the residue was purified through silica gel column (ethyl acetate:petroleum ether = 1:1), to obtain Intermediate 17-1. ¹H NMR (400 MHz, CDCl₃) δ: 7.47 (d, *J* = 1.4 Hz, 1H), 7.19 - 7.13 (m, 1H), 6.77 (d, *J* = 8.4 Hz, 1H), 4.51 (s, 2H), 3.86 - 3.73 (m, 4H), 2.72 (s, 3H), 2.65 (br t, *J* = 11.4 Hz, 2H), 1.92 (br d, *J =* 11.5 Hz, 3H), 1.44 (br dd, *J =* 2.6, 12.1 Hz, 2H).

Step 2: To a solution of Intermediate 17-1 (500 mg, 1.32 mmol) in dichloromethane (5 mL) was added thionyl chloride (0.19 mL, 2.64 mmol) dropwise at 25°C and the mixture was stirred for 30 minutes. LCMS indicated the reaction was completed. The reaction was quenched by adding saturated sodium bicarbonate solution (12 mL), and extracted with ethyl acetate (15 mL × 3). The combined organic phase was washed with saturated saline solution (20 mL), dried over anhydrous sodium sulfate, and filtered. The filtrate was concentrated under reduced pressure to obtain Intermediate 17-2. LCMS: m/z 397.9 [M+H]⁺.

Step 3: To a solution of triethylamine (4.89 mmol) and Intermediate 17-2 (4.20 g, 10.59 mmol) in acetonitrile (50 mL) was added 2,3-dihydro-1H-isoindole (0.84 g, 7.06 mmol) at 25°C, and the mixture was stirred for 18 hours. The reaction mixture was direclty concentrated, and the residue was purified through silica gel column (ethyl acetate:petroleum ether = 1:1) to obtain compound 17. LCMS: m/z 480.9 [M+H]⁺; ¹H NMR (400 MHz, CDCl₃) δ:7.62 (d, *J* = 1.8 Hz, 1H), 7.30 (dd, *J* = 1.8, 8.4 Hz, 1H), 7.19 (s, 4H), 6.85 (d, *J* = 8.3 Hz, 1H), 3.95 - 3.87 (m, 8H), 3.83 (s, 2H), 2.81 (s, 3H), 2.78 - 2.69 (m, 2H), 2.08 - 1.94 (m, 3H), 1.65 - 1.47 (m, 2H).

### Example 16: Compound 18

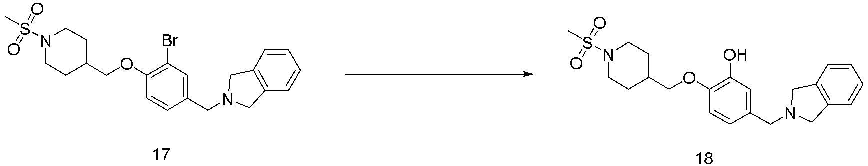

To a solution of dimethyl sulfoxide (6 mL) and water (2 mL) were added compound 17 (200 g, 0.42 mmol), N1,N2-bis(4-hydroxy-2,6-dimethylphenyl)oxalamide (7 mg, 0.02 mmol), and lithium hydroxide (36 mg, 0.88 mmol). The mixture was heated to 120°C and stirred for 18 hours. LCMS indicated the reaction was completed. The reaction mixture was direclty filtered, and the filtrate was concentrated under reduced pressure. The residue was purified through preparative high-performance liquid chromatography (with formic acid) to obtain compound 18. LCMS: m/z 417.1 [M+H]⁺; ¹H NMR (400 MHz, CDCl₃) δ:7.28 - 7.20 (m, 4H), 7.06 (br s, 1H), 6.94 (br d, *J =* 8.1 Hz, 1H), 6.83 (d, *J* = 8.3 Hz, 1H), 4.24 (s, 4H), 4.04 (s, 2H), 3.94 (d, *J* = 6.0 Hz, 2H), 3.92 - 3.86 (m, 2H), 2.81 (s, 3H), 2.76 - 2.68 (m, 2H), 2.03 -1.94 (m, 3H), 1.59 - 1.43 (m, 2H).

### Example 17: Compound 19

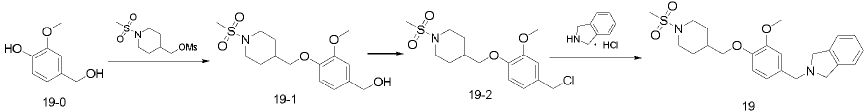

Step 1: To a solution of starting material 19-0 (213 mg, 1.38 mmol) and (1-(methyl sulfonyl)piperidin-4-yl)methyl mesylate (250 mg, 0.92 mmol) in N,N-dimethyl formamide (8 mL) was added potassium carbonate (254 mg, 1.84 mmol) at 25°C. The mixture was heated to 80°C and stirred for 16 hours. LCMS indicated the reaction was completed. The reaction was quenched by adding water (20 mL) and the mixture was extracted with ethyl acetate (20 mL×3). The combined organic phase was washed with saturated saline solution (40 mL), dried over anhydrous sodium sulfate, and filtered. The filtrate was concentrated under reduced pressure, and the residue was purified through silica gel column chromatography (ethyl acetate:petroleum ether=1:3) to obtain Intermediate 19-1. LCMS: m/z 330.1[M+H]⁺.

Step 2: To a solution of Intermediate 19-1 (190 mg, 0.58 mmol) in dichloromethane (10 mL) was added thionyl chloride (0.62 mL, 8.50 mmol) dropwise at 25°C, and the mixture was stirred for 6 hours. LCMS indicated the reaction was completed. The reaction mixture was concentrated under reduced pressure to obtain Intermediate 19-2. LCMS: m/z 348.1[M+H]⁺.

Step 3: To a solution of 2,3-dihydroisoindole hydrochloride (127 mg, 0.83 mmol) and potassium carbonate (457 mg, 3.3 mmol) in acetonitrile (10 mL) was added Intermediate 19-2 (190 mg, 0.55 mmol) at 25°C, and the mixture was stirred for 16 hours. LCMS indicated the reaction was completed. The reaction mixture was added with water (15 mL) and extracted with ethyl acetate (15 mL×3). The combined organic phase was washed with saturated saline solution (30 mL), dried over anhydrous sodium sulfate, and filtered. The filtrate was concentrated under reduced pressure, and the residue was purified through preparative high-performance liquid chromatography (with ammonium bicarbonate) to obtain compound 19. LCMS: m/z 431.2[M+H]⁺; ¹H NMR (400 MHz, DMSO-*d₆*) δ: 7.19 (br d, *J =* 9.3 Hz, 4H), 6.94 - 6.98 (m, 1H), 6.90 (s, 1H), 6.83 - 6.88 (m, 1H), 3.84 - 3.77 (m, 8H),3.75 (s, 3H), 3.54 - 3.63 (m, 2H), 2.85 (s, 3H), 2.67 - 2.78 (m, 2H), 1.81 - 1.92 (m, 3H), 1.27-1.42 (m, 2H).

### Example 18: Compound 20

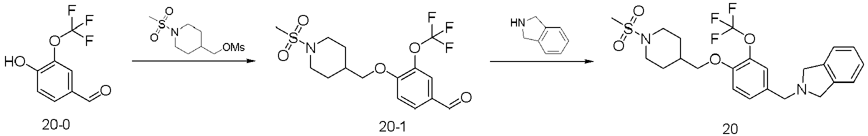

Step 1: To a solution of starting material 20-0 (700 mg, 3.40 mmol) and potassium carbonate (938 mg, 6.79 mmol) in N,N-dimethyl formamide (3.00 mL) was added methyl (1-(methyl sulfonyl)piperidin-4-yl)mesylate (645 mg, 2.38 mmol) at 25°C. The mixture was heated to 100°C and stirred for 1 hour. LCMS indicated the reaction was completed. The reaction mixture was added with water (15 mL) and extracted with ethyl acetate (10 mL×3). The combined organic phase was washed with saturated saline (20 ml), dried over anhydrous sodium sulfate, and filtered. The filtrate was concentrated under reduced pressure to obtain Intermediate 20-1. LCMS: m/z 382.1 [M+H]⁺.

Step 2: To a solution of Intermediate 20-1 (300 mg), isoindoline (362 mg, 2.36 mmol), acetic acid (0.5 mL) in 1,2-dichloroethane (5 mL) was added sodium triacetoxyhydroborate (414 mg, 1.97 mmol) at 25°C, and the mixture was stirred for 2 hours. The reaction mixture was added with saturated sodium bicarbonate aqueous solution (15 mL) and extracted with ethyl acetate (15 mL×3). The combined organic phase was washed with saturated saline solution (30 mL), dried over anhydrous sodium sulfate, and filtered. The filtrate was concentrated under reduced pressure, and the residue was purified through preparative high-performance liquid chromatography to obtain compound 20. LCMS: m/z 485.1 [M+H]⁺; ¹H NMR (400 MHz, CDCl₃) δ: 7.38 - 7.29 (m, 2H), 7.20 (s, 4H), 6.94 (d, *J =* 8.3 Hz, 1H), 3.93 (s, 3H), 3.91 - 3.85 (m, 7H), 2.81 (s, 3H), 2.75 (dt, *J=* 1.8, 12.0 Hz, 2H), 2.06 - 1.91 (m, 3H), 1.59 - 1.45 (m, 2H).

### Example 19: Compound 21

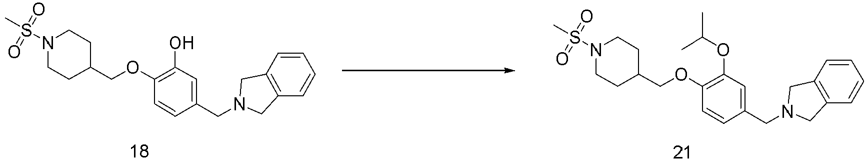

To a solution of compound 18 (35 mg, 0.08 mmol), triphenylphosphine (42 mg, 0.16 mmol) and isopropanol (24, 0.40 mmol) in dichloromethane (1 mL) was added diisopropyl azodicarboxylate (32 mg, 0.16 mmol) dropwise at 25°C, and the mixture was stirred for 18 hours. LCMS indicated the reaction was completed. The reaction mixture was directly concentrated, and the residue was purified through preparative high-performance liquid chromatography (with ammonium bicarbonate) to obtain compound 21. LCMS: m/z 459.3 [M+H]⁺; ¹H NMR (400 MHz, CDCl₃) δ:7.19 (s, 4H), 7.08 - 7.02 (m, 1H), 6.96 - 6.91 (m, 1H), 6.90 - 6.82 (m, 1H), 4.57 - 4.38 (m, 1H), 3.94 (s, 4H), 3.91 - 3.83 (m, 6H), 2.80 (s, 3H), 2.73 (br d, *J =* 1.9 Hz, 2H), 2.06 - 1.97 (m, 3H), 1.56 - 1.48 (m, 2H), 1.34 (s, 3H), 1.33 (s, 3H)

### Example 20: Compound 22

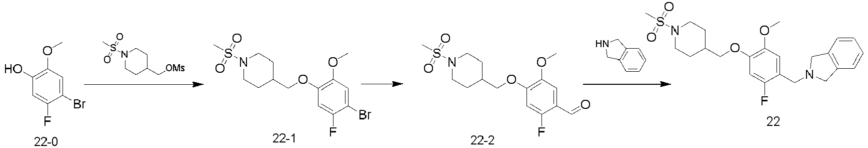

Step 1: To a solution of starting material 22-0 (1.00 g, 4.52mmo) and [1-(methyldioxo-λ6-thiolanyl)piperidin-4-yl ] methyl mesylate (1.47 g, 5.43 mmol) in N,N-dimethyl formamide (40 mL) was added potassium carbonate (1.56 g, 11.31 mmol) at 25°C. The mixture was heated to 70°C and stirred for 12 hours. LCMS indicated the reaction was completed. The mixture was added with water (150 mL) and extracted with ethyl acetate (120 mL×2). The combined organic phase was washed with saturated saline solution (120 mL), dried over anhydrous sodium sulfate, and filtered. The filtrate was concentrated under reduced pressure, and the residue was purified through silica gel column chromatography (ethyl acetate:petroleum ether = 1:4) to obtain Intermediate 22-1. LCMS: m/z 396.0 [M+H]⁺.

Step 2: Into N,N-dimethyl formamide (2 mL) were dissolved Intermediate 22-1 (100mg, 0.25 mmol), bis[5-(diphenylphosphino)cyclopenta-1,3-dienyl]-λ2-iron (II) dichloromethane palladium chloride (10.21 mg, 0.01 mmol) and triethylsilane (161 µL, 1.00 mmol) at 25°C, followed by addition of triethylamine (173µL, 1.25 mmol). The mixture was heated to 90°C and stirred for 6 hours. LCMS indicated the reaction was completed. The reaction was quenched with water (10 mL), and the mixture was extracted with ethyl acetate (30 mL×2). The combined organic phase was washed with saturated sodium chloride solution (30 mL), dried over anhydrous sodium sulfate, and filtered. The filtrate was concentrated under reduced pressure, and the residue was purified through silica gel column chromatography (ethyl acetate:petroleum ether = 7:3) to obtain Intermediate 22-2. LCMS: m/z 346.0 [M+H]⁺.

Step 3: To a mixture of Intermediate 22-2 (40 mg, 0.12 mmol) and isoindoline hydrochloride (27 mg, 0.17 mmol) were added acetic acid (100 µL, 1.75 mmol) and sodium triacetoxyborohydride (61 mg, 0.29 mmol) at 25°C, and the mixture was stirred for 12 hours. LCMS indicated the reaction was completed. The mixture was added with water (10 mL) and extracted with ethyl acetate (10 mL×2). The combined organic phase was washed with saturated sodium chloride solution (10 mL), dried over anhydrous sodium sulfate, and filtered. The filtrate was concentrated under reduced pressure, and the residue was purified through preparative high-performance liquid chromatography to obtain compound 22. LCMS: m/z 449.5 [M+H]⁺; ¹H NMR (400 MHz, DMSO-*d₆*) δ: 7.15 - 7.24 (m, 4H), 7.00 (d, *J* = 7.3 Hz, 1H), 6.89 (d, *J* = 11.5 Hz, 1H), 3.85 - 3.89 (m, 2H), 3.84 (m, 4H), 3.81 (s, 2H), 3.74 (s, 3H), 3.58 (d, *J =* 11.6 Hz, 2H), 2.85 (s, 3H), 2.68 - 2.78 (m, 2H), 1.80 - 1.93 (m, 3H), 1.29 - 1.39 (m, 2H); ⁹ F NMR (DMSO-*d*_{*6*)} δ: -128.05--124.13 (m, 1F).

### Example 21: Compound 23

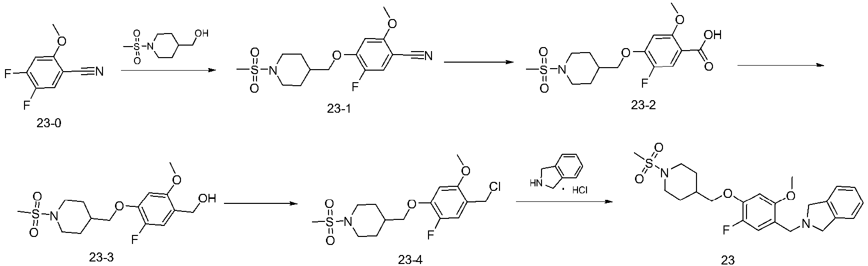

Step 1: A mixture of 1-(methyl sulfonyl)piperidin-4-methanol (1.01 g, 5.68 mmol) and sodium hydride (568 mg, 14.19 mmol) in N,N-dimethyl formamide (5 mL) was stirred at 0°C for 30 minutes, added with starting material 23-0 (800 mg, 4.73 mmol) and the mixture was stirred for 16 hours. The reaction was monitored by LCMS. To the reaction mixture was slowly added methanol (10 mL) dropwise at 0°C and the reaction mixture was stirred for 30 minutes, then extracted with dichloromethane (25 mL x 3). The organic phase was washed with saturated sodium chloride solution (100 mL) and combined, dried over anhydrous sodium sulfate, and filtered. The filtrate was concentrated under reduced pressure to obtain Intermediate 23-1. LCMS: m/z 343.1 [M+H]⁺.

Step 2: A solution of Intermediate 23-1 (800 mg, 5.68 mmol) and potassium hydroxide (10 mL, 6 M) in ethanol (30 mL) was stirred at 70°C for 16 hours. The reaction was monitored by LCMS. To the reaction mixture was slowly added aqueous hydrochloride (10 mL, 6 M) dropwise at 0°C, until the reaction mixture was acidic. The reaction mixture was extracted with dichloromethane (40 mL x 3). The organic phases were washed with saline solution (150 mL) and combined, dried over anhydrous sodium sulfate, and filtered. The filtrate was concentrated under reduced pressure to obtain Intermediate 23-2. LCMS: m/z 362.1 [M+H]⁺.

Step 3: A solution of Intermediate 23-2 (800 mg, 2.21 mmol) and borane tetrahydrofuran solution (8 mL, 1.5 M) in tetrahydrofuran (20 mL) was stirred at 60°C and under nitrogen atmosphere for 4 hours. At 25°C, the reaction mixture was slowly added with methanol (10 mL) dropwise. The mixture was heated to 40°C and stirred for 1 hour. Finally the reaction mixture was cooled to room temperature and extracted with dichloromethane (40 mLx3). The organic phase was washed with saturated sodium chloride solution (150 mL) and combined, dried over anhydrous sodium sulfate, and filtered. The filtrate was concentrated under reduced pressure to obtain Intermediate 23-3.

Step 4: A solution of Intermediate 23-3 (700 mg, 2.01 mmol) and thionyl chloride (2.0 mL, 13.68 mmol) in dichloromethane (10 mL) was stirred at 25°C for 16 hours. The reaction mixture was concentrated under reduced pressure to obtain Intermediate 23-4.

Step 5: A solution of Intermediate 23-4 (730 mg, 2.0 mmol), 2,3-dihydroisoindole hydrochloride (440 mg, 2.87 mmol), and N,N-diisopropylethanamine (1.23 g, 9.55 mmol) in acetonitrile (15 mL) was stirred at 25°C for 2 hours. LCMS indicated the reaction was completed. The reaction mixture was extracted with dichloromethane (30 mL x 3). The organic phases were combined, washed with saturated sodium chloride solution (100 mL), and concentrated under reduced pressure. The residue was purified through high-performance liquid chromatography (with ammonium bicarbonate) to obtain compound 23. LCMS: m/z 449.2 [M+H]⁺; ¹H NMR (400MHz, CDCl₃) δ 7.21 (s, 1H), 7.18 (s, 4H), 6.52 (d, *J=* 7.1 Hz, 1H), 3.97 (s, 4H), 3.92 (d, *J =* 6.1 Hz, 2H), 3.90 (br d, *J =* 1.9 Hz, 2H), 3.86 (s, 2H), 3.83 (s, 3H), 2.81 (s, 3H), 2.67 - 2.77 (m, 2H), 1.96 - 2.06 (m, 3H), 1.43 - 1.58 (m, 2H).

### Example 22: Compound 24

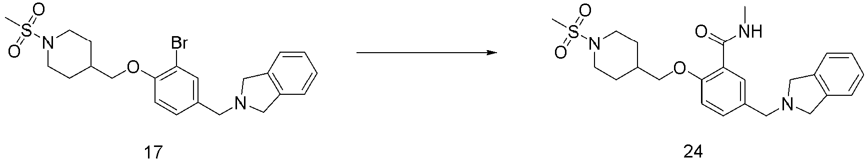

Into dimethyl sulfoxide (5 mL) were added compound 17 (200 g, 0.42 mmol), [1,1'-bis(diphenylphosphino)ferrocene]palladium dichloride (31 mg, 0.04 mmol), triethylamine (211 mg, 2.09 mmol) and methanamine hydrochloride (85 mg, 1.25 mmol). The mixture was heated to 80°C and stirred for 18 hours. LCMS indicated the reaction was completed. The reaction mixture was directly filtered. The filtrate was concentrated under reduced pressure, and the residue was purified through preparative high-performance liquid chromatography (with ammonium bicarbonate) to obtain compound 24. LCMS: m/z 458.3 [M+H]⁺; ¹H NMR (400 MHz, CDCl₃) δ:8.15 (d, *J* = 1.9 Hz, 1H), 7.68 (br d, *J= 2.9* Hz, 1H), 7.63 - 7.52 (m, 1H), 7.18 (s, 4H), 6.95 (d, *J* = 8.5 Hz, 1H), 4.04 (d, *J* = 6.0 Hz, 2H), 3.99 - 3.87 (m, 8H), 3.02 (d, *J* = 4.8 Hz, 3H), 2.82 (s, 3H), 2.80 - 2.68 (m, 2H), 2.09 - 1.87 (m, 3H), 1.56 (dq, *J =* 4.0, 12.3 Hz, 2H).

### Example 23: Compound 25

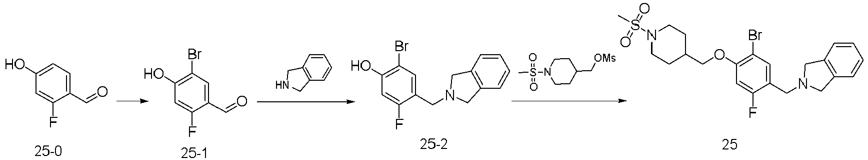

Step 1: To a solution of starting material 25-0 (1.00 g, 7.14 mmol) in acetic acid (32 mL) was added bromine (0.40 mL, 7.85 mmol) at 25°C. The mixture was heated to 45°C and stirred for 6 hours. LCMS indicated the reaction was completed. The reaction mixture was directly concentrated under reduced pressure. The residue was subjected to silica gel column chromatography and eluted (petroleum ether:ethyl acetate=5:1) to obtain Intermediate 25-1. LCMS: m/z 216.9 [M-H]⁻.

Step 2: Into 1,2-dichloroethane (10 mL) were dissolved Intermediate 25-1 (600 mg, 2.74 mmol), 2,3-dihydroisoindole hydrochloride (841 mg, 5.48 mmol), sodium triacetoxyborohydride (1.15 g, 5.48 mmol), and acetic acid (0.16 mL, 2.74 mmol) at 25°C, and the mixture was stirred for 3 hours. LCMS indicated the reaction was completed. The reaction mixture was directly concentrated under reduced pressure. The residue was diluted with water (50 mL) and extracted with ethyl acetate (50 mL×3). The combined organic phase was washed with saturated sodium bicarbonate aqueous solution (30 mL×3), dried over anhydrous sodium sulfate, and filtered. The filtrate was concentrated under reduced pressure. The residue was subjected to silica gel column chromatography and eluted (petroleum ether:ethyl acetate=5: 2) to obtain Intermediate 25-2. LCMS: m/z 322.0 [M+H]⁺.

Step 3: Into N,N-dimethyl formamide (5 mL) were dissolved Intermediate 25-2 (300 mg, 0.93 mmol), methyl (1-(methyl sulfonyl)piperidin-4-yl)mesylate (303 mg, 1.12 mmol) and potassium carbonate (386 mg, 2.79 mmol) at 25°C, and the mixture was stirred at 80°C for 18 hours. LCMS indicated the reaction was completed. The reaction was diluted with water (20 mL) and extracted with ethyl acetate (30 mL×3). The combined organic phase was washed with water (30 mL×3), dried over anhydrous sodium sulfate, and filtered. The filtrate was concentrated under reduced pressure, and the residue was purified through preparative high-performance liquid chromatography (with ammonium bicarbonate) to obtain compound 25. LCMS: m/z 497.1 [M+H]⁺; ¹H NMR (400 MHz, CDCl₃) δ: 7.66 (d, *J* = 7.9 Hz, 1H), 7.19 (s, 4H), 6.63 (d, *J =* 11.1 Hz, 1H), 3.98 - 3.95 (m, 4H), 3.93 - 3.85 (m, 6H), 2.81 (s, 3H), 2.75 (dt, *J =* 2.2, 12.1 Hz, 2H), 2.09 - 1.95 (m, 3H), 1.65 - 1.48 (m, 2H)

### Example 24: Compound 26

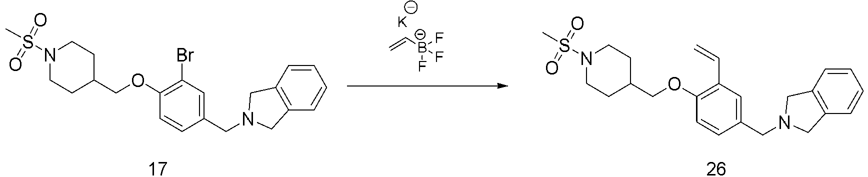

To a solvent mixture of water (1 mL) and 1,4-dioxane (5 mL) were added compound 17 (300 mg, 0.63 mmol), potassium vinyltrifluoroborate (419 mg, 3.13 mmol), [1,1'-bis(diphenylphosphino)ferrocene]palladium dichloride (II) (46 mg, 0.06 mmol), and potassium carbonate(259 mg, 1.88 mmol) at 25°C. After purging with nitrogen three times, the reaction was heated to 100°C and stirred for 2 hours. LCMS indicated the reaction was completed. The reaction mixture was filtered, and the filtrate was concentrated under reduced pressure. The residue was purified through preparative high-performance liquid chromatography (with ammonium bicarbonate) to obtain compound 26. LCMS: m/z 427.2 [M+H]⁺; ¹H NMR (400 MHz, CDCl₃) δ: 7.53 (d, J = 2.1 Hz, 1H), 7.29 - 7.26 (dd, J = 2.0, 8.4 Hz, 1H), 7.20 - 7.17 (m, 4H), 7.04 (dd, J = 11.2, 17.8 Hz, 1H), 6.84 - 6.81 (m, 1H), 5.82 - 5.76 (m, 1H), 5.30 - 5.26 (m, 1H), 3.93 (s, 4H), 3.92 - 3.90 (m, 1H), 3.90 - 3.87 (m, 3H), 3.87 - 3.85 (m, 2H), 2.81 (s, 3H), 2.73 (dt, J = 2.2, 12.0 Hz, 2H), 2.03 - 1.96 (m, 3H), 1.61 - 1.49 (m, 2H).

### Example 25: Compound 27

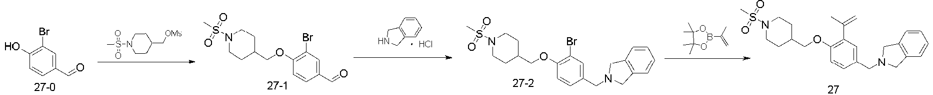

Step 1: To a solution of starting material 27-0 (3.00 g, 14.92 mmol) and (1-(methyl sulfonyl)piperidin-4-yl)methyl mesylate (4.90 g, 17.91 mmol) in N,N-dimethyl formamide (10 mL) was added potassium carbonate (6.20 g, 44.77 mmol) at 25°C. The mixture was heated to 80°C and stirred for 16 hours. LCMS indicated the reaction was completed. The reaction mixture was diluted with water (150 mL) and extracted with dichloromethane (100 mL×3). The organic phases were combined and washed with saturated saline solution (300 mL), dried over anhydrous sodium sulfate, and filtered. The filtrate was concentrated under reduced pressure, and the residue was purified through silica gel column chromatography (ethyl acetate:petroleum ether=1: 3) to obtain Intermediate 27-1. LCMS: m/z 376.0 [M+H]⁺.

Step 2: A solution of Intermediate 27-1 (5.00 g, 13.29 mmol), 2,3-dihydroisoindole hydrochloride (3.10 g, 19.94 mmol), sodium borohydride acetate (8.41 g, 39.87 mmol), and acetic acid (0.8 ml, 13.24 mmol) in 1,2-dichloroethane (15 mL) was stirred at 25°C for 16 hours. The reaction was monitored by LCMS. The reaction mixture was extracted with dichloromethane (40 mL×3). The organic phases were combined and washed with saturated saline solution (120 mL), dried over anhydrous sodium sulfate, and filtered. The filtrate was concentrated under reduced pressure, and the residue was purified through silica gel column chromatography (ethyl acetate:petroleum ether=1: 3) to obtain Intermediate 27-2. LCMS: m/z 479.1 [M+H]⁺.

Step 3: To a solution of Intermediate 27-2 (600 mg, 1.25 mmol), 4,4,5,5-tetramethyl-2-propenyl-1,3,2-dioxaborolane (315.5 mg, 1.88 mmol), potassium carbonate (518 mg, 3.75 mmol) in 1,4-dioxane (10 mL) and water (3 mL) were added bis-triphenylphosphine palladium dichloride (91 mg, 0.13 mmol) at 25°C and under nitrogen atmosphere. The mixture was heated to 90°C and stirred for 16 hours. LCMS indicated the reaction was completed. The reaction mixture was extracted with dichloromethane (40×3 mL). The organic phases were combined and washed with saturated saline solution (100 mL). The organic phase was concentrated under reduced pressure, and the residue was purified through silica gel column chromatography (ethyl acetate:petroleum ether=3:1) to obtain crude compound 27. 50 mg of the crude compound was purified through high-performance liquid chromatography (with ammonium bicarbonate) to obtain compound 27. LCMS: m/z 441.3 [M+H]⁺.¹H NMR (400MHz, CDCl₃) δ: 7.20 (br d, *J* = 2.3 Hz, 1H), 7.16 (d, *J* = 2.1 Hz, 1H), 7.11 (s, 4H), 6.74 (d, *J* = 8.3 Hz, 1H),4.90 - 5.08 (m, 2H), 3.86 (s, 4H), 3.81 - 3.83 (m, 1H), 3.76-3.80 (m, 5H), 2.72 (s, 3H), 2.64 (td, *J=* 12.1, 2.1 Hz, 2H), 2.04 (s,3H), 1.81 - 1.93 (m, 3H), 1.35 - 1.51 (m, 2H)

### Example 26: Compound 28

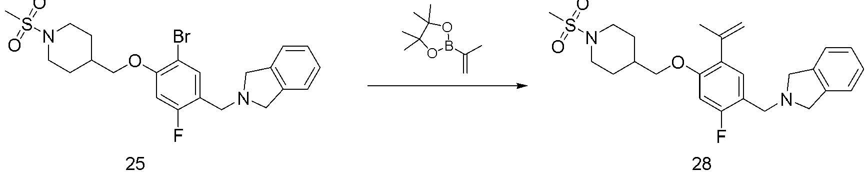

Into a mixture of water (0.2 mL) and 1,4-dioxane (1 mL) were added compound 25 (100 mg, 0.20 mmol), 4,4,5,5-tetramethyl-2-(2-propenyl)-1,3,2-dioxaborolane (0.11 mL, 0.60 mmol), [1,1'-bis(diphenylphosphino)ferrocene] palladium dichloride(II) (15 mg, 0.02 mmol), and potassium carbonate (83 mg, 0.60 mmol) at 25°C. After purging with nitrogen three times, the reaction was heated to 100°C and stirred for 2 hours. LCMS indicated the reaction was completed. The reaction mixture is filtered and concentrated under reduced pressure, and the residue was purified through preparative high-performance liquid chromatography (with ammonium bicarbonate) to obtain compound 28. LCMS: m/z 459.3 [M+H]⁺; ¹H NMR (400 MHz, CDCl₃) δ: 7.29 - 7.26 (d, *J =* 8.8 Hz, 1H), 7.18 (s, 4H), 6.58 (d, *J =* 11.8 Hz, 1H), 5.13 - 5.11 (m, 1H), 5.06 - 5.03 (m, 1H), 3.97 (s, 4H), 3.90 (s, 3H), 3.87 (br d, *J* = 2.1 Hz, 1H), 3.83 (d, *J= 5.8* Hz, 2H), 2.80 (s, 3H), 2.72 (dt, *J* = 2.2, 12.1 Hz, 2H), 2.09 (s, 3H), 1.99 - 1.92 (m, 3H), 1.52 (br dd, *J* = 2.9, 12.2 Hz, 2H).

### Example 27: Compound 29

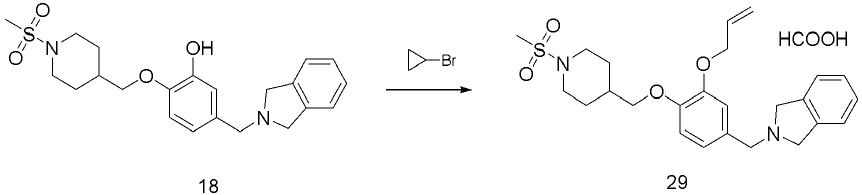

Into N,N-dimethylacetamide (5 mL) were added compound 18 (300 mg, 0.72 mmol) and bromocyclopropane (871 mg, 7.20 mmol) at 25°C under nitrogen atmosphere, followed by potassium carbonate (299 mg, 2.16 mmol). The reaction mixture was continuously stirred at 130°C for 2 hours. LCMS indicated the reaction was completed. The reaction mixture was filtered, and the filtrate was concentrated under reduced pressure. The residue was purified through preparative high-performance liquid chromatography (with formic acid) to obtain compound 29. LCMS: m/z 457.3 [M+H]⁺; ¹HNMR(400MHz, CDCl₃) δ:7.31 - 7.18 (m, 4H), 7.09 (d, *J* = 1.4 Hz, 1H), 6.96 - 6.90 (m, 1H), 6.89 - 6.82(m, 1H), 6.17 - 5.97 (m, 1H), 5.43 (dd, *J* = 1.2, 17.2 Hz, 1H), 5.32 - 5.21 (m, 1H), 4.59 (d, *J=* 5.1 Hz, 2H), 4.21 (s, 4H), 4.05 (s, 2H), 3.94 - 3.81 (m, 4H), 2.80 (s, 3H), 2.77 - 2.68 (m, 2H), 2.01 (d, *J=* 10.3 Hz, 3H), 1.58 - 1.43 (m, 2H).

### Example 28: Compound 30

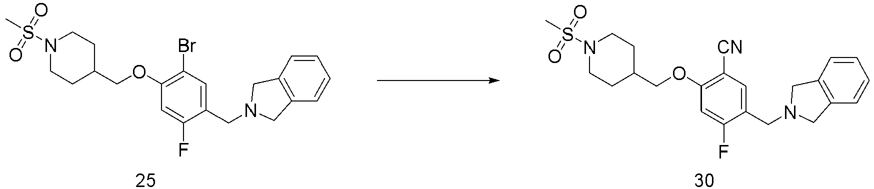

Compound 25 (100 mg, 0.20 mmol), zinc cyanide (24 mg, 0.20 mmol), tris(dibenzylideneacetone) dipalladium(0) (18 mg, 0.02 mmol), 1,1'-bis(diphenylphosphino)ferrocene (23 mg, 0.04 mmol), zinc powder (3 mg, 0.04 mmol) and N,N-dimethylacetamide (1 mL) were sequentially charged into a dry 8 mL reaction flask with stirring. After purging with nitrogen three times, the reaction was reaction mixture was stirred at 105°C for 2 hours. LCMS indicated the reaction was completed. The reaction mixture was concentrated under reduced pressure, and the residue was diluted with water (20 mL) and extracted with ethyl acetate (30 mL×3). The combined organic phase was washed with water (30 mL×3), dried over anhydrous sodium sulfate, and filtered. The filtrate was concentrated under reduced pressure, and the residue was purified through preparative high-performance liquid chromatography (with ammonium bicarbonate) to obtain compound 30. LCMS: m/z 444.2 [M+H]⁺; ¹H NMR (400 MHz, CDCl₃) δ: 7.74 (d, *J=* 7.9 Hz, 1H), 7.20 (s, 4H), 6.68 (d, *J=* 11.1 Hz, 1H), 3.96 (s, 4H), 3.93 - 3.87 (m, 6H), 2.82 (s, 3H), 2.75 (dt, *J* = 2.1, 12.1 Hz, 2H), 2.10 - 1.99 (m, 3H), 1.57 - 1.45 (m, 2H).

### Example 29: Compound 31

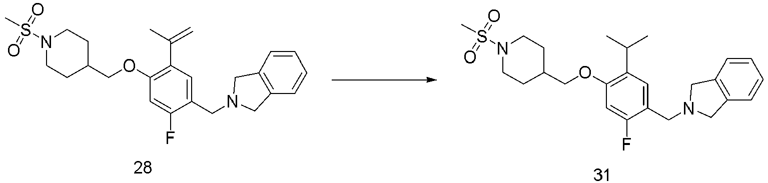

Into methanol (2 mL) were added compound 28(120 mg, 0.26 mmol) and palladium/carbon (28 mg, 0.26 mmol) at 25°C. After purging with hydrogen for three times, the reaction mixture was stirred under hydrogen atmosphere for 2 hours. LCMS indicated the reaction was completed. The reaction mixture was filtered, and the filtrate was concentrated under reduced pressure. The residue was purified through preparative high-performance liquid chromatography (with ammonium bicarbonate) to obtain compound 31. LCMS: m/z 461.3 [M+H]⁺; ¹H NMR (400 MHz, CDCl₃) δ: 7.25 (d, *J =* 8.5 Hz, 1H), 7.18 (s, 4H), 6.55 (d, *J* = 11.8 Hz, 1H), 3.96 (s, 4H), 3.93 - 3.87 (m, 4H), 3.83 (d, *J =* 5.6 Hz, 2H), 3.24 (quin, *J* = 6.9 Hz, 1H), 2.81 (s, 3H), 2.74 (dt, *J=* 2.1, 12.0 Hz, 2H), 2.03 - 1.90 (m, 3H), 1.64 - 1.50 (m, 2H), 1.23 (s, 3H), 1.21 (s, 3H).

### Example 30: Compound 32

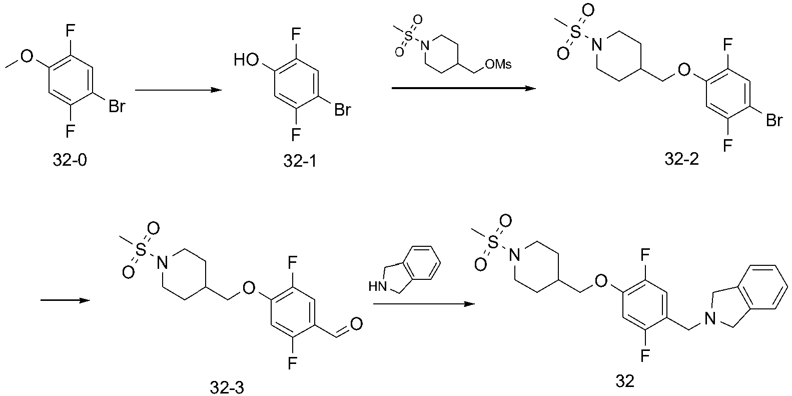

Step 1: Into dichloromethane (10 mL) was dissolved starting material 32-0 (1.00 g, 4.50 mmol) at 25°C, added with boron tribromide (2.26 g, 9.0 mmol), and continued stirring for 18 hours. LCMS indicated the reaction was completed. Saturated sodium bicarbonate solution (50 mL) was added for adjusting the reaction mixture to basic. The mixture was extracted with dichloromethane (50 mL×3). The combined organic phases were washed with saturated saline solution (50 mL), dried over anhydrous sodium sulfate, and filtered. The filtrate was concentrated under reduced pressure, and the residue was separated by silica gel column chromatography (petroleum ether:ethyl acetate=5:1) to obtain Intermediate 32-1.

Step 2: Into N,N-dimethyl formamide (10 mL) were dissolved Intermediate 32-1 (750 mg, 3.60 mmol), methyl (1-(methyl sulfonyl)piperidin-4-yl)mesylate (1,074 mg, 3.96 mmol) at 25°C, followed by addition of potassium carbonate (1.49 g, 10.79 mmol). After purging with nitrogen, the reaction was heated to 85°C and stirred for 3 hours. TLC indicated the reaction was completed. The mixture was diluted with water (20 mL) and extracted with dichloromethane (20 mL×3). The combined organic phases were washed with saturated saline solution (20 mL), dried over anhydrous sodium sulfate, and filtered. The filtrate was concentrated under reduced pressure, and the residue was separated by silica gel column chromatography (petroleum ether:ethyl acetate=2:1) to obtain Intermediate 32-2. LCMS: m/z 384.0 [M+H]⁺.

Step 3: Into dimethyl sulfoxide (8 mL) were dissolved Intermediate 32-2 (800 mg, 2.08 mmol) and triethylsilane (1.21 g, 10.43 mmol) at 25°C, followed by adding triethylamine (1.05 g, 10.39 mmol) and 1,1-bis(diphenylphosphino)ferrocene palladium dichloride (152 mg, 0.21 mmol). After purging with carbon monoxide, the mixture was heated to 85°C and stirred for 3 hours. LCMS indicated the reaction was completed. The reaction was quenched by adding water (30 mL) and extracted with dichloromethane (30 mL×3). The combined organic phase was washed with saturated saline solution (30 mL), dried over anhydrous sodium sulfate, and filtered. The filtrate was concentrated under reduced pressure, and the residue was separated by silica gel column chromatography (petroleum ether:ethyl acetate=1.5:1) to obtain Intermediate 32-3. LCMS: m/z 334.1 [M+H]⁺.

Step 4: Into 1,2-dichloroethane (6 mL) were dissolved Intermediate 32-3 (200 mg, 0.60 mmol) and isoindoline hydrochloride (140 mg, 0.90 mmol) at 25°C, and added with acetic acid (180 mg, 3.00 mmol) and sodium triacetoxyborohydride (255 mg, 1.20 mmol), and the mixture was stirred for 18 hours. LCMS indicated the reaction was completed. The reaction was quenched by adding water (30 mL) and extracted with dichloromethane (30 mL×3). The combined organic phases were washed with saturated saline solution (30 mL), dried over anhydrous sodium sulfate, and filtered. The filtrate was concentrated under reduced pressure, and the residue was purified through preparative high-performance liquid chromatography (with ammonium bicarbonate) to obtain compound 32. LCMS: m/z 437.1 [M+H]⁺; ¹H NMR (400 MHz, CDCl₃) δ: 7.29 - 7.18 (m, 5H), 6.70 (dd, *J=* 6.9, 10.7 Hz, 1H), 3.98 (s, 4H), 3.94 - 3.85(m, 6H), 2.82 (s, 3H), 2.74 (t, *J=* 11.2 Hz, 2H), 2.01 (d, *J =* 11.3 Hz, 3H), 1.58 - 1.46 (m, 2H).

### Example 31: Compound 33

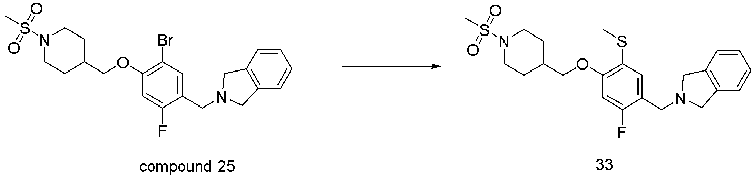

Into toluene (2 mL) were added compound 25 (100 mg, 0.20 mmol), sodium methyl mercaptide (56 mg, 0.80 mmol), tris(dibenzylideneacetone) dipalladium(0) (32 mg, 0.04 mmol), potassium phosphate (171 mg, 0.80 mmol), and 4,5-bisdiphenylphosphino-9,9-dimethyl xanthene(23 mg, 0.04 mmol) at 25°C. The reaction mixture was stirred at 150°C for 5 hours. LCMS indicated the reaction was completed. The reaction mixture was filtered, and the filtrate was concentrated under reduced pressure. The residue was purified through preparative high-performance liquid chromatography (with ammonium bicarbonate) to obtain compound 33. LCMS: m/z 465.2 [M+H]⁺; ¹H NMR (400 MHz, CDCl₃) δ: 7.25 (d, *J* = 7.9 Hz, 1H), 7.19 (s, 4H), 6.58 (d, *J* = 11.3 Hz, 1H), 3.96 (s, 4H), 3.93 - 3.86 (m, 6H), 2.81 (s, 3H), 2.74 (dt, *J* = 2.2, 12.1 Hz, 2H), 2.42 (s, 3H), 2.06 - 1.96 (m, 3H), 1.62 - 1.49 (m, 2H)

### Example 32: Compound 34

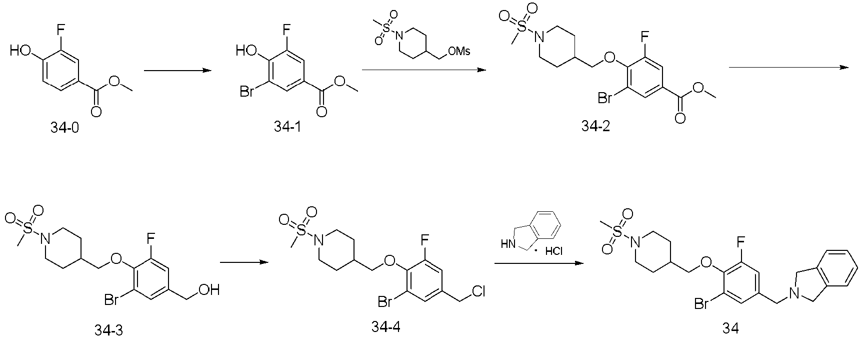

Step 1: A solution of starting material 34-0 (5.0 g, 29.39 mmol) and liquid bromine (2.82 g, 35.27 mmol) in acetic acid (20 mL) and dichloromethane (20 mL) was stirred at 25°C for 16 hours. Thin layer chromatography (ethyl acetate:petroleum ether=1:10) indicated the reaction was completed. The reaction mixture was added with potassium carbonate aqueous solution (20 mL) and stirred for 30 minutes. The mixture was extracted with ethyl acetate (50 mL×3). The combined organic phase was washed with saturated saline solution (150 mL) and concentrated under reduced pressure. The residue was purified through silica gel column chromatography (ethyl acetate:petroleum ether=1:10) to obtain Intermediate 34-1.

Step 2: A solution of Intermediate 34-1 (1.0 g, 4.02 mmol), methyl (1-(methyl sulfonyl)piperidin-4-yl)mesylate (1.31 g, 4.82 mmol) and potassium carbonate (1.94 g, 14.05 mmol) in N,N-dimethyl formamide (8 mL) was heated to 80°C and stirred for 16 hours. LCMS indicated the reaction was completed. The reaction mixture was diluted by ading water (80 mL) and extracted with dichloromethane (80 mL×3). The combined organic phase was washed with saturated saline solution (250 mL). The organic phase was concentrated under reduced pressure, and the residue was purified through silica gel column chromatography (ethyl acetate:petroleum ether=1: 3) to obtain Intermediate 34-2. LCMS: m/z 426.0[M+H]⁺.

Step 3: Into toluene (5 mL) was dissolved Intermediate 34-2 (400 mg, 0.94 mmol) at 0°C and under nitrogen atmosphere, followed by slowly adding a solution of diisobutyl aluminum hydride (1.89 mL, 2.83 mmol) in toluene dropwise. The reaction was heated to 25°C and stirred for 16 hours. LCMS indicated the reaction was completed. The reaction mixture was slowly added into a potassium sodium tartrate aqueous solution (10 mL) and stirred for 2 hours. The mxiture was extracted with dichloromethane (30 mL×3). The combined organic phase was washed with saturated saline solution (150 mL). The organic phase was concentrated under reduced pressure to obtain Intermediate 34-3. LCMS: m/z 396.0[M+H]⁺.

Step 4: To a solution of Intermediate 34-3 (175 mg, 0.44 mmol) in dichloromethane (5 mL) was added thionyl chloride (0.25 mL, 3.45 mmol) dropwise at 25°C and the mixture was stirred for 6 hours. LCMS indicated the reaction was completed. The reaction mixture was concentrated under reduced pressure to obtain Intermediate 34-4. LCMS: m/z 416.0 [M+H]⁺.

Step 5: A solution of Intermediate 34-4 (175 mg, 0.42 mmol), 2,3-dihydroisoindole hydrochloride (100 mg, 0.65 mmol) and triethylamine (0.3 mL, 2.2 mmol) in acetonitrile (5 mL) was stirred at 25°C for 16 hours. LCMS indicated the reaction was completed. The reaction mixture was extracted with ethyl acetate (15 mL×3). The combined organic phase was washed with saturated saline solution (40 mL) and concentrated under reduced pressure. The residue was purified through silica gel column chromatography (ethyl acetate:petroleum ether=1: 3) to obtain a crude product, which was purified through preparative high-performance liquid chromatography (with ammonium bicarbonate) to obtain compound 34. LCMS: m/z 497.0 [M+H]⁺; ¹H NMR (400 MHz, CDCl₃) δ: 7.39 (s, 1H), 7.21 (s, 4H), 7.16 (dd, *J* = 1.9, 11.5 Hz, 1H), 3.97 (d, *J =* 6.0 Hz, 2H), 3.94 (s, 4H), 3.88 (br d, *J =* 11.9 Hz, 2H), 3.83 (s, 2H), 2.81 (s, 3H), 2.74 (dt, *J* = 2.5, 12.1 Hz, 2H), 2.05 (br d, *J* = 13.4 Hz, 3H), 1.5 - 1.6 (m, 2H).

### Example 33: Compound 35

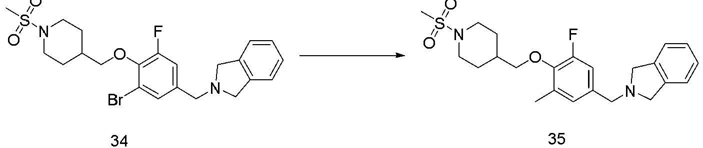

To a solution of compound 34 (60.0 mg, 0.14 mmol), trimethylboroxine (0.04 mL, 0.24 mmol), potassium carbonate (62.0 mg, 0.45 mmol) in 1,4-dioxane (3 mL) and water (1 mL) was added tetra[triphenylphosphine]palladium (15 mg, 0.01 mmol) at 25°C and under nitrogen atmosphere. The reaction was heated to 90°C and stirred for 16 hours. The reaction was monitored by LCMS. The reaction mixture was extracted with dichloromethane(10 mL×3). The combined organic phase was washed with saturated saline solution (25 mL), dried over anhydrous sodium sulfate, and filtered. The filtrate was concentrated under reduced pressure, and the residue was purified through silica gel column chromatography (ethyl acetate:petroleum ether=1: 1) to obtain a crude product, which was purified through preparative high-performance liquid chromatography (with ammonium bicarbonate) to obtain compound 35. LCMS: m/z 433.2 [M+H]⁺; ¹H NMR (CDCl₃, 400 MHz) δ: 7.19 (s, 4H), 6.96 - 7.04 (m, 2H), 3.93 (s, 4H), 3.86 - 3.92 (m, 4H), 3.81 (s, 2H), 2.81 (s, 3H), 2.73 (td, *J* = 12.0, 2.4 Hz, 2H), 2.27 (s, 3H), 1.97 - 2.07 (m, 3H), 1.46 - 1.59 (m, 2H).

### Example 34: Compound 36

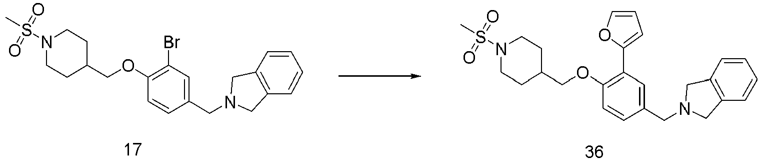

To a solution of compound 17 (50 mg, 0.10 mmol), potassium carbonate (29 mg, 0.21 mmol) and 2-(2-furanyl)-4,4,5,5-tetramethyl-1,3,2-dioxaborolane (20 mg, 0.10 mmol) in dioxane-water (3 mL, 5:1) was added tetra[triphenylphosphine]palladium (24 mg, 0.02 mmol) under nitrogen atmosphere The reaction was heated to 110°C and stirred for 3 hours. LCMS indicated the reaction was completed. The reaction mixture was direclty filtered. The filtrate was concentrated under reduced pressure, and the residue was purified through preparative high-performance liquid chromatography (with ammonium bicarbonate) to obtain compound 36. LCMS: m/z 467.2 [M+H]⁺; ¹H NMR (400 MHz, CDCl₃) δ:7.90 (d, *J* = 1.6 Hz, 1H), 7.47 (d, *J =* 0.6 Hz, 1H), 7.30 (dd, *J* = 1.6, 8.4 Hz,1H), 7.19 (s, 4H), 6.98 - 6.87 (m, 2H), 6.50 (dd, *J =* 1.7, 2.9 Hz, 1H), 3.99 (br d, *J =* 5.9 Hz, 2H), 3.96 (s, 3H), 3.94 - 3.88 (m, 4H), 2.81 (s, 3H), 2.80 - 2.71 (m, 2H), 2.13 - 1.95 (m, 4H), 1.67 - 1.51 (m, 2H).

### Example 35: Compound 37

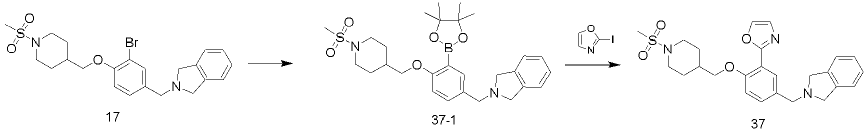

Step 1: Into 1,4-dioxane (5.00 ml) were dissolved compound 17 (120 mg, 0.25 mmol) and bis(pinacolato)diboron (0.16 mL, 0.63 mmol), into which were added acetic anhydride (0.09 ml, 1.00 mmol) and bis[5-(diphenylphosphino)cyclopenta-1,3-dienyl]-λ2-iron (II) dichloromethane palladium chloride (18 mg, 0.02 mmol). The reaction mixture was stirred at 100°C and under nitrogen atmosphere for 3 hours. LCMS indicated the reaction was completed. The mixture was concentrated under reduced pressure, and the residue (Intermediate 37-1) was directly used in the next reaction. LCMS: m/z 527.3[M+H]⁺.

Step 2: Into 1,4-dioxane (2 mL) and water (0.5mL) were dissolved Intermediate 37-1 (120 mg, 0.23 mmol), tetra[triphenylphosphine]palladium (263 mg, 0.23 mmol) and 2-iodo-1,3-oxazole (44 mg, 0.23mmol), and the mixture was added with sodium carbonate (31 mg, 0.23 mmol). The reaction was heated to 90°C under nitrogen atmosphere and stirred for 4 hours. LCMS indicated the reaction was completed. The mixture was quenched by adding water (10 mL) and extracted with ethyl acetate (10 mL×2). The combined organic phase was washed with saturated sodium chloride solution (20 mL), dried over anhydrous sodium sulfate, and filtered. The filtrate was concentrated under reduced pressure, and the residue was purified through reverse-phase preparative chromatography to obtain compound 37. LCMS: m/z 468.3[M+H]⁺; ¹H NMR (400 MHz, DMSO-*d₆*) δ: 8.18 (s, 1H), 7.85 (s, 1H), 7.47 (dd, *J* = 8.5, 1.6 Hz, 1H), 7.35 (s, 1H), 7.20 - 7.25 (m, 2H), 7.15 - 7.19 (m,3H), 3.99 (d, J = 5.8 Hz, 2H), 3.79 - 3.90 (m, 6H), 3.60 (d, *J* = 11.6 Hz, 2H), 2.86 (s, 3H), 2.75 (t, *J =* 11.2 Hz, 2H), 1.92 (d, *J* = 10.8Hz, 3H), 1.33-1.45 (m, 2H).

### Example 36: Compound 38

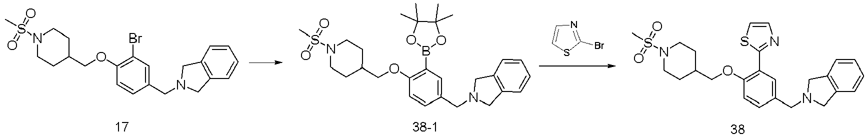

Step 1: To a solution of compound 17 (120 mg, 0.25 mol) and bis(pinacolato)diboron (159 mg, 0.6 mmol) in dioxane (2.0 mL) were added potassium acetate (74 mg, 0.7 mmol) and [1,1'-bis(diphenylphosphino)ferrocene]palladium dichloride (18 mg, 0.03 mmol) at 25°C. After purging with nitrogen, the reaction mixture was continuously stirred at 90°C for 4 hours. LCMS indicated the reaction was completed. The reaction mixture cooled down to 25°C and used directly in the next reaction. LCMS: m/z 527.2 [M+H]⁺.

Step 2: Into the reaction mixture from previous step 1 were added dioxane (1.0 mL) and water (0.7 mL) at 25°C, followed by 2-bromothiazole (48 mg, 0.30 mmol), potassium carbonate (102 mg, 0.70 mmol) and tetra(triphenylphosphine)palladium (28 mg, 0.02 mmol). After purging with nitrogen, the reaction mixture was heated to 110°C and stirred for 16 hours. LCMS indicated the reaction was completed. The reaction mixture was filtered, and the filtrate was dried by rotatory evaporation. The residue was purified through preparative high-performance liquid chromatography to obtain compound 38. LCMS: m/z 484.1 [M+H]⁺; ¹H NMR (400 MHz, CDCl₃) δ = 8.41 (s, 1H), 7.93 (d, *J =* 2.8 Hz, 1H), 7.54 (d, *J* = 8.1 Hz, 1H), 7.42 (d, *J* = 2.6Hz, 1H), 7.19 (s, 4H), 7.02 (d, *J=* 8.4 Hz, 1H), 4.10 (d, *J=* 5.6 Hz, 2H), 4.06 - 3.88 (m, 8H), 2.82 (s, 3H), 2.81 - 2.72 (m, 2H), 2.12 (d, *J=* 12.8 Hz, 3H), 1.70 - 1.57 (m, 2H).

### Example 37: Compound 39

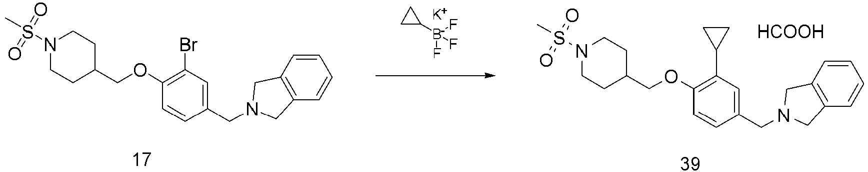

Into dioxane (3 mL) were added compound 17 (150 mg, 0.31 mmol) and potassium cyclopropylfluoroborate (92 mg, 0.62 mmol) at 25°C, followed by cesium carbonate (307 mg, 0.94 mmol), 1,1-bis(diphenylphosphino)ferrocene palladium dichloride (23 mg, 0.03 mmol), and water (0.5 mL). After purging with nitrogen, the reaction mixture was continuously stirred at 100°C for 18 hours. LCMS indicated the reaction was completed. The reaction mixture was filtered. The filtrate was diluted with water (10 mL) and extracted with ethyl acetate (20 mL×3). The combined organic phase was washed with saturated saline solution (40 mL×1), dried over anhydrous sodium sulfate, and filtered. The filtrate was concentrated under reduced pressure, and the residue was purified through preparative high-performance liquid chromatography (with formic acid) to obtain formate salt of compound 39. LCMS: m/z 441.3 [M+H]⁺; ¹H NMR (400 MHz, CDCl₃) δ:7.31 - 7.27 (m, 2H), 7.26 - 7.17 (m, 3H), 6.95 (s, 1H), 6.80 (d, *J* = 8.1 Hz, 1H),4.29 (s, 4H), 4.09 (s, 2H), 3.90 (d, *J* = 5.8 Hz, 4H), 2.81 (s, 3H), 2.74 (dt, *J* = 2.0, 12.0 Hz, 2H), 2.14 (ddd, *J=* 3.3, 5.2, 8.5Hz, 1H), 2.04 - 1.96 (m, 3H), 1.65 - 1.53 (m, 2H), 0.98 - 0.90 (m, 2H), 0.71 - 0.63 (m, 2H).

### Example 38: Compound 40

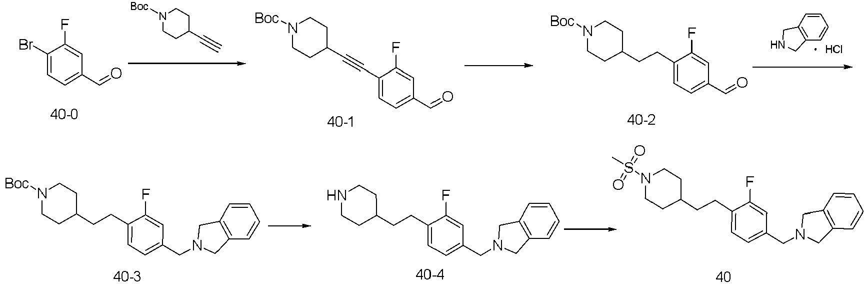

Step 1: A solution of starting material 40-0 (2.00 g, 9.85 mmol), tert-butyl 4-acetynylpiperidine-1-carboxylate (3.09 g, 14.78 mmol), bis[triphenylphosphine] palladium dichloride (690 mg, 0.98 mmol), and cuprous iodide (90 mg, 0.49 mmol) in triethylamine (5 mL) and tetrahydrofuran (15 mL) was stirred at 80°C and under nitrogen atmosphere for 16 hours. LCMS indicated the reaction was completed. The reaction mixture was extracted with dichloromethane (30 x 3 mL). The organic phases were combined and washed with saturated saline solution (100 mL). The organic phase was concentrated under reduced pressure and purified through silica gel column chromatography (ethyl acetate:petroleum ether = 5:1) to obtain Intermediate 40-1. LCMS: m/z 276.1 [M+H]⁺.

Step 2: A solution of Intermediate 40-1 (1.5 g, 4.53 mmol) and palladium-carbon (150 mg, 4.53 mmol) in isopropanol (10 mL) was stirred at 25°C and under nitrogen atmosphere. After purging with hydrogen three times, the solution was stirred for 8 hours. The reaction was monitored by LCMS. The reaction was filtered, and the filtrate was concentrated under reduced pressure to obtain Intermediate 40-2. LCMS: m/z 280.1 [M+H]⁺.

Step 3: A solution of Intermediate 40-2 (1.5 g, 4.47 mmol), 1H-isoindole hydrochloride (1.03 g, 6.71 mmol), sodium borohydride acetate (2.83 g, 13.42 mmol), acetic acid (0.26 ml, 4.47 mmol) in 1,2-dichloroethane (20 mL) was stirred at 25°C for 16 hours. LCMS indicated the reaction was completed. The reaction mixture was extracted with dichloromethane (30 x 3 mL). The organic phases were combined and washed with saturated sodium chloride solution (100 mL), dried over anhydrous sodium sulfate, and filtered. The filtrate was concentrated under reduced pressure, and the residue was purified through silica gel column chromatography (ethyl acetate:petroleum ether = 3:1) to obtain Intermediate 40-3. LCMS: m/z 437.0 [M+H]⁺.

Step 4: A solution of Intermediate 40-3(500 mg, 1.15 mmol), trifluoroacetic acid (1 mL, 13.42 mmol) in dichloromethane (5 mL) was stirred at 25°C for 30 minutes. LCMS indicated the reaction was completed. The reaction mixture was concentrated under reduced pressure to obtain Intermediate 40-4. LCMS: m/z 337.1 [M+H]⁺.

Step 5: Into dichloromethane(5 mL) were dissolved Intermediate 40-4 (385 mg, 1.14 mmol), triethylamine (0.47 mL, 3.42 mmol), and methanesulfonyl chloride (0.44 mL, 5.72 mmol) at 25°C and the mixture was stirred for 16 hours. LCMS indicated the reaction was completed. The reaction mixture was concentrated under reduced pressure, and the residue was purified through high-performance liquid chromatography (with ammonium bicarbonate) to obtain compound 40. LCMS: m/z 417.2 [M+H]⁺; ¹H NMR (400MHz, CDCl₃) δ: 7.12 - 7.25 (m, 7H), 4.03 (br s, 4H), 3.93 (s, 2H), 3.81 (br d, *J =* 11.6 Hz, 2H), 2.78 (s, 3H), 2.60 - 2.71 (m, 4H), 1.88 (br d, *J* = 9.9 Hz, 2H), 1.61 (br d, *J =* 5.8 Hz, 2H), 1.26 - 1.49 (m, 3H).

### Example 39: Compound 41

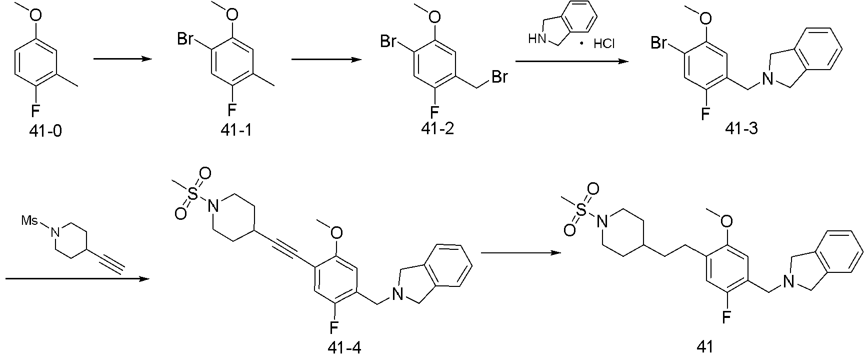

Step 1: To a solution of Intermediate 41-0 (3.5 g, 24.97 mmol) in dichloromethane (20 mL) was added liquid bromine (3.59 g, 22.47 mmol) at 25°C and the mixture was stirred for 16 hours. Thin layer chromatography (ethyl acetate:petroleum ether=1:10) indicated the reaction was completed. The reaction mixture was added with potassium carbonate aqueous solution (20 mL) and stirred for 30 minutes. The mixture was extracted with ethyl acetate (50 mL×3). The combined organic phase was washed with saturated saline solution (150 mL). The organic phase was concentrated under reduced pressure, and the residue was purified through silica gel column chromatography (ethyl acetate:petroleum ether=1:10) to obtain Intermediate 41-1. ¹H NMR (400 MHz, CDCl₃) δ: 7.13 (d, *J =* 8.6 Hz, 1H), 6.62 (d, *J =* 6.8 Hz, 1H), 3.8 - 3.8 (m, 3H), 2.16 (d, *J*=2.0 Hz, 3H).

Step 2: To a solution of Intermediate 41-1 (2.50 g, 11.41 mmol) and N-bromosuccinimide (2.03 g, 11.41 mmol) in carbon tetrachloride (15 mL) was added azobisisobutyronitrile (0.19 g, 1.14 mmol) at 60°C and the mixture was stirred for 16 hours. Thin layer chromatography (ethyl acetate:petroleum ether=1:10) indicated the reaction was completed. The reaction mixture was extracted with dichloromethane (20 mL×3). The combined organic phase was washed with saturated saline solution (60 mL). The organic phase was concentrated under reduced pressure, and the residue was purified through silica gel column chromatography (ethyl acetate:petroleum ether=1:10) to obtain Intermediate 41-2. ¹H NMR (400 MHz, CDCl₃) δ: 7.23 (d, *J =* 8.6 Hz, 1H), 6.81 (d, *J* = 6.5 Hz, 1H), 4.40 (d, *J* = 0.8 Hz, 2H), 3.8 - 3.9 (m, 3H).

Step 3: A solution of Intermediate 41-2 (2.00 g, 6.71 mmol), 2,3-dihydroisoindole hydrochloride (1.55 g, 10.07 mmol), and triethylamine (2.79 mL, 20.14 mmol) in acetonitrile (15 mL) was stirred at 25°C for 16 hours. The reaction was monitored by LCMS. The reaction mixture was extracted with dichloromethane (30 mL×3), and the combined organic phase was washed with saturated saline solution (100 mL). The organic phase was concentrated under reduced pressure, and the residue was purified through silica gel column chromatography (ethyl acetate:petroleum ether=1: 5) to obtain Intermediate 41-3. LCMS: m/z 338.1 [M+H]⁺.

Step 4: To a solution of Intermediate 41-3 (500.0 mg, 1.49 mmol), 4-ethynyl-1-(methyl sulfonyl)piperidine (1.12 g, 5.96 mmol), cuprous iodide (28.32 mg, 0.15 mmol) in triethylamine (2 mL) and tetrahydrofuran (6 mL) was added bis[triphenylphosphine]palladium dichloride (104.0 mg, 0.15 mmol) at 25°C and under nitrogen atmosphere. The reaction was heated to 80°C and stirred for 16 hours. LCMS indicated the reaction was completed. The reaction mixture was extracted with dichloromethane (30×3 mL). The organic phases were combined, washed with saturated saline solution (100 mL), and concentrated under reduced pressure. The residue was purified through silica gel column chromatography (ethyl acetate:petroleum ether=5:1) to obtain Intermediate 41-4. LCMS: m/z 443.2 [M+H]⁺.

Step 5: To a solution of Intermediate 41-4 (200.0 mg, 0.45 mmol) in methanol (5 mL) was added palladium-carbon (48.09 mg, 0.45 mmol) at 25°C and under nitrogen atmosphere. The reaction was heated to 50°C. After purging with hydrogen 3 times, the reaction was stirred for 8 hours. LCMS indicated the reaction was completed. The reaction mixture was filtered, and the filtrate was concentrated under reduced pressure. The residue was purified through preparative high-performance liquid chromatography (with ammonium bicarbonate) to obtain compound 41. LCMS: m/z 447.3 [M+H]⁺.¹H NMR (400 MHz, CDCl₃) δ: 7.20 (s, 4H), 6.96 (d, *J* = 6.1 Hz, 1H), 6.84 (d, *J=* 10.1 Hz, 1H), 4.00 (s, 4H), 3.94 (s, 2H), 3.77 - 3.84 (m, 5H), 2.78 (s, 3H), 2.57 - 2.69 (m, 4H), 1.88 (br d, *J* = 9.8 Hz, 2H), 1.52 - 1.61 (m, 2H), 1.26 - 1.42 (m, 3H).

### Example 40: Compound 42

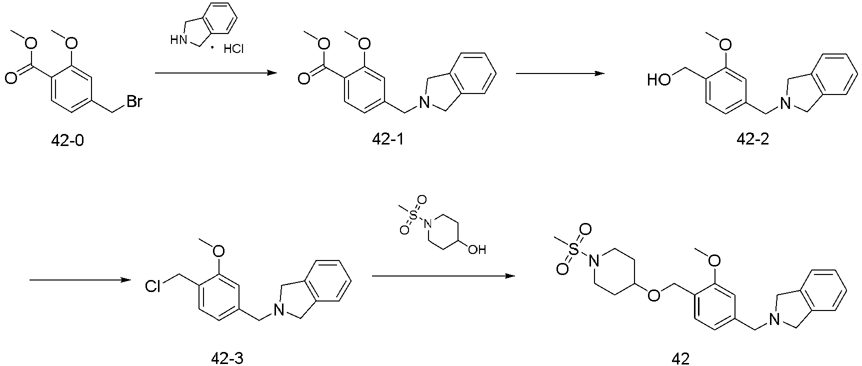

Step 1: To a solution of starting material 42-0 (1.20 g, 4.63 mmol) and diisopropylethanamine (6.14 mL, 37.04 mmol) in acetonitrile (30 mL) was added 1H-isoindole hydrochloride (1.20 g, 8.9 mmol) at 25°C and the mixture was stirred for 16 hours. LCMS indicated the reaction was completed. The reaction mixture was concentrated under reduced pressure, and the residue was purified through silica gel column chromatography (ethyl acetate:petroleum ether = 5:1) to obtain Intermediate 42-1. LCMS: m/z 298.2 [M+H]⁺.

Step 2: To a solution of Intermediate 42-1 (1 g, 1.17 mmol) in toluene (10 mL) was slowly added a solution of diisobutyl aluminum hydride (5.6 mL, 8.4 mmol, 1.5 M) in toluene dropwise at 0°C and the mixture was stirred for 16 hours. LCMS indicated the reaction was completed. The reaction was quenched by adding methanol (10 mL), after being stirred for 30 minutes, added with potassium sodium tartrate solution (10 mL, 4 M) and stirred for 2 hours. The mixture was extracted with dichloromethane (40 mL ×2). The combined organic phase was washed with saline solution (40 mL), dried over anhydrous sodium sulfate, and filtered. The filtrate was concentrated under reduced pressure, and the residue was purified through preparative thin layer chromatography silica gel plate (dichloromethane:methanol = 10:1) to obtain Intermediate 42-2. LCMS: m/z 270.2 [M+H]⁺.

Step 3: To a solution of Intermediate 42-2 (500 mg, 1.86 mmol) in dichloromethane (10 mL) was added thionyl chloride (0.68 mL, 9.3 mmol) dropwise at 25°C and the mixture was stirred for 2 hours. LCMS indicated the reaction was completed. The reaction was concentrated under reduced pressure to obtain Intermediate 42-3. LCMS: m/z 288.1 [M+H]⁺.

Step 4: To a solution of 1-(methyl sulfonyl)piperidin-4-ol (340 mg, 1.81 mmol) in N,N-dimethyl formamide (8 mL) was added sodium hydride (175 mg, 4.34 mmol) at 0°C and the mixture was stirred for 30 minutes, followed by Intermediate 42-3 (420 mg, 1.46 mmol) and continuously stirred for 16 hours. LCMS indicated the reaction was completed. The reaction was cooled to 0°C, quenched by slowly adding water (10 mL) dropwise. After being stirred for 30 minutes, the mixture was extracted with dichloromethane (15 mL×3). The combined organic phase was washed with saline solution (20 mL), dried over anhydrous sodium sulfate, and filtered. The filtrate was concentrated under reduced pressure, and the residue was purified through preparative high-performance liquid chromatography (with ammonium bicarbonate) to obtain compound 42. LCMS: m/z 431.2 [M+H]⁺; ¹H NMR (400 MHz, CDCl₃) δ: 7.35 (d, *J =* 7.5 Hz, 1H), 7.20 (s, 4H), 7.01 (s, 1H), 6.98 (d, *J* = 7.6 Hz, 1H), 4.58 (s, 2H),3.97 (s, 4H), 3.92 (s, 2H), 3.85 (s, 3H), 3.64-3.72 (m, 1H), 3.39 (ddd, *J =* 11.8, 8.2, 3.9 Hz, 2H), 3.19-3.30 (m, 2H), 2.79 (s,3H), 1.84 - 2.00 (m, 4H).

### Example 41: Compound 43

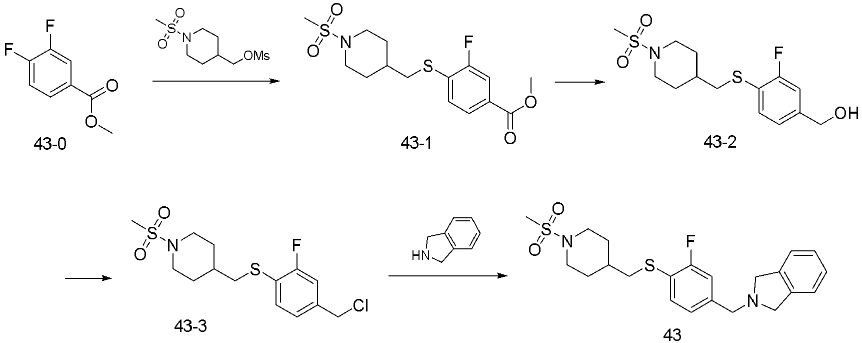

Step 1: Into dimethyl sulfoxide (5 mL) were added starting material 43-0 (100 mg, 0.58 mmol) and sodium sulfide nonahydrate (84 mg, 0.87 mmol). The mixture was heated to 80°C and stirred for 30 minutes. LCMS indicated the reaction was completed. The reaction mixture was added with methyl [1-(methyldioxo-λ6-thiolanyl)piperidin-4-yl]mesylate and the reaction was continued for 30 minutes. LCMS indicated the reaction was completed. The reaction mixture was poured into water (20 mL), extracted with ethyl acetate (20 mL), dried over anhydrous sodium sulfate, and filtered. The filtrate was concentrated under reduced pressure to obtain Intermediate 43-1. LCMS: m/z 362.1 [M+H]⁺.

Step 2: Into toluene (3 mL) was dissolved Intermediate 43-1 (150 mg, 0.41 mmol), cooled to 0°C and added with a solution of diisobutyl aluminum hydride-toluene (0.69 mL, 1.04 mmol, 1.5 M) dropwise and the mixture was stirred for 2 hours. LCMS indicated the reaction was completed. The reaction was quenched by adding saturated potassium sodium tartrate solution (10 mL) dropwise and extracted with ethyl acetate (20 mL×3). The combined organic phase was washed with saturated sodium chloride solution (30 mL), dried over anhydrous sodium sulfate, and filtered. The filtrate was concentrated under reduced pressure to obtain Intermediate 43-2. LCMS: m/z 316.1 [M-OH]⁺.

Step 3: Into dichloromethane (2 mL) was dissolved Intermediate 43-2 (90 mg, 0.27 mmol), added with thionyl chloride (64 mg, 0.54 mmol) dropwise and the mixture was stirred at 25°C for 30 minutes. LCMS indicated the reaction was completed. The reaction mixture was concentrated under reduced pressure to obtain Intermediate 43-3. LCMS: m/z 352.1 [M+H]⁺.

Step 4: Into acetonitrile (5 mL) were dissolved Intermediate 43-3 (105 mg, 0.30 mmol) and 2,3-dihydroisoindole hydrochloride (46 mg, 0.30), added with triethylamine (91 mg, 0.90 mmol) dropwise and the mixture was stirred at 25°C for 18 hours. LCMS indicated the reaction was completed. The reaction mixture was directly concentrated under reduced pressure, and the residue was purified through preparative high-performance liquid chromatography (ammonium bicarbonate) to obtain compound 43. LCMS: m/z 435.2 [M+H]⁺; ¹H NMR (400 MHz, CDCl₃) & 7.23 (t, *J= 7.9* Hz, 1H), 7.09 - 7.06 (m, 4H), 7.05 - 7.01 (m, 2H), 3.83 (s, 4H), 3.77 (s, 2H), 3.69 (br d, *J =* 12.0 Hz, 2H), 2.73 (d, *J =* 6.9 Hz, 2H), 2.65 (s, 3H), 2.51 (dt, *J =* 2.4, 12.0 Hz, 2H), 1.93 - 1.82 (m, 2H), 1.52 - 1.41 (m, 1H), 1.37 - 1.24 (m, 2H).

### Example 42: Compound 44

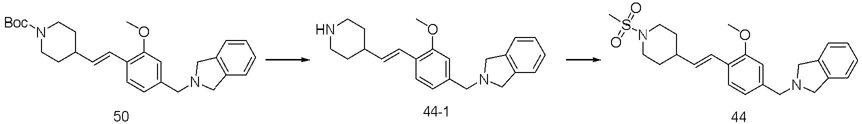

Step 1: A solution of compound 50 (200 mg, 0.45 mmol) in trifluoroacetic acid (1 mL) was allowed to react at 25°C under the action of ultrasound for 1 minute. LCMS indicated the reaction was completed. The reaction mixture was concentrated under reduced pressure to obtain Intermediate 44-1. LCMS: m/z 349.2 [M+H]⁺.

Step 2: A solution of Intermediate 44-1 (150 mg, 0.43 mmol), methanesulfonyl chloride (0.07 mL, 0.86 mmol), triethylamine (0.18 mL, 1.29 mmol) in dichloromethane (2 mL) was stirred at 25°C for 2 hours. LCMS indicated the reaction was completed. The reaction mixture was concentrated under reduced pressure, and the residue was purified through preparative high-performance liquid chromatography (with ammonium bicarbonate) to obtain compound 44. LCMS: m/z 427.2 [M+H]⁺; ¹H NMR (400 MHz, CDCl₃) δ: 7.39 (d, *J* = 7.8 Hz, 1H), 7.22 - 7.17 (m, 4H), 6.99 (s, 1H), 6.95 (d, *J* = 7.9 Hz, 1H), 6.75 (d, *J =* 16.0 Hz, 1H), 6.14 (dd, *J* = 6.9, 16.1 Hz, 1H), 3.95 (s, 4H), 3.90 (s, 2H), 3.86 (s, 3H), 3.85 (br s, 1H), 3.82 (br s, 1H), 2.80 (s, 3H), 2.79 - 2.71 (m, 2H), 2.35 - 2.24 (m, 1H), 1.96 - 1.89 (m, 2H), 1.68 - 1.56 (m, 2H).

### Example 43: Compound 45

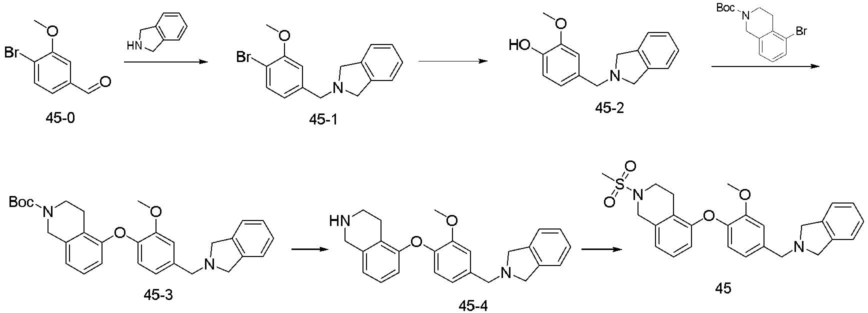

Step 1: Into dichloroethane (20 mL) were dissolved starting material 45-0 (2 g, 9.30 mmol) and isoindoline (1.66 g, 13.95 mmol) at 25°C, to which were sequentially added with acetic acid (2.79 g, 46.5 mmol) and sodium triacetoxyborohydride (3.94 g, 18.60 mmol). The reaction mixture was continuously stirred at 25°C for 12 hours. LCMS indicated the reaction was completed. The reaction was diluted with water (50 mL) and extracted with dichloromethane (60 mL×3). The combined organic phase was washed with saturated saline solution (10 mL×1), dried over anhydrous sodium sulfate, and filtered. The filtrate was concentrated under reduced pressure, and the residue was separated by silica gel column chromatography (petroleum ether:ethyl acetate=1:1) to obtain Intermediate 45-1. LCMS: m/z 318.0 [M+H]⁺.

Step 2: Into a mixture of dimethyl sulfoxide (12 mL) and water (4 mL) were dissolved Intermediate 45-1 (1.20 g, 3.80 mmol), cupric acetylacetonate (100 mg, 0.38 mmol) at 25°C, and then added with lithium hydroxide monohydrate (400 mg, 9.52 mmol) and N₁,N₂-bis(4-hydroxy-2,6-dimethylphenyl)oxalamide (120 mg, 0.36 mmol). After purging with nitrogen, the reaction mixture was continuously stirred at 80°C for 6 hours. LCMS indicated the reaction was completed. The mixture was adjusted to acidic with 2M aqueous hydrochloride (4 mL), further diluted with water (20 mL), and extracted with dichloromethane (40 mL×3). The combined organic phase was washed with saturated saline solution (5 mL×1), dried over anhydrous sodium sulfate, and filtered. The filtrate was concentrated under reduced pressure, and the residue was separated by silica gel column chromatography (petroleum ether:ethyl acetate=1:2) to obtain Intermediate 45-2. LCMS: m/z 256.1 [M+H]⁺.

Step 3: In to dimethyl formamide (2 mL) were dissolved Intermediate 45-2 (100 mg, 0.39 mmol), tertbutyl 5-bromo-3,4-dihydroisoquinolin-2(1H)-carboxylate (245 mg, 0.78 mmol) at 25°C, followed by addition of cesium carbonate (383 mg, 1.17 mmol), cuprous iodide (8 mg, 0.05 mmol), and Nₗ-(2-methyl-1-naphthyl)-N₂-(phenylmethyl)-oxalamide (10 mg, 0.03 mmol). After purging with nitrogen, the reaction mixture was continuously stirred at 120°C for 18 hours. LCMS indicated the reaction was completed. The reaction mixture was filtered, and the filtrate was diluted with water (10 mL) and extracted with dichloromethane (20 mL×3). The combined organic phase was washed with saturated saline solution (5 mL×1), dried over anhydrous sodium sulfate, and filtered. The filtrate was concentrated under reduced pressure, and the residue was separated by silica gel column chromatography (petroleum ether:ethyl acetate=2:1) to obtain Intermediate 45-3. LCMS: m/z 487.3 [M+H]⁺.

Step 4: Into dichloromethane (3 mL) was dissolved Intermediate 45-3 (50 mg, 0.1 mmol) at 25°C, followed by adding trifluoroacetic acid (1 mL). The reaction mixture was stirred at 25°C for 1 hour. LCMS indicated the reaction was completed. The reaction mixture was concentrated to obtain Intermediate 45-4. LCMS: m/z 387.1 [M+H]⁺.

Step 5: Into dichloromethane (4 mL) were dissolved Intermediate 45-4 (40 mg, 0.1 mmol) and triethylamine (36 mg, 0.3 mmol) at 25°C, added with methanesulfonyl chloride (20 mg, 0.17 mmol) dropwise. The reaction mixture was stirred at 25°C for 2 hours. LCMS indicated the reaction was completed. The reaction was quenched by adding water (10 mL) and extracted with ethyl acetate (20 mL×3). The combined organic phase was washed with saturated saline solution (5 mL×1), dried over anhydrous sodium sulfate, and filtered. The filtrate was concentrated under reduced pressure, and the residue was purified through preparative high-performance liquid chromatography to obtain compound 45. LCMS: m/z 465.2 [M+H]⁺; ¹H NMR (400 MHz, CDCl₃) δ:7.22 (s, 4H), 7.17 - 7.03 (m, 2H), 6.91 (d, *J =* 7.9 Hz, 1H), 6.82 (dd, J= 7.9,12.9 Hz, 2H), 6.64 (d, *J=* 8.1 Hz, 1H), 4.50 (s, 2H), 3.99 (s, 4H), 3.92 (s, 2H), 3.85 (s, 3H), 3.60 (t, *J=* 6.0 Hz, 2H), 3.03 (t, *J=* 5.8 Hz, 2H), 2.86 (s, 3H).

### Example 44: Compound 46

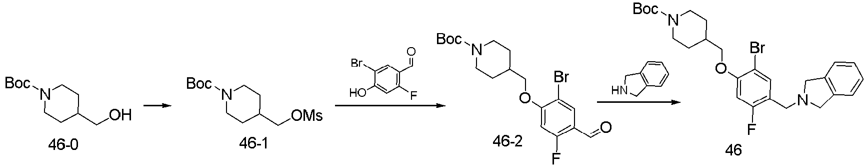

Step 1: To a solution of starting material 46-0 (3.00 g, 13.95 mmol) in dichloromethane (30 mL) were added triethylamine (4.64 g, 45.94 mmol), methanesulfonyl chloride (2.07 g, 18.15 mmol) sequentially at 25°C, and the mixture was stirred for 3 hours. LCMS indicated the reaction was completed. The reaction was quenched by adding water (80 mL) and extracted with dichloromethane (80 mL×3). The combined organic phase was washed with saturated saline solution (80 mL), dried over anhydrous sodium sulfate, and filtered. The filtrate was concentrated under reduced pressure to obtain Intermediate 46-1. LCMS: m/z 238.2 [M-55]⁺;

Step 2: Into N,N-dimethyl formamide (40 mL) were dissolved Intermediate 46-1 (4 g, 13.65 mmol), 5-bromo-2-fluoro-4-hydroxybenzaldehyde (3.00 g, 13.76 mmol) at 25°C, followed by addition of potassium carbonate (5.7 g, 41.30 mmol). The reaction mixture was continuously stirred at 25°C for 3 hours. LCMS indicated the reaction was completed. The reaction was diluted with water (100 mL) and extracted with ethyl acetate (100 mL×3). The combined organic phase was washed with saturated saline solution (100 mL), dried over anhydrous sodium sulfate, and filtered. The filtrate was concentrated under reduced pressure, and the residue was separated by silica gel column chromatography (petroleum ether:ethyl acetate=2: 1) to obtain Intermediate 46-2. LCMS: m/z 360.0 [M-55]⁺;

Step 3: Into 1,2-dichloroethane(15 mL) was dissolved Intermediate 46-2 (800 mg, 1.92 mmol), isoindoline hydrochloride (480 mg, 3.09 mmol) at 25°C, followed by addition of acetic acid (578 mg, 9.63 mmol) and sodium triacetoxyborohydride (817 mg, 3.85 mmol). The reaction mixture was stirred at 25°C for 18 hours. LCMS indicated the reaction was completed. The reaction was quenched by adding water (40 mL) and extracted with dichloromethane (40 mL×3). The combined organic phase was washed with saturated saline solution (40 mL), dried over anhydrous sodium sulfate, and filtered. The filtrate was concentrated under reduced pressure, and the residue was purified through preparative high-performance liquid chromatography (with ammonium bicarbonate) to obtain compound 46. LCMS: m/z 519.2 [M+H]⁺; ¹H NMR (400 MHz, CDCl₃) δ: 7.66 (d, *J* = 7.8 Hz, 1H), 7.19 (s, 4H), 6.62 (d, *J* = 11.3 Hz, 1H), 4.19 (s, 2H), 3.98 (s, 4H), 3.89 (s, 2H), 3.84 (d, *J=* 6.4 Hz, 2H), 2.78 (t, *J=* 12.3 Hz, 2H), 2.10 - 1.99 (m, 1H), 1.91 - 1.83 (m, 2H), 1.48(s, 9H), 1.39 - 1.27 (m, 2H).

### Example 45: Compound 47

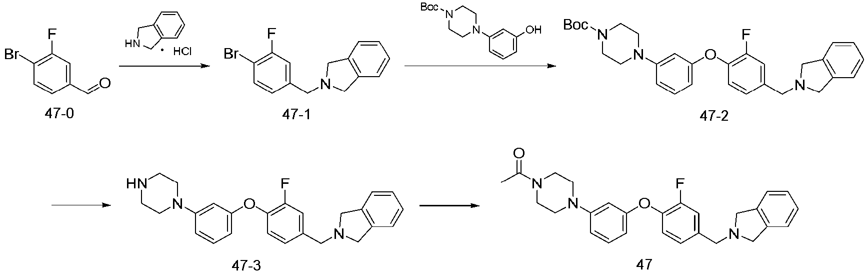

Step 1: A solution of starting material 47-0 (3.00 g, 14.78 mmol), 2,3-dihydroisoindole hydrochloride (2.72 g, 17.73 mmol), acetic acid (0.85 mL, 14.78 mmol), sodium triacetoxyborohydride (9.35 g, 44.33 mmol) in 1,2-dichloroethane (20 mL) was stirred at 25°C for 16 hours. LCMS indicated the reaction was completed. The reaction mixture was added with saturated sodium bicarbonate solution (20 mL) and extracted with dichloromethane (30 mL×3). The combined organic phase was washed with saturated saline solution (100 mL), dried over anhydrous sodium sulfate, and filtered. The filtrate was concentrated under reduced pressure, and the residue was purified through silica gel column chromatography (ethyl acetate:petroleum ether=3:1) to obtain Intermediate 47-1. LCMS: m/z 306.0[M+H]⁺.

Step 2: Into N,N-dimethyl formamide (5 mL) were added Intermediate 47-1 (660.0 mg, 2.16 mmol), tertbutyl 4-(3-hydroxyphenyl)piperizine-1-carboxylate (500 mg, 1.80 mmol), cuprous iodide (171.2 mg, 0.90 mmol), cesium carbonate (1.76 g, 5.39 mmol), and N-(2-phenyl-4-methylphenyl) N-benzyl oxalamide (31.0 mg, 0.09 mmol) at 25°C. After purging with nitrogen three times, the reaction was heated to 120°C and stirred for 16 hours. LCMS indicated the reaction was completed. The reaction mixture was diluted by adding water (30 mL) and extracted with dichloromethane (30 mL×3). The organic phases were combined, washed with saturated saline solution (100 mL), dried over anhydrous sodium sulfate, and filtered. The filtrate was concentrated under reduced pressure, and the residue was purified through silica gel column chromatography (ethyl acetate:petroleum ether=5:1) to obtain Intermediate 47-2. LCMS: m/z 504.3[M+H]⁺.

Step 3: To a solution of Intermediate 47-2 (150 mg, 0.30 mmol) in dichloromethane (5 mL) was added trifluoroacetic acid (1 mL, 13.42 mmol) at 25°C and the mixture was stirred for 2 hours. LCMS indicated the reaction was completed. The mixture was concentrated under reduced pressure and the residue was purified through thin layer chromatography plate (petroleum ether:ethyl acetate=1:1) to obtain Intermediate 47-3. LCMS: m/z 404.3[M+H]⁺.

Step 4: To a solutin of Intermediate 47-3 (100 mg, 0.25 mmol) in dichloromethane (5 mL) were sequentially added triethylamine (0.10 mL, 0.74 mmol) and acetyl chloride (0.02 mL, 0.25 mmol) dropwise at 25°C, and the mixture was stirred for 4 hours. LCMS indicated the reaction was completed. The reaction mixture was concentrated under reduced pressure and purified through thin layer chromatography plate (petroleum ether:ethyl acetate=1:1) to obtain compound 47 (60 mg). The crude product was further purified through preparative high-performance liquid chromatography (with ammonium bicarbonate) to obtain compound 47. LCMS: m/z 446.3[M+H]⁺; ¹H NMR (400 MHz, CDCl₃) δ: 7.30 (dd, *J* = 1.8 Hz, 1H), 7.21 - 7.24 (m, 4H), 7.18-7.21 (m, 1H), 7.16 (br d, *J* = 8.3 Hz, 1H), 7.01-7.07 (m, 1H), 6.66 (dd, *J* = 8.3, 2.0 Hz, 1H), 6.61 (t, *J=* 2.3 Hz, 1H), 6.47 (dd, *J=* 8.0, 2.1 Hz, 1H), 4.00 (s, 4H), 3.92 (s, 2H), 3.72-3.81 (m, 2H), 3.58 - 3.65 (m, 2H), 3.18 (dt, *J* = 13.4, 5.3 Hz, 4H), 2.14 (s, 3H).

### Example 46: Compound 48

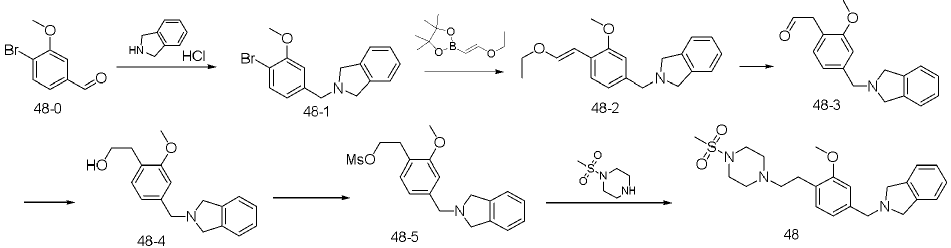

Step 1: Into 1,2-dichloroethane (10 mL) were dissolved starting material 48-0 (2.00 g, 9.30 mmol), isoindoline hydrochloride (1.66 g, 13.95 mmol), sodium borohydride acetate (3.92 g, 46.50 mmol), and acetic acid (2.66 ml, 46.50 mmol) at 25°C, and the mixture was stirred for 18 hours. The reaction was monitored by LCMS until completion. The reaction mixture was added with saturated sodium bicarbonate solution (50 mL) and extracted with dichloromethane (50 mL x 3). The combined organic phase was dried over anhydrous sodium sulfate and filtered. The filtrate was concentrated under reduced pressure and the residue was purified through silica gel column chromatography (ethyl acetate:petroleum ether = 5:1) to obtain Intermediate 48-1. LCMS: m/z 318.1[M+H]⁺.

Step 2: Into a solvent mixture of dioxane and water (6 mL, 5:1) were dissolved Intermediate 48-1 (300 mg, 0.94 mmol), (E)-1-ethoxyvinyl-2-boronic acid pinacol ester (373 mg, 1.89 mmol) and potassium carbonate (260 mg, 1.89 mmol), to which was added tetra(triphenylphosphine)palladium (109 mg, 0.09 mmol) under nitrogen atmosphere, and the mixture was stirred at 80°C for 2 hours. LCMS indicated the reaction was completed. The reaction mixture was filtered, and the filtrate was concentrated under reduced pressure. The residue was separated by silica gel column chromatography (petroleum ether:ethyl acetate = 3: 1) to obtain Intermediate 48-2. LCMS: m/z 310.2 [M+H]⁺.

Step 3: Into tetrahydrofuran-water (3 mL, 2:1) was dissolved Intermediate 48-2, added concentrated sulfuric acid (63 mg, 0.65 mmol) dropwise, and the mixture was stirred at 25°C for 18 hours. LCMS indicated the reaction was completed. The reaction mixture was added with saturated sodium bicarbonate solution (10 mL) and extracted with ethyl acetate (10 mL x 3). The combined organic phase was dried over anhydrous sodium sulfate and filtered. The filtrate was concentrated under reduced pressure to obtain Intermediate 48-3. LCMS: m/z 382.2 [M+H]⁺.

Step 4: Into methanol (3 mL) was dissolved Intermediate 48-3 (100 mg, 0.36 mmol), followed by addition of sodium borohydride (13 mg, 0.36 mmol), and the mixture was stirred at 25°C for 1 hour. LCMS indicated the reaction was completed. The reaction mixture was added with water (10 mL) and extracted with ethyl acetate (10 mL x 3). The combined organic phase was washed with saturated sodium chloride solution (20 mL), dried over anhydrous sodium sulfate, and filtered. The filtrate was concentrated under reduced pressure to obtain Intermediate 48-4. LCMS: m/z 284.2 [M+H]⁺.

Step 5: Into dichloromethane (3 mL) were dissolved Intermediate 48-4 (80 mg, 0.28 mmol) and triethylamine (114 mg, 1.13 mmol), added with methanesulfonyl chloride (64 mg, 0.56 mmol) dropwise, and the mixture was stirred at 25°C for 1 hour. LCMS indicated the reaction was completed. The reaction mixture was concentrated under reduced pressure to obtain Intermediate 48-5. LCMS: m/z 362.1 [M+H]⁺.

Step 6: Into acetonitrile (5 mL) was dissolved Intermediate 48-5 (100 mg, 0.28 mmol) and potassium carbonate (153 mg, 1.11 mmol), added with N-methanesulfonyl piperazine (136 mg, 0.83 mmol), and the mixture was stirred at 80°C for 3 hours. LCMS indicated the reaction was completed. The reaction mixture was added with water (5 mL) and extracted with ethyl acetate (5 mL x 3). The combined organic phase was washed with saturated sodium chloride solution (10 mL), dried over anhydrous sodium sulfate, filtered, and concentrated under reduced pressure. The residue was purified through preparative high-performance liquid chromatography (with ammonium bicarbonate) to obtain compound 48. LCMS: m/z 430.3 [M+H]⁺; ¹H NMR (400 MHz, CDCl₃) δ 7.19 (s, 4H), 7.10 (d, *J=* 7.5 Hz, 1H), 6.97 (s, 1H), 6.91 (d, *J=* 7.5 Hz, 1H),3.95 (s, 4H), 3.89 (s, 2H), 3.84 (s, 3H), 3.32 - 3.25 (m, 4H), 2.85 - 2.76 (m, 5H), 2.71 - 2.60 (m, 6H).

### Example 47: Compound 49

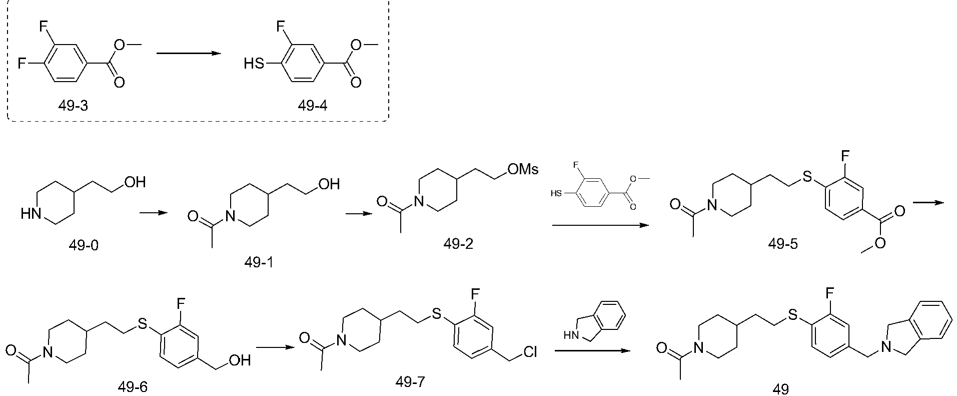

Step 1: Into dichloromethane (5 mL) was dissolved starting material 49-0 (500 mg, 3.80 mmol) at 25°C, and cooled to 0°C, followed by adding acetic anhydride (395 mg, 3.08 mmol) dropwise. The reaction mixture was continuously stirred at 0°C for 2 hours. TLC indicated the reaction was completed. The reaction mixture was diluted with dichloromethane (50 mL) and washed with saturated sodium chloride solution (50 mL). The organic phase was dried over anhydrous sodium sulfate and filtered. The filtrate was concentrated under reduced pressure to obtain Intermediate 49-1.

Step 2: Into dichloromethane (8 mL) were added Intermediate 49-1 (600 mg, 3.50 mmol), triethylamine (800 mg, 7.91 mmol) at 25°C, followed by adding methanesulfonyl chloride (480 mg, 4.21 mmol) dropwise. The reaction mixture was stirred at 25°C for 2 hours. LCMS indicated the reaction was completed. The reaction mixture was diluted with dichloromethane (40 mL), washed with saturated sodium bicarbonate solution (50 mL), and then washed with saturated sodium chloride solution (50 mL). The organic phase was dried over anhydrous sodium sulfate and filtered. The filtrate was concentrated under reduced pressure to obtain Intermediate 49-2. LCMS: m/z 250.0 [M+H]⁺.

Step 3: Into dimethyl sulfoxide (8 mL) was dissolved starting material 49-3(500 mg, 2.90 mmol) at 25°C, followed by addition of sodium sulfide nonahydrate (837 mg, 3.50 mmol). The reaction mixture was stirred at 80°C for 1.5 hours. At that time, LCMS indicated that the reaction was completed. The resulted reaction mixture containing Intermediate 49-4 was used directly in the next step. LCMS: m/z 373.2 [2M+H]⁺.

Step 4: Into the reaction mixture resulted from the previous step 3 were added Intermediate 49-2 (700 mg, 2.80 mmol) and triethylamine (850 mg, 8.40 mmol) at 25°C. The reaction mixture was stirred at 80°C for 2 hours. LCMS indicated the reaction was completed. The reaction mixture was diluted with dichloromethane (40 mL) and washed with saturated sodium chloride solution (50 mL). The organic phase was dried over anhydrous sodium sulfate and filtered. The filtrate was concentrated under reduced pressure, and the residue was separated by silica gel column chromatography (petroleum ether:ethyl acetate=1:3) to obtain Intermediate 49-5. LCMS: m/z 340.1 [M+H]⁺.

Step 5: Into tetrahydrofuran (4 mL) was dissolved Intermediate 49-5 (80 mg, 0.2 mmol) at 25°C, followed by adding lithium triethylborohydride-tetrahydrofuran (0.7 mL, 0.6 mmol, 1M) dropwise at 0°C. The reaction mixture was stirred at 0°C for 10 minutes. LCMS indicated the reaction was completed. The reaction mixture was diluted with ethyl acetate (40 mL), quenched with saturated sodium bicarbonate solution (50 mL), and washed with saturated sodium chloride solution (50 mL). The organic phase was dried over anhydrous sodium sulfate and filtered. The filtrate was concentrated under reduced pressure to obtain Intermediate 49-6. LCMS: m/z 312.2 [M+H]⁺.

Step 6: Into dichloromethane (3 mL) was dissolved Intermediate 49-6 (80 mg, 0.2 mmol) at 25°C, followed by addition of thionyl chloride (1 mL). The reaction mixture was stirred at 25°C for 1 hour. LCMS indicated the reaction was completed. The reaction mixture was concentrated under reduced pressure to obtain Intermediate 49-7. LCMS: m/z 330.1 [M+H]⁺.

Step 7: Into acetonitrile (4 mL) were dissolved Intermediate 49-7 (90 mg, 0.3 mmol) and isoindoline (51 mg, 0.3 mmol) at 25°C, followed by addition of triethylamine (83 mg, 0.8 mmol). The reaction mixture was stirred at 25°C for 3 hours. LCMS indicated the reaction was completed. The reaction was quenched with water (10 mL), and the mixture was extracted with ethyl acetate (20 mL×3). The combined organic phase was washed with saturated saline solution (50 mL), dried over anhydrous sodium sulfate, and filtered. The filtrate was concentrated under reduced pressure, and the residue was purified through preparative high-performance liquid chromatography to obtain compound 49. LCMS: m/z 413.2 [M+H]⁺; ¹H NMR (400 MHz, CDCl₃) δ: 7.34 (t, *J* = 7.9 Hz, 1H), 7.25 - 7.09 (m, 6H), 4.60 (td, *J* = 2.0, 13.2 Hz, 1H), 3.95 (s, 4H), 3.89 (s, 2H), 3.85 - 3.69 (m, 1H), 3.03 (dt, *J=* 2.4, 13.0 Hz, 1H), 2.94 (t, J= 7.4 Hz, 2H), 2.53 (dt, *J=* 2.3, 12.9 Hz, 1H), 2.08 (s, 3H), 1.81 - 1.65 (m, 3H), 1.63 - 1.53 (m, 2H), 1.22 - 1.02 (m, 2H).

### Example 48: Compound 50

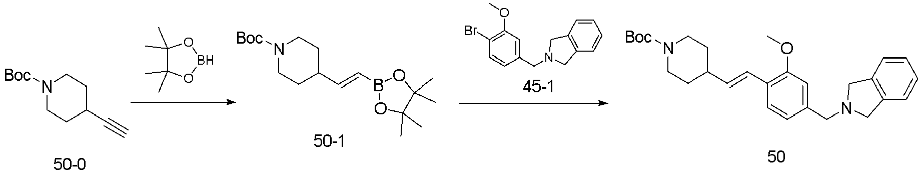

Step 1: Into toluene (15 mL) were added starting material 50-0 (1.00 g, 4.78 mmol), 4,4,5,5-tetramethyl-1,3,2-dioxaborolane (0.59 mL, 4.06 mmol) and zirconocene chloride hydride (0.12 g, 0.48 mmol) at 25°C. After purging with nitrogen three times, the reaction was heated to 60°C and stirred for 18 hours. LCMS indicated the reaction was completed. The reaction mixture was concentrated under reduced pressure. The residue was dissolved with petroleum ether (20 mL) and filtered. The filtrate was concentrated under reduced pressure to obtain Intermediate 50-1. LCMS: m/z 338.2 [M+H]⁺;

Step 2: Into a solvent mixture of water (1 mL) and 1,4-dioxane (5 mL) were added Intermediate 50-1 (1.3 g, 3.77 mmol), Intermediate 45-1 (400 mg, 1.26 mmol), [1,1'-bis(diphenylphosphino)ferrocene]palladium (II) dichloride (92 mg, 0.13 mmol), and potassium carbonate (521 mg, 3.77 mmol) at 25°C. After purging with nitrogen three times, the reaction was heated to 100°C and stirred for 2 hours. LCMS indicated the reaction was completed. The reaction mixture was filtered and concentrated under reduced pressure. The residue was purified through preparative high-performance liquid chromatography (with ammonium bicarbonate) to obtain compound 50. LCMS: m/z 449.3 [M+H]⁺; ¹H NMR (400 MHz, CDCl₃) δ:7.39 (d, *J* = 7.8 Hz, 1H), 7.19 (s, 4H), 6.97 (s, 1H), 6.94 (d, *J =* 7.9 Hz, 1H), 6.73 (d, *J=* 16.0 Hz, 1H), 6.15 (dd, *J=* 6.9, 16.1 Hz, 1H), 4.13 (br d, *J =* 7.1 Hz, 2H), 3.94 (s, 4H), 3.89 (s, 2H), 3.86 (s, 3H), 2.80 (br t, *J* = 11.9 Hz, 2H), 2.37 - 2.26 (m, 1H), 1.78 (br d, *J =* 11.6 Hz, 2H), 1.49 (s, 9H), 1.46 - 1.35 (m, 2H)

### Example 49: Compound 51

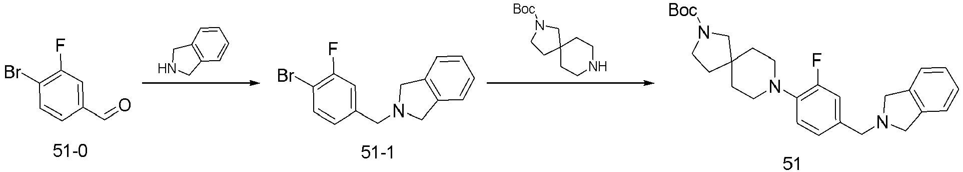

Step 1: Into 1,2-dichloroethane (50 mL) were added starting material 51-0 (2.7 g, 13.30 mmol), 2,3-dihydroisoindole hydrochloride (4.09 g, 526.60 mmol), sodium triacetoxyborohydride (5.61 g, 26.60 mmol), and acetic acid (0.76 mL, 13.30 mmol) at 25°C. The reaction mixture was stirred at 25°C for 2 hours and directly concentrated under reduced pressure. The residue was diluted with water (50 mL) and extracted with ethyl acetate (50 mL×3). The combined organic phase was washed with saturated sodium bicarbonate aqueous solution (50 mL×3), dried over anhydrous sodium sulfate, and filtered. The filtrate was concentrated under reduced pressure, and the residue was subjected to silica gel column chromatography and eluted (petroleum ether:ethyl acetate=10:1) to obtain Intermediate 51-1. LCMS: m/z 306.0 [M+H]⁺;

Step 2: Into 1,4-dioxane (10 mL) were added Intermediate 51-1 (508 mg, 1.66 mmol), tertbutyl 2,8-diazaspiro[4.5]decane-2-carboxylate (500 mg, 1.66 mmol), (4,5-bis(diphenylphosphino)-9,9-dimethylxanthene)(2'-methylamino-1,1'-biphen-2-yl)palladium( II) mesylate(269 mg, 0.17 mmol), and cesium carbonate(1.6 g, 4.98 mmol) at 25°C. After purging with nitrogen three times, the reaction was heated to 120°C and stirred for 3 hours. LCMS indicated the reaction was completed. The reaction mixture was filtered, and the filtrate was concentrated under reduced pressure. The residue was diluted with water (50 mL) and extracted with ethyl acetate (30 mL×3). The combined organic phase was washed with water (30 mL×3), dried over anhydrous sodium sulfate, and filtered. The filtrate was concentrated under reduced pressure, and the residue was subjected to silica gel column chromatography and eluted (petroleum ether:ethyl acetate=5:2) to obtain compound 51. LCMS: m/z 466.2 [M+H]⁺; ₁H NMR (400 MHz, CDCl₃) δ: 7.19 (s, 4H), 7.14 - 7.07 (m, 2H), 6.94 (q, *J* = 8.1 Hz, 1H), 3.93 (s, 4H), 3.84 (s, 2H), 3.45 (br t, *J =* 7.0 Hz, 1H), 3.40 (br t, *J =* 7.1 Hz, 1H), 3.29 (s, 1H), 3.20 (s, 1H), 3.17 - 3.07 (m, 2H), 3.04 - 2.92 (m, 2H), 1.81 - 1.73 (m, 6H), 1.48 (s, 9H).

### Example 50: Compound 52

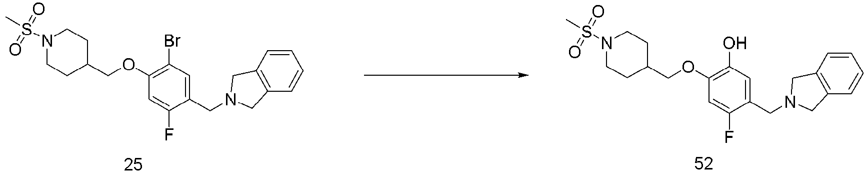

Into dimethyl sulfoxide (1.2 mL) were dissolved compound 25 (200 mg, 0.40 mmol), cupric acetylacetonate (10 mg, 0.04 mmol), lithium hydroxide monohydrate (42 mg, 1.01 mmol), N₁,N₂-bis(4-hydroxy-2,6-dimethylphenyl)oxalamide (13 mg, 0.04 mmol), and water (0.4 mL). After purging with nitrogen three times, the reaction was heated to 100°C and stirred for 5 hours. LCMS indicated the reaction was completed. The reaction mixture was adjusted to pH = 4-5 with 3 M aqueous hydrochloride, diluted with water (50 mL) and extracted with ethyl acetate (100 mL×3). The aqueous phase was filtered and concentrated under reduced pressure, and the residue was purified through preparative high-performance liquid chromatography (with ammonium bicarbonate) to obtain compound 52. LCMS: m/z 435.2 [M+H]⁺; ¹H NMR (400 MHz, CDCl₃) δ: 7.17 (s, 4H), 7.00 (d, *J =* 7.1 Hz, 1H), 6.60 (d, *J =* 10.6 Hz, 1H), 3.96 (s, 4H), 3.92 - 3.85 (m, 6H), 2.80 (s, 3H), 2.71 (dt, *J=* 2.0, 12.1 Hz, 2H), 2.04 - 1.92 (m, 4H), 1.57 - 1.45 (m, 2H).

### Example 51: Compound 53

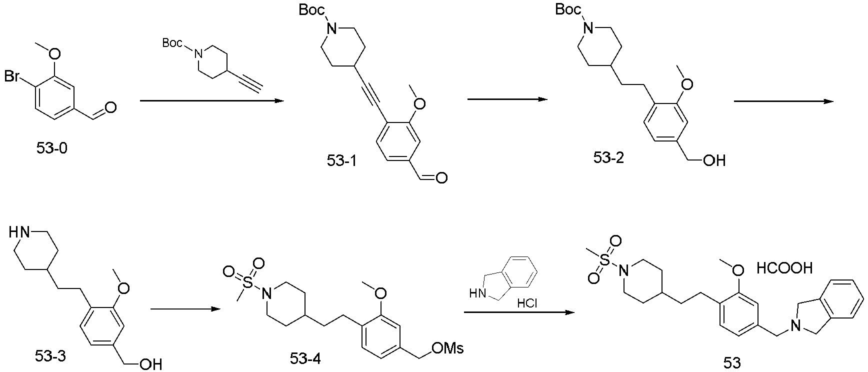

Step 1: A solution of starting material 53-0 (200 mg, 0.93 mmol), tertbutyl 4-ethynylpiperidine-1-carboxylate (583 mg, 2.79 mmol), and bis[triphenylphosphine]palladium dichloride (65 mg, 0.09 mmol) in solvent mixture of triethylamine (1.30 mL) and tetrahydrofuran (1 mL) was heated to 80°C under nitrogen atmosphere and stirred for 1 hour. LCMS indicated the reaction was completed. The reaction mixture was concentrated under reduced pressure, and the residue was purified through silica gel column chromatography (petroleum ether:ethyl acetate =10:1) to obtain Intermediate 53-1. LCMS: m/z 288.0 [M-55]⁺.

Step 2: Into isopropanol (5 mL) were dissolved Intermediate 53-1 (175 mg, 0.51mmol) and palladium-carbon (53 mg, 0.05 mmol, 10%) at 25°C under nitrogen atmosphere. After purging with hydrogen three times, the mixture was stirred for 2 hours. The reaction mixture was filtered, and the filtrate was concentrated under reduced pressure to obtain Intermediate 53-2.

Step 3: To a solution of Intermediate 53-2 (1.50 mg, 0.43 mmol) in dichloromethane (3 mL) was added trifluoroacetic acid (98 mg, 0.86 mmol) at 25°C, and the mixture was stirred for 2 hours. TLC indicated the reaction was completed. The reaction mixture was concentrated under reduced pressure to obtain Intermediate compound 53-3.

Step 4: To a solution of Intermediate 53-3 (110 mg, crude) and triethylamine (0.6 mL, 4.30 mmol) in dichloromethane (5 mL) was added methanesulfonyl chloride (246 mg, 2.15 mmol) dropwise at 25°C, and the mixture was stirred for 4 hours. LCMS indicated the reaction was completed. The reaction mixture was diluted with water (10 mL) and extracted with dichloromethane (10 mL×3). The combined organic phase was washed with saturated saline solution (20 mL), dried over anhydrous sodium sulfate, and filtered. The filtrate was concentrated under reduced pressure to obtain Intermediate 53-4. LCMS: m/z 310.1 [M-95]⁺.

Step 5: To a solutin of Intermediate 53-4 (151 mg, crude) and isoindoline hydrochloride (153 mg, 0.74 mmol) in N,N-dimethyl formamide (5 mL) was added potassium carbonate(103 mg, 0.74 mmol) at 25°C. The mixture was heated to 80°C and stirred for 2 hours. LCMS indicated the reaction was completed. The reaction mixture was concentrated under reduced pressure, and the residue was purified through preparative high-performance liquid chromatography (with formic acid) to obtain compound 53. LCMS: m/z 429.3 [M+H]⁺; ¹H NMR (400MHz, CDCl₃) δ: 8.37 (br s, 1H), 7.34 - 7.25 (m, 4H), 7.15 (s, 1H), 7.11 (d, *J=* 7.5 Hz, 1H), 6.8(br d, *J =* 7.5 Hz, 1H), 4.38 (s, 4H), 4.19 (s, 2H), 3.85 (s, 3H), 3.84 - 3.76 (m, 2H), 2.78 (s, 3H), 2.71 - 2.59 (m, 4H), 1.88 (brd, J= 10.1 Hz, 2H), 1.62 - 1.51 (m, 2H), 1.45 - 1.26 (m, 3H).

### Example 52: Compound 54

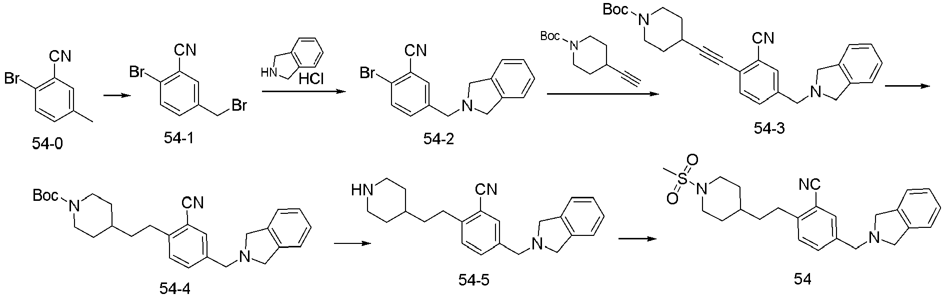

Step 1: Into carbon tetrachloride (15 mL) were dissolved starting material 54-0 (1.50 g, 7.69 mmol), N-bromosuccinimide (1.50 g, 8.42 mmol), azobisisobutyronitrile (630 mg, 3.84 mmol) at 25°C. The mixture was heated to 80°C and stirred for 2 hours. TLC indicated the reaction was completed. The reaction was quenched with water (50 mL) and extracted with dichloromethane (50 mL×3). The combined organic phase was washed with saturated sodium chloride solution (50 mL), dried over anhydrous sodium sulfate and filtered. The filtrate was concentrated under reduced pressure to obtain Intermediate 54-1.

Step 2: Into N,N-dimethyl formamide (20 mL) were added Intermediate 54-1 (2.50 g, 9.15 mmol) and isoindoline hydrochloride (1.85 g, 11.85 mmol) at 25°C, followed by potassium carbonate (3.79 g, 27.46 mmol). The reaction mixture was stirred at 25°C for 18 hours. LCMS indicated the reaction was completed. The reaction mixture was diluted with water (50 mL) and extracted with ethyl acetate (50 mL×3). The combined organic phase was washed with saturated sodium chloride solution (150 mL×1), dried over anhydrous sodium sulfate, and filtered. The filtrate was concentrated under reduced pressure, and the residue was separated by silica gel column chromatography (petroleum ether:ethyl acetate=8:1) to obtain Intermediate 54-2. LCMS: m/z 313.0 [M+H]⁺.

Step 3: Into tetrahydrofuran (9 mL) were added Intermediate 54-2 (700 mg, 2.24 mmol) and tertbutyl 4-ethynylpiperidine-1-carboxylate (703 mg, 3.36 mmol) at 25°C, followed by bis(triphenylphosphino)palladium dichloride (157 mg, 0.22 mmol), cuprous iodide (43 mg, 0.22 mmol), and triethylamine (3 mL). The reaction mixture was stirred at 80°C for 18 hours. LCMS indicated the reaction was completed. The reaction mixture was filtered. The filtrate was diluted with water (20 mL) and extracted with ethyl acetate (20 mL×3). The combined organic phase was washed with saturated sodium chloride solution (60 mL×1), dried over anhydrous sodium sulfate, and filtered. The filtrate was concentrated under reduced pressure, and the residue was separated by silica gel column chromatography (petroleum ether:ethyl acetate=4:1) to obtain Intermediate 54-3. LCMS: m/z 386.2 [M-55]⁺.

Step 4: Into methanol (10 mL) was dissolved Intermediate 54-3 (500 mg, 1.13 mmol) at 25°C under nitrogen atmosphere, followed by addition of palladium-carbon (500 mg, 10%). After purging with hydrogen three times, the reaction mixture was stirred at 25°C for 3 hours. LCMS indicated the reaction was completed. The reaction mixture was filtered, and the filtrate was concentrated under reduced pressure to obtain Intermediate 54-4. LCMS: m/z 446.2 [M+H]⁺.

Step 5: Into dioxane (4 mL) was dissolved Intermediate 54-4 (400 mg, 0.89 mmol) at 25°C, and added with a solution of hydrochloride in dioxane (4 mL). The reaction mixture was stirred at 25°C for 2 hours. LCMS indicated the reaction was completed. The reaction mixture was concentrated under reduced pressure to obtain Intermediate 54-5. LCMS: m/z 346.2 [M+H]⁺.

Step 6: Into dichloromethane (8 mL) were dissolved Intermediate 54-5 (200 mg, 0.58 mmol) and triethylamine (200 mg, 1.98 mmol) at 25°C, followed by addition of methane sulfonyl chloride (80 mg, 0.70 mmol). The reaction mixture was stirred at 25°C for 3 hours. LCMS indicated the reaction was completed. The reaction was quenched with water (20 mL) and extracted with ethyl acetate (20 mL×3). The combined organic phase was washed with saturated saline solution (50 mL×1), dried over anhydrous sodium sulfate, and filtered. The filtrate was concentrated under reduced pressure, and the residue was purified through preparative high-performance liquid chromatography (with ammonium bicarbonate) to obtain compound 54. LCMS: m/z 424.1 [M+H]⁺; ¹H NMR (400 MHz, CDCl₃) δ: 7.68 (d, *J =* 1.4 Hz, 1H), 7.60 (dd, J = 1.4, 8.0 Hz, 1H), 7.30 (d, *J =* 8.0 Hz,1H), 7.21 (s, 4H), 3.95 (s, 4H), 3.92 (s, 2H), 3.82 (d, *J* = 12.1 Hz, 2H), 2.90 - 2.84 (m, 2H), 2.78 (s, 3H), 2.71 - 2.61 (m, 2H),1.94 - 1.86 (m, 2H), 1.69 - 1.62 (m, 2H), 1.48 - 1.32 (m, 3H).

### Example 53: Compound 55

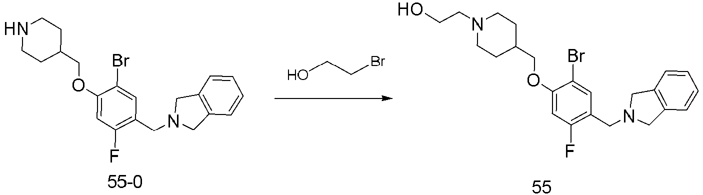

Into N,N-dimethyl formamide (8 mL) was dissolved Intermediate 55-0 (300 mg, 0.71 mmol) and 2-bromo-1-ethanol (133 mg, 1.07 mmol) at 25°C, followed by addition of potassium carbonate (396 mg, 2.86 mmol). The reaction mixture was continuously stirred at 25°C for 18 hours. LCMS indicated the reaction was completed. The mixture was diluted with water (30 mL) and extracted with ethyl acetate (30 mL×3). The combined organic phase was washed with saturated saline solution (30 mL), dried over anhydrous sodium sulfate, and filtered. The filtrate was concentrated under reduced pressure, and the residue was purified through preparative high-performance liquid chromatography (with ammonium bicarbonate) to obtain compound 55. LCMS: m/z 463.2 [M+H]⁺; ¹H NMR (400 MHz, CDCl₃) δ: 7.64 (d, J = 7.9 Hz, 1H), 7.19 (s, 4H), 6.63 (d, J = 11.3 Hz, 1H), 3.96 (s, 4H), 3.88 (s, 2H), 3.84 (d, J = 6.0 Hz, 2H), 3.64 (t, J = 5.3 Hz, 2H), 3.01 (d, J = 11.4 Hz, 2H), 2.58 (t, J = 5.4 Hz, 2H), 2.42 - 2.29 (m, 1H), 2.21 - 2.13 (m, 2H), 1.92 (d, J = 10.3 Hz, 3H), 1.55 - 1.40 (m, 2H).

### Example 54: Compound 56

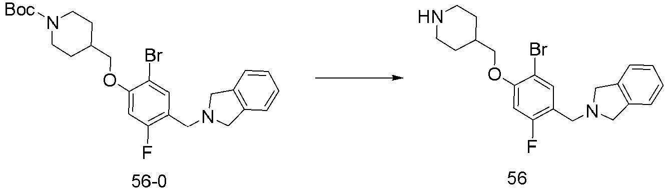

Into dichloromethane (6 mL) was dissolved Intermediate 56-0 (100 mg, 0.19 mmol) at 25°C, followed by addition of trifluoroacetic acid (2 mL). The reaction mixture was continuously stirred at 25°C for 1.5 hours. LCMS indicated the reaction was completed. The reaction mixture was concentrated under reduced pressure, and the residue was purified through preparative high-performance liquid chromatography (with ammonium bicarbonate) to obtain compound 56. LCMS: m/z 419.1 [M+H]⁺; ¹H NMR (400 MHz, CDCl₃) δ: 7.64 (d, *J=* 7.9 Hz, 1H), 7.19 (s, 4H), 6.62 (d, *J=* 11.4 Hz, 1H), 3.95 (s, 4H),3.87 (s, 2H), 3.83 (d, *J=* 6.3 Hz, 2H), 3.22 (s, 2H), 2.73 (s, 2H), 2.14 - 1.80 (m, 3H), 1.39 (d, *J=* 11.8 Hz, 2H).

### Example 55: Compound 57

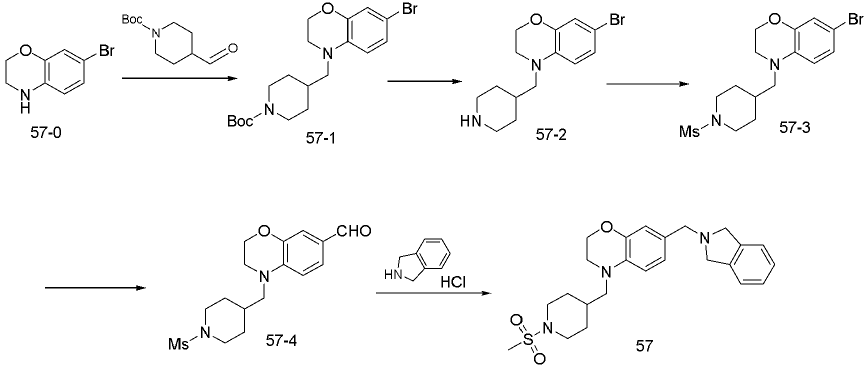

Step 1: To a solution of starting material 57-0 (2.00 g, 9.34 mmol), 1-tertbutoxycarbonyl piperidine-4-carbaldehyde (3.99 g, 18.69 mmol) and acetic acid (0.56 mL, 9.34 mmol) in 1,2-dichloroethane (30 mL) was added sodium triacetoxyborohydride (3.94 g, 18.68 mmol) at 0°C. The mixture was heated to 25°C and stirred for 18 hours. LCMS indicated the reaction was completed. The reaction was quenched with saturated sodium bicarbonate solution (50 mL) and extracted with dichloromethane (50 mL×3). The combined organic phase was washed with saturated saline solution (100 mL), dried over anhydrous sodium sulfate, and filtered. The filtrate was concentrated under reduced pressure, and the residue was purified through silica gel column chromatography (ethyl acetate:petroleum ether = 2:1) to obtain Intermediate 57-1. LCMS: m/z 355.1[M-55]⁺.

Step 2: To a solution of Intermediate 57-1 (2.90 g, 7.05 mmol) in dichloromethane (20 mL) was added trifluoroacetic acid (1.58 mL, 21.15 mmol) dropwise at 25°C and the mixture was stirred for 3 hours. LCMS indicated the reaction was completed. The reaction mixture was concentrated under reduced pressure to obtain Intermediate 57-2. LCMS: m/z 311.1[M+H]⁺.

Step 3: Into a solution of Intermediate 57-2 (2.20 g) and triethylamine (2.93 mL, 21.15 mmol) in dichloromethane (20 mL) was added methanesulfonic anhydride (2.46 g, 14.10 mmol) at 25°C, and the mixture was stirred for 2 hours. LCMS indicated the reaction was completed. The reaction mixture was filtered, and the filtrate was concentrated under reduced pressure. The residue was purified through silica gel column chromatography (ethyl acetate:petroleum ether = 2:1) to obtain Intermediate 57-3. LCMS: m/z 389.0[M+H]⁺.

Step 4: A solution of Intermediate 57-3 (2.20 g, crude), triethylamine (4.45 mL, 32.11 mmol), triethylsilane (5.19 mL, 32.11mmol) and 1,1-bis(diphenylphosphino)ferrocene palladium dichloride (0.47 g, 0.64 mmol) in dimethyl sulfoxide (20 mL) was heated to 80°C under carbon monoxide atmosphere and stirred for 8 hours. LCMS indicated the reaction was completed. The reaction mixture was diluted with water (100 mL) and extracted with ethyl acetate (100 mL×3). The combined organic phase was washed with saturated saline solution (500 mL), dried over anhydrous sodium sulfate, and filtered. The filtrate was concentrated under reduced pressure, and the residue was purified through silica gel column chromatography (ethyl acetate:petroleum ether = 1:1) to obtain Intermediate 57-4. LCMS: m/z 339.1[M+H]⁺.

Step 5: To a solution of Intermediate 57-4 (200 mg, 0.59 mmol), 2,3-dihydroisoindole hydrochloride (181 mg, 1.18 mmol) and acetic acid (30 µL, 0.59 mmol) in 1,2-dichloroethane (5 mL) was added sodium triacetoxyborohydride (248 mg, 1.18 mmol) at 25°C, and the mixture was stirred for 18 hours. LCMS indicated the reaction was completed. The reaction was quenched with saturated sodium bicarbonate solution (20 mL) and extracted with dichloromethane (20 mL×3). The combined organic phase was washed with saturated saline solution (50 mL), dried over anhydrous sodium sulfate, and filtered. The filtrate was concentrated under reduced pressure, and the residue was purified through silica gel column chromatography (ethyl acetate:petroleum ether = 1:1) to obtain compound 57. LCMS: m/z 323.2[M-118]⁺; ¹H NMR (400 MHz, CDCl₃) δ:7.17 (s, 4H), 6.90 - 6.83 (m, 2H), 6.55 (d, *J =* 8.1 Hz, 1H), 4.26 - 4.21 (m, 2H), 3.93 (s, 4H), 3.90 - 3.83 (m, 2H), 3.78 (s, 2H), 3.42 - 3.33 (m, 2H), 3.13 (d, *J= 6.6* Hz, 2H), 2.78 (s, 3H), 2.64 (dt, *J =* 2.0, 12.1 Hz, 2H), 1.94 - 1.84 (m, 3H), 1.46 - 1.33 (m, 2H).

### Example 56: Compound 58

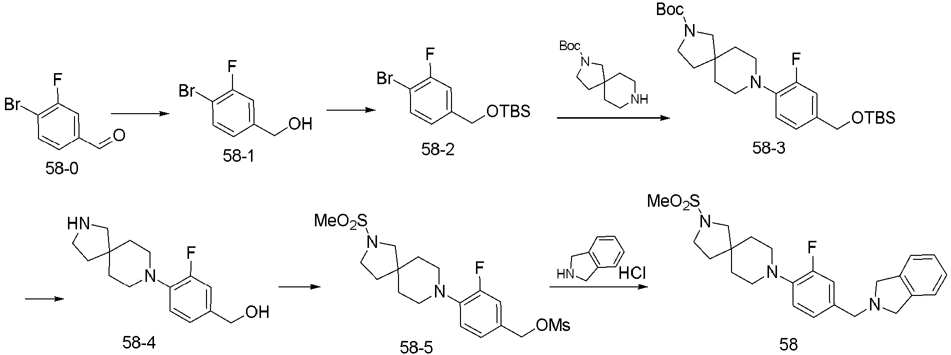

Step 1: To a solution of starting material 58-0 (1.00 g, 4.93 mmol), methanol (5 mL) and tetrahydrofuran (10 mL) in a dry 40 mL flask was added sodium borohydride (0.28 g, 7.39 mmol) in portions under stirring, and the reaction mixture was stirred at 25°C for 1 hour. TLC indicated the reaction was completed. The reaction was quenched with saturated ammonium chloride aqueous solution (20 mL), and the mixture was diluted with water (50 mL), and extracted with ethyl acetate (50 mL×3). The combined organic phase was dried over anhydrous sodium sulfate and filtered. The filtrate was concentrated under reduced pressure to obtain Intermediate 58-1.

Step 2: A solution of Intermediate 58-1 (900 mg, 4.39 mmol), tert-butyldimethylchlorosilane (992 mg, 6.58 mmol) and imidazole (896 mg, 13.17 mmol) in N,N-dimethyl formamide (10 mL) was stirred at 25°C for 1 hour. TLC indicated the reaction was completed. The reaction mixture was diluted with water (20mL) and extracted with ethyl acetate (30mL×3). The combined organic phase was washed with water (30 mL×3), dried over anhydrous sodium sulfate, and filtered. The filtrate was concentrated under reduced pressure to obtain Intermediate 58-2.

Step 3: Into a dry 40 mL flask was sequentially added Intermediate 58-2 (500 mg, 1.57 mmol), tertbutyl 2,8-diazasipro[4.5]decane-2-carboxlate (377 mg, 1.57 mmol), cesium carbonate (1.53 g, 4.7 mmol), (2-dicyclohexylphosphino-2',6'-dimethoxybiphenyl)(2'-methylamino-1,1'-biphen-2-yl)palladium( II) mesylate (124 mg, 0.16 mmol) and 1,4-dioxane (10 mL) under stirring. After purging with nitrogen three times, the reaction was heated to 120°C and stirred for 5 hours. LCMS indicated the reaction was completed. The reaction mixture was concentrated under reduced pressure, and the residue was subjected to silica gel column chromatography and eluted (petroleum ether:ethyl acetate=10:1) to obtain Intermediate 58-3. LCMS: m/z 379.2 [M+H]⁺.

Step 4: Into a dry 8 mL flask were sequentially added tertbutyl 8-(4-(((tert-butyldimethylsilyl)oxy)methyl)-2-fluorophenyl)-2,8-diazaspiro[4.5]decane-2-carbox ylate (100 mg, 0.21 mmol), trifluoroacetic acid (0.1 mL) and dichloromethane (1 mL) under stirring, and the mixture was stirred at 25°C for 5 hours. LCMS indicated the reaction was completed. The reaction mixture was diluted with saturated sodium bicarbonate aqueous solution (50 mL) and extracted with ethyl acetate (20 mL×3). The combined organic phase was washed with water (30 mL×3), dried over anhydrous sodium sulfate, and filtered. The filtrate was concentrated under reduced pressure to obtain Intermediate 58-4. LCMS: m/z 265.1 [M+H]⁺.

Step 5: Into a dry 8 mL flask were sequentially added Intermediate 58-4 (50 mg, 0.19 mmol), methanesulfonyl chloride (0.06 mL, 0.76 mmol), triethylamine (0.08 mL, 0.57 mmol) and dichloromethane (2 mL) under stirring. The reaction mixture was stirred at 25°C for 2 hours. LCMS indicated the reaction was completed. The reaction mixture was concentrated under reduced pressure to obtain Intermediate 58-5. LCMS: m/z 325.0.

Step 6: Into a dry 8 mL flask were sequentially added Intermediate 58-5 (50 mg, 0.12 mmol), isoindoline hydrochloride (36 mg, 0.24 mmol), cesium carbonate (116 mg, 0.36 mmol) and N,N-dimethyl formamide (2 mL) under stirring. The reaction mixture was stirred at 100°C for 18 hours. LCMS indicated the reaction was completed. The reaction mixture was diluted with water (20 mL) and extracted with ethyl acetate (30 mL×3). The combined organic phase was washed with water (30 mL×3), dried over anhydrous sodium sulfate, and filtered. The filtrate was concentrated under reduced pressure, and the residue was purified through preparative high-performance liquid chromatography (with ammonium bicarbonate) to obtain compound 58. LCMS: m/z 444.3 [M+H]⁺;¹H NMR (400 MHz, CDCl₃) δ: 7.19 (s, 4H), 7.15 - 7.08 (m, 2H), 6.95 - 6.89 (m, 1H), 3.93 (s, 4H), 3.84 (s, 2H), 3.45 (t, *J=* 7.1 Hz, 2H), 3.26 (s, 2H), 3.16 - 3.09 (m, 2H), 3.02 - 2.95 (m, 2H), 2.87 (s, 3H), 1.90 (t, *J= 7.1* Hz, 2H), 1.81 (t, *J =* 5.5 Hz, 4H).

### Example 57: Compound 59

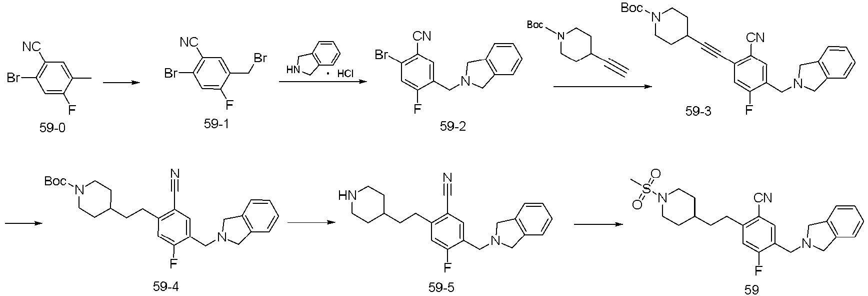

Step 1: A solution of starting material 59-0 (3.00 g, 14.02 mmol), azobisisobutyronitrile (230 mg, 1.40 mmol), N-bromosuccinimide (2.74 g, 15.42 mmol) in carbon tetrachloride (15 mL) was stirred at 60°C for 4 hours. After cooled down to 25°C, the reaction mixture was concentrated under reduced pressure, and the residue was purified through silica gel column chromatography (petroleum ether:ethyl acetate = 5:1) to obtain Intermediate 59-1. ¹H NMR (400MHz, CDCl₃) δ: 7.75 (d, *J =* 7.3 Hz, 1H), 7.46 (d, *J =* 8.8 Hz, 1H), 4.43 (s, 2H).

Step 2: A solution of Intermediate 59-1(2.30 g, 7.85 mmol), 2,3-dihydroisoindole hydrochloride (1.45 g, 9.42 mmol), and triethylamine (3.27 mL, 23.56 mmol) in dichloromethane (15 mL) was stirred at 25°C for 4 hours. LCMS indicated the reaction was completed. The reaction mixture was extracted with ethyl acetate (30 mL×3). The combined organic phase was washed with saturated saline solution (100 mL), concentrated under reduced pressure, and purified through silica gel column chromatography (petroleum ether:ethyl acetate= 3:1) to obtain Intermediate 59-2. LCMS: m/z 331.1 [M+H]⁺.

Step 3: A solution of Intermediate 59-2 (300 mg, 0.91 mmol), tertbutyl 4-ethynylpiperidine-1-carboxylate (474 mg, 2.26 mmol), bis[triphenylphosphine]palladium dichloride (64 mg, 0.09 mmol), and cuprous iodide (18 mg, 0.09 mmol) in solvent mixture of triethylamine (2 mL) and tetrahydrofuran (6 mL) was stirred at 80°C under nitrogen atmosphere for 2 hours. LCMS indicated the reaction was completed. The reaction mixture was concentrated under reduced pressure, and the residue was purified through silica gel column chromatography (petroleum ether:ethyl acetate = 1:1) to obtain Intermediate 59-3. LCMS: m/z 404.2 [M-55]⁺.

Step 4: The nitrogen atmosphere of a solution of Intermediate 59-3 (200 mg, 0.44 mmol) and palladium-carbon (20 mg, 0.19 mmol) in methanol (5 mL) was replaced by hydrogen three times at 25°C. The solution was stirred at 50°C under hydrogen atmosphere for 16 hours. LCMS indicated the reaction was completed. The mixture was cooled down to 25°C and filtered. The filtrate was concentrated under reduced pressure to obtain Intermediate 59-4. LCMS: m/z 464.3 [M+H]⁺.

Step 5: To a solution of Intermediate 59-4 (150 mg, 0.32 mmol) in dichloromethane (5 mL) was added trifluoroacetic acid (0.02 mL, 0.32 mmol) at 25°C, and the mixture was stirred for 2 hours. LCMS indicated the reaction was completed. The reaction was added with saturated sodium bicarbonate aqueous solution (5 mL) and stirred for 30 minutes. The reaction mixture was extracted with dichloromethane (10 mL×3). The combined organic phase was washed with saturated saline solution (30 mL) and concentrated under reduced pressure to obtain Intermediate 59-5. LCMS: m/z 364.2 [M+H]⁺.

Step 6: To a solution of Intermediate 59-5 (100 mg, 0.28 mmol) and potassium carbonate (114 mg, 0.84 mmol) in acetonitrile (5 mL) was added methanesulfonic anhydride (143 mg, 0.84 mmol) at 25°C, and the mixture was stirred for 2 hours. LCMS indicated the reaction was completed. The reaction mixture was filtered, and the filtrate was concentrated under reduced pressure. The residue was purified through preparative high-performance liquid chromatography (with ammonium bicarbonate) to obtain compound 59. LCMS: m/z 442.2 [M+H]⁺; ¹H NMR (400MHz, CDCl₃) δ: 7.83 (d, *J=* 7.1 Hz, 1H), 7.21 (s, 4H), 7.03 (d, *J =* 10.3 Hz, 1H), 3.93-4.05 (m, 6H), 3.82 (br d, *J* = 12.3 Hz, 2H), 2.83-2.90 (m, 2H), 2.78 (s, 3H), 2.61-2.72 (m, 2H), 1.83-1.95 (m, 2H), 1.63-1.75 (m, 2H), 1.32-1.47 (m,3H).

### Example 58: Compound 60

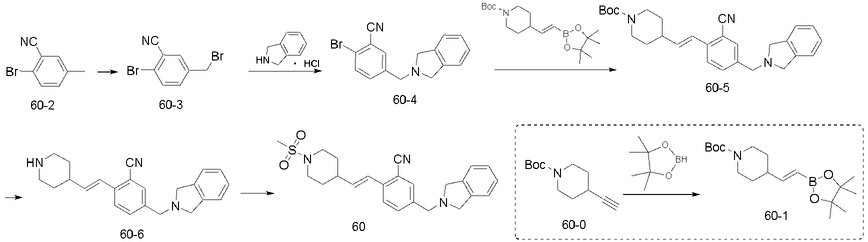

Step 1: A solution of starting material 60-0 (500 mg, 2.39 mmol), pinacolborane (257 mg, 2.03 mmol), zirconocene chloride hydride (61 mg, 0.24 mmol) in toluene (10 mL) was heated to 80°C and stirred for 6 hours. TLC indicated the reaction was completed. The reaction mixture was cooled down to 25°C, added with water (10 mL), and extracted with ethyl acetate (10 mL×3). The combined organic phase was washed with saturated saline solution (30 mL) and concentrated under reduced pressure to obtain Intermediate 60-1.

Step 2: To a solution of starting material 60-2 (3.20 g, 16.32 mmol) and N-bromosuccinimide (3.20 g, 17.95 mmol) in carbon tetrachloride (20 mL) was added azobisisobutyronitrile (270 mg, 1.63 mmol) at 60°C, and the mixture was stirred for 4 hours. TLC indicated the reaction was completed. The reaction mixture was cooled down to 25°C, quenched with water (20 mL), and extracted with ethyl acetate (30 mL×3). The combined organic phase was washed with saturated saline solution (100 mL) and concentrated under reduced pressure The residue was purified through silica gel column chromatography (petroleum ether:ethyl acetate = 5:1) to obtain Intermediate 60-3. ¹H NMR (400MHz, CDCl₃) δ: 7.65-7.70 (m, 2H), 7.49 (dd, *J =* 8.4, 2.3 Hz, 1H), 4.43 (s, 2H).

Step 3: To a solution of Intermediate 60-3 (3.00 g, 10.91 mmol) and triethylamine (4.54 ml, 32.73 mmol) in dichloromethane (15 mL) was added 2,3-dihydroisoindole hydrochloride (2.01 g, 13.09 mmol) at 25°C and the mixture was stirred for 4 hours. LCMS indicated the reaction was completed. The reaction was quenched with water (15 mL) and the mixture was extracted with ethyl acetate (15 mL×3). The combined organic phase was washed with saturated saline solution (50 mL) and concentrated under reduced pressure. The residue was purified through silica gel column chromatography (petroleum ether:ethyl acetate = 3:1) to obtain Intermediate 60-4. LCMS: m/z 313.0 [M+H]⁺.

Step 4: To a solvent mixture of 1,4-dioxane (10 mL) and water (3 mL) were added Intermediate 60-4 (300 mg, 1.09 mmol), tertbutyl (E)-4-(2-(4,4,5,5-tetramethyl-1,3,2-dioxaborolane-2-yl)vinyl)piperidine-1-carboxylate (552 mg, 1.64 mmol), tetra[triphenylphosphine]palladium (40 mg, 0.05 mmol), and potassium carbonate (452 mg, 3.27 mmol). After purging with nitrogen three times, the reaction was heated to 80°C and stirred for 16 hours. LCMS indicated the reaction was completed. The mixture was extracted with ethyl acetate (20 mL×3). The organic phases were combined, washed with saturated saline solution (60 mL) and concentrated under reduced pressure. The residue was purified through silica gel column chromatography (petroleum ether:ethyl acetate = 1:1) to obtain Intermediate 60-5. LCMS: m/z 444.3 [M+H]⁺.

Step 5: To a solution of Intermediate 60-5 (200 mg, 0.45 mmol) in dichloromethane (5 mL) was added trifluoroacetic acid (0.03 mL, 0.45 mmol) dropwise at 25°C, and the mixture was stirred for 2 hours. LCMS indicated the reaction was completed. The reaction mixture was added with saturated sodium bicarbonate aqueous solution (5 mL), stirred for 30 minutes, and extracted with dichloromethane (10 mL×3). The organic phases were combined, washed with saturated saline solution (30 mL) and concentrated under reduced pressure to obtain Intermediate 60-6 (150 mg). LCMS: m/z 344.2 [M+H]⁺.

Step 6: To a solution of Intermediate 60-6 (150 mg, 0.44 mmol) and potassium carbonate (182 mg, 1.32 mmol) in acetonitrile (5 mL) was added methanesulfonic anhydride (228 mg, 1.31 mmol) at 25°C, and the mixture was stirred for 2 hours. LCMS indicated the reaction was completed. The reaction mixture was concentrated under reduced pressure, and the residue was purified through preparative high-performance liquid chromatography (with ammonium bicarbonate) to obtain compound 60. LCMS: m/z 422.2 [M+H]⁺; ¹H NMR (400MHz, CDCl₃) δ: 7.67 (s, 1H), 7.60 (s, 2H), 7.15-7.24 (m, 4H), 6.79 (dd, *J=* 15.9, 0.9 Hz, 1H), 6.32 (dd, *J =* 15.9, 7.3 Hz, 1H), 3.90-3.99 (m, 6H), 3.87 (br d, *J =* 11.9 Hz, 2H), 2.71-2.83 (m, 5H), 2.38 (tdt, *J =* 11.1, 7.4,3.6 Hz, 1H), 1.94 (br dd, *J =* 13.1, 2.2 Hz, 2H), 1.51-1.72 (m, 2H).

### Example 59: Compound 61

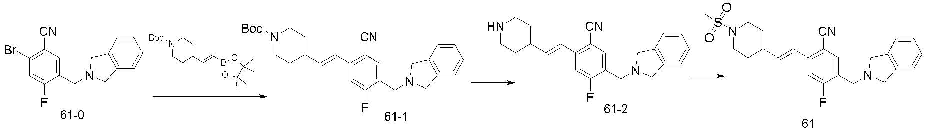

Step 1: Into a solvent mixture of 1,4-dioxane (5 mL) and water (1.5 mL) were dissolved starting material 61-0 (500 mg, 1.51 mmol), tertbutyl (E)-4-(2-(4,4,5,5-tetramethyl-1,3,2-dioxaborolane-2-yl)vinyl)piperidine-1-carboxylate (763 mg, 2.26 mmol), tetra[triphenylphosphine]palladium (55 mg, 0.08 mmol), and potassium carbonate(625 mg, 4.53 mmol). After purging with nitrogen three times, the reaction was heated to 80°C and stirred for 16 hours. LCMS indicated the reaction was completed. The reaction mixture was cooled down to 25°C, and extracted with ethyl acetate (8 mL×3). The organic phases were combined, washed with saturated saline solution (25 mL), and concentrated under reduced pressure. The residue was purified through silica gel column chromatography (petroleum ether:ethyl acetate= 1:1) to obtain Intermediate 61-1. LCMS: m/z 462.3 [M+H]⁺.

Step 2: To a solution of Intermediate 61-1 (250 mg, 0.54 mmol) in dichloromethane (5 mL) was added trifluoroacetic acid (0.5 mL, 6.71 mmol) dropwise at 25°C, and the mixture was stirred for 2 hours. TLC indicated the reaction was completed. The reaction mixture was added with saturated sodium bicarbonate aqueous solution (5 mL), stirred for 30 minutes, and extracted with dichloromethane (10 mL×3). The organic phases were combined, washed with saturated saline solution (30 mL), and concentrated under reduced pressure to obtain Intermediate 61-2.

Step 3: To a solution of Intermediate 61-2 (100 mg, 0.28 mmol) and potassium carbonate (116 mg, 0.84 mmol) in acetonitrile (5 mL) was added methanesulfonic anhydride (146 mg, 0.84 mmol) at 25°C, and the mixture was stirred for 2 hours. LCMS indicated the reaction was completed. The reaction mixture was filtered, and the filtrate was concentrated under reduced pressure. The residue was purified through preparative high-performance liquid chromatography (with ammonium bicarbonate) to obtain compound 61. LCMS: m/z 440.1 [M+H]⁺; ¹H NMR(400MHz, CDCl₃) δ: 7.82 (d, *J =* 7.0 Hz, 1H), 7.30 (d, *J=* 10.6 Hz, 1H), 7.21 (s, 4H), 6.75 (d, *J= 15.8* Hz, 1H), 6.32 (dd, *J=* 15.9, 7.3 Hz, 1H), 3.93-4.04 (m, 6H), 3.87 (br d, *J=* 12.0 Hz, 2H), 2.82 (s, 3H), 2.77 (td, *J=* 12.0, 2.4 Hz, 2H), 2.38 (tdt, *J=* 11.1, 7.3, 3.6 Hz, 1H), 1.94 (br dd, *J=* 13.1, 2.4 Hz, 2H), 1.55 -1.70 (m, 2H).

### Example 60: Compound 62

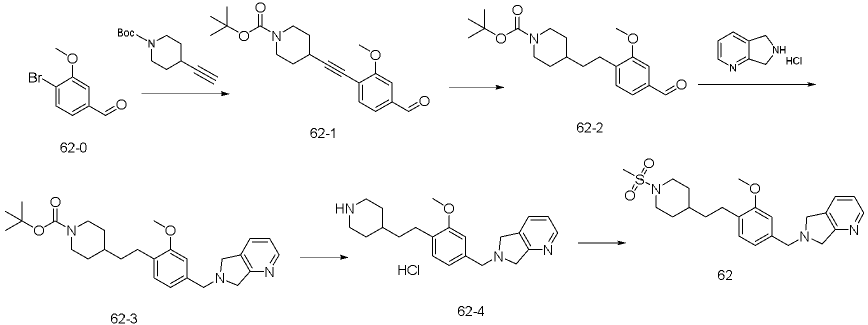

Step 1: Into tetrahydrofuran (50 mL) were added starting material 62-0 (10.00 g, 46.50 mmol), tertbutyl 4-ethynylpiperidine-1-carboxylate (14.60 g, 69.75 mmol), bis[triphenylphosphine]palladium dichloride (3.26 g, 4.65 mmol), cuprous iodide (890 mg, 4.65 mmol), and triethylamine (15 mL). After purging with nitrogen three times, the reaction was heated to 80°C and stirred for 2 hours. LCMS indicated the reaction was completed. The reaction mixture was cooled down to 25°C, washed with saturated saline solution (300 mL), and extracted with ethyl acetate (100 mL×3) The cobined organic phase was concentrated under reduced pressure and purified through silica gel column chromatography (petroleum ether:ethyl acetate = 1:10) to obtain Intermediate 62-1. LCMS: m/z 244.1 [M-99]⁺.

Step 2: To a solution of Intermediate 62-1 (2.00 g, 5.82 mmol) in isopropanol (5 mL) was added palladium-carbon (200 mg, 0.28 mmol) at 25°C under nitrogen atmosphere. After purging with hydrogen three times, the reaction was stirred for 30 minutes. LCMS indicated the reaction was completed. The reaction mixture was filtered, and the filtrate was concentrated to obtain Intermediate 62-2. LCMS: m/z 292.2 [M-55]⁺.

Step 3: A solution of Intermediate 62-2 (500 mg, 1.44 mmol), 6,7-dihydro-5H-pyrrolo[3, 4-b]pyridine hydrochloride (416 mg, 2.16 mmol), sodium borohydride acetate (910 mg, 4.32 mmol), and acetic acid (0.08 ml, 1.44 mmol) in 1,2-dichloroethane (10 mL) was stirred at 25°C for 4 hours. LCMS indicated the reaction was completed. The reaction mixture was washed with saturated saline solution (30 mL) and extracted with dichloromethane (10 mL×3). The combined organic phase was concentrated under reduced pressure and purified through silica gel column chromatography (petroleum ether:ethyl acetate = 1:3) to obtain Intermediate 62-3. LCMS: m/z 452.3 [M+H]⁺.

Step 4: To a solution of Intermediate 62-3 (300 mg, 0.66 mmol) in dioxane (5 mL) was added hydrochloride in dioxane (0.83 mL, 3.32 mmol, 4M) dropwise at 25°C, and the mixture was stirred for 2 hours. LCMS indicated the reaction was completed. The reaction mixture was concentrated under reduced pressure to obtain Intermediate 62-4. LCMS: m/z 352.3 [M+H]⁺.

Step 5: To a solution of Intermediate 62-4 (200 mg, 2.02 mmol), potassium carbonate (235 mg, 1.71 mmol) in acetonitrile (5 mL) was added methanesulfonic anhydride (297 mg, 1.71 mmol) at 25°C, and the mixture was stirred for 2 hours. LCMS indicated the reaction was completed. The reaction mixture was washed with saturated saline solution (30 mL) and extrated with ethyl acetate (10×3 mL). The organic phase was concentrated under reduced pressure and purified through preparative high-performance liquid chromatography (with ammonium bicarbonate) to obtain compound 62. LCMS: m/z 430.3 [M+H]⁺.¹H NMR (400MHz, CDCl₃) δ: 8.40 (d, J=4.8 Hz, 1H), 7.48 (d, J=7.4 Hz, 1H), 7.10-7.14 (m, 1H), 7.09 (d, J=7.8 Hz, 1H), 6.98 (br s, 1H), 6.90 (d, J=7.5 Hz, 1H), 4.04 (br d, J=17.3 Hz, 4H), 3.94 (s, 2H), 3.74-3.88 (m, 5H), 2.77 (s, 3H), 2.58-2.71 (m, 4H), 1.89 (br d, J=9.9 Hz, 2H), 1.50-1.61 (m, 2H), 1.30-1.47 (m, 3H).

### Example 61: Compound 63

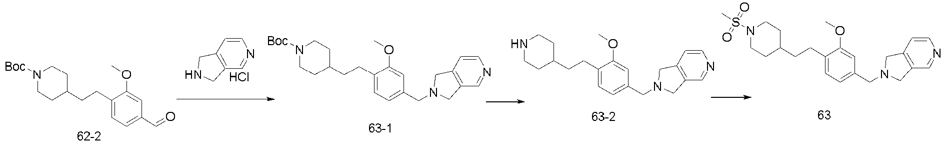

Step 1: A solution of Intermediate 62-2 (200 mg, 0.58 mmol), 2,3-dihydro-1H-pyrrolo[3,4-c]pyridine hydrochloride (182 mg, 1.16 mmol), sodium borohydride acetate (244 mg, 1.16 mmol), and acetic acid (34 mg, 0.58 mmol) in 1,2-dichloroethane (10 mL) was stirred at 25°C for 18 hours. LCMS indicated the reaction was completed. The reaction mixture was added with saturated sodium bicarbonate solution (10 mL) and extracted with dichloromethane (10 mL x 3). The combined organic phase was dried over anhydrous sodium sulfate and filtered. The filtrate was concentrated under reduced pressure, and the residue was purified through silica gel column chromatography (petroleum ether:ethyl acetate = 1:1) to obtain Intermediate 63-1. LCMS: m/z 452.1[M+H]⁺.

Step 2: To Intermediate 63-1(300 mg, crude) in dichloromethane (10 mL) was added trifluoroacetic acid (0.50 mL, 6.64 mmol) dropwise at 25°C, and the mixture was stirred for 20 minutes. LCMS indicated the reaction was completed. The mixture was added with saturated sodium bicarbonate solution (10 mL) and extracted with dichloromethane (10 mL×2). The combined organic phase was washed with saturated saline solution (20 mL), dried over anhydrous sodium sulfate, and filtered. The filtrate was concentrated to obtain Intermediate 63-2. LCMS: m/z 352.2 [M+H]⁺.

Step 3: To a solution of Intermediate 63-2 (270 mg, crude) and triethylamine (0.32 mL, 2.30 mmol) in dichloromethane (5 mL) was added methanesulfonic anhydride (267 mg, 1.54 mmol) at 25°C, and the mixture was stirred for 30 minutes. LCMS indicated the reaction was completed. The reaction was quenched with saturated ammonium chloride solution (10 mL) and extracted with dichloromethane (10 mL×2). The combined organic phase was washed with saturated saline solution (20 mL), dried over anhydrous sodium sulfate, and filtered. The filtrate was concentrated, and the residue was purified through preparative high-performance liquid chromatography (with ammonium bicarbonate) to obtain compound 63. LCMS: m/z 430.2 [M+H]⁺; ¹H NMR (400 MHz, CDCl₃) δ: 8.50 - 8.37 (m, 2H), 7.16 (d, *J=* 4.9 Hz, 1H), 7.08 (d, *J* = 7.5 Hz, 1H), 6.93 (s, 1H), 6.89 (d, *J =* 7.5 Hz, 1H), 3.98 (br d, *J =* 14.5 Hz, 4H), 3.90 (s, 2H), 3.84 (s, 3H), 3.80 (br d, *J =* 12.0 Hz, 2H), 2.77 (s, 3H), 2.71 - 2.51 (m, 4H), 1.89 (br d, *J =* 10.3 Hz, 2H), 1.63 - 1.54 (m, 2H), 1.44 - 1.29 (m, 3H).

### Example 62: Compound 64

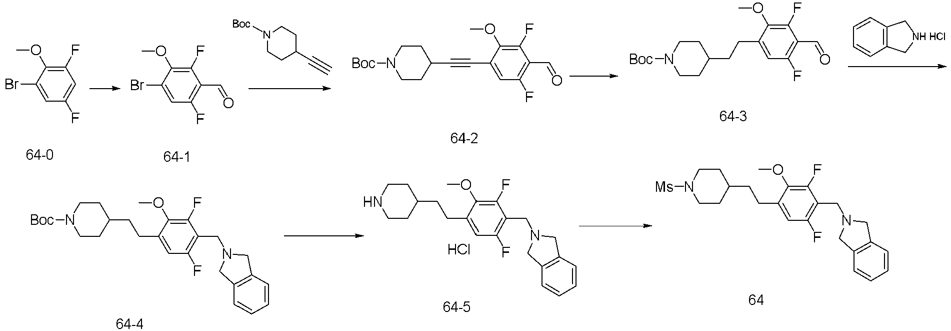

Step 1: To a solution of starting material 64-0 (3.00 g, 13.45 mmol) in tetrahydrofuran (30 mL) was slowly added a solution of lithium diisopropylamide in tetrahydrofuran (16.81 mL, 33.63 mmol) dropwise at -78°C and under nitrogen atmosphere, and after being stirred for 1 hour, N,N-dimethyl formamide (3.14 mL, 40.36 mmol) was slowly added dropwise and the mixture was stirred for 3 hours. Thin layer chromatography (petroleum ether:ethyl acetate = 5:1) indicated that the starting material had been completely consumed. The reaction was quenched with saturated ammonium chloride aqueous solution (20 mL) and stirred for 30 minutes. The mixture was heated to 25°C and washed with saturated saline solution (300 mL), and extracted with ethyl acetate (100 mL×3). The combined organic phase was concentrated under reduced pressure, and the residue was purified through silica gel column chromatography (petroleum ether:ethyl acetate = 10:1) to obtain Intermediate 64-1. ¹H NMR (400MHz, CDCl₃) δ: 10.29 (s, 1H), 7.27 (s, 1H), 3.93-4.02 (m, 3H).

Step 2: Into tetrahydrofuran (10 mL) were added Intermediate 64-1 (1.60 g, 6.37 mmol), tertbutyl 4-ethynylpiperidine-1-carboxylate (2.00 g, 9.56 mmol), bis(triphenylphosphine)palladium dichloride (450 mg, 0.64 mmol), cuprous iodide (120 mg, 0.64 mmol), and triethylamine (3 mL). After purging with nitrogen three times, the reaction was heated to 80°C and stirred for 2 hours. LCMS indicated the reaction was completed. The reaction mixture was cooled down to 25°C and concentrated under reduced pressure. The residue was purified through silica gel column chromatography (petroleum ether:ethyl acetate = 5:1) to obtain Intermediate 64-2. LCMS: m/z 324.0 [M-55]⁺.

Step 3: To a solution of Intermediate 64-2 (1.00 g, 2.64 mmol) in isopropanol (10 mL) was added palladium-carbon (100 mg, 0.26 mmol) at 25°C under nitrogen atmosphere. After purging with hydrogen three times, the reaction was stirred for 30 minutes. LCMS indicated the reaction was completed. The reaction mixture was filtered, and the filtrate was concentrated to obtain Intermediate 64-3. LCMS: m/z 328.1 [M-55]⁺.

Step 4: To a solution of Intermediate 64-3 (1.00 g, 2.61 mmol), isoindoline hydrochloride (600 mg, 3.91 mmol), and acetic acid (0.15 ml, 2.61 mmol) in 1,2-dichloroethane (10 mL) was added sodium borohydride acetate (1.65 g, 7.82 mmol) at 25°C, and the mixture was stirred for 4 hours. LCMS indicated the reaction was completed. The reaction was quenched with water (10 mL) and the mixture was extracted with dichloromethane (10 mL×3). The organic phases were combined, washed with saturated saline solution (30 mL), and concentrated under reduced pressure. The residue was purified through silica gel column chromatography (petroleum ether:ethyl acetate = 3:1) to obtain Intermediate 64-4. LCMS: m/z 487.2 [M+H]⁺.

Step 5: To a solution of Intermediate 64-4 (500 mg, 1.03 mmol) in dioxane (5 mL) was added hydrochloride in dioxane (5 mL, 0.32 mmol) dropwise at 25°C, and the mixture was stirred for 2 hours. LCMS indicated the reaction was completed. The reaction mixture was concentrated under reduced pressure to obtain Intermediate 64-5. LCMS: m/z 387.3 [M+H]⁺.

Step 6: Into acetonitrile (5 mL) were dissolved Intermediate 64-5 (300 mg, 0.78 mmol), potassium carbonate (323 mg, 2.34 mmol), and methanesulfonic anhydride (407 mg, 2.34 mmol) at 25°C, and the mixture was stirred for 2 hours. LCMS indicated the reaction was completed. The reaction mixture was extracted with ethyl acetate (10 mL×3). The organic phases were combined, washed with saturated saline solution (30 mL) and concentrated under reduced pressure. The residue was purified through preparative high-performance liquid chromatography (with ammonium bicarbonate) to obtain compound 64. LCMS: m/z 465.2 [M+H]⁺; ¹H NMR (400MHz, CDCl₃) δ: 7.17 (s, 4H), 6.70 (dd, *J =* 9.6, 1.8 Hz, 1H), 4.00 (s, 6H), 3.88 (d, *J =* 1.1 Hz, 3H), 3.80 (br d, *J =* 11.6 Hz, 2H), 2.77 (s, 3H), 2.56 - 2.68 (m, 4H), 1.86 (br d, *J =* 9.8 Hz, 2H), 1.50 - 1.59 (m, 2H), 1.32 - 1.44 (m, 3H).

### Example 63: Compound 65

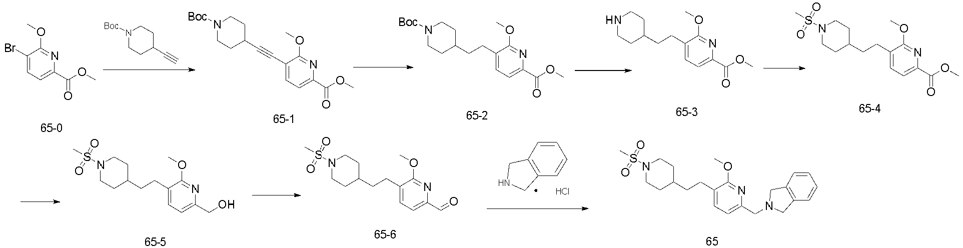

Step 1: Into tetrahydrofuran(10 mL) were added starting material 65-0 (1.01 g, 4.06 mmol), tertbutyl 4-ethynylpiperidine-1-carboxylate (1.70 g, 8.13 mmol), bis[triphenylphosphine]palladium dichloride (290 mg, 0.41 mmol), cuprous iodide (80 mg, 0.41 mmol) and triethylamine (5.6 mL). After purging with nitrogen three times, the reaction was stirred at 25°C for 1.5 hours. LCMS indicated the reaction was completed. The reaction mixture was filtered, and the filtrate was concentrated under reduced pressure. The residue was purified through silica gel column chromatography (petroleum ether:ethyl acetate = 5: 1) to obtain Intermediate 65-1. LCMS: m/z 375.2 [M+H]⁺.

Step 2: Into methanol (30 mL) were added Intermediate 65-1 (1.34 g, 3.57 mmol) and palladium/carbon (80 mg, 0.71 mmol) at 25°C under nitrogen atmosphere. After purging with hydrogen three times, the reaction was stirred at 25°C for 16 hours. LCMS indicated the reaction was completed. The reaction mixture was filtered, and the filtrate was concentrated under reduced pressure to obtain Intermediate 65-2. LCMS: m/z 323.2 [M-55]⁺.

Step 3: A hydrochloride-dioxane solution (10 mL, 4 M) was added to Intermediate 65-2 (1.01 g, 2.46 mmol) at 25°C, and the mixture was stirred for 10 minutes. LCMS indicated the reaction was completed. The reaction mixture was concentrated under reduced pressure to obtain Intermediate 65-3. LCMS: m/z 279.2 [M+H]⁺.

Step 4: Into dichloromethane (10 mL) were added Intermediate 65-3 (730 mg, 2.62 mmol), triethylamine (1.09 mL, 7.86 mmol) and methanesulfonic anhydride (685 mg, 3.93 mmol) at 25°C, and the mixture was stirred for 30 minutes. LCMS indicated the reaction was completed. The reaction mixture was concentrated under reduced pressure, and the residue was purified through silica gel column chromatography (petroleum ether:ethyl acetate = 1:1) to obtain Intermediate 65-4. LCMS: m/z 357.2 [M+H]⁺.

Step 5: Into tetrahydrofuran (6 mL) was dissolved Intermediate 65-4 (497 mg, 1.39 mmol). After purging with nitrogen three times, the reaction was slowly added with a solution of diisobutyl aluminum hydride (5.58 mL, 8.37 mmol, 1.5 M) in toluene dropwise at -78°C. The mixture was heated to 25°C and stirred for 1 hour. LCMS indicated the reaction was completed. The reaction was quenched with saturated potassium sodium tartrateaqueous solution (100 mL) and the mixture was extracted with ethyl acetate (100 mL×2). The combined organic phase was washed with saturated saline solution (100 mL), dried over anhydrous sodium sulfate, and filtered. The filtrate was concentrated under reduced pressure to obtain Intermediate 65-5. LCMS: m/z 329.3 [M+H]⁺.

Step 6: To a solution of Intermediate 65-5 (240 mg, 0.73 mmol) in dichloromethane (5 mL) was added manganese dioxide (634 mg, 7.3 mmol), and the mixture was stirred at 25°C for 16 hours. LCMS indicated the reaction was completed. The reaction mixture was filtered, and the filtrate was concentrated under reduced pressure to obtain Intermediate 65-6. LCMS: m/z 327.2[M+H]⁺.

Step 7: Into 1,2-dichloroethane (6 mL) were added Intermediate 65-6 (238 mg, 0.74 mmol), 2,3-dihydroisoindole hydrochloride (343 mg, 2.21 mmol) and acetic acid (0.04 mL, 0.74 mmol) at 25°C, and the mixture was stirred for 30 min. The reaction was added with sodium borohydride acetate (310 mg, 1.47 mmol), and continuously stirred for 1 hour. LCMS indicated the reaction was completed. The reaction was quenched with water and the mixture was extracted with ethyl acetate (30 mL×3). The combined organic phase was washed with saturated saline solution (50 mL), dried over anhydrous sodium sulfate, and filtered. The filtrate was concentrated under reduced pressure, and the residue was purified through silica gel column chromatography (ethyl acetate:petroleum ether = 1:1) to obtain a crude product, which was purified through preparative high-performance liquid chromatography (with ammonium bicarbonate) to obtain compound 65. LCMS: m/z 430.3 [M+H]⁺; ¹H NMR (400 MHz, CDCl₃) δ: 7.35 (d, *J* = 7.3 Hz, 1H), 7.20 (s, 4H), 6.98 (d, *J=* 7.3 Hz, 1H), 4.12 (s, 4H), 3.99 (s, 2H), 3.95 (s, 3H), 3.80 (br d, *J =* 11.8 Hz, 2H), 2.77 (s, 3H), 2.68 - 2.56 (m, 4H), 1.87 (br d, *J =* 9.9 Hz, 2H), 1.61 - 1.53 (m, 2H), 1.43 - 1.33 (m, 3H).

### Example 64: Compound 66

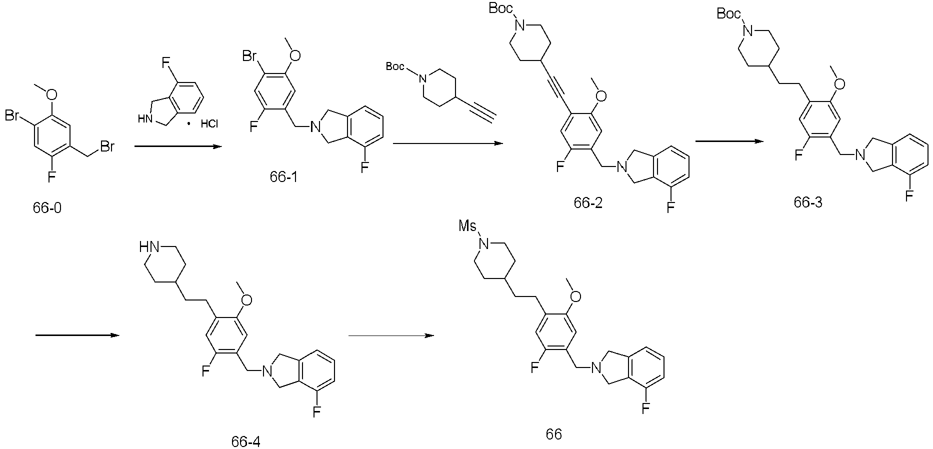

Step 1: A soltuion of starting material 66-0 (2.00 g, 6.71 mmol), 4-fluoro isoindoline hydrochloride (1.75 g, 10.07 mmol) and triethylamine (2.79 mL, 20.14 mmol) in acetonitrile (10 mL) was stirred at 25°C for 24 hours. The reaction mixture was filtered, and the filtrate was concentrated under reduced pressure. The residue was purified through silica gel column chromatography (petroleum ether:ethyl acetate= 2:1) to obtain Intermediate 66-1. LCMS: 354.0 [M+H]+.

Step 2: To a solvent mixture of triethylamine (2 mL) and tetrahydrofuran (15 mL) were added Intermediate 66-1 (1.00 g, 2.82 mmol), bis[triphenylphosphine]palladium dichloride (198 mg, 0.28 mmol), tertbutyl 4-ethynylpiperidine-1-carboxylate (1.18 g, 5.65 mmol), and cuprous iodide (54 mg, 0.28 mmol). After purging with nitrogen for three times, the reaction was heated to 80°C and stirred for 18 hours. The reaction mixture was filtered, and the filtrate was concentrated under reduced pressure. The residue was purified through silica gel column chromatography (petroleum ether:ethyl acetate = 5:1) to obtain Intermediate 66-2. LCMS: m/z 483.2 [M+H]+.

Step 3: Into methanol (10 mL) were dissolved Intermediate 66-2 (310 mg, 0.64 mmol) and palladium-carbon (7 mg, 0.05 mmol, 10wt %) at 25°C under nitrogen atmosphere. After purging with hydrogen for three times, the reaction was heated to 50°C and stirred for 18 hours. LCMS indicated the reaction was completed. The mixture was filtered, and the filtrate was concentrated under reduced pressure to obtain Intermediate 66-3. LCMS: m/z 487.3 [M+H]+.

Step 4: To a solution of Intermediate 66-3 (250 mg, 0.51 mmol) in dichloromethane (5 mL) was added trifluoroacetic acid (0.04 mL, 0.51 mmol) dropwise at 25°C, and the mixture was stirred for 1 hour. The reaction mixture added to saturated sodium bicarbonate solution (10 mL) and extracted with dichloromethane (10 mLx3). The organic phases were combined, washed with saturated sodium chloride solution (30 mL), dried over anhydrous sodium sulfate, and filtered. The filtrate was concentrated under reduced pressure to obtain Intermediate 66-4.

Step 5: To a solution of Intermediate 66-4 (80 mg, 0.21 mmol) and triethylamine (0.04 mL, 0.31 mmol) in dichloromethane (5 mL) was added methanesulfonic anhydride (36 mg, 0.21 mmol) at 25°C, and the mixture was stirred for 2 hours. LCMS indicated the reaction was completed. The reaction mixture was added with saturated ammonium chloride solution (10 mL) and extracted with dichloromethane (10 mLx3). The organic phases were combined, washed with saturated sodium chloride solution (10 mL), dried over anhydrous sodium sulfate, and filtered. The filtrate was concentrated under reduced pressure, and the residue was purified through high-performance liquid chromatography (with ammonium bicarbonate) to obtain compound 66. LCMS: m/z 465.2 [M+H]+; 1H NMR (400MHz, CDCl₃) δ: 7.18 (dt, J = 5.2, 7.8 Hz, 1H), 6.97 (d, J = 7.4 Hz, 1H), 6.92 (d, J = 6.0 Hz, 1H),6.90 - 6.87 (m, 1H), 6.84 (d, J = 10.3 Hz, 1H), 4.03 (br d, J = 10.8 Hz, 4H), 3.94 (d, J = 0.8 Hz, 2H), 3.81 (s, 3H), 3.79 (br s, 2H), 2.77 (s, 3H), 2.71 - 2.57 (m, 4H), 1.88 (br d, J = 9.8 Hz, 2H), 1.64 - 1.52 (m, 2H), 1.43 - 1.33 (m, 3H)

### Example 65: Compound 67

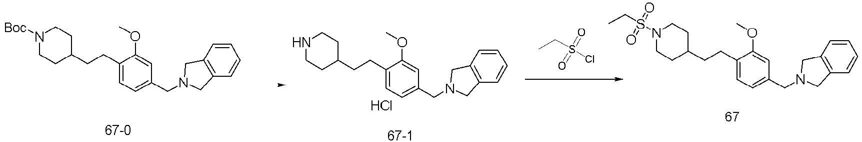

Step 1: To a solution of 67-0 (150 mg, 0.33 mmol) in dioxane (1 mL) was added 1,4-dioxane solution of hydrochloride (1 mL, 4 mol/L) at 25°C, and the mixture was stirred for 1 hour. LCMS indicated the reaction was completed. The reaction mixture was concentrated under reduced pressure to obtain Intermediate 67-1. LCMS: m/z 351.2 [M+H]+.

Step 2: To a solution of Intermediate 67-1 (120 mg, 0.31 mmol) and triethylamine (167 mg, 1.65 mmol) in tetrahydrofuran (3 mL) was added ethanesulfonyl chloride (64 mg, 0.49 mmol) at 25°C, and the mixture was stirred for 2 hours. LCMS indicated the reaction was completed. The reaction was quenched with water (30 mL) and the mixture was extracted with ethyl acetate (30 mL×3). The combined organic phase was washed with saturated saline solution (30 mL), dried over anhydrous sodium sulfate, and filtered. The filtrate was concentrated under reduced pressure, and the residue was purified through preparative high-performance liquid chromatography (with ammonium bicarbonate) to obtain compound 67. LCMS: m/z 443.3 [M+H]+: 1H NMR (400 MHz, CDCl3) δ: 7.20 (s, 4H), 7.08 (d, J = 7.5 Hz, 1H), 7.02 (s, 1H), 6.90 (d, J = 7.4 Hz, 1H),4.06 - 3.90 (m, 6H), 3.84 (s, 3H), 3.82 (s, 1H), 3.79 (s, 1H), 2.99 - 2.91 (m, 2H), 2.82 - 2.72 (m, 2H), 2.67 - 2.59 (m, 2H),1.86 (dd, J = 2.0, 12.6 Hz, 2H), 1.61 - 1.53 (m, 3H), 1.39 (s, 1H), 1.38 - 1.36 (m, 2H), 1.35 (s, 1H), 1.34 (d, J = 3.8 Hz, 1H).

### Example 66: Compound 68

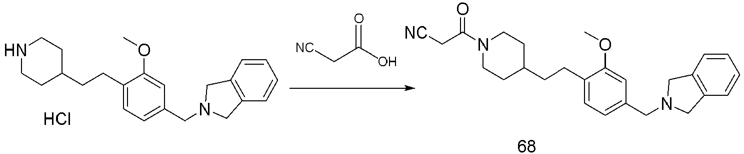

Into tetrahydrofuran (5 mL) were added 2-cyanoacetic acid (300 mg, 3.53 mmol) and N,N'-carbonyldiimidazole (514 mg, 3.17 mmol) at 25°C, and the mixture was stirred for 1 hour. The reaction mixture was added with N,N-dimethyl formamide (2 mL) and 2-(3-methoxy-4-(2-(piperidin-4-yl)ethyl)benzyl)isoindoline hydrochloride (200 mg, 0.57 mmol), and continuously stirred for 1 hour. LCMS indicated the reaction was completed. The reaction mixture was washed with water (20 mL) and extracted with dichloromethane (30 mL×2). The combined organic phase was washed with saturated saline solution (50 mL), dried over anhydrous sodium sulfate, and filtered. The filtrate was concentrated under reduced pressure, and the residue was purified through thin layer chromatography (dichloromethane: methanol= 20:1) to obtain a crude product, which was purified through preparative high-performance liquid chromatography (with ammonium bicarbonate) to obtain compound 68. LCMS: m/z 418.3 [M+H]⁺; ¹H NMR (400 MHz, CDCl₃) δ: 7.20 (s, 4H), 7.07 (d, *J=* 7.5 Hz, 1H), 6.97 (s, 1H), 6.91 (d, *J =* 7.5 Hz, 1H), 4.60 - 4.49 (m, 1H), 3.96 (s, 4H), 3.90 (s, 2H), 3.84 (s, 3H), 3.70 (br d, *J =* 13.9 Hz, 1H), 3.49 (d, *J =* 1.8 Hz, 2H), 3.14 (dt, *J=* 2.6, 13.0 Hz, 1H), 2.71 - 2.61 (m, 3H), 1.88 (br t, *J=* 15.0 Hz, 2H), 1.57 (br t, *J =* 6.0 Hz, 3H), 1.30 - 1.20 (m, 2H).

### Example 67: Compound 69

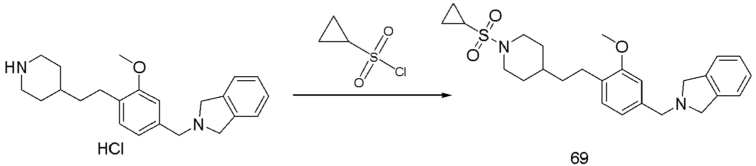

Into tetrahydrofuran (5 mL) were added 2-(3-methoxy-4-(2-(piperidin-4-yl)ethyl)benzyl)isoindoline hydrochloride (120 mg, 0.34 mmol), triethylamine (0.14 mL, 1.02 mmol) and cyclopropane sulfonyl chloride (0.05 mL, 0.51 mmol) at 25°C, and the mixture was stirred at 25°C for 1 hour. LCMS indicated the reaction was completed. The reaction mixture was concentrated under reduced pressure, and the residue was purified through thin layer chromatography (petroleum ether:ethyl acetate= 1: 1) to obtain a crude product, which was purified through preparative high-performance liquid chromatography (with ammonium bicarbonate) to obtain compound 69. LCMS: m/z 455.3 [M+H]⁺; ¹H NMR (400 MHz, CDCl₃) δ: 7.19 (s, 4H), 7.08 (d, *J =* 7.5 Hz, 1H), 6.97 (s, 1H), 6.91 (d, *J =* 7.5 Hz, 1H), 3.96 (s, 4H), 3.90 (s, 2H), 3.84 (s, 3H), 3.80 (br d, *J =* 12.1 Hz, 2H), 2.85 - 2.75 (m, 2H), 2.68 - 2.59 (m, 2H), 2.26 (tt, *J* = 4.8, 8.1 Hz, 1H), 1.87 (br d, *J* = 11.1 Hz, 2H), 1.57 (td, *J =* 6.1, 9.5 Hz, 2H), 1.42 - 1.31 (m, 3H), 1.21 - 1.14 (m, 2H), 1.01 - 0.93 (m, 2H).

### Example 68: Compound 70

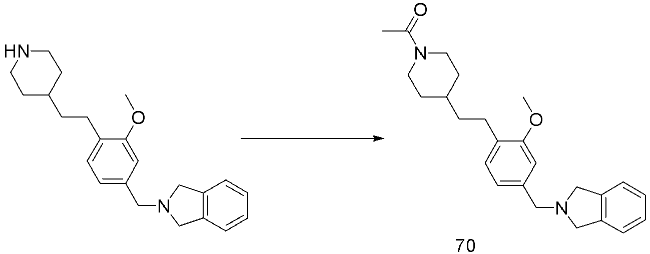

Into dichloromethane (5 mL) were dissolved 2-(3-methoxy-4-(2-(piperidin-4-yl)ethyl)benzyl)isoindoline (300 mg, 0.86 mmol), triethylamine (0.36 mL, 2.58 mmol), and acetyl chloride (0.18 mL, 1.29 mmol) at 25°C, and the mixture was stirred for 3 hours. LCMS indicated the reaction was completed. The reaction mixture wasquenched with water (5 mL) and extracted with ethyl acetate (5 mL×3). The organic phases were combined, washed with saturated saline solution (15 mL), and concentrated under reduced pressure. The residue was purified through preparative high-performance liquid chromatography (with ammonium bicarbonate) to obtain compound 70. LCMS: m/z 393.2 [M+H]⁺; ¹H NMR (400MHz, CDCl₃) δ: 7.19 (s, 4H), 7.08 (d, *J =* 7.5 Hz, 1H), 6.95 (s, 1H), 6.90 (d, *J* = 7.5 Hz, 1H), 4.55-4.65 (m, 1H), 3.95 (s, 4H), 3.89 (s, 2H), 3.77 - 3.85 (m, 4H), 3.03 (td, *J =* 12.9, 2.7 Hz, 1H), 2.60 - 2.68 (m, 2H), 2.56 (td, *J=* 12.8, 2.7 Hz, 1H), 2.10 (s, 3H), 1.78 - 1.88 (m, 2H), 1.50 - 1.60 (m, 3H), 1.07 - 1.24 (m, 2H).

### Example 69: Compound 71

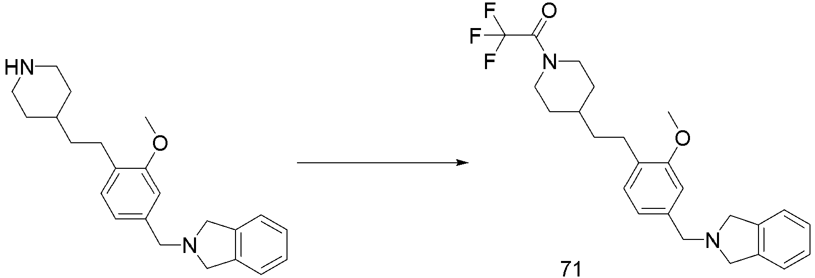

Into dichloromethane (5 mL) were dissolved 2-(3-methoxy-4-(2-(piperidin-4-yl)ethyl)benzyl)isoindoline (100 mg, 0.29 mmol), triethylamine (0.20 mL, 1.43 mmol), trifluoroacetic anhydride (0.08 mL, 0.58 mmol) at 25°C, and the mixture was stirred for 3 hours. LCMS indicated the reaction was completed. The reaction was quenched with water (5 mL) and the mixture was extracted with ethyl acetate (5 mL×3). The organic phases were combined, washed with saturated saline solution (15 mL), and concentrated under reduced pressure. The residue was purified through preparative high-performance liquid chromatography (with ammonium bicarbonate) to obtain compound 71. LCMS: m/z 447.2 [M+H]⁺; ¹H NMR (400MHz, CDCl₃) δ: 7.19 (s, 4H), 7.07 (d, *J =* 7.5 Hz, 1H), 6.96 (s, 1H), 6.91 (d, *J=* 7.5 Hz, 1H), 4.53 (br d, *J =* 13.4 Hz, 1H), 4.01 (br d, *J =* 13.4 Hz, 1H), 3.95 (s, 4H), 3.89 (s, 2H), 3.84 (s, 3H), 3.06 - 3.17 (m, 1H), 2.77 (br t, *J=* 12.2 Hz, 1H), 2.60 - 2.69 (m, 2H), 1.91 (br d, *J* = 13.4 Hz, 2H), 1.51 - 1.61 (m, 2H), 1.18 - 1.33 (m, 3H).

### Example 70: Compound 72

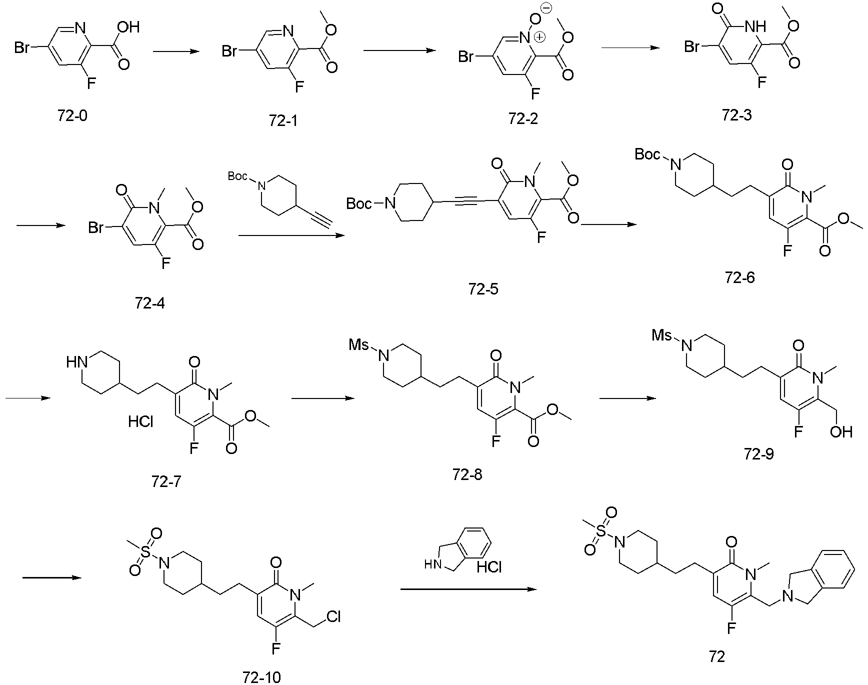

Step 1: To a solution of Intermediate 72-0 (4.40 g, 20.09 mmol) in methanol (30 mL) was added concentrated sulfuric acid (0.50 mL) at 25°C. The mixture was heated to 80°C and stirred for 12 hours. LCMS indicated the reaction was completed. The reaction mixture was cooled down to 25°C, concentrated under reduced pressure, neutralized with saturated sodium bicarbonate solution (50 mL), and extracted with ethyl acetate (50 mL×3). The combined organic phase washed with was saturated saline solution (50 mL), dried over anhydrous sodium sulfate, and filtered. The filtrate was concentrated under reduced pressure to obtain Intermediate 72-1. LCMS: m/z 234.2 [M+H]⁺.

Step 2: To a solution of Intermediate 72-1 (4.00 g, 17.16 mmol) and trifluoroacetic anhydride (10.80 g, 51.42 mmol) in dichloromethane (60 mL) was added hydrogen peroxide solution (5.30 g, 33wt %) at 25°C, and the mixture was stirred for 18 hours. LCMS indicated the reaction was completed. The reaction was quenched by adding saturated sodium bicarbonate solution (50 mL) followed by saturated sodium sulfite solution (50 mL) and the mixture was extracted with dichloromethane (100 mL×3). The combined organic phase was washed with saturated saline solution (100 mL), dried over anhydrous sodium sulfate, and filtered. The filtrate was concentrated under reduced pressure, and the residue was purified through silica gel column chromatography (petroleum ether:ethyl acetate = 3:1) to obtain Intermediate 72-2. LCMS: m/z 249.9 [M+H]⁺.

Step 3: To a solution of Intermediate 72-2 (3.00 g, 12.04 mmol) in N,N-dimethyl formamide (50 mL) was added trifluoroacetic anhydride (5.06 g, 24.09 mmol) at 0°C. The mixture was heated to 50°C and stirred for 1 hour. LCMS indicated the reaction was completed. The reaction was quenched with water (100 mL) and the mixture was extracted with ethyl acetate (100 mL×3). The combined organic phase was washed with saturated sodium bicarbonate solution (100 mL), and further washed with saturated saline solution (100 mL), dried over anhydrous sodium sulfate, and filtered. The filtrate was concentrated under reduced pressure, and the residue was purified by slurring in solvent (petroleum ether:ethyl acetate = 8:1) to obtain Intermediate 72-3. LCMS: m/z 249.8 [M+H]⁺.

Step 4: To a solution of Intermediate 72-3 (1.50 g, 6.02 mmol) and methyl iodide (2.56 g, 18.02 mmol) in N,N-dimethyl formamide (10 mL) was added potassium carbonate (1.66 g, 12.02 mmol) at 25°C. The reaction mixture was stirred at 25°C for 3 hours. LCMS indicated the reaction was completed. The reaction was quenched with water (50 mL) and the mixture was extracted with ethyl acetate (50 mL×3). The combined organic phase was washed with saturated saline solution (50 mL), dried over anhydrous sodium sulfate, and filtered. The filtrate was concentrated under reduced pressure, and the residue was purified through silica gel column chromatography (petroleum ether:ethyl acetate = 1:1.5) to obtain Intermediate 72-4. LCMS: m/z 263.9 [M+H]⁺.

Step 5: Into tetrahydrofuran (10 mL) were added Intermediate 72-4 (500 mg, 1.89 mmol), tertbutyl 4-ethynylpiperidine-1-carboxylate (792 mg, 3.79 mmol), cuprous iodide (36 mg, 0.19 mmol) and triethylamine (1.31 mL, 9.47 mmol) at 25°C, followed by bis(triphenylphosphine)palladium dichloride (133 mg, 0.19 mmol). After purging with nitrogen three times, the reaction was stirred for 5 hours. LCMS indicated the reaction was completed. The reaction mixture was filtered, and the filtrate was concentrated under reduced pressure. The residue was purified through purified through silica gel column (petroleum ether:ethyl acetate = 1:1) to obtain Intermediate 72-5. LCMS: m/z 393.2 [M+H]⁺.

Step 6: To a solution of Intermediate 72-5 (450 mg, 1.15 mmol) in methanol (10 mL) was added palladium/carbon (122 mg, 0.11 mmol, 10wt %) at 25°C under nitrogen atmosphere, and after purging with hydrogen three times, the reaction was stirred for 18 hours. LCMS indicated the reaction was completed. The reaction mixture was filtered, and the filtrate was concentrated under reduced pressure to obtain Intermediate 72-6. LCMS: m/z 297.2 [M-99]⁺.

Step 7: A solution of Intermediate 72-6 (430 mg, 1.08 mmol) in hydrochloride-dioxane (10 mL, 4M/L) was stirred at 25°C for 20 minutes. LCMS indicated the reaction was completed. The reaction mixture was concentrated under reduced pressure to obtain Intermediate 72-7. LCMS: m/z 297.2 [M+H]⁺.

Step 8: To a solution of Intermediate 72-7 (350 mg, 1.18 mmol) and triethylamine (0.49 mL, 3.54 mmol) in dichloromethane (10 mL) was added methanesulfonic anhydride (308 mg, 1.77 mmol) at 25°C and stirred for 20 minutes. LCMS indicated the reaction was completed. The reaction was quenched with saturated ammonium chloride solution (10 mL) and extracted with dichloromethane (10 mL×2). The combined organic phase was washed with saturated saline solution (20 mL), dried over anhydrous sodium sulfate, and filtered. The filtrate was concentrated to obtain Intermediate 72-8. LCMS: m/z 375.2 [M+H]⁺.

Step 9: To a solution of Intermediate 72-8 (110 mg, 0.29 mmol) in tetrahydrofuran (5 mL) were added sodium borohydride (111 mg, 2.94 mmol) and lithium chloride (124 mg, 2.94 mmol) at 25°C, and the mixture was stirred for 4 hours. LCMS indicated the reaction was completed. The reaction was quenched with water (10 mL) and extracted with dichloromethane (10 mL×2). The combined organic phase was washed with saturated saline solution (20 mL), dried over anhydrous sodium sulfate, and filtered. The filtrate was concentrated to obtain Intermediate 72-9. LCMS: m/z 347.0 [M+H]⁺.

Step 10: To a solution of Intermediate 72-9 (40 mg, 0.12 mmol) in dichloromethane (3 mL) was added thionyl chloride (68 mg, 0.58 mmol) at 25°C, and the mixture was stirred for 30 minutes. LCMS indicated the reaction was completed. The reaction mixture was concentrated to obtain Intermediate 72-10. LCMS: m/z 365 [M+H]⁺.

Step 11: A solution of potassium carbonate (33 mg, 0.24 mmol), Intermediate 72-10 (43 mg, 0.12 mmol) in N,N-dimethyl formamide (3 mL) was heated to 80°C and stirred for 30 minutes. LCMS indicated the reaction was completed. The reaction mixture was filtered, and the filtrate was purified through preparative high-performance liquid chromatography (with ammonium bicarbonate) to obtain compound 72. LCMS: m/z 448.2 [M+H]⁺; ¹H NMR (400 MHz, CDCl₃) δ: 7.25 - 7.18 (m, 4H), 7.15 (d, *J* = 9.0 Hz, 1H), 3.99 (s, 4H), 3.91 (d, *J* = 4.1 Hz, 2H), 3.81 (br d, *J* = 12.0 Hz, 2H), 3.73 (s, 3H), 2.78 (s, 3H), 2.71 - 2.56 (m, 4H), 1.96 - 1.83 (m, 2H), 1.69 - 1.55 (m, 3H),1.42 - 1.31 (m, 2H).

### Example 71: Compound 73

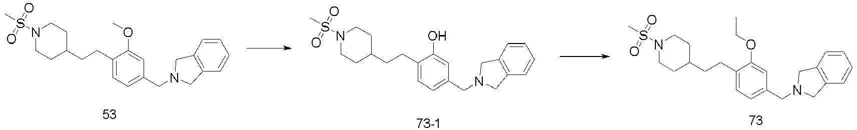

Step 1: Boron tribromide (176 mg, 0.70 mmol) was added to a solution of compound 53 (100 mg, 0.23 mmol) in dichloromethane (2 mL) at 25 °C. The mixture was stirred for 12 hours. LCMS indicated the reaction was completed. The reaction mixture was quenched by adding saturated sodium bicarbonate solution (5 mL) and water (25 mL) and extracted with dichloromethane (30 mL×3). The organic phases were combined, washed with saturated brine (30 mL), anhydrous, dried over sodium sulfate, and filtered. The filtrate was concentrated under reduced pressure to obtain crude compound 73-1. LCMS: m/z 415.2 [M+H]⁺.

Step 2: Compound 73-1 (100 mg, 0.24 mmol), anhydrous ethanol (55 mg, 1.19 mmol), diisopropyl azodicarboxylate (98 mg, 0.48 mmol) and triphenylphosphine (126 mg, 0.48 mmol) were added to dichloromethane (2.5 mL). After purging with nitrogen three times, the reaction mixture was stirred at 25 °C for 18 hours. LCMS indicated the reaction was completed. The reaction mixture was filtered, the filtrate was concentrated under reduced pressure, and the residue was purified by preparative high-performance liquid chromatography (ammonium bicarbonate conditions) to obtain compound 73. LCMS: m/z 443.2 [M+H]⁺;¹H NMR (400 MHz, CDCl₃) δ : 7.26 - 7.17 (m, 4H), 7.09 (d, *J=* 7.5 Hz, 2H), 6.88 (dd, *J =* 1.3, 7.5 Hz, 1H),4.18 - 4.00 (m, 6H), 3.97 (s, 2H), 3.79 (d, *J =* 11.9 Hz, 2H), 2.78 (s, 3H), 2.69 - 2.62 (m, 4H), 1.89 (d, *J* = 10.5 Hz, 2H), 1.58(td, *J=* 1.9, 4.0 Hz, 2H), 1.43 (s, 1H), 1.41 (s, 2H), 1.40 (s, 1H), 1.39 - 1.36 (m, 2H).

### Example 72: Compound 74

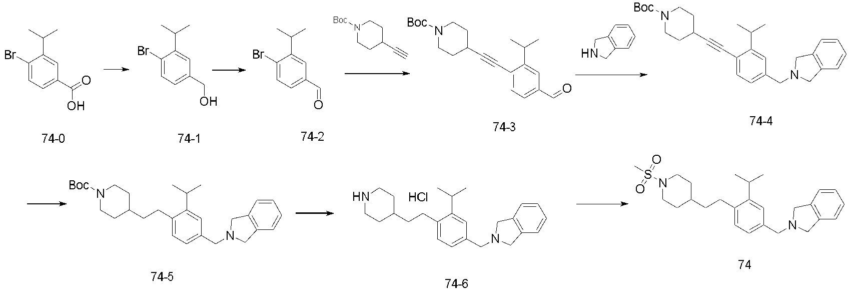

Step 1: Borane-tetrahydrofuran complex (6.12 mL, 6.12 mmol, 1M) was added dropwise into a solution of starting material 74-0 (500 mg, 2.06 mmol) in tetrahydrofuran (10 mL) at 25 °C, and the mixture was stirred for 2 hours. TLC indicated the reaction was completed The reaction mixture was quenched by adding methanol (10 mL) and concentrated under reduced pressure to obtain Intermediate 74-1.

Step 2: Manganese dioxide (3.40 g, 39.28 mmol) was added to a solution of Intermediate 74-1 (450 mg, 1.96 mmol) in dichloromethane (10 mL) at 25 °C, and the mixture was stirred for 1 hour. TLC indicated the reaction was completed. The reaction mixture was filtered, and the filtrate was concentrated under reduced pressure to obtain Intermediate 74-2.

Step 3: Intermediate 74-2 (400 mg, 1.76 mmol), tertbutyl 4-ethynylpiperidine-1-carboxylate (737 mg, 3.52 mmol), copper iodide (34 mg, 0.18 mmol), bis(triphenylphosphine) palladium dichloride (124 mg, 0.18 mmol) and triethylamine (1.22 mL) were added to tetrahydrofuran (5 mL) at 25°C. After purging with nitrogen three times, the mixture was stirred for 7 hours. LCMS indicated the reaction was completed. The reaction mixture was filtered, and the filtrate was concentrated under reduced pressure. The residue was purified by silica gel column chromatography (petroleum ether: ethyl acetate = 5: 1) to obtain Intermediate 74-3. LCMS: m/z 300.1 [M-55]⁺.

Step 4: At 25 °C, Intermediate 74-3 (300 mg, 0.84 mmol), 2,3-dihydroisoindole hydrochloride (259 g, 1.69 mmol) and acetic acid (0.05 mL, 0.05 mmol) were added into 1, 2-dichloroethane (5 mL), and the mixture was stirred for 30 min. Sodium borohydride acetate (356 mg, 1.69 mmol) was added and the mixture was stirred continuously for 5 hours. LCMS indicated the reaction was completed. The reaction mixture was quenched with water (20 mL) and extracted with ethyl acetate (20 mL×3). The organic phases were combined, washed with saturated brine (20 mL), dried over anhydrous sodium sulfate, and filtered. The filtrate was concentrated under reduced pressure, and the residue was purified by silica gel column chromatography (ethyl acetate:petroleum ether = 5:1) to obtain Intermediate 74-4. LCMS: m/z 459.3 [M+H]⁺.

Step 5: At 25°C, under nitrogen atmosphere, palladium/carbon (4 mg, 0.039 mmol, 10wt %) was added to a solution of Intermediate 74-4 (280 mg, 0.62 mmol) in methanol (4 mL). After purging with hydrogen three times, the mixture was stirred for 1 hour. LCMS indicated the reaction was completed. The reaction mixture was filtered, and the filtrate was concentrated under reduced pressure to obtain Intermediate 74-5. LCMS: m/z 463.4 [M+H]⁺.

Step 6: Hydrochloric acid-dioxane solution (1.50 mL, 4 M) was added to Intermediate 74-5 (198 mg, crude) at 25 °C and the mixture was stirred for 20 minutes. LCMS indicated the reaction was completed. The reaction mixture was concentrated under reduced pressure to obtain Intermediate 74-6. LCMS: m/z 363.2 [M+H]⁺.

Step 7: Methylsulfonic anhydride (73 mg, 0.42 mmol) was added to a solution of Intermediate 74-6 (100 mg, crude) and triethylamine (0.83 mL, 0.83 mmol) in dichloromethane (4 mL) at 25 °C and the mixture was stirred for 20 minutes. LCMS indicated the reaction was completed. The reaction mixture was quenched by adding water (15 mL)and extracted with ethyl acetate (15 mL×3). The organic phases were combined, washed with saturated brine (15 mL), dried over anhydrous sodium sulfate, and filtered. The filtrate was concentrated under reduced pressure, and the residue was purified by preparative high-performance liquid chromatography (with ammonium bicarbonate) to obtain compound 74. LCMS: m/z 441.3 [M+H]⁺; ¹H NMR (400 MHz, CDCl₃) *δ:* 7.33 (s, 1H), 7.20 (s, 4H), 7.18 - 7.18 (m, 1H), 7.18 - 7.12 (m, 1H), 4.02 (s, 4H), 3.94 (s, 2H), 3.82 (br d, *J =* 12.0 Hz, 2H), 3.17 - 3.10 (m, 1H), 2.78 (s, 3H), 2.71 - 2.66 (m, 4H), 1.89 (br d, *J =* 11.0 Hz, 2H), 1.61 - 1.53 (m, 2H), 1.45 - 1.32 (m, 3H), 1.26 (d, *J =* 6.8 Hz, 6H).

### Example 73: Compound 75

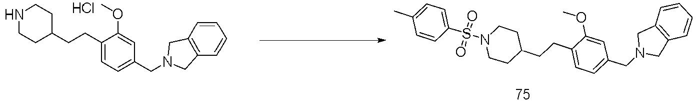

At 25°C, p-toluenesulfonyl chloride (79.45 mg, 0.43 mmol) was added to a solution of 2-(3-methoxy-4-(2-(piperidin-4-yl)ethyl)benzyl)isoindoline hydrochloride (100 mg, crude) and triethylamine (0.12 mL, 0.87 mmol) in dichloromethane (1 mL), and the mixture was stirred for 1 hour. LCMS indicated the reaction was completed. The reaction mixture was quenched with water (20 mL)and extracted with ethyl acetate (20 mL×3). The organic phases were combined, washed with saturated brine (20 mL), and dried over anhydrous sodium sulfate. After filtration, the filtrate was concentrated under reduced pressure, and the residue was purified by preparative high-performance liquid chromatography (ammonium bicarbonate conditions) to obtain compound 75. LCMS: m/z 505.2 [M+H]⁺; ¹H NMR (400 MHz, CDCl₃) *δ*: 7.65 (d, *J =* 8.1 Hz, 2H), 7.32 (br d, *J =* 8.0 Hz, 2H), 7.25 - 7.14 (m, 4H), 7.03 (d, *J =* 7.5 Hz, 1H), 6.98 (s, 1H), 6.87 (br d, *J* = 7.4 Hz, 1H), 3.99 (s, 4H), 3.91 (s, 2H), 3.81 (s, 3H), 3.76 (br d, *J=* 11.6 Hz, 2H), 2.62 - 2.53 (m, 2H), 2.44 (s, 3H), 2.29 - 2.18 (m, 2H), 1.81 (br d, *J=* 11.6 Hz, 2H), 1.56 - 1.47 (m, 2H), 1.42 - 1.28 (m,3H).

### Example 74: Compound 76

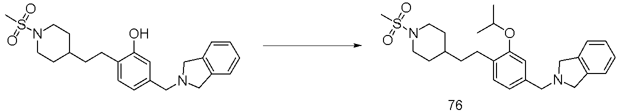

5-(isoindolin-2-ylmethyl)-2-(2-(1-(methylsulfonyl)piperidin-4-yl)ethyl)phenol (40 mg, 0.09 mmol), isopropanol (29 mg, 0.48 mmol), diisopropyl azodicarboxylate (39 mg, 0.19 mmol) and triphenylphosphine (50 mg, 0.19 mmol) were added to dichloromethane (1.5 mL). After purging with nitrogen three times, the reaction mixture was stirred at 25°C for 18 hours. LCMS indicated the reaction was completed. The reaction mixture was filtered, and the filtrate was concentrated under reduced pressure. The residue was purified by preparative high-performance liquid chromatography (ammonium bicarbonate conditions) to obtain compound 76. LCMS: m/z 457.2 [M+H]⁺; ¹H NMR (400 MHz, CDCl₃) ) δ: 7.21 (s, 4H), 7.08 (d, *J =* 7.6 Hz, 1H), 7.01 (s, 1H), 6.87 (d, *J =* 7.5 Hz, 1H), 4.70 - 4.55 (m, 1H), 4.01 (s, 4H), 3.92 (s, 2H), 3.79 (d, *J=* 11.6 Hz, 2H), 2.77 (s, 3H), 2.68 - 2.59 (m, 4H), 1.89 (d, *J =* 9.8 Hz, 2H), 1.61 - 1.55 (m, 2H), 1.42 - 1.35 (m, 3H), 1.34 (s, 3H), 1.33 (s, 3H).

### Example 75: Compound 77

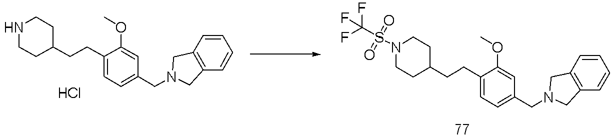

Trifluoromethanesulfonic anhydride (146 mg, 0.52 mmol) was added to a solution of 2-(3-methoxy-4-(2-(piperidin-4-yl)ethyl)benzyl)isoindoline hydrochloride (100 mg, 0.26 mmol) and triethylamine (131 mg, 1.29 mmol) in dichloromethane (3 mL) at 25 °C, and the mixture was stirred for 3 hours. LCMS indicated the reaction was completed. The reaction mixture was quenched by adding water (30 mL)and extracted with dichloromethane (30 mL×3). The organic phases were combined, washed with saturated brine (30 mL), dried over anhydrous sodium sulfate, and filtered. The filtrate was concentrated under reduced pressure. The residue was purified by preparative high performance liquid chromatography (ammonium bicarbonate conditions) to afford compound 77. LCMS: m/z 483.2 [M+H]⁺; ¹H NMR (400 MHz, CDCl₃) δ: 7.20 (s, 4H), 7.07 (d, *J =* 7.5 Hz, 1H), 7.00 (s, 1H), 6.91 (d, *J =* 7.6 Hz, 1H), 4.03 - 3.95 (m, 5H), 3.95 - 3.90 (m, 3H), 3.84 (s, 3H), 3.03 (t, *J =* 12.3 Hz, 2H), 2.69 - 2.58 (m, 2H), 1.90 (d, *J =* 11.5 Hz, 2H), 1.63 - 1.55 (m, 2H), 1.50 (dt, *J =* 3.4, 7.1 Hz, 1H), 1.40 - 1.31 (m, 2H).

### Example 76: Compound 78

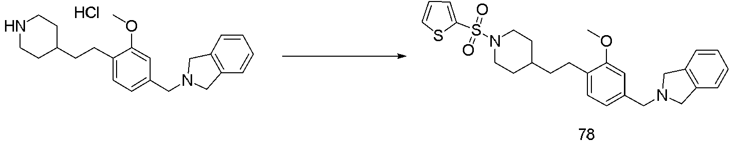

2-Thiophenesulfonyl chloride (79 mg, 0.43 mmol) was added to a solution of 2-(3-methoxy-4-(2-(piperidin-4-yl)ethyl)benzyl)isoindoline hydrochloride (100 mg, crude) and triethylamine (0.12 mL, 0.87 mmol) in dichloromethane (1 mL) at 25 °C, and the mixture was stirred for 1 hour. LCMS indicated the reaction was completed. The reaction mixture was quenched with water (20 mL)and extracted with ethyl acetate (20 mL×3). The organic phases were combined, washed with saturated brine (20 mL), and dried over anhydrous sodium sulfate. After filtration, the filtrate was concentrated under reduced pressure, and the residue was purified by preparative high-performance liquid chromatography (ammonium bicarbonate conditions) to obtain compound 78. LCMS: m/z 497.1 [M+H]⁺; ¹H NMR (400 MHz, CDCl₃) *δ*: 7.52 (dd, *J =* 1.1, 5.0 Hz, 1H), 7.45 (dd, *J =* 1.2, 3.7 Hz, 1H), 7.16 - 7.09 (m, 3H), 7.06 (dd, *J =* 3.8, 4.9 Hz, 1H), 6.97 (d, *J =* 7.5 Hz, 1H), 6.90 (s, 1H), 6.79 (d, *J =* 7.6 Hz, 1H), 6.83 - 6.77 (m, 1H), 3.93 (s, 3H), 3.85 (s, 2H), 3.73 (s, 3H), 3.69 (br d, *J=* 5.5 Hz, 2H), 2.56 - 2.47 (m, 2H), 2.27 (dt, *J* = 2.1, 11.7 Hz, 3H), 1.84 - 1.73 (m, 2H), 1.50 - 1.41 (m, 2H), 1.38 - 1.26 (m, 3H).

### Example 77: Compound 79

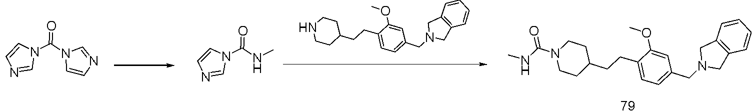

Step 1: 1,1'-carbonyldiimidazole (500 mg, 3.08 mmol) and methylamine hydrochloride (416 mg, 6.17 mmol) were added to acetonitrile (3 mL) and N,N-dimethylformamide (1 mL) at 25 °C, and the mixture was stirred for 24 hours. Saturated ammonium chloride solution (10 mL) was added to quench the reaction, and the mixture was extracted with ethyl acetate (20 mL). The combined organic phases were washed with saturated brine (20 mL), dried over anhydrous sodium sulfate, and filtered. The filtrate was concentrated under reduced pressure to obtain N-Methyl-1H-imidazole-1-carboxamide.

Step 2: N-Methyl-1H-imidazole-1-carboxamide (150 mg, 1.20 mmol) and 2-(3-methoxy-4-(2-(piperidin-4-yl)ethyl)benzyl)isoindoline (100 mg, 0.29 mmol) were dissolved in dichloromethane (10 mL) at 25 °C, triethylamine (0.12 mL, 0.86 mmol) was added dropwise, and the mixture was stirred for 3 hours. LCMS indicated the reaction was completed. The reaction mixture was filtered, and the filtrate was concentrated under reduced pressure. The residue was purified by preparative high-performance liquid chromatography (ammonium bicarbonate conditions) to obtain compound 79. LCMS: m/z 408.3 [M+H]⁺; ¹H NMR (400 MHz, CDCl₃) δ: 7.19 (s, 4H), 7.08 (d, *J =* 7.6 Hz, 1H), 6.97 (s, 1H), 6.90 (d, *J =* 7.5 Hz, 1H), 4.45 - 4.34 (m, 1H), 3.99 - 3.96 (m, 4H), 3.95 - 3.92 (m, 1H), 3.91 - 3.88 (m, 3H), 3.83 (s, 3H), 2.84 - 2.81 (m, 3H), 2.80 - 2.72 (m, 2H), 2.66 - 2.60 (m, 2H), 1.85 - 1.74 (m, 3H), 1.60 - 1.49 (m, 2H), 1.25 - 1.11 (m, 2H).

### Example 78: Compound 80

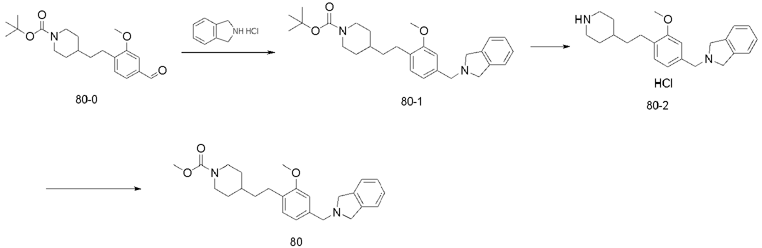

Step 1: A solution of starting material 80-0 (2.20 g, 6.33 mmol), isoindoline hydrochloride (1.46 g, 9.49 mmol), sodium borohydride acetate (4.01 g, 18.99 mmol) and acetic acid (0.36 mL, 6.33 mmol) in 1,2-dichloroethane (15 mL) was stirred at 25°C for 4 hours. LCMS indicated the reaction was completed. Water (20 mL) was added to quench the reaction, and the mixture was extracted with dichloromethane (20 mL×3). The organic phases were combined, washed with saturated brine (60 mL) and concentrated under reduced pressure. The resulting residue was purified by silica gel column chromatography (petroleum ether: ethyl acetate = 1: 1) to obtain Intermediate 80-1. LCMS: m/z 451.3 [M+H]⁺.

Step 2: Hydrochloric acid-dioxane (3 mL, 12.00 mmol, 4M/L) solution was added dropwise to a solution of Intermediate 80-1 (1.00 g, 1.03 mmol) in dioxane (5 mL) at 25 °C, and the mixture was stirred for 2 hours. LCMS indicated the reaction was completed, and the reaction mixture was concentrated under reduced pressure to obtain Intermediate 80-2. LCMS: m/z 351.2 [M+H]⁺.

Step 3: Dimethyl dicarbonate (58 mg, 0.43 mmol) was added to a solution of Intermediate 80-2 (100 mg, 0.29 mmol) and triethylamine (0.12 mg, 0.86 mmol) in dichloromethane (5 mL) at 25 °C, and the mixture was stirred for 2 hours. LCMS indicated the reaction was completed. Water (5 mL) was added to quench the reaction, and the mixture was extracted with ethyl acetate (5 mL×3). The organic phases were collected, washed with saturated brine (15 mL) and concentrated under reduced pressure. The resulting residue was purified by preparative high-performance liquid chromatography (ammonium bicarbonate) to obtain compound 80. LCMS: m/z 409.2 [M+H]⁺; ¹H NMR (400MHz, CDCl₃) δ: 7.21 (s, 4H), 7.08 (d, *J=* 7.5 Hz, 1H), 6.99 (s, 1H), 6.89 (dd, *J=* 7.5, 1.1 Hz, 1H), 4.11 (br d, *J=* 10.6 Hz, 2H), 4.01 (s, 4H), 3.93 (s, 2H), 3.84 (s, 3H), 3.70 (s, 3H), 2.76 (br t, *J =* 12.3 Hz, 2H), 2.56 - 2.68 (m, 2H), 1.77 (br d, *J*= 12.8 Hz, 2H), 1.41-1.59 (m, 3H), 1.05 - 1.25 (m, 2H).

### Example 79: Compound 81

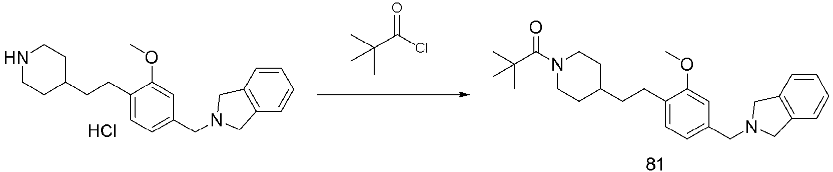

To a solution of 2-(3-methoxy-4-(2-(piperidin-4-yl)ethyl)benzyl)isoindoline hydrochloride (120 mg, 0.34 mmol) in dichloromethane (5 mL), triethylamine (0.14 mL, 1.02 mmol) and pivaloyl chloride (0.13 mL, 1.02 mmol) were added successively at 25°C. The mixture was stirred at 25°C for 1 hour. LCMS indicated the reaction was completed. The reaction mixture was concentrated under reduced pressure, and the residue was purified by thin-layer chromatography (petroleum ether: ethyl acetate = 5: 1) to obtain a crude product. The crude product was purified by preparative high-performance liquid chromatography (ammonium bicarbonate conditions) to obtain compound 81. LCMS: m/z 435.3 [M+H]⁺; ¹H NMR (400 MHz, CDCl₃) δ: 7.19 (s, 4H), 7.08 (d, *J = 7.5* Hz, 1H), 6.97 (s, 1H), 6.90 (dd, *J =* 1.0, 7.6 Hz, 1H), 4.41 (d, *J =* 12.8 Hz, 2H), 3.96 (s, 4H), 3.90 (s, 2H), 3.84 (s, 3H), 2.78 (t, *J =* 12.4 Hz, 2H), 2.67 - 2.60 (m, 2H), 1.82 (d, *J =* 12.5 Hz, 2H), 1.55 (t, *J =* 6.1 Hz, 3H), 1.29 (s, 9H), 1.22 - 1.11 (m, 2H).

### Example 80: Compound 82

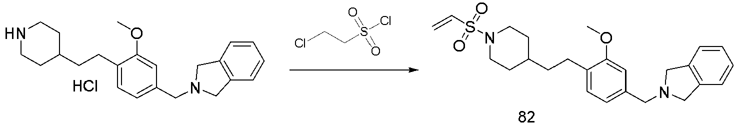

To a solution of 2-(3-methoxy-4-(2-(piperidin-4-yl)ethyl)benzyl)isoindoline hydrochloride (150 mg, 0.43 mmol) in dichloromethane (4 mL) at 25 °C, triethylamine (0.36 mL, 2.58 mmol) and 2-chloroethane-1-sulfonyl chloride ( 0.23 mL, 2.15 mmol) were added in sequence. The mixture was stirred at 25°C for 1.5 hours. LCMS indicated the reaction was completed. The reaction mixture was concentrated under reduced pressure, and the residue was purified by thin-layer chromatography (dichloromethane: methanol = 10: 1) to obtain a crude product. The crude product was purified by preparative high-performance liquid chromatography (ammonium bicarbonate conditions) to obtain compound 82. LCMS: m/z 441.3 [M+H]⁺; ¹H NMR (400 MHz, CDCl₃) δ: 7.20 (s, 4H), 7.07 (d, *J= 7.5* Hz, 1H), 6.99 (s, 1H), 6.90 (dd, *J= 0.9,* 7.5 Hz, 1H), 6.48 - 6.37 (m, 1H), 6.23 (d, *J =* 16.6 Hz, 1H), 6.01 (d, *J =* 9.9 Hz, 1H), 3.98 (s, 4H), 3.91 (s, 2H), 3.84 (s, 3H), 3.73 (d, *J=* 12.0 Hz, 2H), 2.66 - 2.55 (m, 4H), 1.86 (d, *J =* 9.8 Hz, 2H), 1.60 - 1.52 (m, 2H), 1.41 - 1.31 (m, 3H).

### Example 81: Compound 83

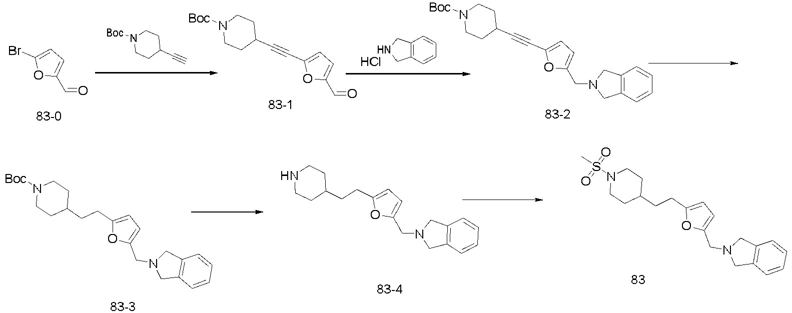

Step 1: starting material 83-0 (1.5 g, 8.57 mmol), tertbutyl 4-ethynylpiperidine-1-carboxylate (1.79 g, 8.57 mmol), bis(triphenylphosphine)palladium dichloride (600 mg, 0.86 mmol), copper iodide (80 mg, 0.43 mmol) and triethylamine (12 mL) were added to tetrahydrofuran (10 mL) at 25 °C. After purging with nitrogen three times, the mixture was heated to 100 °C and stirred for 3 hours. LCMS indicated the reaction was completed. The reaction mixture was filtered, and the filtrate was concentrated under reduced pressure. The residue was purified by silica gel column chromatography (petroleum ether: ethyl acetate = 5: 1) to obtain Intermediate 83-1. LCMS: m/z 204.2 [M-99]⁺.

Step 2: At 25 °C, sodium borohydride acetate (4.59 g, 21.76 mmol) was added to a solution of Intermediate 83-1 (2.20 g, 7.25 mmol), isoindoline hydrochloride (2.59 g, 21.76 mmol) and acetic acid (2.0 mL, 36.26 mmol) in 1,2-dichloromethane (25 mL) and the mixture was stirred for 2 hours. LCMS indicated the reaction was completed. The reaction mixture was quenched with sodium bicarbonate solution (100 mL)and extracted with ethyl acetate (100 mL×3). The organic phases were combined, washed with saturated brine (100 mL), and dried over anhydrous sodium sulfate. After filtration, the filtrate was concentrated under reduced pressure, and the residue was purified by silica gel column chromatography (petroleum ether: ethyl acetate = 1: 1) to obtain Intermediate 83-2. LCMS: m/z 407.2 [M+H]⁺.

Step 3: Under nitrogen atmosphere, Intermediate 83-2 (1.0 g, 2.46 mmol) and palladium/carbon (0.26 g, 0.25 mmol, 10 wt%) were added into methanol (20 mL). After purging with hydrogen three times, the mixture was stirred at 25°C for 1 hour. LCMS indicated the reaction was completed. The reaction mixture was filtered, and the filtrate was concentrated under reduced pressure to obtain Intermediate 83-3. LCMS: m/z 411.2 [M+H]⁺.

Step 4: A solution of Intermediate 83-3 (300 mg, 0.73 mmol) in HCl-dioxane (4 mL, 2M) was stirred at 25 °C for 1 hour. LCMS indicated the reaction was completed, and the reaction mixture was concentrated under reduced pressure to obtain Intermediate 83-4. LCMS: m/z 311.2 [M+H]⁺.

Step 5: Methanesulfonic anhydride (168 mg, 0.97 mmol) was added to a solution of Intermediate 83-4 (200 mg, 0.64 mmol) and triethylamine (0.27 mL, 1.93 mmol) in dichloromethane (5 mL) at 25 °C, the mixture was stirred for 10 minutes. LCMS indicated the reaction was completed. Saturated ammonium chloride solution (20 mL) was added to quench the reaction, and the mixture was extract with dichloromethane (20 mL×2). The combined organic phases were washed with saturated brine (20 mL), dried over anhydrous sodium sulfate, and filtered. The filtrate was concentrated, and the residue was purified by preparative high-performance liquid chromatography (ammonium bicarbonate conditions) to obtain compound 83. LCMS: m/z 389.2 [M+H]⁺; ¹H NMR (400 MHz, CDCl₃) δ: 7.18 (s, 4H), 6.15 (d, *J* = 3.0 Hz, 1H), 5.93 (d, *J=* 3.0 Hz, 1H), 3.99 (s, 4H), 3.87 (s, 2H), 3.77 (br d, *J=* 12.1 Hz, 2H), 2.74 (s, 3H), 2.66 (t, *J =* 7.8 Hz, 2H), 2.63 - 2.55 (m, 2H), 1.81 (br d, *J =* 9.9 Hz, 2H), 1.65 (br s, 2H), 1.39 - 1.29 (m, 3H).

### Example 82: Compound 84

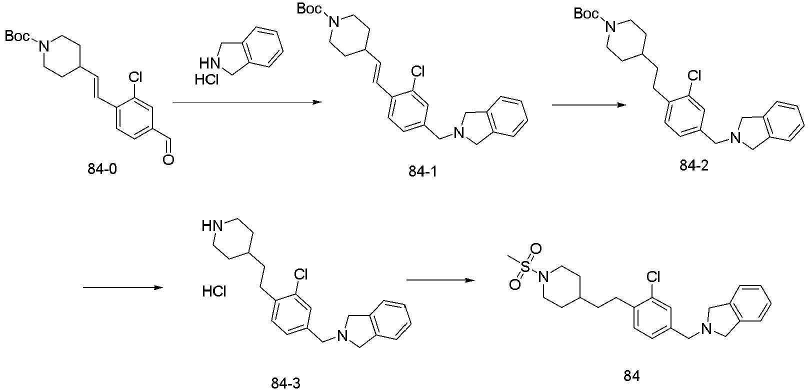

Step 1: At 25 °C, starting material 84-0 (1.00 g, 2.86 mmol), isoindoline hydrochloride (0.88 g, 5.72 mmol) and acetic acid (0.26 mL, 2.86 mmol) were dissolved in 1,2-dichloroethane (20 mL), and the mixture was stirred for 2 hours. Sodium triacetoxyborohydride (1.21 g, 5.72 mmol) was added to the reaction system, and the reaction mixture was continuously stirred for 18 hours. LCMS indicated the reaction was completed. The reaction was quenched with saturated sodium bicarbonate solution (20 mL) and the mixture was extracted with dichloromethane (20 mL×2). The combined organic phase was washed with saturated brine (50 mL), dried over anhydrous sodium sulfate, and filtered. The filtrate was concentrated, and the resulting residue was purified by silica gel column chromatography (petroleum ether: ethyl acetate = 4: 1) to obtain Intermediate 84-1. LCMS: m/z 453.3 [M+H]⁺.

Step 2: At 25°C, a solution of Intermediate 84-1 (450 mg, 0.99 mmol) and platinum dioxide (23 mg, 0.10 mmol) in ethyl acetate (5 mL) was purged with hydrogen three times and stirred for 3 hours. LCMS indicated the reaction was completed. The reaction mixture was filtered, and the filtrate was concentrated under reduced pressure to obtain Intermediate 84-2.

Step 3: Hydrochloric acid-dioxane solution (2.00 mL, 8.00 mmol, 4 M) was added dropwise to a solution of Intermediate 84-2 (450 mg, 0.99 mmol) in dioxane (2 mL) at 25 °C. The mixture was stirred for 2 hours. The reaction mixture was concentrated under reduced pressure to obtain Intermediate 84-3. LCMS: m/z 430.2 [M+H]⁺.

Step 4: Methylsulfonic anhydride (258 mg, 1.45 mmol) was added to a solution of Intermediate 84-3 (350 mg, 0.99 mmol) and triethylamine (0.27 mL, 1.97 mmol) in dichloromethane (2 mL) at 25 °C, and the mixture was stirred for 2 hours. LCMS indicated the reaction was completed. The reaction was quenched with water (10 mL) and the mixture was extracted with dichloromethane (10 mL×3). The combined organic phase was washed with saturated brine (20 mL), dried over anhydrous sodium sulfate, and filtered. The filtrate was concentrated, and the resulting residue was purification by preparative high-performance liquid chromatography (ammonium bicarbonate conditions) to obtain compound 84. LCMS: m/z 433.2 [M+H]⁺; ¹H NMR (400 MHz, CDCl₃) δ7.43 (s, 1H), 7.29 (s, 1H), 7.20 (s, 4H), 7.18 (s, 1H), 3.99 (s, 4H), 3.90 (s, 2H), 3.81 (br d, *J =* 12.0 Hz, 2H), 2.78 (s, 3H), 2.75 (br d, *J =* 2.3 Hz, 2H), 2.67 (br t, *J=* 11.5 Hz, 2H), 1.93 - 1.85 (m, 2H), 1.65 - 1.57 (m, 2H), 1.49 - 1.34 (m, 3H).

### Example 83: Compound 85

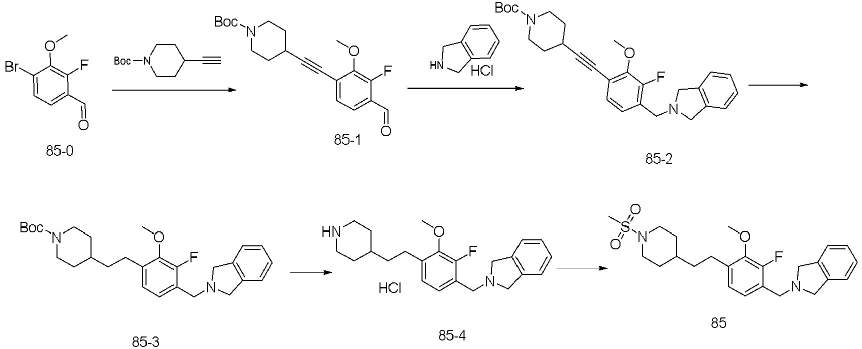

Step 1: Starting material 85-0 (900 mg, 3.87 mmol), tertbutyl 4-ethynylpiperidine-1-carboxylate (1.60 g, 7.65 mmol), bis(triphenylphosphine) palladium dichloride (280 mg, 0.39 mmol) and copper iodide (74 mg, 0.38 mmol) were added to triethylamine (10 mL). After purging with nitrogen three times, the reaction mixture was heated to 80 °C and stirred for 18 hours. LCMS indicated the reaction was completed. The reaction mixture was filtered, and the filtrate was concentrated under reduced pressure. The residue was purified by silica gel column chromatography (petroleum ether: ethyl acetate = 6: 1) to obtain Intermediate 85-1. LCMS: m/z 306.2 [M-55]⁺.

Step 2: At 25 °C, acetic acid (1.08 g, 18.01 mmol) and sodium triacetoxyborohydride (1.52 g, 7.16 mmol) were added to a solution of Intermediate 85-1 (1.30 g, 3.60 mmol) and isoindoline hydrochloride (900 mg, 5.78 mmol) in 1,2-dichloroethane (20 mL), and the mixture was stirred for 2 hours. LCMS indicated the reaction was completed. The reaction mixture was quenched by adding water (50 mL) and the mixture was extracted with ethyl acetate (50 mL×3). The organic phases were combined, washed with saturated brine (50 mL), and dried over anhydrous sodium sulfate. After filtration, the filtrate was concentrated under reduced pressure, and the residue was purified by silica gel column chromatography (petroleum ether: ethyl acetate = 3:1) to obtain Intermediate 85-2. LCMS: m/z 465.2 [M+H]⁺.

Step 3: Under nitrogen atmosphere, Intermediate 85-2 (700 mg, 1.50 mmol) and palladium/carbon (700 mg, 0.66 mmol) were added into methanol (8 mL). After purging with hydrogen three times, the temperature was raised to 25 °C and the mixture was stirred for 18 hours. LCMS indicated the reaction was completed. The reaction mixture was filtered, and the filtrate was concentrated under reduced pressure. The residue was purified by silica gel column chromatography (petroleum ether: ethyl acetate = 4: 1) to obtain Intermediate 85-3. LCMS: m/z 469.2 [M+H]⁺.

Step 4: Hydrochloric acid-dioxane solution (5 mL, 4M) was added to Intermediate 85-3 (90 mg, 0.19 mmol) at 25°C, and the mixture was stirred for 1 hour. LCMS indicated the reaction was completed. The reaction mixture was concentrated under reduced pressure to obtain crude product Intermediate 85-4. LCMS: m/z 369.2 [M+H]⁺.

Step 5: At 25 °C, methanesulfonic anhydride (51 mg, 0.29 mmol) was added to a solution of Intermediate 85-4 (71 mg, 0.17 mmol) and triethylamine (98 mg, 0.97 mmol) in dichloromethane (4 mL), and the mixture was stirred for 1 hour. LCMS indicated the reaction was completed. The reaction mixture was diluted with water (20 mL) and extracted with ethyl acetate (20 mLx3). The organic phases were combined, washed with saturated sodium chloride solution (20 mLx1). The organic phase was dried over anhydrous sodium sulfate, filtered, and the filtrate was concentrated under reduced pressure. The residue was purified by preparative high performance liquid chromatography (ammonium bicarbonate system) to obtain compound 85. LCMS: m/z 447.3 [M+H]⁺; ¹H NMR (400 MHz, CDCl₃) δ: 7.20 (s, 4H), 7.13 (t, *J=* 7.3 Hz, 1H), 6.91 (d, *J =* 7.9 Hz, 1H), 4.03 (s, 4H), 3.98 (s, 2H), 3.92 (d, *J* = 1.5 Hz, 3H), 3.80 (d, *J =* 11.8 Hz, 2H), 2.77 (s, 3H), 2.69 - 2.60 (m, 4H), 1.88 (d, *J =* 9.8 Hz, 2H), 1.60 - 1.54 (m, 2H), 1.43 - 1.31 (m, 3H).

### Example 84: Compound 86

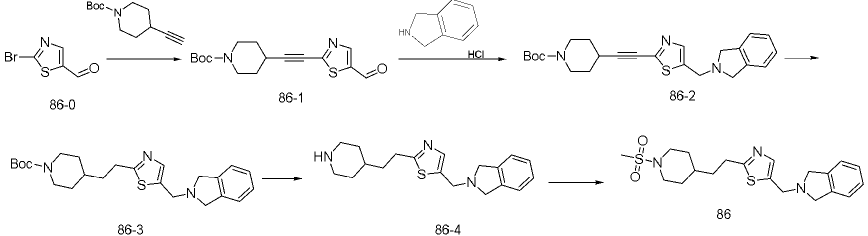

Step 1: Starting material 86-0 (500 mg, 2.60 mmol), tertbutyl 4-ethynylpiperidine-1-carboxylate (1.08 g, 5.20 mmol), copper iodide (50 mg, 0.26 mmol), bis(triphenylphosphine) palladium dichloride (182 mg, 0.26 mmol) and triethylamine (3.60 mL) were added to tetrahydrofuran (10 mL). After purging with nitrogen three times, the mixture was stirred at 25°C for 1.5 hours. LCMS indicated the reaction was completed. The reaction mixture was filtered, and the filtrate was concentrated under reduced pressure. The residue was purified by silica gel column chromatography (petroleum ether: ethyl acetate = 5: 1) to obtain Intermediate 86-1. LCMS: m/z 265.0 [M-55]⁺.

Step 2: At 25°C, Intermediate 86-1 (800 mg, 2.50 mmol), 2,3-dihydroisoindole hydrochloride (1.16 g, 7.49 mmol) and acetic acid (0.14 mL, 2.50 mmol) were added to 1,2-dichloroethane (10 mL) and the mixture was stirred at 25°C for 30 minutes. Sodium borohydride acetate (1.05 g, 4.99 mmol) was added and the mixture was continuously stirred for 1 hour. LCMS indicated the reaction was completed. The reaction was quenched by adding water (10 mL) and the mixture was extracted with ethyl acetate (50 mL×3). The organic phases were combined, washed with saturated brine (50 mL), and dried over anhydrous sodium sulfate. After filtration, the filtrate was concentrated under reduced pressure, and the residue was purified by silica gel column chromatography (ethyl acetate:petroleum ether =3:1) to obtain Intermediate 86-2. LCMS: m/z 424.2 [M+H]⁺.

Step 3: Under nitrogen atmosphere, Intermediate 86-2 (1 g, 2.36 mmol) and palladium/carbon (0.20 g, 1.88 mmol) were added into methanol (15 mL). After purging with hydrogen three times, the mixture was stirred at 25°C for 1 hour. LCMS indicated the reaction was completed. The reaction mixture was filtered, and the filtrate was concentrated under reduced pressure to obtain crude product Intermediate 86-3. LCMS: m/z 428.2 [M+H]⁺.

Step 4: Hydrochloric acid-dioxane solution (6 mL, 4 M) was added to Intermediate 86-3 (562 mg, crude product) at 25 °C, and the mixture was stirred at 25 °C for 20 minutes. LCMS indicated the reaction was completed. The reaction mixture was concentrated under reduced pressure to obtain Intermediate 86-4. LCMS: m/z 328.2 [M+H]⁺.

Step 5: At 25°C, Intermediate 86-4 (400 mg, crude product), triethylamine (0.51 mL, 3.66 mmol) and methylsulfonic anhydride (319 mg, 1.83 mmol) were added to dichloromethane (5 mL), and the mixture was stirred at 25 °C for 20 minutes. LCMS indicated the reaction was completed. The reaction mixture was quenched with water and the mixture was extracted with ethyl acetate (50 mL×3). The organic phases were combined, washed with saturated brine (50 mL), and dried over anhydrous sodium sulfate. After filtration, the filtrate was concentrated under reduced pressure, and the residue was purified by preparative high performance liquid chromatography (ammonium bicarbonate conditions) to obtain compound 86. LCMS: m/z 406.2 [M+H]⁺; ¹H NMR (400 MHz, CDCl₃) δ: 7.52 (s, 1H), 7.20 (s, 4H), 4.08 (s, 2H), 3.99 (s, 4H), 3.80 (d, *J= 11.9* Hz, 2H), 3.02 (t, *J =* 7.8 Hz, 2H), 2.76 (s, 3H), 2.68 - 2.60 (m, 2H), 1.91 - 1.75 (m, 5H), 1.42 - 1.30 (m, 2H).

### Example 85: Compound 87

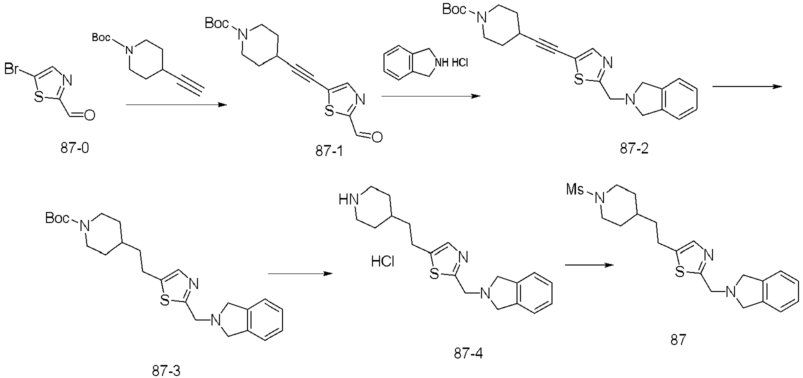

Step 1: Starting material 87-0 (1.00 g, 5.21 mmol), tertbutyl 4-ethynylpiperidine-1-carboxylate (1.64 g, 7.82 mmol), bis(triphenylphosphine) palladium dichloride (370 mg, 0.52 mmol), and copper iodide (100 mg, 0.52 mmol) were added to a solution of triethylamine (3 mL) and tetrahydrofuran (10 mL). After purging with nitrogen three times, the reaction mixture was heated to 80 °C and stirred for 2 hours. LCMS indicated the reaction was completed. The temperature was cooled to 25 °C, the reaction was quenched with water (40 mL), and the mixture was extracted with ethyl acetate (40 mL×3). The organic phases were collected, washed with saturated brine (150 mL), concentrated under reduced pressure and purified by silica gel column chromatography (petroleum ether: ethyl acetate = 5: 1) to obtain Intermediate 87-1. LCMS: m/z 321.1 [M+1]⁺.

Step 2: At 25 °C, Intermediate 87-1 (1.00 g, 3.12 mmol), isoindoline hydrochloride (720 mg, 4.68 mmol), sodium borohydride acetate (1.97 g, 9.36 mmol), and acetic acid (0.18 ml, 3.12 mmol) were added into a 1,2-dichloroethane solution (10 mL), and the mixture was stirred for 4 hours. LCMS indicated the reaction was completed. The reaction mixture was added with water (15 mL) and extracted with dichloromethane (30 mL×3). The organic phases were collected and washed with saturated brine (100 mL). The organic phase was concentrated under reduced pressure and purified by silica gel column chromatography (petroleum ether: ethyl acetate = 3:1) to obtain Intermediate 87-2. LCMS: m/z 424.2 [M+H]⁺.

Step 3: Under nitrogen atmosphere, palladium/carbon (50 mg, 0.47 mmol, 10 wt%) was added to a solution of Intermediate 87-2 (500 mg, 1.18 mmol) in methanol (10 mL) at 25°C. After purging with hydrogen three times, the mixture was heated to 50°C and stirred for 4 hours. LCMS indicated the reaction was completed. The mixture was cooled to 25°C, filtered, and concentrated under reduced pressure to obtain Intermediate 87-3. LCMS: m/z 428.2 [M+H]⁺.

Step 4: Dioxane hydrochloride (3.0 mL, 12.00 mmol) was added dropwise to a solution of Intermediate 87-3 (400 mg, 1.15 mmol) in dioxane (5 mL) at 25 °C, and the mixture was stirred for 2 hours. LCMS indicated the reaction was completed, and the reaction mixture was concentrated under reduced pressure to obtain Intermediate 87-4. LCMS: m/z 328.2 [M+H]⁺.

Step 5: At 25°C, Intermediate 87-4 (300 mg, 0.92 mmol), potassium carbonate (982 mg, 2.76 mmol), and methanesulfonic anhydride (480 mg, 2.76 mmol) were dissolved in a solution of acetonitrile (5 mL), and the mixture was stirred for 2 hours. LCMS indicated the reaction was completed. The reaction mixture was added with water (10 mL), and extracted with ethyl acetate (10 mL×3). The organic phases were collected and washed with saturated brine (30 mL). The organic phase was concentrated under reduced pressure and purified by preparative high-performance liquid chromatography (ammonium bicarbonate conditions) to obtain compound 87. LCMS: m/z 406.1 [M+H]⁺¹; H NMR (400MHz, CDCl₃) δ: 7.15 (s, 1H), 7.09 (s, 4H), 4.11 (s, 2H), 4.02 (s, 4H), 3.68 (br d, *J=* 12.0 Hz, 2H), 2.74 (t, *J =* 7.7 Hz, 2H), 2.65 (s, 3H), 2.44-2.59 (m, 2H), 1.68-1.71 (m, 2H), 1.45-1.63 (m, 2H), 1.11-1.33 (m, 3H).

### Example 86: Compound 88

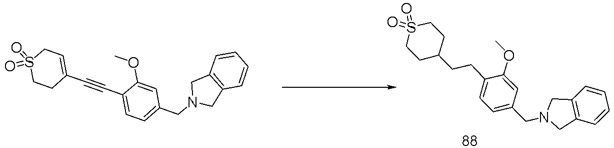

Under nitrogen atmosphere, 4-((4-(isoindolin-2-ylmethyl)-2-methoxyphenyl)ethynyl)-3,6-dihydro-2H-thiopyran 1,1-dioxide (100 mg, 0.25 mmol) and palladium/carbon (3 mg, 0.03 mmol, 10 wt%) were dissolved in methanol (10 mL) at 25 °C. After purging with hydrogen three times, and mixture was stirred for 18 hours. LCMS indicated the reaction was completed. The reaction mixture was filtered, and the filtrate was concentrated under reduced pressure. The residue was purified by preparative high-performance liquid chromatography (ammonium bicarbonate conditions) to obtain compound 88. LCMS: m/z 400.1[M+H]⁺; ¹H NMR (400 MHz, CDCl₃) δ: 7.44 - 7.38 (m, 2H), 7.33 (dd, *J=* 3.3, 5.3 Hz, 2H), 7.17 - 7.09 (m, 2H), 6.83 (d, *J=* 7.5 Hz, 1H), 4.93 (d, *J=* 14.3 Hz, 2H), 4.45 - 4.28 (m, 4H), 3.83 (s, 3H), 3.16 - 2.89 (m, 4H), 2.72 - 2.55 (m, 2H), 2.19 (d, *J=* 13.0 Hz, 2H), 1.92 (br d, *J=* 12.3 Hz, 2H), 1.69 - 1.56 (m, 3H).

### Example 87: Compound 89

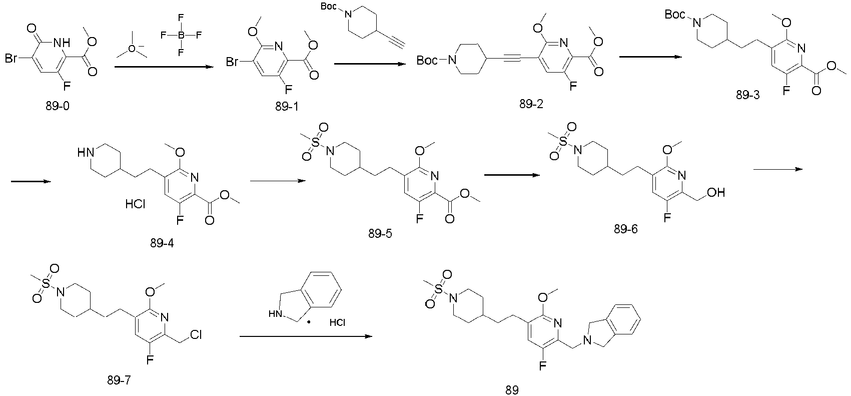

Step 1: To a solution of starting material 89-0 (1.00 g, 4.00 mmol) in dichloromethane (15 mL), was add trimethyloxonium tetrafluoroborate (1.18 g, 8.00 mmol), and the mixture was stirred at 25°C for 18 hours. TLC indicated the reaction was completed. The reaction mixture was concentrated under reduced pressure, and the residue was purified by silica gel column chromatography (petroleum ether: ethyl acetate = 5: 1) to obtain Intermediate 89-1.

Step 2: Intermediate 89-1 (600 mg, 2.27 mmol), tertbutyl 4-ethynylpiperidine-1-carboxylate (950 mg, 4.54 mmol), copper iodide (22 mg, 0.11 mmol), bis(triphenylphosphine) palladium dichloride (159 mg, 0.23 mmol) and triethylamine (1.50 mL) were added to tetrahydrofuran (10 mL). After purging with nitrogen three times, the mixture was stirred at 25°C for 18 hours. LCMS indicated the reaction was completed. The reaction mixture was concentrated under reduced pressure, and the residue was purified by silica gel column chromatography (petroleum ether: ethyl acetate = 5: 1) to obtain Intermediate 89-2. LCMS: m/z 393.2 [M+H]⁺.

Step 3: Under nitrogen atmosphere, Intermediate 89-2 (1.07 g, 2.75 mmol) and palladium/carbon (1.01 g, 9.4 mmol, 10 wt%) were added into methanol (15 mL). After purging with hydrogen three times, the mixture was stirred at 25°C for 16 hours. LCMS indicated the reaction was completed. The reaction mixture was filtered, and the filtrate was concentrated under reduced pressure to obtain Intermediate 89-3. LCMS: m/z 341.1 [M-55]⁺.

Step 4: Hydrochloric acid-dioxane solution (6 mL, 4 M) was added to Intermediate 89-3 (940 mg, crude) at 25 °C, and the mixture was stirred at 25 °C for 20 minutes. LCMS indicated the reaction was completed. The reaction mixture was concentrated under reduced pressure to obtain Intermediate 89-4. LCMS: m/z 297.1 [M+H]⁺.

Step 5: At 25°C, Intermediate 89-4 (700 mg, crude), triethylamine (0.98 mL, 7.09 mmol) and methylsulfonic anhydride (617 mg, 3.54 mmol) were added to dichloromethane (10 mL). The mixture was stirred at 25°C for 30 minutes. LCMS indicated the reaction was completed. The reaction mixture was concentrated under reduced pressure, and the residue was purified by silica gel column chromatography (petroleum ether: ethyl acetate = 1:1) to obtain Intermediate 89-5. LCMS: m/z 375.1 [M+H]⁺.

Step 6: To a solution of Intermediate 89-5 (730 mg, 1.95 mmol) in tetrahydrofuran (10 mL) were add lithium chloride (826 mg, 19.50 mmol) and sodium borohydride (737 mg, 19.50 mmol) in sequence, and the mixture was stirred at 25 °C for 16 hours. LCMS indicated the reaction was completed. The reaction mixture was quenched with water and the mixture was extracted with dichloromethane (100 mL×2). The organic phases were combined, washed with saturated brine (100 mL×2), dried over anhydrous sodium sulfate, and filtered. The filtrate was concentrated under reduced pressure to obtain Intermediate 89-6. LCMS: m/z 347.1 [M+H]⁺.

Step 7: To a solution of Intermediate 89-6 (300 mg, crude) in dichloromethane (4 mL) was add thionyl chloride (1 mL), and the mixture was stirred at 25°C for 30 minutes. LCMS indicated the reaction was completed. The reaction mixture was concentrated under reduced pressure to obtain Intermediate 89-7. LCMS: m/z 365.1 [M+H]⁺.

Step 8: To a solution of Intermediate 89-7 (310 mg, crude) in N,N-dimethylformamide (8 mL) were added successively potassium carbonate (352 mg, 2.55 mmol) and 2,3-dihydroisoindole hydrochloride (396 mg, 2.55 mmol), and the mixture was stirred at 80°C for 1.5 hours. LCMS indicated the reaction was completed. The reaction was quenched with water (50 mL) and the mixture was extracted with ethyl acetate (50 mL×3). The organic phases were combined, washed with saturated brine (100 mL×2), dried over anhydrous sodium sulfate, and filtered. The filtrate was concentrated under reduced pressure. The residue was purified by silica gel column chromatography (petroleum ether: ethyl acetate = 1:1) to obtain a crude product, which was purified by preparative high-performance liquid chromatography (ammonium bicarbonate conditions) to obtain compound 89. LCMS: m/z 448.3 [M+H]⁺; ¹H NMR (400 MHz, CDCl₃) δ: 7.22 - 7.11 (m, 5H), 4.16 (s, 4H), 4.04 (d, *J =* 2.6 Hz, 2H), 3.93 (s, 3H), 3.80 (d, *J=* 11.6 Hz, 2H), 2.77 (s, 3H), 2.69 - 2.53 (m, 4H), 1.86 (d, *J =* 9.8 Hz, 2H), 1.60 - 1.52 (m, 2H), 1.42 - 1.30 (m, 3H).

### Example 88: Compound 90

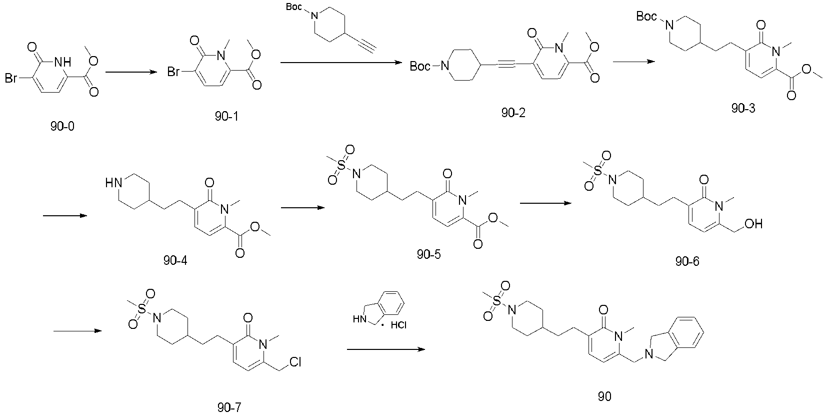

Step 1: At 25°C, starting material 90-0 (2.01 g, 8.62 mmol), potassium carbonate (2.38 g, 17.24 mmol) and methyl iodide (1.61 mL, 25.86 mmol) were added in sequence to N,N-dimethylformamide (25 mL). The mixture was stirred at 25 °C for 16 hours. LCMS indicated the reaction was completed. The reaction was quenched with water (100 mL) and the mixture was extracted with ethyl acetate (100 mL×2). The organic phases were combined, washed with saturated brine (100 mL×2), dried over anhydrous sodium sulfate, and filtered. The filtrate was concentrated under reduced pressure. The residue was purified by silica gel column chromatography (petroleum ether:ethyl acetate = 3:1) to obtain Intermediate 90-1. LCMS: m/z 245.9 [M+H]⁺.

Step 2: Intermediate 90-1 (1.18 g, 4.80 mmol), tertbutyl 4-ethynylpiperidine-1-carboxylate (2.01 g, 9.59 mmol), copper iodide (90 mg, 0.48 mmol), bis(triphenylphosphine) palladium dichloride (340 mg, 0.48 mmol) and triethylamine (6.60 mL) were added to tetrahydrofuran (20 mL). After purging with nitrogen three times, the mixture was stirred at 25°C for 3 hours. LCMS indicated the reaction was completed. The reaction mixture was concentrated under reduced pressure, and the residue was purified by silica gel column chromatography (petroleum ether: ethyl acetate = 3: 1) to obtain Intermediate 90-2. LCMS: m/z 375.2 [M+H]⁺.

Step 3: Under nitrogen atmosphere, Intermediate 90-2 (1.69 g, 4.51 mmol) and palladium/carbon (1.25 g, 11.75 mmol, 10 wt%) were added into methanol (15 mL). After purging with hydrogen three times, the mixture was stirred at 25°C for 16 hours. LCMS indicated the reaction was completed. The reaction mixture was filtered, and the filtrate was concentrated under reduced pressure to obtain Intermediate 90-3. LCMS: m/z 279.2 [M-99]⁺.

Step 4: Hydrochloric acid-dioxane solution (10 mL, 4 M) was added to Intermediate 90-3 (1.42 g, crude) at 25°C. The mixture was stirred at 25°C for 30 minutes. LCMS indicated the reaction was completed. The reaction mixture was concentrated under reduced pressure to obtain Intermediate 90-4. LCMS: m/z 279.1 [M+H]⁺.

Step 5: Intermediate 90-4 (1.05 g, crude), triethylamine (1.57 mL, 11.32 mmol) and methylsulfonic anhydride (990 mg, 5.66 mmol) were added to dichloromethane (10 mL) at 25 °C. The mixture was stirred at 25°C for 30 minutes. LCMS indicated the reaction was completed. The reaction mixture was concentrated under reduced pressure, and the residue was purified by silica gel column chromatography (ethyl acetate) to obtain Intermediate 90-5. LCMS: m/z 357.1 [M+H]⁺.

Step 6: To a solution of Intermediate 90-5 (470 mg, 1.32 mmol) in tetrahydrofuran (8 mL) were added lithium chloride (560 mg, 13.2 mmol) and sodium borohydride (500 mg, 13.2 mmol) in sequence. The mixture was stirred at 25 °C for 16 hours. LCMS indicated the reaction was completed. The reaction mixture was quenched with water and the mixture was extracted with dichloromethane (100 mL×2). The organic phases were combined, washed with saturated brine (100 mL×2), dried over anhydrous sodium sulfate, filtered. The filtrate was concentrated under reduced pressure to obtain Intermediate 90-6. LCMS: m/z 329.1 [M+H]⁺.

Step 7: To a solution of Intermediate 90-6 (200 mg, crude) in dichloromethane (4 mL) was added thionyl chloride (1 mL). The mixture was stirred at 25°C for 30 minutes. LCMS indicated the reaction was completed. The reaction mixture was concentrated under reduced pressure to obtain Intermediate 90-7. LCMS: m/z 347.1 [M+H]⁺.

Step 8: To a solution of Intermediate 90-7 (210 mg, crude) in N,N-dimethylformamide (4 mL) were added successively potassium carbonate (250 mg, 1.82 mmol) and 2,3-dihydroisoindole hydrochloride (285 mg, 1.83 mmol). The mixture was stirred at 80°C for 16 hours. LCMS indicated the reaction was completed. The reaction was quenched with water (50 mL) and the mixture was extracted with ethyl acetate (50 mL×3). The organic phases were combined, washed with saturated brine (50 mL×2), dried over anhydrous sodium sulfate, and filtered. The filtrate was concentrated under reduced pressure. The residue was purified by thin layer chromatography (petroleum ether: ethyl acetate = 1:3) to obtain a crude product, and the crude product was purified by preparative high-performance liquid chromatography (ammonium bicarbonate conditions) to obtain compound 90. LCMS: m/z 430.3 [M+H]⁺; ¹H NMR (400 MHz, CDCl₃) δ: 7.24 - 7.17 (m, 4H), 7.13 (d, *J =* 6.9 Hz, 1H), 6.18 (d, *J =* 6.9 Hz, 1H), 3.96 (s, 4H), 3.83 - 3.75 (m, 4H), 3.71 (s, 3H), 2.77 (s, 3H), 2.70 - 2.61 (m, 2H), 2.60 - 2.53 (m, 2H), 1.89 (dd, *J=* 2.0, 12.6 Hz, 2H), 1.62 - 1.54 (m, 2H), 1.50 - 1.29 (m, 3H).

### Example 89: Compound 91

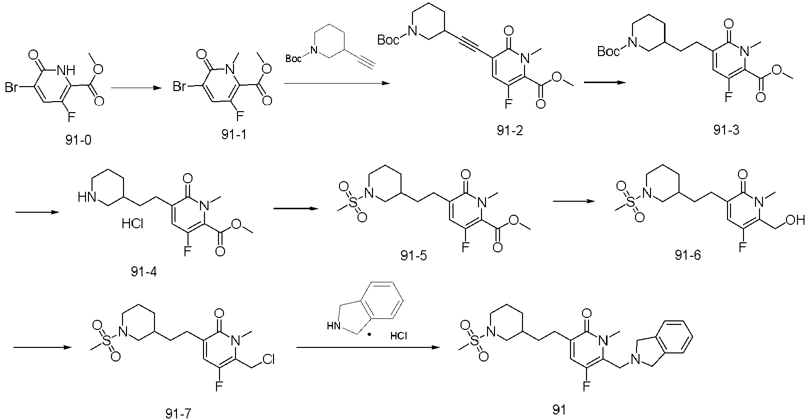

Step 1: At 25°C, starting material 91-0 (1.01 g, 4.01 mmol), potassium carbonate (1.11 g, 8.02 mmol) and methyl iodide (0.94 mL, 12.01 mmol) were added in sequence to N,N-dimethylformamide (10 mL). The mixture was stirred at 25 °C for 16 hours. LCMS indicated the reaction was completed. The reaction was quenched with water (100 mL) and the mixture was extracted with ethyl acetate (100 mL×2). The organic phases were combined, washed with saturated brine (100 mL×2), dried over anhydrous sodium sulfate, and filtered. The filtrate was concentrated under reduced pressure. The residue was purified by silica gel column chromatography (petroleum ether:ethyl acetate = 3:1) to obtain Intermediate 91-1. LCMS: m/z 264.0 [M+H]⁺.

Step 2: Intermediate 91-1 (218 mg, 0.83 mmol), tert-butyl 3-ethynylpiperidine-1-carboxylate (345 mg, 1.65 mmol), copper iodide (16 mg, 0.08 mmol), bis(triphenylphosphine) palladium dichloride (58 mg, 0.08 mmol) and triethylamine (1.15 mL) were added to tetrahydrofuran (5 mL). After purging with nitrogen three times, the mixture was stirred at 25°C for 2 hours. LCMS indicated the reaction was completed. The reaction mixture was concentrated under reduced pressure, and the residue was purified by silica gel column chromatography (petroleum ether: ethyl acetate = 3: 1) to obtain Intermediate 91-2. LCMS: m/z 293.1 [M-99]⁺.

Step 3: Under nitrogen atmosphere, Intermediate 91-2 (200 mg, 0.51 mmol) and palladium/carbon (100 mg, 0.94 mmol, 10 wt%) were added into methanol (5 mL). After purging with hydrogen three times, the mixture was stirred at 25°C for 1 hour. LCMS indicated the reaction was completed. The reaction mixture was filtered, and the filtrate was concentrated under reduced pressure to obtain Intermediate 91-3. LCMS: m/z 397.2 [M+H]⁺.

Step 4: Hydrochloric acid-dioxane solution (4 mL, 4 M) was added to Intermediate 91-3 (200 mg, crude) at 25°C. The mixture was stirred for at 25°C 30 minutes. LCMS indicated the reaction was completed. The reaction mixture was concentrated under reduced pressure to obtain Intermediate 91-4. LCMS: m/z 297.1 [M+H]⁺.

Step 5: At 25°C, Intermediate 91-4 (150 mg, crude), triethylamine (0.21 mL, 1.52 mmol) and methylsulfonic anhydride (132 mg, 0.76 mmol) were added to dichloromethane (4 mL). The mixture was stirred at 25°C for 30 minutes. LCMS indicated the reaction was completed. The reaction mixture was concentrated under reduced pressure, and the residue was purified by silica gel column chromatography (petroleum ether: ethyl acetate = 1:1) to obtain Intermediate 91-5. LCMS: m/z 375.1 [M+H]⁺.

Step 6: To a solution of Intermediate 91-5 (120 mg, 0.32 mmol) in tetrahydrofuran (4 mL) were added lithium chloride (135 mg, 3.2 mmol) and sodium borohydride (121 mg, 3.2 mmol) in sequence. The mixture was stirred at 25 °C for 16 hours. LCMS indicated the reaction was completed. The reaction mixture was quenched with water and the mixture was extracted with dichloromethane (30 mL×3). The organic phases were combined, washed with saturated brine (20 mL), dried over anhydrous sodium sulfate, and filtered. The filtrate was concentrated under reduced pressure to obtain Intermediate 91-6. LCMS: m/z 347.1 [M+H]⁺.

Step 7: To a solution of Intermediate 91-6 (90 mg, crude) in dichloromethane (3 mL) was added thionyl chloride (0.8 mL). The mixture was stirred at 25°C for 30 minutes. LCMS indicated the reaction was completed. The reaction mixture was concentrated under reduced pressure to obtain Intermediate 91-7. LCMS: m/z 365.1 [M+H]⁺.

Step 8: To a solution of Intermediate 91-7 (95 mg, crude) in N,N-dimethylformamide (3 mL) were added successively potassium carbonate (107 mg, 0.78 mmol) and 2,3-dihydroisoindole hydrochloride (121 mg, 0.78 mmol). The mixture was stirred at 80°C for 1.5 hours. LCMS indicated the reaction was completed. The reaction mixture was washed with water and extracted with ethyl acetate (20 mL×3). The organic phases were combined, washed with saturated brine (30 mL×2), dried over anhydrous sodium sulfate, and filtered. The filtrate was concentrated under reduced pressure. The residue was purified by thin layer chromatography (petroleum ether: ethyl acetate = 1:2) to obtain a crude product, which was purified by preparative high performance liquid chromatography (ammonium bicarbonate conditions) to obtain compound 91. LCMS: m/z 448.2 [M+H]⁺; ¹H NMR (400 MHz, CDCl₃) δ: 7.20 (s, 4H), 7.16 (d, *J=* 8.9 Hz, 1H), 3.98 (s, 4H), 3.90 (d, *J= 4.3* Hz, 2H), 3.75 - 3.69 (m, 4H), 3.69 - 3.62 (m, 1H), 2.77 (s, 3H), 2.69 (dt, *J=* 2.9, 11.4 Hz, 1H), 2.65 - 2.53 (m, 2H), 2.49 - 2.41 (m, 1H), 1.98 - 1.90 (m, 1H), 1.85 - 1.77 (m, 1H), 1.77 - 1.72 (m, 1H), 1.67 - 1.54 (m, 3H), 1.19 - 1.06 (m, 1H).

### Example 90: Compound 92

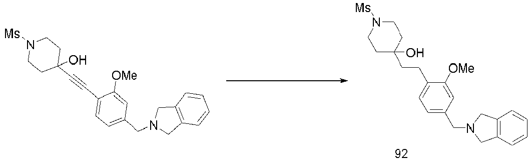

Under nitrogen atmosphere, palladium/carbon (22 mg, 0.20 mmol, 10 wt%) was added to a solution of 4-((4-(isoindolin-2-ylmethyl)-2-methoxyphenyl)ethynyl)-1-(methylsulfonyl)piperidin-4-ol (90 mg, 0.20 mmol) in methanol (10 mL) at 25 °C, and the mixture was stirred for 1 hour. LCMS indicated the reaction was completed. The reaction mixture was filtered, and the filtrate was concentrated under reduced pressure. The residue was purified by preparative high-performance liquid chromatography (ammonium bicarbonate conditions) to obtain compound 92. LCMS: m/z 445.3 [M+H]⁺; ¹H NMR (400 MHz, CDCl₃) δ: 7.12 (s, 4H), 7.02 (d, *J* = 7.5 Hz, 1H), 6.91 (s, 1H), 6.84 (d, *J =* 7.4 Hz, 1H), 3.88 (s, 4H), 3.82 (s, 2H), 3.78 (s, 3H), 3.52 (d, *J =* 11.5 Hz, 2H), 2.99 (dt, *J* = 5.2, 10.6 Hz, 2H), 2.72 (s, 3H), 2.66 - 2.61 (m, 2H), 1.74 - 1.66 (m, 6H).

### Example 91: Compound 93

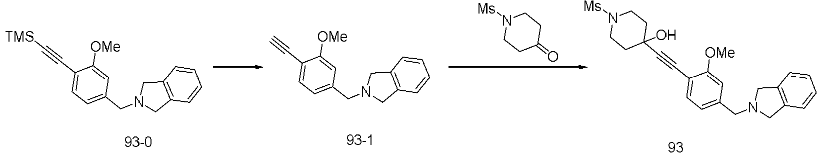

Step 1: At 25°C, tetrabutylammonium fluoride (1.09 g, 4.17 mmol) was added to a solution of starting material 93-0 (700 mg, 2.09 mmol) in tetrahydrofuran (5 mL), and the mixture was stirred for 1 hour. LCMS indicated the reaction was completed. The reaction mixture was diluted with ethyl acetate (20 mL), washed with water (20 mL), dried over anhydrous sodium sulfate, and filtered. The filtrate was concentrated under reduced pressure to obtain Intermediate 93-1. LCMS: m/z 264.0 [M+H]⁺.

Step 2: At -78°C, n-Butyllithium solution (0.36 mL, 0.57 mmol, 1.6 M) was added dropwise to a solution of Intermediate 93-1 (100 mg, 0.38 mmol) in tetrahydrofuran (4 mL). After being stirred for 30 minutes, the reaction mixture was added with 1-N-methanesulfonyl-4-piperidone (67 mg, 0.38 mmol), and continuously stirred for 40 minutes. LCMS indicated the reaction was completed. The reaction mixture was quenched by adding water (20 mL) and the mixture was extracted with ethyl acetate (20 mL×3). The organic phases were combined, washed with saturated brine (30 mL×2), dried over anhydrous sodium sulfate, and filtered. The filtrate was concentrated under reduced pressure, and the residue was purified by preparative high-performance liquid chromatography (ammonium bicarbonate conditions) to obtain compound 93. LCMS: m/z 441.2 [M+H]⁺; ¹H NMR (400 MHz, CDCl₃) δ: 7.32 (d, *J =* 7.8 Hz, 1H), 7.20 (s, 4H), 7.04 (s, 1H), 6.94 (d, *J =* 7.6 Hz, 1H), 3.96 (s, 4H), 3.91 (s, 2H), 3.89 (s, 3H), 3.58 - 3.50 (m, 2H), 3.30 (ddd, *J* = 3.3, 8.7, 11.9 Hz, 2H), 2.81 (s, 3H), 2.14 - 2.06 (m, 2H), 1.96 (ddd, *J=* 3.6, 8.8, 12.8 Hz, 3H).

### Example 92: Compound 94

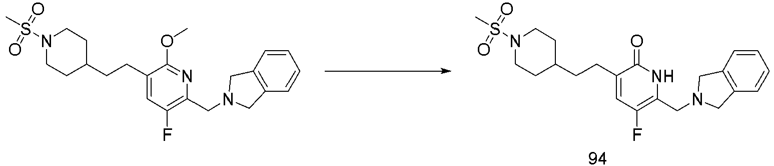

At 25 °C, hydrobromic acid-acetic acid solution (4 ml, 33 wt%) was added to 2-((3-fluoro-6-methoxy-5-(2-(1-(methylsulfonyl)piperidin-4-yl)ethyl)pyridin-2-yl)methyl)isoind oline (50 mg, 0.11 mmol), and the mixture was stirred at 60 °C for 2 hours. LCMS indicated the reaction was completed. The reaction mixture was concentrated under reduced pressure, and the residue was purified by preparative high-performance liquid chromatography (ammonium bicarbonate conditions) to obtain compound 94. LCMS: m/z 434.2 [M+H]⁺; ¹H NMR (400 MHz, CDCl₃) δ: 7.26 - 7.14 (m, 5H), 4.05 (s, 4H), 3.93 (d, *J =* 2.1 Hz, 2H), 3.79 (d, *J =* 11.9 Hz, 2H), 2.77 (s, 3H), 2.69 - 2.60 (m, 2H), 2.58 - 2.51 (m, 2H), 1.87 (d, *J=* 12.4 Hz, 2H), 1.62 - 1.53 (m, 2H), 1.41 - 1.24 (m, 3H).

### Example 93: Compound 95

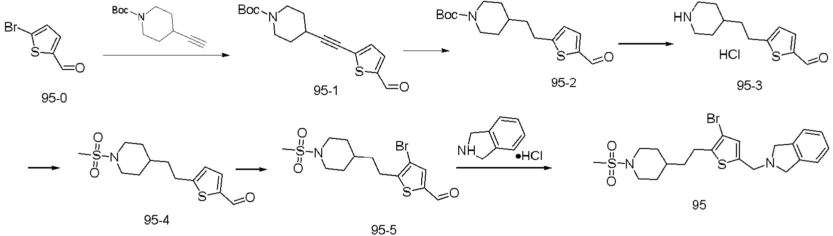

step 1: Starting material 95-0 (1.01 g, 5.23 mmol), tert-butyl 4-ethynylpiperidine-1-carboxylate (2.19 g, 10.47 mmol), bis(triphenylphosphine) palladium dichloride (180 mg, 0.26 mmol) and copper iodide (100 mg, 0.52 mmol) were added to triethylamine (3.6 mL) and tetrahydrofuran (10 mL). After purging with nitrogen three times, the mixture was stirred at 25°C for 3 hours. LCMS indicated the reaction was completed. The reaction mixture was filtered, and the filtrate was concentrated under reduced pressure. The residue was purified by silica gel column chromatography (petroleum ether: ethyl acetate = 5: 1) to obtain Intermediate 95-1. LCMS: m/z 220.0 [M-99]⁺.

Step 2: At 25°C, under nitrogen atmosphere, Intermediate 95-1 (1.00 g, 3.13 mmol) and palladium/carbon (0.33 g, 3.13 mmol, 10wt%) were added to isopropyl alcohol (10 mL). After purging with hydrogen three times, the mixture was stirred for 3 hours. LCMS indicated the reaction was completed. The reaction liquid was filtered, and the filtrate was concentrated under reduced pressure to obtain crude product Intermediate 95-2. LCMS: m/z 268.0 [M-55]⁺.

Step 3: At 25°C, hydrochloric acid-dioxane solution (5 mL, 4M) was added to Intermediate 95-2 (800 mg, 2.47 mmol), and the mixture was stirred for 1 hour. LCMS indicated the reaction was completed. The reaction mixture was concentrated under reduced pressure to obtain crude product Intermediate 95-3. LCMS: m/z 224.0 [M+H]⁺.

Step 4: At 25°C, methanesulfonic anhydride (780 mg, 4.48 mmol) was added to a solution of Intermediate 95-3 (500 mg, 2.24 mmol) and triethylamine (1.55 mL, 11.20 mmol) in dichloromethane (5 mL), and the mixture was stirred for 20 minutes. LCMS indicated the reaction was completed. The reaction mixture was diluted with water (30 mL) and extracted with ethyl acetate (30 mLx3). The organic phases were combined, washed with saturated sodium chloride solution (30 mLx1). The organic phase was dried with anhydrous sodium sulfate, filtered, and the filtrate was concentrated under reduced pressure. The residue was purified by silica gel column chromatography (petroleum ether: ethyl acetate = 1: 1) to obtain Intermediate 95-4. LCMS: m/z 319.0 [M+NH₄]⁺.

Step 5: At 25 °C, to a solution of Intermediate 95-4 (270 mg, 0.90 mmol) in acetonitrile (5 mL) were added N-bromosuccinimide (192 mg, 1.07 mmol) and ferric chloride (436 mg, 2.69 mmol). The mixture was stirred for at 25°C 2 hours. LCMS indicated the reaction was completed. The reaction was quenched by adding water (30 mL) and the mixture was extracted with ethyl acetate (30 mL×3). The organic phases were combined, washed with saturated brine (30 mL), and dried over anhydrous sodium sulfate. After filtration, the filtrate was concentrated under reduced pressure, and the residue was purified by silica gel column chromatography (petroleum ether: ethyl acetate = 2: 1) to obtain Intermediate 95-5. LCMS: m/z 397.1 [M+NH₄]⁺.

Step 6: At 25°C, acetic acid (0.07 mL, 1.18 mmol), sodium triacetoxyborohydride (100 mg, 0.47mmol) were added to a solution of Intermediate 95-5 (90 mg, 0.24 mmol), isoindoline hydrochloride (57 mg, 0.47 mmol) in 1,2-dichloroethane (5 mL), and the mixture was stirred for 2 hours. LCMS indicated the reaction was completed. The reaction was quenched with saturated aqueous sodium bicarbonate solution (20 mL) and the mixture was extracted with ethyl acetate (20 mL×3). The organic phases were combined, washed with saturated brine (20 mL), and dried over anhydrous sodium sulfate. After filtration, the filtrate was concentrated under reduced pressure, and the residue was purified by silica gel column chromatography (petroleum ether: ethyl acetate = 2:1) to obtain compound 95. LCMS: m/z 483.1 [M+H]⁺; ¹H NMR (400 MHz, CDCl₃) δ : 7.20 (s, 4H), 6.82 (s, 1H), 4.06 - 3.97 (m, 6H), 3.80 (br d, *J =* 12.0 Hz, 2H),2.83 - 2.75 (m, 5H), 2.68 - 2.60 (m, 2H), 1.87 (br dd, *J=* 1.7, 12.6 Hz, 2H), 1.68 - 1.60 (m, 2H), 1.44 - 1.30 (m, 3H).

### Example 94: Compound 96

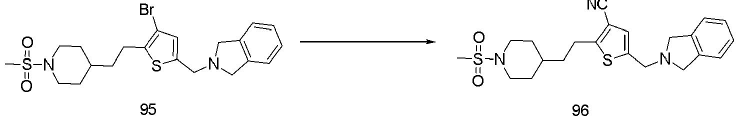

To a solution of compound 95 (40 mg, 0.08 mmol) in N,N-dimethylformamide (1 mL) were added zinc cyanide (7 mg, 0.06 mmol), zinc powder (1 mg, 0.01 mmol), 1, 1'-Bis(diphenylphosphine)ferrocene (3 mg, 0.01 mmol) and tris(dibenzylideneacetone)dipalladium (8 mg, 0.01 mmol). After purging with nitrogen three times, the mixture was heated to 110°C and stirred for 3 hours. LCMS indicated the reaction was completed. The reaction mixture was filtered, and the filtrate was concentrated under reduced pressure. The residue was purified by silica gel column chromatography (petroleum ether: ethyl acetate = 2: 1) to obtain compound 96. LCMS: m/z 430.2 [M+H]⁺; ¹H NMR (400 MHz, CDCl₃) δ : 7.21 (d, *J* = 1.3 Hz, 4H), 6.99 (s, 1H), 4.04 (s, 2H), 4.01 (s, 4H), 3.81 (br d, *J =* 12.1 Hz, 2H), 3.02 (t, *J =* 7.8 Hz, 2H), 2.77 (s, 3H), 2.70 - 2.61 (m, 2H), 1.90 - 1.84 (m, 2H), 1.75 - 1.68 (m, 2H), 1.45 - 1.34(m, 3H).

### Example 95: Compound 97

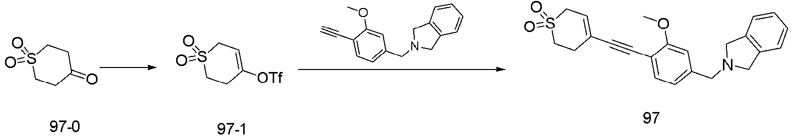

Step 1: At -78°C, lithium diisopropylamide-tetrahydrofuran solution (2.02 mL, 4.05 mmol, 2M) was added dropwise to a solution of tetrahydrothiopyran-4-one 1,1-dioxide 97-0 (0.40 g, 2.70 mmol) in tetrahydrofuran (10 mL), and the mixture was stirred for 1 hour. A solution of N-phenylbis(trifluoromethanesulfonyl)imide (1.93 g, 2.00 mmol) in tetrahydrofuran (5 mL) was added dropwise to the reaction system, and the mixture was continuously stirred for 2 hours. The reaction was quenched with saturated sodium bicarbonate solution (10 mL) and the mixture was extracted with ethyl acetate (10 mL). The organic phase was washed with saturated brine (20 mL), dried over anhydrous sodium sulfate, and filtered. The filtrate was concentrated under reduced pressure to obtain Intermediate 97-1 crude.

Step 2: At 25°C, the above crude Intermediate 97-1 product, 2-(4-ethynyl-3-methoxybenzyl)isoindoline (130 mg), triethylamine (0.34 mL, 2.47 mmol), copper iodide (5 mg, 0.02 mmol) and bis(triphenylphosphine)palladium dichloride (35 mg, 0.05 mmol) were dissolved in tetrahydrofuran (5 mL). After purging with nitrogen three times, the mixture was heated to 100 °C and stirred for 18 hours. LCMS indicated the reaction was completed. The reaction mixture was filtered, and the filtrate was concentrated under reduced pressure. The residue was purified by preparative high-performance liquid chromatography (ammonium bicarbonate conditions) to obtain compound 97. LCMS: m/z 394.2[M+H]⁺; ¹H NMR (400 MHz, CDCl₃) δ: 7.35 (d, *J =* 7.6 Hz, 1H), 7.21 (s, 4H), 7.04 (s, 1H), 6.95 (d, *J =* 7.6 Hz, 1H), 6.06 (ddd, *J =* 1.4, 3.1, 4.5 Hz, 1H), 3.97 (s, 4H), 3.93 (s, 2H), 3.90 (s, 3H), 3.79 - 3.73 (m, 2H), 3.21 - 3.16 (m, 2H), 3.08 - 3.00 (m, 2H).

### Example 96: Compound 98

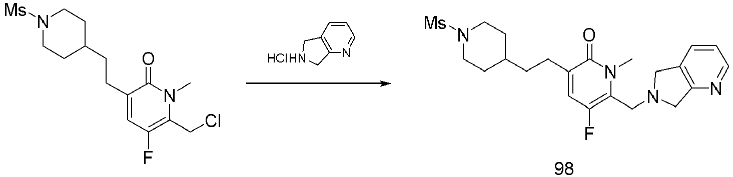

At 80 °C, potassium carbonate (115 mg, 0.82 mmol) was added to a solution of 6-(chloromethyl)-5-fluoro-1-methyl-3-(2-(1-(methylsulfonyl)piperidin-4-yl)ethyl)pyridin-2(1H)-one (100 mg, 0.27 mmol) and 6,7-dihydro-5H-pyrrolo[3,4-b]pyridine hydrochloride (80 mg, 0.41 mmol) in N,N-dimethylformamide (2 mL), and the mixture was stirred for 2 hours. LCMS indicated the reaction was completed. The reaction mixture was cooled to 25 °C, added with water (20 mL) and extracted with ethyl acetate (20 mL×3). The organic phases were collected, washed with saturated brine (60 mL), and purified by preparative high performance liquid chromatography (ammonium bicarbonate conditions) to obtain compound 98. LCMS: m/z 449.2 [M+H]⁺; ¹H NMR (400MHz, CDCl₃) δ: 8.41 (d, *J* = 4.6 Hz, 1H), 7.51 (d, *J* = 7.4 Hz, 1H), 7.11-7.19 (m, 2H), 3.99-4.09 (m, 4H), 3.94 (d, *J* = 4.1 Hz, 2H), 3.81 (br d, *J =* 12.0 Hz, 2H), 3.72 (s, 3H), 2.78 (s, 3H), 2.56-2.72 (m, 4H), 1.85-1.99 (m, 2H), 1.53-1.60 (m, 2H), 1.28-1.44 (m, 3H).

### Example 97: Compound 99

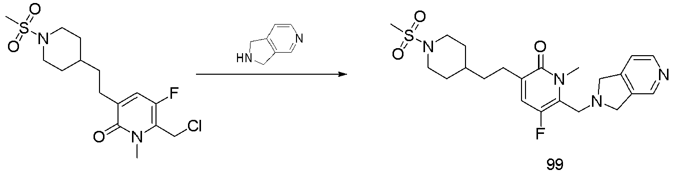

At 25 °C , to a solution of 6-(chloromethyl)-5-fluoro-1-methyl-3-(2-(1-(methylsulfonyl)piperidin-4-yl)ethyl)pyridin-2(1H)-one (120 mg, 0.33 mmol) in N,N-dimethylformamide (4 mL) were added 2,3-dihydropyrro[3,4-c]pyridine (59 mg, 0.49 mmol) and potassium carbonate (136 mg, 0.99 mmol). The reaction mixture was heated to 80° C and stirred for 1 hour. LCMS indicated the reaction was completed. The reaction mixture was cooled to 25° C. The reaction was quenched with water and the mixture was extracted with ethyl acetate (30 mL × 3). The organic phases were combined, washed with saturated brine (30 mL), and dried over anhydrous sodium sulfate. After filtration, the filtrate was concentrated under reduced pressure, and the residue was purified by silica gel column chromatography (dichloromethane: methanol = 10:1) to obtain compound 99. LCMS: m/z 449.2 [M+H]⁺; ¹H NMR (400 MHz, CDCl₃) δ : 8.52 - 8.43 (m, 2H), 7.20 - 7.13 (m, 2H), 4.01 (br d, *J =* 16.4 Hz, 4H), 3.93 (d, *J* = 4.0 Hz, 2H), 3.80 (br d, *J =* 12.0 Hz, 2H), 3.70 (s, 3H), 2.77 (s, 3H), 2.70 - 2.62 (m, 2H), 2.62 - 2.56 (m, 2H), 1.92 - 1.87(m, 3H), 1.61 - 1.55 (m, 2H), 1.38 (dt, *J* = 3.8*,* 12.2 Hz, 2H).

### Example 98: Compound 100

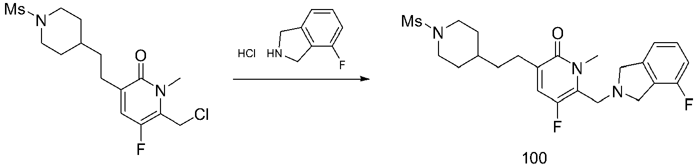

At 80 °C , potassium carbonate (203 mg, 1.47 mmol) was added to 6-(chloromethyl)-5-fluoro-1-methyl-3-(2-(1-(methylsulfonyl)piperidin-4-yl)ethyl)pyridin-2(1H)-one (170 mg, 0.49 mmol) and 4-fluoroisoindoline hydrochloride (128 mg, 0.74 mmol) in a solution of N,N-dimethylformamide (2 mL), and the mixture was stirred for 2 hours. LCMS indicated the reaction was completed. The reaction mixture was cooled to 25 °C, added with water (20 mL) and extracted with ethyl acetate (20 mL×3). The organic phases were collected, washed with saturated brine (60 mL)and purified by preparative high performance liquid chromatography (ammonium bicarbonate conditions) to obtain compound 100. LCMS: m/z 466.1 [M+H]⁺; ¹H NMR (400MHz, CDCl₃) δ: 7.20 (td, *J* = 7.8, 5.2 Hz, 1H), 7.15 (d, *J* = 9.0 Hz, 1H), 6.98 (d, *J =* 7.5 Hz, 1H), 6.91 (t, *J =* 8.6 Hz, 1H), 4.03 (br d, *J =* 12.6 Hz, 4H), 3.92 (d, *J* = 4.0 Hz, 2H), 3.81 (br d, *J* = 12.0 Hz, 2H), 3.72 (s, 3H), 2.78 (s, 3H), 2.63-2.71 (m, 2H), 2.56-2.62 (m, 2H), 1.85-1.94 (m, 2H), 1.56 (br s, 2H), 1.30-1.51 (m, 3H).

### Example 99: Compound 101

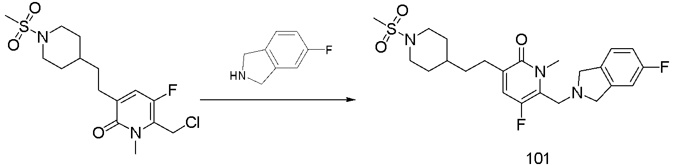

At 25 °C , to a solution of 6-(chloromethyl)-5-fluoro-1-methyl-3-(2-(1-(methylsulfonyl)piperidin-4-yl)ethyl)pyridin-2(1H)-one (120 mg, 0.33 mmol) in N,N-dimethylformamide (4 mL) was added 5-fluoroisoindoline (68 mg, 0.49 mmol) and potassium carbonate (136 mg, 0.99 mmol). The reaction mixture was heated to 80° C and stirred for 1.5 hours. LCMS indicated the reaction was completed. The reaction mixture was cooled to 25 °C. The reaction was quenched with water and the mixture was extracted with ethyl acetate (40 mL × 3). The organic phases were combined, washed with saturated brine (40 mL), and dried over anhydrous sodium sulfate. After filtration, the filtrate was concentrated under reduced pressure, and the residue was purified by silica gel column chromatography (petroleum ether: ethyl acetate = 1:1) to obtain compound 101. LCMS: m/z 466.2 [M+H]⁺; ¹H NMR (400 MHz, CDCl₃) δ : 7.17 - 7.09 (m, 2H), 6.93 - 6.85 (m, 2H), 3.94 (br d, *J* = 10.4 Hz, 4H), 3.89 (d, *J=* 4.1 Hz, 2H), 3.79 (br d, *J=* 12.0 Hz, 2H), 3.71 (s, 3H), 2.77 (s, 3H), 2.70 - 2.61 (m, 2H), 2.61 - 2.55 (m, 2H), 1.89 (br dd, *J=* 1.7, 12.6 Hz, 2H), 1.62 - 1.53 (m, 2H), 1.48 - 1.29 (m, 3H).

### Example 100: Compound 102

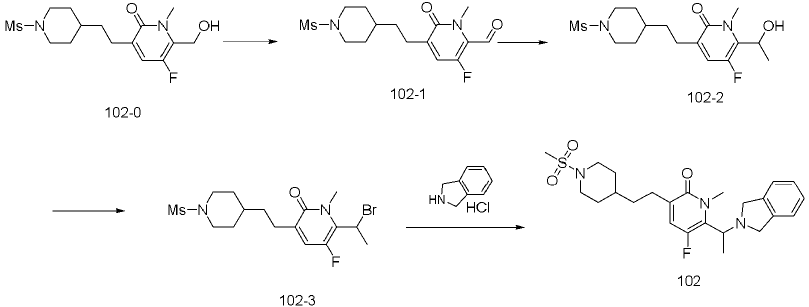

Step 1: At 25°C, starting material 102-0 (500 mg, 1.44 mmol) and manganese dioxide (2.50 g, 28.8 mmol) were added to the dichloromethane (10 mL) solution. The reaction mixture was heated to 60 °C and stirred for 3 hours. LCMS indicated the reaction was completed. The reaction mixture was cooled to 25°C and concentrated under reduced pressure to obtain Intermediate 102-1. LCMS: m/z 345.0 [M+H]⁺.

Step 2: Intermediate 102-1 (150 mg, 0.44 mmol) was added to tetrahydrofuran (4 mL). After purging with nitrogen three times, the reaction mixture was cooled to -78 °C, added with methylmagnesium bromide (1.31 mL, 1 M) and stirred for 0.5 hours. LCMS indicated the reaction was completed. The reaction was quenched with water (10 mL) and the mixture was extracted with ethyl acetate (10 mL×3). The organic phases were combined, washed with saturated brine (10 mL), and dried over anhydrous sodium sulfate. After filtration, the filtrate was concentrated under reduced pressure, and the residue was purified by silica gel column chromatography (petroleum ether: ethyl acetate = 1: 1) to obtain Intermediate 102-2. LCMS: m/z 361.2 [M+H]⁺.

Step 3: At 0°C, to a solution of Intermediate 102-2 (40 mg, 0.11 mmol) in dichloromethane (2 mL) was added phosphorus tribromide (0.02 mL, 0.17 mmol), and the mixture was stirred for 0.5 hours. LCMS indicated the reaction was completed. The reaction was quenched with saturated sodium bicarbonate solution (5 mL) and the mixture was extracted with ethyl acetate (10 mL×2). The organic phases were combined, washed with saturated brine (10 mL), and dried over anhydrous sodium sulfate. After filtration, the filtrate was concentrated under reduced pressure, and the residue was purified by silica gel column chromatography (petroleum ether: ethyl acetate = 1: 1) to obtain Intermediate 102-3. LCMS: m/z 423.1 [M+H]⁺.

Step 4: At 25°C, to a solution of Intermediate 102-3 (20 mg, 0.05 mmol) in N,N-dimethylformamide (1 mL) were added isoindoline hydrochloride (17 mg, 0.14 mmol) and potassium carbonate (20 mg, 0.14 mmol) The reaction mixture was heated to 60°C and stirred for 0.5 hours. LCMS indicated the reaction was completed. The reaction mixture was cooled to 25°C. The reaction was quenched with water (10 mL) and the mixture was extracted with ethyl acetate (30 mL×3). The organic phases were combined, washed with saturated brine (100 mL), and dried over anhydrous sodium sulfate. After filtration, the filtrate was concentrated under reduced pressure, and the residue was subjected to preparative high performance liquid chromatography (ammonium bicarbonate system) to obtain compound 102. LCMS: m/z 462.2 [M+H]⁺; ¹H NMR (400 MHz, CDCl₃) δ : 7.24 - 7.17 (m, 4H), 7.13 (d, *J =* 9.8 Hz, 1H), 4.38 (dq, *J=* 1.8, 6.9 Hz, 1H),4.03 (d, *J=* 11.1 Hz, 2H), 3.92 (s, 3H), 3.82 (br d, *J=* 11.0 Hz, 4H), 2.78 (s, 3H), 2.73 - 2.63 (m, 2H), 2.62 - 2.55 (m, 2H),2.01 (s, 1H), 1.91 (br d, *J =* 12.4 Hz, 2H), 1.61 - 1.56 (m, 5H), 1.43 - 1.32 (m, 2H).

### Example 101: Compound 103

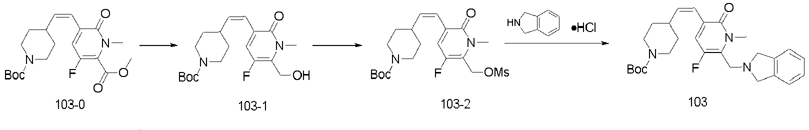

Step 1: At 25 °C, to a solution of starting material 103-0 (200 mg, 0.51 mmol) in tetrahydrofuran (10 mL), sodium borohydride (384 mg, 10.14 mmol) and lithium chloride (430 mg, 10.14 mmol) were added, and the mixture was stirred for 12 hours. LCMS indicated the reaction was completed. The reaction mixture was diluted with water (30 mL) and extracted with ethyl acetate (30 mLx3). The organic phases were combined, washed with saturated sodium chloride solution (30 mLx1). The organic phase was dried with anhydrous sodium sulfate, filtered, and the filtrate was concentrated under reduced pressure. The residue was purified by silica gel column chromatography (petroleum ether: ethyl acetate = 2: 1) to obtain Intermediate 103-1. LCMS: m/z 367.2 [M+H]⁺.

Step 2: At 25 °C, to a solution of Intermediate 103-1 (60 mg, 0.16 mmol) in dichloromethane (3 mL), triethylamine (0.07 mL, 0.49 mmol) and methylsulfonic anhydride (57 mg, 0.33 mmol) were added, and the mixture was stirred for 5 minutes. LCMS indicated the reaction was completed. The reaction mixture was diluted with water (20 mL) and extracted with ethyl acetate (20 mLx3). The organic phases were combined, washed with saturated sodium chloride solution (20 mLx1). The organic phase was dried with anhydrous sodium sulfate, filtered, and the filtrate was concentrated under reduced pressure. The residue was purified by silica gel column chromatography (petroleum ether: ethyl acetate = 1: 1) to obtain Intermediate 103-2. LCMS: m/z 345.2 [M-99]⁺.

Step 3: At 25 °C , to a solution of Intermediate 103-2 (60 mg, 0.13 mmol) in N,N-dimethylformamide (3 mL) was added isoindoline hydrochloride (49 mg, 0.40 mmol) and potassium carbonate (56 mg, 0.40 mmol), and the mixture was stirred for 1 hour. LCMS indicated the reaction was completed. The reaction was quenched by adding water (30 mL) and the mixture was extracted with ethyl acetate (30 mL × 3). The organic phases were combined, washed with saturated brine (30 mL), and dried over anhydrous sodium sulfate. After filtration, the filtrate was concentrated under reduced pressure, and the residue was purified by silica gel column chromatography (petroleum ether: ethyl acetate = 1: 1) to obtain compound 103. LCMS: m/z 468.3 [M+H]⁺; ¹H NMR (400 MHz, CDCl₃) δ : 7.23 - 7.19 (m, 5H), 6.45 (d, *J =* 11.8 Hz, 1H), 5.68 - 5.60 (m, 1H), 4.06 - 3.98(m, 5H), 3.94 (br d, *J=* 3.9 Hz, 2H), 3.74 (s, 3H), 2.79 (br t, *J= 12.3* Hz, 2H), 2.64 - 2.55 (m, 1H), 1.77 - 1.64 (m, 5H), 1.48(s, 9H).

### Example 102: Compound 104

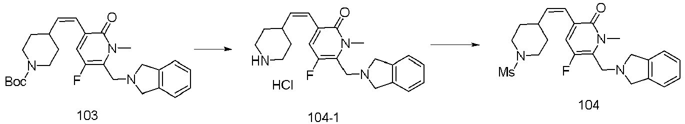

Step 1: Hydrochloric acid-dioxane solution (1 mL, 4M) was added to compound 103 (25 mg, 0.05 mmol) at 25 °C, and the mixture was stirred for 0.5 hours. LCMS indicated the reaction was completed. The reaction mixture was concentrated under reduced pressure to obtain Intermediate 104-1. LCMS: m/z 368.2 [M+H]⁺.

Step 2: At 25°C, methanesulfonic anhydride (20 mg, 0.11 mmol) was added to a solution of Intermediate 104-1 (20 mg, 0.05 mmol) and triethylamine (17 mg, 0.16 mmol) in dichloromethane (1 mL), and the mixture was stirred for 5 minutes. LCMS indicated the reaction was completed. The reaction mixture was diluted with water (10 mL) and extracted with ethyl acetate (10 mLx3). The organic phases were combined, washed with saturated sodium chloride solution (10 mLx1). The organic phase was dried over anhydrous sodium sulfate, filtered, and the filtrate was concentrated under reduced pressure. The residue was purified by preparative high performance liquid chromatography (ammonium bicarbonate system) to obtain compound 104. LCMS: m/z 446.2 [M+H]⁺; ¹H NMR (400 MHz, CDCl₃) δ = 7.25 - 7.16 (m, 5H), 6.47 (d, *J* = 11.9 Hz, 1H), 5.71 - 5.62 (m, 1H), 4.02 (br s,4H), 3.95 (br s, 2H), 3.81 (br d, *J=* 11.9 Hz, 2H), 3.75 (s, 3H), 2.80 (s, 3H), 2.75 (dt, *J* = 2.3, 12.0 Hz, 2H), 1.88 - 1.80 (m,2H), 1.67 - 1.61 (m, 3H).

### Example 103: Compound 105

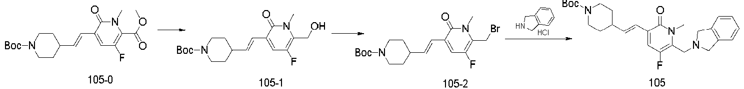

Step 1: At 25 °C, sodium borohydride (1.53 g, 40.60 mmol) was added to a solution of starting material 105-0 (800 mg, 2.03 mmol) and lithium chloride (860 mg, 20.28 mmol) in tetrahydrofuran (10 mL), and the mixture was stirred for 6 hours. LCMS indicated the reaction was completed. The reaction mixture was extracted with ethyl acetate (20 mL×3). The organic phase was collected, and washed with saturated brine (60 mL). The combined organic phases were concentrated under reduced pressure to obtain Intermediate 105-1. LCMS: m/z 367.2 [M+H]⁺.

Step 2: At 25 °C, Intermediate 105-1 (300 mg, 0.31 mmol) was added to a solution of carbon tetrabromide (544 mg, 1.64 mmol) and triphenylphosphine (430 mg, 1.64 mmol) in dichloromethane (3 mL), and the mixture was stirred for 2 hours. LCMS indicated the reaction was completed. The reaction mixture was extracted with ethyl acetate (5 mL×3). The organic phase was collected, and washed with saturated brine (15 mL). The organic phase was concentrated under reduced pressure, and the residue was purified by thin layer chromatography (petroleum ether: ethyl acetate = 2: 1) to obtain Intermediate 105-2.

Step 3: At 25 °C, a solution of Intermediate 105-2 (50 mg, 0.11 mmol), isoindoline hydrochloride (30 mg, 0.17 mmol) and potassium carbonate (47 mg, 0.34 mmol) in N,N-dimethylformamide (2 mL) was heated to 60 °C and stirred for 3 hours. LCMS indicated the reaction was completed. The solution was cooled to 25 °C. The reaction was quenched with water (5 mL) and the mixture was extracted with dichloromethane (5 mL × 3). The organic phases were collected and washed with saturated brine (15 mL). The organic phase was concentrated under reduced pressure, and the residue was purified by preparative high-performance liquid chromatography (ammonium bicarbonate conditions) to obtain compound 105. LCMS: m/z 468.3 [M+H]⁺; ¹H NMR (400MHz, CDCl₃) δ: 7.32 (d, *J =* 9.5 Hz, 1H), 7.17 - 7.25 (m, 4H), 6.56 (d, *J* = 2.8 Hz, 2H), 4.08 - 4.21 (m, 2H), 4.02 (s, 4H), 3.95 (d, *J =* 3.9 Hz, 2H), 3.75 (s, 3H), 2.78 (br t, *J =* 11.8 Hz, 2H), 2.28 - 2.39 (m, 1H), 1.77 (br s, 2H), 1.47 (s, 9H), 1.39 (qd, *J =* 12.3, 4.4 Hz, 2H).

### Example 104: Compound 106

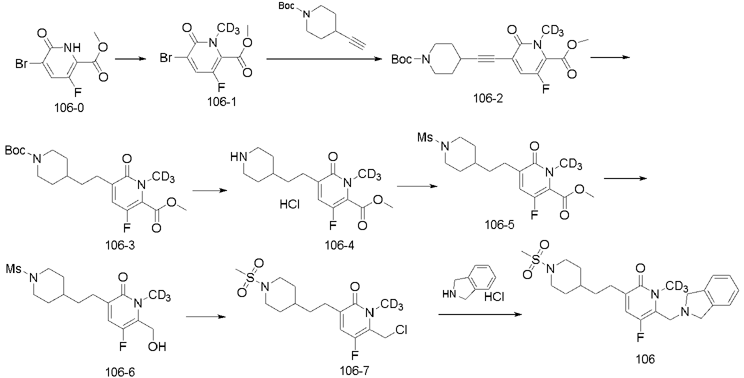

Step 1: At 25°C, potassium carbonate (1.66 g, 12.02 mmol) was added to a solution of starting material 106-0 (1.50 g, 6.02 mmol) and deuterated methyl iodide (1.31 g, 9.03 mmol) in N,N-dimethylformamide (10 mL). The reaction mixture was stirred at 25°C for 3 hours, and LCMS indicated the reaction was completed. The reaction was quenched by adding water (50 mL) and the mixture was extracted with ethyl acetate (50 mL×3). The organic phases were combined, washed with saturated brine (50 mL), dried over anhydrous sodium sulfate, filtered, and the filtrate was concentrated under reduced pressure. The residue was purified by silica gel column chromatography (petroleum ether: ethyl acetate = 1: 1.5) to obtain Intermediate 106-1. LCMS: m/z 266.9 [M+H]⁺.

Step 2: At 25°C, Intermediate 106-1 (540 mg, 2.03 mmol), tert-butyl 4-ethynylpiperidine-1-carboxylate (849 mg, 4.06 mmol), copper iodide (39 mg, 0.21 mmol) and triethylamine ( 4 mL) were added to tetrahydrofuran (4 mL), followed by bis(triphenylphosphine)palladium dichloride (143 mg, 0.20 mmol). After purging with nitrogen three times, the mixture was stirred for 2 hours. LCMS indicated the reaction was completed. The reaction mixture was filtered, and the filtrate was concentrated under reduced pressure. The residue was purified by silica gel column (petroleum ether: ethyl acetate = 1: 1) to obtain Intermediate 106-2. LCMS: m/z 340.0 [M-55]⁺.

Step 3: At 25 °C, under nitrogen atmosphere, palladium/carbon (300 mg, 0.27 mmol, 10wt %) was added to a solution of Intermediate 106-2 (600 mg, 1.51 mmol) in ethyl acetate (10 mL). After purging with hydrogen three times, and the mixture was stirred for 18 hours. LCMS indicated the reaction was completed. The reaction mixture was filtered, and the filtrate was concentrated under reduced pressure to obtain crude product Intermediate 106-3. LCMS: m/z 300.1 [M-99]⁺.

Step 4: A solution of Intermediate 106-3 (500 mg, 1.25 mmol) in hydrochloric acid-dioxane (6 mL, 4M/L) was stirred at 25 °C for 30 minutes. LCMS indicated the reaction was completed, and the reaction mixture was concentrated under reduced pressure to obtain crude product Intermediate 106-4. LCMS: m/z 300.1 [M+H]⁺.

Step 5: At 25 °C, methanesulfonic anhydride (322 mg, 1.85 mmol) was added to a solution of Intermediate 106-4 (370 mg, 1.10 mmol) and triethylamine (390 mg, 3.86 mmol) in dichloromethane (10 mL), and the mixture was stirred for 30 minutes. LCMS indicated the reaction was completed. The reaction was quenched with saturated ammonium chloride solution (10 mL) and the mixture was extracted with dichloromethane (10 mL×2). The combined organic phase was washed with saturated brine (20 mL), dried over anhydrous sodium sulfate, and filtered. The filtrate was concentrated under reduced pressure. The residue was purified by silica gel column (petroleum ether: ethyl acetate = 1: 2) to obtain Intermediate 106-5. LCMS: m/z 378.1 [M+H]⁺.

Step 6: At 25°C, sodium borohydride (564 mg, 14.84 mmol) and lithium chloride (624 mg, 14.85 mmol) were added to a solution of Intermediate 106-5 (280 mg, 0.74 mmol) in tetrahydrofuran (15 mL), and the mixture was stirred for 48 hours. LCMS indicated the reaction was completed. The reaction was quenched with water (10 mL) and the mixture was extracted with dichloromethane (10 mL×2). The combined organic phase was washed with saturated brine (20 mL), dried over anhydrous sodium sulfate, and filtered. The filtrate was concentrated to obtain Intermediate 106-6. LCMS: m/z 350.1 [M+H]⁺.

Step 7: At 25°C, thionyl chloride (136 mg, 1.14 mmol) was added to a solution of Intermediate 106-6 (200 mg, 0.57 mmol) in dichloromethane (4 mL) and the mixture was stirred for 30 minutes. LCMS indicated the reaction was completed, and the reaction mixture was concentrated to obtain crude product Intermediate 106-7. LCMS: m/z 368.1 [M+H]⁺.

Step 8: A solution of potassium carbonate (236 mg, 1.71 mmol), isoindoline hydrochloride (178 mg, 1.14 mmol) and Intermediate 106-7 (210 mg, 0.57 mmol) in N,N-dimethylformamide (3 mL) was heated to 50 °C and stirred for 30 minutes. LCMS indicated the reaction was completed, the reaction mixture was filtered, the filtrate was concentrated under reduced pressure, and the residue was purified by preparative high-performance liquid chromatography (ammonium bicarbonate conditions) to obtain compound 106. LCMS: m/z 451.2 [M+H]⁺; ¹H NMR (400 MHz, CDCl₃) δ: 7.20 (s, 4H), 7.15 (d, *J =* 8.9 Hz, 1H), 4.00 (s, 4H), 3.92 (s, 2H), 3.79 (d, *J* =11.9 Hz, 2H), 2.77 (s, 3H), 2.70 - 2.56 (m, 4H), 1.89 (d, *J =* 12.0 Hz, 2H), 1.62 - 1.54 (m, 2H), 1.50 - 1.28 (m, 3H).

### Example 105: Compound 107

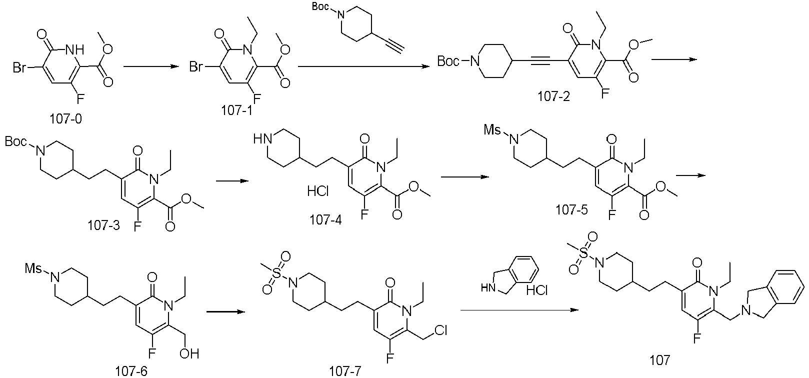

Step 1: At 25°C, potassium carbonate (1.66 g, 12.02 mmol) was added to a solution of starting material 107-0 (1.50 g, 6.02 mmol) and ethyl iodide (1.40 g, 8.97 mmol) in N,N-dimethylformamide (10 mL), and the reaction was stirred at 25°C for 3 hours. LCMS indicated the reaction was completed. The reaction was quenched by adding water (50 mL) and the mixture was extracted with ethyl acetate (50 mL×3). The organic phases were combined, washed with saturated brine (50 mL), dried over anhydrous sodium sulfate, filtered, and the filtrate was concentrated under reduced pressure. The residue was purified by silica gel column chromatography (petroleum ether: ethyl acetate = 1: 1.5) to obtain Intermediate 107-1. LCMS: m/z 277.9 [M+H]⁺.

Step 2: At 25°C, Intermediate 107-1 (180 mg, 0.65 mmol), tert-butyl 4-ethynylpiperidine-1-carboxylate (271 mg, 1.29 mmol), copper iodide (12 mg, 0.06 mmol) and triethylamine ( 2 mL) were added to tetrahydrofuran (2 mL), followed by bis(triphenylphosphine)palladium dichloride (45 mg, 0.06 mmol). After purging with nitrogen three times, the mixture was stirred for 2 hours. LCMS indicated the reaction was completed. The reaction mixture was filtered, and the filtrate was concentrated under reduced pressure. The residue was purified by silica gel column (petroleum ether: ethyl acetate = 1: 1) to obtain Intermediate 107-2. LCMS: m/z 307.1 [M-99]⁺.

Step 3: At 25°C, under nitrogen atmosphere, palladium/carbon (100 mg, 0.09 mmol, 10wt %) was added to a solution of Intermediate 107-2 (200 mg, 0.49 mmol) in ethyl acetate (8 mL). After purging with hydrogen three times, the mixture was stirred for 18 hours. LCMS indicated the reaction was completed. the reaction mixture was filtered, and the filtrate was concentrated under reduced pressure to obtain Intermediate 107-3. LCMS: m/z 311.1 [M-99]⁺.

Step 4: A solution of Intermediate 107-3 (180 mg, 0.44 mmol) in HCl-dioxane (6 mL, 4M) was stirred at 25 °C for 30 min. LCMS indicated the reaction was completed and the reaction mixture was concentrated under reduced pressure to obtain crude product Intermediate 107-4. LCMS: m/z 311.1 [M+H]⁺.

Step 5: At 25°C, methanesulfonic anhydride (126 mg, 0.40 mmol) was added to a solution of Intermediate 107-4 (150 mg, 0.48 mmol) and triethylamine (171 mg, 1.69 mmol) in dichloromethane (6 mL), and the mixture was stirred for 30 minutes. LCMS indicated the reaction was completed. The reaction was quenched with saturated ammonium chloride solution (10 mL) and the mixture was extracted with dichloromethane (10 mL×2). The combined organic phase was washed with saturated brine (20 mL), dried over anhydrous sodium sulfate, and filtered. The filtrate was concentrated under reduced pressure. The residue was purified by silica gel column (petroleum ether: ethyl acetate = 1: 2) to obtain Intermediate 107-5. LCMS: m/z 389.1 [M+H]⁺.

Step 6: Sodium borohydride (235 mg, 6.18 mmol) and lithium chloride (259 mg, 6.16 mmol) were added to a solution of Intermediate 107-5 (120 mg, 0.31 mmol) in tetrahydrofuran (10 mL) at 25 °C. The mixture was stirred for 48 hours. LCMS indicated the reaction was completed. The reaction was quenched with water (10 mL) and the mixture was extracted with dichloromethane (10 mL×2). The combined organic phase was washed with saturated brine (20 mL), dried over anhydrous sodium sulfate, and filtered. The filtrate was concentrated to obtain Intermediate 107-6. LCMS: m/z 361.1 [M+H]⁺.

Step 7: Thionyl chloride (66 mg, 0.55 mmol) was added to a solution of Intermediate 107-6 (100 mg, 0.27 mmol) in dichloromethane (3 mL) at 25 °C, and the mixture was stirred for 30 minutes. LCMS indicated the reaction was completed, and the reaction mixture was concentrated to obtain crude product Intermediate 107-7. LCMS: m/z 379.1 [M+H]⁺.

Step 8: A solution of potassium carbonate (109 mg, 0.78 mmol), isoindoline hydrochloride (82 mg, 0.52 mmol) and Intermediate 107-7 (100 mg, 0.26 mmol) in N,N-dimethylformamide (3 mL) solution was heated to 80 °C and stirred for 30 minutes. LCMS indicated the reaction was completed. The reaction mixture was filtered, and the filtrate was purified by preparative high-performance liquid chromatography (ammonium bicarbonate conditions) to obtain compound 107. LCMS: m/z 462.2 [M+H]⁺. ¹H NMR (400 MHz, CDCl₃) δ: 7.22 (s, 4H), 7.14 (d, *J= 9.1* Hz, 1H), 4.34 (d, *J =* 6.6 Hz, 2H), 4.28 - 3.84 (m, 6H), 3.81 (d, *J =* 12.0 Hz, 2H), 2.78 (s, 3H), 2.70 - 2.57 (m, 4H), 1.94 - 1.86 (m, 2H), 1.60 - 1.55 (m, 2H), 1.46 - 1.30 (m, 6H).

### Example 106: Compound 108

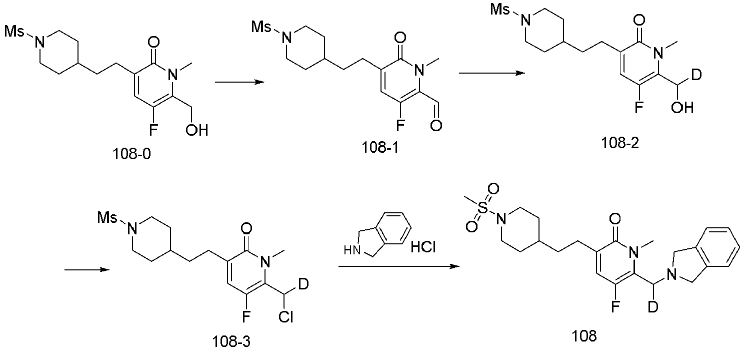

Step 1: Manganese dioxide (1.25 g, 14.36 mmol) was added to a solution of starting material 108-0 (250 mg, 0.72 mmol) in dichloromethane (8 mL) at 25 °C, and the reaction mixture was stirred at 25 °C for 18 hours. LCMS indicated the reaction was completed. The reaction mixture was filtered, the filter cake was washed with dichloromethane (50 mL×3), and the filtrate was concentrated under reduced pressure to obtain crude product Intermediate 108-1. LCMS: m/z 345.2 [M+H]⁺.

Step 2: Sodium deuterated borohydride (51 mg, 1.21 mmol) was added to a solution of Intermediate 108-1 (140 mg, 0.41 mmol) in methanol (5 mL) at 25°C, and the mixture was stirred for 4 hours. LCMS indicated the reaction was completed. The reaction mixture was quenched by adding water (30 mL) and the mixture was extracted with ethyl acetate (30 mL×3). The organic phases were combined, washed with saturated brine (30 mL), and dried over anhydrous sodium sulfate and filtered. The filtrate is concentrated under reduced pressure to obtain crude product Intermediate 108-2. LCMS: m/z 348.2 [M+H]⁺.

Step 3: Thionyl chloride (100 mg, 0.84 mmol) was added to a solution of Intermediate 108-2 (140 mg, 0.40 mmol) in dichloromethane (5 mL), and the reaction mixture was stirred at 25°C for 1 hour. LCMS indicated the reaction was completed. The reaction mixture was concentrated under reduced pressure to obtain Intermediate 108-3. LCMS: m/z 366.2 [M+H]⁺.

Step 4: At 25°C, potassium carbonate (160 mg, 1.16 mmol) was added to a solution of Intermediate 108-3 (140 mg, 0.38 mmol) and isoindoline hydrochloride (120 mg, 0.77 mmol) in N,N-dimethylformamide (4 mL), and the reaction mixture was stirred at 50°C for 0.5 hours. LCMS indicated the reaction was completed. The reaction mixture was diluted with water (20 mL) and extracted with ethyl acetate (20 mLx3). The organic phases were combined and washed with saturated sodium chloride solution (20 mLx1). The organic phase was dried over anhydrous sodium sulfate, filtered, and the filtrate was concentrated under reduced pressure, and the residue The residue was purified by preparative high performance liquid chromatography (ammonium bicarbonate system) to obtain compound 108. LCMS: m/z 449.2 [M+H]⁺; ¹H NMR (400 MHz, CDCl₃) δ: 7.21 (s, 4H), 7.15 (d, *J* = 9.0 Hz, 1H), 3.99 (s, 4H), 3.91 (d, *J* = 4.3 Hz, 1H), 3.80 (d, *J* = 11.8 Hz, 2H), 3.73 (s, 3H), 2.77 (s, 3H), 2.71 - 2.55 (m, 4H), 1.90 (d, *J =* 12.0 Hz, 2H), 1.63 - 1.54 (m, 2H), 1.38(dt, *J* = 3.4, 12.1 Hz, 3H).

### Example 107: Compound 109

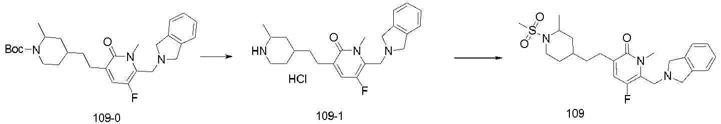

Step 1: Hydrochloric acid-dioxane solution (1 mL, 4.00 mmol, 4M) was added to starting material 109-0 (50 mg, 0.10 mmol) at 25 °C, and the mixture was stirred for 1 hour. LCMS indicated the reaction was completed. The reaction mixture was concentrated under reduced pressure to obtain crude product Intermediate 109-1. LCMS: m/z 384.0 [M+H]⁺.

Step 2: At 25°C, methanesulfonic anhydride (27 mg, 0.16 mmol) was added to a solution of Intermediate 109-1 (30 mg, 0.08 mmol) and triethylamine (24 mg, 0.23 mmol) in dichloromethane (1 mL), and the mixture was stirred for 10 minutes. LCMS indicated the reaction was completed. The reaction mixture was diluted with water (10 mL) and extracted with ethyl acetate (10 mL×3). The organic phases were combined, washed with saturated sodium chloride solution (10 mL×1). The organic phase was dried over anhydrous sodium sulfate, filtered, and the filtrate was reduced to pressure concentration. The residue was purified by preparative high performance liquid chromatography (ammonium bicarbonate) to obtain compound 109. LCMS: m/z 462.2 [M+H]⁺; ¹H NMR (400 MHz, CDCl₃) δ: 7.21 (d, *J =* 1.5 Hz, 4H), 7.14 (d, *J =* 8.9 Hz, 1H), 4.32 - 4.24 (m, 1H), 3.99 (s, 4H), 3.91 (d, *J =* 3.8 Hz, 2H), 3.73 (s, 3H), 3.71 - 3.67 (m, 1H), 3.05 (dt, *J =* 2.1, 13.1 Hz, 1H), 2.85 (s, 3H), 2.63 - 2.53 (m, 2H), 2.01 (s, 2H), 1.83 (d, *J=* 13.4 Hz, 1H), 1.71 (br d, *J=* 13.4 Hz, 2H), 1.54 - 1.48 (m, 2H), 1.26 (d, *J=* 6.5 Hz, 3H).

### Example 108: Compound 110

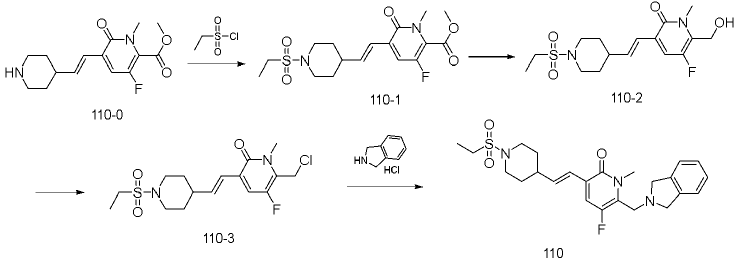

Step 1: Ethylsulfonyl chloride (393 mg, 3.06 mmol) was added dropwise into a solution of starting material 110-0 (300 mg, 1.02 mmol) in dichloromethane (5 mL) at 25 °C, and the mixture was stirred for 3 hours. LCMS indicated the reaction was completed. The reaction was quenched with water (5 mL) and the mixture was extracted with ethyl acetate (5 mL×3). The organic phase was collected, and washed with saturated brine (30 mL). The organic phase was concentrated under reduced pressure, and the residue was purified by silica gel column chromatography (petroleum ether: ethyl acetate = 3: 1) to obtain Intermediate 110-1. LCMS: m/z 387.0 [M+H]⁺.

Step 2: Sodium borohydride (123 mg, 3.25 mmol) was added to a solution of Intermediate 110-1 (250 mg, 0.65 mmol) and lithium chloride (271 mg, 6.50 mmol) in tetrahydrofuran (10 mL) at 25 °C. The mixture was stirred for 3 hours. LCMS indicated the reaction was completed. The reaction was quenched with water (20 mL) and the mixture was extracted with ethyl acetate (20 mL×3). The organic phases were collected and washed with saturated brine (60 mL). The organic phase was concentrated under reduced pressure to obtain Intermediate 110-2. LCMS: m/z 359.0 [M+H]⁺.

Step 3: Thionyl chloride (0.1 ml, 0.84 mmol) was added dropwise to a solution of Intermediate 110-2 (100 mg, 0.31 mmol) in dichloromethane (3 mL) at 25°C, and the mixture was stirred for 3 hours. LCMS indicated the reaction was completed. The reaction mixture was concentrated under reduced pressure to obtain Intermediate 110-3. LCMS: m/z 377.0 [M+H]⁺.

Step 4: At 25°C, a solution of Intermediate 110-3 (50 mg, 0.13 mmol), isoindoline hydrochloride (60 mg, 0.39 mmol) and potassium carbonate (54 mg, 0.39 mmol) in N,N-dimethylformamide (2 mL) was heated to 60°C and stirred for 3 hours. LCMS indicated the reaction was completed. Cool to 25°C, quench the reaction mixture with water (10 mL) and the mixture was extracted with dichloromethane (10 mL × 3). The organic phases were collected and washed with saturated brine (30 mL). The organic phase was concentrated under reduced pressure, and the residue was purified by preparative high-performance liquid chromatography (ammonium bicarbonate conditions) to obtain compound 110. LCMS: m/z 460.0 [M+H]⁺; ¹H NMR (400MHz, CDCl₃) δ: 7.33 (d, *J* = 9.4 Hz, 1H), 7.16 - 7.25 (m, 4H), 6.57 (d, *J =* 2.5 Hz, 2H), 3.99 (s, 4H), 3.93 (br d, *J* = 3.9 Hz, 2H), 3.84 (br d, *J =* 12.4 Hz, 2H), 3.74 (s, 3H), 2.97 (q, *J=* 7.4 Hz, 2H), 2.87 (td, *J=* 12.1, 2.1 Hz, 2H), 2.31 (ddd, *J=* 14.6, 8.0, 3.3 Hz, 1H), 1.82 - 1.95 (m, 2H), 1.51 - 1.64 (m, 2H), 1.38 (t, *J=* 7.4 Hz, 3H).

### Example 109: Compound 111

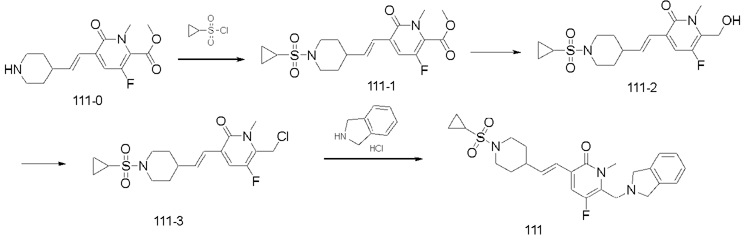

Step 1: At 25°C, cyclopropylsulfonyl chloride (215 mg, 1.53 mmol) was added dropwise to a solution of starting material 111-0 (300 mg, 1.02 mmol) and triethylamine (0.42 mL, 3.06 mmol) in N,N-dimethylformamide (3 mL). the reaction mixture was heated to 60°C and stirred for 3 hours. LCMS indicated the reaction was completed. The temperature was cooled to 25°C, the reaction mixture was extracted with ethyl acetate (5 mL×3). The organic phase was collected, and washed with saturated brine (30 mL). The organic phase was concentrated under reduced pressure, and the residue was purified by silica gel column chromatography (petroleum ether: ethyl acetate = 3:1) to obtain Intermediate 111-1. LCMS: m/z 399.0 [M+H]⁺.

Step 2: A solution of Intermediate 111-1 (300 mg, 0.75 mmol), sodium borohydride (285 mg, 7.53 mmol) and lithium chloride (320 mg, 7.53 mmol) in tetrahydrofuran (10 mL) was stirred at 25 °C for 3 hours. LCMS indicated the reaction was completed. The reaction was quenched with water (20 mL) and the mixture was extracted with ethyl acetate (20 mL×3). The organic phase was collected, and washed with saturated brine (60 mL). The organic phase was concentrated under reduced pressure to obtain Intermediate 111-2. LCMS: m/z 371.0 [M+H]⁺.

Step 3: Thionyl chloride (1.28 mL, 1.08 mmol) was added dropwise to a solution of Intermediate 111-2 (200 mg, 0.54 mmol) in dichloromethane (3 mL) at 25 °C, and the mixture was stirred for 3 hours. LCMS indicated the reaction was completed. The reaction mixture was concentrated under reduced pressure to obtain Intermediate 111-3. LCMS: m/z 389.0 [M+H]⁺.

Step 4: At 25 °C, a solution of Intermediate 111-3 (200 mg, 0.51 mmol), isoindoline hydrochloride (237 mg, 1.54 mmol) and potassium carbonate (213 mg, 1.54 mmol) in N,N-dimethylformamide (2 mL) was heated to 60°C and stirred for 3 hours. LCMS indicated the reaction was completed. The solution was cooled to 25°C. The reaction was quenched with water (10 mL) and the mixture was extracted with dichloromethane (10 mL × 3). The organic phases were collected, washed with saturated brine (30 mL). The organic phase was concentrated under reduced pressure, and the residue was purified by preparative high-performance liquid chromatography (ammonium bicarbonate conditions) to obtain compound 111. LCMS: m/z 472.2 [M+H]⁺; ¹H NMR (400MHz, CDCl₃) δ: 7.34 (d, *J =* 9.4 Hz, 1H), 7.18 - 7.25 (m, 4H), 6.55-6.59 (m, 2H), 3.98 (s, 4H), 3.93 (d, *J =* 4.0 Hz, 2H), 3.84 (br d, *J =* 12.0 Hz, 2H), 3.74 (s, 3H), 2.89 (td, *J=* 12.0, 2.3 Hz, 2H), 2.23-2.36 (m, 2H), 1.83 - 1.94 (m, 2H), 1.52 - 1.66 (m, 2H), 1.13 - 1.22 (m, 2H), 0.92-1.03 (m, 2H).

### Example 110: Compound 112

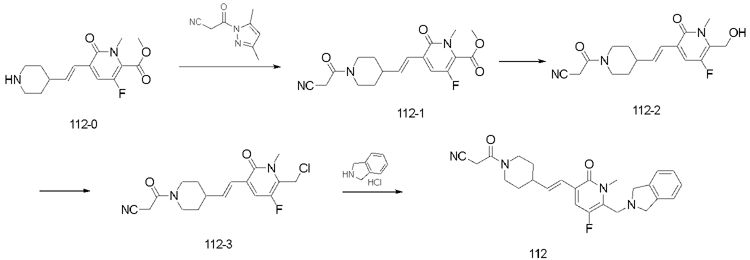

Step 1: At 25°C, 3-(3,5-dimethyl-1H-pyrazol-1-yl)-3-oxopropionitrile (333 mg, 2.04 mmol) was added to a solution of starting material 112-0 (300 mg, 1.02 mmol) in tetrahydrofuran (5 mL). The reaction mixture was heated to 60°C, and stirred for 16 hours. LCMS indicated the reaction was completed. The reaction mixture was cooled to 25 °C. The reaction was quenched with water (10 mL) and the mixture was extracted with ethyl acetate (10 mL×3). The organic phase was collected and washed with saturated brine (30 mL). The organic phase was concentrated under reduced pressure, and the residue was purified by silica gel column chromatography (petroleum ether: ethyl acetate = 3: 1) to obtain Intermediate 112-1. LCMS: m/z 362.0 [M+H]⁺.

Step 2: A solution of Intermediate 112-1 (140 mg, 0.39 mmol), sodium borohydride (325 mg, 3.88 mmol) and lithium chloride (182 mg, 3.88 mmol) in tetrahydrofuran (10 mL) was stirred at 25 °C for 3 h. LCMS indicated the reaction was completed. The reaction was quenched with water (10 mL) and the mixture was extracted with ethyl acetate (20 mL×3). The organic phases were collected, and washed with saturated brine (60 mL). The organic phase was concentrated under reduced pressure to obtain Intermediate 112-2. LCMS: m/z 334.0 [M+H]⁺.

Step 3: Thionyl chloride (0.1 ml, 1.38 mmol) was added dropwise to a solution of Intermediate 112-2 (100 mg, 0.31 mmol) in dichloromethane (3 mL) at 25 °C, and the mixture was stirred for 3 hours. LCMS indicated the reaction was completed. The reaction mixture was concentrated under reduced pressure to obtain Intermediate 112-3. LCMS: m/z 352.0 [M+H]⁺.

Step 4: A solution of Intermediate 112-3 (100 mg, 0.43 mmol), isoindoline hydrochloride (131 mg, 1.28 mmol) and potassium carbonate (118 mg, 1.28 mmol) in N,N-dimethylformamide (2 mL) was heated from 25°C to 60°C and stirred for 3 hours. LCMS indicated the reaction was completed. The solution was cooled to 25°C. The reaction was quenched with water (10 mL) and the mixture was extracted with dichloromethane (10 mL × 3). The organic phases were collected and washed with saturated brine (30 mL). The organic phase was concentrated under reduced pressure, and the residue was purified by preparative high-performance liquid chromatography (ammonium bicarbonate conditions) to obtain compound 112. LCMS: m/z 435.0 [M+H]⁺; ¹H NMR (400MHz, CDCl₃) δ: 7.33 (d, *J =* 9.4 Hz, 1H), 7.17-7.24 (m, 4H), 6.53 - 6.61 (m, 2H), 4.57 (br d, *J* = 12.9 Hz, 1H), 3.99 (s, 4H), 3.89 - 3.95 (m, 2H), 3.74 (s, 3H), 3.48 - 3.52 (m, 2H), 3.19 - 3.30 (m, 1H), 2.78 (td, *J =* 12.8, 2.8 Hz, 1H), 2.39 - 2.52 (m, 1H), 1.82 - 1.97 (m, 2H), 1.38 - 1.55 (m, 3H).

### Example 111: Compound 113

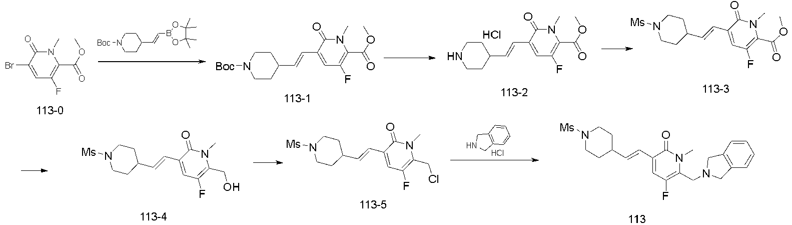

Step 1: At 25°C, starting material 113-0 (6.00 g, 22.72 mmol), tert-butyl (E)-4-(2-(4,4,5,5-tetramethyl-1,3,2-dioxaborolan-2-yl)vinyl)piperidine-1-carboxylate (7.60 g, 22.72 mmol), tetrakis triphenylphosphine palladium (2.63 g, 2.27 mmol), and potassium carbonate (9.42 g, 68.17 mmol) were dissolved in dioxane (3 mL) and water (10 mL). After purging with nitrogen three times, the reaction mixture was heated to 90°C and stirred for 6 hours. LCMS indicated the reaction was completed. The reaction mixture was cooled to 25 °C. The reaction was quenched with water (30 mL), and the mixture was extracted with ethyl acetate (30 × 3 mL). The organic phases were collected and washed with saturated brine (100 mL). The organic phase was concentrated under reduced pressure, and the residue was purified by silica gel column chromatography (petroleum ether: ethyl acetate = 5: 1) to obtain Intermediate 113-1. LCMS: m/z 339.0 [M-55]⁺.

Step 2: Hydrochloric acid-dioxane solution (10 mL, 40.00 mmol, 4M) was added dropwise to a solution of Intermediate 113-1 (3.00 g, 1.15 mmol) in dioxane (10 mL) at 25°C. The mixture was stirred for 3 hours. LCMS indicated the reaction was completed, and the reaction mixture was concentrated under reduced pressure to obtain Intermediate 113-2. LCMS: m/z 295.0 [M+H]⁺.

Step 3: At 25°C, methanesulfonic anhydride (230 mg, 1.33 mmol) was added to a solution of Intermediate 113-2 (130 mg, 0.44 mmol) and triethylamine (222 mg, 2.22 mmol) in dichloromethane (5 mL), and the mixture was stirred for 2 hours. LCMS indicated the reaction was completed. The reaction was quenched with water (10 mL) and the mixture was extracted with ethyl acetate (10 mL×3). The organic phase was collected, and washed with saturated brine (30 mL). The organic phase was concentrated under reduced pressure, and the residue was purified by silica gel column chromatography (petroleum ether: ethyl acetate = 3: 1) to obtain Intermediate 113-3. LCMS: m/z 373.0 [M+H]⁺.

Step 4: A solution of Intermediate 113-3 (160 mg, 0.43 mmol), sodium borohydride (325 mg, 8.59 mmol) and lithium chloride (182 mg, 4.30 mmol) in tetrahydrofuran (10 mL) was stirred at 25 °C for 3 hours. LCMS indicated the reaction was completed. The reaction was quenched with water (20 mL) and the mixture was extracted with ethyl acetate (20 mL×3). The organic phase was collected, and washed with saturated brine (60 mL). The organic phase was concentrated under reduced pressure to obtain Intermediate 113-4. LCMS: m/z 345.2 [M+H]⁺.

Step 5: Thionyl chloride (0.1 mL, 1.38 mmol) was added dropwise to a solution of Intermediate 113-4 (100 mg, 0.31 mmol) in dichloromethane (3 mL) at 25 °C, and the mixture was stirred for 3 hours. LCMS indicated the reaction was completed. The reaction mixture was concentrated under reduced pressure to obtain Intermediate 113-5. LCMS: m/z 363.1 [M+H]⁺.

Step 6: At 25°C, a solution of Intermediate 113-5 (100 mg, 0.28 mmol), isoindoline hydrochloride (63 mg, 0.42 mmol), potassium carbonate (114 mg, 0.82 mmol) in N,N-dimethylformamide (2 mL) was heated to 60°C and stirred for 3 hours. LCMS indicated the reaction was completed. The solution was cooled to 25°C, The reaction was quenched with water (10 mL) and the mixture was extracted with dichloromethane (10 mL × 3). The organic phases were collected and washed with saturated brine (30 mL). The organic phase was concentrated under reduced pressure, and the residue was purified by preparative high-performance liquid chromatography (ammonium bicarbonate conditions) to obtain compound 113. LCMS: m/z 446.0 [M+H]⁺; ¹H NMR (400MHz, CDCl₃) δ: 7.33 (d, *J* = 9.4 Hz, 1H), 7.15 - 7.24 (m, 4H), 6.57 (d, *J =* 2.3 Hz, 2H), 3.98 (s, 4H), 3.93 (d, *J =* 4.1 Hz, 2H), 3.82 (br d, *J* = 12.0 Hz, 2H), 3.74 (s, 3H), 2.79 (s, 3H), 2.71 - 2.79 (m, 2H), 2.29 (m, 1H), 1.90 (br dd, *J* = 13.3, 2.3 Hz, 2H), 1.51 - 1.67 (m, 2H).

### Example 112: Compound 114

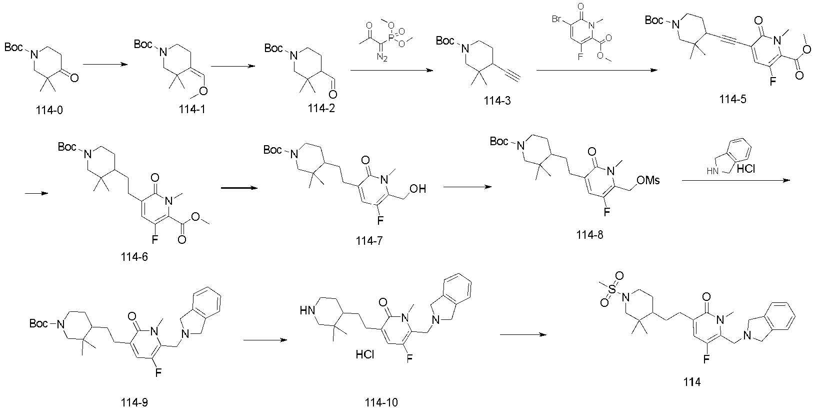

Step 1: At 0°C, potassium tert-butoxide (0.74 g, 6.60 mmol) was added to a solution of (methoxymethyl)triphenylphosphonium chloride (2.26 g, 6.60 mmol) in tetrahydrofuran (15 mL) and the reaction mixture was stirred for 1 hour. After adding starting material 114-0 (1.01 g, 4.40 mmol), the reaction mixture was continuously stirred for 18 hours. TLC indicated the reaction was completed. The reaction was quenched with saturated ammonium chloride solution (10 mL) and the mixture was extracted with ethyl acetate (10 mL×3). The combined organic phase was washed with saturated brine (30 mL), dried over anhydrous sodium sulfate, and filtered. The filtrate was concentrated under reduced pressure, and the residue was purified by silica gel column chromatography (petroleum ether: ethyl acetate = 10: 1) to obtain Intermediate 114-1.

Step 2: Hydrochloric acid (5.00 mL, 5.00 mmol, 1M) was added dropwise to a solution of Intermediate 114-1 (1.00 g, 3.92 mmol) in acetonitrile (20 mL) at 0 °C, and the mixture was stirred for 3 hours. TLC indicated the reaction was completed. The reaction was quenched with saturated sodium bicarbonate solution (20 mL) and the mixture was extracted with ethyl acetate (20 mL×3). The combined organic phase was washed with saturated brine (50 mL), dried over anhydrous sodium sulfate, and filtered. The filtrate was concentrated under reduced pressure to obtain Intermediate 114-2.

Step 3: Potassium carbonate (973 mg, 7.05 mmol) was added to a solution of Intermediate 114-2 (900 mg, 3.52 mmol) in methanol (10 mL) at 25 °C. After being stirred for 15 minutes, the mixture was added with dimethyl (1-diazo-2-oxopropyl)phosphonate (1.34 g, 7.05 mmol) and continuously stirred for 2 hours. TLC indicated the reaction was completed. The reaction was quenched with water (20 mL) and the mixture was extracted with ethyl acetate (20 mL×3). The organic phase was washed with saturated brine (40 mL), dried over anhydrous sodium sulfate, and filtered. The filtrate was concentrated under reduced pressure, and the residue was purified by silica gel column chromatography (petroleum ether: ethyl acetate = 10: 1) to obtain Intermediate 114-3.

Step 4: At 25°C, Intermediate 114-3 (898 mg, 3.79 mmol), methyl 5-bromo-3-fluoro-1-methyl-6-oxo-1,6-dihydropyridine-2-carboxylate (500 mg, 1.89 mmol), triethylamine (1.31 mL, 9.47 mmol) and bis(triphenylphosphine) palladium dichloride were added to tetrahydrofuran (15 mL). After purging with nitrogen three times, and the mixture was stirred for 2 hours. LCMS indicated the reaction was completed. The reaction mixture was filtered, the filtrate was concentrated under reduced pressure, and the residue was purified by silica gel column chromatography (petroleum ether: ethyl acetate = 3:1) to obtain Intermediate 114-5. LCMS: m/z 321.2 [M-99]⁺.

Step 5: Under nitrogen atmosphere, Intermediate 114-5 (450 mg, 1.07 mmol) and palladium/carbon (11 mg, 0.01 mmol, 10 wt%) were added to methanol (10 mL) at 25 °C. After purging with hydrogen three times, the mixture was stirred for 48 hours. LCMS indicated the reaction was completed. The reaction mixture was filtered, and the filtrate was concentrated under reduced pressure to obtain Intermediate 114-6. LCMS: m/z 325.2 [M-99]⁺.

Step 6: Lithium chloride (899 mg, 21.20 mmol) and sodium borohydride (802 mg, 21.20 mmol) were added successively to a solution of Intermediate 114-6 (450 mg, 1.06 mmol) in tetrahydrofuran (10 mL) at 25 °C, and the mixture was stirred for 48 hours. LCMS indicated the reaction was completed. The reaction was quenched with water (10 mL) and the mixture was extracted with ethyl acetate (10 mL×3). The organic phase was washed with saturated brine (20 mL), dried over anhydrous sodium sulfate, and filter. The filtrate was concentrated under reduced pressure to obtain Intermediate 114-7. LCMS: m/z 297.2 [M-99]⁺.

Step 7: Triethylamine (0.1 mL, 0.76 mmol) and methylsulfonic anhydride (66 mg, 0.38 mmol) was added to a solution of Intermediate 114-7 (100 mg, 0.25 mmol) in dichloromethane (2 mL) at 25 °C, and the mixture was stirred for 30 minutes. LCMS indicated the reaction was completed. The reaction was quenched with water (5 mL) and the mixture was extracted with dichloromethane (5 mL×3). The organic phase was washed with saturated brine (15 mL), dried over anhydrous sodium sulfate, and filter. The filtrate was concentrated under reduced pressure to obtain crude product Intermediate 114-8. LCMS: m/z 375.0 [M-99]⁺.

Step 8: At 25°C, potassium carbonate (29 mg, 0.21 mmol) and isoindoline hydrochloride (32 mg, 0.21 mmol) were added to a solution of Intermediate 114-8 (120 mg, crude product) in N,N-dimethylformamide (3 mL). The reaction mixture was heated to 50°C and stirred for 30 minutes. LCMS indicated the reaction was completed. The reaction was quenched with water (10 mL) and the mixture was extracted with ethyl acetate (10 mL×2). The organic phase was washed with saturated brine (20 mL), dried over anhydrous sodium sulfate, and filtered. The filtrate was concentrated under reduced pressure, and the residue was purified by preparative thin layer chromatography (petroleum ether: ethyl acetate = 1: 1) to obtain Intermediate 114-9. LCMS: m/z 498.1 [M+H]⁺.

Step 9: Hydrochloric acid-dioxane solution (1.00 mL, 4.00 mmol, 4M) was added dropwise to a solution of Intermediate 114-9 (60 mg, 0.12 mmol) in dioxane (1 mL) at 25 °C, and the mixture was stirred for 20 minutes. The reaction mixture was concentrated under reduced pressure to obtain crude product Intermediate 114-10.

Step 10: At 25°C, triethylamine (0.03 mL, 0.19 mmol) and methylsulfonic anhydride (22 mg, 0.13 mmol) were added sequentially to a solution of Intermediate 114-10 (55 mg, crude product) in dichloromethane (2 mL), and the mixture was stirred for 30 minutes. Water (10 mL) was added to quench the reaction, and the mixture was extracted with dichloromethane (10 mL×2). The organic phase was washed with saturated brine (10 mL), dried over anhydrous sodium sulfate, filtered, and the filtrate was concentrated under reduced pressure, and the residue was subjected to preparative high performance liquid chromatography purification (ammonium bicarbonate conditions) to obtain compound 114. LCMS: m/z 476.0 [M+H]⁺; ¹H NMR (400 MHz, CDCl₃) δ: 7.18 - 7.10 (m, 4H), 7.09 (d, *J* = 9.0 Hz, 1H), 3.91 (s, 4H), 3.84 (d, *J* = 4.0 Hz, 2H), 3.75 (td, *J =* 2.2, 4.6 Hz, 1H), 3.65 (s, 3H), 3.24 (dd, *J =* 1.9, 11.4 Hz, 1H), 2.68 (s, 3H), 2.62 - 2.48 (m, 2H), 2.43 - 2.32 (m, 1H), 2.29 (d, *J* = 11.5 Hz, 1H), 1.88 - 1.72 (m, 2H), 1.51 - 1.36 (m, 1H), 1.24 - 1.02 (m, 2H), 0.88 (d, *J =* 4.5 Hz, 6H).

### Example 113: Compound 115

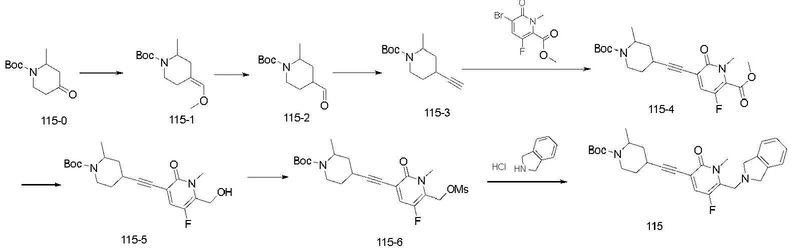

Step 1: At 25°C, (methoxymethyl)triphenylphosphonium chloride (2.41 g, 7.03 mmol) and potassium tert-butoxide (1.58 g, 14.07 mmol) were added to tetrahydrofuran (20 mL), and the mixture was stirred for 1 hour. Then starting material 115-0 (1.0 g, 4.69 mmol) was added to the reaction mixture and the mixture was stirred for 2 hours. TLC (PE/EA=10:1) indicated the reaction was completed. The reaction mixture was quenched by adding water (30 mL) and the mixture was extracted with ethyl acetate (30 mL×3). The organic phases were combined, washed with saturated brine (30 mL), and dried over anhydrous sodium sulfate. After filtration, the filtrate was concentrated under reduced pressure to obtain crude product Intermediate 115-1.

Step 2: 1M hydrochloric acid aqueous solution (5 mL) was added to a solution of Intermediate 115-1 (0.85 g, 3.52 mmol) and acetonitrile (30 mL) at 25 °C, and the mixture was stirred for 2 hours. TLC (PE/EA=10:1) indicated the reaction was completed. The reaction mixture was quenched with saturated sodium bicarbonate (30 mL) solution and the mixture was extracted with ethyl acetate (30 mL×3). The organic phases were combined, washed with saturated brine (30 mL), and dried over anhydrous sodium sulfate. After filtration, the filtrate was concentrated under reduced pressure to obtain crude product Intermediate 115-2.

Step 3: Dimethyl (1-diazo-2-oxopropyl)phosphonate (1.48 g, 7.7 mmol) was added to a solution of crude Intermediate 115-2 (0.7 g, 3.08 mmol) and potassium carbonate (1.28 g, 9.24 mmol) in methanol (10 mL) at 25 °C. The mixture was stirred for 20 minutes. TLC (PE/EA=10:1) indicated the reaction was completed. The reaction mixture was quenched with water (20 mL) and the mixture was extracted with ethyl acetate (20 mL×3). The organic phases were combined, washed with saturated brine (20 mL), and dried over anhydrous sodium sulfate. After filtration, the filtrate was concentrated under reduced pressure to obtain crude product Intermediate 115-3.

Step 4: At 25°C, Intermediate 115-3 crude product (650 mg, 2.91 mmol), 5-bromo-3-fluoro-1-methyl-6-oxo-1,6-dihydropyridine-2-carboxylic acid methyl ester (577 mg, 2.18 mmol), copper iodide (14 mg, 0.07 mmol) and triethylamine (2.0 mL, 14.55 mmol) were added to tetrahydrofuran (2 mL), followed by bis(triphenylphosphine)palladium dichloride (102 mg, 0.15 mmol). After purging with nitrogen three times, the mixture was stirred for 2 hours. LCMS indicated the reaction was completed. The reaction mixture was filtered, the filtrate was concentrated under reduced pressure, and the residue was purified by silica gel column chromatography (petroleum ether: ethyl acetate = 2: 1) to obtain Intermediate 115-4. LCMS: m/z 407.2 [M+H]⁺.

Step 5: Sodium borohydride (47 mg, 1.23 mmol) and lithium chloride (52 mg, 1.23 mmol) were added to a solution of Intermediate 115-4 (50 mg, 0.12 mmol) in tetrahydrofuran (4 mL) at 25 °C. The mixture was stirred for 2 hours. LCMS indicated the reaction was completed. The reaction was quenched with water (10 mL) and the mixture was extracted with ethyl acetate (10 mL×3). The combined organic phase was washed with saturated brine (20 mL), dried over anhydrous sodium sulfate, and filtered. The filtrate was concentrated to obtain Intermediate 115-5. LCMS: m/z 379.0 [M+H]⁺.

Step 6: At 25°C, methanesulfonic anhydride (32 mg, 0.18 mmol) was added to a solution of Intermediate 115-5 (35 mg, 0.09 mmol) and triethylamine (0.04 mL, 0.28 mmol) in dichloromethane (1 mL), and the mixture was stirred for 10 minutes. LCMS indicated the reaction was completed. The reaction was quenched with water (10 mL) and the mixture was extracted with ethyl acetate (10 mL×3). The combined organic phase was washed with saturated brine (10 mL), dried over anhydrous sodium sulfate, and filtered. The filtrate was concentrated to obtain Intermediate 115-6 crude product. LCMS: m/z 457.0 [M+H]⁺.

Step 7: At 25 °C, crude Intermediate 115-6 (40 mg, 0.09 mmol), potassium carbonate (36 mg, 0.26 mmol) and isoindoline hydrochloride (40 mg, 0.26 mmol) were added to N,N-dimethylformamide (2 mL).The reaction mixture was heated to 60°C and stirred for 1 hour. LCMS indicated the reaction was completed. The reaction mixture was filtered, and the filtrate was purified by preparative high-performance liquid chromatography (ammonium bicarbonate conditions) to obtain compound 115. LCMS: m/z 480.1 [M+H]⁺; ¹H NMR (400 MHz, CDCl₃) δ = 7.41 (d, J = 8.9 Hz, 1H), 7.31 - 7.27 (m, 2H), 7.25 (br s, 2H), 4.46 (br s, 1H), 4.27 - 3.91 (m, 6H), 3.78 (s, 3H), 2.97 - 2.80 (m, 2H), 2.03 - 1.96 (m, 3H), 1.90 - 1.75 (m, 2H), 1.47 (s, 9H), 1.15 (d, J = 7.0 Hz, 3H).

### Example 114: Compound 116

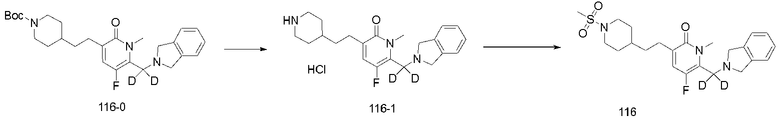

Step 1: Hydrochloric acid-dioxane solution (1 mL, 4M) was added to starting material 116-0 (150 mg, 0.32 mmol) at 25 °C, and the mixture was stirred for 1 hour. LCMS indicated the reaction was completed. The reaction mixture was concentrated under reduced pressure to obtain crude product Intermediate 116-1. LCMS: m/z 373.2 [M+H]⁺.

Step 2: At 25°C, methanesulfonic anhydride (94 mg, 1.35 mmol) was added to a solution of Intermediate 116-1 (100 mg, 0.27 mmol) and triethylamine (0.19 mL, 1.35 mmol) in dichloromethane (4 mL), and the mixture was stirred for 10 minutes. LCMS indicated the reaction was completed. The reaction mixture was diluted with water (20 mL) and the mixture was extracted with ethyl acetate (20 mL×3). The organic phases were combined, washed with saturated sodium chloride solution (20 mL). The organic phase was dried over anhydrous sodium sulfate, filtered, the filtrate was concentrated under reduced pressure, and the residue was purified by preparative high performance liquid chromatography (with ammonium bicarbonate) to obtain compound 116. LCMS: m/z 450.0 [M+H]⁺; ¹H NMR (400 MHz, CDCl₃) δ: 7.20 (d, *J* = 1.8 Hz, 4H), 7.15 (d, *J* = 9.0 Hz, 1H), 3.98 (s, 4H), 3.80 (d, *J* = 11.9 Hz, 2H), 3.72 (s, 3H), 2.77 (s, 3H), 2.71 - 2.62 (m, 2H), 2.62 - 2.55 (m, 2H), 1.89 (dd, *J* = 1.8, 12.6 Hz, 2H), 1.75 (s, 1H), 1.62 - 1.54 (m, 2H), 1.43 - 1.30 (m, 2H).

### Example 115: Compound 117

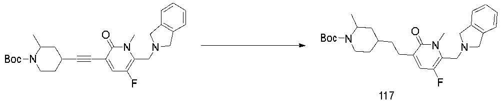

At 25°C, under nitrogen atmosphere, the tert-butyl 4-((5-fluoro-6-(isoindolin-2-ylmethyl)-1-methyl-2-oxo-1,2-dihydropyridin-3-yl)ethynyl)-2-meth ylpiperidine-1-carboxylate (10 mg, 0.02 mmol) and palladium/carbon (22 mg, 0.02 mmol, 10wt %) were added to methanol (5 mL). After purging with hydrogen three times, the mixture was stirred for 3 hours. LCMS indicated the reaction was completed. The reaction mixture was filtered, and the filtrate was concentrated under reduced pressure to obtain compound 117. LCMS: m/z 484.1 [M+H]⁺; ¹H NMR (400 MHz, CDCl₃) δ: 7.20 (d, *J* = 1.9 Hz, 4H), 7.14 (d, *J =* 9.1 Hz, 1H), 3.98 (s, 4H), 3.90 (d, *J* = 4.1 Hz, 2H), 3.72 (s, 3H), 2.85 (br t, *J* = 12.4 Hz, 1H), 2.63 - 2.54 (m, 2H), 1.77 - 1.61 (m, 5H), 1.53 - 1.48 (m, 2H), 1.46 (s, 9H), 1.36 - 1.24 (m, 2H), 1.13 (d, *J* = 7.0 Hz, 3H).

### Example 116: Compound 118

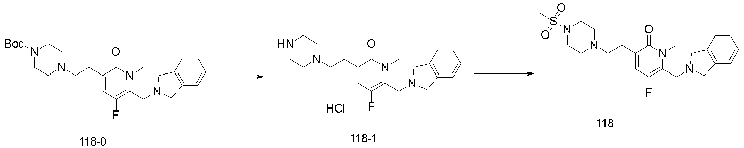

Step 1: A solution of starting material 118-0 (80 mg, 0.17 mmol) in hydrochloric acid-dioxane (6 mL, 4M/L) was stirred at 25 °C for 1 hour. LCMS indicated the reaction was completed, and the reaction mixture was concentrated under reduced pressure to obtain crude product Intermediate 118-1. LCMS: m/z 371.1 [M+H]⁺.

Step 2: At 25 °C, methanesulfonic anhydride (22 mg, 0.12 mmol) was added to a solution of Intermediate 118-1 (40 mg, 0.09 mmol) and triethylamine (30 mg, 0.29 mmol) in dichloromethane (4 mL), and the mixture was stirred for 1 hour. LCMS indicated the reaction was completed. The reaction was quenched with water (5 mL) and the mixture was extracted with dichloromethane (20 mL×2). The combined organic phase was washed with saturated brine (20 mL), dried over anhydrous sodium sulfate, and filter. The filtrate was concentrated under reduced pressure. The residue was purified by preparative high performance liquid chromatography (ammonium bicarbonate conditions) to afford compound 118. LCMS: m/z 449.2 [M+H]⁺. ¹H NMR (400 MHz, CDCl₃) δ: 7.25 - 7.15 (m, 5H), 3.98 (s, 4H), 3.90 (d, *J=* 4.1 Hz, 2H), 3.72 (s, 3H), 3.32 - 3.20 (m, 4H), 2.79 (s, 3H), 2.75 (d, *J =* 7.5 Hz, 2H), 2.72 - 2.67 (m, 2H), 2.67 - 2.62 (m, 4H).

### Example 117: Compound 119

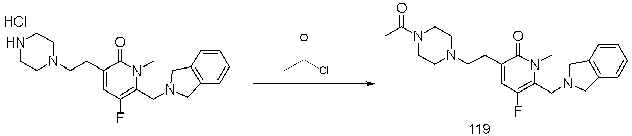

Step 1: At 25°C, acetyl chloride (10 mg, 0.12 mmol) was added to a solution of 5-fluoro-6-(isoindolin-2-ylmethyl)-1-methyl-3-(2-(piperazin-1-yl)ethyl)pyridin-2(1H)-one (40 mg, 0.09 mmol) and triethylamine (30 mg, 0.29 mmol) in tetrahydrofuran (4 mL), and the mixture was stirred for 1 hour. LCMS indicated the reaction was completed. The reaction was quenched with water (5 mL) and the mixture was extracted with ethyl acetate (20 mL×2). The combined organic phase was washed with saturated brine (20 mL), dried over anhydrous sodium sulfate, and filter. The filtrate was concentrated under reduced pressure. The residue was purified by preparative high performance liquid chromatography (ammonium bicarbonate conditions) to afford compound 119. LCMS: m/z 413.3 [M+H]⁺. ¹H NMR (400 MHz, CDCl₃) δ: 7.24 (d, *J =* 9.0 Hz, 1H), 7.22 - 7.16 (m, 4H), 3.98 (s, 4H), 3.90 (d, *J* = 4.1 Hz, 2H), 3.72 (s, 3H), 3.67 - 3.60 (m, 2H), 3.51 - 3.45 (m, 2H), 2.80 - 2.73 (m, 2H), 2.69 - 2.62 (m, 2H), 2.57 - 2.52 (m, 2H), 2.52 - 2.47 (m, 2H), 2.09 (s, 3H).

### Example 118: Compound 120

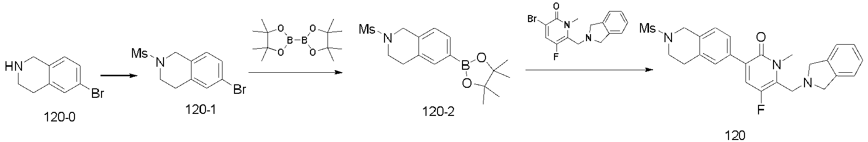

Step 1: At 25°C, methanesulfonic anhydride (2.50 g, 14.13 mmol) was added to a solution of starting material 120-0 (1.00 g, 4.71 mmol) and triethylamine (1.96 mL, 14.13 mmol) in dichloromethane (15 mL), and the mixture was stirred for 3 hours. LCMS indicated the reaction was completed. The reaction mixture was quenched with water (15 mL) and the mixture was extracted with ethyl acetate (15 mL×3). The organic phases were collected, and washed with saturated brine (40 mL). The organic phase was concentrated under reduced pressure, and the residue was purified by silica gel column chromatography (petroleum ether: ethyl acetate = 3: 1) to obtain Intermediate 120-1. LCMS: m/z 291.0 [M+H]⁺.

Step 2: At 25°C, Intermediate 120-1 (300 mg, 1.03 mmol), 4,4,4',4',5,5,5',5'-octamethyl-2,2'-bi(1,3,2-dioxaborolane) (788 mg, 3.10 mmol), potassium acetate (303 mg, 3.09 mmol), and bis(triphenylphosphine) palladium dichloride (77 mg, 0.10 mmol) were dissolved in 1,4-dioxane (15 mL). After purging with nitrogen three times, the reaction mixture was heated to 90°C and stirred for 6 hours. LCMS indicated the reaction was completed. The reaction mixture was cooled to 25°C. The reaction was quenched with water (15 mL), and the mixture was extracted with ethyl acetate (15 mL×3). The organic phases were collected and washed with saturated brine (40 mL). The organic phase was concentrated under reduced pressure, and the residue was purified by silica gel column chromatography (petroleum ether: ethyl acetate = 5: 1) to obtain Intermediate 120-2. LCMS: m/z 338.0 [M+H]⁺.

Step 3: At 25°C, Intermediate 120-2 (40 mg, 0.12 mmol), 3-bromo-5-fluoro-6-(isoindolin-2-ylmethyl)-1-methylpyridin-2(1H)-one (20 mg, 0.06 mmol), potassium carbonate (25 mg, 0.18 mmol), and tetrakis triphenylphosphine palladium (7 mg, 0.01 mmol) were dissolved in 1,4-dioxane (3 mL) and water (1 mL). After purging with nitrogen three times, the reaction mixture was heated to 90°C and stirred for 6 hours. LCMS indicated the reaction was completed. The reaction mixture was cooled to 25°C, The reaction was quenched with water (5 mL), and the mixture was extracted with ethyl acetate (5 mL×3). The organic phases were collected and washed with saturated brine (15 mL). The organic phase was concentrated under reduced pressure, and the residue was purified by preparative high-performance liquid chromatography (ammonium bicarbonate conditions) to obtain compound 120. LCMS: m/z 468.1 [M+H]⁺; ¹H NMR (400MHz, CDCl₃) δ: 7.63 (s, 1H), 7.52 (dd, *J* = 8.0, 1.6 Hz, 1H), 7.46 (d, *J* = 9.3 Hz, 1H), 7.22 (s, 4H), 7.16 (d, *J* = 8.0 Hz, 1H), 4.50 (s, 2H), 4.04 (s, 4H), 3.97 (d, *J* = 4.1 Hz, 2H), 3.79 (s, 3H), 3.60 (t, *J* = 5.9 Hz, 2H), 3.04 (t, *J* = 5.9 Hz, 2H), 2.86 (s, 3H).

### Example 119: Compound 121

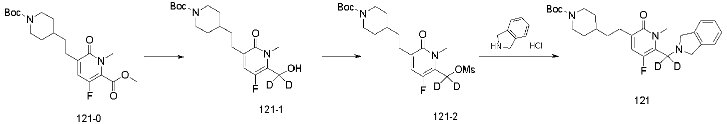

Step 1: At 25°C, starting material 121-0 (300 mg, 0.76 mmol), sodium deuterated borohydride (1.48 g, 7.60 mmol) and lithium chloride (322 mg, 7.60 mmol) were added to tetrahydrofuran (5 mL), and the mixture was stirred for 24 hours. LCMS indicated the reaction was completed. The reaction was quenched with water and the mixture was extracted with ethyl acetate (50 mL×3). The organic phases were combined, washed with saturated brine (50 mL), and dried over anhydrous sodium sulfate. After filtration, the filtrate was concentrated under reduced pressure, and the residue was purified by silica gel column chromatography (petroleum ether: ethyl acetate = 2:1) to obtain Intermediate 121-1. LCMS: m/z 271.1 [M+H-100]⁺.

Step 2: At 25°C, methanesulfonic anhydride (188 mg, 1.08 mmol) was added to a solution of Intermediate 121-1 (200 mg, 0.54 mmol) and triethylamine (0.22 mL, 1.62 mmol) in dichloromethane (5 mL), and the mixture was stirred for 10 minutes. LCMS indicated the reaction was completed. The reaction mixture was diluted with water (30 mL) and extracted with ethyl acetate (30 mL×3). The organic phases were combined, washed with saturated sodium chloride solution (30 mL×1), dried over anhydrous sodium sulfate, and filtered. The filtrate was concentrated under reduced pressure, and the residue was purified by silica gel column chromatography (petroleum ether: ethyl acetate = 2:1) to obtain Intermediate 121-2. LCMS: m/z 349.0 [M+H-100]⁺.

Step 3: At 25°C, Intermediate 121-2 (200 mg, 0.45 mmol), potassium carbonate (187 mg, 1.35 mmol) and isoindoline hydrochloride (138 mg, 0.89 mmol) were added to N,N-dimethylformamide (4 mL ) solution. The reaction mixture was heated to 60°C and the mixture was stirred for 2 hours. LCMS indicated the reaction was completed. The reaction mixture was cooled to 25°C. The reaction was quenched with water and the mixture was extracted with ethyl acetate (30 mL×3). The organic phases were combined, washed with saturated brine (30 mL), and dried over anhydrous sodium sulfate. After filtration, the filtrate was concentrated under reduced pressure, and the residue was purified by silica gel column chromatography (petroleum ether: ethyl acetate = 1:1) to obtain compound 121. LCMS: m/z 472.3 [M+H]⁺; ¹H NMR (400 MHz, CDCl₃) δ: 7.20 (d, *J* = 1.4 Hz, 4H), 7.14 (d, *J* = 9.1 Hz, 1H), 4.17 - 4.02 (m, 2H), 3.98 (s, 4H), 3.72 (s, 3H), 2.70 (t, *J =* 12.1 Hz, 2H), 2.62 - 2.55 (m, 2H), 1.74 (d, *J* = 12.6 Hz, 2H), 1.59 - 1.49 (m, 3H), 1.46 (s, 9H), 1.21 - 1.09 (m, 2H).

### Example 120: Compound 122

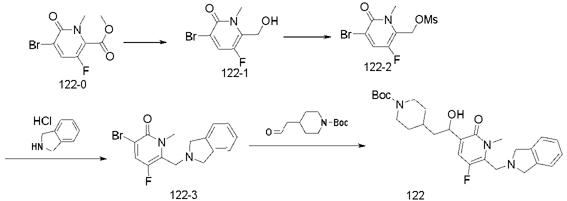

Step 1: At 25°C, starting material 122-0 (1.00 g, 3.79 mmol) was dissolved in tetrahydrofuran (10 mL), then lithium chloride (1.61 g, 37.87 mmol) and sodium borohydride (1.43 g, 37.87 mmol) were added in sequence. The mixture was stirred for 12 hours. LCMS indicated the reaction was completed. The reaction was quenched with water (20 mL) and the mixture was extracted with ethyl acetate (20 mL×3). The combined organic phase was washed with saturated brine (50 mL), dried over anhydrous sodium sulfate, and filtered. The filtrate was concentrated under reduced pressure to obtain Intermediate 122-1. LCMS: m/z 237.8 [M+H]⁺.

Step 2: At 25°C, Intermediate 122-1 (350 mg, 1.48 mmol) was dissolved in dichloromethane (5 mL), then triethylamine (0.41 mL, 2.97 mmol) and methylsulfonic anhydride (387 mg, 2.22 mmol) were added in sequence. The mixture was stirred for 2 hours. LCMS indicated the reaction was completed. The reaction was quenched with water (10 mL) and the mixture was extracted with dichloromethane (10 mL×2). The combined organic phase was washed with saturated brine (20 mL), dried over anhydrous sodium sulfate, and filtered. The filtrate was concentrated under reduced pressure to obtain crude Intermediate 122-2. LCMS: m/z 314.8 [M+H]⁺.

Step 3: At 25°C, potassium carbonate (389 mg, 2.82 mmol) was added to a solution of Intermediate 122-2 (590 mg, crude) and isoindoline hydrochloride (432 mg, 2.82 mmol) in N,N-dimethylformamide (10 mL). The reaction mixture was heated to 50°C and the mixture was stirred for 2 hours. LCMS indicated the reaction was completed. The reaction was quenched with water (10 mL) and the mixture was extracted with ethyl acetate (10 mL×3). The combined organic phase was washed with saturated brine (40 mL), dried over anhydrous sodium sulfate, and filtered. The filtrate was concentrated. The residue was purified by silica gel column chromatography (petroleum ether: ethyl acetate = 1: 1) to obtain Intermediate 122-3. LCMS: m/z 337.0 [M+H]⁺.

Step 4: At 0 °C, isopropylmagnesium chloride-lithium chloride solution (0.15 mL, 0.15 mmol, 1M) was added dropwise to a solution of Intermediate 122-3 (25 mg, 0.07 mmol) in tetrahydrofuran (1 mL), and the mixture was stirred for 1 hour. Tert-butyl 4-(2-oxoethyl)piperidine-1-carboxylate (34 mg, 0.15 mmol) was added and the mixture was continuously stirred for 2 hours. LCMS indicated the reaction was completed. The reaction was quenched with saturated aqueous ammonium chloride solution (5 mL) and the mixture was extracted with ethyl acetate (5 mL×3). The combined organic phase was washed with saturated brine (10 mL), dried over anhydrous sodium sulfate, and filtered. The filtrate was concentrated, and the residue was purified by preparative thin-layer chromatography (petroleum ether: ethyl acetate = 1: 1) to obtain compound 122. LCMS: m/z 486.1 [M+H]⁺; ¹H NMR (400 MHz, CDCl₃) δ: 7.27 (s, 1H), 7.21 (s, 4H), 4.73 (s, 1H), 4.30 (s, 1H), 4.17 - 4.12 (m,1H), 3.99 (s, 4H), 3.92 (s, 2H), 3.74 (s, 3H), 2.74 (s, 2H), 1.92 - 1.68 (m, 5H), 1.46 (s, 9H), 1.23 - 1.07 (m, 2H).

### Example 121: Compound 123

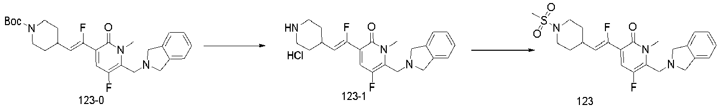

Step 1: At 25°C, hydrochloric acid-dioxane solution (1 mL, 4.00 mmol, 4M) was added dropwise into a solution of starting material 123-0 (5.00 g, 23.44 mmol) in dioxane (1 mL), and the mixture was stirred for 1 hour. The reaction mixture was concentrated under reduced pressure to obtain crude product Intermediate 123-1.

Step 2: At 25°C, diisopropylethylamine (12 mg, 0.09 mmol) and methylsulfonic anhydride (12 mg, 0.07 mmol) were added successively to a solution of Intermediate 123-1 (18 mg, crude product) in dichloromethane (2 mL) and the mixture was stirred for 1 hour. LCMS indicated the reaction was completed. The reaction was quenched with water (10 mL) and the mixture was extracted with dichloromethane (10 mL×2). The combined organic phase was washed with saturated brine (10 mL), dried over anhydrous sodium sulfate, and filtered. The filtrate was concentrated under reduced pressure, and the residue was purified by preparative high-performance liquid chromatography (ammonium bicarbonate conditions) to obtain compound 123. LCMS: m/z 464.0 [M+H]⁺; ¹H NMR (400 MHz, CDCl₃) δ: 7.59 (d, *J* = 9.8 Hz, 1H), 7.26 - 7.13 (m, 4H), 6.84 - 6.57 (m, 1H), 4.00 (s, 4H), 3.97 (d, *J =* 3.9 Hz, 2H), 3.81 - 3.72 (m, 5H), 2.88 - 2.80 (m, 2H), 2.80 (s, 3H), 1.89 (dd, *J* = 3.1, 13.4 Hz, 2H), 1.70 -1.59 (m, 3H).

### Example 122: Compound 124

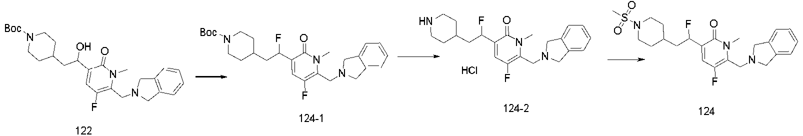

Step 1: At 0°C, diethylaminosulfur trifluoride (13 mg, 0.08 mmol) was added dropwise to compound 122 (20 mg, 0.05 mmol) in dichloromethane (10 mL), and the mixture was heated to 25 °C and stirred for 1 hour. LCMS indicated the reaction was completed. The reaction was quenched with saturated sodium bicarbonate solution (5 mL) and the mixture was extracted with dichloromethane (5 mL×3). The combined organic phase was washed with saturated brine (10 mL), dried over anhydrous sodium sulfate, and filtered. The filtrate was concentrated under reduced pressure, and the residue was purified by silica gel column chromatography (petroleum ether: ethyl acetate = 1: 1) to obtain Intermediate 124-1. LCMS: m/z 388.2 [M-99]⁺.

Step 2: hydrochloric acid-dioxane solution (0.26 mL, 1.03 mmol, 4M) was added dropwise to a solution of Intermediate 124-1 (10 mg, 0.02 mmol) in dioxane (2 mL) at 25 °C, and the mixture was stirred for 1 hour. LCMS indicated the reaction was completed. The reaction mixture was concentrated under reduced pressure to obtain Intermediate 124-2. LCMS: m/z 388.0 [M+H]⁺.

Step 3: At 25°C, Intermediate 124-2 (9 mg, crude product) was dissolved in dichloromethane (1 mL), then triethylamine (0.01 mL, 0.07 mmol) and methylsulfonic anhydride (6.07 mg, 0.03 mmol) were added in sequence, and the mixture was stirred for 1 hour. LCMS indicated the reaction was completed. The reaction was quenched with water (10 mL) and the mixture was extracted with dichloromethane (10 mL). The organic phase was washed with saturated brine (10 mL), dried over anhydrous sodium sulfate, and filtered. The filtrate was concentrated, and the residue is purified by preparative high performance liquid chromatography purification (ammonium bicarbonate conditions) to obtain compound 124. LCMS: m/z 466.1 [M+H]⁺; ¹H NMR (400 MHz, CDCl₃) δ: 7.51 - 7.38 (m, 1H), 7.25 - 7.17 (m, 4H), 5.81 - 5.63 (m, 1H), 4.04 - 3.98 (m, 4H), 3.97 - 3.92 (m, 2H), 3.87 - 3.76 (m, 2H), 3.74 - 3.70 (m, 3H), 2.78 (s, 3H), 2.71 (dt, *J =* 2.4, 11.9 Hz, 2H), 2.05 - 1.97 (m, 2H), 1.95 - 1.84 (m, 2H), 1.54 - 1.39 (m, 3H).

### Example 123: Compound 125

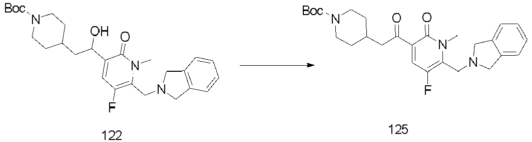

Dess-Martin oxidant (18 mg, 0.04 mmol) was added to compound 122 (14 mg, 0.05 mmol) in dichloromethane (1 mL) at 25 °C, and the mixture was stirred for 2 hours. LCMS indicated the reaction was completed. The reaction mixture was filtered, and the filtrate was concentrated under reduced pressure. The residue was purified by preparative high-performance liquid chromatography (ammonium bicarbonate conditions) to obtain compound 125. LCMS: m/z 428.1 [M-55]⁺; ¹H NMR (400 MHz, CDCl₃) δ: 8.06 (d, *J* = 9.1 Hz, 1H), 7.26 - 7.18 (m, 4H), 4.14 - 3.95 (m, 7H), 3.79 (s, 3H), 3.15 - 3.15 (m, 1H), 3.12 (d, *J* = 6.6 Hz, 2H), 2.75 (t, *J =* 11.6 Hz, 2H), 2.19 - 2.06 (m, 1H), 1.72 (d, *J* = 12.6 Hz, 2H),1.46 (s, 9H), 1.25 - 1.12 (m, 2H).

### Example 124: Compound 126

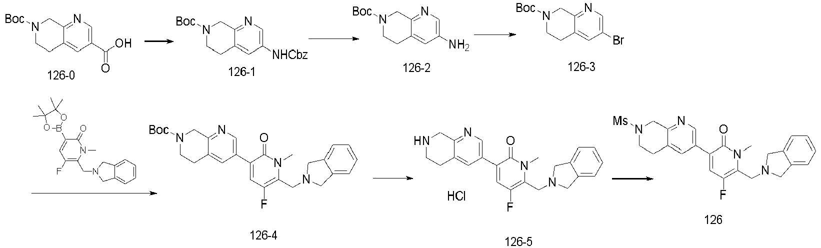

Step 1: At 25°C, starting material 126-0 (500 mg, 1.8 mmol), N,N-diisopropylethylamine (0.46 mL, 2.16 mmol), diphenylphosphoryl azide (593 mg, 2.16 mmol) and benzyl alcohol (0.93 mL, 8.9 mmol) were added to toluene (5 mL). After purging with nitrogen three times, the reaction mixture was heated to 105°C and stirred for 3 hours. LCMS indicated the reaction was completed. The reaction mixture was cooled to 25°C. The reaction was quenched with water (20 mL) and the mixture was extracted with ethyl acetate (50 mL×3). The organic phases were combined, washed with saturated brine (50 mL), and dried over anhydrous sodium sulfate. After filtration, the filtrate was concentrated under reduced pressure, and the residue was purified by silica gel column chromatography (petroleum ether: ethyl acetate = 2: 1) to obtain Intermediate 126-1. LCMS: m/z 384.2 [M+H]⁺.

Step 2: Intermediate 126-1 (600 mg, 1.56 mmol) and palladium/carbon (160 mg, 1.56 mmol, 10 wt%) were added into methanol (20 mL) at 25°C under nitrogen atmosphere. After purging with hydrogen three times, the mixture was stirred for 2 hours. LCMS indicated the reaction was completed. The reaction mixture was filtered, and the filtrate was concentrated under reduced pressure to obtain Intermediate 126-2. LCMS: m/z 250.0 [M+H]⁺.

Step 3: At 0°C, Intermediate 126-2 (320 mg, 1.28 mmol), copper bromide (276 mg, 1.93 mmol) and isoamyl nitrite (180 mg, 1.54 mmol) were added to acetonitrile (6 mL). The reaction mixture was heated to 25 °C, and stirred for 12 hours. LCMS indicated the reaction was completed. The reaction mixture was added with water (30 mL) was and extracted with ethyl acetate (30 mL×3). The organic phases were combined, washed with saturated brine (30 mL), and dried over anhydrous sodium sulfate. After filtration, the filtrate was concentrated under reduced pressure, and the residue was purified by silica gel column chromatography (petroleum ether: ethyl acetate = 5:1) to obtain Intermediate 126-3. LCMS: m/z 259.0 [M-55]⁺.

Step 4: At 25°C, Intermediate 126-3 (50 mg, 0.16 mmol), 5-fluoro-6-(isoindolin-2-ylmethyl)-1-methyl-3-(4,4,5,5-tetramethyl-1,3,2-dioxaborolan-2-yl)pyri din-2(1H)-one (74 mg, 0.16 mmol), potassium carbonate (66 mg, 0.48 mmol) and tetrakis(triphenylphosphine)palladium (19 mg, 0.02 mmol) were added to a solution of dioxane (2 mL) and water (0.4 mL). After purging with nitrogen three times, the reaction mixture was heated to 100°C and stirred for 3 hours. LCMS indicated the reaction was completed. The reaction mixture was filtered, and the filtrate was concentrated under reduced pressure. The residue was purified by silica gel column chromatography (petroleum ether: ethyl acetate = 10: 1) to obtain Intermediate 126-4. LCMS: m/z 491.2 [M+H]⁺.

Step 5: Hydrochloric acid-dioxane solution (1 mL, 4.00 mmol, 4M) was added to Intermediate 126-4 (50 mg, 0.1 mmol) at 25 °C, and the mixture was stirred for 1 hour. LCMS indicated the reaction was completed. The reaction mixture was concentrated under reduced pressure to obtain crude product Intermediate 126-5. LCMS: m/z 391.2 [M+H]⁺.

Step 6: At 25°C, methanesulfonic anhydride (27 mg, 0.15 mmol) was added to a solution of Intermediate 126-5 (30 mg, 0.08 mmol) and triethylamine (24 mg, 0.23 mmol) in dichloromethane (1 mL), and the mixture was stirred for 10 minutes. LCMS indicated the reaction was completed. The reaction mixture was diluted with water (10 mL) and extracted with ethyl acetate (10 mL×3). The organic phases were combined, washed with saturated sodium chloride solution (10 mL), dried over anhydrous sodium sulfate, and filtered. The filtrate was concentrated under reduced pressure, and the residue was purified by preparative high performance liquid chromatography (ammonium bicarbonate) to obtain compound 126. LCMS: m/z 469.1 [M+H]⁺; ¹H NMR (400 MHz, CDCl₃) δ: 8.64 (d, *J* = 1.9 Hz, 1H), 8.10 (d, *J* = 1.4 Hz, 1H), 7.53 (d, *J* = 9.0 Hz, 1H), 7.24(d, *J* = 1.3 Hz, 4H), 4.58 (s, 2H), 4.09 (s, 4H), 4.02 (d, *J =* 3.6 Hz, 2H), 3.82 (s, 3H), 3.62 (t, *J =* 5.9 Hz, 2H), 3.06 (t, *J* = 5.7 Hz, 2H), 2.91 (s, 3H).

### Example 125: Compound 127

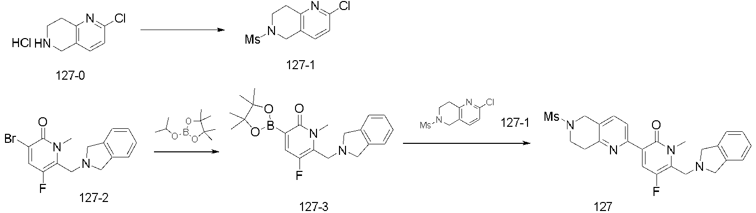

Step 1: At 25°C, methanesulfonic anhydride (854 mg, 4.90 mmol) was added to a solution of starting material 127-0 (200 mg, 0.98 mmol) and triethylamine (0.68 mL, 4.90 mmol) in acetonitrile (10 mL), and the mixture was stirred for 16 hours. LCMS indicated the reaction was completed. The reaction was quenched with water (15 mL) and the mixture was extracted with ethyl acetate (15 mL×3). The organic phases were collected, and washed with saturated brine (40 mL). The organic phase was concentrated under reduced pressure, and the residue was purified by silica gel column chromatography (dichloromethane:methanol = 10:1) to obtain Intermediate 127-1. LCMS: m/z 247.0 [M+H]⁺.

Step 2: At 0°C, after purging with nitrogen three times, isopropylmagnesium chloride lithium chloride complex solution (0.46 mL, 0.59 mmol, 1.3 M) was slowly added dropwise into a solution of Intermediate 127-2 (100 mg, 0.30 mmol) in tetrahydrofuran (5 mL), and the mixture was stirred for 0.5 hours, then 2-isopropoxy-4,4,5,5-tetramethyl-1,3,2-dioxaborolane (165 mg, 0.89 mmol) was slowly added dropwise and the mixture was stirred for 2.5 hours. LCMS indicated the reaction was completed. The temperature was raised to 25°C, the reaction was quenched with water (5 mL), and the mixture was extracted with ethyl acetate (5 mL×3). The organic phases were collected, washed with saturated brine (15 mL) and concentrated under reduced pressure to obtain Intermediate 127-3. LCMS: m/z 303.0 [M-81]⁺.

Step 3: At 25°C, Intermediate 127-3 (50 mg, 0.13 mmol), Intermediate 127-1 (100 mg, 0.39 mmol), potassium carbonate (100 mg, 0.72 mmol), tetrakis triphenylphosphine palladium (30 mg, 0.02 mmol) were dissolved in 1,4-dioxane (3 mL) and water (1 mL). After purging with nitrogen three times, the reaction mixture was heated to 90°C, and the mixture was stirred for 16 hours. LCMS indicated the reaction was completed. The reaction mixture was cooled the temperature to 25°C. The reaction was quenched with water (5 mL), and the mixture was extracted with ethyl acetate (5 mL×3). The organic phases were collected and washed with saturated brine (15 mL). The organic phase was concentrated under reduced pressure, and the residue was purified by preparative high-performance liquid chromatography (ammonium bicarbonate conditions) to obtain compound 127. LCMS: m/z 469.0 [M+H]⁺; ¹H NMR (400MHz, CDCl₃) δ: 8.53 (d, *J =* 8.1 Hz, 1H), 8.39 (d, *J* = 9.9 Hz, 1H), 7.45 - 7.55 (m, 1H), 7.19 - 7.27 (m, 4H), 4.53 (s, 2H), 3.99-4.29 (m, 6H), 3.84 (s, 3H), 3.71 (t, *J* = 6.1 Hz, 2H), 3.20 (t, *J* = 5.9 Hz, 2H), 2.90 (s, 3H).

### Example 126: Compound 128

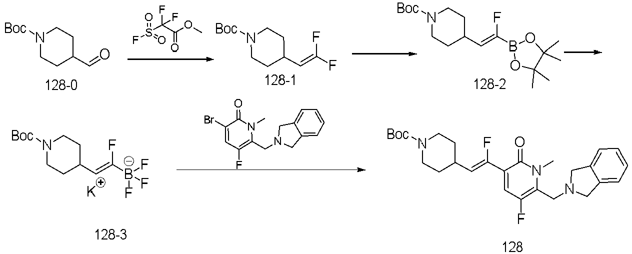

Step 1: At 80°C, triphenylphosphine (18.45 g, 70.33 mmol), potassium iodide (11.67 g, 70.33 mmol) and starting material 128-0 (5.00 g, 23.44 mmol) were dissolved in acetonitrile (50 mL), and the mixture was stirred for 30 minutes. Methyl 2,2-difluoro-2-(fluorosulfonyl)acetate (5.97 mL, 23.44 mmol) was added dropwise, and the mixture was continuously stirred for 16 hours. The reaction mixture was cooled to 25°C. The reaction was quenched with water (100 mL) and the mixture was extracted with ethyl acetate (100 mL). The organic phase was dried over anhydrous sodium sulfate, and filtered. The filtrate was concentrated under reduced pressure. The residue was purified through silica gel column chromatography (petroleum ether: Ethyl acetate = 10:1) to obtain Intermediate 128-1. ¹H NMR (400 MHz, CD₃Cl) δ: 4.19 - 3.95 (m, 3H), 2.87 - 2.67 (m, 2H), 2.42 - 2.18 (m, 1H), 1.67 (dd, *J* = 2.6, 13.2 Hz, 2H), 1.46 (s, 9H), 1.37 - 1.15 (m, 2H).

Step 2: At 25°C, potassium tert-butoxide (91 mg, 0.81 mmol) was added to a solution of Intermediate 128-1 (100 mg, 0.40 mmol), copper chloride (2.00 mg, 0.02 mmol), bis(pinacolato)diboron (256 mg, 1.01 mmol) and 4,5-bis(diphenylphosphino)-9,9-dimethylxanthene (12 mg, 0.02 mmol) in N,N-dimethylformamide (1 mL). The reaction mixture was heated to 30°C and stirred for 3 hours. TLC indicated the reaction was completed. The reaction mixture was filtered, and the filtrate was concentrated under reduced pressure to obtain crude product Intermediate 128-2.

Step 3: Potassium hydrofluoride (132 mg, 1.69 mmol) was added to a solution of crude Intermediate 128-2 in methanol (10 mL) at 25 °C, and the mixture was stirred for 16 h. The reaction mixture was filtered, the filter cake was washed three times with acetonitrile and dried to obtain crude product Intermediate 128-3.

Step 4: At 25°C, 3-bromo-5-fluoro-6-(isoindolin-2-ylmethyl)-1-methylpyridin-2(1H)-one (50 mg, 0.15 mmol), potassium carbonate (41 mg, 0.30 mmol), tetrakis(triphenylphosphine)palladium (17 mg, 0,01 mmol) and Intermediate 128-3 (220 mg, 0.74 mmol) were dissolved in dioxane (2 mL) and water (0.5 mL). After purging with nitrogen three times, the reaction mixture was heated to 100°C and stirred for 1 hour. LCMS indicated the reaction was completed. The reaction mixture was directly filtered, and the filtrate was concentrated under reduced pressure. The residue was purified by preparative thin-layer chromatography (petroleum ether: ethyl acetate = 1: 1) to obtain compound 128. LCMS: m/z 486.2 [M+H]⁺; ¹H NMR (400 MHz, CDCl₃) δ: 7.50 (d, *J =* 9.8 Hz, 1H), 7.18 - 7.10 (m, 4H), 6.73 - 6.51 (m, 1H), 4.12 - 3.81 (m, 8H), 3.69 (s, 3H), 2.89 - 2.65 (m, 3H), 1.72 - 1.61 (m, 2H), 1.39 (s, 9H), 1.38 - 1.27 (m, 2H).

### Example 127: Compound 129

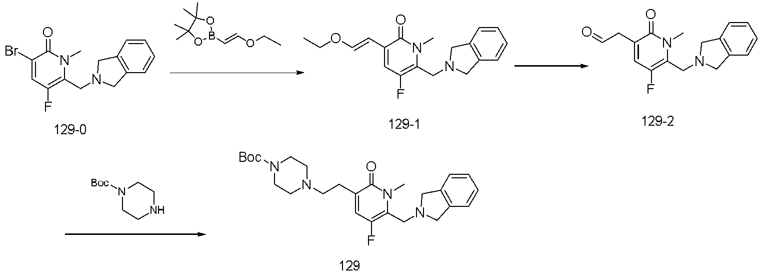

Step 1: At 25°C, starting material 129-0 (200 mg, 0.59 mmol), (E)-2-(2-ethoxyvinyl)-4,4,5,5-tetramethyl-1,3,2-dioxaborolane (218 mg, 1.19 mmol), potassium carbonate (246 mg, 1.78 mmol), and tetrakis triphenylphosphine palladium (69 mg, 0.06 mmol) were dissolved in 1,4-dioxane (10 mL) and water (3 mL). After purging with nitrogen three times, the reaction mixture was heated to 90°C, and the mixture was stirred for 16 hours. LCMS indicated the reaction was completed. The reaction mixture was cooled to 25°C. The reaction mixture was added with water (15 mL) and extracted with ethyl acetate (15 mL×3). The organic phases were collected, washed with saturated brine (15 mL), and concentrated under reduced pressure. The residue was purified by silica gel column chromatography (petroleum ether: ethyl acetate = 3: 1) to obtain Intermediate 129-1. LCMS: m/z 329.2 [M+H]⁺.

Step 2: Intermediate 129-1 (30 mg, 0.09 mmol) and trifluoroacetic acid (1.01 mL, 0.90 mmol) were dissolved in acetonitrile (1 mL) and water (1 mL) at 25 °C, and the mixture was stirred for 5 hours. LCMS indicated the reaction was completed. the reaction mixture was added with water (5 mL) and extracted with ethyl acetate (5 mL×3). The organic phases were collected, washed with saturated brine (15 mL) and concentrated under reduced pressure to obtain Intermediate 129-2. LCMS: m/z 301.0 [M+H]⁺.

Step 3: At 25°C, Intermediate 129-2 (15 mg, 0.05 mmol), tert-butyl piperazine-1-carboxylate (20 mg, 0.10 mmol), sodium borohydride acetate (22 mg, 0.10 mmol) and acetic acid (0.1 ml, 0.1 mmol) were dissolved in 1,2-dichloroethane (3 mL) solution, the mixture was stirred for 4 hours. LCMS indicated the reaction was completed. The reaction was quenched by adding water (5 mL) and the mixture was extracted with dichloromethane (5 mL×3). The organic phases were collected, washed with saturated brine (10 mL) and concentrated under reduced pressure. The residue was purified by preparative high-performance liquid chromatography (ammonium bicarbonate conditions) to obtain compound 129. LCMS: m/z 471.3 [M+H]⁺; ¹H NMR (400MHz, CDCl₃) δ: 7.21 (s, 1H), 7.09 - 7.16 (m, 4H), 3.91 (s, 4H), 3.83 (d, *J* = 4.1 Hz, 2H), 3.65 (s, 3H), 3.44(br s, 4H), 2.60 - 2.81 (m, 4H), 2.50 (br s, 4H), 1.39 (s, 9H).

### Bioassay examples

### Bioassay example 1: NCI-H295R cellular pregnenolone biosynthesis inhibition assay (ELISA method)

### I. Experiment purpose

The experiment aims to test the inhibitory effect of the compounds of the present disclosure on cellular pregnenolone biosynthesis, and to assess the inhibitory effect of the compounds on CYP11A1 target based on their IC50 values.

### II. Experiment procedures

NCI-H295R cells (MINGZHOUBIO, MZ-2138) cultured in NCI-H295R complete medium (MINGZHOUBIO, MCM-0399).

Using culture medium, NCI-H295R cells were seeded into a new 384-well cell culture plate (Greiner, 781091) at a density of 5,000 cells per well, with 45µL of cell suspension per well. The test compounds were prepared by adding 5µL of compound solution at 10× concentration to each well, to a maximum final concentration of 10µM, followed by 3× serial dilution for 10 points, with a final DMSO concentration of 0.1%, centrifuging at 1,000 rpm for 1 minute at room temperature, and then incubating in a 5% CO₂ cell incubator at 37°C for 3 days.

Pregnenolone ELISA kit (Abnova, KA1912) was used for detecting the content of pregnenolone in the supernatant of cell culture. Before detection, the reagents were allowed to come back to room temperature for 1 hour. After taking out the 384-well plate, the cellular supernatant was pipetted and subjected to 5× dilution with the Assay Buffer provided in the kit. A working solution of competitive molecule and a washing solution in accordance with the instructions was prepared. 50µL of diluted cellular supernatant were pipetted into the 96-well plate provided in the ELISA kit, added with 100µL of working solution of competitive molecule per well and the plate was incubated at room temperature for 1.5 hours. After discarding the liquid, the plate was washed with 300µL of washing solution per well three times and dried. After adding 150 µL of substrate chromogenic solution per well, the plate was incubated at room temperature for 10-15 minutes. After adding 50 µL of termination solution per well, the plate was incubated at room temperature for 10-15 minutes and read for absorbance at 450nm with Envision HTS Multilabel Reader.

### III. Data analysis

The wells added with 1µM ODM-208 were defined as 100% inhibition (positive control), the wells containing DMSO at a final concentration of 0.1% were defined as 0% inhibition (negative control), and inhibitory ratio = (absorbance of tested sample - average absorbance of negative control)/(average absorbance of positive control - average absorbance of negative control)*100%. The data were subjected to four-parameter fitting using GraphPad Prism to calculate the IC₅₀ values of the compounds. The results are as shown in the table below.

ODM-208 has a structure of

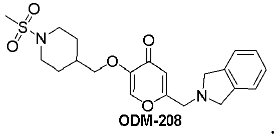

**Table 1**

| **Compound number** | **IC₅₀** | **Compound number** | **IC₅₀** | **Compound number** | **IC₅₀** |
|---|---|---|---|---|---|
| compound 1 | C | compound 2 | C | | |
| compound 4 | B | compound 5 | C | compound 6 | C |
| compound 7 | C | compound 8 | C | compound 9 | C |
| compound 10 | A | compound 11 | A | compound 13 | A |
| compound 14 | A | compound 15 | A | compound 16 | A |
| compound 17 | A | compound 18 | A | compound 19 | B |
| compound 20 | B | compound 21 | A | compound 22 | A |
| compound 23 | C | compound 24 | C | compound 25 | A |
| compound 26 | A | compound 27 | B | compound 28 | A |
| compound 29 | A | compound 30 | A | compound 31 | C |
| compound 32 | A | compound 33 | A | compound 34 | A |
| compound 35 | B | compound 12 | A | compound 52 | A |
| compound 36 | C | compound 37 | B | compound 38 | C |
| | | compound 40 | A | compound 53 | A |
| compound 41 | B | compound 54 | A | compound 59 | A |
| compound 42 | C | compound 43 | B | compound 44 | A |
| compound 60 | A | compound 57 | C | compound 45 | C |
| compound 46 | A | compound 55 | C | compound 56 | C |
| compound 47 | C | compound 48 | B | compound 49 | A |
| compound 50 | A | compound 51 | C | compound 58 | C |
| compound 61 | B | compound 62 | B | compound 63 | A |
| compound 64 | A | compound 65 | B | compound 66 | A |
| compound 67 | A | compound 68 | A | compound 69 | A |
| compound 70 | A | compound 71 | B | compound 72 | A |
| compound 73 | B | compound 74 | A | compound 75 | A |
| compound 76 | B | compound 77 | B | compound 78 | A |
| compound 79 | A | compound 80 | A | compound 81 | A |
| compound 82 | B | compound 83 | C | compound 84 | A |
| compound 85 | A | compound 86 | B | compound 87 | C |
| compound 88 | C | compound 89 | B | compound 90 | A |
| compound 91 | C | compound 92 | B | compound 93 | B |
| compound 94 | B | compound 95 | C | compound 96 | C |
| compound 97 | C | compound 98 | B | compound 99 | C |
| compound 100 | C | compound 101 | C | compound 102 | A |
| compound 103 | B | compound 104 | A | compound 105 | A |
| compound 106 | A | compound 107 | A | compound 108 | A |
| compound 109 | A | compound 110 | A | compound 111 | A |
| compound 112 | A | compound 113 | A | compound 114 | A |
| compound 115 | A | compound 116 | A | compound 117 | A |
| compound 118 | B | compound 119 | B | compound 120 | A |
| compound 121 | A | compound 122 | A | compound 123 | A |
| compound 124 | A | compound 125 | A | compound 126 | A |
| compound 127 | C | compound 128 | B | compound 129 | A |

The experiment demonstrates that the compounds of the present disclosure can effectively inhibit the conversion of cholesterol into pregnenolone in NCI-H295R cells, with activity results of some compounds of the present disclosure obtained by the above method shown in Table 1, wherein A means IC₅₀ < 100nM, B means 100nM<IC₅₀<300nM, and C means IC₅₀>300nM.

### Bioassay example 2: PK study

### Experimental procedures

### 1. Animals

ICR mice (male/female, total 6 animals) were assigned to two groups for oral administration and intravenous administration, respectively, with 3 mice in each group. Before administration, the animals were fasted for 10-14 hours, with water available ad lib.

SD Rat (male/female, total 6 animals) were assigned to two groups for oral administration and intravenous administration, respectively, with 3 mice in each group. Before administration, the animals were fasted for 10-14 hours, with water available ad lib.

### 2. Preparation of the formulation to be administered

The compounds to be tested were weighed out in accordance with intended dosage, formulated with vehicles (5% DMSO + 10% Solutol + 85% physiological saline) to dosage form at appropriate concentrations (administered intravenously and orally in rats or mice at a volume of 5 mL/kg and 10 mL/kg, respectively). The dosage form for intravenous injection is a clear solution, and the dosage form for oral administration is a clear solution or homogenous suspension.

### 3. Administration and blood sample collection

Administration to animals was conducted through intravenous injection and oral gavage, and blood samples were collected at 0.033, 0.083, 0.25, 0.5, 1, 2, 4, 8, and 24 hours after intravenous injection, or at 0.083, 0.25, 0.5, 1, 2, 4, 6, 8, and 24 hours after oral administration. After centrifugation of whole blood samples at 4°C and 6,800g for 6 minutes, the upper layers of plasma were stored at -80°C refrigerator before analysis.

### 4. Assay of plasma samples

The DMSO stock solutions of the substance to be tested were diluted with methanol or acetonitrile solution to serial working solutions and added to blank plasma matrix to formulate samples for standard curve and quality control. An appropriate volume of plasma sample was added with appropriate fold of methanol or acetonitrile containing internal standards, according to response, for protein precipitation. After centrifugation of all samples at 4°C and 18,000g for 10 min, appropriate volumes of supernatant were taken for LC-MS/MS analysis.

### 5. Parameter calculation

Plasma drug concentration-time curves were plotted in accordance with tested concentrations, and pharmacokinetic parameters were calculated based on non-compartmental model using WinNonlin software, including half-life (T1/2), area under the drug concentration-time curve (AUC0-t), clearance rate (CL), steady-state distribution volume (Vss), bioavailability (F), etc.

As showed by results, at least some of the compounds of the present disclosure exhibit good pharmacokinetic parameters. For example, at oral administration of 5 mpk, ODM-208 has a Cmax of 807 ng/ml, an AUC of 1309 ng/ml*h, a half-life (t1/2) of 1.8 h, and a bioavailability (F) of 59%, and the Cmax of some of the compounds of the present disclosure is greater than 1,000 ng/ml, e.g., compounds 112, 113, the AUC of some of the compounds of the present disclosure is greater than 1700 ng/ml*h, e.g., compounds 53, 72, 112, 113, the half-life (t1/2) of some of the compounds of the present disclosure is greater than 3 h, e.g., compounds 53, 72, and the bioavailability (F) of some of the compounds of the present disclosure is greater than 60%, e.g., compounds 72, 112, 113.

### Bioassay example 3: Compound stability experiment:

### Experiment purpose: to test the chemical stability of the compound of the present disclosure.

Experiment procedures: 1mg of the compounds to be tested were weighed out and treated separately as shown in Table 1, and the purity was tested before and after the treatment to assess their stability under different conditions. Compound purity was analysed with Reversed Phase High Performance Liquid Chromatography (HPLC), with the following conditions: SUIMADZU HPLC (LC-2030 Plus), Waters Sunfire C18 column (3.5 µm, 4.6 × 100 mm), detected at UV detection wavelengths of 220 and 254 nm, eluted by 5-95% acetonitrile (containing 0.05% - 0.1% v/v formic acid or trifluoroacetic acid or ammonium bicarbonate), at a flow rate of 1.8 mL/min.

| Group | Storage form | Container for Placement | Placing Temperature & Duration |
|---|---|---|---|
| 1 | DMSO solution | placed in open EP tube | 0°C, 5 days |
| 2 | | placed in open EP tube | 25°C, 5 days |
| 3 | solid compound powder | placed in open EP tube | 0°C, 5 days |
| 4 | | placed in open EP tube | 25°C, 5 days |

The experiment demonstrates that the compounds of the present disclosure exhibit good stability.

### Bioassay example 4: In vivo pharmacodynamic study of the test substance in a VCaP castration-resistant prostate cancer subcutaneous transplant tumor model

Experiment purpose: to evaluate the pharmacological effects and efficacy of the compound of the present disclosure in a VCaP castration-resistant prostate cancer subcutaneous transplant tumor model.

Animals: experimental male SCID-Beige mice, 6-8-weeks old or 18-22g, provided by Beijing Vital River Laboratory Animal Technology Co., Ltd.

Experiment procedures: all experimental animals were kept within specific-pathogen-free animal barrier facility, and allowed 3 days of adaptation before the experiment began. VCaP tumor cells were in vitro cultured as monolayer in DMEM complete medium (with 10% fetal bovine serum and 1% penicillin & streptomycin) at 37°C in air containing 5% CO₂. Tumor cells were routinely passaged 2-3 times a week, and cells in exponential growth phase were collected and counted for tumor cell inoculation. 10x10⁶ VCaP tumor cells were resuspended in 0.1ml PBS, thoroughly mixed with 0.1ml matrigel, and then inoculated subcutaneously on the right flank of each mouse to form a tumor. The tumor-bearing mice were allowed to develop to an average tumor volume of around 200mm³ and underwent castration surgery. The tumor volumes of all mice were measured after one week of castration surgery. Suitable tumor-bearing mice were selected and assigned randomly into the following groups: blank control group, prednisone (0.6mg/kg, QD) group, abiraterone (33.73mg/kg, QD) + prednisone (0.6mg/kg, QD) group, ODM-208 (30mg/kg, BID) + prednisone (0.6mg/kg, QD) group, and the compound of the present disclosure (30mg/kg, BID) + prednisone (0.6mg/kg, QD) group. Animals in blank control group were orally administered with vehicles (0.5%MC+0.5%Tween80 and 0.5%CMCNa), and the animals in other groups were orally administered with corresponding compound. The experiment lasted for 32 days, and during the experiment, the animals in each group were measured for tumor volume and body weight twice a week.

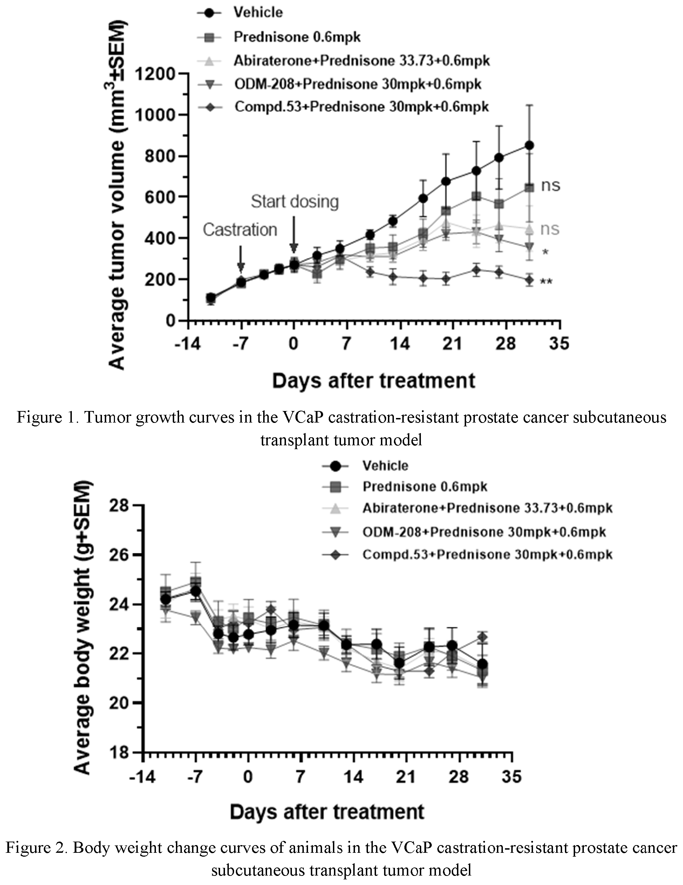

The experiment demonstrates at least some of that the compounds of the present disclosure exhibit good inhibitory activity on tumor proliferation and safety in the VCaP castration-resistant prostate cancer subcutaneous transplant tumor model, and their tumor inhibitory activity is significantly better than ODM-208, without significant weight loss, e.g., compound 53 and compound 72. Among them, the tumor inhibition rate TGI of compound 53 reaches 112% (see Figure 1), which is superior to that of ODM-208 (TGI = 85%); and the effect of compound 53 on animal weight loss is significantly less than that of ODM-208 (-2% and - 10%, see Figure 2), suggesting that compound 53 not only exhibits good tumor inhibitory activity, but also exhibits good safety. Compound 72 exhibits similar advantages to compound 53 in this model, such as having a better tumor inhibition rate TGI, and significantly better than ODM-208.

### Bioassay example 5: detection of concentrations of testosterone, progesterone, corticosterone, and tested compounds in mouse plasma, tumors and adrenal glands by LC-MS

### Experimental materials:

### Tissue sample homogenization:

Mouse tumors were homogenized using PBS in a 1:5 ratio (tumor weight (g): PBS volume (mL), w/v);
Mouse adrenal glands were homogenized using PBS in a 1:10 ratio (adrenal gland weight (g): PBS volume (mL), w/v);
Standard sample: testosterone (LGC, C17322500), progesterone (MCE, HY-N0437), adrenal ketone (MCE, HY-B1618), and standard solution of test substance.

Experiment procedures: since the three markers, i.e., testosterone, progesterone, and adrenal ketone, are all endogenous substances and present in the blank matrix as well, PBS was alternatively used as matrix in the production of the standard curve.

Testosterone: multiple reaction monitoring (MRM) method was used for detecting the concentrations of testosterone in mouse tumors and adrenal glands. Mass spectrum: AB Sciex Triple Quad 6500+; liquid chromatography: Shimadzu 40A, column: Waters XBridge BEH C18 2.5 µm 2.1*50mm, testosterone ion pair: 289.4/97.2. Both tumor and adrenal tissues were treated with methanol as precipitant and tolbutamide as internal standard for sample pretreatment, with linear range in 50-50,000pg/mL.

Progesterone and corticosterone: multi-reaction detection method (MRM) was used for detecting the concentrations of progesterone and corticosterone in mouse tumors and adrenal glands. Mass spectrum: AB Sciex Triple Quad 6500+; liquid chromatography: Shimadzu 40A, column: Waters XSelect HSS T3 2.5 µm 2.1*50mm, progesterone ion pair: 315.4/97.2, adrenone ion pair: 347.4/121.3. For tumor tissues, acetonitrile was used as precipitant and propranolol diclofenac as internal standard for sample pretreatment, with linear ranges in 0.05-10 ng/mL of progesterone and 0.10-20 ng/mL of corticosterone. For adrenal tissues, methanol was used as precipitant and propranolol diclofenac as internal standard for sample pretreatment, with linear ranges in 5-2,500 ng/mL of progesterone and 5-2,500 ng/mL of corticosterone.

Concentrations of tested compounds in mouse plasma, tumors and adrenal glands: The multiple reaction monitoring (MRM) method was used to detect the concentrations in mouse plasma, tumors and adrenal glands. Mass spectrum: AB Sciex Triple Quad 6500+, liquid chromatography: Shimadzu 40A, column: XBridge BEH C18 2.5 µm 2.1*50mm, ODM208 ion pair: 419.20/176.20, tested compounds ion pair: 429.20/200.20. Acetonitrile was used as precipitant, and propranolol diclofenac were used as the internal standard for sample pretreatment in plasma, tumor, and adrenal tissue. The linear ranges of the standard in plasma tissue was 0.5-1000 ng/mL, the linear ranges of the standard in tumor tissue was 3-6000 ng/mL, and the linear ranges of the standard in adrenal tissue was 5.5-11000 ng/mL.

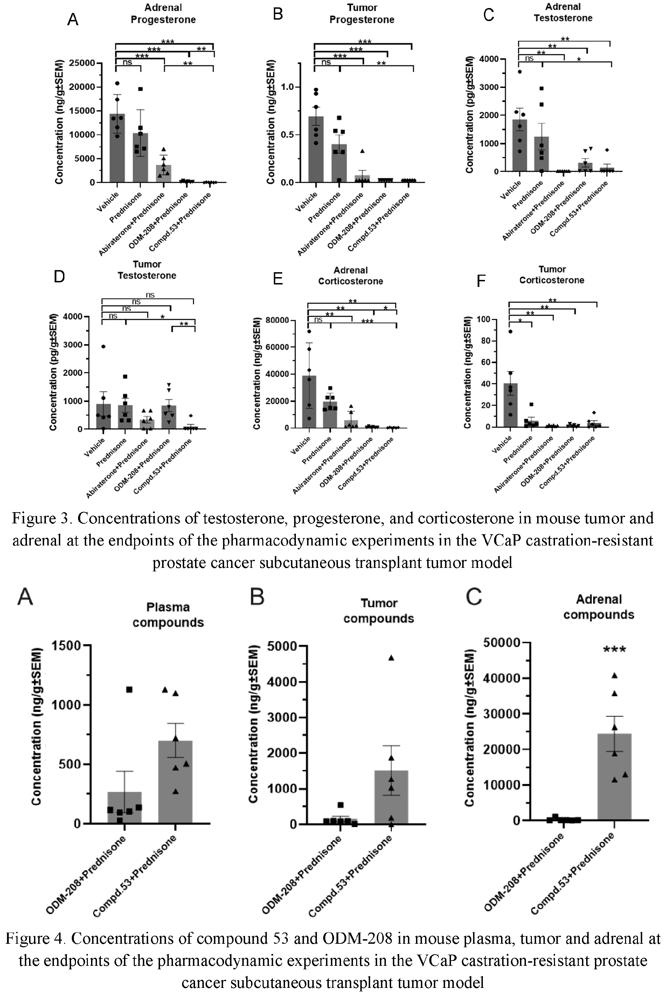

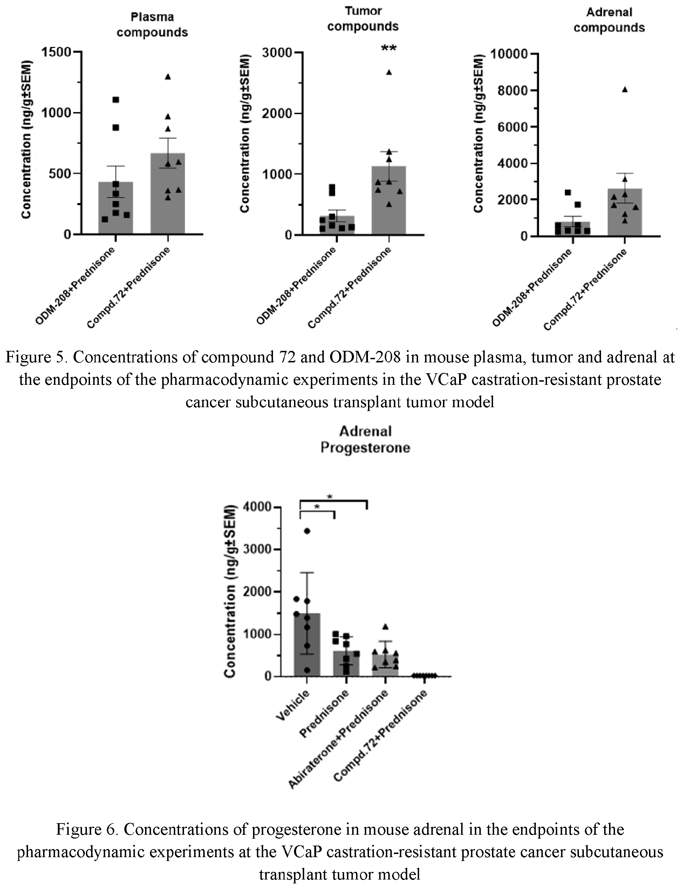

At least some of the compounds of the present disclosure, e.g., Compound 53 and Compound 72, were tested to have significantly higher drug concentrations in plasma, tumors, and adrenal glands than ODM-208, with superior target-organ enrichment effects, and significantly inhibited the secretion of testosterone, progesterone, and corticosterone in both tumors and adrenal glands in mice, and the inhibitory effects were significantly better than those of ODM-208. For example, based on the in vivo pharmacodynamic studies of Compound 53 and ODM-208 in the VCaP castration-resistant prostate cancer subcutaneous transplant tumor model in bioassay example 4, using the method of the present assay, the concentrations of ODM-208 and Compound 53 in plasma, tumors and adrenal glands in the ODM-208+prednisone group and the Compound 53+prednisone group were tested, as well as the concentrations of testosterone, progesterone and corticosterone in mouse tumors and adrenal glands, and the results are shown in Figures 3 and 4: ① Compound 53 demonstrates higher drug concentrations in plasma, tumors, and adrenal glands, which are 3-fold, 10-fold, and 80-fold higher than those of ODM-208, respectively, and Compound 53 possesses a higher drug concentration and superior target-organ-enrichment effect in mice. ② Compound 53 and ODM-208 significantly inhibits the secretion of testosterone, progesterone and adrenal ketone in mouse tumors and adrenal glands in the VCaP castration-resistant prostate cancer subcutaneous transplant tumor model, with Compound 53 being more effective, e.g., in the tumors, the concentration of testosterone of the compound 53 group is only 1/8 of that of the ODM-208 group, in the adrenal glands, the concentration of progesterone of the compound 53 group is only 1/4 of that of ODM-208, and it is not inferior to ODM-208 for the rest of the indexes. Compound 72 also shows similar advantages to Compound 53. The results are shown in Figure 5 and Figure 6: ① Compound 72 shows higher drug concentrations in plasma, tumors and adrenal glands, which are 1.6-fold, 3.6-fold and 3.3-fold higher than those of ODM-208, respectively, compound 72 has higher drug concentration and better target organ enrichment effect in mice. ② Compound 72 has a significant inhibitory effect on the secretion of progesterone in the adrenal glands of mice in the VCaP castration-resistant prostate cancer subcutaneous transplant tumor model, which is significantly better than that of the control group.

### Bioassay example 6: hERG testing

### I. Experiment procedures:

The cells used in this experiment were HEK293 cell lines transfected with hERG cDNA and stably expressing hERG channels (#60187, provided by BPS Bioscience Inc., P3-P23 generations were used in the experiment). The cells were cultured in a culture medium containing the following components: MEM medium, 10% (v/v) inactivated fetal bovine serum, 1 mM sodium pyruvate, 500µg/ml geneticin, 0.1mM non-essential amino acid, 100U/ml penicillin-streptomycin. HEK293 hERG cells were grown in petri dishes containing the aforementioned culture medium and cultured in an incubator at 37°C and 5% CO₂, passaged approximately three times a week and maintained at a cell fusion rate between 40% and 80%. 24 to 48 hours before the electrophysiological experiment, HEK293 hERG cells were transferred to glass slides pre-treated with 0.05mg/ml PDL, with 1×10⁴ cells were placed in a 48-well plate and grew under the same culture medium and conditions. The density of HEK293 hERG cells on each circular slide should meet the requirement that the majority of cells are independent and individual cells. The constituents of extracellular fluid used in the hERG experiment were as follows (in mM): 145 NaCl, 4 KCl, 2 CaCl₂, 1 MgCl₂, 10 glucose, and 10 HEPES (adjusting to pH 7.40 with NaOH). The constituents of intracellular fluid were as follows (in mM): 130 KCl, 2 MgCl₂, 5 EGTA, 10 HEPES and 5 Na₂ATP (adjusting to pH 7.25 with KOH). For measuring the IC₅₀, the compounds were used in the following concentrations (30, 10, 3, 1, 0.3, and 0.1µM) in the experiment (the concentration of the compounds might be adjusted appropriately in cases that it led to too strong effects). Prior to the experiment, stock solutions at 10, 3, 1, 0.3, and 0.1 mM were produced by gradient dilution with DMSO, which were then diluted with extracellular fluid to the final testing concentrations in µM. The final concentration of DMSO in the compound solutions of each concentration should range from 0.1% to 0.3%. All compound solutions will undergo regular ultrasound of 5 to 10 minutes and sufficient shaking to ensure complete dissolution of the compound. All test solutions should be rotated for at least 10 minutes for homogeneity.

The electrophysiological experiment was carried out using a manual patch clamp system (HEKA EPC-10 signal amplifier and digital conversion system, HEKA Electronics, Germany) to record the whole cell currents. Concrete experiment procedures were described as follows: placing a circular slide with CHO hERG cells growing on the surface in an electrophysiological recording chamber under an inverted microscope, with the recording chamber continuously perfused with extracellular fluid (approximately 1 millilitre per minute). Conventional whole cell patch clamp current recording technology were used in all experiment processes. Unless otherwise specified, all experiments were conducted at regular room temperature (~25°C). The cells were held at a clamp voltage of -80 mV, the cell clamp voltage was depolarized to +30 mV to activate the hERG potassium channel, and after 5 seconds the clamp voltage was held down to -50 mV to eliminate inactivation and generate a tail current, with the peak of the tail current used as a numerical value for the magnitude of hERG current. Only when the hERG potassium current recorded in the previous step achieved stable state under continuous extracellular fluid perfusion within the recording chamber, could the compound to be tested be continuously perfused until the inhibitory effect of the compound on the hERG current reaches a stable state. Generally, the coincidence of the latest three consecutive current recording lines was used as the criterion for determining the stable state.

### II. Data analysis

The dose-response curve of the tested compound was plotted with the % hERG inhibition rate as the vertical axis and the concentration of the tested compound as the horizontal axis, and then IC₅₀ was calculated.

**Table 2**

| Compound number | hERG (µM) | Compound number | hERG (µM) |
|---|---|---|---|
| 72 | >30 | 112 | >30 |

The results of the experiment demonstrates that at least some of the compounds of the present disclosure exhibit no significant inhibition effect on hERG. Some of the test results are shown in Table 2.

## Claims

1. A compound represented by formula (0), or a stereoisomer, a tautomer, a diastereoisomer, a racemate, a cis-trans isomer, an isotope labeled compound (preferably deuterated compound), an N-oxide, a metabolite, an ester, a prodrug, a crystalline form, hydrate, a solvate, or a pharmaceutically acceptable salt thereof: 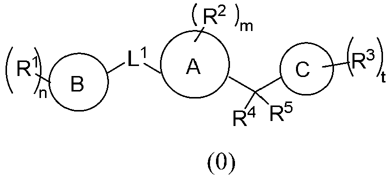 wherein:
ring A is C₆₋₁₀ aryl, 5-6 membered heteroaryl, or 5-12 membered heterocyclyl, wherein the heteroatom in the 5-6 membered heteroaryl is selected from N, O and S, and the number of the heteroatom is 1, 2, 3, or 4; the heteroatom in the 5-12 membered heterocyclyl is selected from N, O and S, the number of the heteroatom is 1, 2, 3, or 4;
L^{!} is a bond, O, S, -NH-, C₁₋₆ alkylene that is unsubstituted or substituted by one or more L¹⁻¹, C₂₋₆ alkenylene that is unsubstituted or substituted by one or more L¹⁻², C₂₋₆ alkynylene that is unsubstituted or substituted by one or more L¹⁻³, *-C₁₋₆ alkylene-S- that is unsubstituted or substituted by one or more L¹⁻⁴, *-S-C₁₋₆ alkylene- that is unsubstituted or substituted by one or more L¹⁻⁵, *-C₁₋₆ alkylene-O- that is unsubstituted or substituted by one or more L¹⁻⁶, *-O-C₁₋₆ alkylene- that is unsubstituted or substituted by one or more L¹⁻⁷, *-O-C₂₋₆ alkenylene-, *-O-C₆₋₁₀ arylene- that is unsubstituted or substituted by one or more L¹⁻⁸, *-C₆₋₁₀ arylene-O- that is unsubstituted or substituted by one or more L¹⁻⁸, *-SO₂-C₁₋₃ alkylene that is unsubstituted or substituted by one or more L¹⁻⁹, *-C(=O)-NH-, *-NH-C(=O)-, *-C₁₋₆ alkylene-O-C₁₋₆ alkylene-that is unsubstituted or substituted by one or more L¹⁻¹⁰ or *-C₁₋₆ alkylene-C(O)- that is unsubstituted or substituted by one or more L¹⁻¹⁰; wherein the mark "*" indicates the bond connected to ring B;
preferably, L¹ is a bond, O, -NH-, C₁₋₆ alkylene that is unsubstituted or substituted by one or more L¹⁻¹, C₂₋₆ alkenylene that is unsubstituted or substituted by one or more L¹⁻², C₂₋₆ alkynylene, *-C₁₋₆ alkylene-S-, *-C₁₋₆ alkylene-O-, *-O-C₁₋₆ alkylene-, *-C₆₋₁₀ arylene-O-, *-C₁₋₆ alkylene-C(O)-;
each L¹⁻¹, L¹⁻², L¹⁻³, L¹⁻⁴, L¹⁻⁵, L¹⁻⁶, L¹⁻⁷, L¹⁻⁸, L¹⁻⁹, L¹⁻¹⁰ is independently halogen, OH, C₁₋₆ alkyl, C₃₋₆ cycloalkyl or 3-6 membered heterocyclyl, wherein the heteroatom in the 3-6 membered heterocyclyl is selected from N, O and S, and the number of the heteroatom is 1, 2, 3, or 4; wherein the C₁₋₆ alkyl, the C₃₋₆ cycloalkyl or the 3-6 membered heterocyclyl is optionally substituted by deuterium, halogen, OH, CN or -NH₂,
ring B is 5-12 membered heterocyclyl, wherein the heteroatom in the 5-12 membered heterocyclyl is selected from N, O and S, and the number of the heteroatom is 1, 2, 3, or 4;
each R¹ is independently deuterium, halogen, cyano, hydroxy, oxo, C₁₋₆ alkoxy, R¹⁻¹, -C(=O)-R¹⁻¹, -(CR¹⁻²R¹⁻³)_{q}-C(=O)-OR¹⁻⁴, -NR¹⁻⁷R¹⁻⁸, -C(=O)-NR¹⁻⁷R¹⁻⁸, -C(=NH)-(R¹⁻⁹), -S(O)₂-R¹⁻⁹, -S(O)₂-NR¹⁻⁵R¹⁻⁶, -NR¹⁻⁵S(O)₂-R¹⁻¹⁰, -R¹⁻¹⁰-OH, or -P(=O)-R¹⁻²R¹⁻³;
preferably, each R¹ is independently oxo, -C(=O)-R¹⁻¹, -(CR¹⁻²R¹⁻³)_{q}-C(=O)-OR¹⁻⁴, NR¹⁻⁷R¹⁻⁸, -C(=O)-NR¹⁻⁷R¹⁻⁸, -C(=NH)-(R¹⁻⁹), -S(O)₂-R¹⁻⁹, -NR¹⁻⁵S(O)₂-R¹⁻¹⁰, -R¹⁻¹⁰-OH or -P(=O)-R¹⁻²R¹⁻³;
each R² is independently deuterium, OH, NH₂, halogen, CN, NO₂, C₁₋₆ alkyl, C₂₋₆ alkenyl, C₁₋₆ haloalkyl, C₁₋₆ deuteroalkyl, C₂₋₆ haloalkenyl, -O-C₁₋₆ alkyl, -C₁₋₆ alkylene-O-C₁₋₆ alkyl, -O-C₁₋₆ haloalkyl, -O-C₂₋₆ alkenyl, -O-C₃₋₁₀ cyclohydrocarbyl, -S-C₁₋₆ alkyl, -S-C₃₋₁₀ cyclohydrocarbyl, -C₃₋₁₀ cyclohydrocarbyl, 3-10 membered heterocyclyl, C₆₋₁₀ aryl, 5-10 membered heteroaryl, -C₁₋₆ alkylene-C₃₋₁₀ cyclohydrocarbyl, -OC₁₋₆ alkylene-C₃₋₁₀ cyclohydrocarbyl, -C(=O)NR²⁻¹R²⁻², -NR²⁻³-C(=O)-R²⁻⁴, or alternatively, two R² connected to the same carbon atom form an oxo group (=O), wherein the heteroatom in the 3-10 membered heterocyclyl or the 5-10 membered heteroaryl is selected from N, O and S, and the number of the heteroatom is 1, 2, 3, or 4;
optionally, two adjacent R² form 5-6 membered heterocyclyl, wherein the heteroatom in the 5-6 membered heterocyclyl is selected from N, O and S, and the number of the heteroatom is 1, 2, 3, or 4;
R¹⁻¹, R¹⁻², R¹⁻³, R¹⁻⁴, R¹⁻⁵, R¹⁻⁶, R¹⁻⁷ and R¹⁻⁸ are each independently H, C₁₋₆ alkyl, C₁₋₆ haloalkyl, C₁₋₆ deuteroalkyl, -C₁₋₆ alkylene-OH, -C₁₋₆ alkylene-CN, -C₃₋₁₀ cyclohydrocarbyl, or 3-10 membered heterocyclyl, wherein the heteroatom in the 3-10 membered heterocyclyl is selected from N, O and S, and the number of the heteroatom is 1, 2, 3, or 4;
R¹⁻⁹ and R¹⁻¹⁰ are each independently C₁₋₆ alkyl, C₁₋₆ haloalkyl, -C₁₋₆ alkylene-CN, C₂₋₆ alkenyl, C₂₋₆ haloalkenyl, C₃₋₁₀ cyclohydrocarbyl, C₆₋₁₀ aryl, 5-10 membered heteroaryl, C₁₋₆ alkyl-C₆₋₁₀ arylene-;
R²⁻¹, R²⁻², R²⁻³ and R²⁻⁴ are each independently H, halogen or C₁₋₆ alkyl;
ring C is independently 5-membered nitrogen-containing heterocyclobenzene or 5-membered nitrogen-containing heterocyclopyridine;
each R³ is independently cyano, hydroxy, halogen, or C₁₋₆ alkyl, or alternatively, two R³ connected to the same carbon atom form an oxo group (=O);
R⁴ and R⁵ are each independently H, deuterium, halogen, C₁₋₆ alkyl, C₁₋₆ haloalkyl, C₁₋₆ deuteroalkyl or R⁴ and R⁵ together with the atoms to which they are connected form C₃₋₁₀ cyclohydrocarbyl or 3-10 membered heterocyclyl,
preferably, R⁴ and R⁵ are each independently H, deuterium, halogen, C₁₋₆ alkyl; more preferably, R⁴ and R⁵ are both H or deuterium, or one of R⁴ and R⁵ is H, and the other is deuterium, halogen, or C₁₋₄ alkyl;
q is 0 or 1;
m, t and n are each independently 0, 1, 2, 3 or 4; and
wherein:
1) when ring A is benzene ring and L¹ is *-CH₂-O-, wherein the mark "*" indicates the bond connected to ring B, the compound represented by formula (0) is not 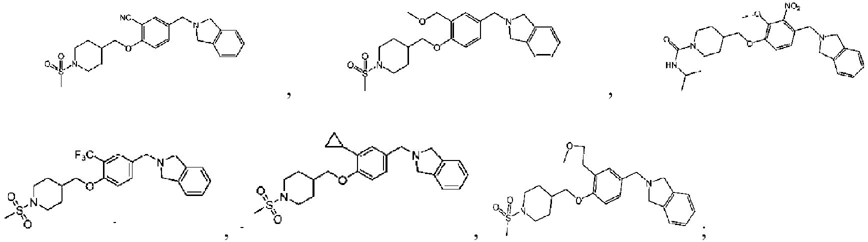
2) 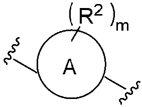 is not

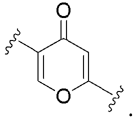

2. The compound represented by formula (0) of claim 1, wherein, the compound represented by formula (0) satisfies one or more of the following conditions:
(1) the R¹ is each independently oxo, -C(=O)-R¹⁻¹, -(CR¹⁻²R¹⁻³)_{q}-C(=O)-OR¹⁻⁴, -C(=O)-NR¹⁻⁷R¹⁻⁸, -(C=NH)-(C₁₋₆ alkyl), -S(O)₂-C₁₋₆ alkyl, -S(O)₂-C₁₋₆ haloalkyl, -S(O)₂-C₆₋₁₀ arylene-C₁₋₆ alkyl, -S(O)₂-C₃₋₁₀ cycloalkyl, -S(O)₂-5-10 membered heteroaryl, -S(O)₂-C₂₋₆ alkenyl, -NR¹⁻⁵S(O)₂-C₁₋₆ alkyl, or -P(=O)-R¹⁻²R¹⁻³; preferably oxo, -C(=O)-R¹⁻¹, -C(=O)-OR¹⁻⁴, -C(=O)-NR¹⁻⁷R¹⁻⁸, -(C=NH)-(C₁₋₃ alkyl), -S(O)₂-C₁₋₃ alkyl, -S(O)₂-C₁₋₃ haloalkyl, -S(O)₂-phenylene-C₁₋₃ alkyl, -S(O)₂-C₃₋₆ cycloalkyl, -S(O)₂-5-6 membered heteroaryl, -S(O)₂-C₂₋₃ alkenyl, -NR¹⁻⁵S(O)₂-C₁₋₃ alkyl, -P(=O)-R¹⁻²R¹⁻³; more preferably -S(O)₂-C₁₋₃ alkyl, -C(=O)-OC₁₋₆ alkyl, -S(O)₂-C₁₋₃ haloalkyl, -S(O)₂-phenylene-C₁₋₃ alkyl, -S(O)₂-C₃₋₆ cycloalkyl, -S(O)₂-5-6 membered heteroaryl, -C(=O)-C₁₋₆ alkyl, -C(=O)-C₁₋₆ haloalkyl, -C(=O)-C₁₋₆ alkylene-CN, -S(O)₂-C₂₋₃ alkenyl, -C(=O)-NHC₁₋₆ alkyl; most preferably -S(O)₂-C₁₋₃ alkyl;
(2) the ring B is 5-12 membered heterocyclyl, wherein the heteroatom in the 5-12 membered heterocyclyl is selected from N, O and S, and the number of the heteroatom is 1, 2, 3, or 4, wherein at least one heteroatom is N, preferably the number of the heteroatom is 1, 2 or 3; preferably, the ring B is 5-6 membered monocyclic heterocyclyl, 6-12 membered fused bicyclic heterocyclyl or 9-12 membered spirocyclic heterocyclyl; more preferably, the ring B is 5-6 membered monocyclic heterocyclyl or 9-12 membered fused bicyclic heterocyclyl (such as benzo-6-membered heterocycloalkyl or pyrido-6-membered heterocycloalkyl); most preferably, the ring B is 6 membered monocyclic heterocyclyl (such as piperidinyl, piperazinyl);
(3) the L¹ is a bond, O, -NH-, C₁₋₄ alkylene that is unsubstituted or substituted by one or more L¹⁻¹, C₂₋₄ alkenylene that is unsubstituted or substituted by one or more L¹⁻², C₂₋₄ alkynylene, *-C₁₋₄ alkylene-S-, *-C₁₋₄ alkylene-O-, *-O-C₁₋₄ alkylene-, *-phenylene-O-, *-C₁₋₄ alkylene-C(O)-; the "more" in the C₁₋₄ alkylene that is unsubstituted or substituted by one or more L¹⁻¹, C₂₋₄ alkenylene that is unsubstituted or substituted by one or more L¹⁻² is preferably 1, 2, 3, or 4, more preferably 1 or 2; preferably the L¹ is a bond, C₁₋₂ alkylene that is unsubstituted or substituted by one or more L¹⁻¹, C₂₋₃ alkenylene that is unsubstituted or substituted by one or more L¹⁻², C₂₋₃ alkynylene, *-C₁₋₂ alkylene-S-, *-C₁₋₂ alkylene-O-, *-C₁₋₂ alkylene-C(O)-; more preferably the L¹ is C₁₋₂ alkylene that is unsubstituted or substituted by one or more L¹⁻¹, C₂₋₃ alkenylene that is unsubstituted or substituted by one or more L¹⁻²;
(4) the R² is independently OH, halogen, CN, C₁₋₆ alkyl, C₂₋₆ alkenyl, C₁₋₆ haloalkyl, C₁₋₆ deuteroalkyl, -O-C₁₋₆ alkyl, -O-C₁₋₆ haloalkyl, -O-C₂₋₆ alkenyl, -O-C₃₋₁₀ cycloalkyl, -S-C₁₋₆ alkyl, -C₃₋₁₀ cycloalkyl, 3-10 membered heterocyclyl, 5-10 membered heteroaryl, -C₁₋₆ alkylene-C₃₋₁₀ cycloalkyl, -OC₁₋₆ alkylene-C₃₋₁₀ cycloalkyl, or -C(=O)NR²⁻¹R²⁻², or alternatively, two R² connected to the same carbon atom form an oxo group (=O), wherein the heteroatom in the 3-10 membered heterocyclyl or the 5-10 membered heteroaryl is selected from N, O and S, and the number of the heteroatom is 1, 2, 3, or 4; or alternatively, adjacent R² groups form 5-6 membered heterocyclyl, wherein the heteroatom in the 5-6 membered heterocyclyl is selected from N, O and S, and the number of the heteroatom is 1, 2, 3, or 4; preferably each R² is independently OH, halogen, CN, C₁₋₄ alkyl, C₂₋₄ alkenyl, C₁₋₄ haloalkyl, C₁₋₄ deuteroalkyl, -O-C₁₋₄ alkyl, -O-C₃₋₆ cycloalkyl, -S-C₁₋₄ alkyl, -O-C₂₋₄ alkenyl, -O-C₁₋₄ haloalkyl, -C₃₋₆ cycloalkyl, 3-6 membered heterocyclyl, 5-6 membered heteroaryl, -C₁₋₄ alkylene-C₃₋₆ cycloalkyl, -OC₁₋₄ alkylene-C₃₋₆ cycloalkyl, -C(=O)NHC₁₋₄ alkyl or two R² connected to the same carbon atom form an oxo group (=O);
(5) m is independently 0, 1, 2 or 3, preferably 0,1 or 2; n is independently 0, 1, 2 or 3, preferably 0 or 1; t is independently 0, 1, 2 or 3, preferably 0 or 1;
(6) ring A is C₆₋₁₀ aryl, 5-6 membered heteroaryl or 6 membered heterocyclyl;
(7) ring C is

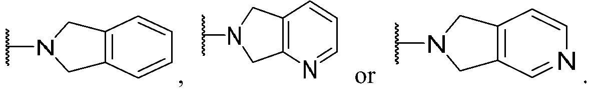

3. The compound represented by formula (0) of claim 1 or 2, wherein, the compound represented by formula (0) satisfies one or more of the following conditions:
(1) the C₁₋₆ alkyl in R¹⁻¹, R¹⁻², R¹⁻³, R¹⁻⁴, R¹⁻⁵, R¹⁻⁶, R¹⁻⁷, R¹⁻⁸, R¹⁻⁹ and R¹⁻¹⁰ is independently methyl, ethyl, isopropyl, n-butyl, isobutyl or tert-butyl, preferably C₁₋₄ alkyl, more preferably methyl or tert-butyl; the -C₁₋₆ alkylene-CN in R¹⁻¹, R¹⁻², R¹⁻³, R¹⁻⁴, R¹⁻⁵, R¹⁻⁶, R¹⁻⁷ , R¹⁻⁸, R¹⁻⁹ and R¹⁻¹⁰ is independently -methylene-CN, -ethylene-CN, -isopropylene-CN, -n-butylene-CN, -isobutylene-CN or -tert-butylene-CN, preferably -C₁₋₄ alkylene-CN, more preferably -methylene-CN; the-C₃₋₁₀ cyclohydrocarbyl in R¹⁻¹, R¹⁻², R¹⁻³ , R¹⁻⁴, R¹⁻⁵, R¹⁻⁶, R¹⁻⁷ , R¹⁻⁸, R¹⁻⁹ and R¹⁻¹⁰ is independently -C₃₋₁₀ cycloalkyl, preferably -C₃₋₆ cycloalkyl, for example cyclopropyl, cyclobutyl, cyclopentyl or cyclohexyl, and further for example cyclopropyl; preferably the R¹⁻¹ and R¹⁻⁴ are each independently H, C₁₋₆ alkyl (for example C₁₋₄ alkyl), C₁₋₆ haloalkyl, -C₁₋₆ alkylene-CN, or -C₃₋₁₀ cyclohydrocarbyl; the R¹⁻², R¹⁻³, R¹⁻⁵, R¹⁻⁶, R¹⁻⁷ and R¹⁻⁸ are each independently H or C₁₋₆ alkyl, preferably H or C₁₋₃ alkyl;
(2) the 5-6 membered monocyclic heterocyclyl in ring B is independently 5-6 membered monocyclic heterocyclyl, the number of the heteroatom is 1, for example azacyclopentane or azacyclohexane, preferably azacyclohexane; the 6-12 membered fused bicyclic heterocyclyl in the ring B is independently 9-12 membered fused bicyclic heterocyclyl, for example benzoazacyclopentane, benzoazaclohexane, benzoazepane, pyridoazacyclohexane, pyrimidoazacyclohexane, thienoazaclohexane, pyrrolidinocyclopentane, preferably benzoazaclohexane, or pyridoazacyclohexane; the 9-12-membered spirocyclic heterocyclyl in ring B is independently 9-12-membered mono-spirocyclic heterocyclyl, optionally wherein the heteroatom may be N or O, preferably 4-membered/6-membered ring system, 5-membered/5-membered ring system, 5-membered/6-membered ring system and 6-membered/6-membered ring system, for example 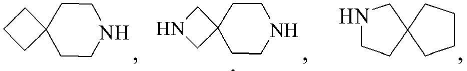 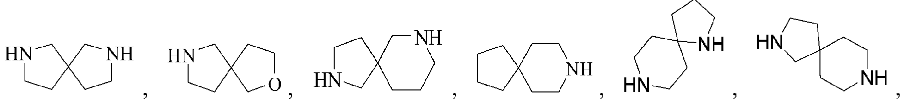 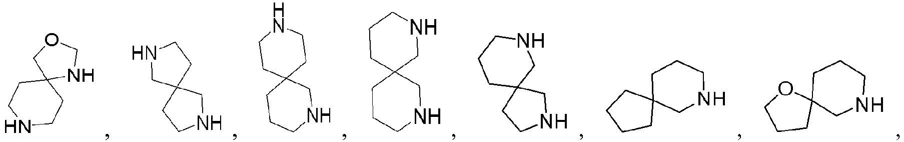 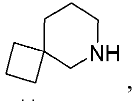 and further for example 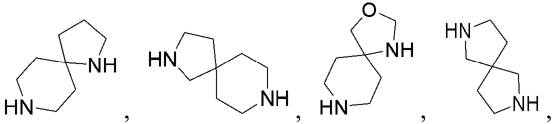 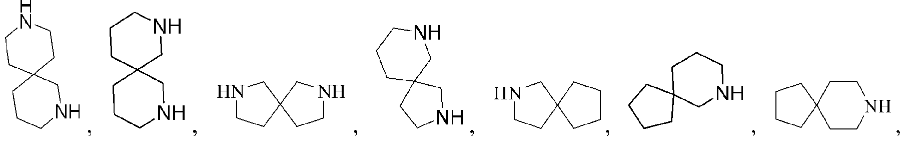 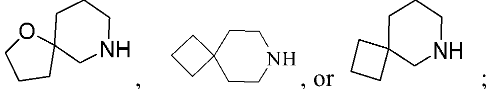
(3) the L¹⁻¹, L¹⁻², L¹⁻³, L¹⁻⁴, L¹⁻⁵, L¹⁻⁶, L¹⁻⁷, L¹⁻⁸, L¹⁻⁹, and L¹⁻¹⁰ are each independently halogen, OH, C₁₋₆ alkyl or C₃₋₆ cycloalkyl; the halogen is each independently F, Cl, Br or I, preferably F, Cl, or Br, more preferably F; the C₁₋₆ alkyl is independently methyl, ethyl, n-propyl, isopropyl, n-butyl, isobutyl or tert-butyl, preferably methyl; the C₃₋₆ cycloalkyl is each independently cyclopropyl, cyclobutyl, cyclopentyl or cyclohexyl, preferably cyclopropyl;
(4) the C₁₋₆ alkylene in C₁₋₆ alkylene that is unsubstituted or substituted by one or more L¹⁻¹, *-C₁₋₆ alkylene-S-, *-C₁₋₆ alkylene-O-, *-O-C₁₋₆ alkylene- or *-C₁₋₆ alkylene-C(O)- is each independently -CH₂-, -CH₂-CH₂-, -C(CH₃)-CH₂-, -CH(CH₃)-CH(CH₃)-, -CH₂CH₂-CH(CH₃)- or -CH₂CH(CH₃)-CH₂-; the C₂₋₆ alkenylene in C₂₋₆ alkenylene that is unsubstituted or substituted by one or more L¹⁻² is -CH=CH-, -CH=CHCH₂-, -CH₂CH=CHCH₂-, -CH=CHCH₂CH₂-, -C(CH₃)=CHCH₂-, -C(CH₃)=CH- or -C(CH₃)=C(CH₃)-; the C₂₋₆ alkynylene in L¹ is -C ≡ C-, -CH₂-C ≡ C-, -CH₂-C ≡ C-CH₂-, -CH₂-CH₂-C ≡ C-, -C ≡ C-C ≡ C-; the C₆₋₁₀ arylene in *-C₆₋₁₀ arylene-O- of L¹ is phenyl, naphthyl, preferably phenyl;
(5) in R², the halogen is each independently F, Cl, Br or I, preferably F, Cl, or Br; the C₁₋₆ alkyl in C₁₋₆ alkyl, -O-C₁₋₆ alkyl, and -S-C₁₋₆ alkyl are each independently methyl, ethyl, n-propyl, isopropyl, n-butyl, isobutyl, sec-butyl or tert-butyl, preferably methyl, ethyl, isopropyl, sec-butyl or tert-butyl; the C₂₋₆ alkenyl in C₂₋₆ alkenyl and -O-C₂₋₆ alkenyl are each independently vinyl, propenyl, isopropenyl, butenyl or isobutenyl, preferably vinyl, propenyl, or isopropenyl; the C₁₋₆ haloalkyl in C₁₋₆ haloalkyl, -O-C₁₋₆ haloalkyl is preferably C₁₋₃ haloalkyl, for example -CH₂F, -CHF₂, -CF₃, -CH₂Cl, -CHCl₂, -CCl₃, -CH₂Cl, -CH₂CH₂F, -CHClCH₃ or -CH(CH₃)CH₂F, preferably -CF₃; the C₁₋₆ deuteroalkyl in C₁₋₆ deuteroalkyl preferably C₁₋₃ deuteroalkyl, for example -CD₃, -CHD₂, -CH₂D ; preferably -CD₃; the C₃₋₁₀ cyclohydrocarbyl in -O-C₃₋₁₀ cyclohydrocarbyl, -S-C₃₋₁₀ cyclohydrocarbyl, -C₃₋₁₀ cyclohydrocarbyl, -C₁₋₆ alkylene-C₃₋₁₀ cyclohydrocarbyl, -OC₁₋₆ alkylene-C₃₋₁₀ cyclohydrocarbyl is each independently -C₃₋₈ cycloalkyl, -C₃₋₈ cycloalkenyl, preferably -C₃₋₆ cycloalkyl, -C₃₋₆ cycloalkenyl, C₃₋₆ cycloalkyl for example cyclopropyl, cyclobutyl, cyclopentyl or cyclohexyl, and for further example cyclopropyl; C₃₋₆ cycloalkenyl for example cyclopropenyl, cyclobutenyl, cyclopentenyl or cyclohexenyl; the 3-10 membered heterocyclyl is 3-6 membered heterocyclyl, the 3-6 membered heterocyclyl is oxacyclopropyl, azacyclopropyl, azetidinyl, oxetanyl, tetrahydrofuranyl, tetrahydrothienyl, dioxolyl, pyrrolidinyl, pyrrolidinonyl, oxazolidinyl, thiazolidinyl, pyrazolidinyl, imidazolidinyl, pyrrolinyl, tetrahydropyranyl or piperidinyl, preferably oxacyclopropyl, azacyclopropyl, azetidinyl or oxetanyl; the 5-10 membered heteroaryl is independently pyridinyl, pyrimidinyl, pyrazinyl, pyridazinyl, thiazolyl, thienyl, oxazolyl, furanyl, pyrrolyl, pyrazolyl, triazolyl, tetrazolyl, isoxazolyl, isothiazolyl, imidazolyl, triazinyl, oxadiazolyl, thiadiazolyl, benzothiazolyl, benzoisothiazolyl, imidazopyridinyl, quinolinyl, indolyl, pyrrolopyridazinyl or benzofuranyl, preferably oxazolyl, furanyl or thiazolyl; the adjacent R² groups form 5-6 membered heterocyclyl, wherein the heteroatom in the 5-6 membered heterocyclyl is selected from N or O, and the number of the heteroatom is 1 or 2, preferably azocyclopentane, azocyclohexane, azocyclobutane, oxocyclopentane, oxocyclohexane or oxocyclobutane, more preferably oxocyclopentane or oxocyclohexane; the R²⁻¹ and R²⁻² are each independently H or C₁₋₃ alkyl, preferably H or methyl;
(6) the C₆₋₁₀ aryl in ring A is phenyl or naphthyl, preferably phenyl; the 5-6 membered heteroaryl in ring A is 5-6 membered nitrogen-containing heteroaryl, for example pyridinyl, pyridazinyl, pyrimidinyl, pyrazinyl, thiazolyl, pyrrolyl, furanyl, thienyl or imidazolyl, preferably pyridinyl, pyridazinyl, pyrimidinyl or pyrazinyl; the 5-12 membered heterocyclyl in ring A is tetrahydrofuranyl, tetrahydrothienyl, dioxolyl, pyrrolidinyl, oxazolidinyl, thiazolidinyl, pyrazolidinyl, imidazolidinyl, pyrrolinyl, pyranyl, tetrahydropyranyl,

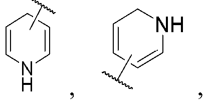

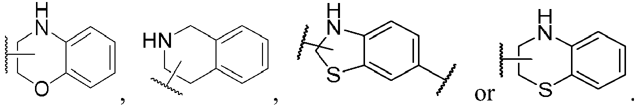

4. The compound represented by formula (0) according to any one of claims 1-3, wherein, the compound represented by formula (0) satisfies one or more of the following conditions:
(1) the R¹ is 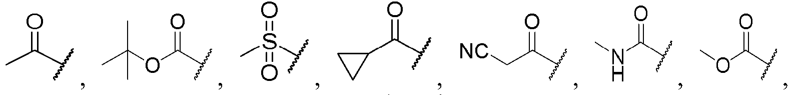 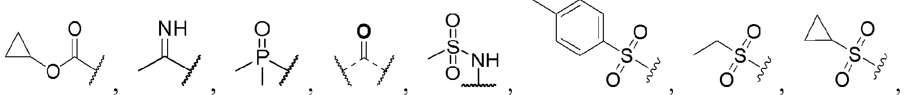 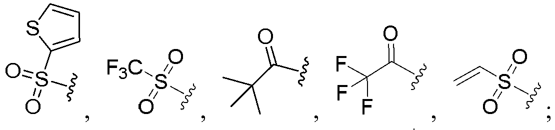
(2) the ring B is 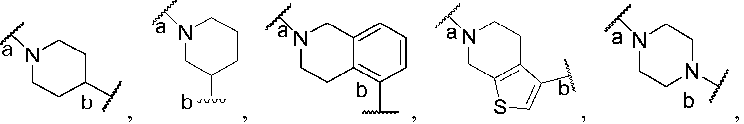 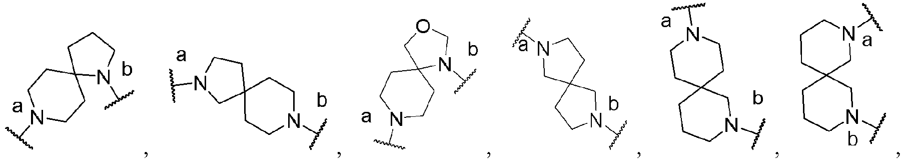 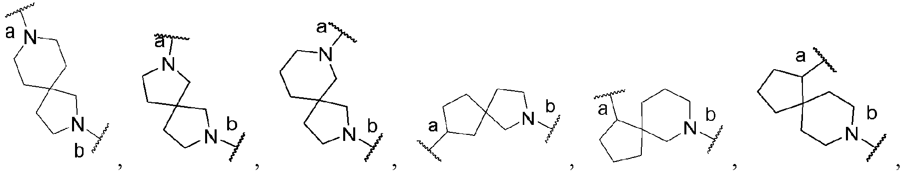 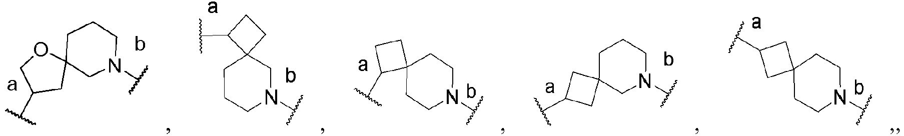 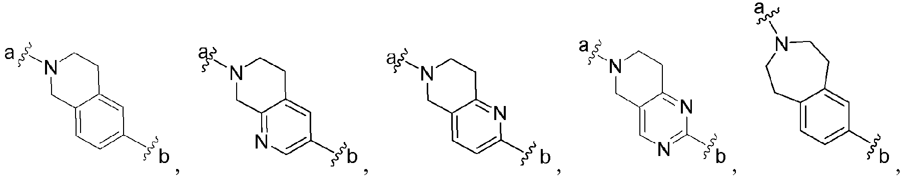 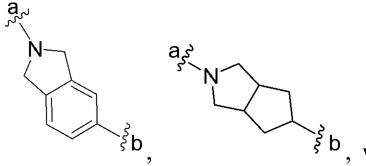 wherein the mark "a" indicates the bond connected to R¹, and the mark "b" indicates the bond connected to L¹; preferably, the ring B is 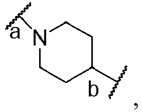 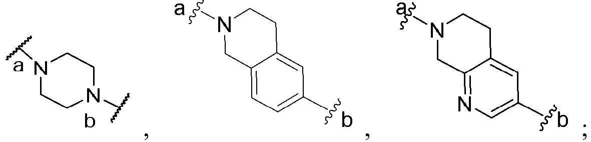 more preferably, the ring B is 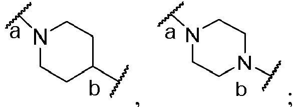
(3) the L¹ is *-CH₂-O-, -CH₂-CH₂-, *-CH₂-CH₂-O-, *-O-CH₂-, *-CH₂-S-, *-CH₂-CH₂-S-, -CH=CH-, -CH₂-, a bond, -NH-, -O-, *-CH=CF-, *-CF=CH-, *-CH=C(CH₃)-, *-C(CH₃)=CH-, -CF=CF-, -C(CH₃)=C(CH₃)-, *-CH₂-CHF-, *-CHF-CH₂-, *-CH₂-CH(CH₃)-, *-C(CH₃)-CH₂-, -CHF-CHF-, -CH(CH₃)-CH(CH₃)-, *-CH₂-CF₂-, *-CF₂-CH₂-, *-CH₂-CH(cyclopropane)-, *-CH(cyclopropane)-CH₂-, *-CH=C(cyclopropane)-, *-C(cyclopropane)=CH-, *-CH₂-CH(OH)-, -C ≡ C-, 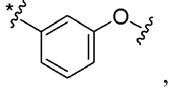 or *-CH₂-C(O)- ; preferably, the L¹ is *-CH₂-O-, -CH₂-CH₂-, *-CH₂-CHF-, *-CH=CF-, *-CH₂-CH(OH)-, *-CH₂-S-, *-CH₂-CH₂-S-, -CH=CH-, a bond, -C≡ C-, *-CH₂-C(O)-; more preferably, the L¹ is *-CH₂-O-, -CH₂-CH₂-, -CH=CH-; more preferably, the L¹ is -CH₂-CH₂-, -CH=CH-; wherein the mark "*" indicates the bond connected to ring B;
(4) the ring A is: 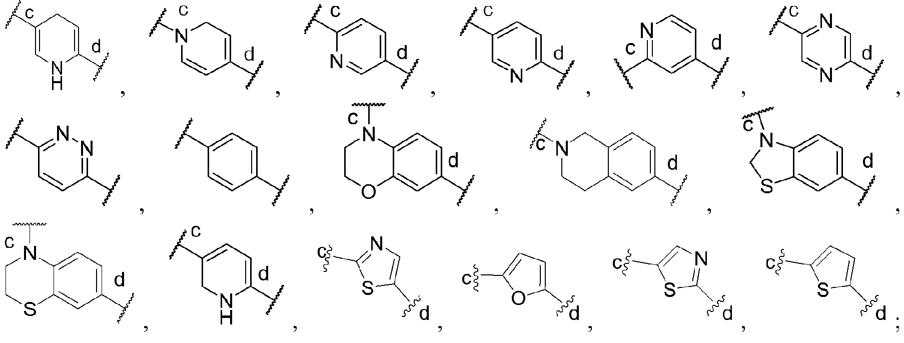 preferably the ring A is 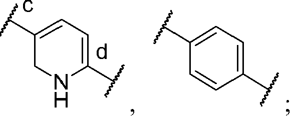 wherein the mark "c" indicates the bond connected to L¹, and the mark "d" indicates the bond connected to 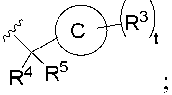
(5) the R² is independently 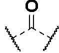 , OH, F, Cl, Br, CN, -CH₂F, -CHF₂, -CF₃, -CD₃, -S-CH₃, -O-CH-(CH₃)₂, -O-CH(CH₃)-CH₂CH₃, methyl, ethyl, n-propyl, isopropyl, vinyl, propenyl, isopropenyl, methoxy, ethoxy, cyclopropyl, cyclopropylmethyl-, 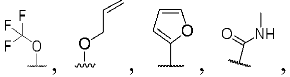 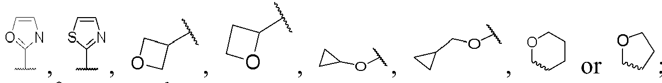 , wherein, the wavy lines " 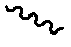 " in 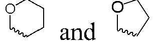 indicate the position of fusion with ring A.

5. The compound represented by formula (0) of claim 4, wherein, the compound represented by formula (0) satisfies one or more of the following conditions: the 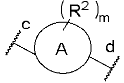 moiety is: 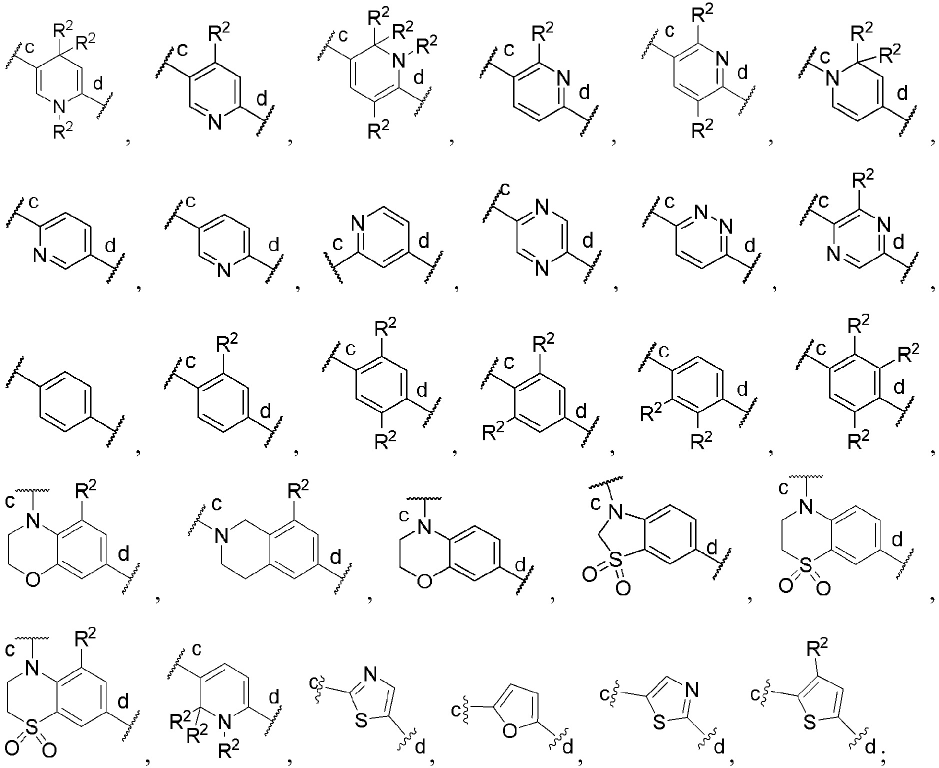 preferably, the 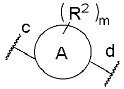 moiety is: 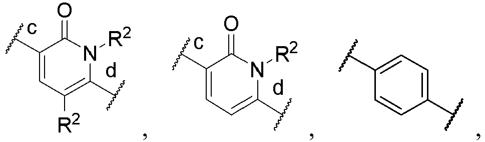 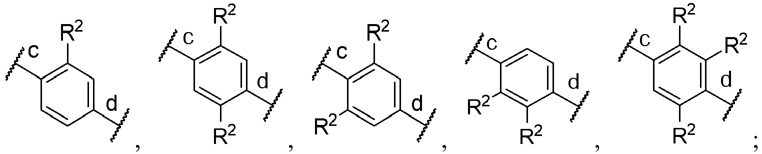 preferably, the 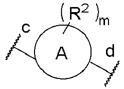 moiety is: 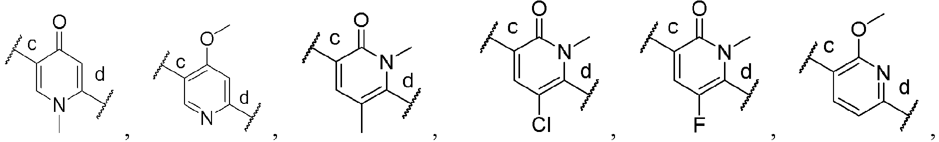 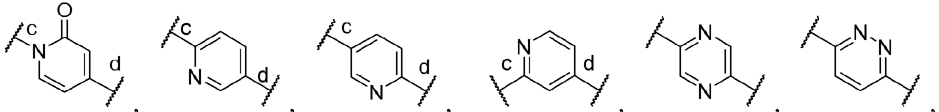 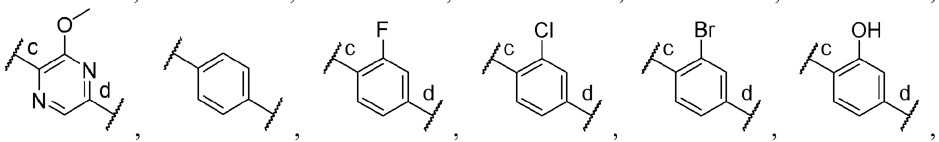 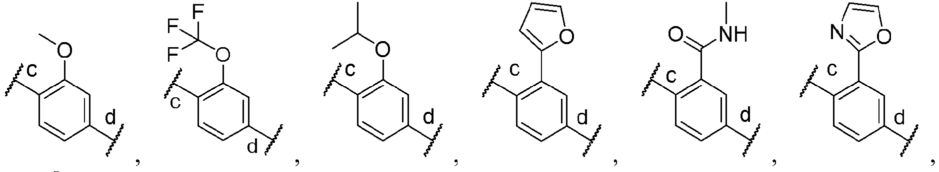 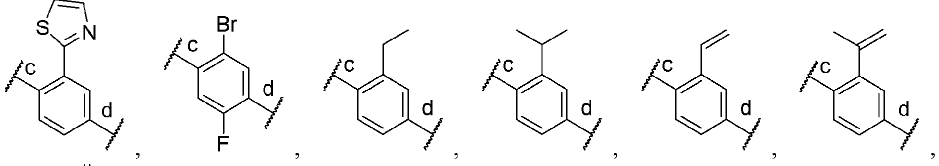 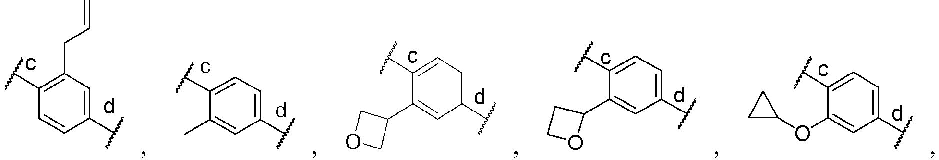 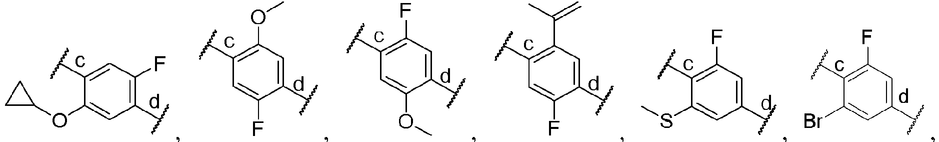 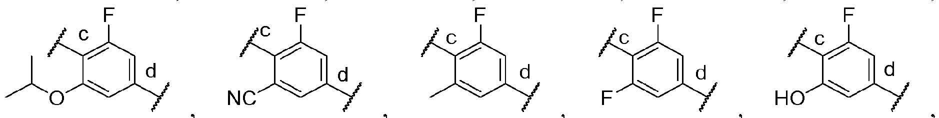 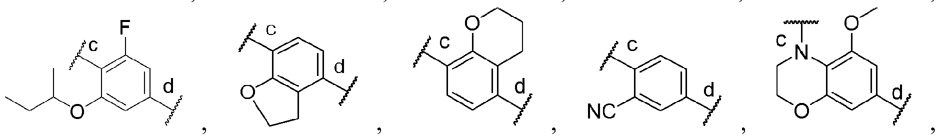 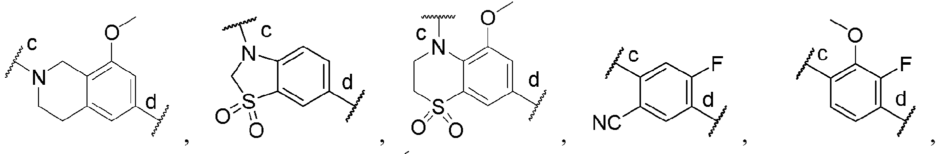 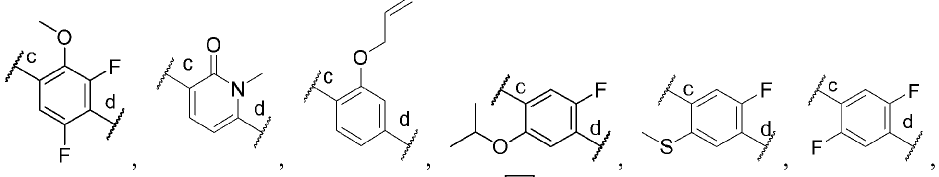 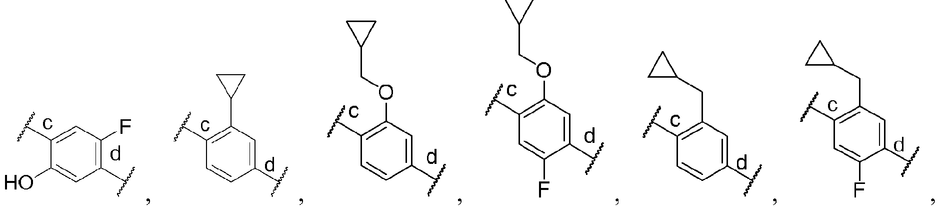 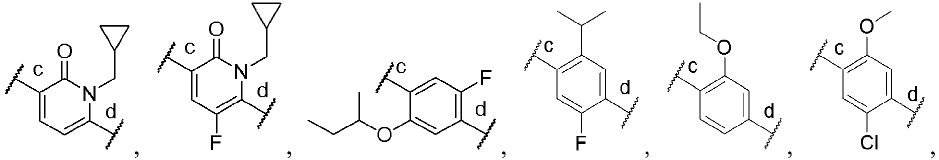 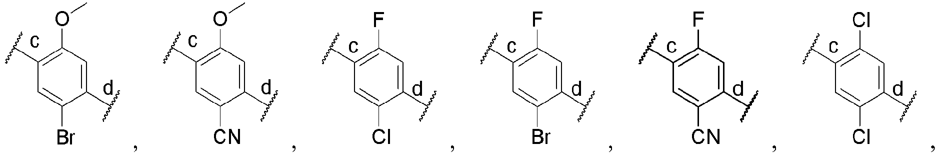 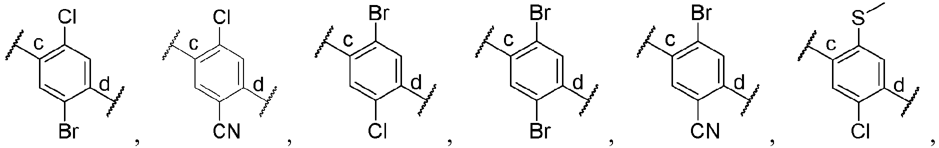 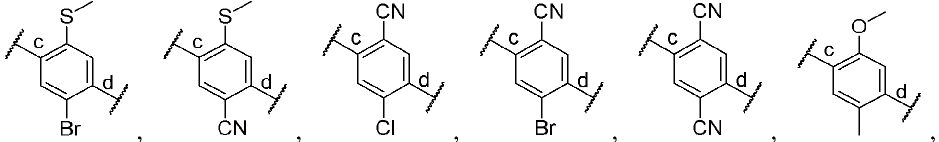 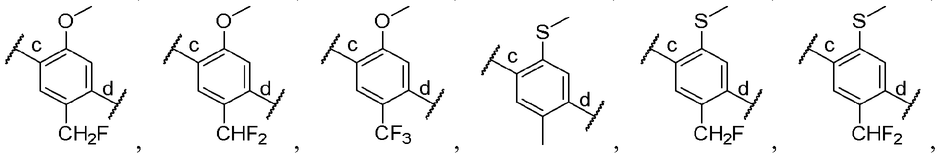 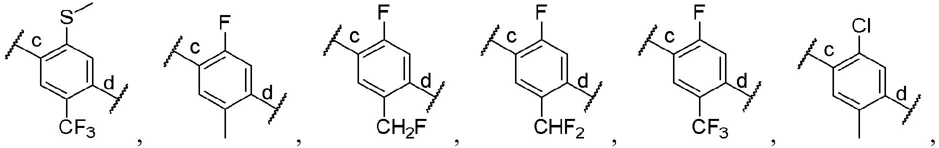 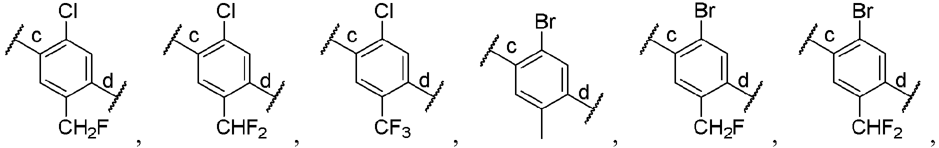 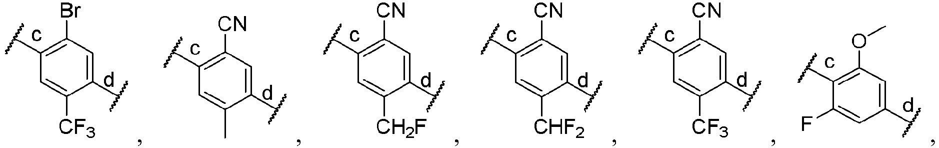 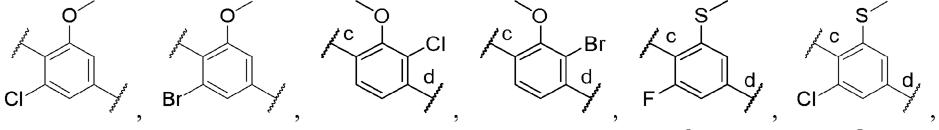 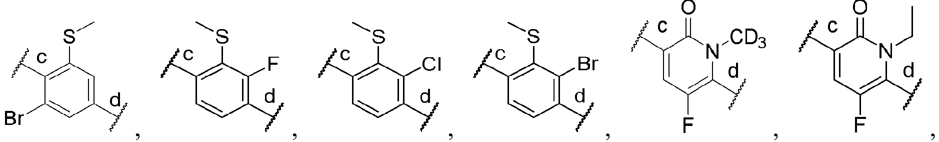 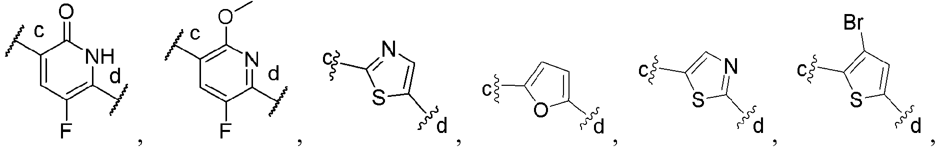 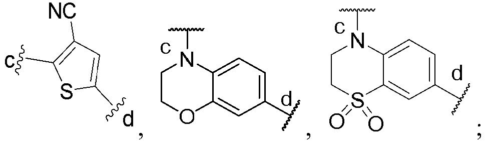 preferably , the 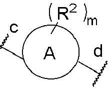 moiety is : 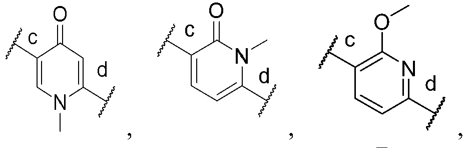 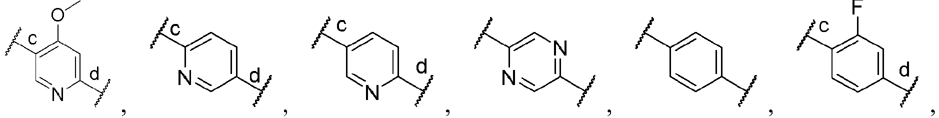 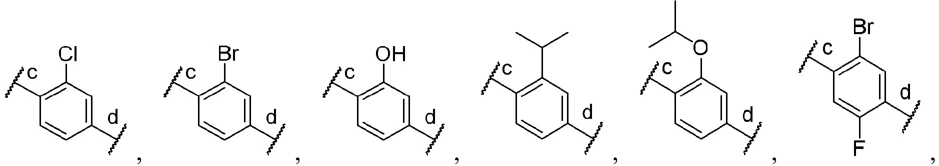 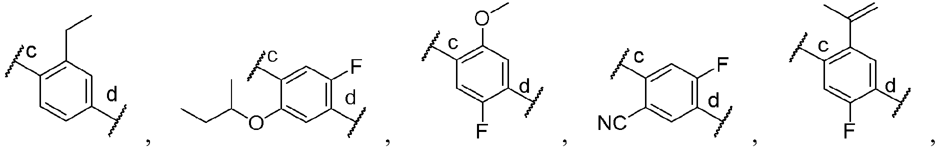 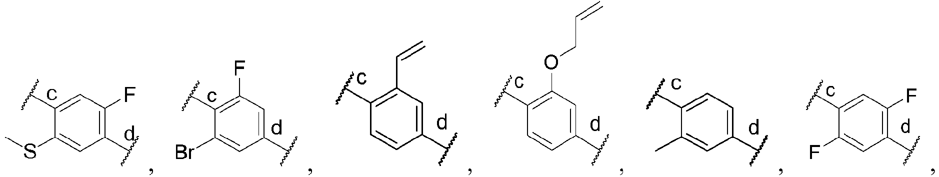 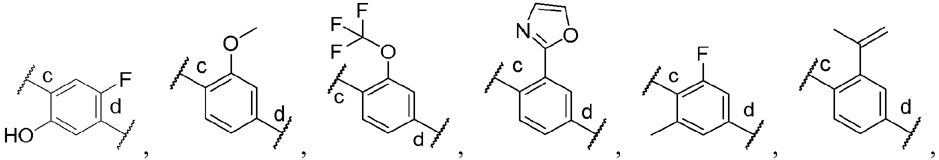 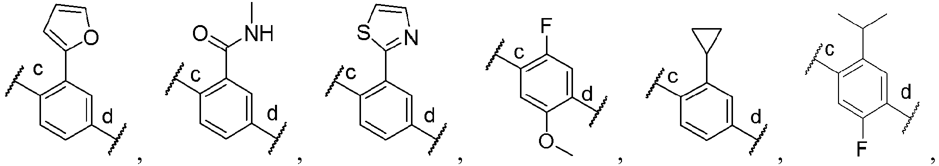 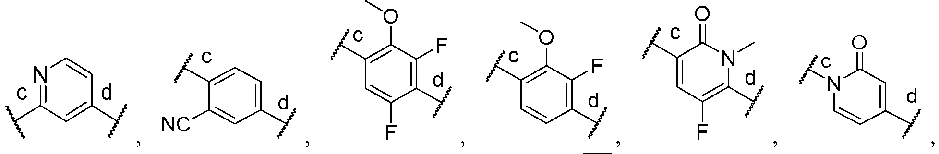 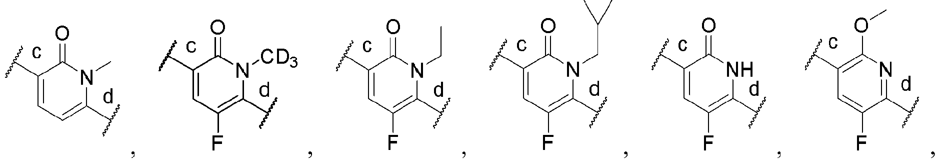 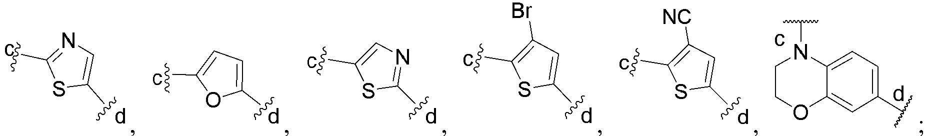 preferably the 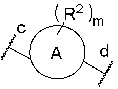 moiety is :

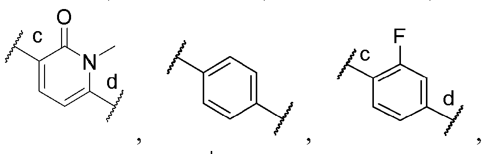

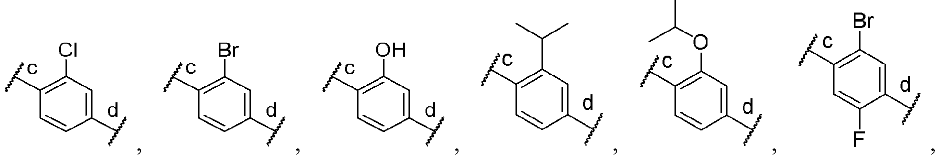

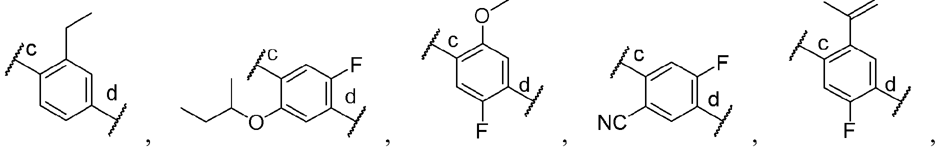

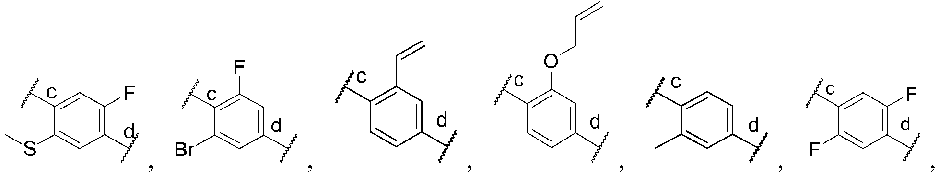

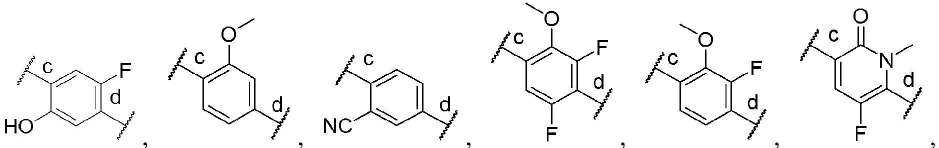

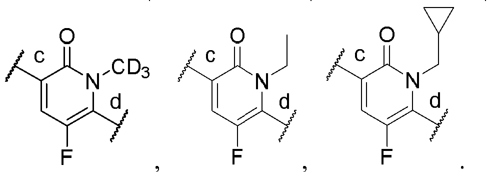

6. The compound represented by formula (0) according to any one of claims 1-5, wherein, the compound has a structure represented by formula (I):

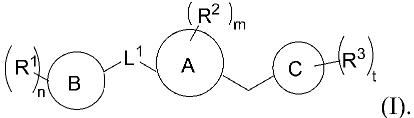

7. The compound represented by formula (0) according to any one of claims 1-5, wherein, the compound has a structure represented by formula (I-20'), (I-21') or (I-22'): 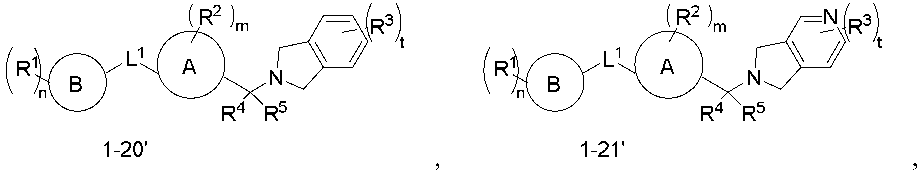 or

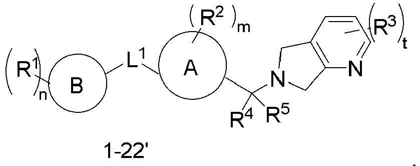

8. The compound represented by formula (I-20'), (I-21') or (I-22') of claim 7, wherein, the compound has a structure represented by formula (I-20), (I-21) or (I-22): 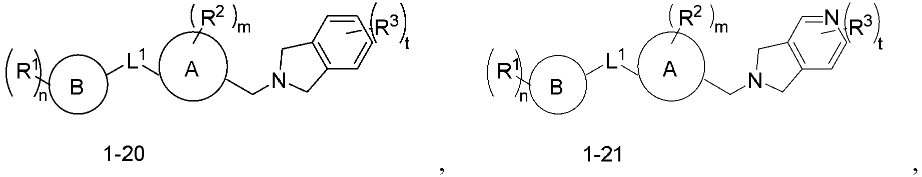 or

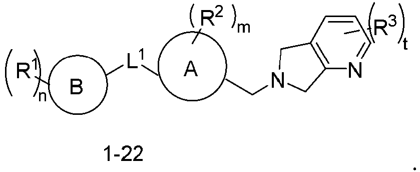

9. The compound represented by formula (I-20') of claim 7, wherein, the compound has a structure represented by formula (I-A), (I-B), (I-C), (I-D), (I-E), (I-F), (I-G), (I-H), (I-I), (I-J), (I-K), (I-L), (I-M), (I-N), (I-O), (I-P), (I-Q) or (I-R): 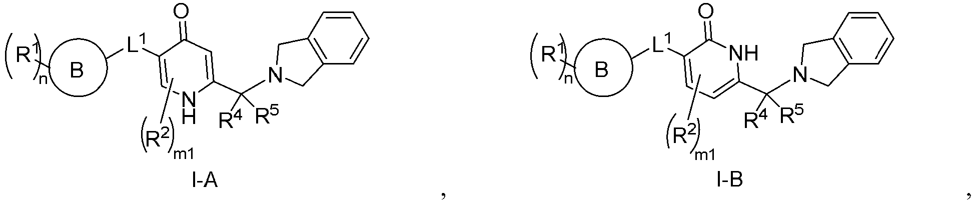 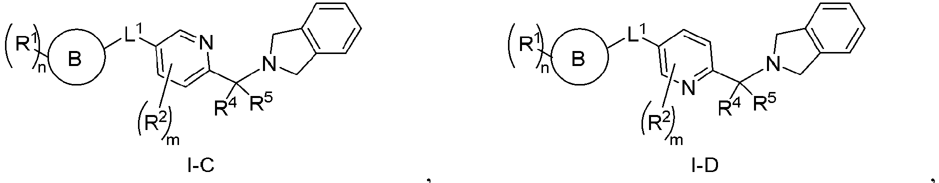 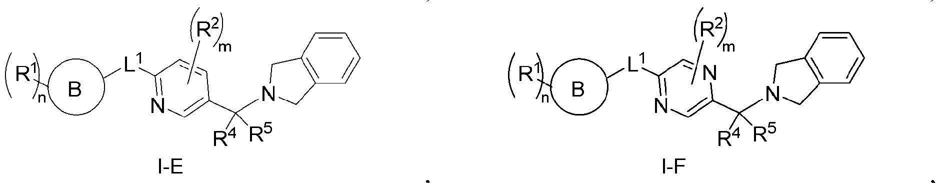 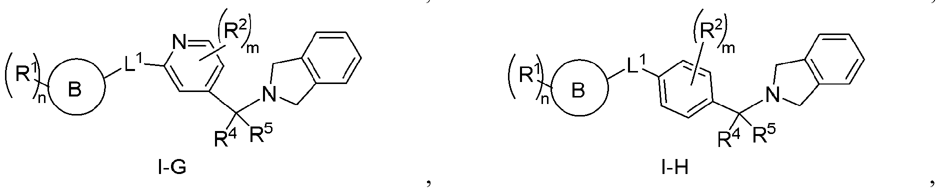 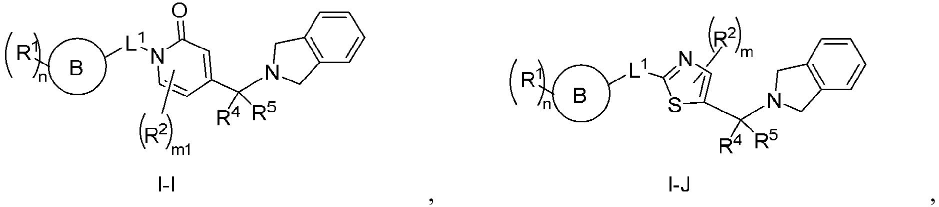 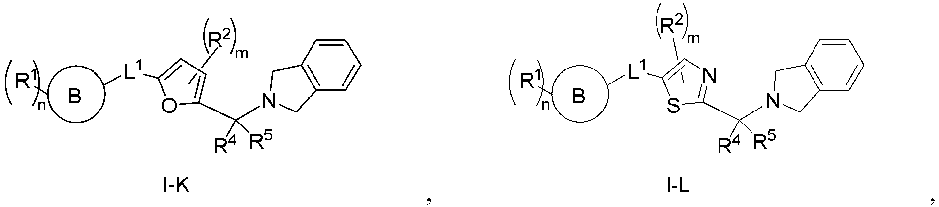 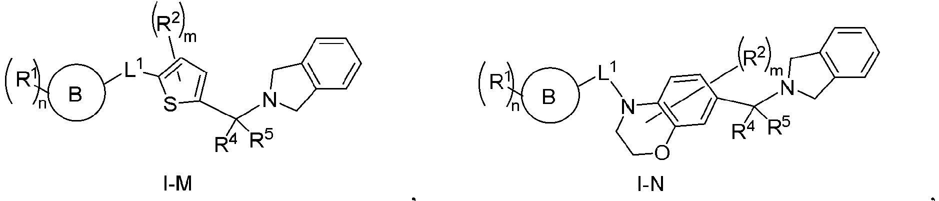 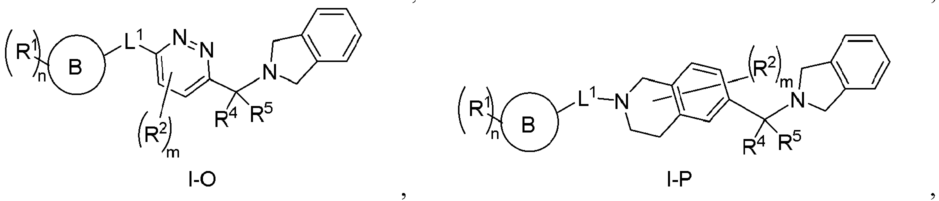 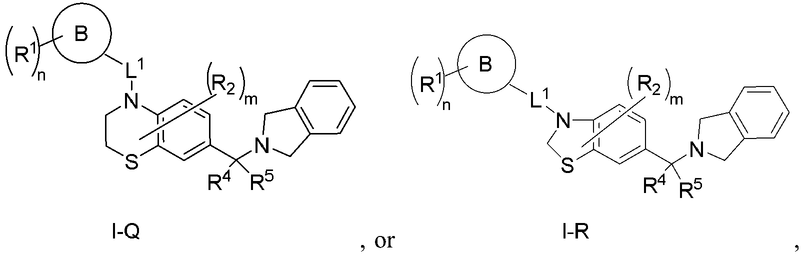 wherein m1 is 0, 1 or 2.

10. The compound represented by formula (0) according to any one of claims 1-5, wherein, the compound has a structure represented by formula (II-A), (II-B), (II-C) or (II-D):

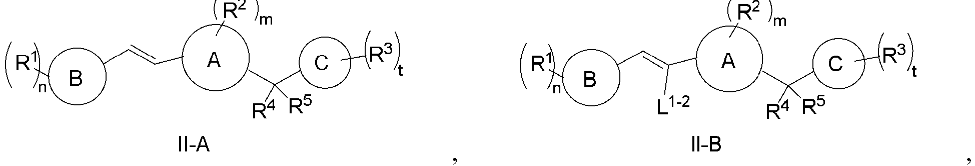

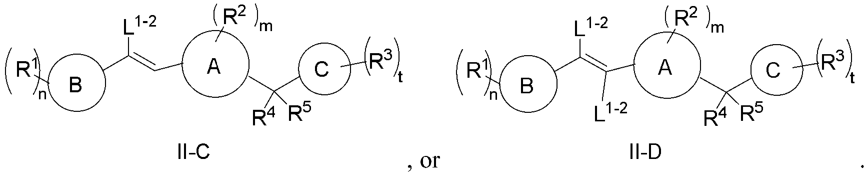

11. The compound according to claim 10, wherein, the compound has a structure represented by formula (II-A-1), (II-B-1), (II-C-1) or (II-D-1):

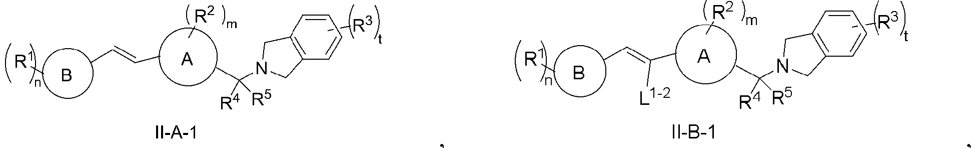

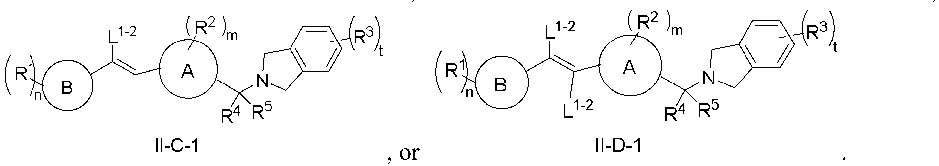

12. The compound according to claim 10, wherein, the compound has a structure represented by formula (II-A-2), (II-B-2), (II-C-2), (II-D-2), (II-A-3), (II-B-3), (II-C-3) or (II-D-3): 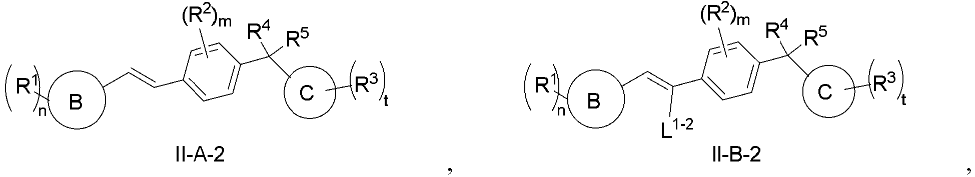 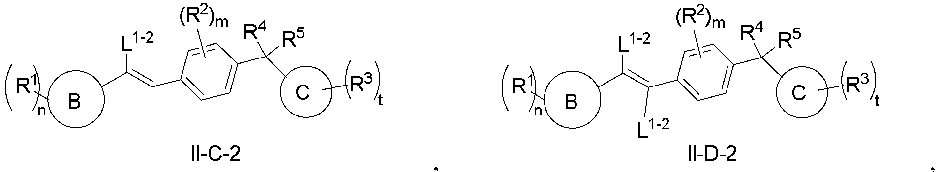 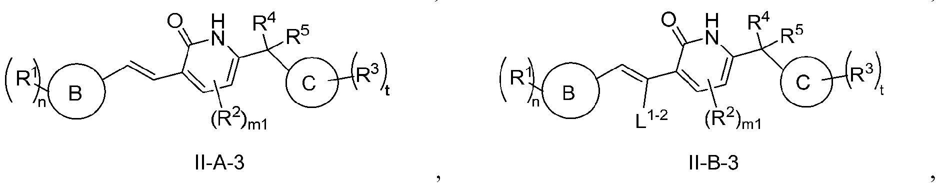 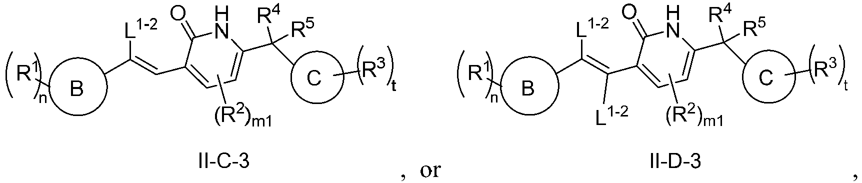 wherein m1 is 0, 1 or 2.

13. The compound according to claim 12, wherein, the compound has a structure represented by formula (II'-A-2), (II'-B-2), (II'-C-2), (II'-D-2), (II'-A-3), (II'-B-3), (II'-C-3) or (II'-D-3): 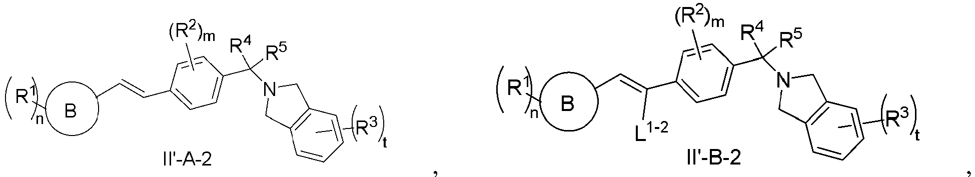 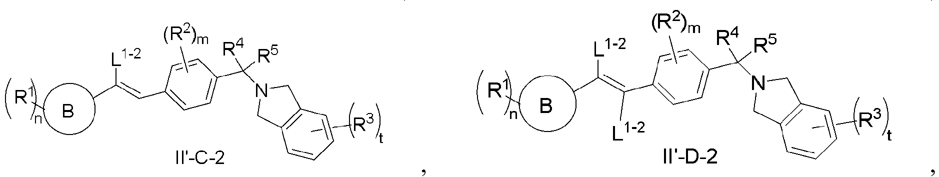 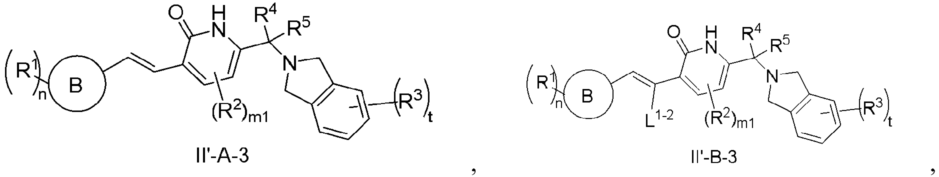 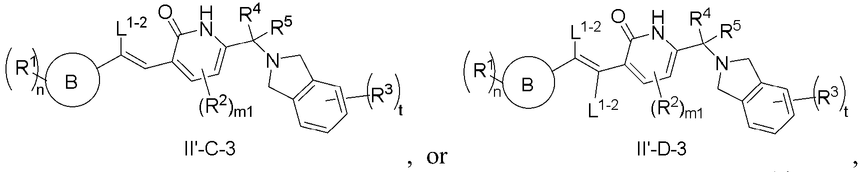 wherein m1 is 0, 1 or 2, preferably m1 is 2; m is 0, 1, 2 or 3, preferably m is 1 or 2; L¹⁻² is halogen, C₁₋₆ alkyl, C₃₋₆ cycloalkyl, preferably L¹⁻² is F, methyl, cyclopropyl; more preferably L¹⁻² is F.

14. The compound according to any one of claims 1-9, wherein, the compound has a structure represented by formula (I-H-1): 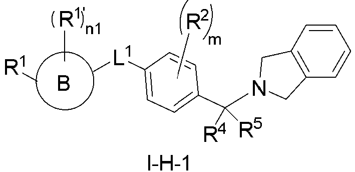 wherein:
R¹ and R¹' are each independently deuterium, halogen, cyano, hydroxy, C₁₋₆ alkoxy, R¹⁻¹, -C(=O)-R¹⁻¹, -C(=O)-OR¹⁻⁴, -NR¹⁻⁷R¹⁻⁸, -C(=O)-NR¹⁻⁷R¹⁻⁸, -C(=NH)-(R¹⁻⁹), -S(O)₂-R¹⁻⁹, -S(O)₂-NR¹⁻⁵R¹⁻⁶, -NR¹⁻⁵S(O)₂-R¹⁻¹⁰, -R¹⁻¹⁰-OH, or -P(=O)-R¹⁻²R¹⁻³;
R¹⁻¹, R¹⁻², R¹⁻³, R¹⁻⁴, R¹⁻⁵, R¹⁻⁶, R¹⁻⁷ and R¹⁻⁸ are each independently H, C₁₋₆ alkyl, C₁₋₆ haloalkyl, C₁₋₆ deuteroalkyl, -C₁₋₆ alkylene-OH, -C₁₋₆ alkylene-CN, -C₃₋₁₀ cycloalkyl, or 3-10 membered heterocyclyl, wherein the heteroatom in the 3-10 membered heterocyclyl is selected from N, O and S, and the number of the heteroatom is 1, 2, 3, or 4; preferably, R¹⁻¹, R¹⁻², R¹⁻³, R¹⁻⁴, R¹⁻⁵, R¹⁻⁶, R¹⁻⁷ and R¹⁻⁸ are each independently H, C₁₋₄ alkyl, C₁₋₄ haloalkyl, C₁₋₄ deuteroalkyl, -C₁₋₄ alkylene-OH, -C₁₋₄ alkylene-CN, -C₃₋₆ cycloalkyl, or 3-6 membered heterocyclyl;
R¹⁻⁹ and R¹⁻¹⁰ are each independently C₁₋₆ alkyl, C₁₋₆ haloalkyl, -C₁₋₆ alkylene-CN, C₂₋₆ alkenyl, C₂₋₆ haloalkenyl, C₃₋₁₀ cycloalkyl, C₆₋₁₀ aryl, 5-10 membered heteroaryl, C₁₋₆ alkyl-C₆₋₁₀ arylene-; preferably, R¹⁻⁹ and R¹⁻¹⁰ are each independently C₁₋₄ alkyl, C₁₋₄ haloalkyl, -C₁₋₄ alkylene-CN, C₂₋₄ alkenyl, C₂₋₄ haloalkenyl, C₃₋₆ cycloalkyl, C₆₋₁₀ aryl, 5-10 membered heteroaryl, C₁₋₄ alkyl-C₆₋₁₀ arylene-;
preferably R¹ is -C(=O)-R¹⁻¹, -C(=O)-OR¹⁻⁴, -C(=O)-NR¹⁻⁷R¹⁻⁸, -C(=NH)-(R¹⁻⁹), -S(O)₂-R¹⁻⁹, -S(O)₂-NR¹⁻⁵R¹⁻⁶, -NR¹⁻⁵S(O)₂-R¹⁻¹⁰ or -P(=O)-R¹⁻²R¹⁻³; R¹' is deuterium, halogen, cyano, hydroxy, C₁₋₆ alkoxy, R¹⁻¹, -NR¹⁻⁷R¹⁻⁸, -R¹⁻¹⁰-OH;
preferably R¹ is -C(=O)-R¹⁻¹, -C(=O)-OR¹⁻⁴, -C(=O)-NR¹⁻⁷R¹⁻⁸, -S(O)₂-R¹⁻⁹; R¹' is deuterium, halogen, cyano, hydroxy, C₁₋₆ alkoxy, R¹⁻¹, -NR¹⁻⁷R¹⁻⁸, -R¹⁻¹⁰-OH;
preferably R¹ is -C(=O)-R¹⁻¹, -C(=O)-OR¹⁻⁴, -S(O)₂-R¹⁻⁹ ; R¹' is hydrogen, deuterium, halogen, cyano, hydroxy, C₁₋₆ alkoxy, -NH₂, -NH(C₁₋₆ alkyl), -N(C₁₋₆ alkyl)₂, C₁₋₆ alkyl, C₁₋₆ haloalkyl, C₁₋₆ deuteroalkyl, -C₁₋₆ alkylene-OH, -C₁₋₆ alkylene-CN, -C₃₋₁₀ cycloalkyl, or 3-10 membered heterocyclyl;
preferably R¹ is -C(=O)-C₁₋₆ alkyl, -C(=O)-C₁₋₆ haloalkyl, -C(=O)-C₃₋₁₀ cycloalkyl, -C(= O)-C₁₋₆ alkylene-CN, -C( = O)-OC₁₋₆ alkyl, -C( = O)-OC₃₋₁₀ cycloalkyl, -S(O)₂-C₁₋₆ alkyl, -S(O)₂-C₆₋₁₀ arylene-C₁₋₆ alkyl, -S(O)₂-C₃₋₁₀ cycloalkyl, -S(O)₂-5-10 membered heteroaryl, -S(O)₂-C₁₋₆ haloalkyl, -S(O)₂-C₂₆ alkenyl, -C(=O)-NH(C₁₋₆ alkyl); R¹' is hydrogen, deuterium, halogen, cyano, hydroxy, C₁₋₆ alkoxy, -NH₂, -NH(C₁₋₆ alkyl), -N(C₁₋₆ alkyl)₂, C₁₋₆ alkyl, C₁₋₆ haloalkyl, C₁₋₆ deuteroalkyl, -C₁₋₆ alkylene-OH, -C₁₋₆ alkylene-CN, -C₃₋₁₀ cycloalkyl, or 3-10 membered heterocyclyl;
preferably R¹ is -C(=O)-C₁₋₄ alkyl, -C(=O)-C₁₋₄ haloalkyl, -C(=O)-C₃₋₆ cycloalkyl, -C(= O)-C₁₋₄ alkylene-CN, -C( = O)-OC₁₋₄ alkyl, -C( = O)-OC₃₋₆ cycloalkyl, -S(O)₂-C₁₋₄ alkyl, -S(O)₂-phenylene-C₁₋₄ alkyl, -S(O)₂-C₃₋₆ cycloalkyl, -S(O)₂-5-6 membered heteroaryl, -S(O)₂-C₁₋₄ haloalkyl, -S(O)₂-C₂₋₄ alkenyl, -C(=O)-NH(C₁₋₄ alkyl); R¹' is hydrogen, deuterium, halogen, cyano, hydroxy, C₁₋₄ alkoxy, -NH₂, -NH(C₁₋₄ alkyl), -N(C₁₋₄ alkyl)₂, C₁₋₄ alkyl, C₁₋₄ haloalkyl, C₁₋₄ deuteroalkyl, -C₁₋₄ alkylene-OH, -C₁₋₄ alkylene-CN, -C₃₋₆ cycloalkyl, or 3-6 membered heterocyclyl;
preferably R¹ is -S(O)₂-C₁₋₄ alkyl, -S(O)₂-C₂₋₄ alkenyl, -S(O)₂-phenylene-C₁₋₄ alkyl, -S(O)₂-C₃₋₆ cycloalkyl, -S(O)₂-5-6 membered heteroaryl, -C(=O)-OC₁₋₄ alkyl, -C(=O)-C₁₋₄ alkyl, -C(=O)-C₁₋₄ alkylene-CN; R¹' is hydrogen, deuterium, halogen, cyano, hydroxy, C₁₋₄ alkyl;
n1 is each independently 0, 1, 2, or 3; preferably, n1 is each independently 0, 1, or 2; more preferably, n1 is 0;
ring B is 5-12 membered heterocyclyl, wherein the heteroatom in the 5-12 membered heterocyclyl is selected from N, O and S, and the number of the heteroatom is 1, 2, 3, or 4; preferably ring B is 5-6 membered monocyclic heterocyclyl, 9-12 membered fused bicyclic heterocyclyl or 9-12 membered spirocyclic heterocyclyl; preferably ring B is 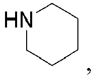 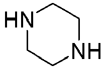 , preferably 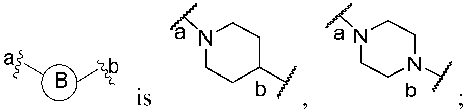
L¹ is a bond, O, -NH-, C₁₋₆ alkylene that is unsubstituted or substituted by one or more L¹⁻¹, C₂₋₆ alkenylene that is unsubstituted or substituted by one or more L¹⁻², C₂₋₆ alkynylene that is unsubstituted or substituted by one or more L¹⁻³, *-C₁₋₆ alkylene-S- that is unsubstituted or substituted by one or more L¹⁻⁴, *-C₁₋₆ alkylene-O- that is unsubstituted or substituted by one or more L¹⁻⁶, *-O-C₁₋₆ alkylene- that is unsubstituted or substituted by one or more L¹⁻⁷, *-C₆₋₁₀ arylene-O- that is unsubstituted or substituted by one or more L¹⁻⁸, *-C₁₋₆ alkylene-C(O)- that is unsubstituted or substituted by one or more L¹⁻¹⁰;
preferably L¹ is a bond, O, -NH-, C₁₋₄ alkylene that is unsubstituted or substituted by one or more L¹⁻¹, C₂₋₄ alkenylene that is unsubstituted or substituted by one or more L¹⁻², C₂₋₄ alkynylene that is unsubstituted or substituted by one or more L¹⁻³, *-C₁₋₄ alkylene-S- that is unsubstituted or substituted by one or more L¹⁻⁴, *-C₁₋₄ alkylene-O- that is unsubstituted or substituted by one or more L¹⁻⁶, *-O-C₁₋₄ alkylene- that is unsubstituted or substituted by one or more L¹⁻⁷, *-phenylene-O- that is unsubstituted or substituted by one or more L¹⁻⁸, *-C₁₋₂ alkylene-C(O)- that is unsubstituted or substituted by one or more L¹⁻¹⁰;
each L¹⁻¹, L¹⁻², L¹⁻³, L¹⁻⁴, L¹⁻⁶, L¹⁻⁷, L¹⁻⁸, L¹⁻¹⁰ is independently halogen, OH, C₁₋₆ alkyl, C₃₋₆ cycloalkyl or 3-6 membered heterocyclyl, wherein the heteroatom in the 3-6 membered heterocyclyl is selected from N, O and S, and the number of the heteroatom is 1, 2, 3, or 4; wherein the C₁₋₆ alkyl, the C₃₋₆ cycloalkyl or the 3-6 membered heterocyclyl is optionally substituted by deuterium, halogen, OH, CN or -NH₂;
preferably L¹ is -CH₂-CH₂-, *-CH₂-O-; -CH=CH-;
more preferably L¹ is -CH₂-CH₂-, -CH=CH-;
each R² is independently deuterium, OH, NH₂, halogen, CN, C₁₋₆ alkyl, C₂₋₆ alkenyl, C₁₋₆ haloalkyl, C₁₋₆ deuteroalkyl, C₂₋₆ haloalkenyl, -O-C₁₋₆ alkyl, -C₁₋₆ alkylene-O-C₁₋₆ alkyl, -O-C₁₋₆ haloalkyl, -O-C₂₋₆ alkenyl, -O-C₃₋₁₀ cycloalkyl, -S-C₁₋₆ alkyl, -S-C₃₋₁₀ cycloalkyl, -C₃₋₁₀ cycloalkyl, 3-10 membered heterocyclyl, C₆₋₁₀ aryl, 5-10 membered heteroaryl, -C₁₋₆ alkylene-C₃₋₁₀ cycloalkyl, -C(=O)NH(C₁₋₆ alkyl), or optionally, two adjacent R² form 5-6 membered heterocyclyl, wherein the heteroatom in the 5-6 membered heterocyclyl is selected from N, O and S, and the number of the heteroatom is 1, 2, 3, or 4;
preferably R² is independently deuterium, OH, NH₂, halogen, CN, C₁₋₄ alkyl, C₂₋₄ alkenyl, C₁₋₄ haloalkyl, C₁₋₄ deuteroalkyl, C₂₋₄ haloalkenyl, -O-C₁₋₄ alkyl, -C₁₋₄ alkylene-O-C₁₋₄ alkyl, -O-C₁₋₄ haloalkyl, -O-C₂₋₄ alkenyl, -O-C₃₋₆ cycloalkyl, -S-C₁₋₄ alkyl, -S-C₃₋₆ cycloalkyl, -C₃₋₆ cycloalkyl, 3-6 membered heterocyclyl, C₆₋₁₀ aryl, 5-6 membered heteroaryl, -C₁₋₄ alkylene-C₃₋₆ cycloalkyl, -C(=O)NH(C₁₋₄ alkyl);
m is 0, 1, 2, 3 or 4; preferably, m is 0, 1, 2 or 3;
R⁴ and R⁵ are each independently H, deuterium, halogen, C₁₋₆ alkyl; preferably, R⁴ and R⁵ are both H or deuterium, or one of R⁴ and R⁵ is H, and the other is deuterium, halogen, or C₁₋₄ alkyl.

15. The compound according to claim 14, wherein, the compound has a structure represented by formula (I-H-1A), (I-H-1B), (I-H-1C), (I-H-1D):

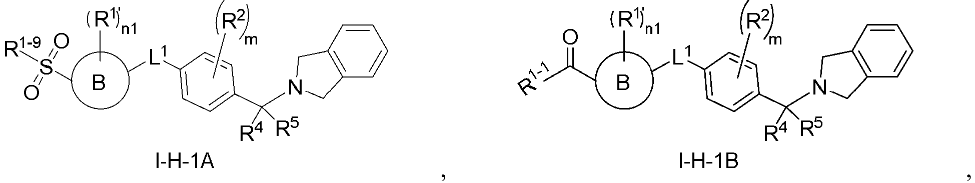

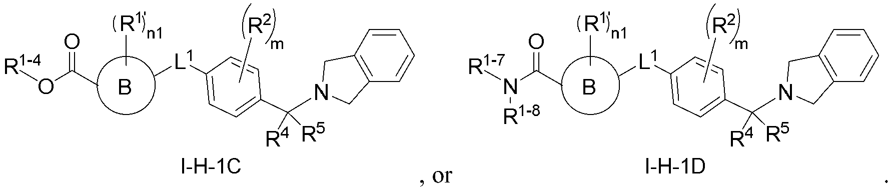

16. The compound according to any one of claims 1-9, wherein, the compound has a structure represented by formula (I-6), formula (I-7), formula (I-8), formula (I-23), formula (I-6') or formula (I-7'): 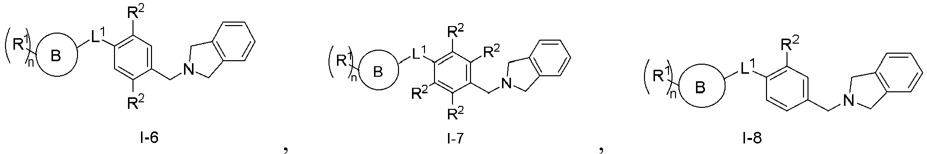 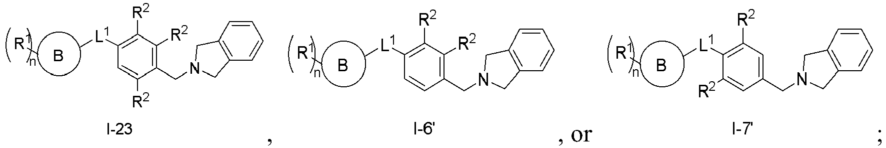 the L¹ is a bond, O, S, -NH-, C₁₋₆ alkylene that is unsubstituted or substituted by one or more L¹⁻¹, C₂₋₆ alkenylene that is unsubstituted or substituted by one or more L¹⁻², *-C₁₋₆ alkylene-S-, *-S-C₁₋₆ alkylene-, *-C₁₋₆ alkylene-O-, *-O-C₁₋₆ alkylene-, preferably *-CH₂-S-, *-CH₂CH₂-S-, *-CH₂-O-, ethylene or vinylene, more preferably *-CH₂-O-, ethylene or vinylene, more preferably ethylene or vinylene.

17. The compound according to any one of claims 1-9, wherein, the compound has a structure represented by formula (I-10), formula (I-11), formula (I-12), formula (I-13), formula (1-12'), formula (I-13'), formula (I-121), formula(I-131), formula(I-120), or formula (I-130): 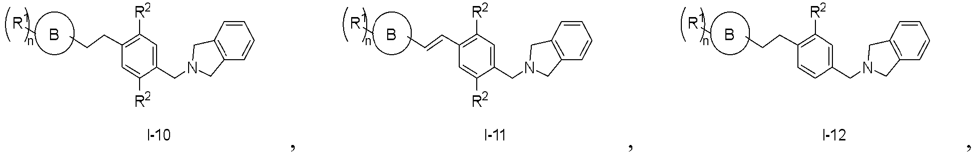 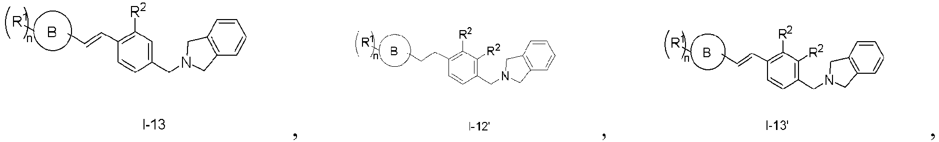 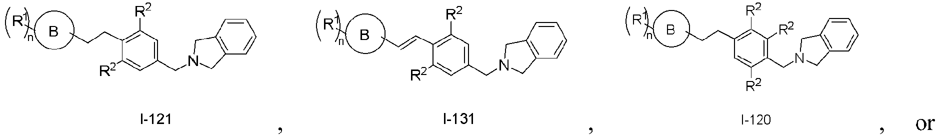 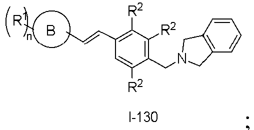 ring B is preferably 5-10 membered heterocyclyl, more preferably 5-10 membered nitrogen-containing heterocyclyl, further more preferably

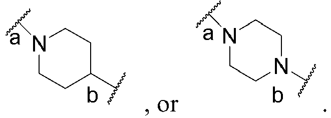

18. The compound according to any one of claims 1-9, wherein, the compound has a structure represented by formula (I-16), formula (I-17), formula (I-18), formula (I-170), formula (I-171) or formula (I-172):

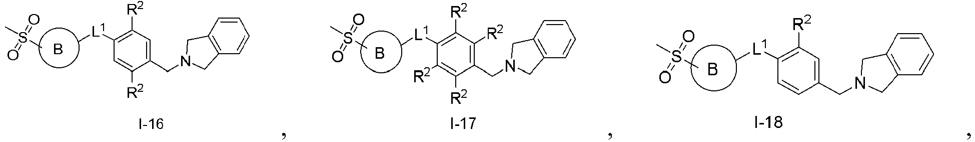

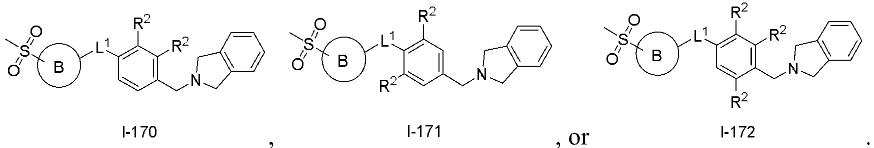

19. The compound according to any one of claims 1-9, wherein, the compound has a structure represented by formula (I-B-1): 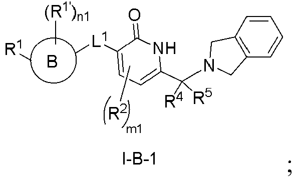 wherein:
R¹ and R^{1'} are each independently deuterium, halogen, cyano, hydroxy, C₁₋₆ alkoxy, R¹⁻¹, -C(=O)-R¹⁻¹, -C(=O)-OR¹⁻⁴, -NR¹⁻⁷R¹⁻⁸, -C(=O)-NR¹⁻⁷R¹⁻⁸, -C(=NH)-(R¹⁻⁹), -S(O)₂-R¹⁻⁹, -S(O)₂-NR¹⁻⁵R¹⁻⁶, -NR¹⁻⁵S(O)₂-R¹⁻¹⁰, -R¹⁻¹⁰-OH, or -P(=O)-R¹⁻²R¹⁻³;
R¹⁻¹, R¹⁻², R¹⁻³, R¹⁻⁴, R¹⁻⁵, R¹⁻⁶, R¹⁻⁷ and R¹⁻⁸ are each independently H, C₁₋₆ alkyl, C₁₋₆ haloalkyl, C₁₋₆ deuteroalkyl, -C₁₋₆ alkylene-OH, -C₁₋₆ alkylene-CN, -C₃₋₁₀ cycloalkyl, or 3-10 membered heterocyclyl, wherein the heteroatom in the 3-10 membered heterocyclyl is selected from N, O and S, and the number of the heteroatom is 1, 2, 3, or 4; preferably, R¹⁻¹, R¹⁻², R¹⁻³, R¹⁻⁴, R¹⁻⁵, R¹⁻⁶, R¹⁻⁷ and R¹⁻⁸ are each independently H, C₁₋₄ alkyl, C₁₋₄ haloalkyl, C₁₋₄ deuteroalkyl, -C₁₋₄ alkylene-OH, -C₁₋₄ alkylene-CN, -C₃₋₆ cycloalkyl, or 3-6 membered heterocyclyl;
R¹⁻⁹ and R¹⁻¹⁰ are each independently C₁₋₆ alkyl, C₁₋₆ haloalkyl, -C₁₋₆ alkylene-CN, C₂₋₆ alkenyl, C₂₋₆ haloalkenyl, C₃₋₁₀ cycloalkyl, C₆₋₁₀ aryl, 5-10 membered heteroaryl, C₁₋₆ alkyl-C₆₋₁₀ arylene-; preferably, R¹⁻⁹ and R¹⁻¹⁰ are each independently C₁₋₄ alkyl, C₁₋₄ haloalkyl, -C₁₋₄ alkylene-CN, C₂₋₄ alkenyl, C₂₋₄ haloalkenyl, C₃₋₆ cycloalkyl, C₆₋₁₀ aryl, 5-10 membered heteroaryl, C₁₋₄ alkyl-C₆₋₁₀ arylene-;
preferably R¹ is -C(=O)-R¹⁻¹, -C(=O)-OR¹⁻⁴, -C(=O)-NR¹⁻⁷R¹⁻⁸, -C(=NH)-(R¹⁻⁹), -S(O)₂-R¹⁻⁹, -S(O)₂-NR¹⁻⁵R¹⁻⁶, -NR¹⁻⁵S(O)₂-R¹⁻¹⁰ or -P(=O)-R¹⁻² R¹⁻³; R^{1'} is deuterium, halogen, cyano, hydroxy, C₁₋₆ alkoxy, R¹⁻¹, -NR¹⁻⁷R¹⁻⁸, -R¹⁻¹⁰-OH;
preferably R¹ is -C(=O)-R¹⁻¹, -C(=O)-OR¹⁻⁴, -S(O)₂-R¹⁻⁹; R^{1'} is hydrogen, deuterium, halogen, cyano, hydroxy, C₁₋₆ alkoxy, -NH₂, -NH(C₁₋₆ alkyl), -N(C₁₋₆ alkyl)₂, C₁₋₆ alkyl, C₁₋₆ haloalkyl, C₁₋₆ deuteroalkyl, -C₁₋₆ alkylene-OH, -C₁₋₆ alkylene-CN, -C₃₋₁₀ cycloalkyl, or 3-10 membered heterocyclyl;
preferably R¹ is -S(O)₂-C₁₋₆ alkyl, -S(O)₂-C₃₋₁₀ cycloalkyl, -C(=O)-OC₁₋₆ alkyl, -C(= O)-C₁₋₆ alkyl, -C(=O)-C₁₋₆ alkylene-CN; R^{1'} is hydrogen, deuterium, halogen, cyano, hydroxy, -NH₂, C₁₋₆ alkyl, C₁₋₆ haloalkyl, C₁₋₆ deuteroalkyl, C₁₋₆ alkoxy;
preferably R¹ is -S(O)₂-C₁₋₄ alkyl, -S(O)₂-C₃₋₆ cycloalkyl, -C(=O)-OC₁₋₄ alkyl, -C(=O)-C₁₋₄ alkyl, -C(=O)-C₁₋₄ alkylene-CN; R^{1'} is hydrogen, deuterium, halogen, cyano, hydroxy, -NH₂, C₁₋₄ alkyl, C₁₋₄ haloalkyl, C₁₋₄ deuteroalkyl, C₁₋₄ alkoxy;
n1 is each independently 0, 1, 2, or 3; preferably, n1 is each independently 0, 1, or 2; more preferably, n1 is 0;
ring B is 5-12 membered heterocyclyl, wherein the heteroatom in the 5-12 membered heterocyclyl is selected from N, O and S, and the number of the heteroatom is 1, 2, 3, or 4; preferably ring B is 5-6 membered monocyclic heterocyclyl, 9-12 membered fused bicyclic heterocyclyl or 9-12 membered spirocyclic heterocyclyl; preferably ring B is 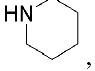 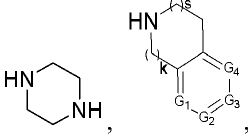 wherein, G₁, G₂, G₃, G₄ is CH or N, at least one of G₁, G₂, G₃, G₄ is CH, s and k are each independently 0, 1 or 2; preferably 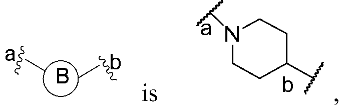 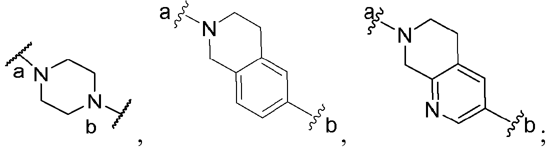
L¹ is a bond, C₁₋₆ alkylene that is unsubstituted or substituted by one or more L¹⁻¹, C₂₋₆ alkenylene that is unsubstituted or substituted by one or more L¹⁻², C₂₋₆ alkynylene that is unsubstituted or substituted by one or more L¹⁻³, *-C₁₋₆ alkylene-S- that is unsubstituted or substituted by one or more L¹⁻⁴, *-C₁₋₆ alkylene-O- that is unsubstituted or substituted by one or more L¹⁻⁶, *-O-C₁₋₆ alkylene- that is unsubstituted or substituted by one or more L¹⁻⁷, *-C₆₋₁₀ arylene-O- that is unsubstituted or substituted by one or more L¹⁻⁸, *-C₁₋₆ alkylene-C(O)- that is unsubstituted or substituted by one or more L¹⁻¹⁰;
preferably L¹ is a bond, C₁₋₄ alkylene that is unsubstituted or substituted by one or more L¹⁻¹, C₂₋₄ alkenylene that is unsubstituted or substituted by one or more L¹⁻², C₂₋₄ alkynylene that is unsubstituted or substituted by one or more L¹⁻³, *-C₁₋₄ alkylene-O- that is unsubstituted or substituted by one or more L¹⁻⁶, *-C₁₋₂ alkylene-C(O)- that is unsubstituted or substituted by one or more L¹⁻¹⁰;
each L¹⁻¹, L¹⁻², L¹⁻³, L¹⁻⁴, L¹⁻⁶, L¹⁻⁷, L¹⁻⁸, L¹⁻¹⁰ is independently halogen, OH, C₁₋₆ alkyl, C₃₋₆ cycloalkyl or 3-6 membered heterocyclyl, wherein the heteroatom in the 3-6 membered heterocyclyl is selected from N, O and S, and the number of the heteroatom is 1, 2, 3, or 4; wherein the C₁₋₆ alkyl, the C₃₋₆ cycloalkyl or the 3-6 membered heterocyclyl is optionally substituted by deuterium, halogen, OH, CN or -NH₂;
preferably L¹ is a bond, -CH₂-CH₂-, *-CH₂-CHF-, *-CH₂-CH(OH)-, *-CH₂-O-, -CH=CH-, *-CH=CF-, -C≡C-, *-CH₂-C(O)-;
preferably L¹ is -CH₂-CH₂- or -CH=CH-;
each R² is independently deuterium, halogen, CN, C₁₋₆ alkyl, C₁₋₆ haloalkyl, C₁₋₆ deuteroalkyl, -C₁₋₆ alkylene-C₃₋₁₀ cycloalkyl;
preferably R² is independently halogen, C₁₋₆ alkyl, C₁₋₆ haloalkyl, C₁₋₆ deuteroalkyl, -C₁₋₄ alkylene-C₃₋₆ cycloalkyl;
preferably R² is independently F, Cl, Br, C₁₋₄ alkyl, C₁₋₄ haloalkyl, C₁₋₄ deuteroalkyl;
m1 is 0, 1 or 2, preferably, m1 is 1 or 2; more preferably, m1 is 2;
R⁴ and R⁵ are each independently H, deuterium, halogen, C₁₋₆ alkyl; preferably, R⁴ and R⁵ are both H or deuterium, or one of R⁴ and R⁵ is H, and the other is deuterium, halogen, or C₁₋₄ alkyl.

20. The compound according to claim 19, wherein, the compound has a structure represented by formula (I-B-1A), (I-B-1B) or (I-B-1C): 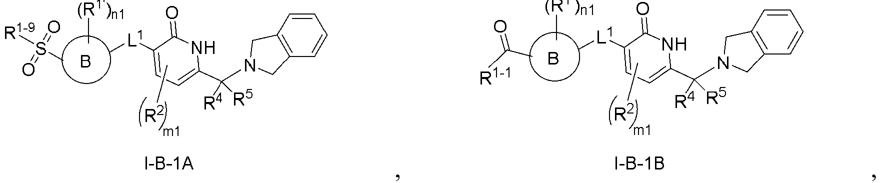 or

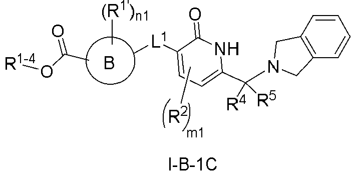

21. The compound according to claim 20, wherein, the compound has a structure represented by formula (I-B-1A-1), (I-B-1A-2), (I-B-1B-1), (I-B-1B-2), (I-B-1C-1) or (I-B-1C-2): 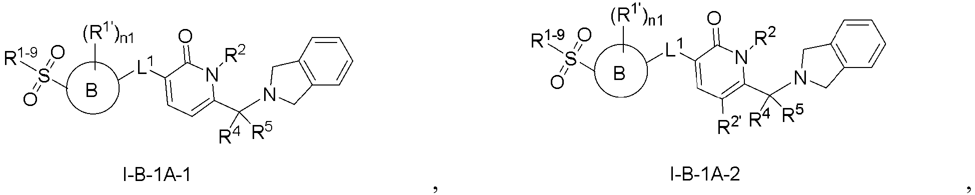 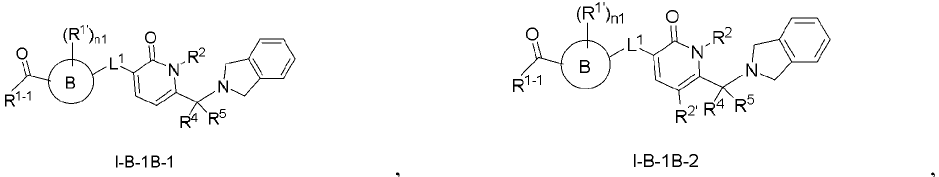 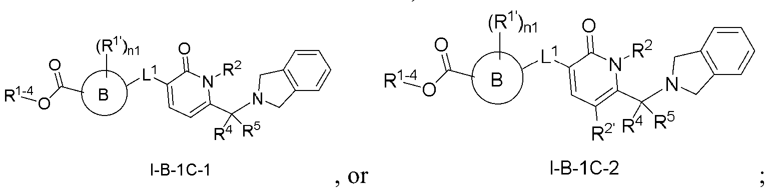 wherein, R² is independently C₁₋₆ alkyl, C₁₋₆ haloalkyl, C₁₋₆ deuteroalkyl, -C₁₋₄ alkylene-C₃₋₆ cycloalkyl; R^{2'} is halogen; preferably R² is independently C₁₋₄ alkyl, C₁₋₄ haloalkyl, C₁₋₄ deuteroalkyl; R^{2'} is F.

22. The compound according to any one of claims 1-21, wherein, the compound has a structure represented by formula (III-1), (III-2), (III-3), (III-4), (III-5), or (III-6): 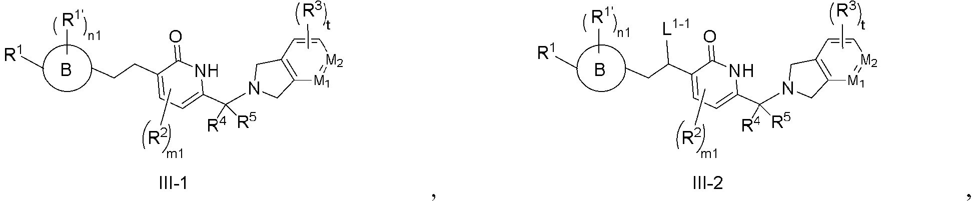 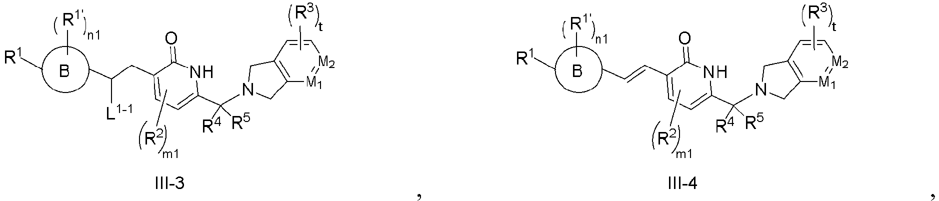 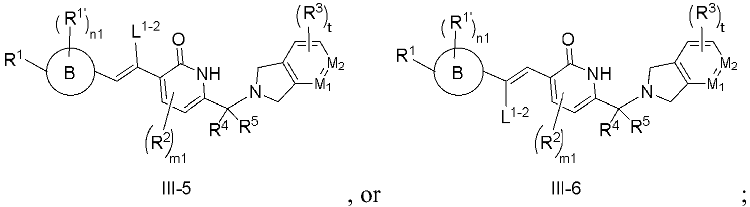 wherein, m1 is 0, 1 or 2, preferably m1 is 1 or 2, more preferably m1 is 2; M₁ and M₂ are each independentlyselected from CH or N, preferably at least one of M₁ and M₂ is selected from CH; L¹⁻¹ is selected from halogen, OH, C₁₋₄ alkyl, preferably F, OH; L¹⁻² is selected from halogen, C₁₋₄ alkyl, preferably F.

23. The compound according to any one of claims 1-22, provided that: when the 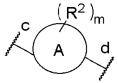 moiety is 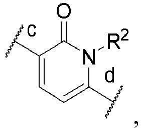 wherein the mark "c" indicates the bond connected to L¹, and the mark "d" indicates the bond connected to -C(R⁴R⁵)-; L¹ is not *-CH₂-O-, wherein the mark "*" indicates the bond connected to ring B.

24. The compound according to any one of claims 1-23, wherein, the compound is selected from:

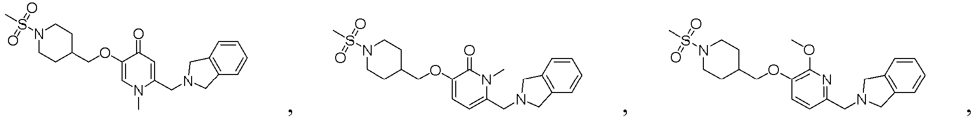

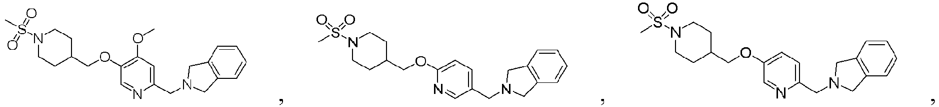

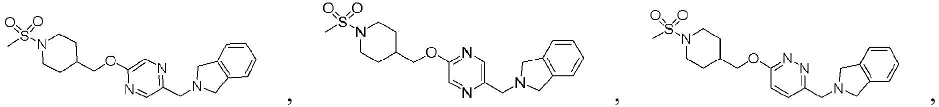

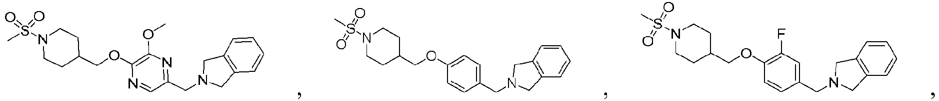

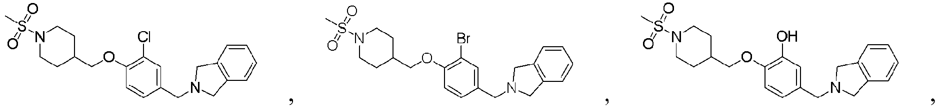

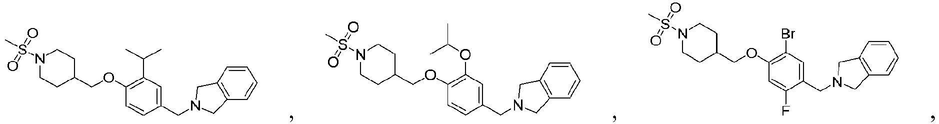

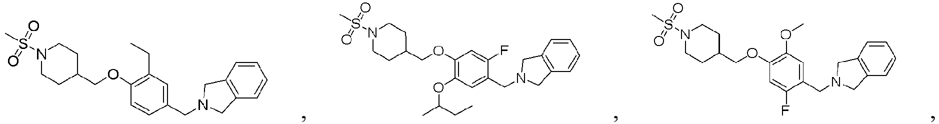

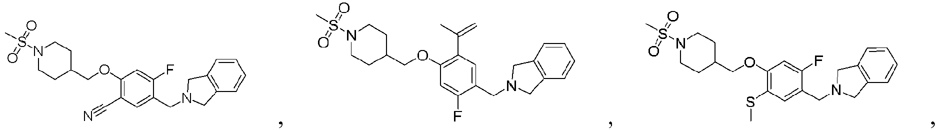

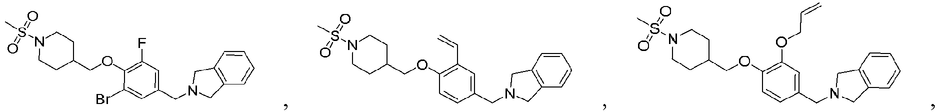

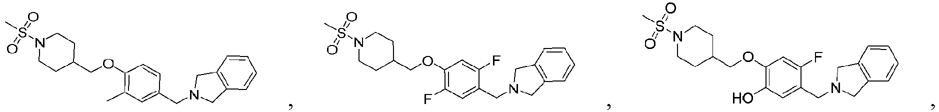

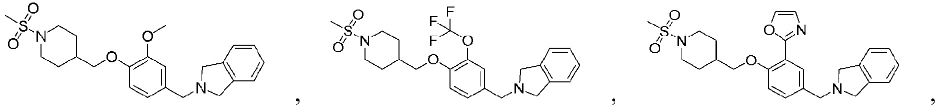

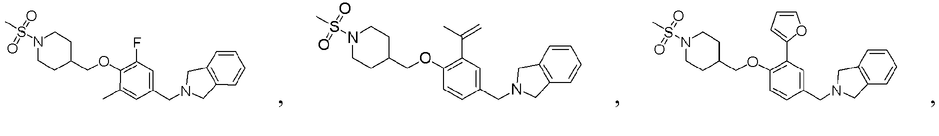

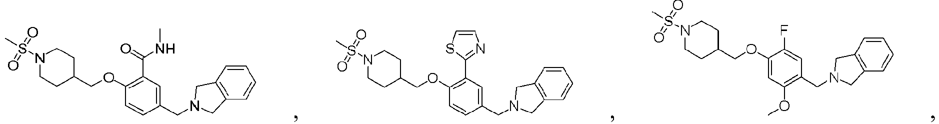

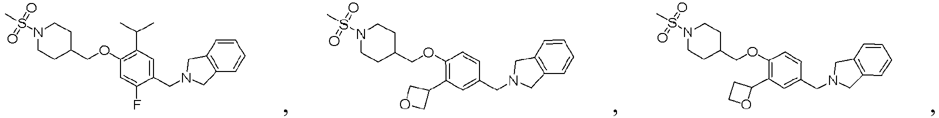

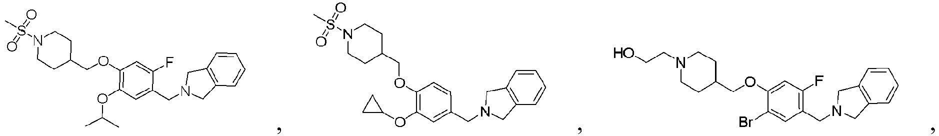

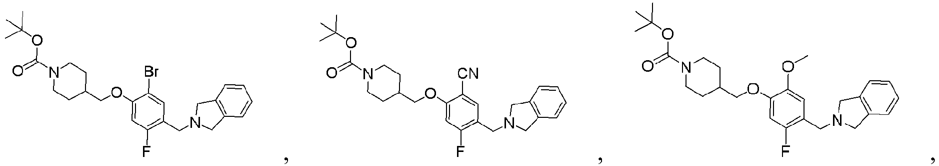

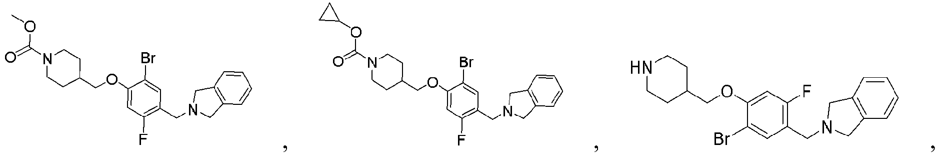

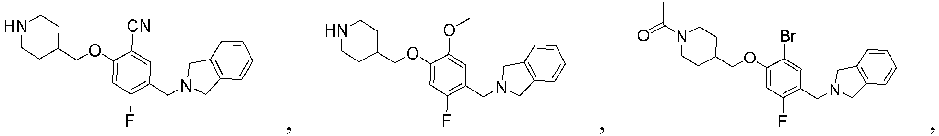

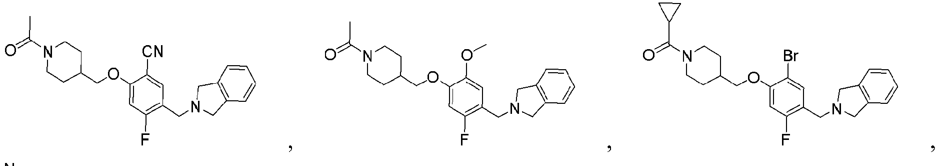

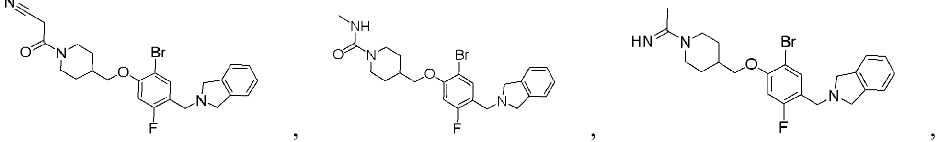

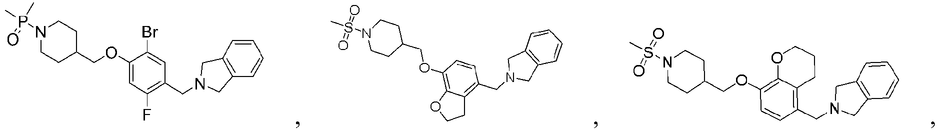

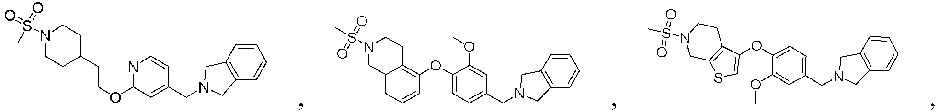

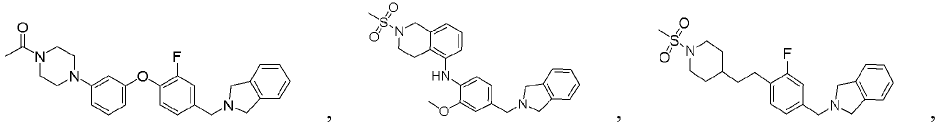

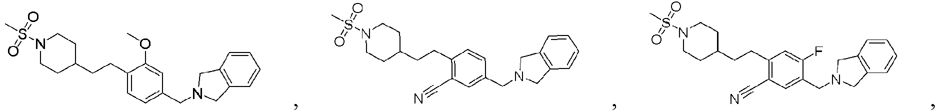

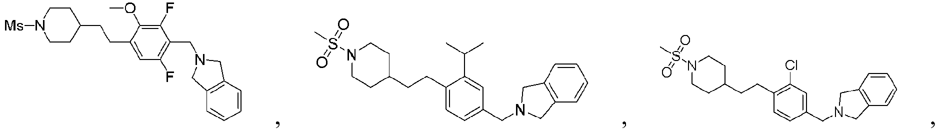

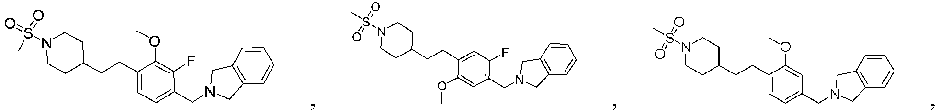

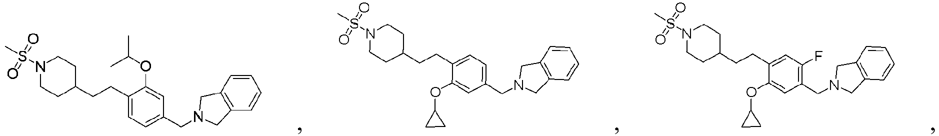

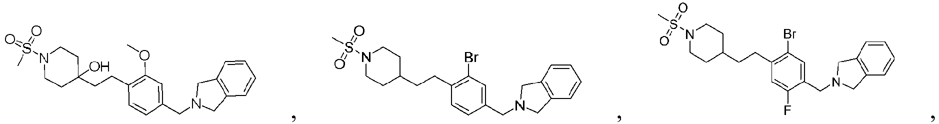

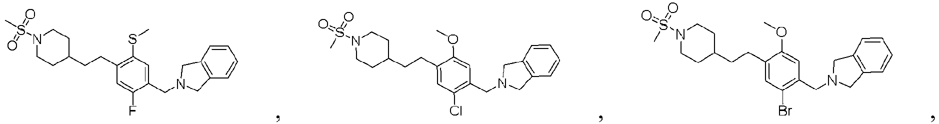

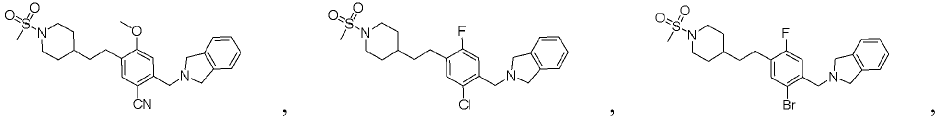

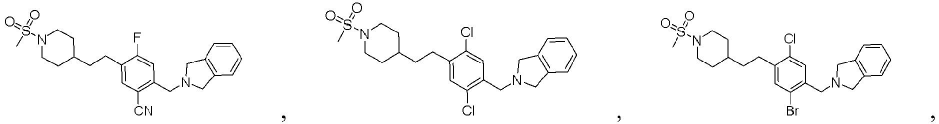

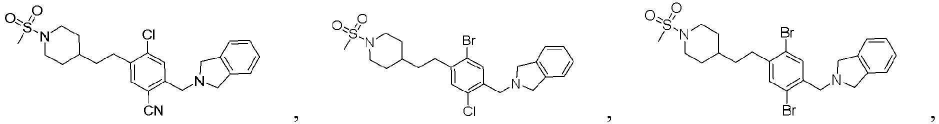

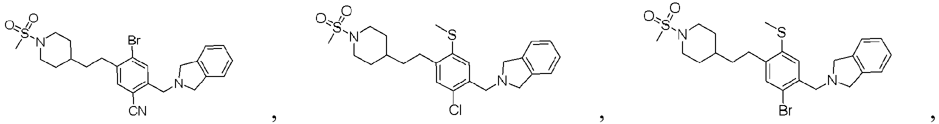

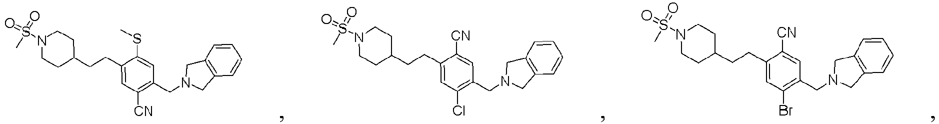

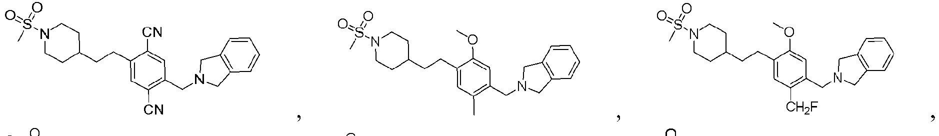

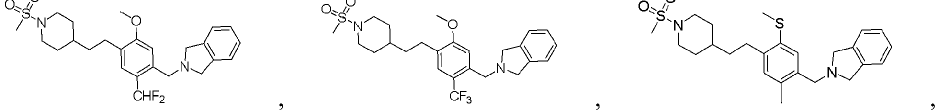

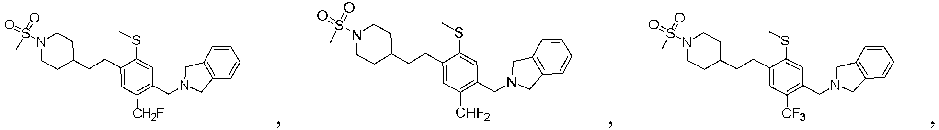

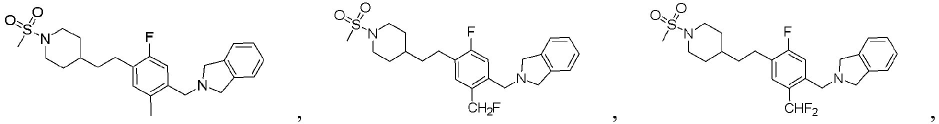

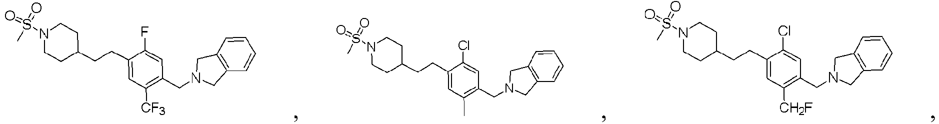

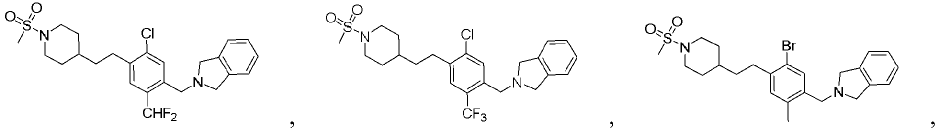

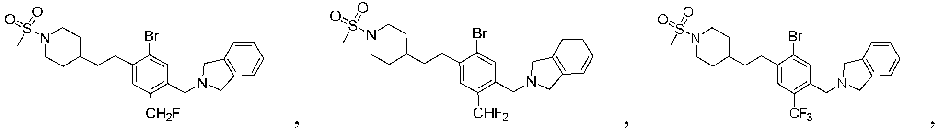

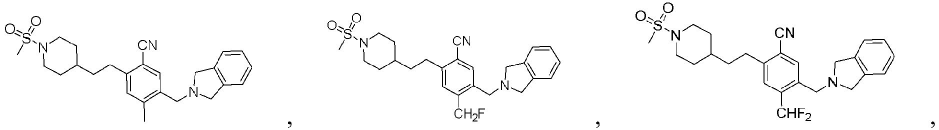

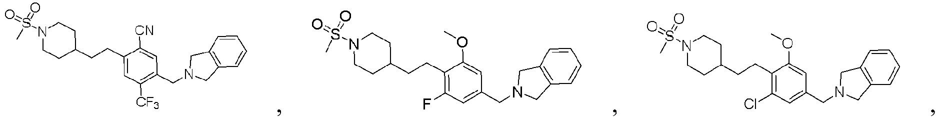

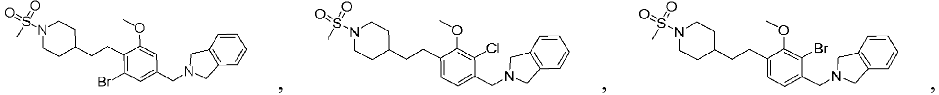

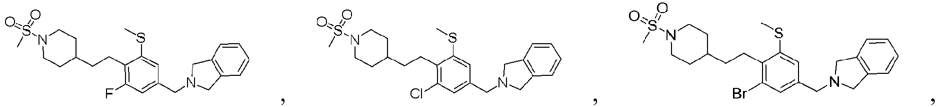

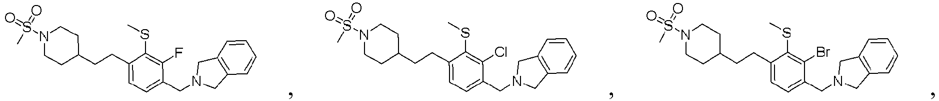

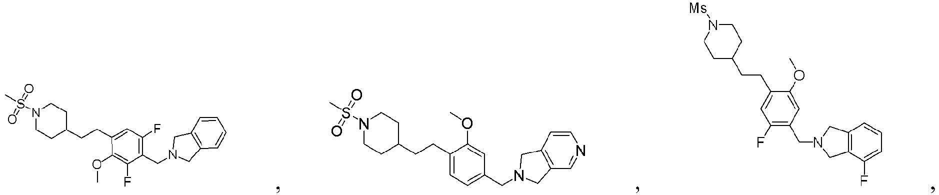

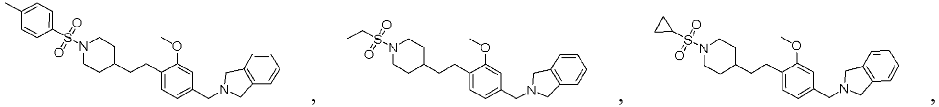

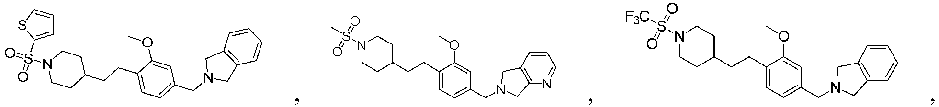

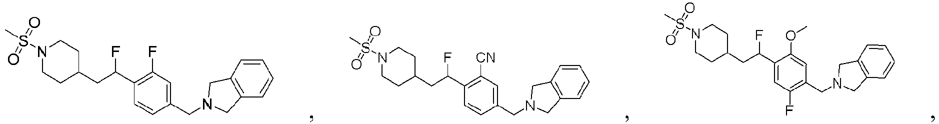

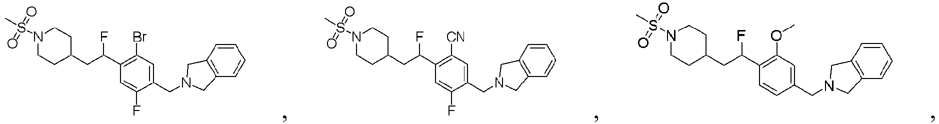

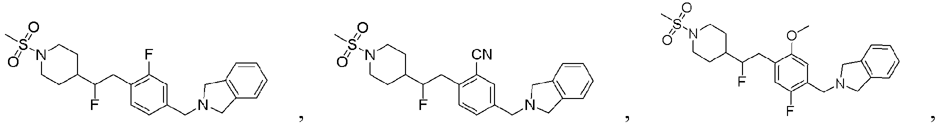

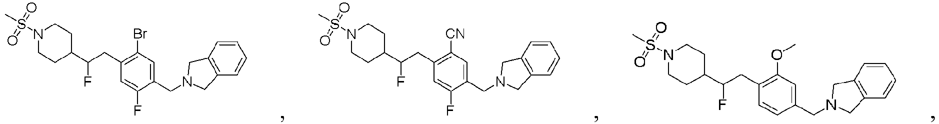

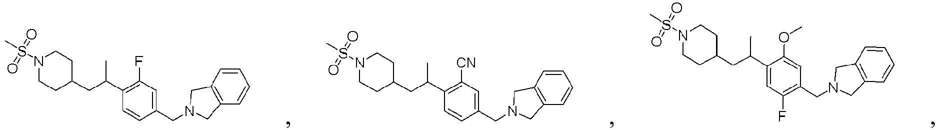

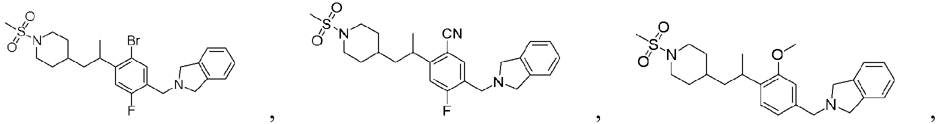

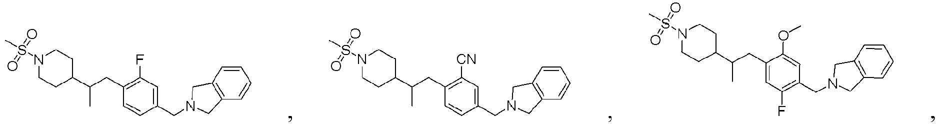

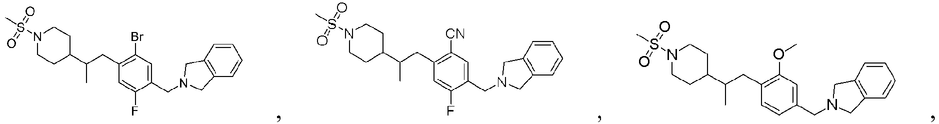

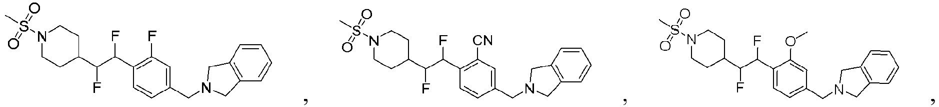

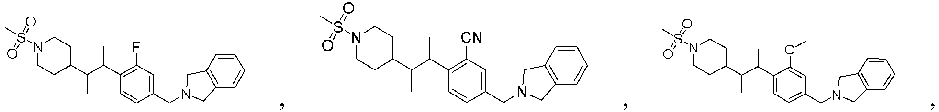

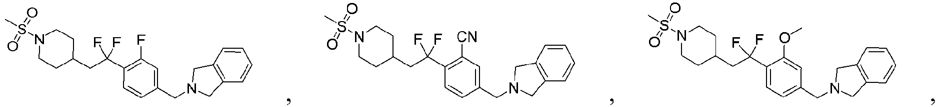

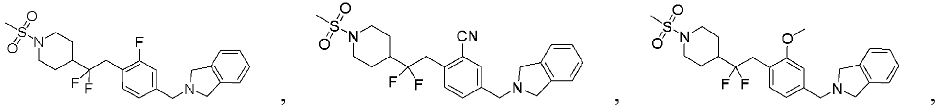

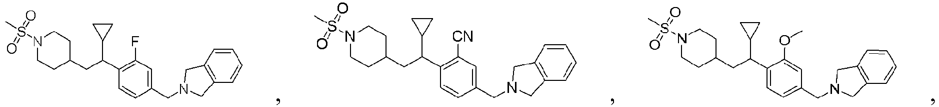

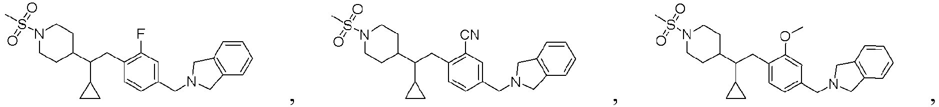

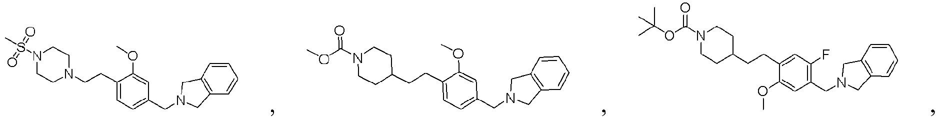

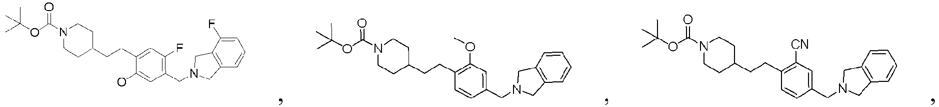

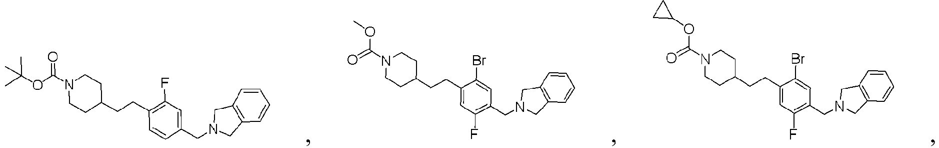

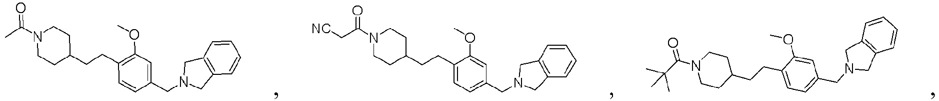

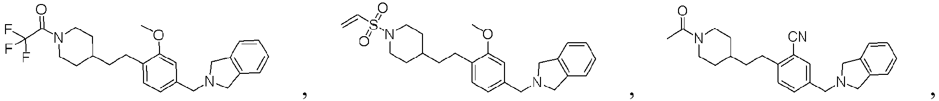

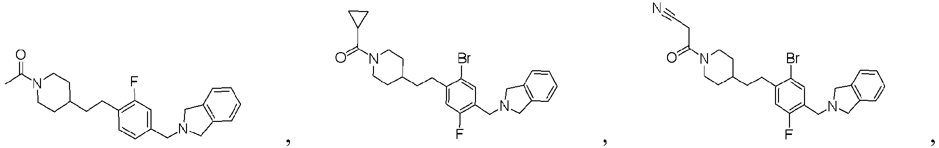

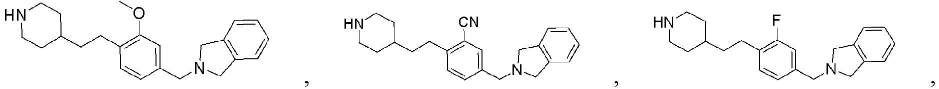

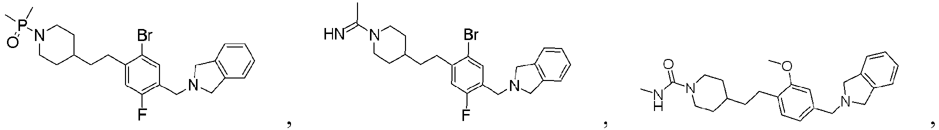

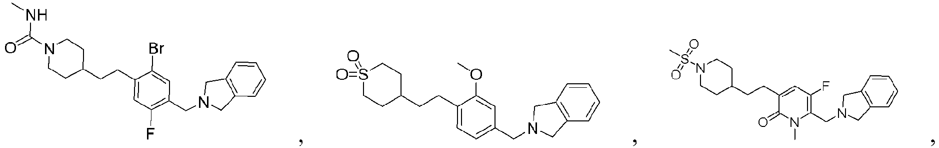

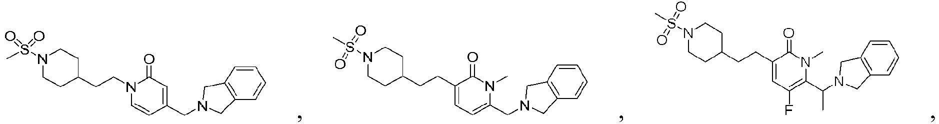

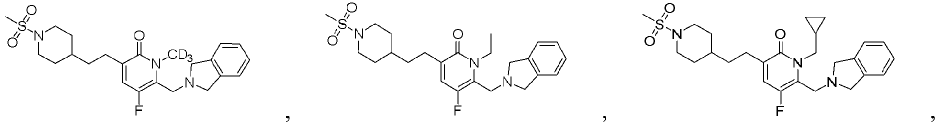

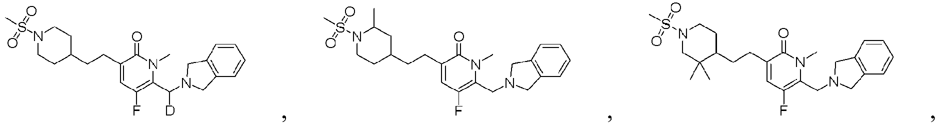

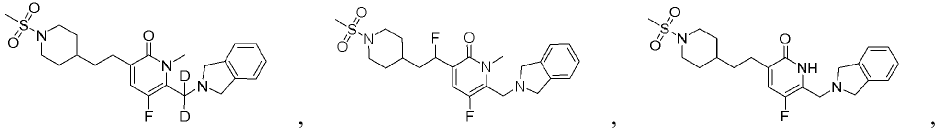

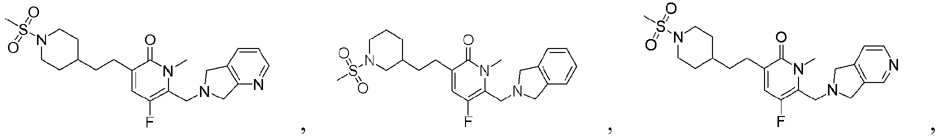

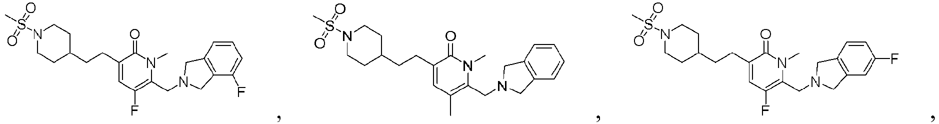

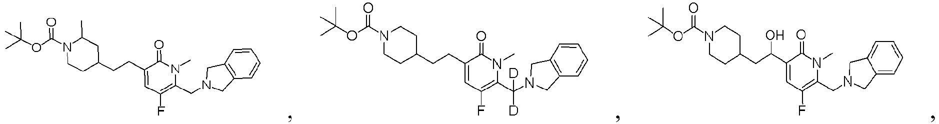

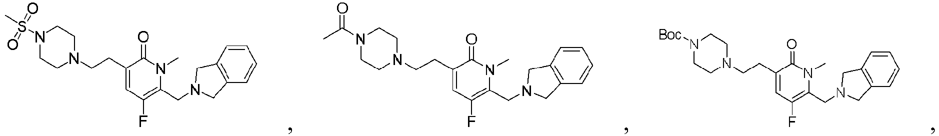

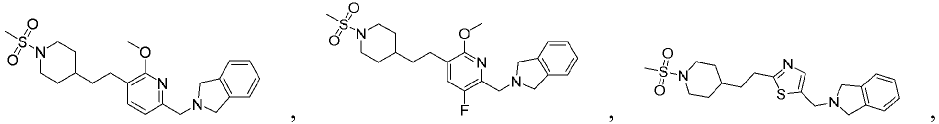

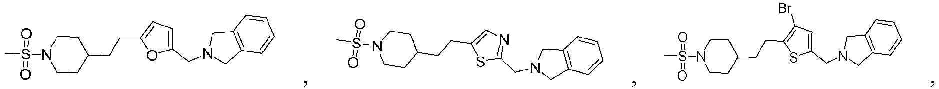

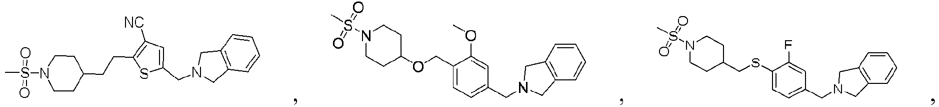

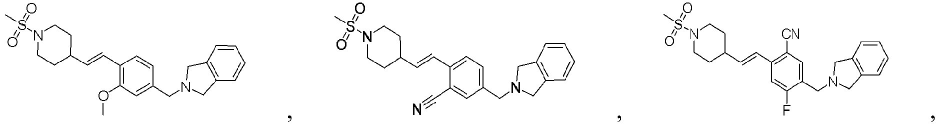

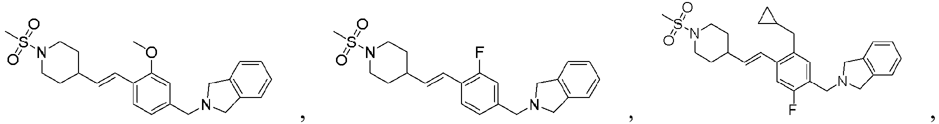

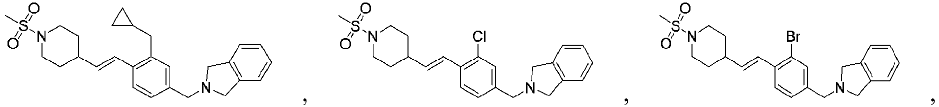

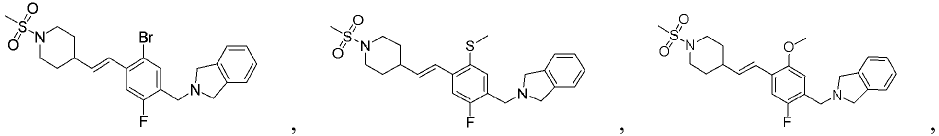

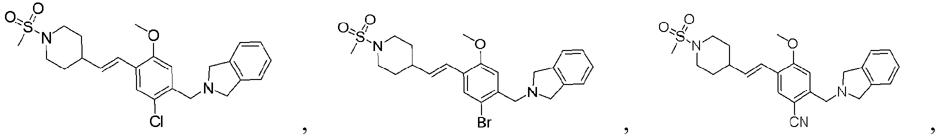

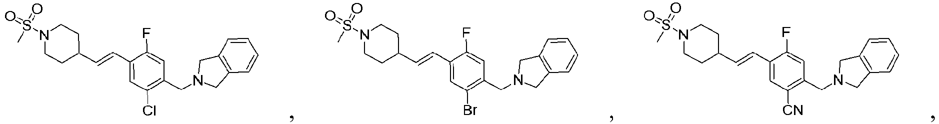

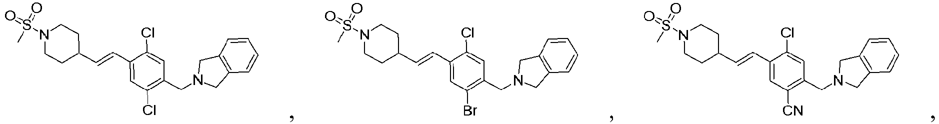

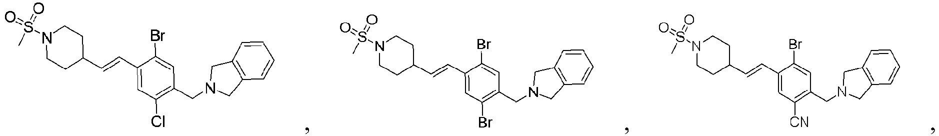

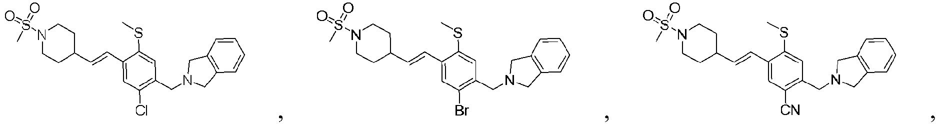

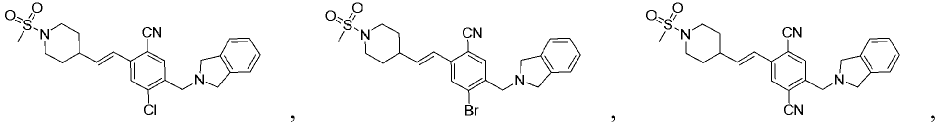

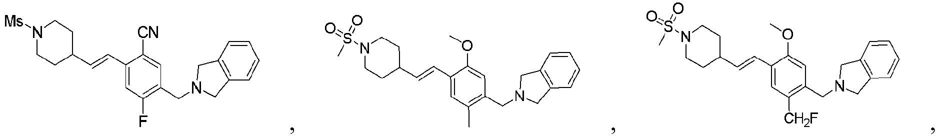

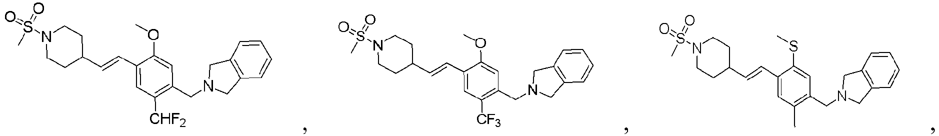

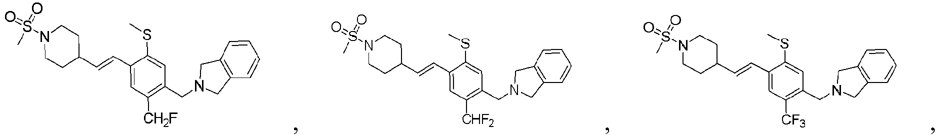

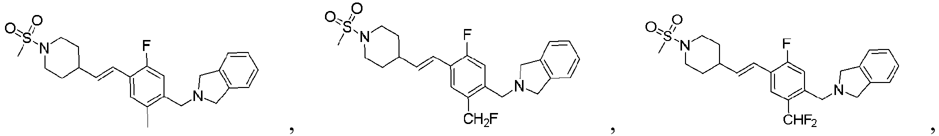

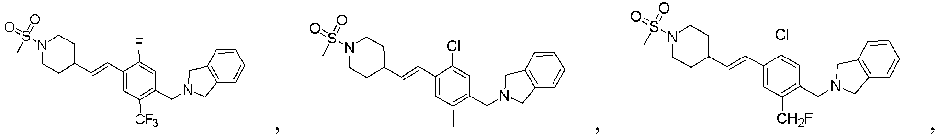

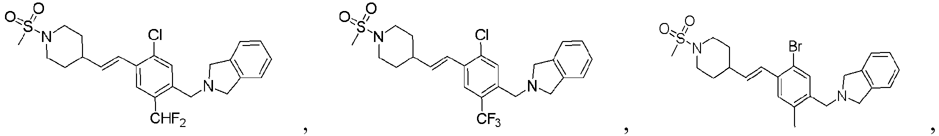

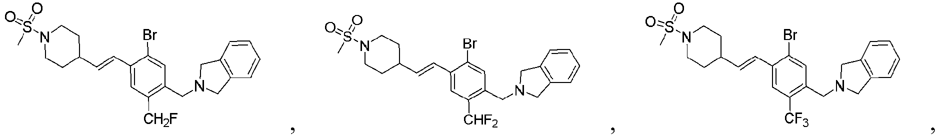

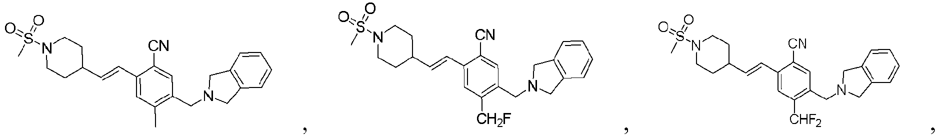

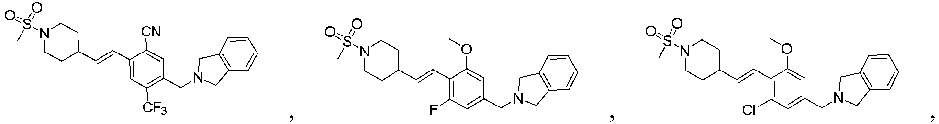

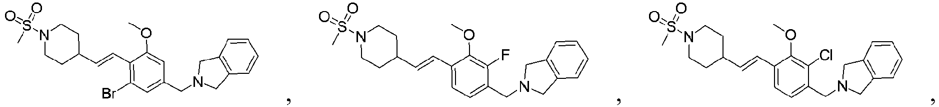

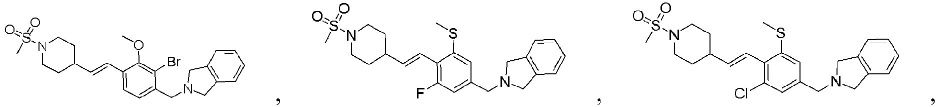

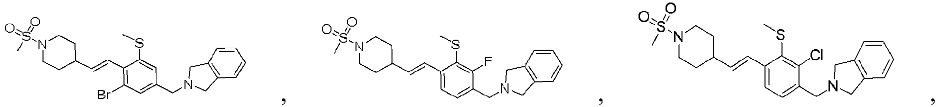

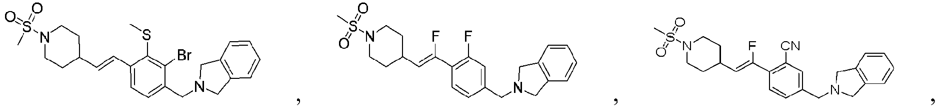

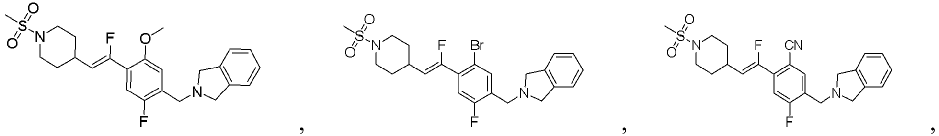

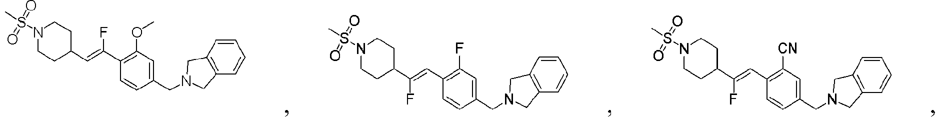

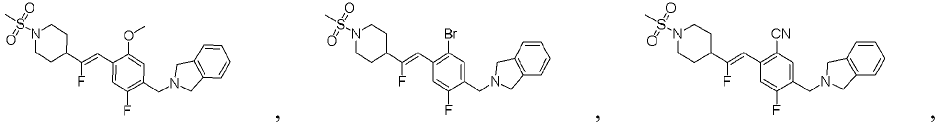

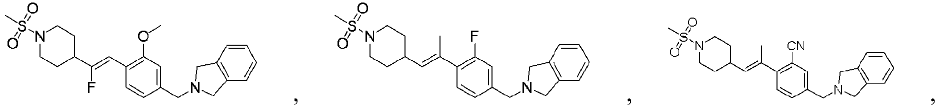

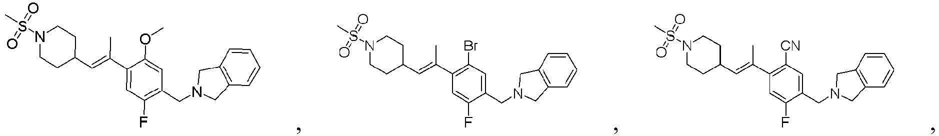

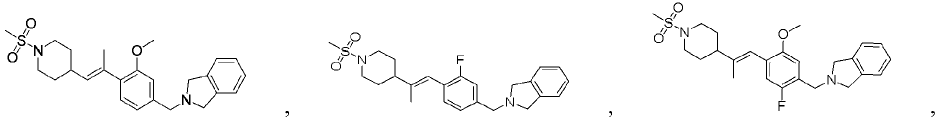

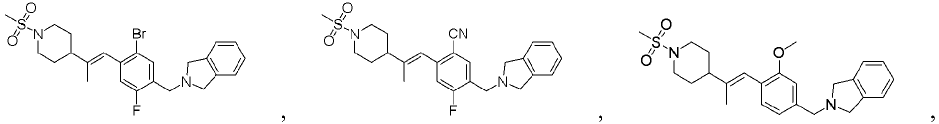

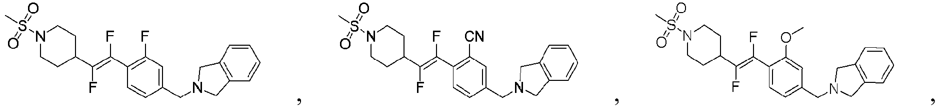

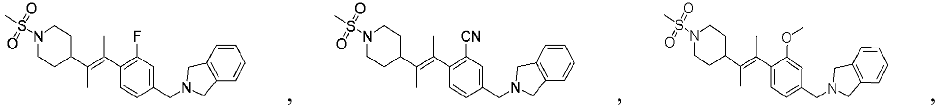

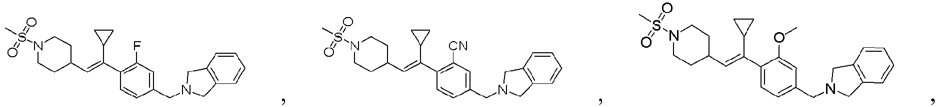

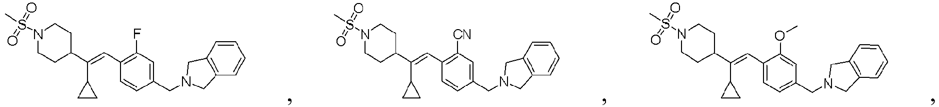

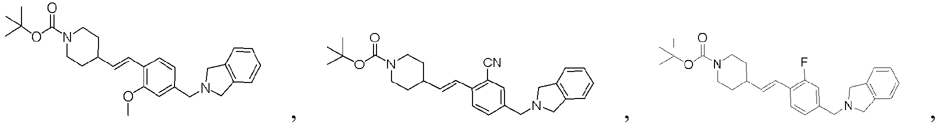

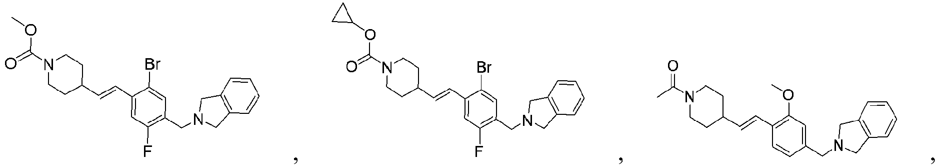

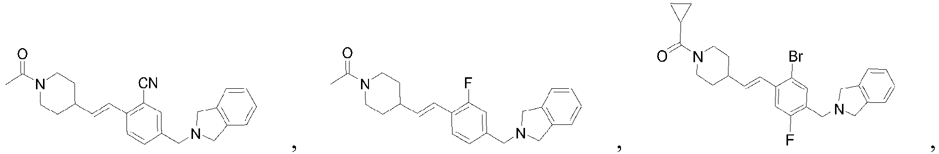

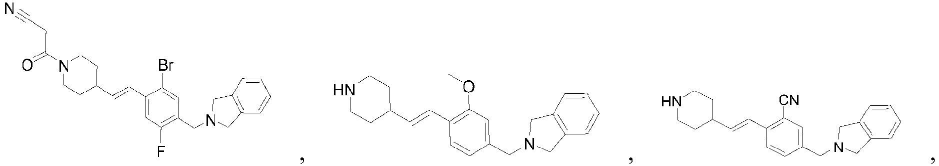

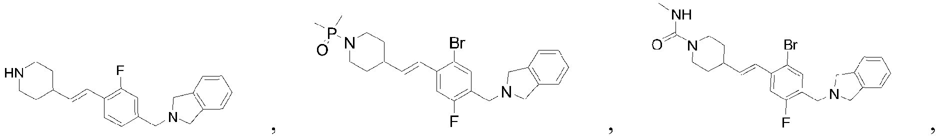

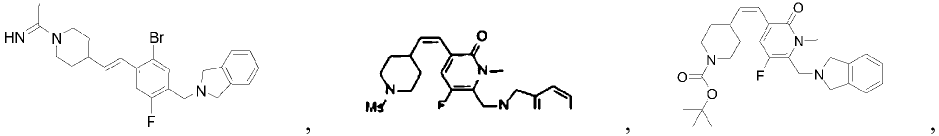

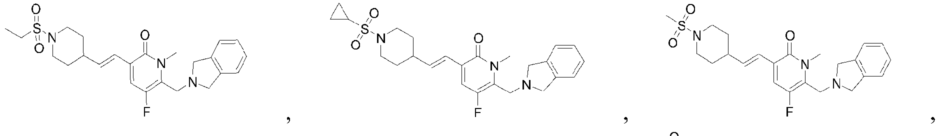

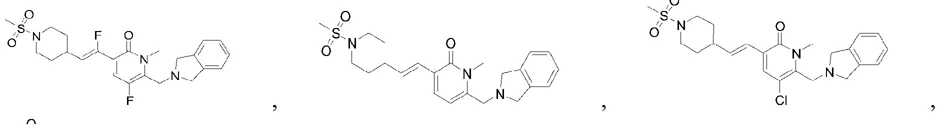

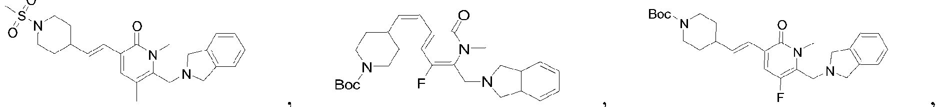

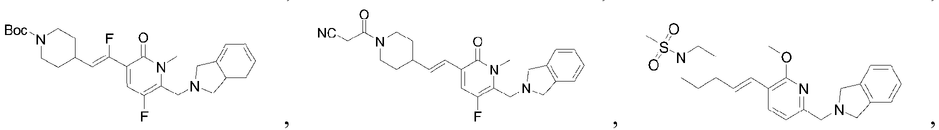

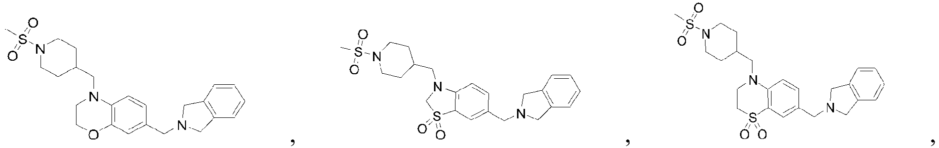

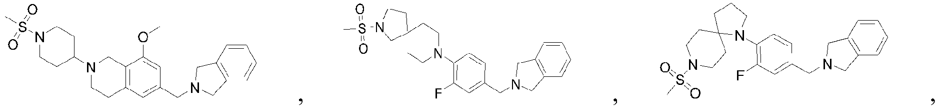

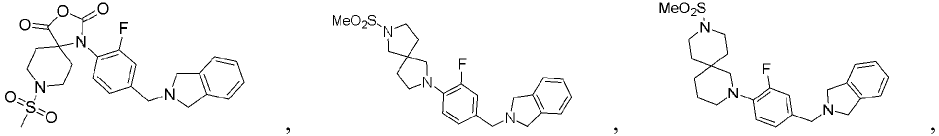

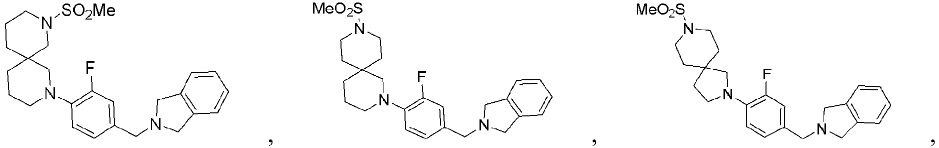

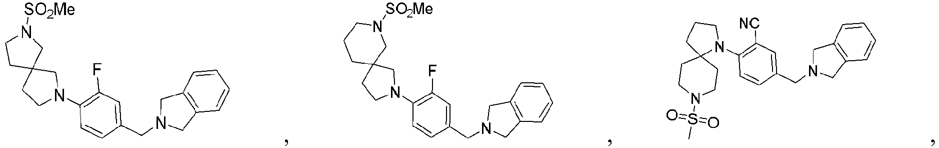

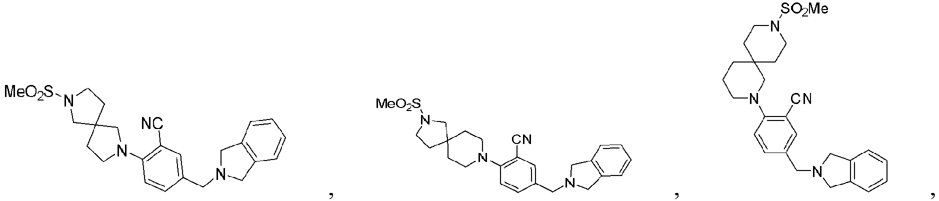

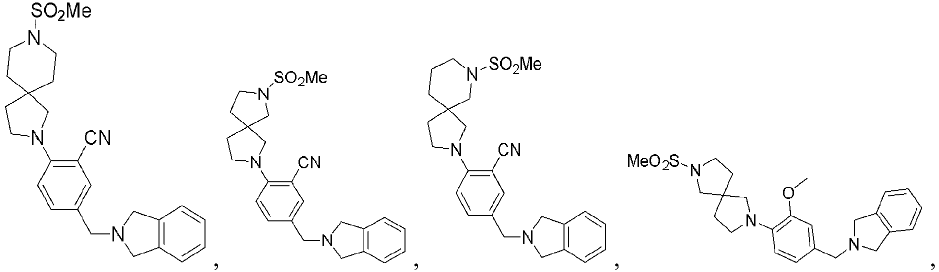

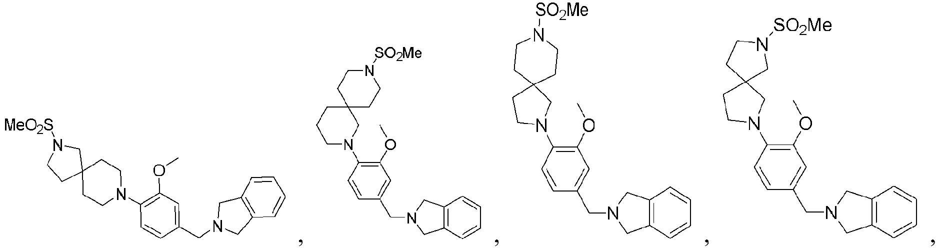

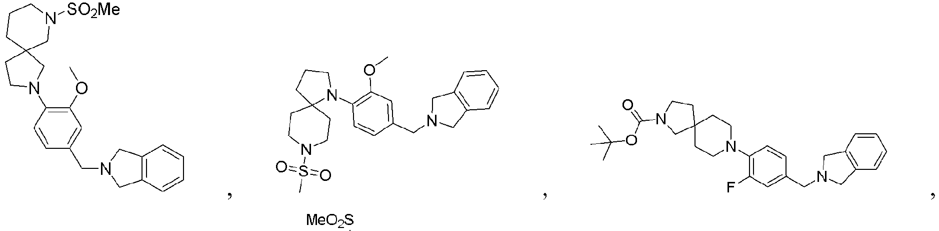

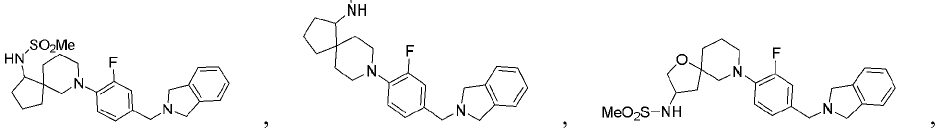

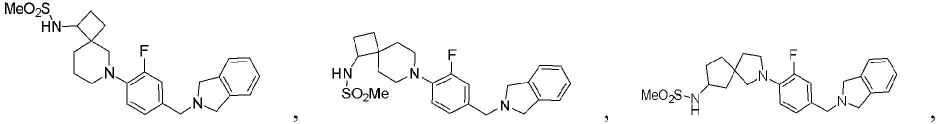

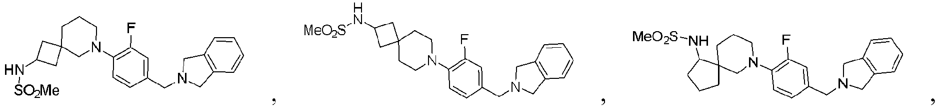

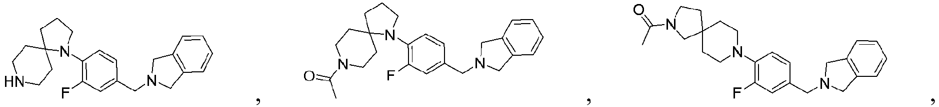

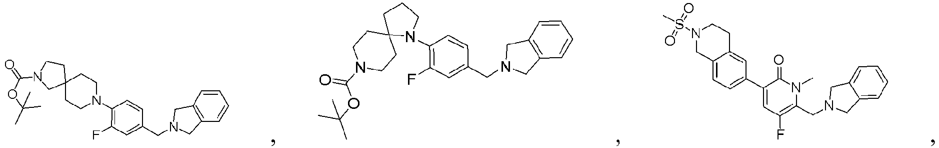

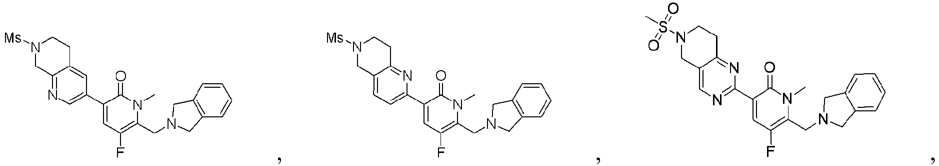

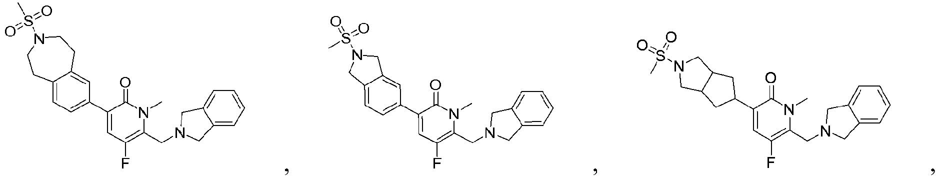

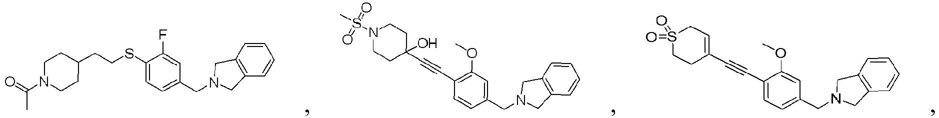

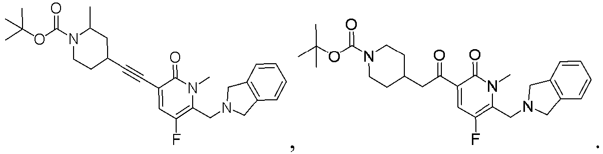

25. The compound represented by formula (I) of claim 6, wherein, the method for preparing the compound is as described in any one of the following methods: 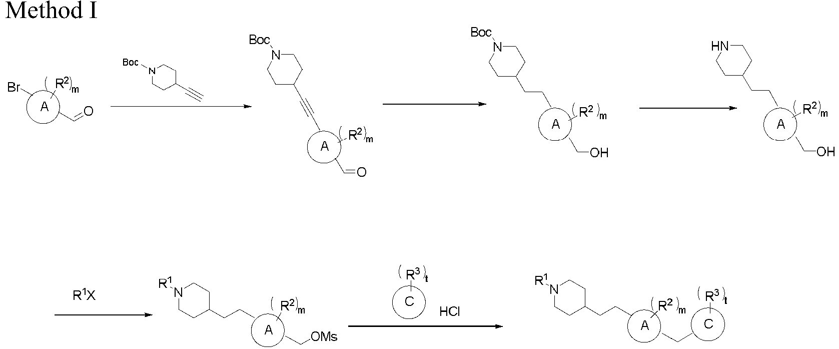
wherein ring A is preferably C₆₋₁₀ aryl; R¹ is preferably R¹⁻¹, -C(=O)-R¹⁻¹, -(CR¹⁻²R¹⁻³)_{q}-C(=O)-OR¹⁻⁴, -C(=O)-NR¹⁻⁷R¹⁻⁸, -S(O)₂-NR¹⁻⁵R¹⁻⁶, -C(=NH)-( R¹⁻⁹), -S(O)₂- R¹⁻⁹, - R¹⁻¹⁰-OH, -P(=O)-R¹⁻²R¹⁻³;
preferably, 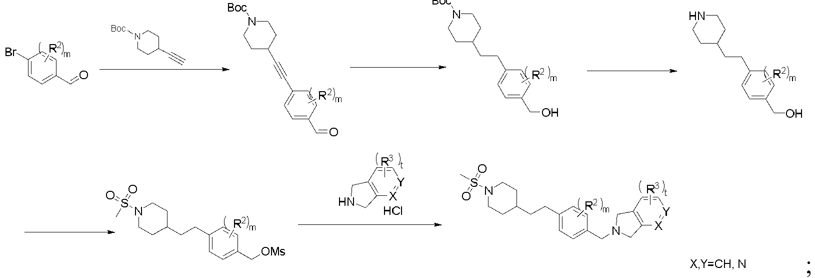 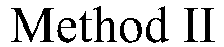 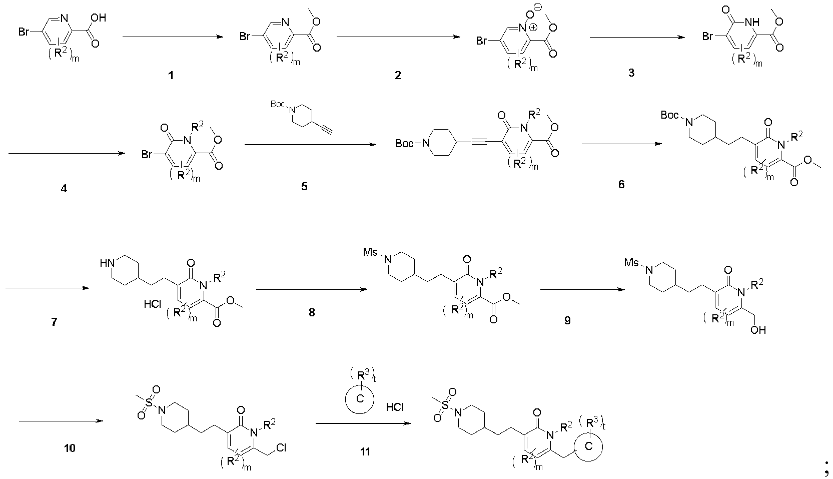
ring C is preferably 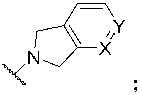
Y or Y is independently CH or N; 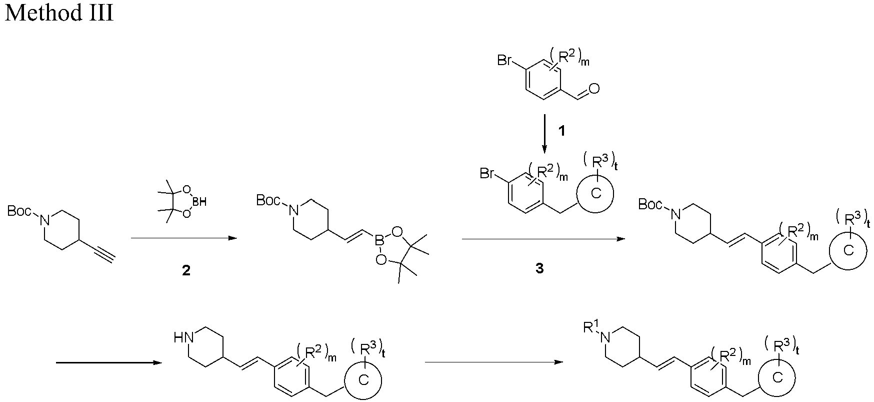
preferably,

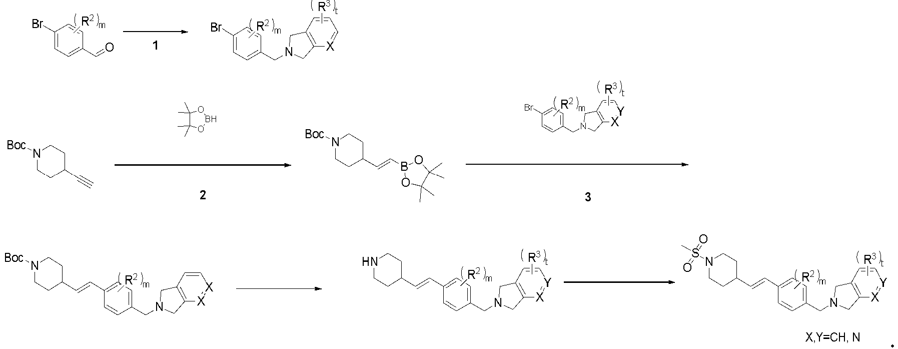

26. A pharmaceutical composition, comprising the compound according to any one of claims 1-25, or a stereoisomer, a tautomer, a diastereoisomer, a racemate, a cis-trans isomer, an isotope labeled compound (preferably deuterated compound), an N-oxide, a metabolite, an ester, a prodrug, a crystalline form, a hydrate, a solvate, or a pharmaceutically acceptable salt thereof, as well as pharmaceutically acceptable carriers;
preferably, the pharmaceutical composition comprises the compound according to any one of claims 1-25 and at least one additional active ingredient selected from the following:
- glucocorticoids;
- mineralocorticoids;
- nonsteroidal androgen receptor antagonists;
- steroidogenesis inhibitors;
- chemotherapeutic agents;
- antiestrogen drugs;
- epigenetic modulators;
- mTOR inhibitors (e.g., Everolimus);
- AKT inhibitors (e.g., AZ5363);
- radiopharmaceuticals (e.g., Alpharadin);
- GnRH/LHRH analogs (e.g., Leuprorelin);
- PI3K inhibitors; and
- CDK4/6 inhibitors.

27. A method for treating steroid receptor (particularly androgen receptor) dependent conditions and diseases by using the compound according to any one of claims 1-25 or the pharmaceutical composition according to claim 26, wherein, the method comprises administering to an individual in need thereof a therapeutically effective amount of the compound according to any one of claims 1-25 or a stereoisomer, a tautomer, a diastereoisomer, a racemate, a cis-trans isomer, an isotope labeled compound (preferably deuterated compound), an N-oxide, a metabolite, an ester, a prodrug, a crystalline form, a hydrate, solvate, or a pharmaceutically acceptable salt thereof or the pharmaceutical composition according to claim 26, wherein the steroid receptor dependent diseases is preferably cancer, preferably prostate cancer, more preferably castration-resistant prostate cancer (CRPC);
wherein administering therapeutically effective amount of the compound according to any one of claims 1-25 or the pharmaceutical composition according to claim 26, preferably also administering glucocorticoids and/or mineralocorticoids, and optionally administering with one or more anticancer drugs;
wherein administering therapeutically effective amount of the compound according to any one of claims 1-25 or the pharmaceutical composition according to claim 26 with one or more anticancer drugs, wherein the anticancer drug is preferably selected from:
- nonsteroidal androgen receptor antagonists;
- steroidogenesis inhibitors;
- chemotherapeutic agents;
- antiestrogen drugs;
- epigenetic regulators;
- mTOR inhibitors (e.g., Everolimus);
- AKT inhibitors (e.g., AZ5363);
- radiopharmaceuticals (e.g., Alpharadin);
- GnRH/LHRH analogs (e.g., Leuprorelin);
- PI3K inhibitors; and
- CDK4/6 inhibitors.
